(19) Europäisches Patentamt
European Patent Office
Office européen des brevets

(11) EP 4 010 330 B1

(12) **EUROPEAN PATENT SPECIFICATION**

(45) Date of publication and mention of the grant of the patent:
**01.10.2025 Bulletin 2025/40**

(21) Application number: **20751164.3**

(22) Date of filing: **07.08.2020**

(51) International Patent Classification (IPC):
***C07D 215/233*** (2006.01) ***C07D 215/38*** (2006.01)
***C07D 401/04*** (2006.01) ***C07D 401/12*** (2006.01)
***C07D 401/14*** (2006.01) ***C07D 405/12*** (2006.01)
***C07D 405/14*** (2006.01) ***C07D 413/12*** (2006.01)
***C07D 413/14*** (2006.01) ***C07D 471/04*** (2006.01)
***C07D 487/04*** (2006.01) ***A61P 25/00*** (2006.01)
***A61P 29/00*** (2006.01) ***A61P 35/00*** (2006.01)
***A61K 31/47*** (2006.01) ***A61K 31/4709*** (2006.01)
***A61K 31/506*** (2006.01) ***A61K 31/501*** (2006.01)
***A61K 31/497*** (2006.01) ***A61K 31/5377*** (2006.01)

(52) Cooperative Patent Classification (CPC):
**C07D 401/12; A61P 3/00; A61P 9/00; A61P 11/06; A61P 19/10; A61P 25/00; A61P 27/02; A61P 29/00; A61P 31/12; A61P 35/00; C07D 215/233; C07D 215/38; C07D 401/04; C07D 401/14; C07D 405/12;** (Cont.)

(86) International application number:
**PCT/EP2020/072322**

(87) International publication number:
**WO 2021/023888 (11.02.2021 Gazette 2021/06)**

(54) **QUINOLINE DERIVATIVES AS PROTEIN KINASE INHIBITORS**

CHINOLINDERIVATE ALS PROTEINKINASEINHIBITOREN

DÉRIVÉS DE QUINOLINE UTILISÉS COMME INHIBITEURS DE PROTÉINE KINASE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(30) Priority: **08.08.2019 EP 19190898**

(43) Date of publication of application:
**15.06.2022 Bulletin 2022/24**

(60) Divisional application:
**25197739.3**

(73) Proprietor: **Hirundo Biosciences**
**4000 Liège (BE)**

(72) Inventors:
- **AMIABLE, Claire**
**34170 Castelnau-le-lez (FR)**
- **SURLERAUX, Dominique**
**1400 Nivelles (BE)**
- **DIEUDONNÉ, François-Xavier**
**95740 Frépillon (FR)**
- **LOUAT, Thierry**
**4102 Ougrée (BE)**
- **DEROO, Sabrina**
**57330 Roussy le Village (FR)**
- **GUILLON, Remi**
**34570 Montarnaud (FR)**

(74) Representative: **Gevers Patents**
**De Kleetlaan 7A**
**1831 Diegem (BE)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(56) References cited:
**EP-A1- 3 293 177** **WO-A1-2006/116713**
**WO-A1-2010/039248** **WO-A1-2017/049462**

- **KUBO KAZUO ET AL: "Synthesis and structure-activity relationship for new series of 4-Phenoxyquinoline derivatives as specific inhibitors of platelet-derived growth factor receptor tyrosine kinase", BIOORGANIC & MEDICINAL CHEMISTRY, vol. 11, no. 23, 2003 - 2003, pages 5117 - 5133, XP085048719, ISSN: 0968-0896, DOI: 10.1016/J.BMC.2003.08.020**
- **J. ZHANG ET AL: "Recent Advances in Antiangiogenic Agents with VEGFR as Target", MINI REVIEWS IN MEDICINAL CHEMISTRY, vol. 11, no. 11, 1 October 2011 (2011-10-01), NL, pages 920 - 946, XP055660190, ISSN: 1389-5575, DOI: 10.2174/138955711797068355**

(52) Cooperative Patent Classification (CPC): (Cont.)
**C07D 405/14; C07D 413/12; C07D 413/14; C07D 471/04; C07D 487/04**

## Description

### Field of the invention

The present invention is in the field of medicinal chemistry and pharmaceuticals.

### Background of the invention

Protein phosphorylation is the most common form of reversible post-translational modification, with an estimated 50% of all proteins undergoing phosphorylation. The phosphorylation state of any given protein is controlled by the coordinated action of specific kinases and phosphatases that add and remove phosphate, respectively. Particularly, protein kinases are a kind of protein phosphotransferases bringing the phosphate of ATP to the specific amino acid residue. They may conventionally be divided into five classes: tyrosine protein kinases, serine/threonine protein kinases, histidine protein kinases, tryptophan protein kinases and aspartyl/glutamoyl protein kinases.
Signaling networks that employ phosphorylation to modulate target activities have been shown to be critically involved in all aspects of cellular function, the abnormal activation of protein phosphorylation is frequently either a driver or direct consequence of the disease. Kinase signaling pathway dysregulation is associated with cancer, inflammatory disease, cardiovascular disease, neurodegenerative disease, and metabolic disease, through the constitutive activation of many downstream pathways, such as phosphatidylinositol 3-kinase/v-akt murine thymoma viral oncogene homolog 1 (PIK3/AKT), mitogen-activated protein kinase/extracellular signal regulated kinase (MAPK/ERK) and signal transducer and activator of transcription 5 (STATS). Consequently, protein kinases represent important therapeutic targets.
In tumours, the abnormal oncogenic activation of protein kinases derives from multiple types of genetic and epigenetic changes. These alterations result in increased specific activity of the kinase itself, its overexpression, or the loss of negative regulation leading to uncontrolled cellular growth and sustained malignant behaviour. The signalling networks operating in cancer cells can also contribute to innate or acquired resistance to treatment, since they are able to create the most common or rare oncogenic mutations different from tumour to tumour. Hence, the search for small-molecule inhibitors targeting the altered protein kinase molecules in tumour cells has become a major research focus in the academia and pharmaceutical companies.
Such inhibitors can be products that are derived (isolated) from sources such as plants, animals or microorganisms, or can be small-molecules that are designed (synthesized).
WO 2004/022572 discloses classes of biologically active compounds interacting with kinases, and the preparation of these compounds.
In cancerology, there are currently multiple examples of small molecule kinase inhibitors with both selectivity and suitable pharmaceutical properties that have produced meaningful clinical benefit. For instance, imatinib is utilized to inhibit BCR-ABL1 in chronic myelogenous leukemia (CML) and acute lymphoblastic leukemia with the Philadelphia chromosome; crizotinib and other ALK kinase inhibitors for cancers driven by ALKfusions; lapatinib for ERBB2/HER2-amplified tumors; gefitinib and erlotinib for *EGFR* mutated tumors; and vemurafenib for *BRAF* mutant tumors.
There is still a great need to develop potent inhibitors of protein kinase that are useful in treating the various protein kinase-related conditions.
In this sense, Kubo Kazuo et Al. (Biooganic & medicinal chemistry, volume 1, issue 23, pages 5117-5133) discloses series of 4-phenoxyquinoline derivatives as potent and selective inhibitors of the platelet-derived growth factor receptor (PDGFr) tyrosine kinase.
WO 2006/116713 discloses substituted amide derivatives as protein kinase inhibitors for prophylaxis and treatment of diseases, such as HGF mediated diseases.
WO 2010/039248 disclsoes methods for treating cancers with at least one HGF-Met inhibitor and at least one EGFR inhibitor.
EP 3 293 177 discloses tyrosine kinase inhibitor and pharmaceutical compositions comprising the same.
EP 0269574 discloses adenosine compounds for use in the treatment of hypertension, cerebrovascular disease, cardiopathy or renal insufficiency.
WO 2003/104482 discloses a composition for modulating cellular senescence and useful for the treatment of Alzheimer's disease or atherosclerosis, said composition comprising the inhibitor of protein kinase A such as adenosine 3'5'-cyclin phosphorothiolates.
WO 1996/040705 discloses adenosine kinase inhibitors compounds 149-175, 413-431, and 241-266, for use in the treatment of cardiovascular and cerebrovascular diseases, inflammation, arthritis, and cancer.
WO 2001/040245 discloses a method of cardioprotection by using adenosine A1 receptor partial or full agonists.
WO 2005/117882 discloses ligands of C5a receptor, for use in the treatment of diseases associated with metalloprotease activity such as arthritis, cancer, cardiovascular disorders, skin disorders, inflammation or allergic conditions.

WO 2011/090738 A2 discloses compounds that are able to inhibit B-RAF and B-RAF mutations and methods for treating diseases related to B-RAF and B-RAF mutation modulation.

US 2009/0325945 describes active compounds, specifically, certain imidazo[4,5-b]pyridin-2-one and oxazolo [4,5-b]pyridin-2-one compounds and analogs inhibiting RAF (e.g., B-RAF) activity in a cell, in vitro or in vivo, inhibiting receptor tyrosine kinase (RTK) activity, such as FGFR, Tie, VEGFR and/or Eph activity, for example, FGFR-1, FGFR-2, FGFR-3, Tie2, VEGFR-2 and/or EphB2 activity, in a cell, in vitro or in vivo.

US 2015/0182526: This document describes therapeutic compounds for treating proliferative disorders, cancer, etc., and more specifically certain pyrido[2,3-b]pyrazin-8-substituted compounds, which, inter alia, inhibit RAF (e.g., B-RAF) activity and inhibit receptor tyrosine kinase (RTK) activity.

J. Zhang et Al. (Mini-Reviews in Medicinal Chemistry, 2011, 11, 920-946) reviews small-molecule VEGFR inhibitors.

WO 2017/049462 A1 discloses a novel kinase inhibitor, a drug composition, and uses and methods for preventing or treating cell proliferative diseases and/or diseases related to FLT3 and c-Kit. This document also discloses used and method for preventing or treating diseases in response to FLT3 kinase inhibition.

However, despite the growing effort in developing new protein kinase inhibitors based therapies, there is still a need for protein kinase inhibitors which may overcome the disadvantages of current protein kinase therapies such as side effects, limited efficacy, the emerging of resistance, and compliance failures.

## Summary of the invention

The inventors have surprisingly found that the use of protein kinase inhibitors according to the invention allows to provide an improved treatment of dysregulated protein kinase related diseases, by developing a therapy that is more effective, that reduces side effects, that limits the emerging of resistance and that facilitates compliance.

Therefore, the present invention provides a compound (C) or the N-oxide, pharmaceutically acceptable salt, pharmaceutically acceptable solvate, or stereoisomer thereof, as defined according to claim 1.

The present invention further relates to a pharmaceutical composition comprising a carrier, and as active ingredient an effective amount of a compound as defined in any one of the embodiments presented herein.

The present invention relates to a compound as defined in any one of the embodiments presented herein, for use as a medicament.

The present invention relates to a compound as defined in any one of the embodiments presented herein for use in the treatment of a disease selected from cancer, metabolic disorders (such as diabetes), inflammatory and autoimmune disorders (such as inflammatory bowel diseases, e.g. Crohn's disease and ulcerative colitis, inflammatory pulmonary diseases, rheumatoid arthritis, lupus nephritis, systemic lupus erythematosus and psoriasis and psoriasis arthritis), neurological disorders (such as Alzheimer's disease, Parkinson's disease, multiple sclerosis, Charcot-Marie-Tooth neuropathy, amyotrophic lateral sclerosis and epilepsy), atherosclerosis and cardiovascular diseases, Sjogren Syndrome, renal allograft rejection, viral induced diseases, circulatory diseases, bone osteolysis and osteoporosis, osteoarthritis, sarcopenia, Langerhans cell histiocytosis, spinal cord injury, endometriosis, asthma and allergic asthma, eye diseases (such as retinopathies, age-related macular degeneration and uveitis) chronic and neuropathic pain, and fibroproliferative diseases.

## Detailed description of the invention

The present invention relates to a compound (C), or the N-oxide, pharmaceutically acceptable salt, pharmaceutically acceptable solvate, or stereoisomer thereof, as defined according to claim 1.

In a preferred embodiment of the present invention, $R_1$ in compound (C), independently from each other and at each occurrence, is selected from the group consisting of hydrogen, halo, $C_{1-6}$ alkyl, $C_{1-6}$ cycloalkyl, aryl, heterocyclyl, heteroaryl, $CF_3$, CN, $OR_{21}$, $N(R_{21})_2$, and $CON(R_{21})_2$, wherein said alkyl, cycloalkyl, aryl, heterocyclyl, heteroaryl group is further optionally substituted with one or more substituents selected from halo, $C_{1-6}$ alkyl, $C_{1-6}$ cycloalkyl, heterocyclyl, $CF_3$, $COR_{21}$, $N(R_{21})_2$, CN, and $OR_{21}$, and each optional alkyl, alkenyl, alkynyl, cycloalkyl, heterocyclyl, aryl or heteroaryl substituent is further optionally substituted with heterocyclyl, $N(R_{11})_2$, or $OR_{11}$, and wherein each of $R_{21}$, independently from each other and at each occurrence, is selected from the group consisting of hydrogen, $C_{1-6}$ alkyl, $C_{1-6}$ cycloalkyl, heterocyclyl, phenyl, and aralkyl, wherein said alkyl, heterocyclyl, phenyl and aralkyl substituents are optionally substituted with halo, $C_{1-6}$ alkyl, cycloalkyl, heterocyclyl, or phenyl; preferably $R_1$ is independently selected from the group consisting of hydrogen, halo, $C_{1-4}$ alkyl, cycloalkyl, heterocyclyl, heteroaryl, CN $OR_{21}$, $N(R_{21})_2$, and $CON(R_{21})_2$, wherein said alkyl, cycloalkyl, heterocyclyl, heteroaryl group is further optionally substituted with one or more substituents selected from halo, $C_{1-6}$ alkyl, $C_{1-6}$ cycloalkyl, heterocyclyl, $CF_3$, $COR_{21}$, $N(R_{21})_2$, CN, and $OR_{21}$, and each optional alkyl substituent is further optionally substituted with heterocyclyl, $N(R_{11})_2$, or $OR_{11}$,and wherein each of $R_{21}$, independently from each other and at each occurrence, is hydrogen, $C_{1-4}$ alkyl such as methyl, ethyl, propyl, isopropyl, butyl, tert-butyl, isobutyl,

optionally substituted with halo or $C_{3-6}$ cycloalkyl such as cyclopropyl, cyclobutyl, cyclopentyl, cyclohexyl; more preferably $R_1$ is independently selected from the group consisting of hydrogen, Cl, Br, F, heterocyclyl, heteroaryl, $CF_3$, CN, $OR_{21}$, $N(R_{21})_2$, and $CON(R_{21})_2$, wherein said heterocyclyl or heteroaryl group is further optionally substituted with one or more substituents selected from halo, $C_{1-6}$ alkyl, $C_{1-6}$ cycloalkyl, heterocyclyl, $CF_3$, $COR_{21}$, $N(R_{21})_2$, CN, and $OR_{21}$, and each optional alkyl substituent is further optionally substituted with heterocyclyl, $N(R_{11})_2$, or $OR_{11}$, and wherein each of $R_{21}$, independently from each other and at each occurrence, is hydrogen, $C_{1-4}$ alkyl such as methyl, ethyl, propyl, isopropyl, butyl, tert-butyl, isobutyl, optionally substituted with halo or $C_{3-6}$ cycloalkyl such as cyclopropyl, cyclobutyl, cyclopentyl, cyclohexyl.

In a preferred embodiment of the present invention, $R_2$ in compound (C), independently from each other and at each occurrence, is selected from the group consisting of of hydrogen, $C_{1-4}$ alkyl, cycloalkyl, heterocyclyl, $OR_{21}$, and $N(R_{21})_2$, and wherein each of $R_{21}$, independently from each other and at each occurrence, is hydrogen, $C_{1-4}$ alkyl such as methyl, ethyl, propyl, isopropyl, butyl, tert-butyl, isobutyl or $C_{3-6}$ cycloalkyl such as cyclopropyl, cyclobutyl, cyclopentyl, cyclohexyl; more preferably $R_2$ is independently selected from the group consisting of hydrogen and $N(R_{21})_2$, and wherein each of $R_{21}$, independently from each other and at each occurrence, is hydrogen, $C_{1-4}$ alkyl such as methyl, ethyl, propyl, isopropyl, butyl, tert-butyl, isobutyl or $C_{3-6}$ cycloalkyl such as cyclopropyl, cyclobutyl, cyclopentyl, cyclohexyl.

In a preferred embodiment of the present invention, $R_3$ in compound (C), independently from each other and at each occurrence, is selected from the group consisting of hydrogen, halo, $C_{1-10}$ alkyl, $C_{1-10}$ cycloalkyl, heterocyclyl, $CF_3$, CN, $OR_{21}$, and $N(R_{21})_2$, wherein said alkyl, cycloalkyl, and heterocyclylgroup is further optionally substituted with one or more substituents selected from halo, $C_{1-10}$ alkyl, $C_{1-10}$ cycloalkyl, $CF_3$, $N(R_{21})_2$, CN, and $OR_{21}$, and wherein each of $R_{21}$, independently from each other and at each occurrence, is selected from the group consisting of hydrogen, $C_{1-10}$ alkyl, $C_{1-10}$ cycloalkyl, heterocyclyl, aryl, and aralkyl, wherein said alkyl, heterocyclyl, aryl and aralkyl substituents are optionally substituted with halo, alkyl, cycloalkyl, heterocyclyl, or aryl; preferably $R_3$ is independently selected from the group consisting of hydrogen, halo, $C_{1-4}$ alkyl, cycloalkyl, heterocyclyl, $OR_{21}$, and $N(R_{21})_2$, and wherein each of $R_{21}$, independently from each other and at each occurrence, is hydrogen, $C_{1-4}$ alkyl such as methyl, ethyl, propyl, isopropyl, butyl, tert-butyl, isobutyl or $C_{3-6}$ cycloalkyl such as cyclopropyl, cyclobutyl, cyclopentyl, cyclohexyl; more preferably $R_3$ is independently selected from the group consisting of hydrogen, Cl, Br, F, OMe, $N(R_{21})_2$, and $C_{1-4}$ alkyl such as methyl, ethyl, propyl, isopropyl, butyl, tert-butyl, isobutyl, and wherein each of $R_{21}$, independently from each other and at each occurrence, is hydrogen, $C_{1-4}$ alkyl such as methyl, ethyl, propyl, isopropyl, butyl, tert-butyl, isobutyl or $C_{3-6}$ cycloalkyl such as cyclopropyl, cyclobutyl, cyclopentyl, cyclohexyl. Even more preferably, $R_3$ is independently chosen from the group consisting of F, methyl or $OCH_3$.

In a preferred embodiment of the present invention, $R_4$ in compound (C) is independently selected from the group consisting of hydrogen, $C_{1-6}$ alkyl, and $C_{2-5}$ alkenyl, wherein said alkyl and alkenyl are optionally substituted with a halogen atom. More preferably, each of $R_4$, independently from each other and at each occurrence is selected from the group consisting of hydrogen and $C_{1-4}$ alkyl such as methyl, ethyl, propyl, isopropyl, butyl, tert-butyl, isobutyl and the like. Even more preferably, $R_4$ is hydrogen.

In a preferred embodiment of the present invention, $R'_4$ in compound (C) is independently selected from the group consisting of hydrogen, $C_{1-6}$ alkyl, and $C_{2-5}$ alkenyl, wherein said alkyl and alkenyl are optionally substituted with a halogen atom. More preferably, each of $R'_4$, independently from each other and at each occurrence is selected from the group consisting of hydrogen and $C_{1-4}$ alkyl such as methyl, ethyl, propyl, isopropyl, butyl, tert-butyl, isobutyl and the like. Even more preferably, $R'_4$ is hydrogen.

In a preferred embodiment of the present invention, p is an integer in the range from 0 to 2.

In a preferred embodiment of the present invention, q is an integer in the range from 0 to 1.

In a preferred embodiment of the present invention, r is an integer in the range from 0 to 2.

In a preferred embodiment of the present invention, x is an integer in the range from 0 to 4. More preferably, x is an integer equal to 0, 1 or 2. Even more preferably, x is an integer equal to 0 or 1. Even more preferably, x is an integer equal to 1.

It is further understood that all preferences and preferred embodiments hereinabove, also apply for all further embodiments, as described below.

In one embodiment of the present invention, the compound (C)according to formula (IIa) to (IXa) as defined in claim 1 or the N-oxide, pharmaceutically acceptable salt, pharmaceutically acceptable solvate, or stereoisomer thereof, is used in the treatment of a disease mediated by a protein kinase, wherein the protein kinase is selected from the group consisting of CSF1R, FLT3, Kit, PDGFRB (PDGFR beta), PDGFRA (PDGFR alfa), ABL1, ACVR1B (ALK4), AKT1 (PKB alpha), AMPK A1/B1/G1, AURKA (Aurora A), BTK, CDK1/cyclin B, CHEK1 (CHK1), CSNK1G2 (CK1 gamma 2), CSNK2A1 (CK2 alpha 1), DYRK3, EGFR (ErbB1), EPHA2, ERBB2 (HER2), FGFR1, FRAP1 (mTOR), GSK3B (GSK3 beta), IGF1R, IKBKB (IKK beta), INSR, IRAK4, JAK3, KDR (VEGFR2), LCK, MAP2K1 (MEK1), MAP4K4 (HGK), MAPK1 (ERK2), MAPK14 (p38 alpha), MAPK3 (ERK1), MAPK8 (JNK1), MARK2, MET (cMet), NEK1, PAK4, PHKG2, PIM1, PLK1, PRKACA (PKA), PRKCB1 (PKC beta I), ROCK1, RPS6KA3 (RSK2), RPS6KB1 (p70S6K), RTK, SRC, SYK, and TEK (Tie2). Preferably, the protein kinase is selected from the group consisting of CSF1R, FLT3, Kit, PDGFRB (PDGFR

beta), PDGFRA (PDGFR alpha).

According to one embodiment of the present invention, the compound (C) for use as a kinase inhibitor, or the N-oxide, pharmaceutically acceptable salt, pharmaceutically acceptable solvate, or stereoisomer thereof, preferably is a compound chosen among those of formulae (IIa) to (IXa) [compound (C) of class (I), herein after]:

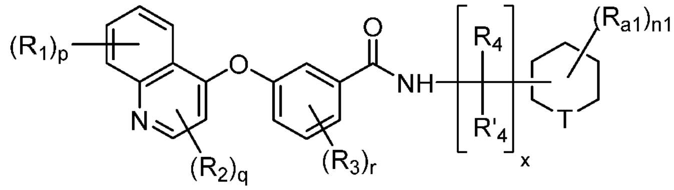

Formula (IIa)

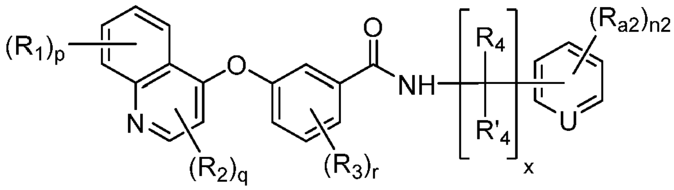

Formula (IIIa)

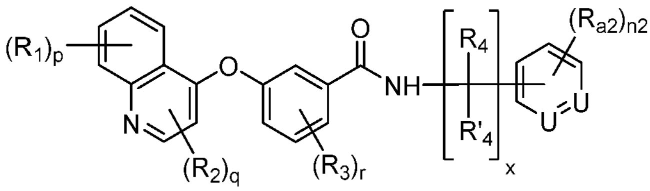

Formula (IVa)

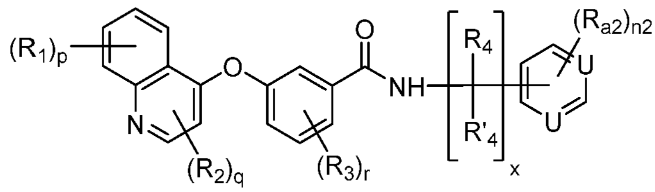

Formula (Va)

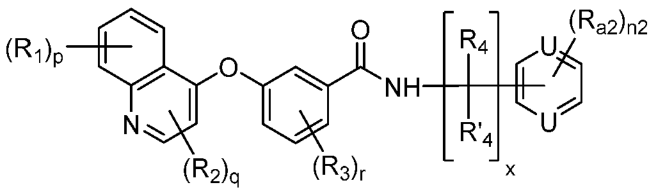

Formula (VIa)

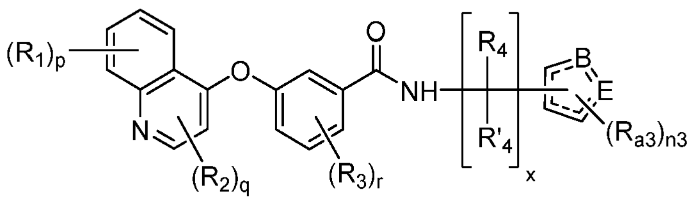

Formula (VIIa)

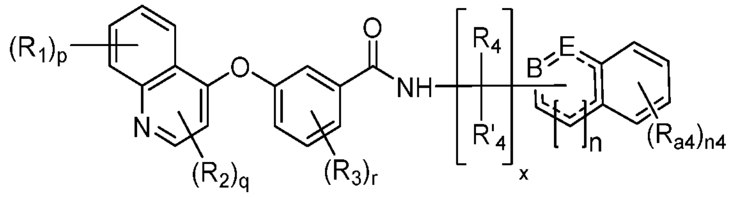

Formula (VIIIa)

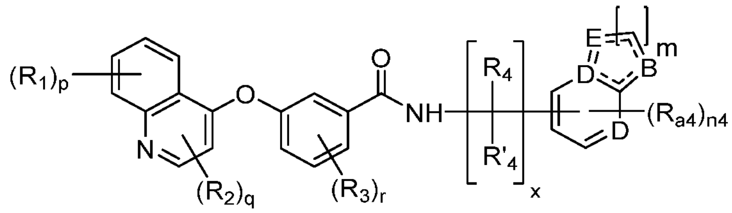

Formula (IXa)

wherein $R_1$, $R_2$, $R_3$, $R_4$, $R'_4$, p, q, r, x have the same meaning as defined above and the dash bond represents an optional double bond and wherein:

- T is selected from $CH_2$, N-R, O or S, wherein R is selected from hydrogen, $C_{1-10}$ alkyl or cycloalkyl which are optionally substituted by a halogen atom, an aryl group or an aralkyl group; preferably T is selected from $CH_2$ or O;
- each of U is selected, independently and at each occurrence, from C-halo, C-R or N; and wherein R is selected from hydrogen, $OR_{11}$, $N(R_{11})_2$, a $C_{1-10}$ alkyl or a cycloalkyl which are optionally substituted by a halogen atom, an aryl group or an aralkyl group, wherein each of $R_{11}$, independently from each other and at each occurrence, is selected from the group consisting of hydrogen and $C_{1-4}$ alkyl; preferably U is selected, independently and at each occurrence from C-halo, C-R or N; and preferably R is hydrogen or $C_{1-4}$ alkyl, more preferably U is selected, independently and at each occurrence from C-R or N; and more preferably R is hydrogen;
- each of D is selected, independently and at each occurrence, from C, C-R or N, wherein R is selected from hydrogen, $C_{1-5}$ alkyl or cycloalkyl; preferably D is selected, independently and at each occurrence from C, C-R or **N,** and preferably R is hydrogen;
- m is an integer equal to 1 or 2;
- n is an integer equal to 0 or 1 ;
- each of $R_{a1}$, independently from each other and at each occurrence, is selected from the group consisting of hydrogen, $C_{1-5}$ alkyl, $C_{2-15}$ alkenyl and $C_{2-15}$ alkynyl, wherein said $C_{1-5}$ alkyl, $C_{2-15}$ alkenyl and $C_{2-15}$ alkynyl are optionally substituted with a halogen atom, an aryl group or an aralkyl group; each of n1 is an integer in the range from 0 to 5; preferably $R_{a1}$ is hydrogen or $C_{1-5}$ alkyl;
- each of $R_{a2}$, independently from each other and at each occurrence is selected from the group consisting of $C_{1-15}$ alkyl, $C_{2-15}$ alkenyl, $C_{2-15}$ alkynyl, cycloalkyl, heterocyclyl, aryl, heteroaryl, aralkyl, halo, $NO_2$, $CF_3$, CN, $OR_{11}$, $SR_{11}$, $N(R_{11})_2$, $OC(R_{11})_2O$, $OC(R_{11})_2C(R_{11})_2O$, $COOR_{11}$, $CO(R_{11})_2$, $CON(R_{11})_2$, and $SO_2N(R_{11})_2$ and each optional alkyl, alkenyl, alkynyl cycloalkyl, aryl, heterocyclyl, heteroaryl substituent is further optionally substituted with halo, $C_{1-6}$ alkyl, cycloalkyl, aryl, $N(R_{11})_2$, $CF_3$, CN, $COOR_{11}$, $CO(R_{11})_2$, $CON(R_{11})_2$, $SO_2N(R_{11})_2$ or $OR_{11}$ and wherein each of $R_{11}$, independently from each other and at each occurrence, is selected from the group consisting of hydrogen, $C_{1-6}$ alkyl, cycloalkyl, heterocyclyl, aryl, heteroaryl, and aralkyl, wherein said alkyl, cycloalkyl,
  heterocyclyl, aryl, heteroaryl, and aralkyl substituents are optionally substituted with halo, $C_{1-6}$ alkyl, cycloalkyl, heterocyclyl, $OR_{31}$ or $N(R_{32})_2$, wherein each of $R_{31}$ and $R_{32}$, independently from each other and at each occurrence, is selected from the group consisting of hydrogen and $C_{1-4}$ alkyl; more preferably, each of $R_{a2}$ is independently selected from the group consisting of halo, $OR_{11}$, $C_{1-6}$ alkyl, cycloalkyl, heterocyclyl, aryl, heteroaryl, $CF_3$, CN, $SR_{11}$, $N(R_{11})_2$, $OC(R_{11})_2O$, $OC(R_{11})_2C(R_{11})_2O$, $COOR_{11}$, $CO(R_{11})_2$, and $CON(R_{11})_2$, and wherein each of $R_{11}$ , independently from each other and at each occurrence, is selected from the group consisting of hydrogen, $C_{1-4}$ alkyl, cycloalkyl and heterocyclyl; even more preferably, each of $R_{a2}$ is independently selected from the group consisting of halo, $OC_{1-4}$ alkyl, $C_{1-4}$ alkyl, cycloalkyl, heterocyclyl, aryl, heteroaryl, $CF_3$, CN, $OCH_2O$, $OCH_2CH_2O$, COOH, COO($C_{1-4}$ alkyl), CO(heterocyclyl), CO($C_{1-4}$ alkyl), CONH($C_{1-4}$ alkyl), and CONH(cycloalkyl); each of n2 is an integer in the range from 0 to 4; preferably each of n2 is an integer in the range from 0 to 2;
- each of B, independently from each other and at each occurrence is selected from C-R, O, N, S and $NR_{7'}$, wherein R is selected from hydrogen or an $C_{1-10}$ alkyl which is optionally substituted by a halogen atom, an aryl group or an aralkyl group, wherein $R_{7'}$ is selected from the group consisting of hydrogen, $C_{1-6}$ alkyl, $C_{1-6}$ alkenyl, cycloalkyl, heterocyclyl, aryl, aralkyl and $CF_3$; preferably each of B, independent from each other and at each occurrence, is each selected from C-R, O and $NR_{7'}$, wherein $R_{7'}$ is selected from hydrogen or $C_{1-4}$ alkyl and R is hydrogen or $C_{1-4}$ alkyl.

- each of E, independently from each other and at each occurrence is selected from C-R, O, N, S and $NR_{7'}$, wherein R is selected from hydrogen or an $C_{1-10}$ alkyl which is optionally substituted by a halogen atom, an aryl group or an aralkyl group, wherein $R_{7'}$ is selected from the group consisting of hydrogen, $C_{1-6}$ alkyl, $C_{1-6}$ alkenyl, cycloalkyl, heterocyclyl, aryl, aralkyl and $CF_3$; preferably each of E, independent from each other and at each occurrence, is selected from C-R, O and $NR_{7'}$, wherein $R_{7'}$ is selected from hydrogen or $C_{1-4}$ alkyl and R is hydrogen or $C_{1-4}$ alkyl.
- each of $R_{a4}$, independently from each other and at each occurrence is selected from the group consisting of $C_{1-15}$ alkyl, $C_{2-15}$ alkenyl, $C_{2-15}$ alkynyl, cycloalkyl, heterocyclyl, aryl, heteroaryl, aralkyl, halo, $NO_2$, $CF_3$, CN, $OR_{11}$, $SR_{11}$, $N(R_{11})_2$, $OC(R_{11})_2O$, $OC(R_{11})_2C(R_{11})_2O$, $COOR_{11}$, $CO(R_{11})_2$, and $CON(R_{11})_2$, and each optional alkyl, alkenyl, alkynyl, cycloalkyl, aryl, heterocyclyl, heteroaryl, aralkyl substituent is further optionally substituted with halo, alkyl, cycloalkyl, aryl, $N(R_{11})_2$, CN, or $OR_{11}$ and wherein each of $R_{11}$ , independently from each other and at each occurrence, is selected from the group consisting of hydrogen, $C_{1-6}$ alkyl, cycloalkyl, heterocyclyl, aryl, heteroaryl, and aralkyl, wherein said alkyl, alkenyl, alkynyl, cycloalkyl, heterocyclyl, aryl, heteroaryl, and aralkyl substituents are optionally substituted with halo, alkyl, cycloalkyl, heterocyclyl, $OR_{31}$ or $N(R_{32})_2$, wherein each of $R_{31}$ and $R_{32}$, independently from each other and at each occurrence, is selected from the group consisting of hydrogen and $C_{1-4}$ alkyl; more preferably, each of $R_{a4}$ is independently selected from the group consisting of halo, $OR_{11}$, $C_{1-6}$ alkyl, cycloalkyl, heterocyclyl, aryl, heteroaryl, $CF_3$, CN, $SR_{11}$, $N(R_{11})_2$, $OC(R_{11})_2O$, $OC(R_{11})_2C(R_{11})_2O$, $COOR_{11}$, $CO(R_{11})_2$, and $CON(R_{11})_2$, and wherein each of $R_{11}$ , independently from each other and at each occurrence, is selected from the group consisting of hydrogen and $C_{1-4}$ alkyl; even more preferably, each of $R_{a4}$ is independently selected from the group consisting of halo, $OC_{1-4}$ alkyl, $C_{1-4}$ alkyl, cycloalkyl, heterocyclyl, aryl, heteroaryl, $CF_3$, CN, $OCH_2O$, and $OCH_2CH_2O$; each of n4 is an integer in the range from 0 to 4; preferably each of n4 is an integer in the range from 0 to 2;
- each of $R_{a3}$, independently from each other and at each occurrence is selected from the group consisting of $C_{1-15}$ alkyl, $C_{2-15}$ alkenyl, $C_{2-15}$ alkynyl, cycloalkyl, heterocyclyl, aryl, heteroaryl, aralkyl, halo, $CF_3$, $OR_{11}$, $SR_{11}$, $N(R_{11})_2$, $COOR_{11}$, $CO(R_{11})_2$, and $CON(R_{11})_2$, and each optional alkyl, alkenyl, alkynyl, cycloalkyl, aryl, heterocyclyl, heteroaryl, aralkyl substituent is further optionally substituted with halo, alkyl, cycloalkyl, aryl, $N(R_{11})_2$, CN, or $OR_{11}$ and wherein each of $R_{11}$ , independently from each other and at each occurrence, is selected from the group consisting of hydrogen, $C_{1-6}$ alkyl, cycloalkyl, heterocyclyl, aryl, heteroaryl, and aralkyl, wherein said alkyl, cycloalkyl, heterocyclyl, aryl, heteroaryl, and aralkyl substituents are optionally substituted with halo, alkyl, cycloalkyl, heterocyclyl, $OR_{31}$ or $N(R_{32})_2$, wherein each of $R_{31}$ and $R_{32}$, independently from each other and at each occurrence, is selected from the group consisting of hydrogen and $C_{1-4}$alkyl; more preferably, each of $R_{a3}$ is independently selected from the group consisting of $C_{1-6}$ alkyl, cycloalkyl, heterocyclyl, aryl and heteroaryl; even more preferably, $R_{a3}$ is $C_{1-4}$ alkyl, each of n3 is an integer in the range from 0 to 2.

wherein said cycloalkyl is a monocyclic, bicyclic or tricyclic ring system of 3-7 ring members per ring; said heterocyclyl is a saturated, partially saturated or completely saturated monocycle, bicycle or tricycle containing 3 to 12 carbon atoms and 1 or 2 heteroatoms independently selected from O or N; said aryl is phenyl, naphthyl or anthracenyl optionally carbocyclic fused with a cycloalkyl or heterocyclyl of 5-7 ring members; said heteroaryl is a monocyclic ring structure containing 5 or 6 ring atoms, or a bicyclic aromatic group having 8 to 10 atoms, containing 1-3 heteroatoms independently selected from O or N.

In a preferred embodiment of the present invention, the compound (C) for use as a kinase inhibitor, or the N-oxide, pharmaceutically acceptable salt, pharmaceutically acceptable solvate, or stereoisomer thereof, is a compound of formulae (IIa) to (IXa) wherein:

- each of $R_1$ and $R_2$, independently from each other and at each occurrence, is selected from the group consisting of hydrogen, halo, $C_{1-6}$ alkyl, $C_{2-5}$ alkenyl, $C_{2-5}$ alkynyl, cycloalkyl, heterocyclyl, aryl, and heteroaryl, $CF_3$, CN, $NO_2$, $OR_{21}$, $SR_{21}$, $N(R_{21})_2$, $COR_{21}$, $C(O)OR_{21}$, $CON(R_{21})_2$, $OC(O)R_{21}$, $OCON(R_{21})_2$, $NC(O)R_{21}$, $NCON(R_{21})_2$, $OC(R_{21})_2O$ and $OC(R_{21})_2C(R_{22})_2O$, wherein said alkyl, alkenyl, alkynyl, cycloalkyl, heterocyclyl, aryl, and heteroaryl are optionally substituted with one or more substituents selected from halo, $C_{1-6}$ alkyl, cycloalkyl, heterocyclyl, $CF_3$, $COR_{21}$, $N(R_{21})_2$, CN, $CONR_{21}$, $C(O)OR_{21}$ or $OR_{21}$, and each optional alkyl substituent is further optionally substituted with heterocyclyl, $N(R_{11})_2$, or $OR_{11}$ ; and wherein each of $R_{21}$ and $R_{22}$, independently from each other and at each occurrence, is selected from the group consisting of hydrogen, $C_{1-6}$ alkyl, $C_{2-4}$ alkenyl, $C_{2-4}$ alkynyl, cycloalkyl, heterocyclyl, phenyl, heteroaryl, and aralkyl and wherein said alkyl, alkenyl, alkynyl, cycloalkyl, heterocyclyl, phenyl, heteroaryl, and aralkyl substituents are optionally substituted with halo, $C_{1-6}$ alkyl, cycloalkyl, heterocyclyl, phenyl, $OR_{31}$ or $N(R_{32})_2$, wherein each of $R_{31}$ and $R_{32}$, independently from each other and at each occurrence, is selected from the group consisting of hydrogen and $C_{1-4}$ alkyl; each of p is an integer in the range from 0 to 4; each of q is an integer in the range from 0 to 2;
- each of $R_3$, independently from each other and at each occurrence, is selected from the group consisting of hydrogen, halo, $C_{1-15}$ alkyl, $C_{2-15}$ alkenyl, $C_{2-15}$ alkynyl, cycloalkyl, heterocyclyl, $CF_3$, CN, $OR_{21}$, $SR_{21}$, $N(R_{21})_2$, $NC(O)R_{21}$,

$NCON(R_{21})_2$, $COR_{21}$, $C(O)OR_{21}$, $CON(R_{21})_2$, $OC(O)R_{21}$, $OCON(R_{21})_2$, $OC(R_{21})_2O$, and $OC(R_{21})_2C(R_{22})_2O$, wherein said alkyl, alkenyl, alkynyl, cycloalkyl, and heterocyclyl, are optionally substituted with one or more substituents selected from halo, $C_{1-15}$ alkyl, $CF_3$, $N(R_{21})_2$, CN, or $OR_{21}$; and wherein each of $R_{21}$ and $R_{22}$, independently from each other and at each occurrence, is selected from the group consisting of hydrogen, $C_{1-15}$ alkyl, $C_{2-15}$ alkenyl, $C_{2-15}$ alkynyl, cycloalkyl, heterocyclyl, aryl, heteroaryl, and aralkyl, and wherein said alkyl, alkenyl, alkynyl, cycloalkyl, heterocyclyl, aryl, heteroaryl, and aralkyl substituents are optionally substituted with halo, alkyl, cycloalkyl, heterocyclyl, aryl, $OR_{31}$ or $N(R_{32})_2$, wherein each of $R_{31}$ and $R_{32}$, independently from each other and at each occurrence, is selected from the group consisting of hydrogen and $C_{1-4}$ alkyl; each of r is an integer in the range from 0 to 3;

- each of $R_4$ and $R'_4$, independently from each other and at each occurrence, are selected from the group consisting of hydrogen, $C_{1-6}$ alkyl, and $C_{2-6}$ alkenyl, wherein said $C_{1-6}$ alkyl, and $C_{2-6}$ alkenyl are optionally substituted with a halogen atom, and x is an integer in the range from 0 to 7;
- T is selected from $CH_2$, N-R, O or S, wherein R is selected from hydrogen, $C_{1-10}$ alkyl or cycloalkyl which are optionally substituted by a halogen atom, an aryl group or an aralkyl group; preferably T is selected from $CH_2$ or O;
- each of U is selected, independently and at each occurrence, from C-halo, C-R or N; and wherein R is selected from hydrogen, $OR_{11}$, $N(R_{11})_2$, a $C_{1-10}$ alkyl or a cycloalkyl which are optionally substituted by a halogen atom, an aryl group or an aralkyl group, wherein each of $R_{11}$, independently from each other and at each occurrence, is selected from the group consisting of hydrogen and $C_{1-4}$ alkyl; preferably U is selected, independently and at each occurrence from C-halo, C-R or N; and preferably R is hydrogen or $C_{1-4}$ alkyl, more preferably U is selected, independently and at each occurrence from C-R or N; and more preferably R is hydrogen;
- each of D is selected, independently and at each occurrence, from C, C-R or N, wherein R is selected from hydrogen, $C_{1-5}$ alkyl or cycloalkyl; preferably D is selected, independently and at each occurrence from C, C-R or N, and preferably R is hydrogen;
- m is an integer equal to 1 or 2;
- n is an integer equal to 0 or 1;
- each of $R_{a1}$, independently from each other and at each occurrence, is selected from the group consisting of hydrogen, $C_{1-5}$ alkyl, $C_{2-15}$ alkenyl and $C_{2-15}$ alkynyl, wherein said $C_{1-5}$ alkyl, $C_{2-15}$ alkenyl and $C_{2-15}$ alkynyl are optionally substituted with a halogen atom, an aryl group or an aralkyl group; each of n1 is an integer in the range from 0 to 5; preferably $R_{a1}$ is hydrogen or $C_{1-5}$ alkyl;
- each of $R_{a2}$, independently from each other and at each occurrence is selected from the group consisting of $C_{1-15}$ alkyl, $C_{2-15}$ alkenyl, $C_{2-15}$ alkynyl, cycloalkyl, heterocyclyl, aryl, heteroaryl, aralkyl, halo, $NO_2$, $CF_3$, CN, $OR_{11}$, $SR_{11}$, $N(R_{11})_2$, $OC(R_{11})_2O$, $OC(R_{11})_2C(R_{11})_2O$, $COOR_{11}$, $CO(R_{11})_2$, $CON(R_{11})_2$, $SO_2N(R_{11})_2$ and each optional alkyl, alkenyl, alkynyl cycloalkyl, aryl, heterocyclyl, heteroaryl substituent is further optionally substituted with halo, $C_{1-6}$ alkyl, cycloalkyl, aryl, $N(R_{11})_2$, $CF_3$, CN, $COOR_{11}$, $CO(R_{11})_2$, $CON(R_{11})_2$, $SO_2N(R_{11})_2$ or $OR_{11}$ and wherein each of $R_{11}$ , independently from each other and at each occurrence, is selected from the group consisting of hydrogen, $C_{1-6}$ alkyl, cycloalkyl, heterocyclyl, aryl, heteroaryl, and aralkyl, wherein said alkyl, alkenyl, alkynyl, cycloalkyl, heterocyclyl, aryl, heteroaryl, and aralkyl substituents are optionally substituted with halo, $C_{1-6}$ alkyl, cycloalkyl, heterocyclyl, $OR_{31}$ or $N(R_{32})_2$, wherein each of $R_{31}$ and $R_{32}$, independently from each other and at each occurrence, is selected from the group consisting of hydrogen and $C_{1-4}$ alkyl; more preferably, each of $R_{a2}$ is independently selected from the group consisting of halo, $OR_{11}$, $C_{1-6}$ alkyl, cycloalkyl, heterocyclyl, aryl, heteroaryl, $CF_3$, CN, $SR_{11}$, $N(R_{11})_2$, $OC(R_{11})_2O$, $OC(R_{11})_2C(R_{11})_2O$, $COOR_{11}$, $CO(R_{11})_2$, and $CON(R_{11})_2$, and wherein each of $R_{11}$ , independently from each other and at each occurrence, is selected from the group consisting of hydrogen, $C_{1-4}$ alkyl, cycloalkyl and heterocyclyl; even more preferably, each of $R_{a2}$ is independently selected from the group consisting of halo, $OC_{1-4}$ alkyl, $C_{1-4}$ alkyl, cycloalkyl, heterocyclyl, aryl, heteroaryl, $CF_3$, CN, $OCH_2O$, $OCH_2CH_2O$, COOH, $COO(C_{1-4}$ alkyl), CO(heterocyclyl), $CO(C_{1-4}$ alkyl), $CONH(C_{1-4}$ alkyl), and CONH(cycloalkyl); each of n2 is an integer in the range from 0 to 4; preferably each of n2 is an integer in the range from 0 to 2;
- each of B, independently from each other and at each occurrence is selected from C-R, O, N, S and $NR_{7'}$, wherein R is selected from hydrogen or an $C_{1-10}$ alkyl which is optionally substituted by a halogen atom, an aryl group or an aralkyl group, wherein $R_{7'}$ is selected from the group consisting of hydrogen, $C_{1-6}$ alkyl, $C_{1-6}$ alkenyl, cycloalkyl, heterocyclyl, aryl, aralkyl and $CF_3$; preferably each of B, independent from each other and at each occurrence, is each selected from C-R, O and $NR_{7'}$, wherein $R_{7'}$ is selected from hydrogen or $C_{1-4}$ alkyl and R is hydrogen or $C_{1-4}$ alkyl.
- each of E, independently from each other and at each occurrence is selected from C-R, O, N, S and $NR_{7'}$, wherein R is selected from hydrogen or an $C_{1-10}$ alkyl which is optionally substituted by a halogen atom, an aryl group or an aralkyl group, wherein $R_{7'}$ is selected from the group consisting of hydrogen, $C_{1-6}$ alkyl, $C_{1-6}$ alkenyl, cycloalkyl, heterocyclyl, aryl, aralkyl and $CF_3$; preferably each of E, independent from each other and at each occurrence, is selected from C-R, O and $NR_{7'}$, wherein $R_{7'}$ is selected from hydrogen or $C_{1-4}$ alkyl and R is hydrogen or $C_{1-4}$ alkyl.
- each of $R_{a4}$, independently from each other and at each occurrence is selected from the group consisting of $C_{1-15}$ alkyl, $C_{2-15}$ alkenyl, $C_{2-15}$ alkynyl, cycloalkyl, heterocyclyl, aryl, heteroaryl, aralkyl, halo, $NO_2$, $CF_3$, CN, $OR_{11}$, $SR_{11}$, $N(R_{11})_2$, $OC(R_{11})_2O$, $OC(R_{11})_2C(R_{11})_2O$, $COOR_{11}$, $CO(R_{11})_2$, and $CON(R_{11})_2$, and each optional alkyl, alkenyl,

alkynyl, cycloalkyl, aryl, heterocyclyl, heteroaryl, aralkyl substituent is further optionally substituted with halo, alkyl, cycloalkyl, aryl, $N(R_{11})_2$, CN, or $OR_{11}$ and wherein each of $R_{11}$ , independently from each other and at each occurrence, is selected from the group consisting of hydrogen, $C_{1-6}$ alkyl, cycloalkyl, heterocyclyl, aryl, heteroaryl, and aralkyl, wherein said alkyl, alkenyl, alkynyl, cycloalkyl, heterocyclyl, aryl, heteroaryl, and aralkyl substituents are optionally substituted with halo, alkyl, cycloalkyl, heterocyclyl, $OR_{31}$ or $N(R_{32})_2$, wherein each of $R_{31}$ and $R_{32}$, independently from each other and at each occurrence, is selected from the group consisting of hydrogen and $C_{1-4}$ alkyl; more preferably, each of $R_{a4}$ is independently selected from the group consisting of halo, $OR_{11}$, $C_{1-6}$ alkyl, cycloalkyl, heterocyclyl, aryl, heteroaryl, $CF_3$, CN, $SR_{11}$, $N(R_{11})_2$, $OC(R_{11})_2O$, $OC(R_{11})_2C(R_{11})_2O$, $COOR_{11}$, $CO(R_{11})_2$, and $CON(R_{11})_2$, and wherein each of $R_{11}$ , independently from each other and at each occurrence, is selected from the group consisting of hydrogen and $C_{1-4}$ alkyl; even more preferably, each of $R_{a4}$ is independently selected from the group consisting of halo, $OC_{1-4}$ alkyl, $C_{1-4}$ alkyl, cycloalkyl, heterocyclyl, aryl, heteroaryl, $CF_3$, CN, $OCH_2O$, and $OCH_2CH_2O$; each of n4 is an integer in the range from 0 to 4; preferably each of n4 is an integer in the range from 0 to 2;

- each of $R_{a3}$, independently from each other and at each occurrence is selected from the group consisting of $C_{1-15}$ alkyl, $C_{2-15}$ alkenyl, $C_{2-15}$ alkynyl, cycloalkyl, heterocyclyl, aryl, heteroaryl, aralkyl, halo, $CF_3$, $OR_{11}$, $SR_{11}$, $N(R_{11})_2$, $COOR_{11}$, $CO(R_{11})_2$, and $CON(R_{11})_2$, and each optional alkyl, alkenyl, alkynyl, cycloalkyl, aryl, heterocyclyl, heteroaryl, aralkyl substituent is further optionally substituted with halo, alkyl, cycloalkyl, aryl, $N(R_{11})_2$, CN, or $OR_{11}$ and wherein each of $R_{11}$ , independently from each other and at each occurrence, is selected from the group consisting of hydrogen, $C_{1-6}$ alkyl, cycloalkyl, heterocyclyl, aryl, heteroaryl, and aralkyl, wherein said alkyl, cycloalkyl, heterocyclyl, aryl, heteroaryl, and aralkyl substituents are optionally substituted with halo, alkyl, cycloalkyl, heterocyclyl, $OR_{31}$ or $N(R_{32})_2$, wherein each of $R_{31}$ and $R_{32}$, independently from each other and at each occurrence, is selected from the group consisting of hydrogen and $C_{1-4}$alkyl; more preferably, each of $R_{a3}$ is independently selected from the group consisting of $C_{1-6}$ alkyl, cycloalkyl, heterocyclyl, aryl, and heteroaryl; even more preferably, $R_{a3}$ is $C_{1-4}$ alkyl, each of n3 is an integer in the range from 0 to 2.

wherein said cycloalkyl is a monocyclic, bicyclic or tricyclic ring system of 3-7 ring members per ring; said heterocyclyl is a saturated, partially saturated or completely saturated monocycle, bicycle or tricycle containing 3 to 12 carbon atoms and 1 or 2 heteroatoms independently selected from O or N; said aryl is phenyl, naphthyl or anthracenyl optionally carbocyclic fused with a cycloalkyl or heterocyclyl of 5-7 ring members; said heteroaryl is a monocyclic ring structure containing 5 or 6 ring atoms, or a bicyclic aromatic group having 8 to 10 atoms, containing 1-3 heteroatoms independently selected from O or N.

According to one embodiment of the present invention, the compound (C) for use as a kinase inhibitor, or the N-oxide, pharmaceutically acceptable salt, pharmaceutically acceptable solvate, or stereoisomer thereof, preferably is a compound chosen among those of formulae (IIa-a) to (IXa-a) [compounds (C) of class (I), herein after]:

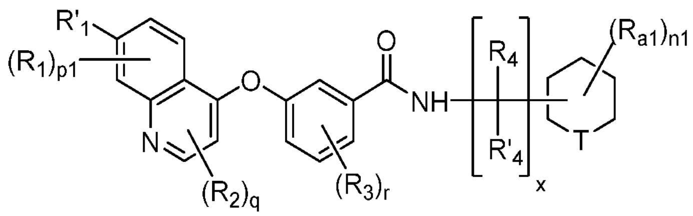

Formula (IIa-a)

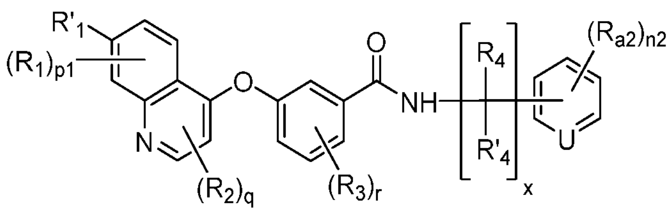

Formula (IIIa-a)

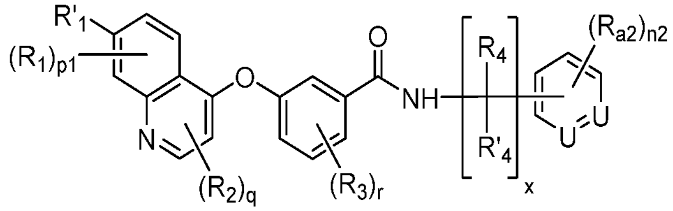

Formula (IVa-a)

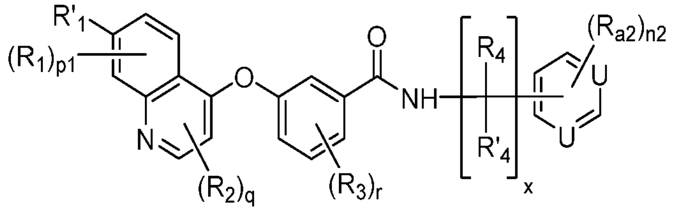

Formula (Va-a)

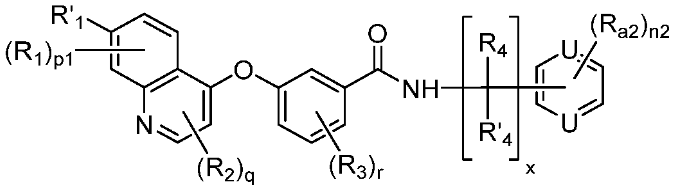

Formula (VIa-a)

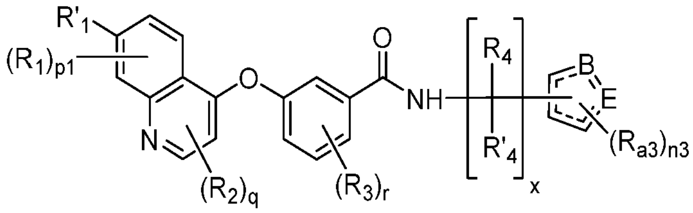

Formula (VIIa-a)

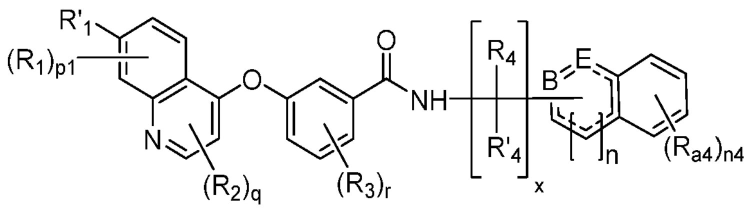

Formula (VIIIa-a)

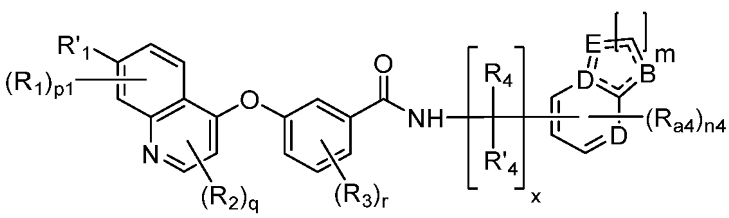

Formula (IXa-a)

wherein $R_1$, $R_2$, $R_3$, $R_4$, $R'_4$, $R_{a1}$, $R_{a2}$, $R_{a3}$, $R_{a4}$, T, U, B, E, D, p, q, r, x, n1, n2, n3, n4, m, n have the same meaning as defined above for formula (IIa) to (IXa) and the dash bond represents an optional double bond, and wherein:

- p1 is an integer in the range from 0 to 3
- $R'_1$ is selected from the group consisting of ($R'_1$-a) to ($R'_1$-d):

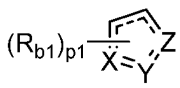

($R'_1$-a)

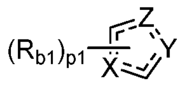

($R'_1$-b)

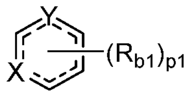

($R'_1$-c)

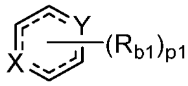

($R'_1$-d)

wherein:

- each of X, Y and Z independently from each other and at each occurrence is selected from C-R, O, N, S and $NR_{7'}$, wherein R is selected from hydrogen or a $C_{1-6}$ alkyl which is optionally substituted by a halogen atom, an aryl group or an aralkyl group, wherein $R_{7'}$ is selected from the group consisting of hydrogen, $C_{1-6}$ alkyl, $C_{1-6}$ alkenyl, cycloalkyl, heterocyclyl, aryl, aralkyl and $CF_3$; preferably each of X, Y and Z, independent from each other and at each occurrence, is each selected from C-R, O and $NR_{7'}$, wherein $R_{7'}$ is selected from hydrogen or $C_{1-4}$ alkyl and R is hydrogen or $C_{1-4}$ alkyl.

  • Each of $R_{b1}$, independently and at each occurrence, are selected from the group consisting of hydrogen, halo, $C_{1-6}$ alkyl, cycloalkyl, heterocyclyl, CN, $COR_{11}$, $CON(R_{11})_2$, and $OR_{11}$, wherein said alkyl, cycloalkyl and heterocyclyl are optionally substituted with one or more substituents selected from heterocyclyl, $OR_{11}$, $NR_{11}$ and wherein each of $R_{11}$ , independently from each other and at each occurrence, is selected from the group consisting of hydrogen, halo and $C_{1-6}$ alkyl; each of p1 is an integer in the range from 0 to 3.

  wherein said cycloalkyl is a monocyclic, bicyclic or tricyclic ring system of 3-7 ring members per ring; said heterocyclyl is a saturated, partially saturated or completely saturated monocycle, bicycle or tricycle containing 3 to 12 carbon atoms and 1 or 2 heteroatoms independently selected from O or N; said aryl is phenyl, naphthyl or anthracenyl optionally carbocyclic fused with a cycloalkyl or heterocyclyl of 5-7 ring members; said heteroaryl is a monocyclic ring structure containing 5 or 6 ring atoms, or a bicyclic aromatic group having 8 to 10 atoms, containing 1-3 heteroatoms independently selected from O or N.

In a preferred embodiment of the present invention, the compound (C) for use as a kinase inhibitor, or the N-oxide, pharmaceutically acceptable salt, pharmaceutically acceptable solvate, or stereoisomer thereof, is a compound of formulae (IIa-a) to (IXa-a) wherein:

- each of $R_1$ and $R_2$, independently from each other and at each occurrence, is selected from the group consisting of hydrogen, halo, $C_{1-6}$ alkyl, $C_{2-5}$ alkenyl, $C_{2-5}$ alkynyl, cycloalkyl, heterocyclyl, aryl, and heteroaryl, $CF_3$, CN, $NO_2$, $OR_{21}$, $SR_{21}$, $N(R_{21})_2$, $COR_{21}$, $C(O)OR_{21}$, $CON(R_{21})_2$, $OC(O)R_{21}$, $OCON(R_{21})_2$, $NC(O)R_{21}$, $NCON(R_{21})_2$, $OC(R_{21})_2O$ and $OC(R_{21})_2C(R_{22})_2O$, wherein said alkyl, alkenyl, alkynyl, cycloalkyl, heterocyclyl, aryl, and heteroaryl are optionally substituted with one or more substituents selected from halo, $C_{1-6}$ alkyl, cycloalkyl, heterocyclyl, $CF_3$, $COR_{21}$, $N(R_{21})_2$, CN, $CONR_{21}$, $C(O)OR_{21}$ or $OR_{21}$, and each optional alkyl substituent is further optionally substituted with heterocyclyl, $N(R_{11})_2$, or $OR_{11}$ ; and wherein each of $R_{21}$ and $R_{22}$, independently from each other and at each occurrence, is selected from the group consisting of hydrogen, $C_{1-6}$ alkyl, $C_{2-4}$ alkenyl, $C_{2-4}$ alkynyl, cycloalkyl, heterocyclyl, phenyl, heteroaryl, and aralkyl, and wherein said alkyl, alkenyl, alkynyl, cycloalkyl, heterocyclyl, phenyl, heteroaryl, and aralkyl substituents are optionally substituted with halo, $C_{1-6}$ alkyl, cycloalkyl, heterocyclyl, phenyl, $OR_{31}$ or $N(R_{32})_2$, wherein each of $R_{31}$ and $R_{32}$, independently from each other and at each occurrence, is selected from the group consisting of hydrogen and $C_{1-4}$ alkyl; each of q is an integer in the range from 0 to 2;
- each of $R_3$, independently from each other and at each occurrence, is selected from the group consisting of hydrogen, halo, $C_{1-15}$ alkyl, $C_{2-15}$ alkenyl, $C_{2-15}$ alkynyl, cycloalkyl, heterocyclyl, $CF_3$, CN, $OR_{21}$, $SR_{21}$, $N(R_{21})_2$, $NC(O)R_{21}$, $NCON(R_{21})_2$, $COR_{21}$, $C(O)OR_{21}$, $CON(R_{21})_2$, $OC(O)R_{21}$, $OCON(R_{21})_2$, $OC(R_{21})_2O$, and $OC(R_{21})_2C(R_{22})_2O$, wherein said alkyl, alkenyl, alkynyl, cycloalkyl, and heterocyclyl, are optionally substituted with one or more substituents selected from halo, $C_{1-15}$ alkyl, $CF_3$, $N(R_{21})_2$, CN, or $OR_{21}$; and wherein each of $R_{21}$ and $R_{22}$, independently from each other and at each occurrence, is selected from the group consisting of hydrogen, $C_{1-15}$ alkyl, $C_{2-15}$ alkenyl, $C_{2-15}$ alkynyl, cycloalkyl, heterocyclyl, aryl, heteroaryl, and aralkyl and wherein said alkyl, alkenyl, alkynyl, cycloalkyl, heterocyclyl, aryl, heteroaryl, and aralkyl substituents are optionally substituted with halo, alkyl, cycloalkyl, hetero-

cyclyl, aryl, $OR_{31}$ or $N(R_{32})_2$, wherein each of $R_{31}$ and $R_{32}$, independently from each other and at each occurrence, is selected from the group consisting of hydrogen and $C_{1-4}$ alkyl; each of r is an integer in the range from 0 to 3;

- each of $R_4$ and $R'_4$, independently from each other and at each occurrence, are selected from the group consisting of hydrogen, $C_{1-6}$ alkyl, and $C_{2-6}$ alkenyl, wherein said $C_{1-6}$ alkyl, and $C_{2-6}$ alkenyl are optionally substituted with a halogen atom, and x is an integer in the range from 0 to 7;
- T is selected from $CH_2$, N-R, O or S, wherein R is selected from hydrogen, $C_{1-10}$ alkyl or cycloalkyl which are optionally substituted by a halogen atom, an aryl group or an aralkyl group; preferably T is selected from $CH_2$ or O;
- each of U is selected, independently and at each occurrence, from C-halo, C-R or N; and wherein R is selected from hydrogen, $OR_{11}$, $N(R_{11})_2$, a $C_{1-10}$ alkyl or a cycloalkyl which are optionally substituted by a halogen atom, an aryl group or an aralkyl group, wherein each of $R_{11}$, independently from each other and at each occurrence, is selected from the group consisting of hydrogen and $C_{1-4}$ alkyl; preferably U is selected, independently and at each occurrence from C-halo, C-R or N; and preferably R is hydrogen or $C_{1-4}$ alkyl, more preferably U is selected, independently and at each occurrence from C-R or N; and more preferably R is hydrogen;
- each of D is selected, independently and at each occurrence, from C, C-R or N, wherein R is selected from hydrogen, $C_{1-5}$ alkyl or cycloalkyl; preferably D is selected, independently and at each occurrence from C, C-R or N, and preferably R is hydrogen;
- m is an integer equal to 1 or 2;
- n is an integer equal to 0 or 1;
- each of $R_{a1}$, independently from each other and at each occurrence, is selected from the group consisting of hydrogen, $C_{1-5}$ alkyl, $C_{2-15}$ alkenyl and $C_{2-15}$ alkynyl, wherein said $C_{1-5}$ alkyl, $C_{2-15}$ alkenyl and $C_{2-15}$ alkynyl are optionally substituted with a halogen atom, an aryl group or an aralkyl group; each of n1 is an integer in the range from 0 to 5; preferably $R_{a1}$ is hydrogen or $C_{1-5}$ alkyl;
- each of $R_{a2}$, independently from each other and at each occurrence is selected from the group consisting **of** $C_{1-15}$ alkyl, $C_{2-15}$ alkenyl, $C_{2-15}$ alkynyl, cycloalkyl, heterocyclyl, aryl, heteroaryl, aralkyl, halo, $NO_2$, $CF_3$, CN, $OR_{11}$, $SR_{11}$, $N(R_{11})_2$, $OC(R_{11})_2O$, $OC(R_{11})_2C(R_{11})_2O$, $COOR_{11}$, $CO(R_{11})_2$, $CON(R_{11})_2$, and $SO_2N(R_{11})_2$, and each optional alkyl, alkenyl, alkynyl cycloalkyl, aryl, heterocyclyl, heteroaryl substituent is further optionally substituted with halo, $C_{1-6}$ alkyl, cycloalkyl, aryl, $N(R_{11})_2$, $CF_3$, CN, $COOR_{11}$, $CO(R_{11})_2$, $CON(R_{11})_2$, $SO_2N(R_{11})_2$ or $OR_{11}$ and wherein each of $R_{11}$ , independently from each other and at each occurrence, is selected from the group consisting of hydrogen, $C_{1-6}$ alkyl, cycloalkyl, heterocyclyl, aryl, heteroaryl, and aralkyl, wherein said alkyl, alkenyl, alkynyl, cycloalkyl, heterocyclyl, aryl, heteroaryl, and aralkyl substituents are optionally substituted with halo, $C_{1-6}$ alkyl, cycloalkyl, heterocyclyl, $OR_{31}$ or $N(R_{32})_2$, wherein each of $R_{31}$ and $R_{32}$, independently from each other and at each occurrence, is selected from the group consisting of hydrogen and $C_{1-4}$ alkyl; more preferably, each of $R_{a2}$ is independently selected from the group consisting of halo, $OR_{11}$, $C_{1-6}$ alkyl, cycloalkyl, heterocyclyl, aryl, heteroaryl, $CF_3$, **CN,** $SR_{11}$, $N(R_{11})_2$, $OC(R_{11})_2O$, $OC(R_{11})_2C(R_{11})_2O$, $COOR_{11}$, $CO(R_{11})_2$, and $CON(R_{11})_2$, and wherein each of $R_{11}$ , independently from each other and at each occurrence, is selected from the group consisting of hydrogen, $C_{1-4}$ alkyl, cycloalkyl and heterocyclyl; even more preferably, each of $R_{a2}$ is independently selected from the group consisting of halo, $OC_{1-4}$ alkyl, $C_{1-4}$ alkyl, cycloalkyl, heterocyclyl, aryl, heteroaryl, $CF_3$, CN, $OCH_2O$, $OCH_2CH_2O$, COOH, $COO(C_{1-4}$ alkyl), CO(heterocyclyl), $CO(C_{1-4}$ alkyl), $CONH(C_{1-4}$ alkyl), and CONH(cycloalkyl); each of n2 is an integer in the range from 0 to 4; preferably each of n2 is an integer in the range from 0 to 2;
- each of B, independently from each other and at each occurrence is selected from C-R, O, N, S and $NR_{7'}$, wherein R is selected from hydrogen or an $C_{1-10}$ alkyl which is optionally substituted by a halogen atom, an aryl group or an aralkyl group, wherein $R_{7'}$ is selected from the group consisting of hydrogen, $C_{1-6}$ alkyl, $C_{1-6}$ alkenyl, cycloalkyl, heterocyclyl, aryl, aralkyl and $CF_3$; preferably each of B, independent from each other and at each occurrence, is each selected from C-R, O and $NR_{7'}$, wherein $R_{7'}$ is selected from hydrogen or $C_{1-4}$ alkyl and R is hydrogen or $C_{1-4}$ alkyl.
- each of E, independently from each other and at each occurrence is selected from C-R, O, N, S and $NR_{7'}$, wherein R is selected from hydrogen or an $C_{1-10}$ alkyl which is optionally substituted by a halogen atom, an aryl group or an aralkyl group, wherein $R_{7'}$ is selected from the group consisting of hydrogen, $C_{1-6}$ alkyl, $C_{1-6}$ alkenyl, cycloalkyl, heterocyclyl, aryl, aralkyl and $CF_3$; preferably each of E, independent from each other and at each occurrence, is selected from C-R, O and $NR_{7'}$, wherein $R_{7'}$ is selected from hydrogen or $C_{1-4}$ alkyl and R is hydrogen or $C_{1-4}$ alkyl.
- each of $R_{a4}$, independently from each other and at each occurrence is selected from the group consisting of $C_{1-15}$ alkyl, $C_{2-15}$ alkenyl, $C_{2-15}$ alkynyl, cycloalkyl, heterocyclyl, aryl, heteroaryl, aralkyl, halo, $NO_2$, $CF_3$, CN, $OR_{11}$, $SR_{11}$, $N(R_{11})_2$, $OC(R_{11})_2O$, $OC(R_{11})_2C(R_{11})_2O$, $COOR_{11}$, $CO(R_{11})_2$, and $CON(R_{11})_2$, and each optional alkyl, alkenyl, alkynyl, cycloalkyl, aryl, heterocyclyl, heteroaryl, aralkyl substituent is further optionally substituted with halo, alkyl, cycloalkyl, aryl, $N(R_{11})_2$, CN, or $OR_{11}$ and wherein each of $R_{11}$ , independently from each other and at each occurrence, is selected from the group consisting of hydrogen, $C_{1-6}$ alkyl, cycloalkyl, heterocyclyl, aryl, heteroaryl, and aralkyl, wherein said alkyl, alkenyl, alkynyl, cycloalkyl, heterocyclyl, aryl, heteroaryl, and aralkyl substituents are optionally substituted with halo, alkyl, cycloalkyl, heterocyclyl, $OR_{31}$ or $N(R_{32})_2$, wherein each of $R_{31}$ and $R_{32}$, independently from each other and at each occurrence, is selected from the group consisting of hydrogen and $C_{1-4}$

alkyl; more preferably, each of $R_{a4}$ is independently selected from the group consisting of halo, $OR_{11}$, $C_{1-6}$ alkyl, cycloalkyl, heterocyclyl, aryl, heteroaryl, $CF_3$, CN, $SR_{11}$, $N(R_{11})_2$, $OC(R_{11})_2O$, $OC(R_{11})_2C(R_{11})_2O$, $COOR_{11}$, $CO(R_{11})_2$, and $CON(R_{11})_2$, and wherein each of $R_{11}$ , independently from each other and at each occurrence, is selected from the group consisting of hydrogen and $C_{1-4}$ alkyl; even more preferably, each of $R_{a4}$ is independently selected from the group consisting of halo, $OC_{1-4}$ alkyl, $C_{1-4}$ alkyl, cycloalkyl, heterocyclyl, aryl, heteroaryl, $CF_3$, CN, $OCH_2O$, and $OCH_2CH_2O$; each of n4 is an integer in the range from 0 to 4; preferably each of n4 is an integer in the range from 0 to 2;

- each of $R_{a3}$, independently from each other and at each occurrence is selected from the group consisting of $C_{1-15}$ alkyl, $C_{2-15}$ alkenyl, $C_{2-15}$ alkynyl, cycloalkyl, heterocyclyl, aryl, heteroaryl, aralkyl, halo, $CF_3$, $OR_{11}$, $SR_{11}$, $N(R_{11})_2$, $COOR_{11}$, $CO(R_{11})_2$, and $CON(R_{11})_2$, and each optional alkyl, alkenyl, alkynyl, cycloalkyl, aryl, heterocyclyl, heteroaryl, aralkyl substituent is further optionally substituted with halo, alkyl, cycloalkyl, aryl, $N(R_{11})_2$, CN, or $OR_{11}$ and wherein each of $R_{11}$ , independently from each other and at each occurrence, is selected from the group consisting of hydrogen, $C_{1-6}$ alkyl, cycloalkyl, heterocyclyl, aryl, heteroaryl, and aralkyl, wherein said alkyl, cycloalkyl, heterocyclyl, aryl, heteroaryl, and aralkyl substituents are optionally substituted with halo, alkyl, cycloalkyl, heterocyclyl, $OR_{31}$ or $N(R_{32})_2$, wherein each of $R_{31}$ and $R_{32}$, independently from each other and at each occurrence, is selected from the group consisting of hydrogen and $C_{1-4}$alkyl; more preferably, each of $R_{a3}$ is independently selected from the group consisting of $C_{1-6}$ alkyl, cycloalkyl, heterocyclyl, aryl, and heteroaryl; even more preferably, $R_{a3}$ is $C_{1-4}$ alkyl, each of n3 is an integer in the range from 0 to 2.
- the dash bond represents an optional double bond
- $R'_1$ is selected from the group consisting of ($R'_1$-a) to ($R'_1$-d):

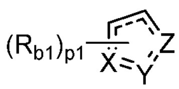

($R'_1$-a)

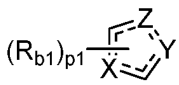

($R'_1$-b)

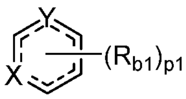

($R'_1$-c)

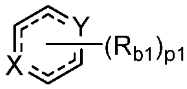

($R'_1$-d)

wherein:

- each of X, Y and Z independently from each other and at each occurrence is selected from C-R, O, N, S and $NR_{7'}$, wherein R is selected from hydrogen or a $C_{1-6}$ alkyl which is optionally substituted by a halogen atom, an aryl group or an aralkyl group, wherein $R_{7'}$ is selected from the group consisting of hydrogen, $C_{1-6}$ alkyl, $C_{1-6}$ alkenyl, cycloalkyl, heterocyclyl, aryl, aralkyl and $CF_3$; preferably each of X, Y and Z, independent from each other and at each occurrence, is each selected from C-R, O and $NR_{7'}$, wherein $R_{7'}$ is selected from hydrogen or $C_{1-4}$ alkyl and R is hydrogen or $C_{1-4}$ alkyl.
- each of $R_{b1}$, independently and at each occurrence, are selected from the group consisting of hydrogen, halo, $C_{1-6}$ alkyl, cycloalkyl, heterocyclyl, CN, $COR_{11}$, $CON(R_{11})_2$, and $OR_{11}$, wherein said alkyl, cycloalkyl and heterocyclyl are optionally substituted with one or more substituents selected from heterocyclyl, $OR_{11}$, $NR_{11}$ and wherein each of $R_{11}$ , independently from each other and at each occurrence, is selected from the group consisting of hydrogen, halo and $C_{1-6}$ alkyl; each of p1 is an integer in the range from 0 to 3;

wherein said cycloalkyl is a monocyclic, bicyclic or tricyclic ring system of 3-7 ring members per ring; said heterocyclyl is a saturated, partially saturated or completely saturated monocycle, bicycle or tricycle containing 3 to 12 carbon atoms and 1 or 2 heteroatoms independently selected from O or N; said aryl is phenyl, naphthyl or anthracenyl optionally carbocyclic fused with a cycloalkyl or heterocyclyl of 5-7 ring members; said heteroaryl is a monocyclic ring structure containing 5 or 6 ring atoms, or a bicyclic aromatic group having 8 to 10 atoms, containing 1-3 heteroatoms independently selected from O or N.

Preferred compounds of class (I) are selected from those of formula (IIa-1) to (IXa-1) herein below:

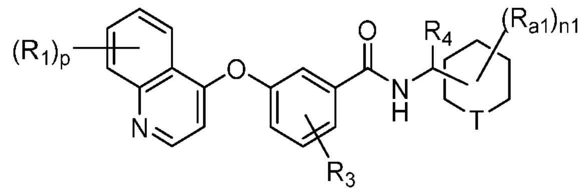

Formula (IIa-1)

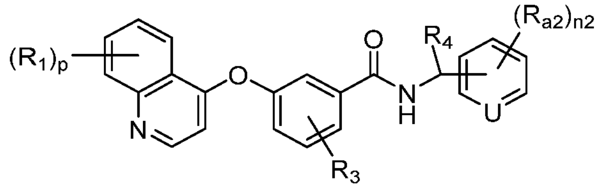

Formula (IIIa-1)

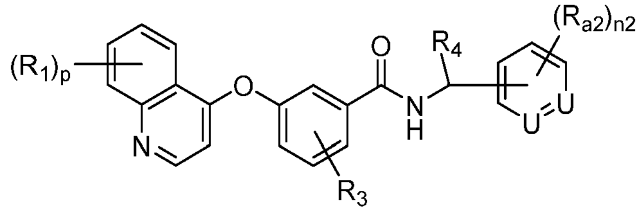

Formula (IVa-1)

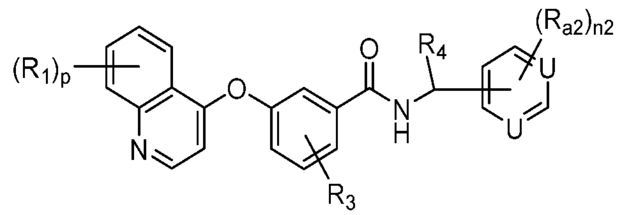

Formula (Va-1)

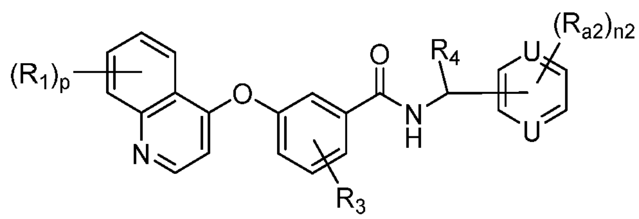

Formula (VIa-1)

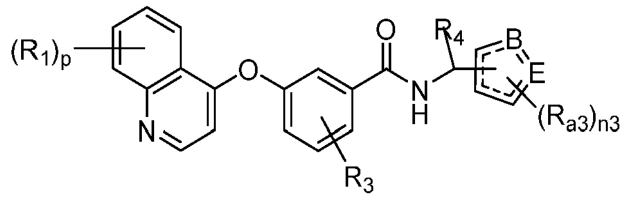

Formula (VIIa-1)

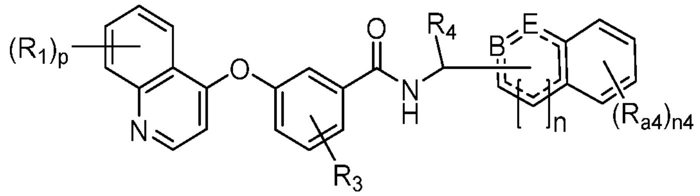

Formula (VIIIa-1)

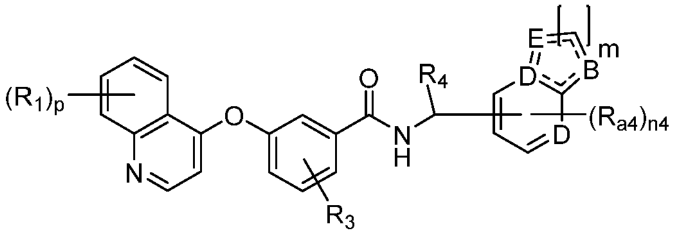

Formula (IXa-1)

wherein, $R_1$, $R_{a1}$, $R_{a2}$, $R_{a3}$, $R_{a4}$, T, U, B, E, D, p, n1, n2, n3, n4, m, n have the same meaning as defined above for formula (IIa) to (IXa) and the dash bond represents an optional double bond and wherein

- each of $R_4$, independently from each other and at each occurrence is selected from hydrogen, $CF_3$, $C_{1-4}$ alkyl, or cycloalkyl; preferably hydrogen, methyl, ethyl, propyl, butyl, tert-butyl, or iso-butyl;
- each of $R_3$, independently from each other and at each occurrence is selected from the group consisting of hydrogen, halo, $CF_3$, $C_{1-4}$ alkyl, cycloalkyl, $OR_{21}$, and $N(R_{21})_2$, and wherein each of $R_{21}$, independently from each other and at each occurrence, is hydrogen, $C_{1-4}$ alkyl such as methyl, ethyl, propyl, isopropyl, butyl, tert-butyl, isobutyl or $C_{3-6}$ cycloalkyl such as cyclopropyl, cyclobutyl, cyclopentyl, cyclohexyl; more preferably $R_3$ is independently selected from the group consisting of hydrogen, Cl, Br, F, OMe, $N(R_{21})_2$, and $C_{1-4}$ alkyl such as methyl, ethyl, propyl, isopropyl, butyl, tert-butyl, isobutyl, and wherein each of $R_{21}$, independently from each other and at each occurrence, is hydrogen, $C_{1-4}$ alkyl such as methyl, ethyl, propyl, isopropyl, butyl, tert-butyl, isobutyl or $C_{3-6}$ cycloalkyl such as cyclopropyl, cyclobutyl, cyclopentyl, cyclohexyl.

In a preferred embodiment of the present invention, the compound (C) for use as a kinase inhibitor, or the N-oxide, pharmaceutically acceptable salt, pharmaceutically acceptable solvate, or stereoisomer thereof, is a compound of formulae (IIa-1) to (IXa-1) wherein:

- each of $R_1$, independently from each other and at each occurrence, is selected from the group consisting of hydrogen, halo, $C_{1-6}$ alkyl, $C_{2-5}$ alkenyl, $C_{2-5}$ alkynyl, cycloalkyl, heterocyclyl, aryl, and heteroaryl, $CF_3$, CN, $NO_2$, $OR_{21}$, $SR_{21}$, $N(R_{21})_2$, $COR_{21}$, $C(O)OR_{21}$, $CON(R_{21})_2$, $OC(O)R_{21}$, $OCON(R_{21})_2$, $NC(O)R_{21}$, $NCON(R_{21})_2$, $OC(R_{21})_2O$ and $OC(R_{21})_2C(R_{22})_2O$, wherein said alkyl, alkenyl, alkynyl, cycloalkyl, heterocyclyl, aryl, and heteroaryl are optionally substituted with one or more substituents selected from halo, $C_{1-6}$ alkyl, cycloalkyl, heterocyclyl, $CF_3$, $COR_{21}$, $N(R_{21})_2$, CN, $CONR_{21}$, $C(O)OR_{21}$ or $OR_{21}$, and each optional alkyl substituent is further optionally substituted with heterocyclyl, $N(R_{11})_2$, or $OR_{11}$ ; and wherein each of $R_{21}$ and $R_{22}$, independently from each other and at each occurrence, is selected from the group consisting of hydrogen, $C_{1-6}$ alkyl, $C_{2-4}$ alkenyl, $C_{2-4}$ alkynyl, cycloalkyl, heterocyclyl, phenyl, heteroaryl, and aralkyl and wherein said alkyl, alkenyl, alkynyl, cycloalkyl, heterocyclyl, phenyl, heteroaryl, and aralkyl substituents are optionally substituted with halo, $C_{1-6}$ alkyl, cycloalkyl, heterocyclyl, phenyl, $OR_{31}$ or $N(R_{32})_2$, wherein each of $R_{31}$ and $R_{32}$, independently from each other and at each occurrence, is selected from the group consisting of hydrogen and $C_{1-4}$ alkyl; each of p is an integer in the range from 0 to 4 ;
- each of $R_4$, independently from each other and at each occurrence is selected from hydrogen, $CF_3$, $C_{1-4}$ alkyl, or cycloalkyl; preferably hydrogen, methyl, ethyl, propyl, butyl, tert-butyl, or iso-butyl;
- each of $R_3$, independently from each other and at each occurrence is selected from the group consisting of hydrogen, halo, $CF_3$, $C_{1-4}$ alkyl, cycloalkyl, $OR_{21}$, and $N(R_{21})_2$, and wherein each of $R_{21}$, independently from each other and at each occurrence, is hydrogen, $C_{1-4}$ alkyl such as methyl, ethyl, propyl, isopropyl, butyl, tert-butyl, isobutyl or $C_{3-6}$ cycloalkyl such as cyclopropyl, cyclobutyl, cyclopentyl, cyclohexyl; more preferably $R_3$ is independently selected from the group consisting of hydrogen, Cl, Br, F, OMe, $N(R_{21})_2$, and $C_{1-4}$ alkyl such as methyl, ethyl, propyl, isopropyl, butyl, tert-butyl, isobutyl, and wherein each of $R_{21}$, independently from each other and at each occurrence, is hydrogen, $C_{1-4}$ alkyl such as methyl, ethyl, propyl, isopropyl, butyl, tert-butyl, isobutyl or $C_{3-6}$ cycloalkyl such as cyclopropyl, cyclobutyl, cyclopentyl, cyclohexyl.
- T is selected from $CH_2$, N-R, O or S, wherein R is selected from hydrogen, $C_{1-10}$ alkyl or cycloalkyl which are optionally substituted by a halogen atom, an aryl group or an aralkyl group; preferably T is selected from $CH_2$ or O;
- each of U is selected, independently and at each occurrence, from C-halo, C-R or N; and wherein R is selected from hydrogen, $OR_{11}$, $N(R_{11})_2$, a $C_{1-10}$ alkyl or a cycloalkyl which are optionally substituted by a halogen atom, an aryl group or an aralkyl group, wherein each of $R_{11}$, independently from each other and at each occurrence, is selected from the group consisting of hydrogen and $C_{1-4}$ alkyl; preferably U is selected, independently and at each occurrence from C-halo, C-R or N; and preferably R is hydrogen or $C_{1-4}$ alkyl, more preferably U is selected, independently and at each occurrence from C-R or N; and more preferably R is hydrogen;
- each of D is selected, independently and at each occurrence, from C, C-R or N, wherein R is selected from hydrogen,

$C_{1-5}$ alkyl or cycloalkyl; preferably D is selected, independently and at each occurrence from C, C-R or N, and preferably R is hydrogen;

- m is an integer equal to 1 or 2;
- n is an integer equal to 0 or 1;
- each of $R_{a1}$, independently from each other and at each occurrence, is selected from the group consisting of hydrogen, $C_{1-5}$ alkyl, $C_{2-15}$ alkenyl and $C_{2-15}$ alkynyl, wherein said $C_{1-5}$ alkyl, $C_{2-15}$ alkenyl and $C_{2-15}$ alkynyl are optionally substituted with a halogen atom, an aryl group or an aralkyl group; each of n1 is an integer in the range from 0 to 5; preferably $R_{a1}$ is hydrogen or $C_{1-5}$ alkyl;
- each of $R_{a2}$, independently from each other and at each occurrence is selected from the group consisting of $C_{1-15}$ alkyl, $C_{2-15}$ alkenyl, $C_{2-15}$ alkynyl, cycloalkyl, heterocyclyl, aryl, heteroaryl, aralkyl, halo, $NO_2$, $CF_3$, CN, $OR_{11}$, $SR_{11}$, $N(R_{11})_2$, $OC(R_{11})_2O$, $OC(R_{11})_2C(R_{11})_2O$, $COOR_{11}$, $CO(R_{11})_2$, $CON(R_{11})_2$, and $SO_2N(R_{11})_2$ and each optional alkyl, alkenyl, alkynyl cycloalkyl, aryl, heterocyclyl, heteroaryl substituent is further optionally substituted with halo, $C_{1-6}$ alkyl, cycloalkyl, aryl, $N(R_{11})_2$, $CF_3$, CN, $COOR_{11}$, $CO(R_{11})_2$, $CON(R_{11})_2$, $SO_2N(R_{11})_2$ or $OR_{11}$ and wherein each of $R_{11}$ , independently from each other and at each occurrence, is selected from the group consisting of hydrogen, $C_{1-6}$ alkyl, cycloalkyl, heterocyclyl, aryl, heteroaryl, and aralkyl, wherein said alkyl, alkenyl, alkynyl, cycloalkyl, heterocyclyl, aryl, heteroaryl, and aralkyl substituents are optionally substituted with halo, $C_{1-6}$ alkyl, cycloalkyl, heterocyclyl, $OR_{31}$ or $N(R_{32})_2$, wherein each of $R_{31}$ and $R_{32}$, independently from each other and at each occurrence, is selected from the group consisting of hydrogen and $C_{1-4}$ alkyl; more preferably, each of $R_{a2}$ is independently selected from the group consisting of halo, $OR_{11}$, $C_{1-6}$ alkyl, cycloalkyl, heterocyclyl, aryl, heteroaryl, $CF_3$, CN, $SR_{11}$, $N(R_{11})_2$, $OC(R_{11})_2O$, $OC(R_{11})_2C(R_{11})_2O$, $COOR_{11}$, $CO(R_{11})_2$, and $CON(R_{11})_2$, and wherein each of $R_{11}$ , independently from each other and at each occurrence, is selected from the group consisting of hydrogen, $C_{1-4}$ alkyl, cycloalkyl and heterocyclyl; even more preferably, each of $R_{a2}$ is independently selected from the group consisting of halo, $OC_{1-4}$ alkyl, $C_{1-4}$ alkyl, cycloalkyl, heterocyclyl, aryl, heteroaryl, $CF_3$, CN, $OCH_2O$, $OCH_2CH_2O$, COOH, $COO(C_{1-4}$ alkyl), CO(heterocyclyl), $CO(C_{1-4}$ alkyl), $CONH(C_{1-4}$ alkyl), and CONH(cycloalkyl); each of n2 is an integer in the range from 0 to 4; preferably each of n2 is an integer in the range from 0 to 2;
- each of B, independently from each other and at each occurrence is selected from C-R, O, N, S and $NR_{7'}$, wherein R is selected from hydrogen or an $C_{1-10}$ alkyl which is optionally substituted by a halogen atom, an aryl group or an aralkyl group, wherein $R_{7'}$ is selected from the group consisting of hydrogen, $C_{1-6}$ alkyl, $C_{1-6}$ alkenyl, cycloalkyl, heterocyclyl, aryl, aralkyl and $CF_3$; preferably each of B, independent from each other and at each occurrence, is each selected from C-R, O and $NR_{7'}$, wherein $R_{7'}$ is selected from hydrogen or $C_{1-4}$ alkyl and R is hydrogen or $C_{1-4}$ alkyl.
- each of E, independently from each other and at each occurrence is selected from C-R, O, N, S and $NR_{7'}$, wherein R is selected from hydrogen or an $C_{1-10}$ alkyl which is optionally substituted by a halogen atom, an aryl group or an aralkyl group, wherein $R_{7'}$ is selected from the group consisting of hydrogen, $C_{1-6}$ alkyl, $C_{1-6}$ alkenyl, cycloalkyl, heterocyclyl, aryl, aralkyl and $CF_3$; preferably each of E, independent from each other and at each occurrence, is selected from C-R, O and $NR_{7'}$, wherein $R_{7'}$ is selected from hydrogen or $C_{1-4}$ alkyl and R is hydrogen or $C_{1-4}$ alkyl.
- each of $R_{a4}$, independently from each other and at each occurrence is selected from the group consisting of $C_{1-15}$ alkyl, $C_{2-15}$ alkenyl, $C_{2-15}$ alkynyl, cycloalkyl, heterocyclyl, aryl, heteroaryl, aralkyl, halo, $NO_2$, $CF_3$, CN, $OR_{11}$, $SR_{11}$, $N(R_{11})_2$, $OC(R_{11})_2O$, $OC(R_{11})_2C(R_{11})_2O$, $COOR_{11}$, $CO(R_{11})_2$, and $CON(R_{11})_2$, and each optional alkyl, alkenyl, alkynyl, cycloalkyl, aryl, heterocyclyl, heteroaryl, aralkyl substituent is further optionally substituted with halo, alkyl, cycloalkyl, aryl, $N(R_{11})_2$, CN, or $OR_{11}$ and wherein each of $R_{11}$ , independently from each other and at each occurrence, is selected from the group consisting of hydrogen, $C_{1-6}$ alkyl, cycloalkyl, heterocyclyl, aryl, heteroaryl, and aralkyl, wherein said alkyl, alkenyl, alkynyl, cycloalkyl, heterocyclyl, aryl, heteroaryl, and aralkyl substituents are optionally substituted with halo, alkyl, cycloalkyl, heterocyclyl, $OR_{31}$ or $N(R_{32})_2$, wherein each of $R_{31}$ and $R_{32}$, independently from each other and at each occurrence, is selected from the group consisting of hydrogen and $C_{1-4}$ alkyl; more preferably, each of $R_{a4}$ is independently selected from the group consisting of halo, $OR_{11}$, $C_{1-6}$ alkyl, cycloalkyl, heterocyclyl, aryl, heteroaryl, $CF_3$, CN, $SR_{11}$, $N(R_{11})_2$, $OC(R_{11})_2O$, $OC(R_{11})_2C(R_{11})_2O$, $COOR_{11}$, $CO(R_{11})_2$, and $CON(R_{11})_2$, and wherein each of $R_{11}$ , independently from each other and at each occurrence, is selected from the group consisting of hydrogen and $C_{1-4}$ alkyl; even more preferably, each of $R_{a4}$ is independently selected from the group consisting of halo, $OC_{1-4}$ alkyl, $C_{1-4}$ alkyl, cycloalkyl, heterocyclyl, aryl, heteroaryl, $CF_3$, CN, $OCH_2O$, and $OCH_2CH_2O$; each of n4 is an integer in the range from 0 to 4; preferably each of n4 is an integer in the range from 0 to 2;
- each of $R_{a3}$, independently from each other and at each occurrence is selected from the group consisting of $C_{1-15}$ alkyl, $C_{2-15}$ alkenyl, $C_{2-15}$ alkynyl, cycloalkyl, heterocyclyl, aryl, heteroaryl, aralkyl, halo, $CF_3$, $OR_{11}$, $SR_{11}$, $N(R_{11})_2$, $COOR_{11}$, $CO(R_{11})_2$, and $CON(R_{11})_2$, and each optional alkyl, alkenyl, alkynyl, cycloalkyl, aryl, heterocyclyl, heteroaryl, aralkyl substituent is further optionally substituted with halo, alkyl, cycloalkyl, aryl, $N(R_{11})_2$, CN, or $OR_{11}$ and wherein each of $R_{11}$ , independently from each other and at each occurrence, is selected from the group consisting of hydrogen, $C_{1-6}$ alkyl, cycloalkyl, heterocyclyl, aryl, heteroaryl, and aralkyl, wherein said alkyl, cycloalkyl, heterocyclyl, aryl, heteroaryl, and aralkyl substituents are optionally substituted with halo, alkyl, cycloalkyl, heterocyclyl, $OR_{31}$ or

$N(R_{32})_2$, wherein each of $R_{31}$ and $R_{32}$, independently from each other and at each occurrence, is selected from the group consisting of hydrogen and $C_{1-4}$alkyl; more preferably, each of $R_{a3}$ is independently selected from the group consisting of $C_{1-6}$ alkyl, cycloalkyl, heterocyclyl, aryl, and heteroaryl; even more preferably, $R_{a3}$ is $C_{1-4}$ alkyl, each of n3 is an integer in the range from 0 to 2.

wherein said cycloalkyl is a monocyclic, bicyclic or tricyclic ring system of 3-7 ring members per ring; said heterocyclyl is a saturated, partially saturated or completely saturated monocycle, bicycle or tricycle containing 3 to 12 carbon atoms and 1 or 2 heteroatoms independently selected from O or N; said aryl is phenyl, naphthyl or anthracenyl optionally carbocyclic fused with a cycloalkyl or heterocyclyl of 5-7 ring members; said heteroaryl is a monocyclic ring structure containing 5 or 6 ring atoms, or a bicyclic aromatic group having 8 to 10 atoms, containing 1-3 heteroatoms independently selected from O or N.

In one embodiment of the present invention, the compounds (C) of class (I) are selected from those of formula (II-a) and (IX-a).

In a preferred embodiment of the present invention, the compound (C), according to the present invention, for use as a kinase inhibitor according to general formula (IIa-1) is a compound chosen among those of formulae (IIa-1-1) herein below:

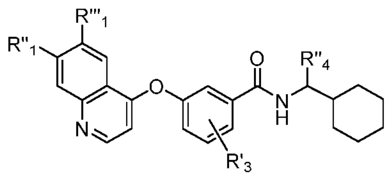

Formula (IIa-1-1)

wherein:

- each of $R''_1$ and $R'''_1$ are independently selected from the group consisting of hydrogen and $OR_{21}$, wherein each of $R_{21}$ is independently $C_{1-4}$ alkyl;
- each of $R'_3$ is selected from the group consisting of hydrogen, halo and $C_{1-4}$ alkyl;
- each of $R''_4$ is selected from the group consisting of hydrogen and $C_{1-4}$ alkyl.

In a preferred embodiment of the present invention, the compound (C), according to the present invention, for use as a kinase inhibitor according to general formula (IIIa-1) is a compound chosen among those of formulae (IIIa-1-1) and (IIIa-1-2) herein below:

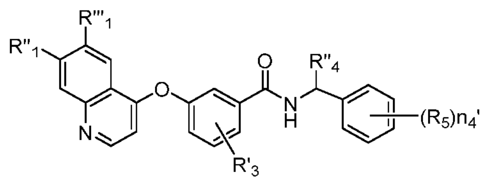

Formula (IIIa-1-1)

wherein:

- each of $R''_1$ and $R'''_1$ are independently selected from the group consisting of hydrogen, halo, $C_{1-4}$ alkyl, heterocyclyl selected from the group consisting of azetidinyl and pyrrolidinyl, heteroaryl selected from the group consisting of imidazolyl, pyrazolyl, triazolyl, and pyrimidinyl, CN, $N(R_{21})_2$ and $OR_{21}$, wherein each of $R_{21}$ is independently hydrogen, $C_{1-4}$ alkyl, or cyclopropyl;
- each of $R'_3$ is selected from the group consisting of hydrogen, halo and $C_{1-4}$ alkyl, or $OR_{21}$, wherein $R_{21}$ is $C_{1-4}$ alkyl;
- each of $R''_4$ is selected from the group consisting of hydrogen and $C_{1-4}$ alkyl;
- each of $R_5$ is selected from the group consisting of hydrogen, halo, $C_{3-6}$ cycloalkyl such as cyclopropyl, heterocyclyl selected from the group consisting of morpholinyl, azetidinyl, heteroaryl such as furanyl, $CF_3$, CN, $COR_{21}$, $CON(R_{21})_2$, $C(O)OR_{21}$, $OR_{21}$ and OCH2CH2O, wherein each of $R_{21}$ is independently hydrogen, $C_{1-4}$ alkyl such as methyl, ethyl, iso-butyl, heterocyclyl selected from the group consisting of azetidinyl, morpholinyl;
- n'4 is an integer equal to 0, 1 or 2.

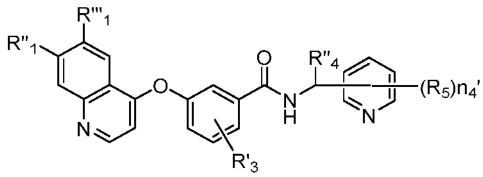

Formula (IIIa-1-2)

wherein:

- each of R"$_1$ and R‴$_1$ are independently selected from the group consisting of hydrogen, halo, $C_{1-4}$ alkyl, azetidinyl, heteroaryl selected from the group consisting of furanyl, oxazolyl, oxadiazolyl, isoxazolyl, pyrazinyl, pyridinyl, pyrrolyl, imidazolyl, pyrazolyl, triazolyl, and pyrimidinyl, $CF_3$, CN, $N(R_{21})_2$, $CON(R_{21})_2$ and $OR_{21}$, wherein each of $R_{21}$ is independently hydrogen, $C_{1-4}$ alkyl optionally substituted with halo, or cyclopropyl;
- each of R'$_3$ is selected from the group consisting of hydrogen, halo and $C_{1-4}$ alkyl;
- each of R"$_4$ is selected from the group consisting of hydrogen and $C_{1-4}$ alkyl;
- each of $R_5$ is selected from the group consisting of hydrogen, halo, cycloalkyl such as cyclopropyl, and heteroaryl selected from the group consisting of furanyl, CN, $COR_{21}$, $CON(R_{21})_2$, $COOR_{21}$, and $OR_{21}$, wherein each of $R_{21}$ is independently hydrogen, $C_{1-4}$ alkyl such as methyl, ethyl, iso-butyl, optionally substituted with halo, cycloalkyl selected from the group consisting of cyclopropyl and cyclobutyl; n'4 is an integer equal to 0, 1, 2 or 3.

In a preferred embodiment of the present invention, the compound (C), according to the present invention, for use as a kinase inhibitor according to general formula (IVa-1) is a compound chosen among those of formula (IVa-1-1) herein below:

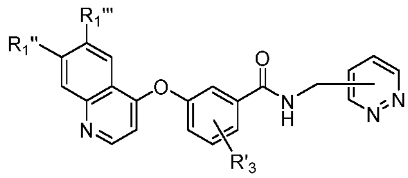

Formula (IVa-1-1)

wherein:

- each of R", and R‴$_1$ is independently selected from the group consisting of hydrogen, halo, and $OR_{21}$, wherein $R_{21}$ is $C_{1-4}$ alkyl;
- each of R'$_3$ is $C_{1-4}$ alkyl.

In a preferred embodiment of the present invention, the compound (C), according to the present invention, for use as a kinase inhibitor according to general formula (IVa-1) is a compound chosen among those of formula (Va-1-1) herein below:

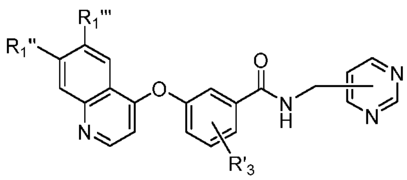

Formula (Va-1-1)

wherein:

- each of R", and R‴$_1$ is independently selected from the group consisting of hydrogen and $OR_{21}$, wherein $R_{21}$ is $C_{1-4}$ alkyl;
- each of R'$_3$ is $C_{1-4}$ alkyl.

In a preferred embodiment of the present invention, the compound (C), according to the present invention, for use as a kinase inhibitor according to general formula (VIIa-1) is a compound chosen among those of formula (VIIa-1-1) herein below:

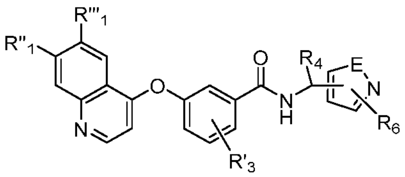

Formula (VIIa-1-1)

wherein:

- each of R", and R‴$_1$ is independently selected from the group consisting of hydrogen, halo, and $OR_{21}$, wherein $R_{21}$ is $C_{1-4}$ alkyl;
- E is selected from the group consisting of N-$R_5$ and O, wherein $R_5$ is selected from the group consisting of hydrogen or $C_{1-4}$ alkyl;
- $R'_3$ is $C_{1-4}$ alkyl;
- $R_4$ is hydrogen;
- $R_6$ is selected from the group consisting of hydrogen, and $C_{1-4}$ alkyl.

Preferred compounds of class (I) are selected from those of formula (IIa-a1) to (IXa-a1) herein below:

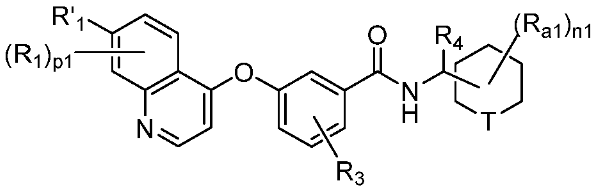

Formula (IIa-a1)

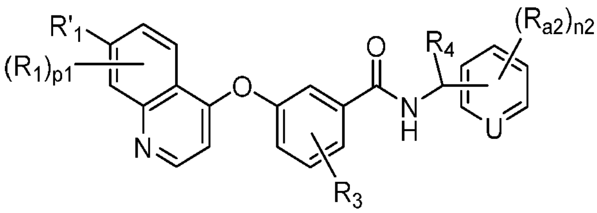

Formula (IIIa-a1)

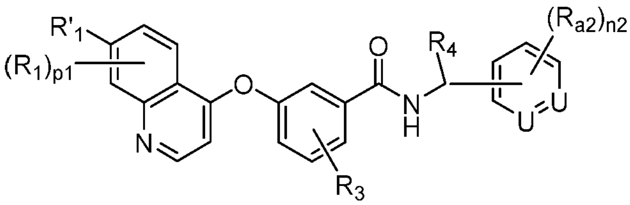

Formula (IVa-a1)

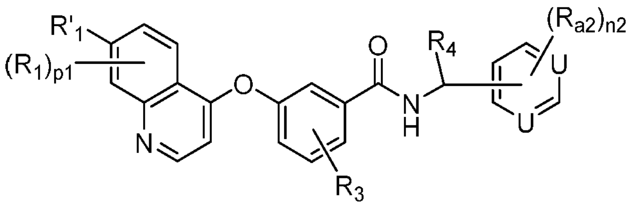

Formula (Va-a1)

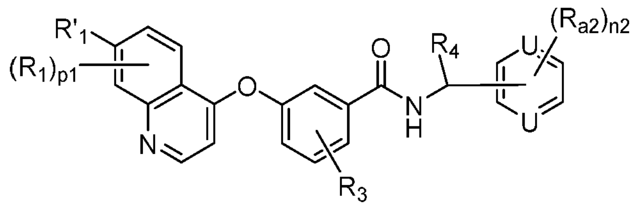

Formula (VIa-a1)

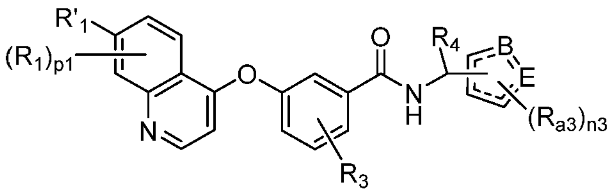

Formula (VIIa-a1)

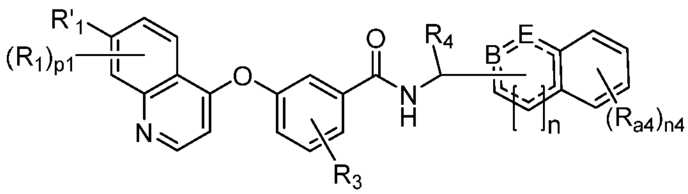

Formula (VIIIa-a1)

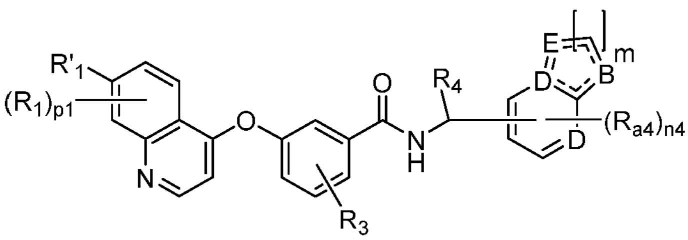

Formula (IXa-a1)

wherein $R_1$, $R'_1$, $R_{a1}$, $R_{a2}$, $R_{a3}$, $R_{a4}$, T, U, B, E, D, p1, n1, n2, n3, n4, m, n have the same meaning as defined above for formula (IIa-a) to (IXa-a) and the dash bond represents an optional double bond and wherein:

- each of $R_4$, independently from each other and at each occurrence is selected from hydrogen, $CF_3$, $C_{1-4}$ alkyl, or cycloalkyl; preferably hydrogen, methyl, ethyl, propyl, butyl, tert-butyl, or iso-butyl;
- each of $R_3$, independently from each other and at each occurrence is selected from the group consisting of hydrogen, $CF_3$, halo, $C_{1-4}$ alkyl, cycloalkyl, $OR_{21}$, and $N(R_{21})_2$, and wherein each of $R_{21}$, independently from each other and at each occurrence, is hydrogen, $C_{1-4}$ alkyl such as methyl, ethyl, propyl, isopropyl, butyl, *tert*-butyl, isobutyl or $C_{3-6}$ cycloalkyl such as cyclopropyl, cyclobutyl, cyclopentyl, cyclohexyl; more preferably $R_3$ is independently selected from the group consisting of hydrogen, Cl, Br, F, OMe, $N(R_{21})_2$, and $C_{1-4}$ alkyl such as methyl, ethyl, propyl, isopropyl, butyl, *tert*-butyl, isobutyl, and wherein each of $R_{21}$, independently from each other and at each occurrence, is hydrogen, $C_{1-4}$ alkyl such as methyl, ethyl, propyl, isopropyl, butyl, *tert*-butyl, isobutyl or $C_{3-6}$ cycloalkyl such as cyclopropyl, cyclobutyl, cyclopentyl, cyclohexyl.

wherein said cycloalkyl is a monocyclic, bicyclic or tricyclic ring system of 3-7 ring members per ring; said heterocyclyl is a saturated, partially saturated or completely saturated monocycle, bicycle or tricycle containing 3 to 12 carbon atoms and 1 or 2 heteroatoms independently selected from O or N; said aryl is phenyl, naphthyl or anthracenyl optionally carbocyclic fused with a cycloalkyl or heterocyclyl of 5-7 ring members; said heteroaryl is a monocyclic ring structure containing 5 or 6 ring atoms, or a bicyclic aromatic group having 8 to 10 atoms, containing 1-3 heteroatoms independently selected from O or N.

In a preferred embodiment of the present invention, the compound (C) for use as a kinase inhibitor, or the N-oxide, pharmaceutically acceptable salt, pharmaceutically acceptable solvate, or stereoisomer thereof, is a compound of formulae (IIa-a1) to (IXa-a1) wherein:

- $R_1$, independently from each other and at each occurrence, is selected from the group consisting of hydrogen, halo,

$C_{1-6}$ alkyl, $C_{2-5}$ alkenyl, $C_{2-5}$ alkynyl, cycloalkyl, heterocyclyl, aryl, and heteroaryl, $CF_3$, CN, $NO_2$, $OR_{21}$, $SR_{21}$, $N(R_{21})_2$, $COR_{21}$, $C(O)OR_{21}$, $CON(R_{21})_2$, $OC(O)R_{21}$, $OCON(R_{21})_2$, $NC(O)R_{21}$, $NCON(R_{21})_2$, $OC(R_{21})_2O$ and $OC(R_{21})_2C(R_{22})_2O$, wherein said alkyl, alkenyl, alkynyl, cycloalkyl, heterocyclyl, aryl, and heteroaryl are optionally substituted with one or more substituents selected from halo, $C_{1-6}$ alkyl, cycloalkyl, heterocyclyl, $CF_3$, $COR_{21}$, $N(R_{21})_2$, CN, $CONR_{21}$, $C(O)OR_{21}$ or $OR_{21}$, and each optional alkyl substituent is further optionally substituted with heterocyclyl, $N(R_{11})_2$, or $OR_{11}$ ; and wherein each of $R_{21}$ and $R_{22}$, independently from each other and at each occurrence, is selected from the group consisting of hydrogen, $C_{1-6}$ alkyl, $C_{2-4}$ alkenyl, $C_{2-4}$ alkynyl, cycloalkyl, heterocyclyl, phenyl, heteroaryl, and aralkyl, and wherein said alkyl, alkenyl, alkynyl, cycloalkyl, heterocyclyl, phenyl, heteroaryl, and aralkyl substituents are optionally substituted with halo, $C_{1-6}$ alkyl, cycloalkyl, heterocyclyl, phenyl, $OR_{31}$ or $N(R_{32})_2$, wherein each of $R_{31}$ and $R_{32}$, independently from each other and at each occurrence, is selected from the group consisting of hydrogen and $C_{1-4}$ alkyl; each of p1 is an integer in the range from 0 to 3;

- $R'_1$ is selected from the group consisting of ($R'_1$-a) to ($R'_1$-d):

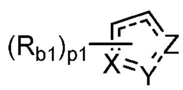

($R'_1$-a)

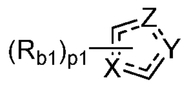

($R'_1$-b)

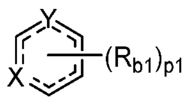

($R'_1$-c)

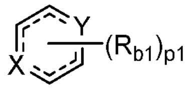

($R'_1$-d)

wherein:

- each of X, Y and Z independently from each other and at each occurrence is selected from C-R, O, N, S and $NR_{7'}$, wherein R is selected from hydrogen or a $C_{1-6}$ alkyl which is optionally substituted by a halogen atom, an aryl group or an aralkyl group, wherein $R_{7'}$ is selected from the group consisting of hydrogen, $C_{1-6}$ alkyl, $C_{1-6}$ alkenyl, cycloalkyl, heterocyclyl, aryl, aralkyl and $CF_3$, and each alkyl, alkenyl, cycloalkyl, heterocyclyl, aryl or aralkyl substituent is further optionally substituted with heterocyclyl, $N(R_{11})_2$, or $OR_{11}$; preferably each of X, Y and Z, independent from each other and at each occurrence, is each selected from C-R, O and $NR_{7'}$, wherein $R_{7'}$ is selected from hydrogen or $C_{1-4}$ alkyl and R is hydrogen or $C_{1-4}$ alkyl.
- each of $R_{b1}$, independently and at each occurrence, are selected from the group consisting of hydrogen, halo, $C_{1-6}$ alkyl, cycloalkyl, heterocyclyl, CN, $CF_3$, $COR_{11}$, $CON(R_{11})_2$, $NR_{11}$, and $OR_{11}$, wherein said alkyl, cycloalkyl and heterocyclyl are optionally substituted with one or more substituents selected from heterocyclyl, $OR_{11}$, $NR_{11}$ and wherein each of $R_{11}$ , independently from each other and at each occurrence, is selected from the group consisting of hydrogen, halo and $C_{1-6}$ alkyl; each of p1 is an integer in the range from 0 to 3.
- the dash bond represents an optional double bond

- each of $R_3$, independently from each other and at each occurrence is selected from the group consisting of hydrogen, halo, $CF_3$, $C_{1-4}$ alkyl, cycloalkyl, $OR_{21}$, and $N(R_{21})_2$, and wherein each of $R_{21}$, independently from each other and at each occurrence, is hydrogen, $C_{1-4}$ alkyl such as methyl, ethyl, propyl, isopropyl, butyl, tert-butyl, isobutyl or $C_{3-6}$ cycloalkyl such as cyclopropyl, cyclobutyl, cyclopentyl, cyclohexyl; more preferably $R_3$ is independently selected from the group consisting of hydrogen, Cl, Br, F, OMe, $N(R_{21})_2$, and $C_{1-4}$ alkyl such as methyl, ethyl, propyl, isopropyl, butyl, tert-butyl, isobutyl, and wherein each of $R_{21}$, independently from each other and at each occurrence, is hydrogen, $C_{1-4}$ alkyl such as methyl, ethyl, propyl, isopropyl, butyl, *tert*-butyl, isobutyl or $C_{3-6}$ cycloalkyl such as cyclopropyl, cyclobutyl, cyclopentyl, cyclohexyl;
- each of $R_4$, independently from each other and at each occurrence is selected from hydrogen, $CF_3$, $C_{1-4}$ alkyl, or cycloalkyl; preferably hydrogen, methyl, ethyl, propyl, butyl, *tert*-butyl, or iso-butyl;
- T is selected from $CH_2$, N-R, O or S, wherein R is selected from hydrogen, $C_{1-10}$ alkyl or cycloalkyl which are optionally substituted by a halogen atom, an aryl group or an aralkyl group; preferably T is selected from $CH_2$ or O;
- each of U is selected, independently and at each occurrence, from C-halo, C-R or N; and wherein R is selected from hydrogen, $OR_{11}$, $N(R_{11})_2$, a $C_{1-10}$ alkyl or a cycloalkyl which are optionally substituted by a halogen atom, an aryl group or an aralkyl group, wherein each of $R_{11}$, independently from each other and at each occurrence, is selected from the group consisting of hydrogen and $C_{1-4}$ alkyl; preferably U is selected, independently and at each occurrence from C-halo, C-R or N; and preferably R is hydrogen or $C_{1-4}$ alkyl, more preferably U is selected, independently and at each

occurrence from C-R or N; and more preferably R is hydrogen;

- each of D is selected, independently and at each occurrence, from C, C-R or N, wherein R is selected from hydrogen, $C_{1-5}$ alkyl or cycloalkyl; preferably D is selected, independently and at each occurrence from C, C-R or N, and preferably R is hydrogen;
- m is an integer equal to 1 or 2;
- n is an integer equal to 0 or 1 ;
- each of $R_{a1}$, independently from each other and at each occurrence, is selected from the group consisting of hydrogen, $C_{1-5}$ alkyl, $C_{2-15}$ alkenyl and $C_{2-15}$ alkynyl, wherein said $C_{1-5}$ alkyl, $C_{2-15}$ alkenyl and $C_{2-15}$ alkynyl are optionally substituted with a halogen atom, an aryl group or an aralkyl group; each of n1 is an integer in the range from 0 to 5; preferably $R_{a1}$ is hydrogen or $C_{1-5}$ alkyl;
- each of $R_{a2}$, independently from each other and at each occurrence is selected from the group consisting of $C_{1-15}$ alkyl, $C_{2-15}$ alkenyl, $C_{2-15}$ alkynyl, cycloalkyl, heterocyclyl, aryl, heteroaryl, aralkyl, halo, $NO_2$, $CF_3$, CN, $OR_{11}$, $SR_{11}$, $N(R_{11})_2$, $OC(R_{11})_2O$, $OC(R_{11})_2C(R_{11})_2O$, $COOR_{11}$, $CO(R_{11})_2$, $CON(R_{11})_2$, and $SO_2N(R_{11})_2$, and each optional alkyl, alkenyl, alkynyl cycloalkyl, aryl, heterocyclyl, heteroaryl substituent is further optionally substituted with halo, $C_{1-6}$ alkyl, cycloalkyl, aryl, $N(R_{11})_2$, $CF_3$, CN, $COOR_{11}$, $CO(R_{11})_2$, $CON(R_{11})_2$, $SO_2N(R_{11})_2$ or $OR_{11}$ and wherein each of $R_{11}$, independently from each other and at each occurrence, is selected from the group consisting of hydrogen, $C_{1-6}$ alkyl, cycloalkyl, heterocyclyl, aryl, heteroaryl, and aralkyl, wherein said alkyl, alkenyl, alkynyl, cycloalkyl, heterocyclyl, aryl, heteroaryl, and aralkyl substituents are optionally substituted with halo, $C_{1-6}$ alkyl, cycloalkyl, heterocyclyl, $OR_{31}$ or $N(R_{32})_2$, wherein each of $R_{31}$ and $R_{32}$, independently from each other and at each occurrence, is selected from the group consisting of hydrogen and $C_{1-4}$ alkyl; more preferably, each of $R_{a2}$ is independently selected from the group consisting of halo, $OR_{11}$, $C_{1-6}$ alkyl, cycloalkyl, heterocyclyl, aryl, heteroaryl, $CF_3$, CN, $SR_{11}$, $N(R_{11})_2$, $OC(R_{11})_2O$, $OC(R_{11})_2C(R_{11})_2O$, $COOR_{11}$, $CO(R_{11})_2$, and $CON(R_{11})_2$, and wherein each of $R_{11}$ , independently from each other and at each occurrence, is selected from the group consisting of hydrogen, $C_{1-4}$ alkyl, cycloalkyl and heterocyclyl; even more preferably, each of $R_{a2}$ is independently selected from the group consisting of halo, $OC_{1-4}$ alkyl, $C_{1-4}$ alkyl, cycloalkyl, heterocyclyl, aryl, heteroaryl, $CF_3$, CN, $OCH_2O$, OCH2CH2O, COOH, COO($C_{1-4}$ alkyl), CO(heterocyclyl), CO($C_{1-4}$ alkyl), CONH($C_{1-4}$ alkyl), and CONH(cycloalkyl); each of n2 is an integer in the range from 0 to 4; preferably each of n2 is an integer in the range from 0 to 2;
- each of B, independently from each other and at each occurrence is selected from C-R, O, N, S and $NR_{7'}$, wherein R is selected from hydrogen or an $C_{1-10}$ alkyl which is optionally substituted by a halogen atom, an aryl group or an aralkyl group, wherein $R_{7'}$ is selected from the group consisting of hydrogen, $C_{1-6}$ alkyl, $C_{1-6}$ alkenyl, cycloalkyl, heterocyclyl, aryl, aralkyl and $CF_3$; preferably each of B, independent from each other and at each occurrence, is each selected from C-R, O and $NR_{7'}$, wherein $R_{7'}$ is selected from hydrogen or $C_{1-4}$ alkyl and R is hydrogen or $C_{1-4}$ alkyl.
- each of E, independently from each other and at each occurrence is selected from C-R, O, N, S and $NR_{7'}$, wherein R is selected from hydrogen or an $C_{1-10}$ alkyl which is optionally substituted by a halogen atom, an aryl group or an aralkyl group, wherein $R_{7'}$ is selected from the group consisting of hydrogen, $C_{1-6}$ alkyl, $C_{1-6}$ alkenyl, cycloalkyl, heterocyclyl, aryl, aralkyl and $CF_3$; preferably each of E, independent from each other and at each occurrence, is selected from C-R, O and $NR_{7'}$, wherein $R_{7'}$ is selected from hydrogen or $C_{1-4}$ alkyl and R is hydrogen or $C_{1-4}$ alkyl.
- each of $R_{a4}$, independently from each other and at each occurrence is selected from the group consisting of $C_{1-15}$ alkyl, $C_{2-15}$ alkenyl, $C_{2-15}$ alkynyl, cycloalkyl, heterocyclyl, aryl, heteroaryl, aralkyl, halo, $NO_2$, $CF_3$, CN, $OR_{11}$, $SR_{11}$, $N(R_{11})_2$, $OC(R_{11})_2O$, $OC(R_{11})_2C(R_{11})_2O$, $COOR_{11}$, $CO(R_{11})_2$, and $CON(R_{11})_2$, and each optional alkyl, alkenyl, alkynyl, cycloalkyl, aryl, heterocyclyl, heteroaryl, aralkyl substituent is further optionally substituted with halo, alkyl, cycloalkyl, aryl, $N(R_{11})_2$, CN, or $OR_{11}$ and wherein each of $R_{11}$ , independently from each other and at each occurrence, is selected from the group consisting of hydrogen, $C_{1-6}$ alkyl, cycloalkyl, heterocyclyl, aryl, heteroaryl, and aralkyl, wherein said alkyl, alkenyl, alkynyl, cycloalkyl, heterocyclyl, aryl, heteroaryl, and aralkyl substituents are optionally substituted with halo, alkyl, cycloalkyl, heterocyclyl, $OR_{31}$ or $N(R_{32})_2$, wherein each of $R_{31}$ and $R_{32}$, independently from each other and at each occurrence, is selected from the group consisting of hydrogen and $C_{1-4}$ alkyl; more preferably, each of $R_{a4}$ is independently selected from the group consisting of halo, $OR_{11}$, $C_{1-6}$ alkyl, cycloalkyl, heterocyclyl, aryl, heteroaryl, $CF_3$, CN, $SR_{11}$, $N(R_{11})_2$, $OC(R_{11})_2O$, $OC(R_{11})_2C(R_{11})_2O$, $COOR_{11}$, $CO(R_{11})_2$, and $CON(R_{11})_2$, and wherein each of $R_{11}$ , independently from each other and at each occurrence, is selected from the group consisting of hydrogen and $C_{1-4}$ alkyl; even more preferably, each of $R_{a4}$ is independently selected from the group consisting of halo, $OC_{1-4}$ alkyl, $C_{1-4}$ alkyl, cycloalkyl, heterocyclyl, aryl, heteroaryl, $CF_3$, CN, $OCH_2O$, and $OCH_2CH_2O$; each of n4 is an integer in the range from 0 to 4; preferably each of n4 is an integer in the range from 0 to 2;
- each of $R_{a3}$, independently from each other and at each occurrence is selected from the group consisting of $C_{1-15}$ alkyl, $C_{2-15}$ alkenyl, $C_{2-15}$ alkynyl, cycloalkyl, heterocyclyl, aryl, heteroaryl, aralkyl, halo, $CF_3$, $OR_{11}$, $SR_{11}$, $N(R_{11})_2$, $COOR_{11}$, $CO(R_{11})_2$, and $CON(R_{11})_2$, and each optional alkyl, alkenyl, alkynyl, cycloalkyl, aryl, heterocyclyl, heteroaryl, aralkyl substituent is further optionally substituted with halo, alkyl, cycloalkyl, aryl, $N(R_{11})_2$, CN, or $OR_{11}$ and wherein each of $R_{11}$ , independently from each other and at each occurrence, is selected from the group consisting of

hydrogen, $C_{1-6}$ alkyl, cycloalkyl, heterocyclyl, aryl, heteroaryl, and aralkyl, wherein said alkyl, cycloalkyl, heterocyclyl, aryl, heteroaryl, and aralkyl substituents are optionally substituted with halo, alkyl, cycloalkyl, heterocyclyl, $OR_{31}$ or $N(R_{32})_2$, wherein each of $R_{31}$ and $R_{32}$, independently from each other and at each occurrence, is selected from the group consisting of hydrogen and $C_{1-4}$alkyl; more preferably, each of $R_{a3}$ is independently selected from the group consisting of $C_{1-6}$ alkyl, cycloalkyl, heterocyclyl, aryl, and heteroaryl; even more preferably, $R_{a3}$ is $C_{1-4}$ alkyl, each of n3 is an integer in the range from 0 to 2.

wherein said cycloalkyl is a monocyclic, bicyclic or tricyclic ring system of 3-7 ring members per ring; said heterocyclyl is a saturated, partially saturated or completely saturated monocycle, bicycle or tricycle containing 3 to 12 carbon atoms and 1 or 2 heteroatoms independently selected from O or N; said aryl is phenyl, naphthyl or anthracenyl optionally carbocyclic fused with a cycloalkyl or heterocyclyl of 5-7 ring members; said heteroaryl is a monocyclic ring structure containing 5 or 6 ring atoms, or a bicyclic aromatic group having 8 to 10 atoms, containing 1-3 heteroatoms independently selected from O or N.

Preferred compounds of class (I) are selected from those of formula (Xa-1) to (XVIIa-1) herein below:

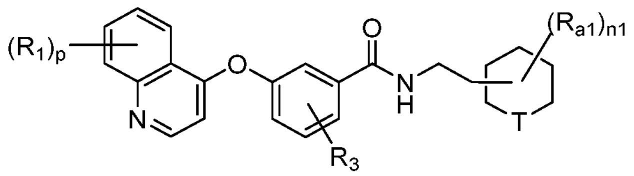

Formula (Xa-1)

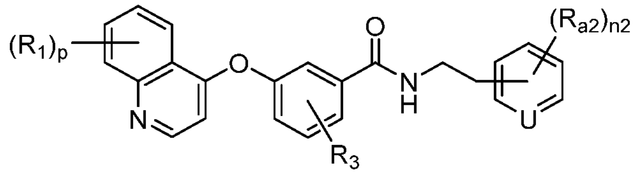

Formula (XIa-1)

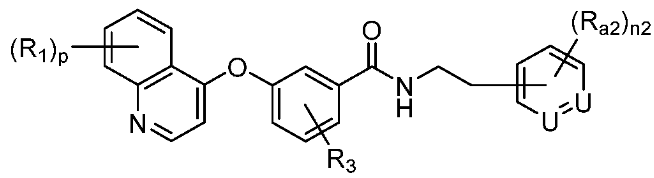

Formula (XIIa-1)

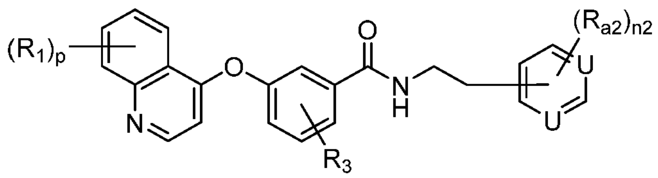

Formula (XIIIa-1)

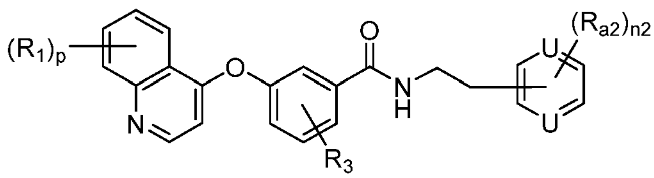

Formula (XIVa-1)

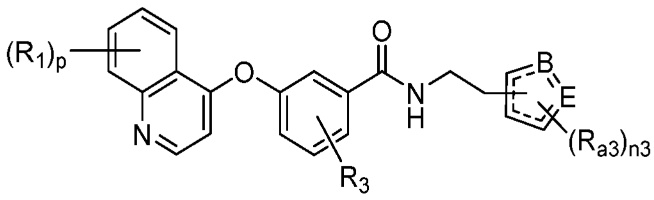

Formula (XVa-1)

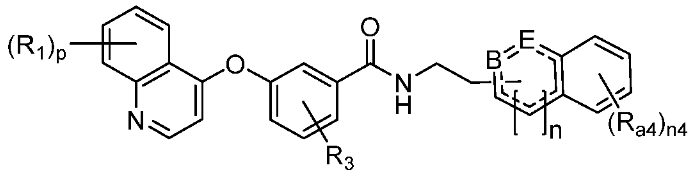

Formula (XVIa-1)

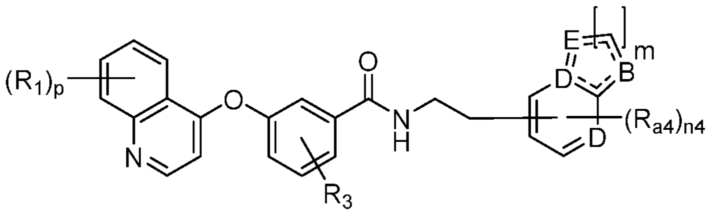

Formula (XVIIa-1)

wherein, $R_1$, $R_{a1}$, $R_{a2}$, $R_{a3}$, $R_{a4}$, T, U, B, E, D, p, n1, n2, n3, n4, m, n have the same meaning as defined above for formula (IIa) to (IXa) and the dash bond represents an optional double bond and wherein

- each of $R_3$, independently from each other and at each occurrence is selected from the group consisting of hydrogen, $CF_3$, halo, $C_{1-4}$ alkyl, cycloalkyl, $OR_{21}$, and $N(R_{21})_2$, and wherein each of $R_{21}$, independently from each other and at each occurrence, is hydrogen, $C_{1-4}$ alkyl such as methyl, ethyl, propyl, isopropyl, butyl, *tert*-butyl, isobutyl or $C_{3-6}$ cycloalkyl such as cyclopropyl, cyclobutyl, cyclopentyl, cyclohexyl; more preferably $R_3$ is independently selected from the group consisting of hydrogen, Cl, Br, F, OMe, $N(R_{21})_2$, and $C_{1-4}$ alkyl such as methyl, ethyl, propyl, isopropyl, butyl, *tert*-butyl, isobutyl and wherein each of $R_{21}$, independently from each other and at each occurrence, is hydrogen, $C_{1-4}$ alkyl such as methyl, ethyl, propyl, isopropyl, butyl, *tert*-butyl, isobutyl or $C_{3-6}$ cycloalkyl such as cyclopropyl, cyclobutyl, cyclopentyl, cyclohexyl.

In a preferred embodiment of the present invention, the compound (C) for use as a kinase inhibitor, or the N-oxide, pharmaceutically acceptable salt, pharmaceutically acceptable solvate, or stereoisomer thereof, is a compound of formulae (Xa-1) to (XVIIa-1) wherein:

- each of $R_1$, independently from each other and at each occurrence, is selected from the group consisting of hydrogen, halo, $C_{1-6}$ alkyl, $C_{2-5}$ alkenyl, $C_{2-5}$ alkynyl, cycloalkyl, heterocyclyl, aryl, and heteroaryl, $CF_3$, CN, $NO_2$, $OR_{21}$, $SR_{21}$, $N(R_{21})_2$, $COR_{21}$, $C(O)OR_{21}$, $CON(R_{21})_2$, $OC(O)R_{21}$, $OCON(R_{21})_2$, $NC(O)R_{21}$, $NCON(R_{21})_2$, $OC(R_{21})_2O$ and $OC(R_{21})_2C(R_{22})_2O$, wherein said alkyl, alkenyl, alkynyl, cycloalkyl, heterocyclyl, aryl, and heteroaryl are optionally substituted with one or more substituents selected from halo, $C_{1-6}$ alkyl, cycloalkyl, heterocyclyl, $CF_3$, $COR_{21}$, $N(R_{21})_2$, CN, $CONR_{21}$, $C(O)OR_{21}$ or $OR_{21}$, and each optional alkyl substituent is further optionally substituted with heterocyclyl, $N(R_{11})_2$, or $OR_{11}$ ; and wherein each of $R_{21}$ and $R_{22}$, independently from each other and at each occurrence, is selected from the group consisting of hydrogen, $C_{1-6}$ alkyl, $C_{2-4}$ alkenyl, $C_{2-4}$ alkynyl, cycloalkyl, heterocyclyl, phenyl, heteroaryl, and aralkyl, and wherein said alkyl, alkenyl, alkynyl, cycloalkyl, heterocyclyl, phenyl, heteroaryl, and aralkyl substituents are optionally substituted with halo, $C_{1-6}$ alkyl, cycloalkyl, heterocyclyl, phenyl, $OR_{31}$ or $N(R_{32})_2$, wherein each of $R_{31}$ and $R_{32}$, independently from each other and at each occurrence, is selected from the group consisting of hydrogen and $C_{1-4}$ alkyl; each of p is an integer in the range from 0 to 4 ;
- each of $R_3$, independently from each other and at each occurrence is selected from the group consisting of hydrogen, halo, $CF_3$, $C_{1-4}$ alkyl, cycloalkyl, $OR_{21}$, and $N(R_{21})_2$, and wherein each of $R_{21}$, independently from each other and at each occurrence, is hydrogen, $C_{1-4}$ alkyl such as methyl, ethyl, propyl, isopropyl, butyl, *tert*-butyl, isobutyl or $C_{3-6}$ cycloalkyl such as cyclopropyl, cyclobutyl, cyclopentyl, cyclohexyl; more preferably $R_3$ is independently selected from the group consisting of hydrogen, Cl, Br, F, OMe, $N(R_{21})_2$, and $C_{1-4}$ alkyl such as methyl, ethyl, propyl, isopropyl, butyl, tert-butyl, isobutyl and wherein each of $R_{21}$, independently from each other and at each occurrence, is hydrogen, $C_{1-4}$

alkyl such as methyl, ethyl, propyl, isopropyl, butyl, *tert*-butyl, isobutyl or $C_{3-6}$ cycloalkyl such as cyclopropyl, cyclobutyl, cyclopentyl, cyclohexyl.

- T is selected from $CH_2$, N-R, O or S, wherein R is selected from hydrogen, $C_{1-10}$ alkyl or cycloalkyl which are optionally substituted by a halogen atom, an aryl group or an aralkyl group; preferably T is selected from $CH_2$ or O;
- each of U is selected, independently and at each occurrence, from C-halo, C-R or N; and wherein R is selected from hydrogen, $OR_{11}$, $N(R_{11})_2$, a $C_{1-10}$ alkyl or a cycloalkyl which are optionally substituted by a halogen atom, an aryl group or an aralkyl group, wherein each of $R_{11}$, independently from each other and at each occurrence, is selected from the group consisting of hydrogen and $C_{1-4}$ alkyl; preferably U is selected, independently and at each occurrence from C-halo, C-R or N; and preferably R is hydrogen or $C_{1-4}$ alkyl, more preferably U is selected, independently and at each occurrence from C-R or N; and more preferably R is hydrogen;
- each of D is selected, independently and at each occurrence, from C, C-R or N, wherein R is selected from hydrogen, $C_{1-5}$ alkyl or cycloalkyl; preferably D is selected, independently and at each occurrence from C, C-R or N, and preferably R is hydrogen;
- m is an integer equal to 1 or 2;
- n is an integer equal to 0 or 1;
- each of $R_{a1}$, independently from each other and at each occurrence, is selected from the group consisting of hydrogen, $C_{1-5}$ alkyl, $C_{2-15}$ alkenyl and $C_{2-15}$ alkynyl, wherein said $C_{1-5}$ alkyl, $C_{2-15}$ alkenyl and $C_{2-15}$ alkynyl are optionally substituted with a halogen atom, an aryl group or an aralkyl group; each of n1 is an integer in the range from 0 to 5; preferably $R_{a1}$ is hydrogen or $C_{1-5}$ alkyl;
- each of $R_{a2}$, independently from each other and at each occurrence is selected from the group consisting of $C_{1-15}$ alkyl, $C_{2-15}$ alkenyl, $C_{2-15}$ alkynyl, cycloalkyl, heterocyclyl, aryl, heteroaryl, aralkyl, halo, $NO_2$, $CF_3$, CN, $OR_{11}$, $SR_{11}$, $N(R_{11})_2$, $OC(R_{11})_2O$, $OC(R_{11})_2C(R_{11})_2O$, $COOR_{11}$, $CO(R_{11})_2$, $CON(R_{11})_2$, and $SO_2N(R_{11})_2$, and each optional alkyl, alkenyl, alkynyl cycloalkyl, aryl, heterocyclyl, heteroaryl substituent is further optionally substituted with halo, $C_{1-6}$ alkyl, cycloalkyl, aryl, $N(R_{11})_2$, $CF_3$, CN, $COOR_{11}$, $CO(R_{11})_2$, $CON(R_{11})_2$, $SO_2N(R_{11})_2$ or $OR_{11}$ and wherein each of $R_{11}$ , independently from each other and at each occurrence, is selected from the group consisting of hydrogen, $C_{1-6}$ alkyl, cycloalkyl, heterocyclyl, aryl, heteroaryl, and aralkyl, wherein said alkyl, alkenyl, alkynyl, cycloalkyl, heterocyclyl, aryl, heteroaryl, and aralkyl substituents are optionally substituted with halo, $C_{1-6}$ alkyl, cycloalkyl, heterocyclyl, $OR_{31}$ or $N(R_{32})_2$, wherein each of $R_{31}$ and $R_{32}$, independently from each other and at each occurrence, is selected from the group consisting of hydrogen and $C_{1-4}$ alkyl; more preferably, each of $R_{a2}$ is independently selected from the group consisting of halo, $OR_{11}$, $C_{1-6}$ alkyl, cycloalkyl, heterocyclyl, aryl, heteroaryl, $CF_3$, CN, $SR_{11}$, $N(R_{11})_2$, $OC(R_{11})_2O$, $OC(R_{11})_2C(R_{11})_2O$, $COOR_{11}$, $CO(R_{11})_2$, and $CON(R_{11})_2$, and wherein each of $R_{11}$ , independently from each other and at each occurrence, is selected from the group consisting of hydrogen, $C_{1-4}$ alkyl, cycloalkyl and heterocyclyl; even more preferably, each of $R_{a2}$ is independently selected from the group consisting of halo, $OC_{1-4}$ alkyl, $C_{1-4}$ alkyl, cycloalkyl, heterocyclyl, aryl, heteroaryl, $CF_3$, CN, $OCH_2O$, OCH2CH2O, COOH, COO($C_{1-4}$ alkyl), CO(heterocyclyl), CO($C_{1-4}$ alkyl), CONH($C_{1-4}$ alkyl), and CONH(cycloalkyl); each of n2 is an integer in the range from 0 to 4; preferably each of n2 is an integer in the range from 0 to 2;
- each of B, independently from each other and at each occurrence is selected from C-R, O, N, S and $NR_{7'}$, wherein R is selected from hydrogen or an $C_{1-10}$ alkyl which is optionally substituted by a halogen atom, an aryl group or an aralkyl group, wherein $R_{7'}$ is selected from the group consisting of hydrogen, $C_{1-6}$ alkyl, $C_{1-6}$ alkenyl, cycloalkyl, heterocyclyl, aryl, aralkyl and $CF_3$; preferably each of B, independent from each other and at each occurrence, is each selected from C-R, O and $NR_{7'}$, wherein $R_{7'}$ is selected from hydrogen or $C_{1-4}$ alkyl and R is hydrogen or $C_{1-4}$ alkyl.
- each of E, independently from each other and at each occurrence is selected from C-R, O, N, S and $NR_{7'}$, wherein R is selected from hydrogen or an $C_{1-10}$ alkyl which is optionally substituted by a halogen atom, an aryl group or an aralkyl group, wherein $R_{7'}$ is selected from the group consisting of hydrogen, $C_{1-6}$ alkyl, $C_{1-6}$ alkenyl, cycloalkyl, heterocyclyl, aryl, aralkyl and $CF_3$; preferably each of E, independent from each other and at each occurrence, is selected from C-R, O and $NR_{7'}$, wherein $R_{7'}$ is selected from hydrogen or $C_{1-4}$ alkyl and R is hydrogen or $C_{1-4}$ alkyl.
- each of $R_{a4}$, independently from each other and at each occurrence is selected from the group consisting **of** $C_{1-15}$ alkyl, $C_{2-15}$ alkenyl, $C_{2-15}$ alkynyl, cycloalkyl, heterocyclyl, aryl, heteroaryl, aralkyl, halo, $NO_2$, $CF_3$, CN, $OR_{11}$, $SR_{11}$, $N(R_{11})_2$, $OC(R_{11})_2O$, $OC(R_{11})_2C(R_{11})_2O$, $COOR_{11}$, $CO(R_{11})_2$, and $CON(R_{11})_2$, and each optional alkyl, alkenyl, alkynyl, cycloalkyl, aryl, heterocyclyl, heteroaryl, aralkyl substituent is further optionally substituted with halo, alkyl, cycloalkyl, aryl, $N(R_{11})_2$, CN, or $OR_{11}$ and wherein each of $R_{11}$ , independently from each other and at each occurrence, is selected from the group consisting of hydrogen, $C_{1-6}$ alkyl, cycloalkyl, heterocyclyl, aryl, heteroaryl, and aralkyl, wherein said alkyl, alkenyl, alkynyl, cycloalkyl, heterocyclyl, aryl, heteroaryl, and aralkyl substituents are optionally substituted with halo, alkyl, cycloalkyl, heterocyclyl, $OR_{31}$ or $N(R_{32})_2$, wherein each of $R_{31}$ and $R_{32}$, independently from each other and at each occurrence, is selected from the group consisting of hydrogen and $C_{1-4}$ alkyl; more preferably, each of $R_{a4}$ is independently selected from the group consisting of halo, $OR_{11}$, $C_{1-6}$ alkyl, cycloalkyl, heterocyclyl, aryl, heteroaryl, $CF_3$, CN, $SR_{11}$, $N(R_{11})_2$, $OC(R_{11})_2O$, $OC(R_{11})_2C(R_{11})_2O$, $COOR_{11}$, $CO(R_{11})_2$, and $CON(R_{11})_2$, and wherein each of $R_{11}$ , independently from each other and at each occurrence, is

selected from the group consisting of hydrogen and $C_{1-4}$ alkyl; even more preferably, each of $R_{a4}$ is independently selected from the group consisting of halo, $OC_{1-4}$ alkyl, $C_{1-4}$ alkyl, cycloalkyl, heterocyclyl, aryl, heteroaryl, $CF_3$, CN, $OCH_2O$, and $OCH_2CH_2O$; each of n4 is an integer in the range from 0 to 4; preferably each of n4 is an integer in the range from 0 to 2;

- each of $R_{a3}$, independently from each other and at each occurrence is selected from the group consisting of $C_{1-15}$ alkyl, $C_{2-15}$ alkenyl, $C_{2-15}$ alkynyl, cycloalkyl, heterocyclyl, aryl, heteroaryl, aralkyl, halo, $CF_3$, $OR_{11}$, $SR_{11}$, $N(R_{11})_2$, $COOR_{11}$, $CO(R_{11})_2$, and $CON(R_{11})_2$, and each optional alkyl, alkenyl, alkynyl, cycloalkyl, aryl, heterocyclyl, heteroaryl, aralkyl substituent is further optionally substituted with halo, alkyl, cycloalkyl, aryl, $N(R_{11})_2$, CN, or $OR_{11}$ and wherein each of $R_{11}$ , independently from each other and at each occurrence, is selected from the group consisting of hydrogen, $C_{1-6}$ alkyl, cycloalkyl, heterocyclyl, aryl, heteroaryl, and aralkyl, wherein said alkyl, cycloalkyl, heterocyclyl, aryl, heteroaryl, and aralkyl substituents are optionally substituted with halo, alkyl, cycloalkyl, heterocyclyl, $OR_{31}$ or $N(R_{32})_2$, wherein each of $R_{31}$ and $R_{32}$, independently from each other and at each occurrence, is selected from the group consisting of hydrogen and $C_{1-4}$alkyl; more preferably, each of $R_{a3}$ is independently selected from the group consisting of $C_{1-6}$ alkyl, cycloalkyl, heterocyclyl, aryl, and heteroaryl; even more preferably, $R_{a3}$ is $C_{1-4}$ alkyl, each of n3 is an integer in the range from 0 to 2.

wherein said cycloalkyl is a monocyclic, bicyclic or tricyclic ring system of 3-7 ring members per ring; said heterocyclyl is a saturated, partially saturated or completely saturated monocycle, bicycle or tricycle containing 3 to 12 carbon atoms and 1 or 2 heteroatoms independently selected from O or N; said aryl is phenyl, naphthyl or anthracenyl optionally carbocyclic fused with a cycloalkyl or heterocyclyl of 5-7 ring members; said heteroaryl is a monocyclic ring structure containing 5 or 6 ring atoms, or a bicyclic aromatic group having 8 to 10 atoms, containing 1-3 heteroatoms independently selected from O or N.

Preferred compounds of class (I) are selected from those of formula (XVIIIa-1) to (XXVa-1) herein below:

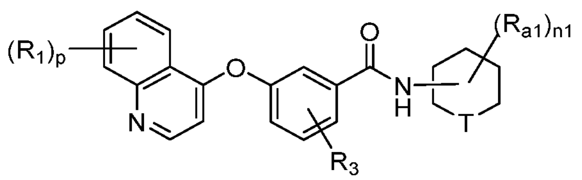

Formula (XVIIIa-1)

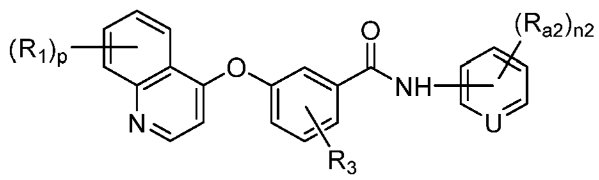

Formula (XIXa-1)

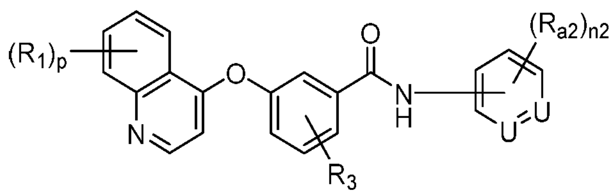

Formula (XXa-1)

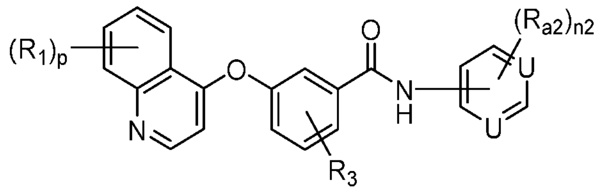

Formula (XXIa-1)

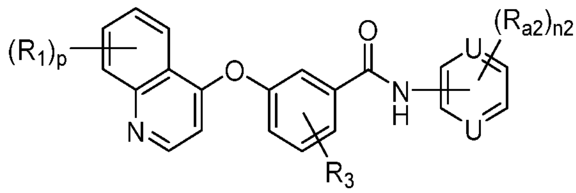

Formula (XXIIa-1)

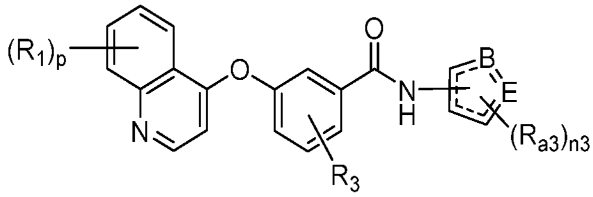

Formula (XXIIIa-1)

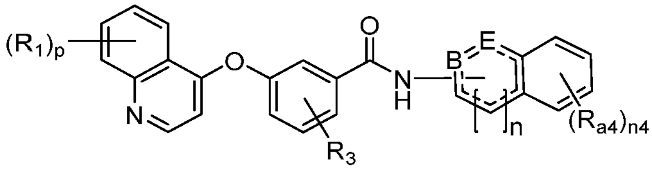

Formula (XXIVa-1)

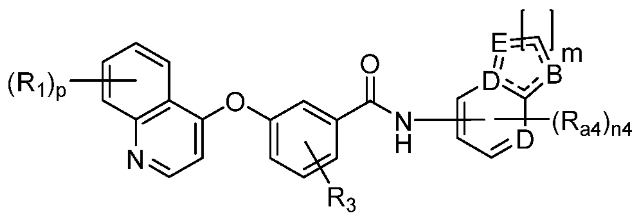

Formula (XXVa-1)

wherein, $R_1$, $R_{a1}$, $R_{a2}$, $R_{a3}$, $R_{a4}$, T, U, B, E, D, p, n1, n2, n3, n4, m, n have the same meaning as defined above for formula (IIa) to (IXa) and the dash bond represents an optional double bond and wherein

- each of $R_3$, independently from each other and at each occurrence is selected from the group consisting of hydrogen, $CF_3$, halo, $C_{1-4}$ alkyl, cycloalkyl, $OR_{21}$, $N(R_{21})_2$, and wherein each of $R_{21}$, independently from each other and at each occurrence, is hydrogen, $C_{1-4}$ alkyl such as methyl, ethyl, propyl, isopropyl, butyl, *tert*-butyl, isobutyl or $C_{3-6}$ cycloalkyl such as cyclopropyl, cyclobutyl, cyclopentyl, cyclohexyl; more preferably $R_3$ is independently selected from the group consisting of hydrogen, Cl, Br, F, OMe, $N(R_{21})_2$, and $C_{1-4}$ alkyl such as methyl, ethyl, propyl, isopropyl, butyl, *tert*-butyl, isobutyl and wherein each of $R_{21}$, independently from each other and at each occurrence, is hydrogen, $C_{1-4}$ alkyl such as methyl, ethyl, propyl, isopropyl, butyl, *tert*-butyl, isobutyl or $C_{3-6}$ cycloalkyl such as cyclopropyl, cyclobutyl, cyclopentyl, cyclohexyl.

In a preferred embodiment of the present invention, the compound (C) for use as a kinase inhibitor, or the N-oxide, pharmaceutically acceptable salt, pharmaceutically acceptable solvate, or stereoisomer thereof, is a compound of formulae (XVIIIa-1) to (XXVa-1) wherein:

- each of $R_1$, independently from each other and at each occurrence, is selected from the group consisting of hydrogen, halo, $C_{1-6}$ alkyl, $C_{2-5}$ alkenyl, $C_{2-5}$ alkynyl, cycloalkyl, heterocyclyl, aryl, and heteroaryl, $CF_3$, CN, $NO_2$, $OR_{21}$, $SR_{21}$, $N(R_{21})_2$, $COR_{21}$, $C(O)OR_{21}$, $CON(R_{21})_2$, $OC(O)R_{21}$, $OCON(R_{21})_2$, $NC(O)R_{21}$, $NCON(R_{21})_2$, $OC(R_{21})_2O$ and $OC(R_{21})_2C(R_{22})_2O$, wherein said alkyl, alkenyl, alkynyl, cycloalkyl, heterocyclyl, aryl, and heteroaryl are optionally substituted with one or more substituents selected from halo, $C_1$-6 alkyl, cycloalkyl, heterocyclyl, $CF_3$, $COR_{21}$, $N(R_{21})_2$, CN, $CONR_{21}$, $C(O)OR_{21}$ or $OR_{21}$, and each optional alkyl substituent is further optionally substituted with heterocyclyl, $N(R_{11})_2$, or $OR_{11}$ ; and wherein each of $R_{21}$ and $R_{22}$, independently from each other and at each occurrence, is selected from the group consisting of hydrogen, $C_{1-6}$ alkyl, $C_{2-4}$ alkenyl, $C_{2-4}$ alkynyl, cycloalkyl, heterocyclyl, phenyl, heteroaryl, and aralkyl and wherein said alkyl, alkenyl, alkynyl, cycloalkyl, heterocyclyl, phenyl, heteroaryl, and aralkyl substituents are optionally substituted with halo, $C_{1-6}$ alkyl, cycloalkyl, heterocyclyl, phenyl, $OR_{31}$ or $N(R_{32})_2$, wherein each of $R_{31}$ and $R_{32}$, independently from each other and at each occurrence, is selected

from the group consisting of hydrogen and $C_{1-4}$ alkyl; each of p is an integer in the range from 0 to 4 ;

- each of $R_3$, independently from each other and at each occurrence is selected from the group consisting of hydrogen, halo, $CF_3$, $C_{1-4}$ alkyl, cycloalkyl, $OR_{21}$, and $N(R_{21})_2$, and wherein each of $R_{21}$, independently from each other and at each occurrence, is hydrogen, $C_{1-4}$ alkyl such as methyl, ethyl, propyl, isopropyl, butyl, *tert*-butyl, isobutyl or $C_{3-6}$ cycloalkyl such as cyclopropyl, cyclobutyl, cyclopentyl, cyclohexyl; more preferably $R_3$ is independently selected from the group consisting of hydrogen, Cl, Br, F, OMe, $N(R_{21})_2$, and $C_{1-4}$ alkyl such as methyl, ethyl, propyl, isopropyl, butyl, *tert*-butyl, isobutyl and wherein each of $R_{21}$, independently from each other and at each occurrence, is hydrogen, $C_{1-4}$ alkyl such as methyl, ethyl, propyl, isopropyl, butyl, *tert*-butyl, isobutyl or $C_{3-6}$ cycloalkyl such as cyclopropyl, cyclobutyl, cyclopentyl, cyclohexyl.
- T is selected from $CH_2$, N-R, O or S, wherein R is selected from hydrogen, $C_{1-10}$ alkyl or cycloalkyl which are optionally substituted by a halogen atom, an aryl group or an aralkyl group; preferably T is selected from $CH_2$ or O;
- each of U is selected, independently and at each occurrence, from C-halo, C-R or N; and wherein R is selected from hydrogen, $OR_{11}$, $N(R_{11})_2$, a $C_{1-10}$ alkyl or a cycloalkyl which are optionally substituted by a halogen atom, an aryl group or an aralkyl group, wherein each of $R_{11}$, independently from each other and at each occurrence, is selected from the group consisting of hydrogen and $C_{1-4}$ alkyl; preferably U is selected, independently and at each occurrence from C-halo, C-R or N; and preferably R is hydrogen or $C_{1-4}$ alkyl, more preferably U is selected, independently and at each occurrence from C-R or N; and more preferably R is hydrogen;
- each of D is selected, independently and at each occurrence, from C, C-R or N, wherein R is selected from hydrogen, $C_{1-5}$ alkyl or cycloalkyl; preferably D is selected, independently and at each occurrence from C, C-R or N, and preferably R is hydrogen;
- m is an integer equal to 1 or 2;
- n is an integer equal to 0 or 1;
- each of $R_{a1}$, independently from each other and at each occurrence, is selected from the group consisting of hydrogen, $C_{1-5}$ alkyl, $C_{2-15}$ alkenyl and $C_{2-15}$ alkynyl, wherein said $C_{1-5}$ alkyl, $C_{2-15}$ alkenyl and $C_{2-15}$ alkynyl are optionally substituted with a halogen atom, an aryl group or an aralkyl group; each of n1 is an integer in the range from 0 to 5; preferably $R_{a1}$ is hydrogen or $C_{1-5}$ alkyl;
- each of $R_{a2}$, independently from each other and at each occurrence is selected from the group consisting of $C_{1-15}$ alkyl, $C_{2-15}$ alkenyl, $C_{2-15}$ alkynyl, cycloalkyl, heterocyclyl, aryl, heteroaryl, aralkyl, halo, $NO_2$, $CF_3$, CN, $OR_{11}$, $SR_{11}$, $N(R_{11})_2$, $OC(R_{11})_2O$, $OC(R_{11})_2C(R_{11})_2O$, $COOR_{11}$, $CO(R_{11})_2$, $CON(R_{11})_2$, and $SO_2N(R_{11})_2$, and each optional alkyl, alkenyl, alkynyl cycloalkyl, aryl, heterocyclyl, heteroaryl substituent is further optionally substituted with halo, $C_{1-6}$ alkyl, cycloalkyl, aryl, $N(R_{11})_2$, $CF_3$, CN, $COOR_{11}$, $CO(R_{11})_2$, $CON(R_{11})_2$, $SO_2N(R_{11})_2$ or $OR_{11}$ and wherein each of $R_{11}$ , independently from each other and at each occurrence, is selected from the group consisting of hydrogen, $C_{1-6}$ alkyl, cycloalkyl, heterocyclyl, aryl, heteroaryl, and aralkyl, wherein said alkyl, alkenyl, alkynyl, cycloalkyl, heterocyclyl, aryl, heteroaryl, and aralkyl substituents are optionally substituted with halo, $C_{1-6}$ alkyl, cycloalkyl, heterocyclyl, $OR_{31}$ or $N(R_{32})_2$, wherein each of $R_{31}$ and $R_{32}$, independently from each other and at each occurrence, is selected from the group consisting of hydrogen and $C_{1-4}$ alkyl; more preferably, each of $R_{a2}$ is independently selected from the group consisting of halo, $OR_{11}$, $C_{1-6}$ alkyl, cycloalkyl, heterocyclyl, aryl, heteroaryl, $CF_3$, CN, $SR_{11}$, $N(R_{11})_2$, $OC(R_{11})_2O$, $OC(R_{11})_2C(R_{11})_2O$, $COOR_{11}$, $CO(R_{11})_2$, and $CON(R_{11})_2$, and wherein each of $R_{11}$ , independently from each other and at each occurrence, is selected from the group consisting of hydrogen, $C_{1-4}$ alkyl, cycloalkyl and heterocyclyl; even more preferably, each of $R_{a2}$ is independently selected from the group consisting of halo, $OC_{1-4}$ alkyl, $C_{1-4}$ alkyl, cycloalkyl, heterocyclyl, aryl, heteroaryl, $CF_3$, CN, $OCH_2O$, OCH2CH2O, COOH, COO($C_{1-4}$ alkyl), CO(heterocyclyl), CO($C_{1-4}$ alkyl), CONH($C_{1-4}$ alkyl), and CONH(cycloalkyl); each of n2 is an integer in the range from 0 to 4; preferably each of n2 is an integer in the range from 0 to 2;
- each of B, independently from each other and at each occurrence is selected from C-R, O, N, S and $NR_{7'}$, wherein R is selected from hydrogen or an $C_{1-10}$ alkyl which is optionally substituted by a halogen atom, an aryl group or an aralkyl group, wherein $R_{7'}$ is selected from the group consisting of hydrogen, $C_{1-6}$ alkyl, $C_{1-6}$ alkenyl, cycloalkyl, heterocyclyl, aryl, aralkyl and $CF_3$; preferably each of B, independent from each other and at each occurrence, is each selected from C-R, O and $NR_{7'}$, wherein $R_{7'}$ is selected from hydrogen or $C_{1-4}$ alkyl and R is hydrogen or $C_{1-4}$ alkyl.
- each of E, independently from each other and at each occurrence is selected from C-R, O, N, S and $NR_{7'}$, wherein R is selected from hydrogen or an $C_{1-10}$ alkyl which is optionally substituted by a halogen atom, an aryl group or an aralkyl group, wherein $R_{7'}$ is selected from the group consisting of hydrogen, $C_{1-6}$ alkyl, $C_{1-6}$ alkenyl, cycloalkyl, heterocyclyl, aryl, aralkyl and $CF_3$; preferably each of E, independent from each other and at each occurrence, is selected from C-R, O and $NR_{7'}$, wherein $R_{7'}$ is selected from hydrogen or $C_{1-4}$ alkyl and R is hydrogen or $C_{1-4}$ alkyl.
- each of $R_{a4}$, independently from each other and at each occurrence is selected from the group consisting of $C_{1-15}$ alkyl, $C_{2-15}$ alkenyl, $C_{2-15}$ alkynyl, cycloalkyl, heterocyclyl, aryl, heteroaryl, aralkyl, halo, $NO_2$, $CF_3$, CN, $OR_{11}$, $SR_{11}$, $N(R_{11})_2$, $OC(R_{11})_2O$, $OC(R_{11})_2C(R_{11})_2O$, $COOR_{11}$, $CO(R_{11})_2$, and $CON(R_{11})_2$, and each optional alkyl, alkenyl, alkynyl, cycloalkyl, aryl, heterocyclyl, heteroaryl, aralkyl substituent is further optionally substituted with halo, alkyl, cycloalkyl, aryl, $N(R_{11})_2$, CN, or $OR_{11}$ and wherein each of $R_{11}$ , independently from each other and at each

occurrence, is selected from the group consisting of hydrogen, $C_{1-6}$ alkyl, cycloalkyl, heterocyclyl, aryl, heteroaryl, and aralkyl, wherein said alkyl, alkenyl, alkynyl, cycloalkyl, heterocyclyl, aryl, heteroaryl, and aralkyl substituents are optionally substituted with halo, alkyl, cycloalkyl, heterocyclyl, $OR_{31}$ or $N(R_{32})_2$, wherein each of $R_{31}$ and $R_{32}$, independently from each other and at each occurrence, is selected from the group consisting of hydrogen and $C_{1-4}$ alkyl; more preferably, each of $R_{a4}$ is independently selected from the group consisting of halo, $OR_{11}$, $C_{1-6}$ alkyl, cycloalkyl, heterocyclyl, aryl, heteroaryl, $CF_3$, CN, $SR_{11}$, $N(R_{11})_2$, $OC(R_{11})_2O$, $OC(R_{11})_2C(R_{11})_2O$, $COOR_{11}$, $CO(R_{11})_2$, and $CON(R_{11})_2$, and wherein each of $R_{11}$ , independently from each other and at each occurrence, is selected from the group consisting of hydrogen and $C_{1-4}$ alkyl; even more preferably, each of $R_{a4}$ is independently selected from the group consisting of halo, $OC_{1-4}$ alkyl, $C_{1-4}$ alkyl, cycloalkyl, heterocyclyl, aryl, heteroaryl, $CF_3$, CN, $OCH_2O$, and $OCH_2CH_2O$; each of n4 is an integer in the range from 0 to 4; preferably each of n4 is an integer in the range from 0 to 2;

- each of $R_{a3}$, independently from each other and at each occurrence is selected from the group consisting of $C_{1-15}$ alkyl, $C_{2-15}$ alkenyl, $C_{2-15}$ alkynyl, cycloalkyl, heterocyclyl, aryl, heteroaryl, aralkyl, halo, $CF_3$, $OR_{11}$, $SR_{11}$, $N(R_{11})_2$, $COOR_{11}$, $CO(R_{11})_2$, and $CON(R_{11})_2$, and each optional alkyl, alkenyl, alkynyl, cycloalkyl, aryl, heterocyclyl, heteroaryl, aralkyl substituent is further optionally substituted with halo, alkyl, cycloalkyl, aryl, $N(R_{11})_2$, CN, or $OR_{11}$ and wherein each of $R_{11}$ , independently from each other and at each occurrence, is selected from the group consisting of hydrogen, $C_{1-6}$ alkyl, cycloalkyl, heterocyclyl, aryl, heteroaryl, and aralkyl, wherein said alkyl, cycloalkyl, heterocyclyl, aryl, heteroaryl, and aralkyl substituents are optionally substituted with halo, alkyl, cycloalkyl, heterocyclyl, $OR_{31}$ or $N(R_{32})_2$, wherein each of $R_{31}$ and $R_{32}$, independently from each other and at each occurrence, is selected from the group consisting of hydrogen and $C_{1-4}$alkyl; more preferably, each of $R_{a3}$ is independently selected from the group consisting of $C_{1-6}$ alkyl, cycloalkyl, heterocyclyl, aryl, and heteroaryl; even more preferably, $R_{a3}$ is $C_{1-4}$ alkyl, each of n3 is an integer in the range from 0 to 2.

wherein said cycloalkyl is a monocyclic, bicyclic or tricyclic ring system of 3-7 ring members per ring; said heterocyclyl is a saturated, partially saturated or completely saturated monocycle, bicycle or tricycle containing 3 to 12 carbon atoms and 1 or 2 heteroatoms independently selected from O or N; said aryl is phenyl, naphthyl or anthracenyl optionally carbocyclic fused with a cycloalkyl or heterocyclyl of 5-7 ring members; said heteroaryl is a monocyclic ring structure containing 5 or 6 ring atoms, or a bicyclic aromatic group having 8 to 10 atoms, containing 1-3 heteroatoms independently selected from O or N.

Preferred compounds of class (I) are selected from those of formula (XXVIa-1) to (XXXIIIa-1) herein below:

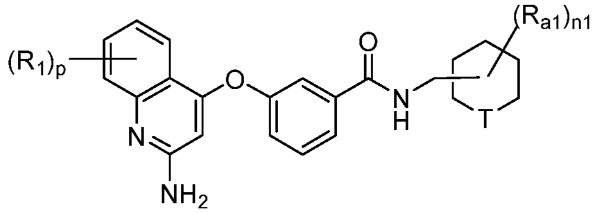

Formula (XXVIa-1)

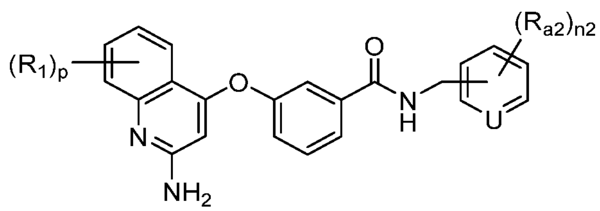

Formula (XXVIIa-1)

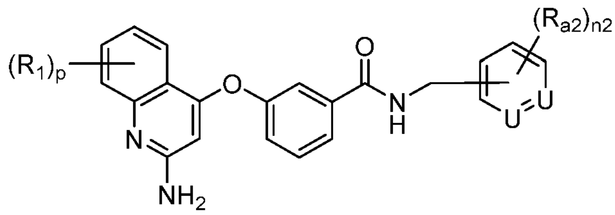

Formula (XXVIIIa-1)

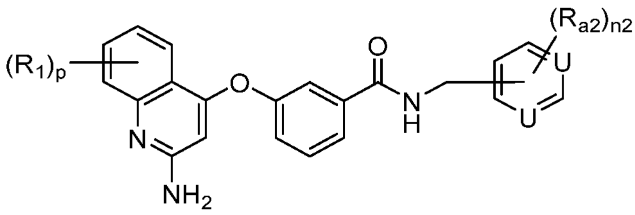

Formula (XXIXa-1)

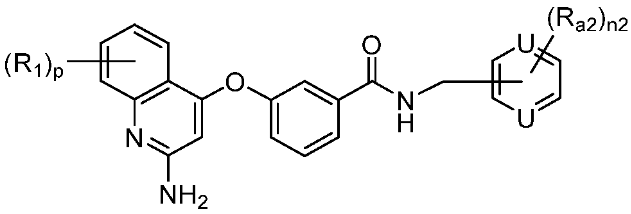

Formula (XXXa-1)

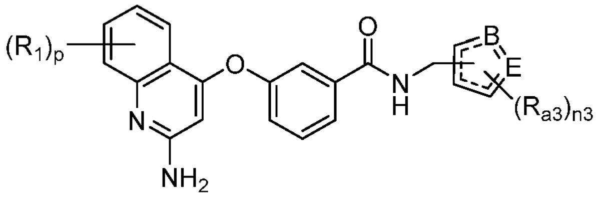

Formula (XXXIa-1)

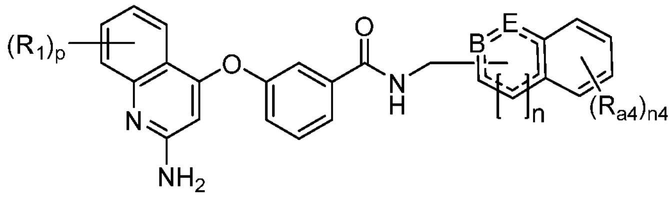

Formula (XXXIIa-1)

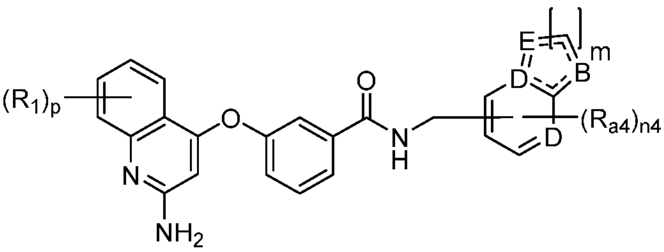

Formula (XXXIIIa-1)

wherein, $R_1$, $R_{a1}$, $R_{a2}$, $R_{a3}$, $R_{a4}$, T, U, B, E, D, p, n1, n2, n3, n4, m, n have the same meaning as defined above for formula (IIa) to (IXa) and the dash bond represents an optional double bond.

In a preferred embodiment of the present invention, the compound (C), according to the invention, for use as a kinase inhibitor according to general formula (IIa-1) is a compound chosen among those of formulae (XXXIV) to (XLII) herein below:

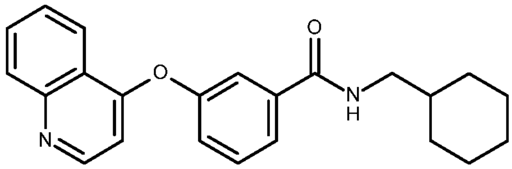

Formula (XXXIV)

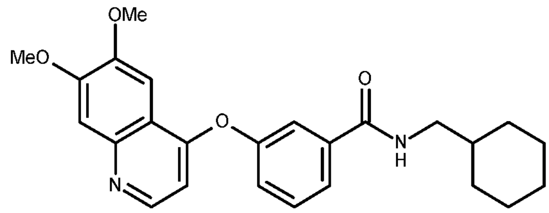

Formula (XXXV)

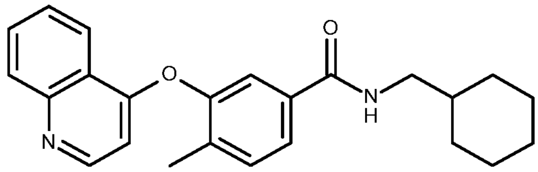

Formula (XXXVI)

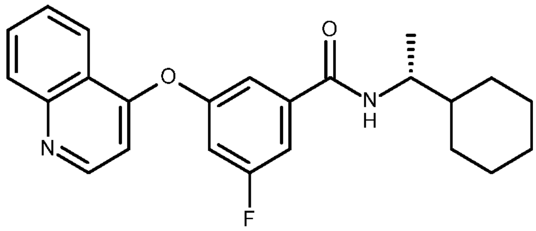

Formula (XXXVII)

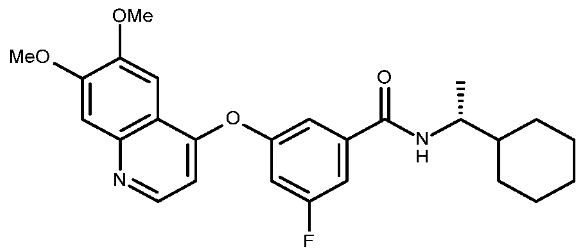

Formula (XXXVIII)

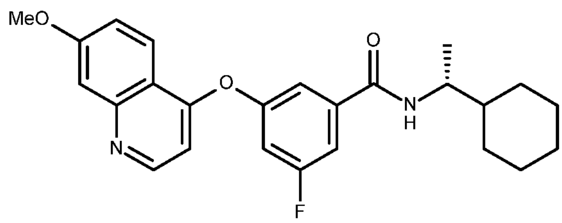

Formula (XXXIX)

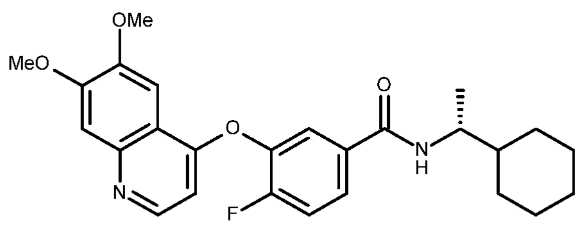

Formula (XL)

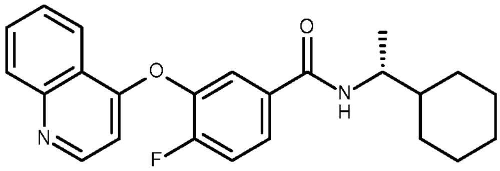

Formula (XLI)

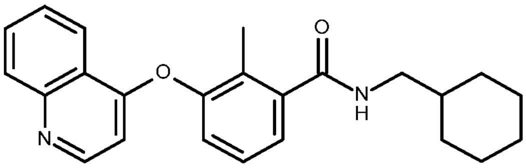

Formula (XLII)

In a preferred embodiment of the present invention, the compound (C), according to the invention, for use as a kinase inhibitor according to general formula (IIIa-1) is a compound chosen among those of formulae (XLIII) to (CXCVIII-39) herein below:

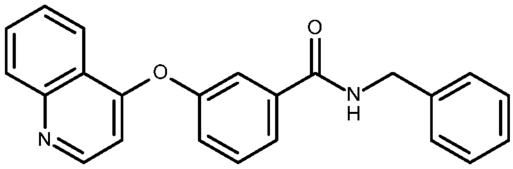

Formula (XLIII)

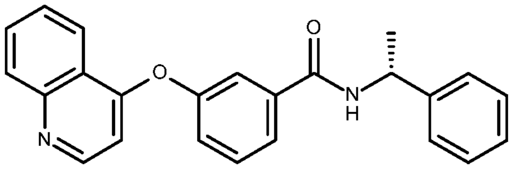

Formula (XLIV)

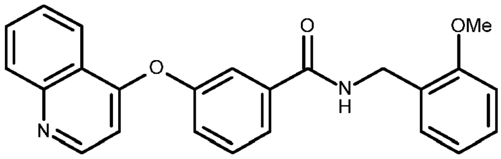

Formula (XLV)

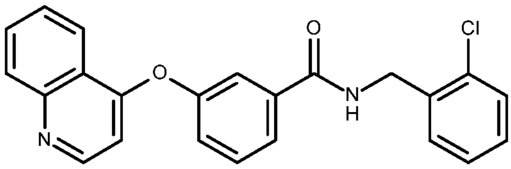

Formula (XLVI)

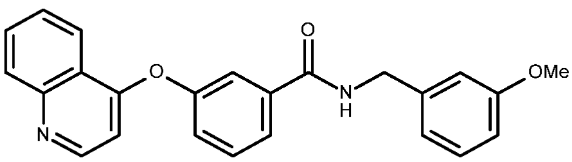

Formula (XLVII)

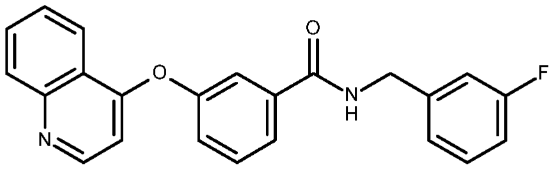

Formula (XLVIII)

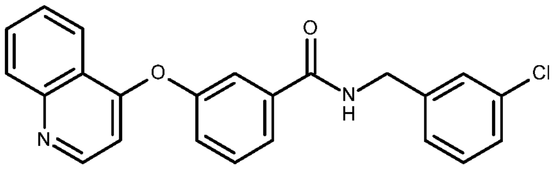

Formula (XLIX)

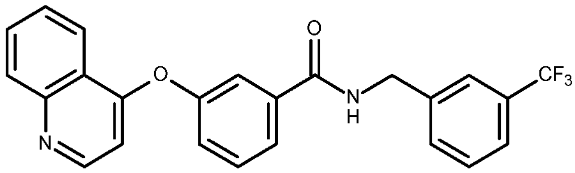

Formula (L)

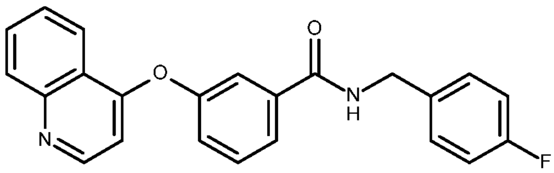

Formula (LI)

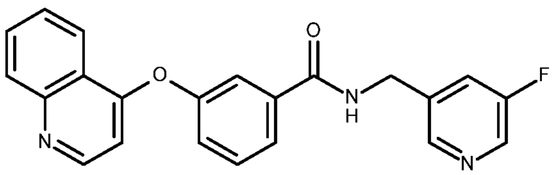

Formula (LII)

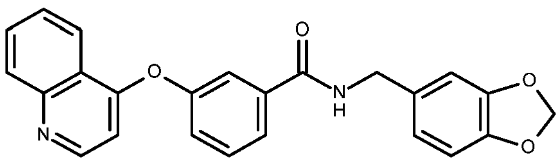

Formula (LIII)

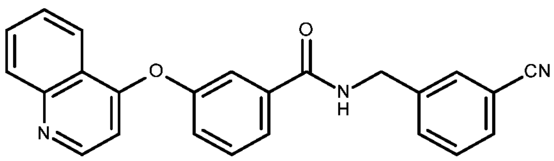

Formula (LIV)

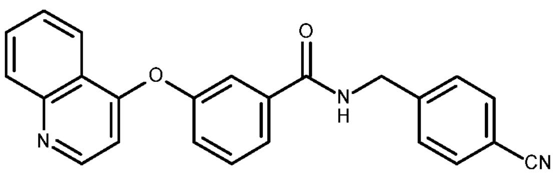

Formula (LV)

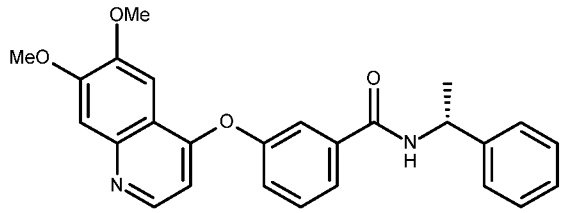

Formula (LVI)

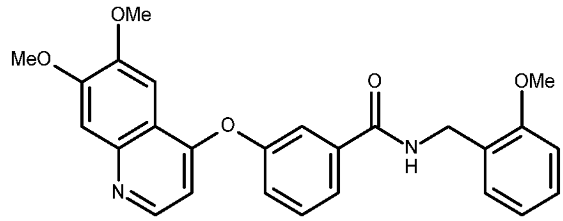

Formula (LVII)

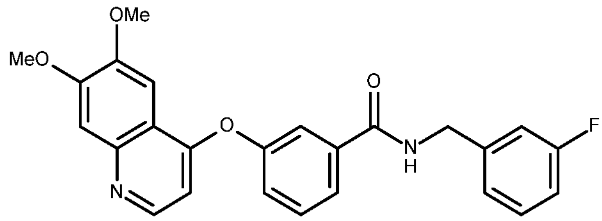

Formula (LVIII)

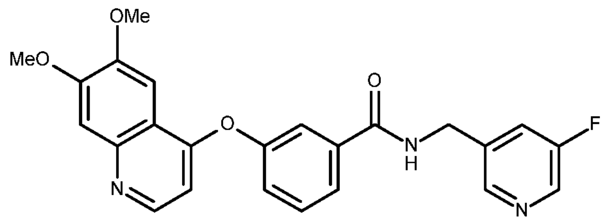

Formula (LIX)

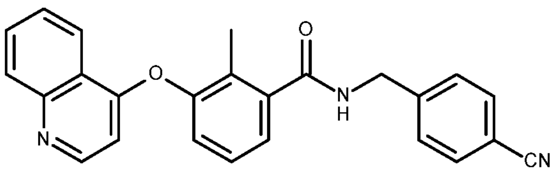

Formula (LX)

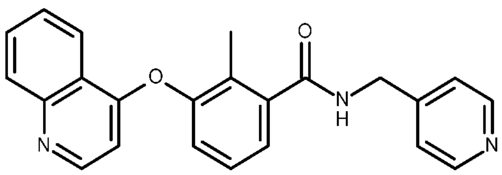

Formula (LXI)

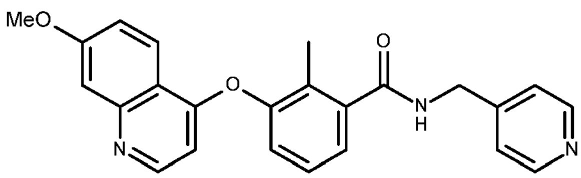

Formula (LXII)

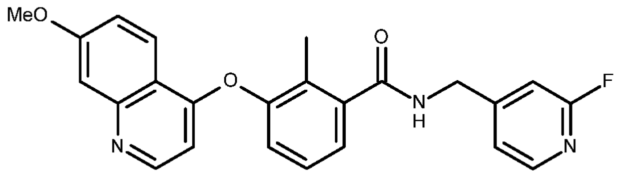

Formula (LXIII)

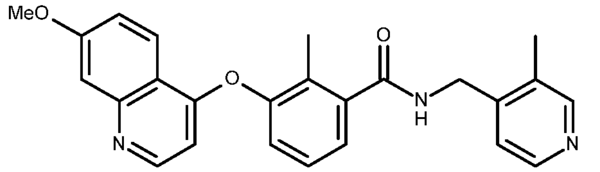

Formula (LXIV)

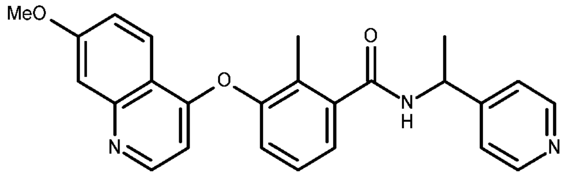

Formula (LXV)

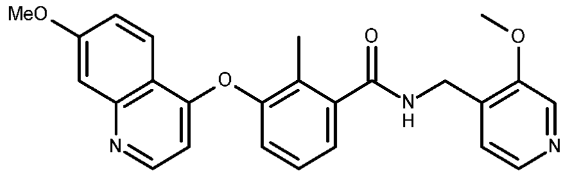

Formula (LXVI)

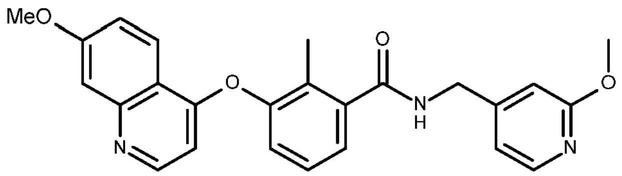

Formula (LXVII)

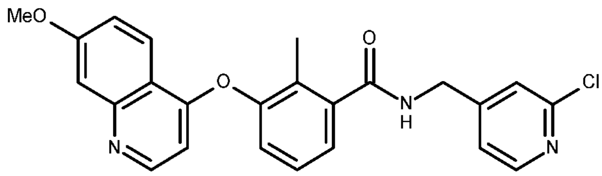

Formula (LXVIII)

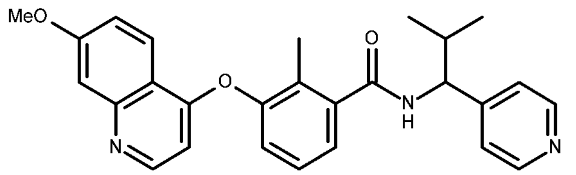

Formula (LXIX)

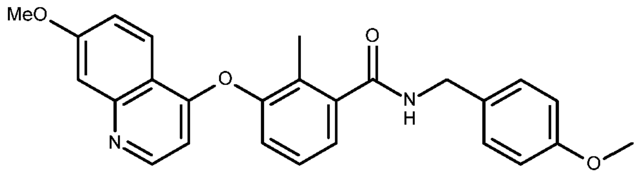

Formula (LXX)

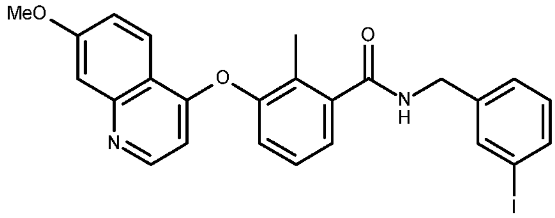

Formula (LXXI)

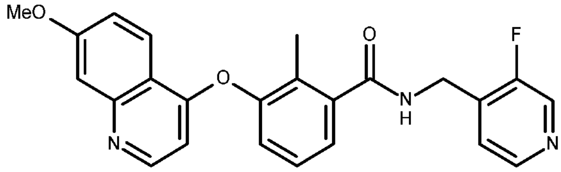

Formula (LXXII)

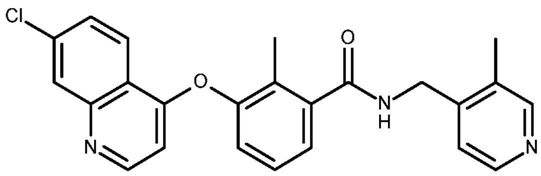

Formula (LXXIII)

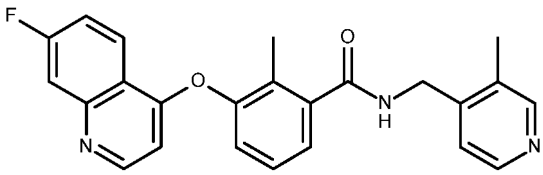

Formula (LXXIV)

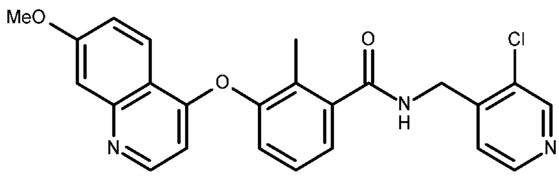

Formula (LXXV)

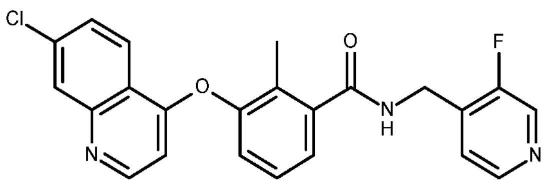

Formula (LXXVI)

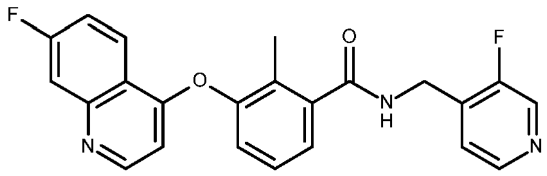

Formula (LXXVII)

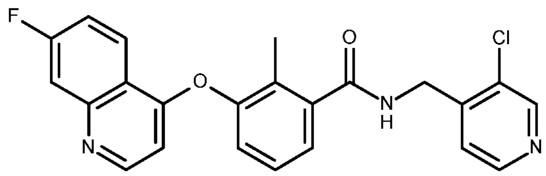

Formula (LXXVIII)

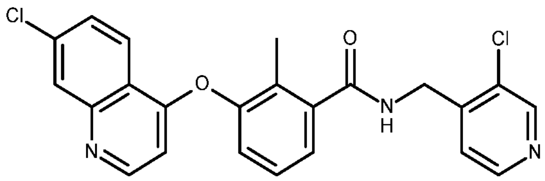

Formula (LXXIX)

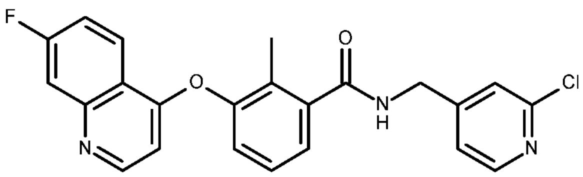

Formula (LXXX)

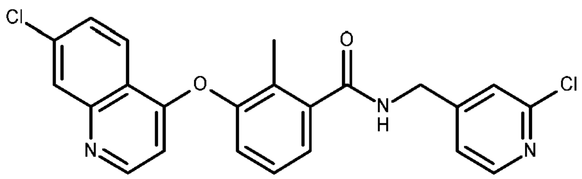

Formula (LXXXI)

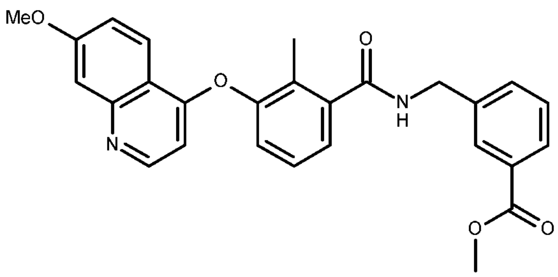

Formula (LXXXII)

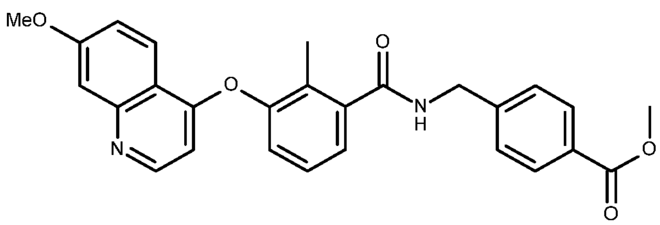

Formula (LXXXIII)

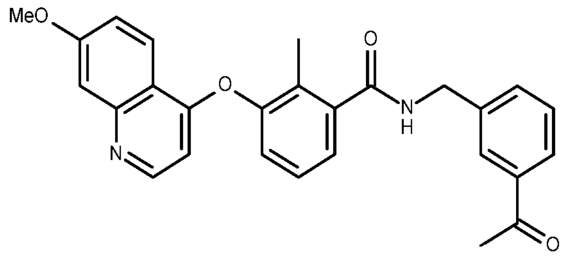

Formula (LXXXIV)

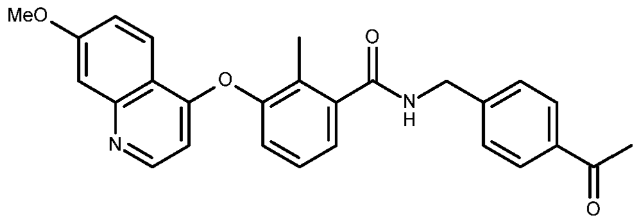

Formula (LXXXV)

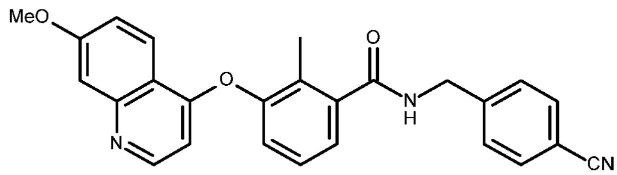

Formula (LXXXVI)

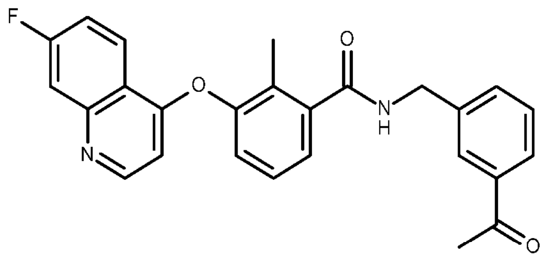

Formula (LXXXVII)

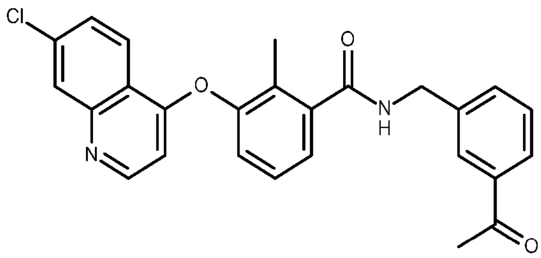

Formula (LXXXVIII)

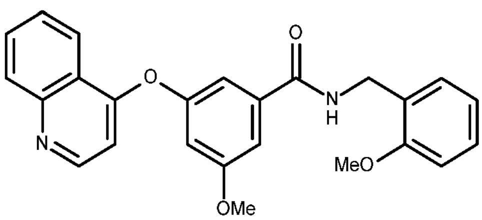

Formula (LXXXIX)

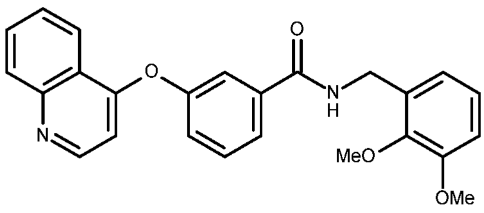

Formula (XC)

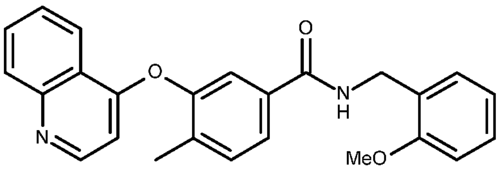

Formula (XCI)

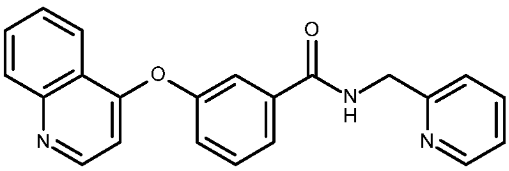

Formula (XCII)

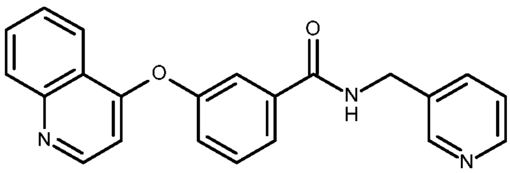

Formula (XCIII)

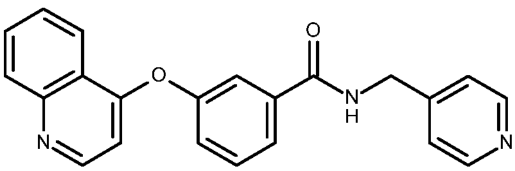

Formula (XCIV)

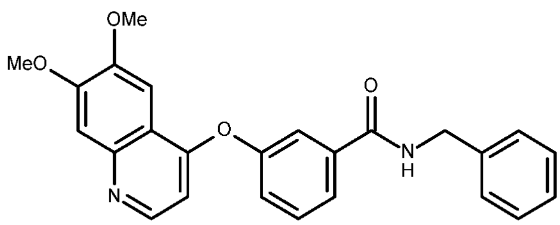

Formula (XCV)

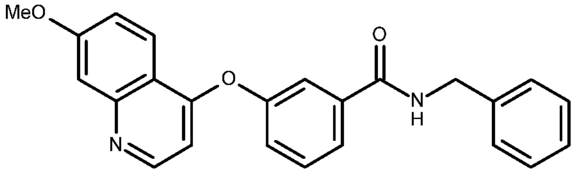

Formula (XCVI)

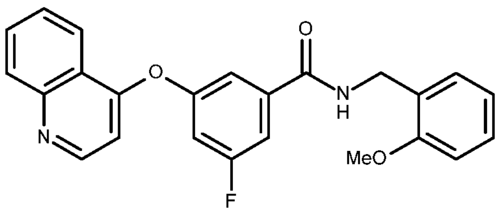

Formula (XCVII)

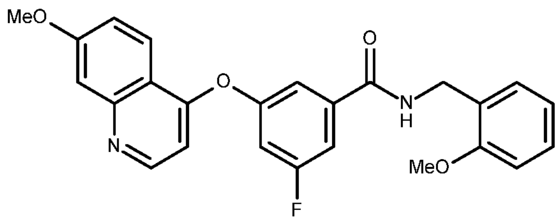

Formula (XCVIII)

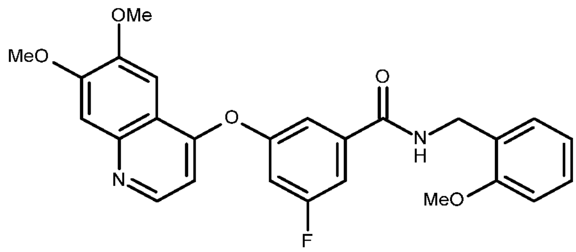

Formula (XCIX)

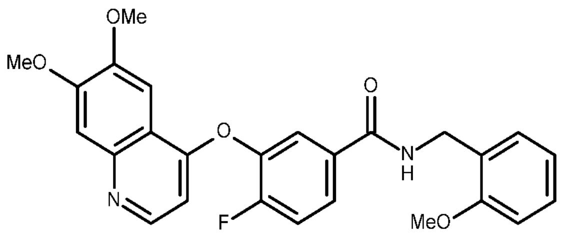

Formula (C)

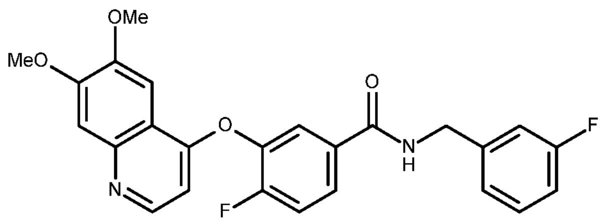

Formula (CI)

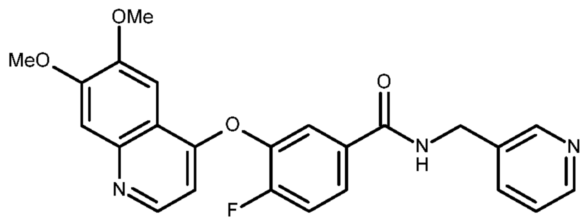

Formula (CII)

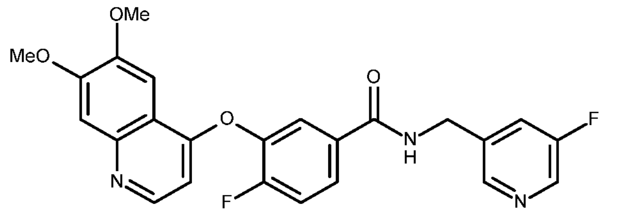

Formula (CIII)

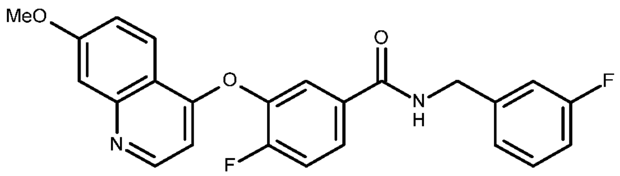

Formula (CIV)

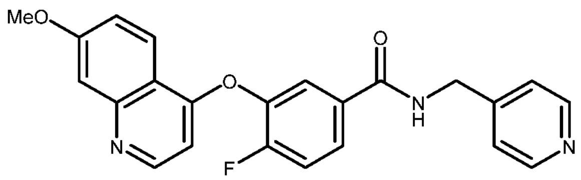

Formula (CV)

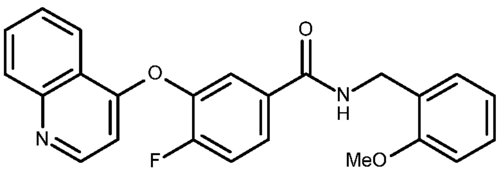

Formula (CVI)

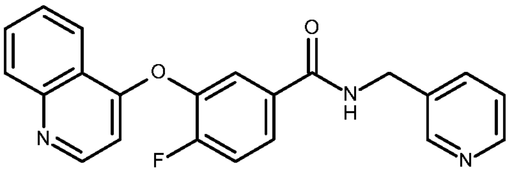

Formula (CVII)

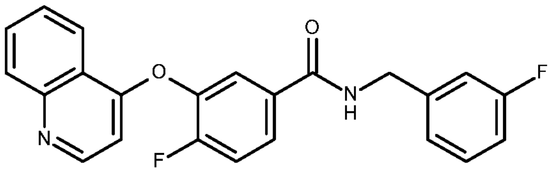

Formula (CVIII)

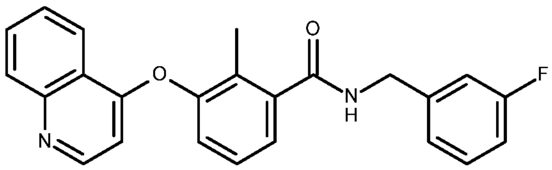

Formula (CIX)

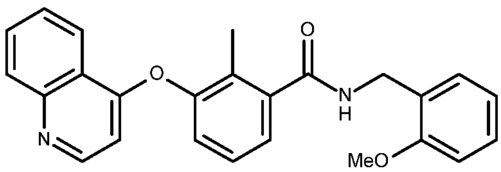

Formula (CX)

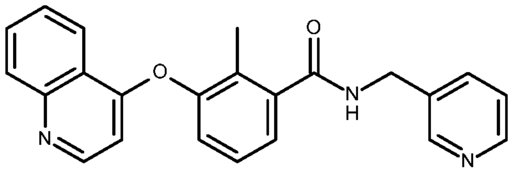

Formula (CXI)

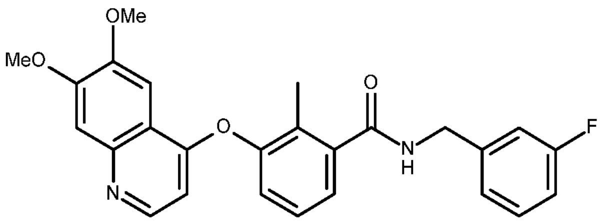

Formula (CXII)

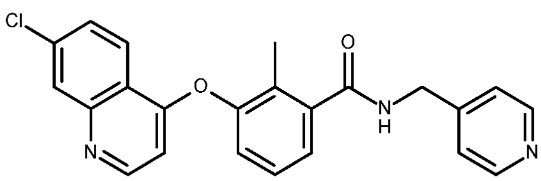

Formula (CXIII)

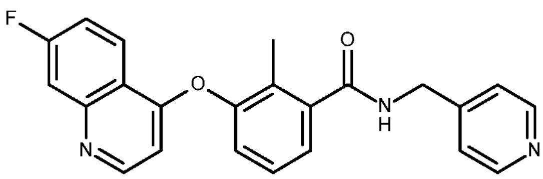

Formula (CXIV)

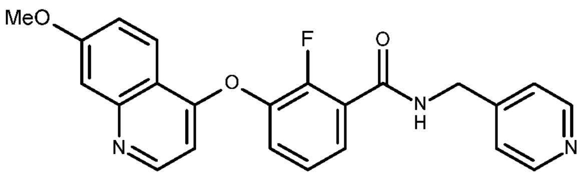

Formula (CXV)

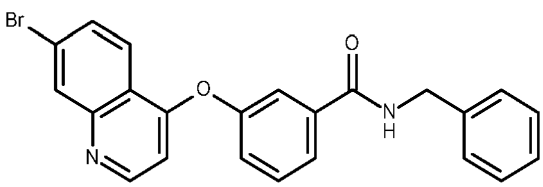

Formula (CXVI)

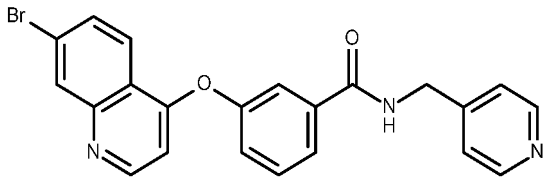

Formula (CXVII)

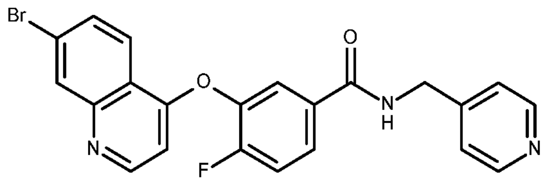

Formula (CXVIII)

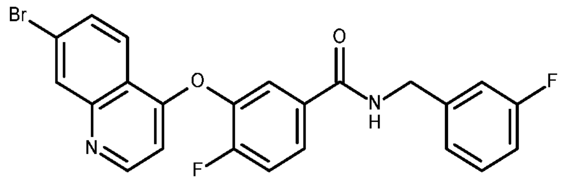

Formula (CXIX)

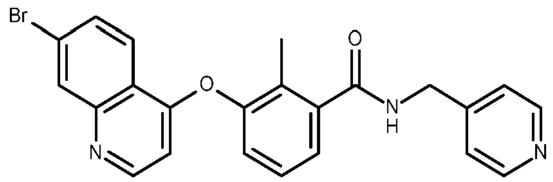

Formula (CXX)

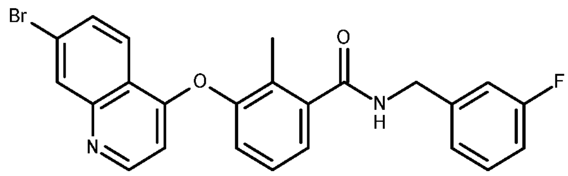

Formula (CXXI)

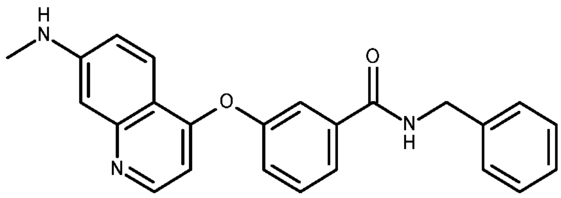

Formula (CXXII)

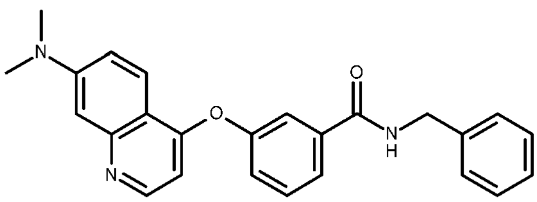

Formula (CXXIII)

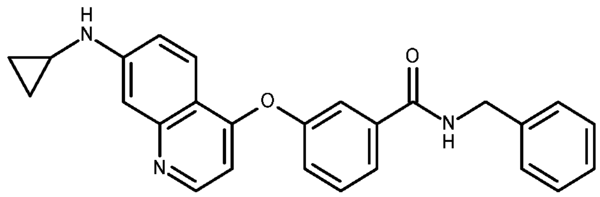

Formula (CXXIV)

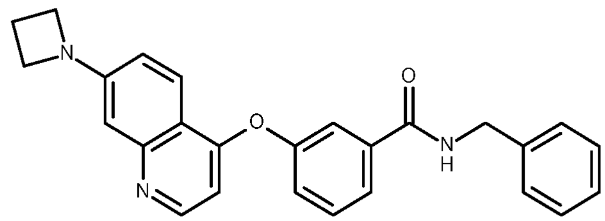

Formula (CXXV)

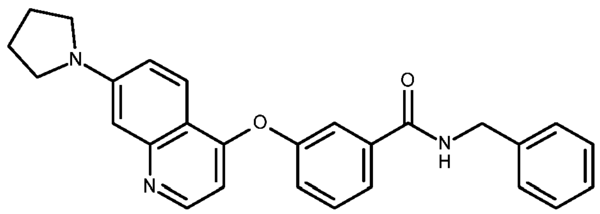

Formula (CXXVI)

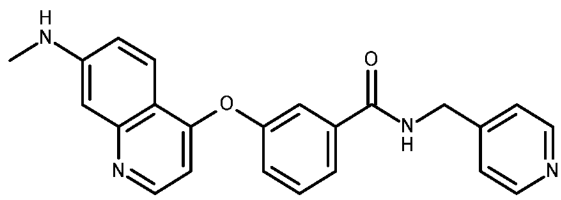

Formula (CXXVII)

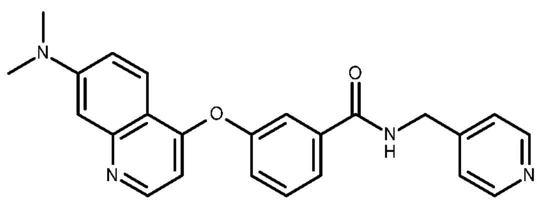

Formula (CXXVIII)

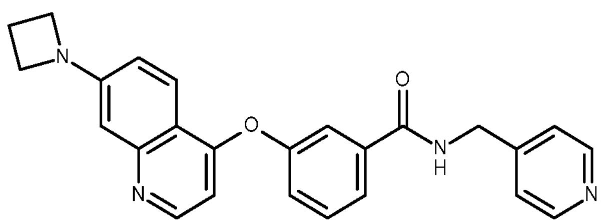

Formula (CXXIX)

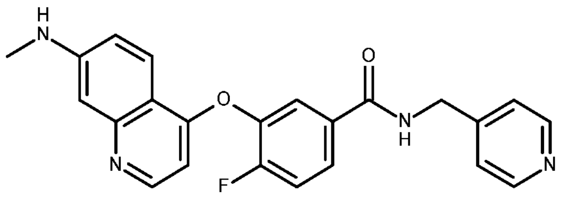

Formula (CXXX)

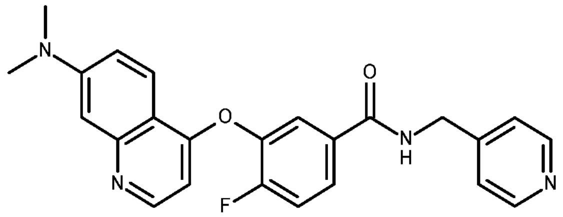

Formula (CXXXI)

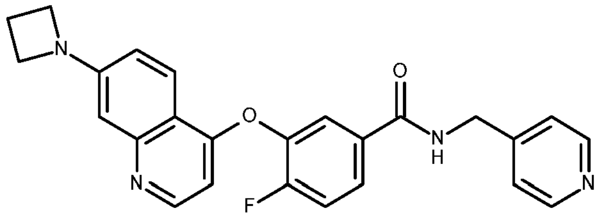

Formula (CXXXII)

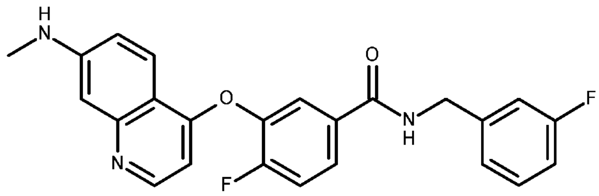

Formula (CXXXIII)

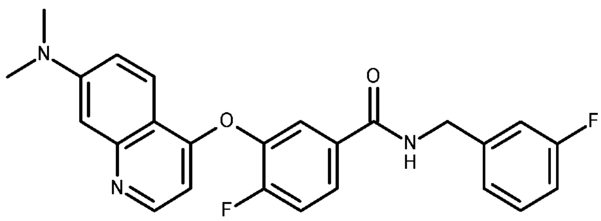

Formula (CXXXIV)

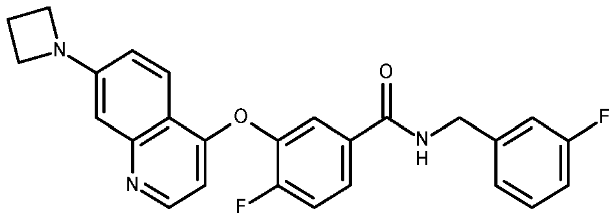

Formula (CXXXV)

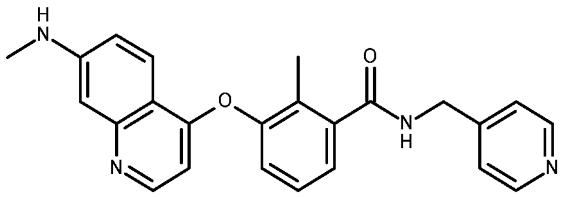

Formula (CXXXVI)

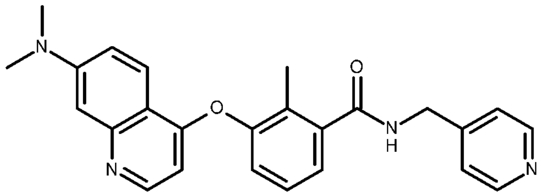

Formula (CXXXVII)

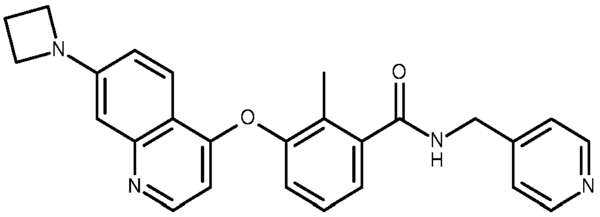

Formula (CXXXVIII)

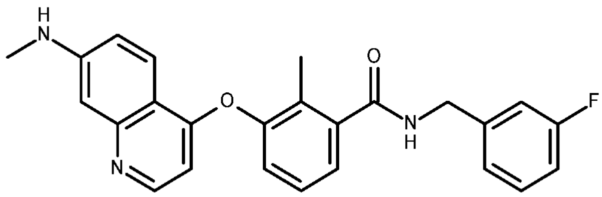

Formula (CXXXIX)

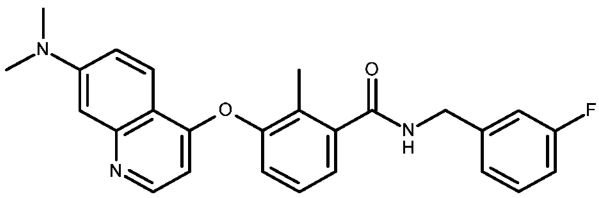

Formula (CXL)

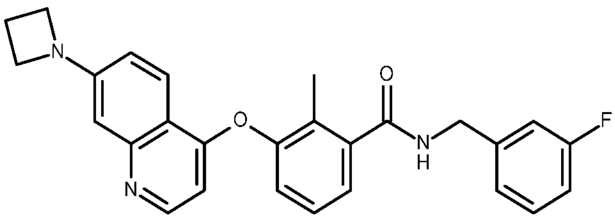

Formula (CXLI)

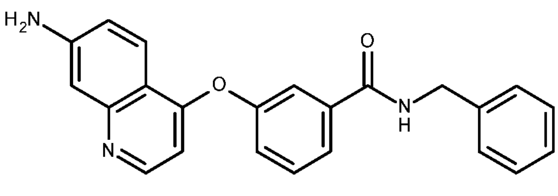

Formula (CXLII)

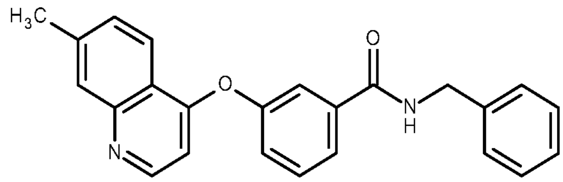

Formula (CXLIII)

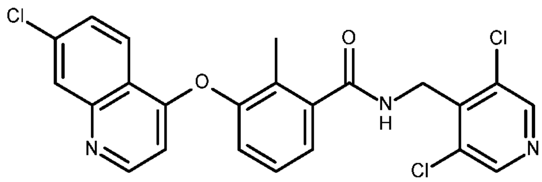

Formula (CXLIV)

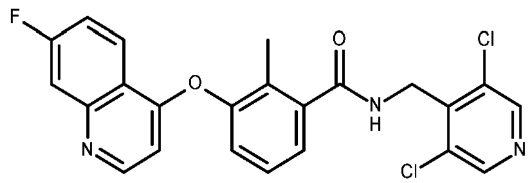

Formula (CXLV)

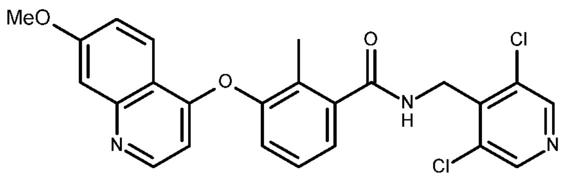

Formula (CXLVI)

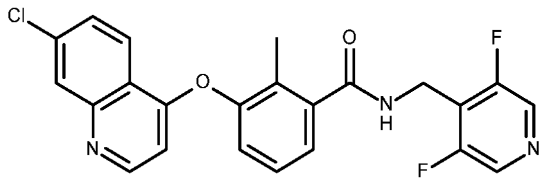

Formula (CXLVII)

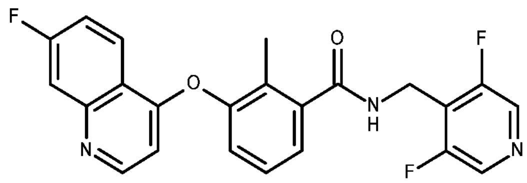

Formula (CXLVIII)

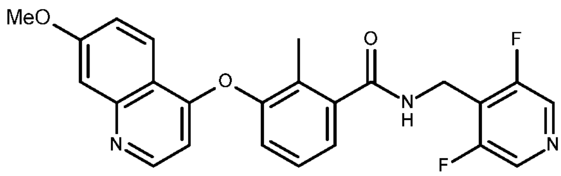

Formula (CXLIX)

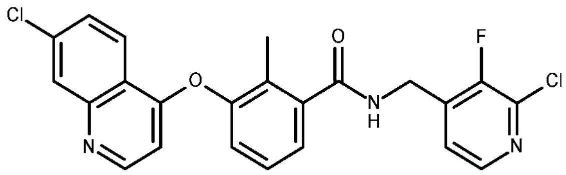

Formula (CL)

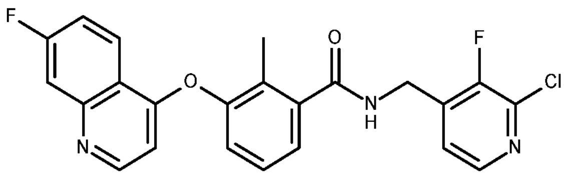

Formula (CLI)

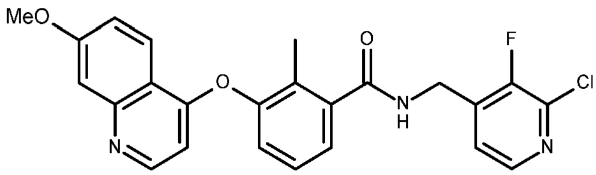

Formula (CLII)

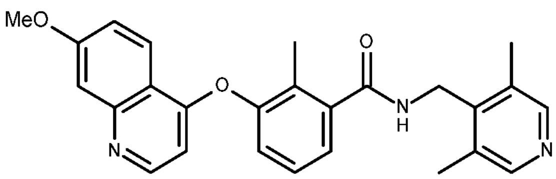

Formula (CLIII)

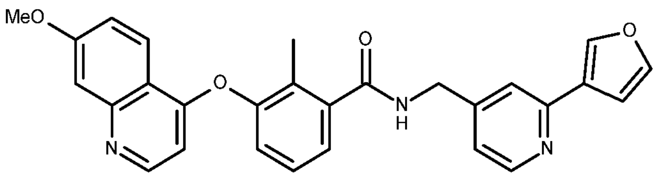

Formula (CLIV)

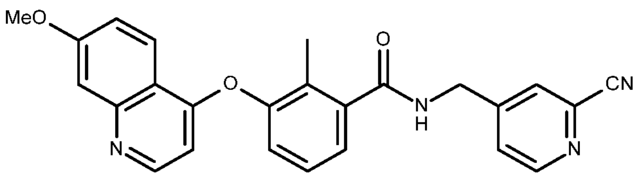

Formula (CLV)

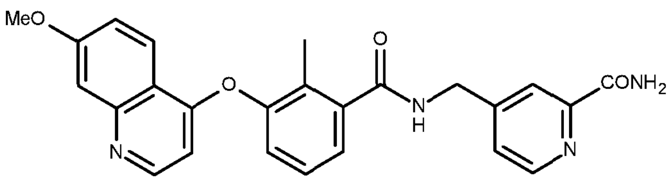

Formula (CLVI)

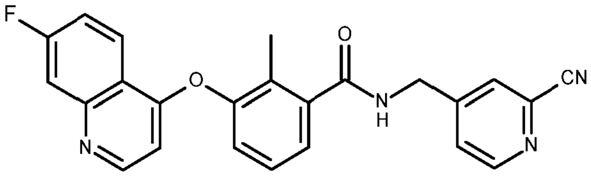

Formula (CLVII)

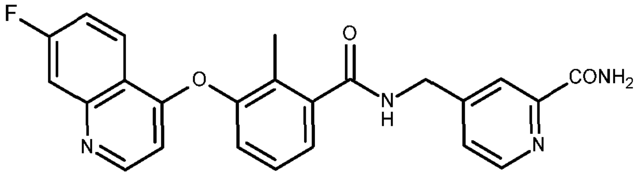

Formula (CLVIII)

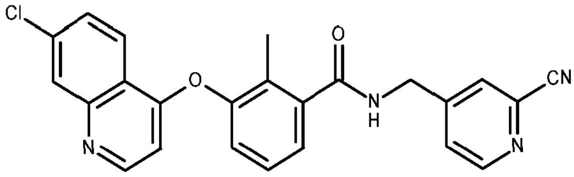

Formula (CLIX)

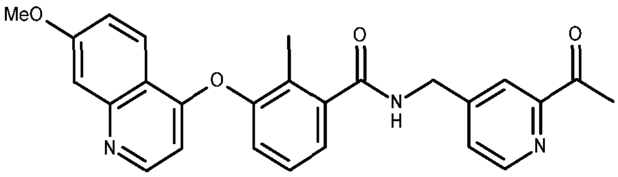

Formula (CLX)

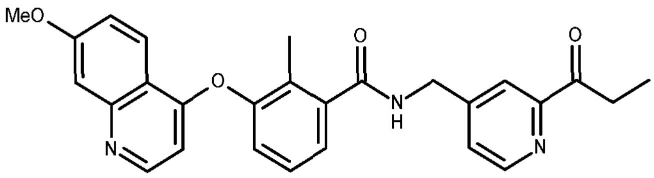

Formula (CLXI)

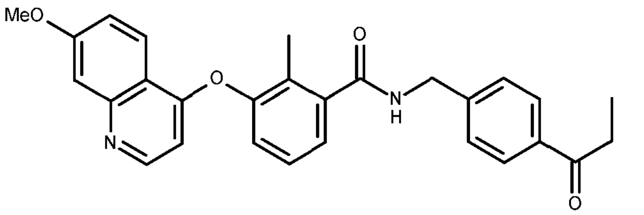

Formula (CLXII)

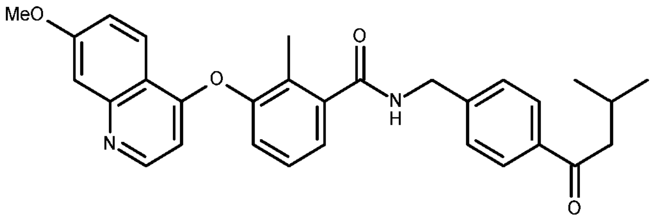

Formula (CLXIII)

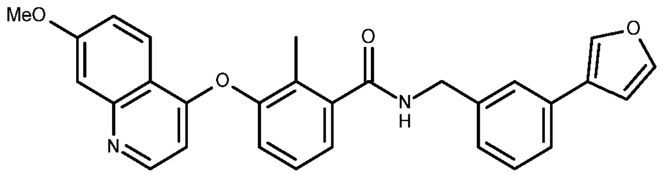

Formula (CLXIV)

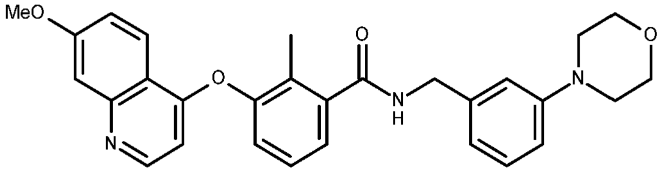

Formula (CLXV)

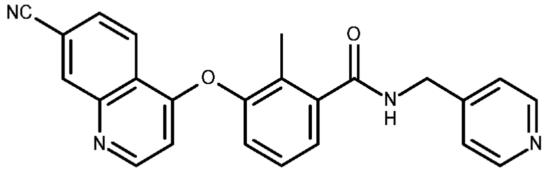

Formula (CLXVI)

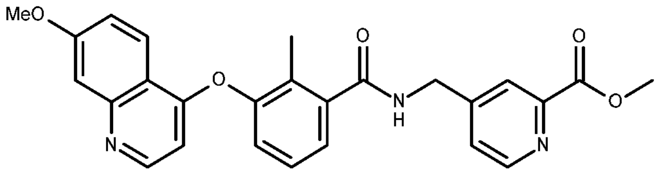

Formula (CLXVII)

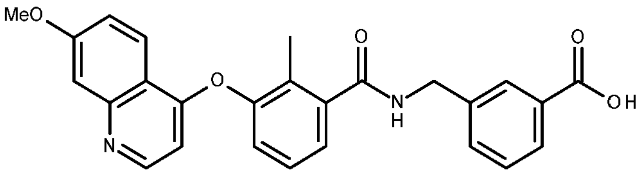

Formula (CLXVIII)

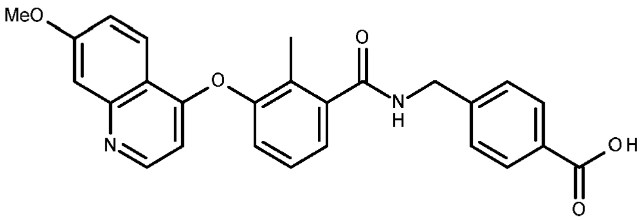

Formula (CLXIX)

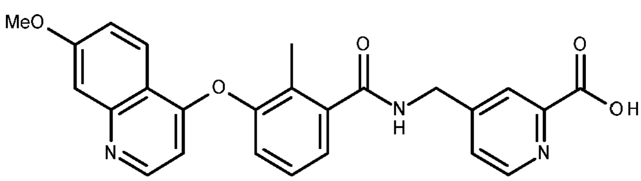

Formula (CLXX)

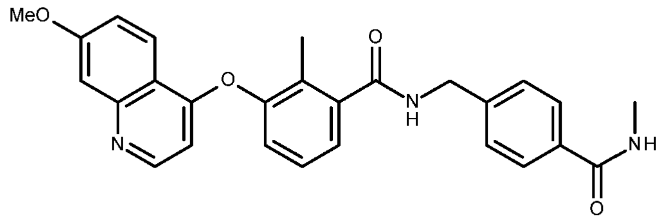

Formula (CLXXI)

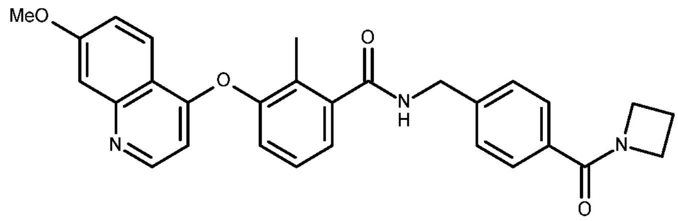

Formula (CLXXII)

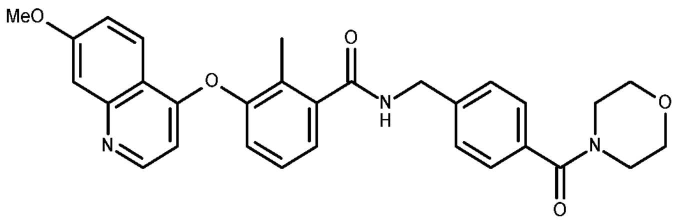

Formula (CLXXIII)

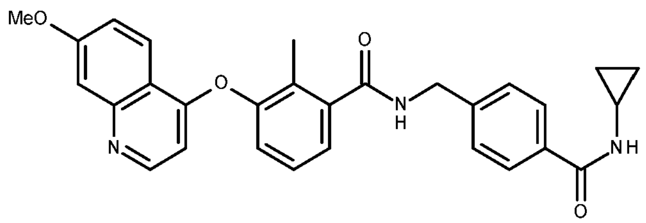

Formula (CLXXIV)

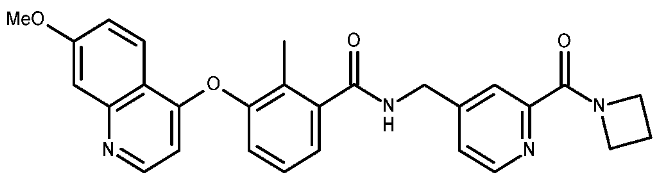

Formula (CLXXV)

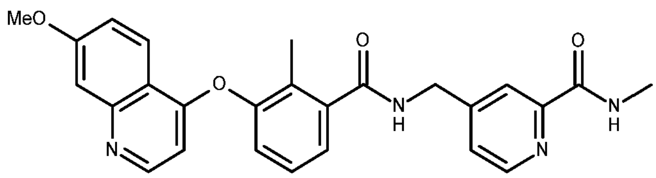

Formula (CLXXVI)

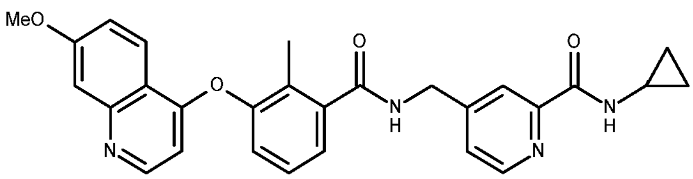

Formula (CLXXVII)

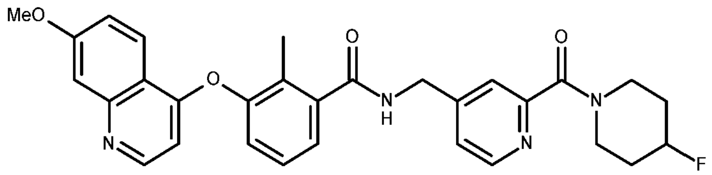

Formula (CLXXVIII)

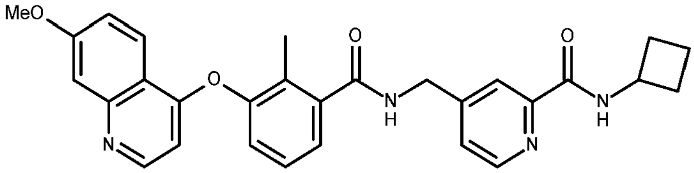

Formula (CLXXIX)

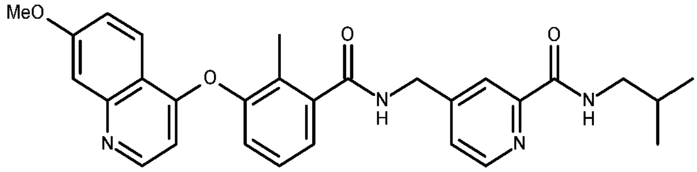

Formula (CLXXX)

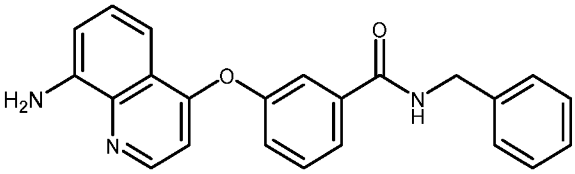

Formula (CLXXXI)

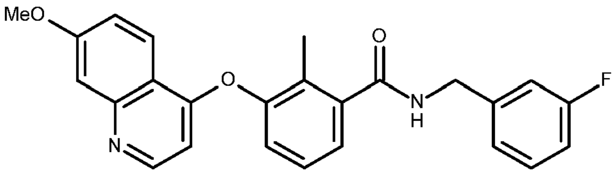

Formula (CLXXXII)

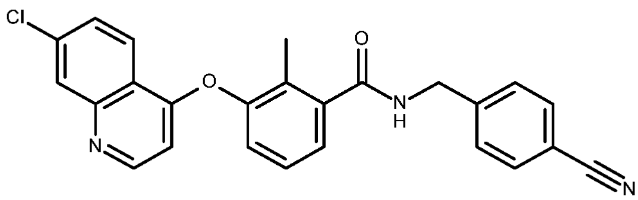

Formula (CLXXXIII)

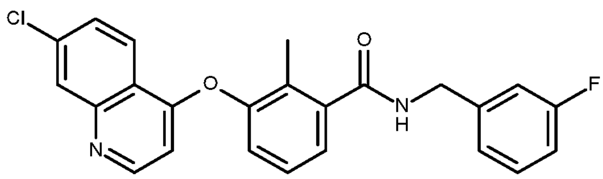

Formula (CLXXXIV)

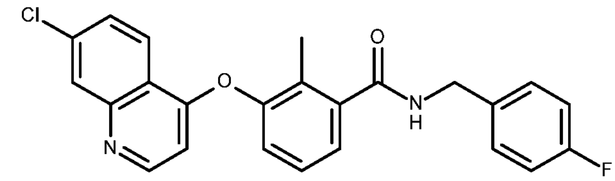

Formula (CLXXXV)

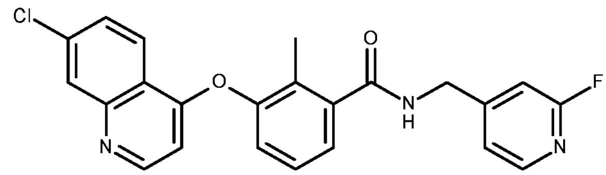

Formula (CLXXXVI)

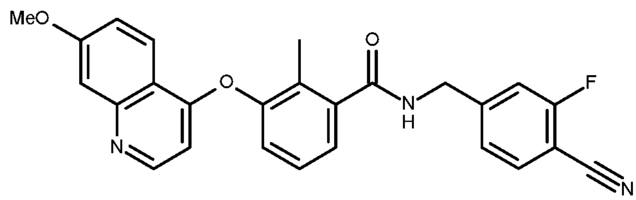

Formula (CLXXXVII)

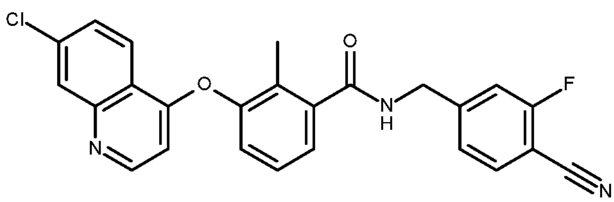

Formula (CLXXXVIII)

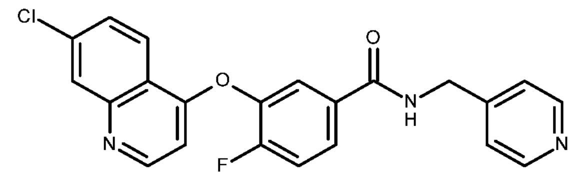

Formula (CLXXXIX)

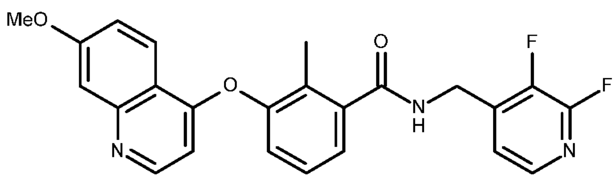

Formula (CXC)

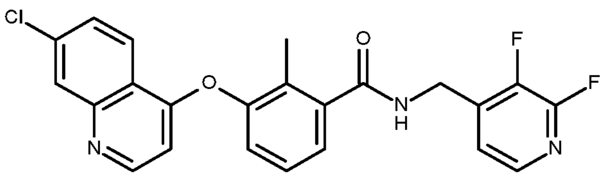

Formula (CXCI)

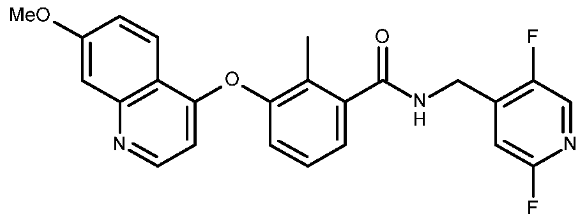

Formula (CXCII)

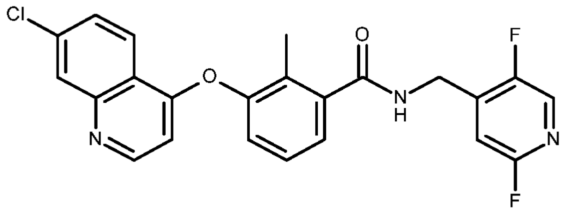

Formula (CXCIII)

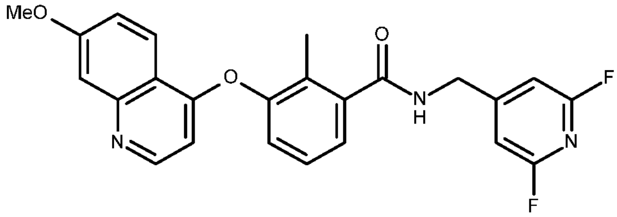

Formula (CXCIV)

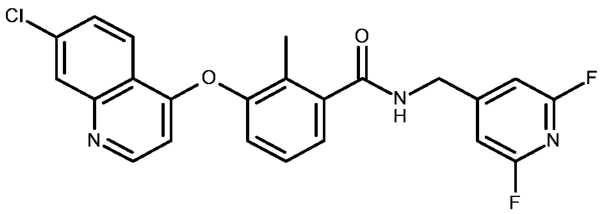

Formula (CXCV)

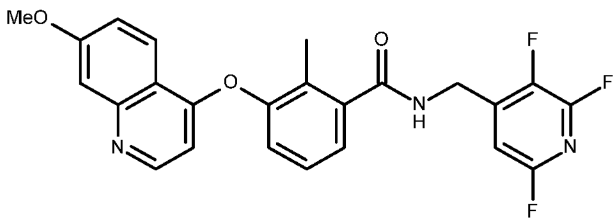

Formula (CXCVI)

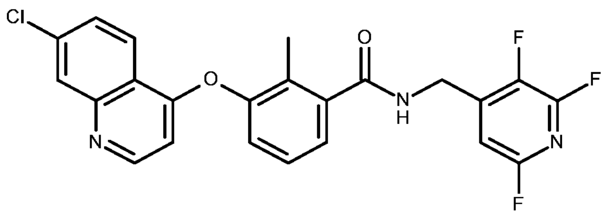

Formula (CXCVII)

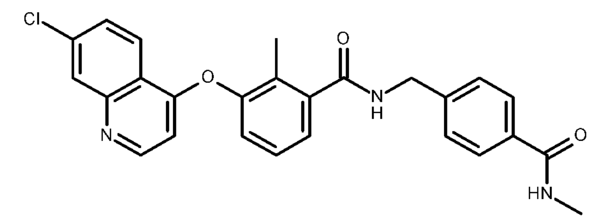

Formula (CXCVIII)

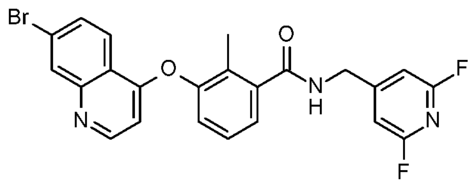

Formula (CXCVIII-1)

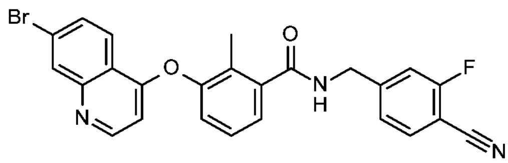

Formula (CXCVIII-2)

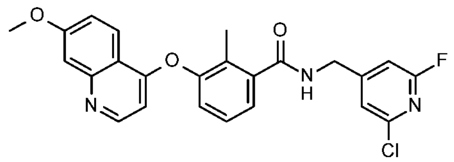

Formula (CXCVIII-3)

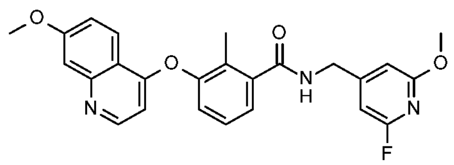

Formula (CXCVIII-4)

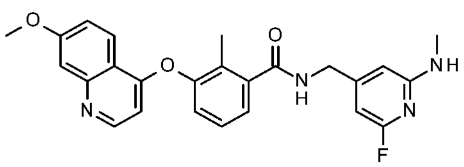

Formula (CXCVIII-5)

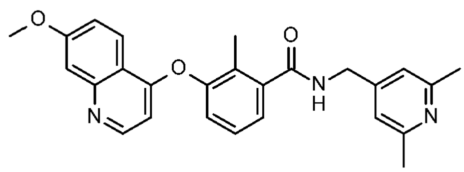

Formula (CXCVIII-6)

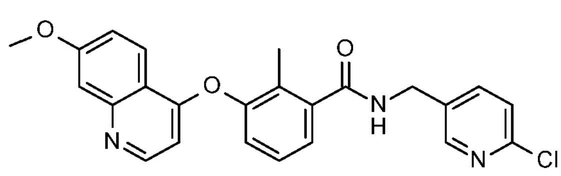

Formula (CXCVIII-7)

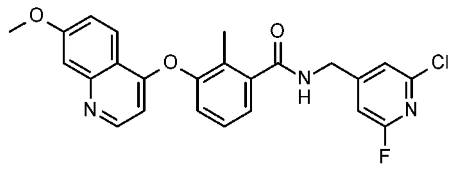

Formula (CXCVIII-8)

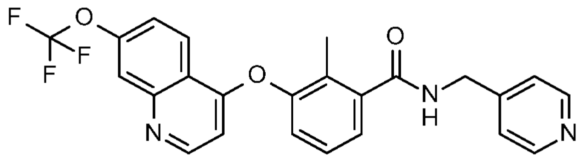

Formula (CXCVIII-9)

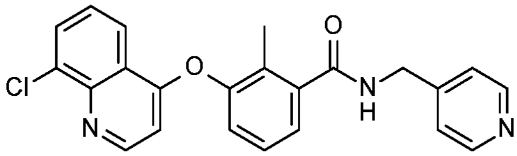

Formula (CXCVIII-10)

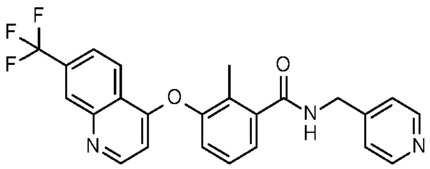

Formula (CXCVIII-11)

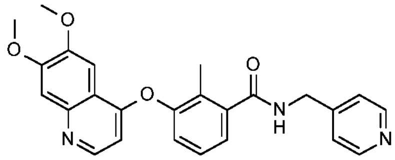

Formula (CXCVIII-12)

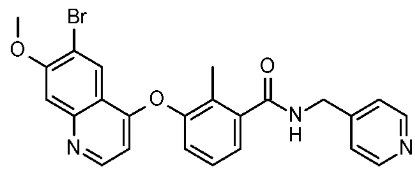

Formula (CXCVIII-13)

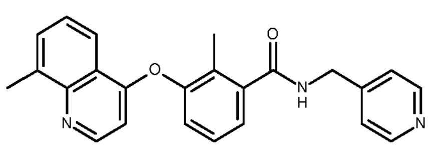

Formula (CXCVIII-14)

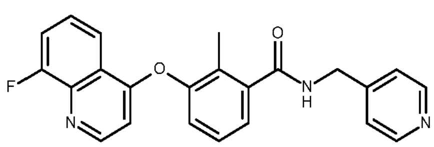

Formula (CXCVIII-15)

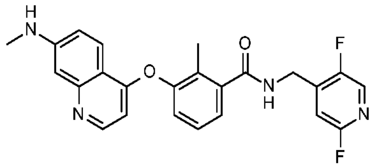

Formula (CXCVIII-16)

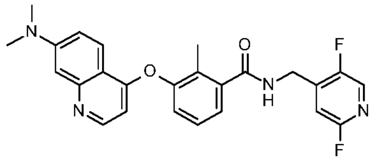

Formula (CXCVIII-17)

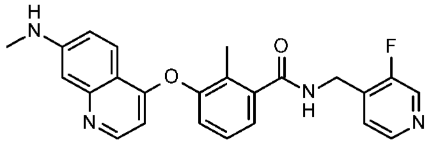

Formula (CXCVIII-18)

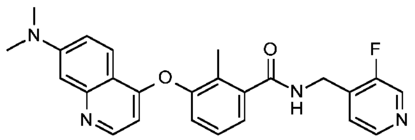

Formula (CXCVIII-19)

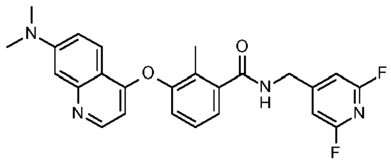

Formula (CXCVIII-20)

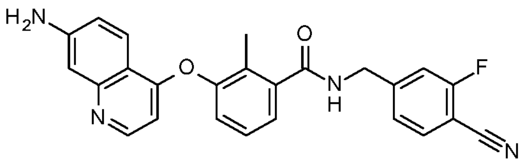

Formula (CXCVIII-21)

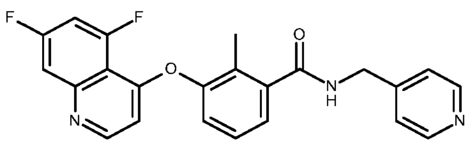

Formula (CXCVIII-22)

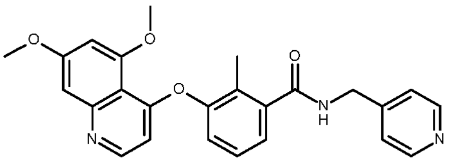

Formula (CXCVIII-23)

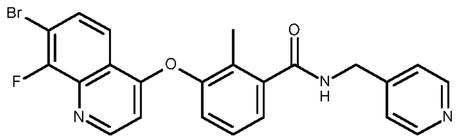

Formula (CXCVIII-24)

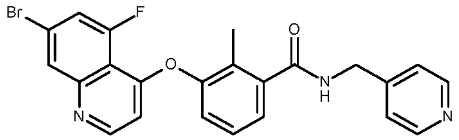

Formula (CXCVIII-25)

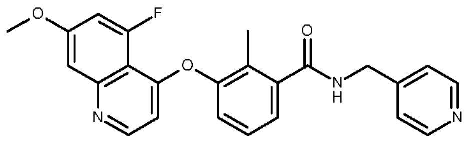

Formula (CXCVIII-26)

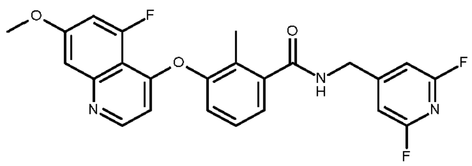

Formula (CXCVIII-27)

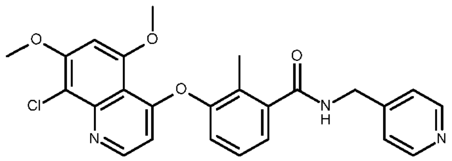

Formula (CXCVIII-28)

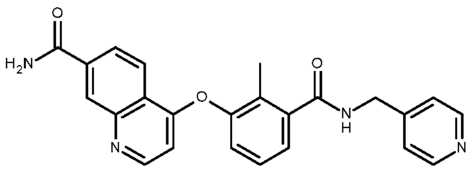

Formula (CXCVIII-29)

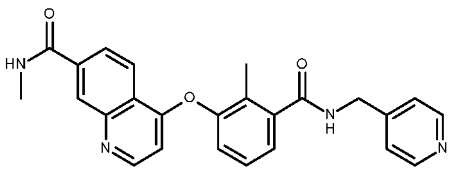

Formula (CXCVIII-30)

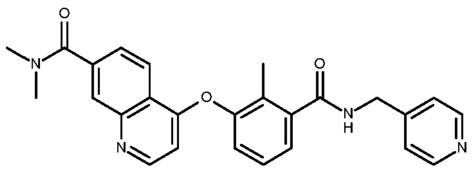

Formula (CXCVIII-31)

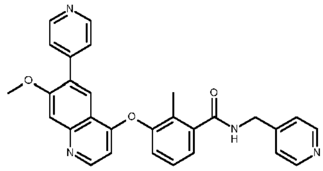

Formula (CXCVIII-32)

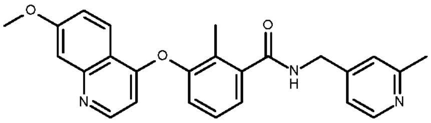

Formula (CXCVIII-33)

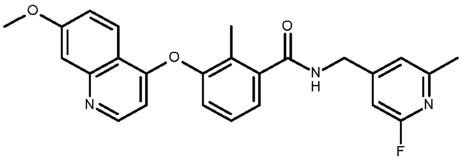

Formula (CXCVIII-34)

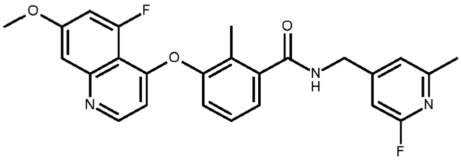

Formula (CXCVIII-35)

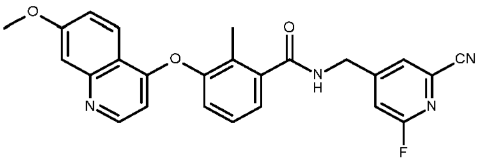

Formula (CXCVIII-36)

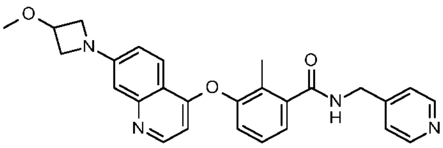

Formula (CXCVIII-37)

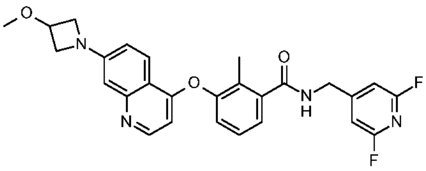

Formula (CXCVIII-38)

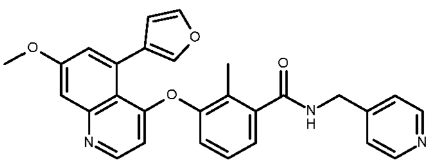

Formula (CXCVIII-39)

In a more preferred embodiment of the present invention, the compound (C), according to the invention, for use as a kinase inhibitor according to general formula (IIIa-1), or the N-oxide, pharmaceutically acceptable salt, pharmaceutically acceptable solvate or stereoisomer thereof is a compound of formula (LXI), (LXIII), (LXXII), (LXXIV)-(LXXXV),

(LXXXVI), (CXIII), (CLXXXVI), (CLXXXVIII), (CXC)-(CXCVI), (CXCVII-6)-(CXCVII-7), (CXCVII-16)-(CXCVII-17), (CXCVII-19)-(CXCVII-20), (CXCVII-27), (CXCVII-33)-(CXCVII-35), or (CXCVII-37)-(CXCVII-38) herein below:

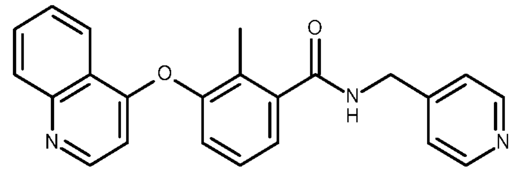

Formula (LXI)

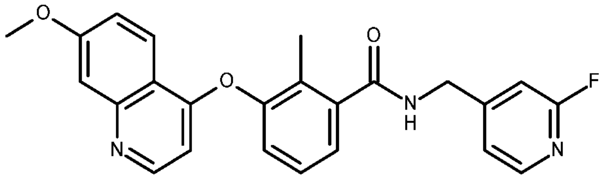

Formula (LXIII)

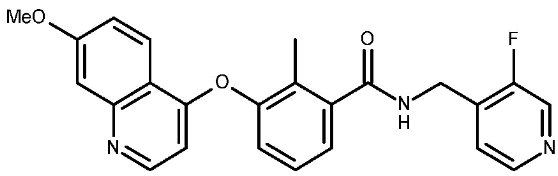

Formula (LXXII)

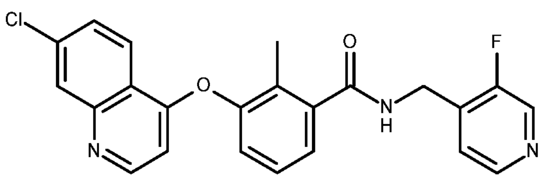

Formula (LXXIV)

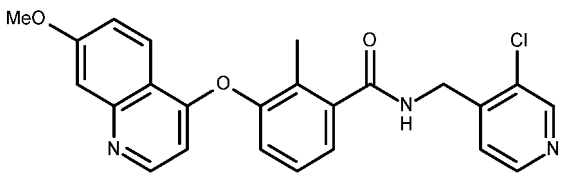

Formula (LXXV)

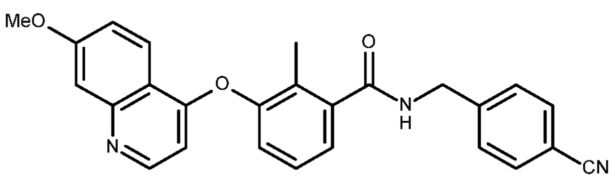

Formula (LXXXVI)

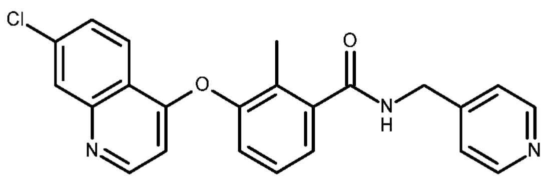

Formula (CXIII)

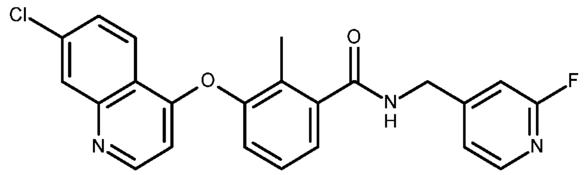

Formula (CLXXXVI)

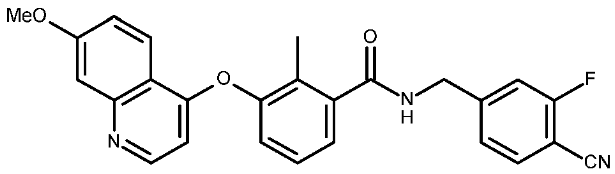

Formula (CLXXXVII)

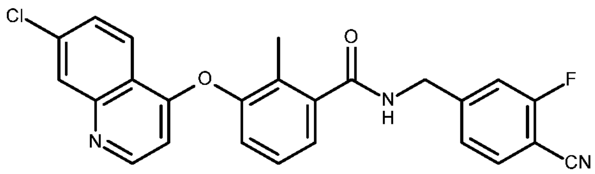

Formula (CLXXXVIII)

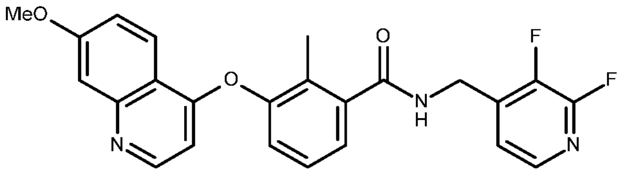

Formula (CXC)

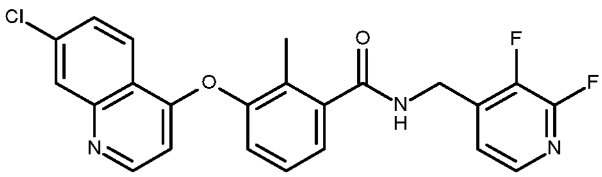

Formula (CXCI)

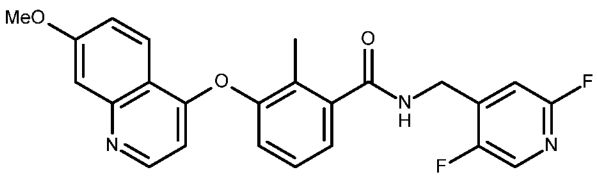

Formula (CXCII)

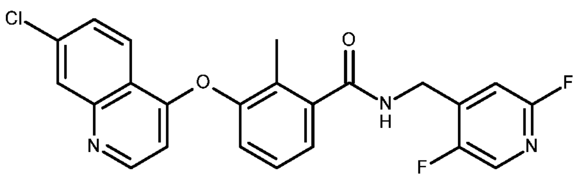

Formula (CXCIII)

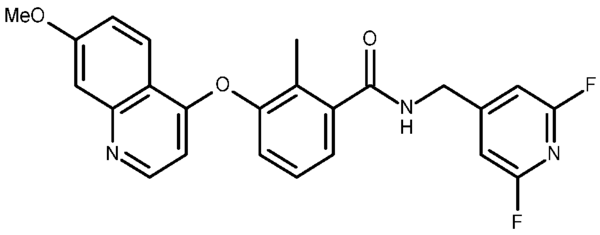

Formula (CXCIV)

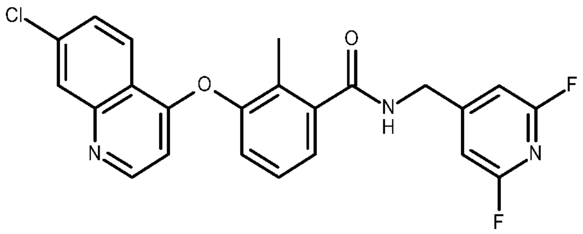

Formula (CXCV)

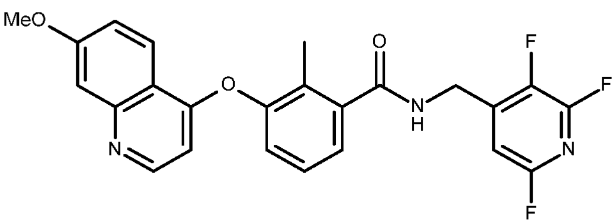

Formula (CXCVI)

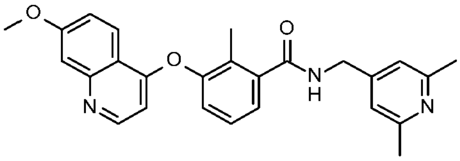

Formula (CXCVIII-6)

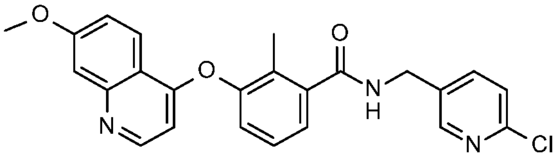

Formula (CXCVIII-7)

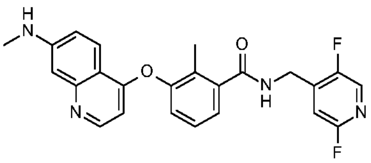

Formula (CXCVIII-16)

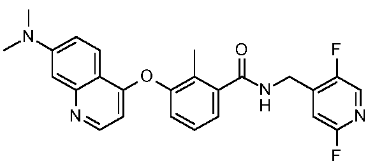

Formula (CXCVIII-17)

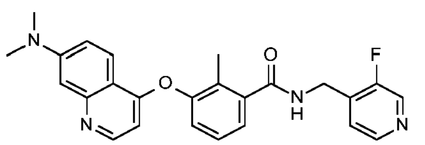

Formula (CXCVIII-19)

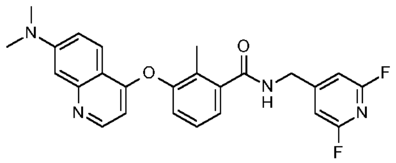

Formula (CXCVIII-20)

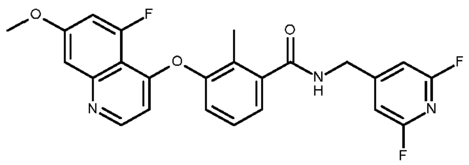

Formula (CXCVIII-27)

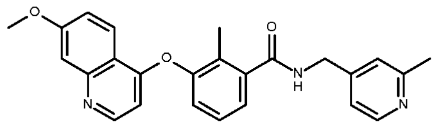

Formula (CXCVIII-33)

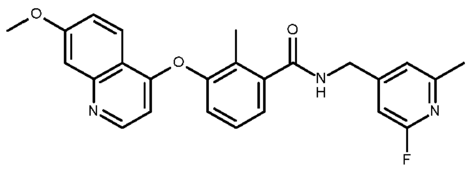

Formula (CXCVIII-34)

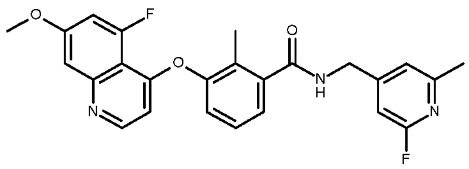

Formula (CXCVIII-35)

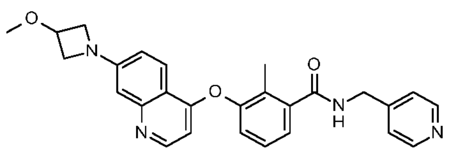

Formula (CXCVIII-37)

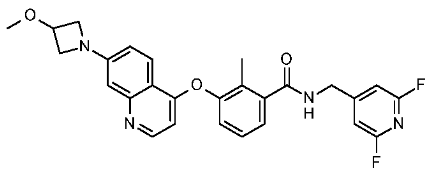

Formula (CXCVIII-38)

In a preferred embodiment of the present invention, the compound (C) according to the invention, for use as a kinase inhibitor according to general formula (IIIa-a1) is a compound chosen among those of formulae (CXCVIII-1) to (CXCVIII-90) herein below:

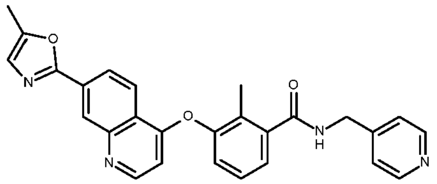

Formula (CXCVIII-1)

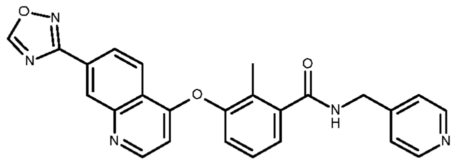

Formula (CXCVIII-2)

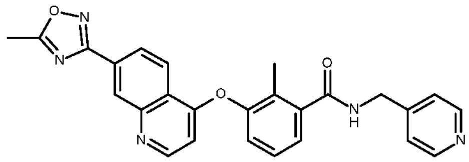

Formula (CXCVIII-3)

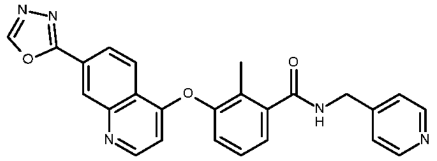

Formula (CXCVIII-4)

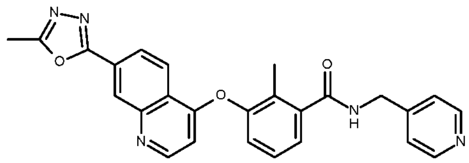

Formula (CXCVIII-5)

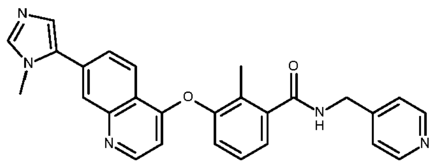

Formula (CXCVIII-6)

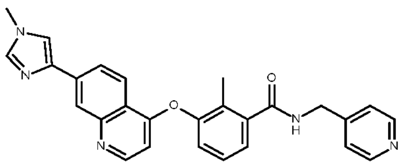

Formula (CXCVIII-7)

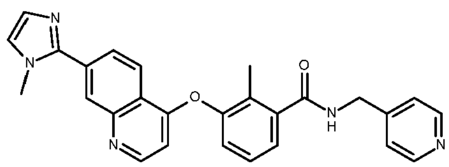

Formula (CXCVIII-8)

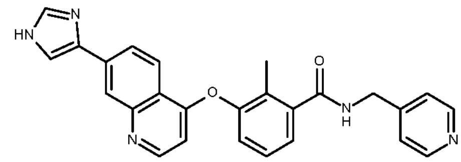

Formula (CXCVIII-9)

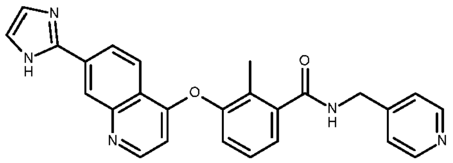

Formula (CXCVIII-10)

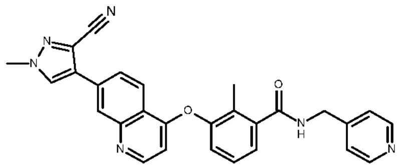

Formula (CXCVIII-11)

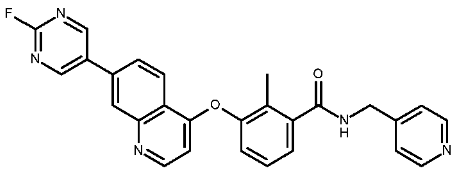

Formula (CXCVIII-12)

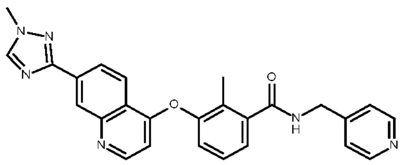

Formula (CXCVIII-13)

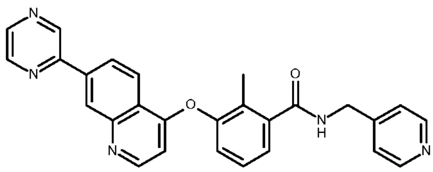

Formula (CXCVIII-14)

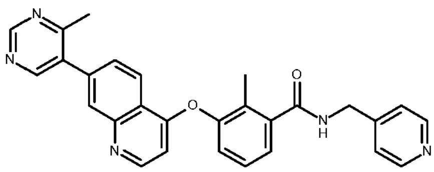

Formula (CXCVIII-15)

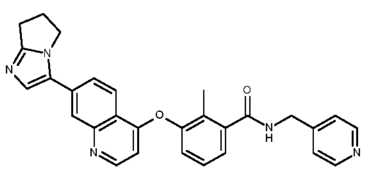

Formula (CXCVIII-16)

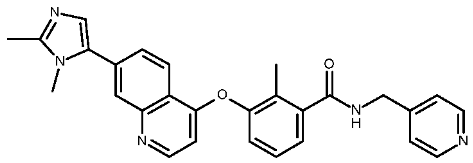

Formula (CXCVIII-17)

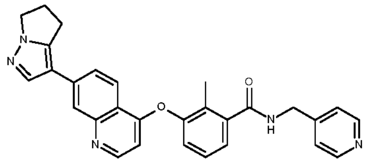

Formula (CXCVIII-18)

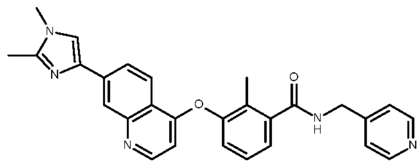

Formula (CXCVIII-19)

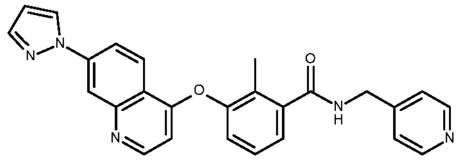

Formula (CXCVIII-20)

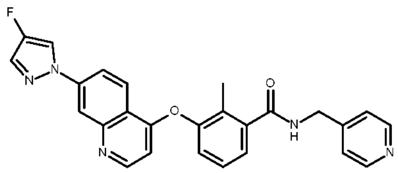

Formula (CXCVIII-21)

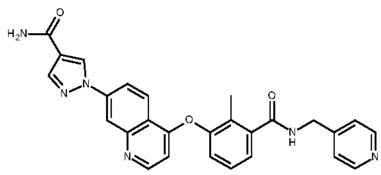

Formula (CXCVIII-22)

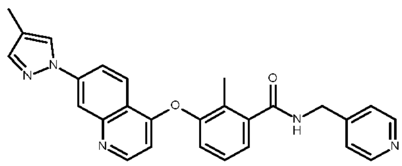

Formula (CXCVIII-23)

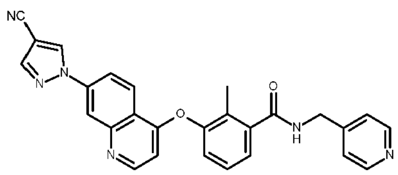

Formula (CXCVIII-24)

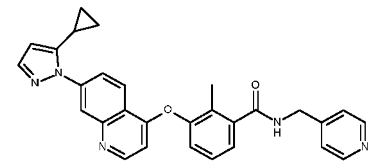

Formula (CXCVIII-25)

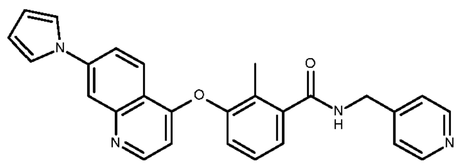

Formula (CXCVIII-26)

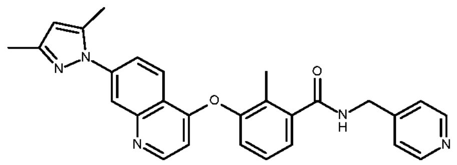

Formula (CXCVIII-27)

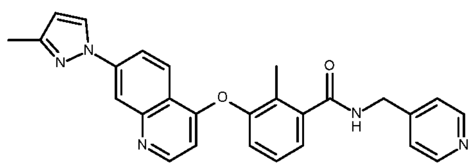

Formula (CXCVIII-28)

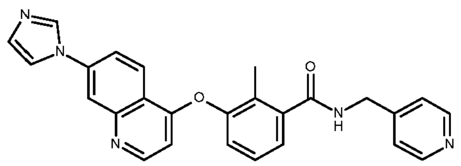

Formula (CXCVIII-29)

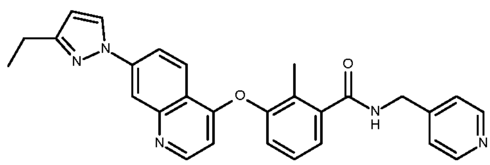

Formula (CXCVIII-30)

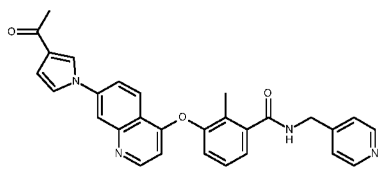

Formula (CXCVIII-31)

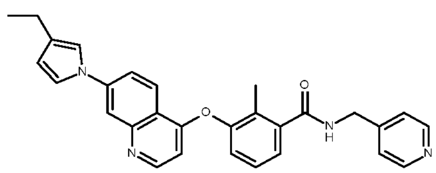

Formula (CXCVIII-32)

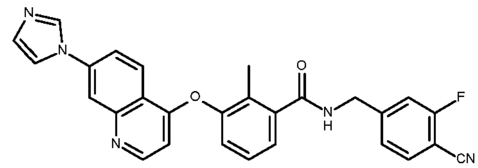

Formula (CXCVIII-33)

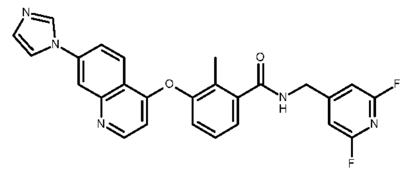

Formula (CXCVIII-34)

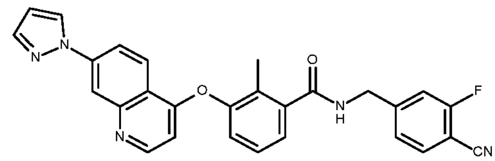

Formula (CXCVIII-35)

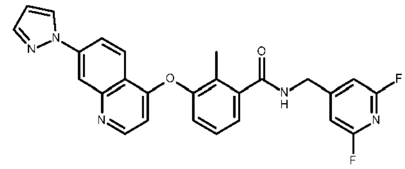

Formula (CXCVIII-36)

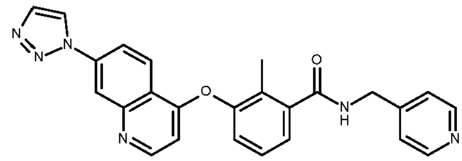

Formula (CXCVIII-37)

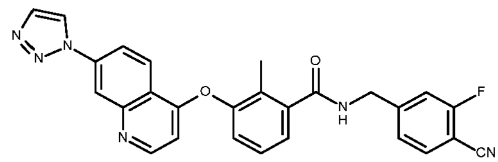

Formula (CXCVIII-38)

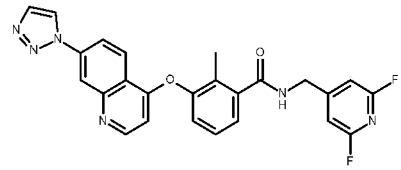

Formula (CXCVIII-39)

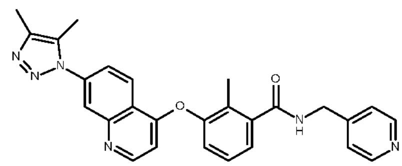

Formula (CXCVIII-40)

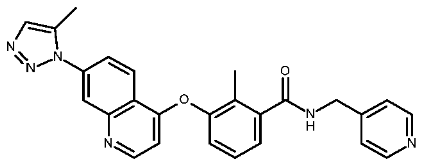

Formula (CXCVIII-41)

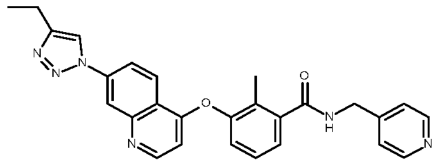

Formula (CXCVIII-42)

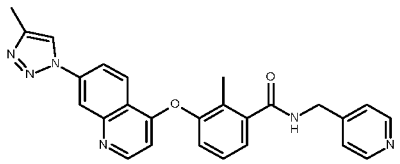

Formula (CXCVIII-43)

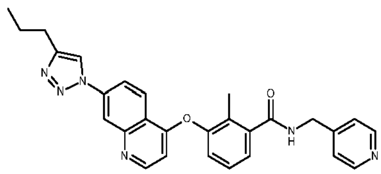

Formula (CXCVIII-44)

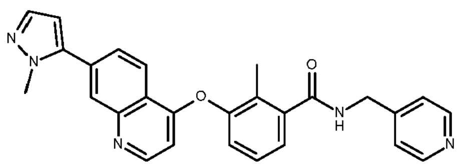

Formula (CXCVIII-45)

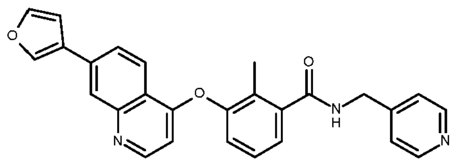

Formula (CXCVIII-46)

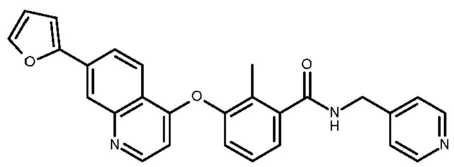

Formula (CXCVIII-47)

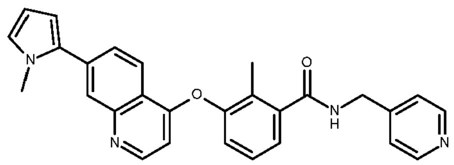

Formula (CXCVIII-48)

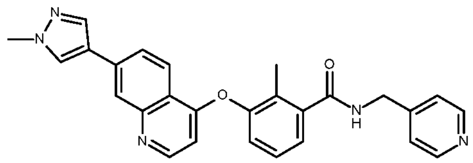

Formula (CXCVIII-49)

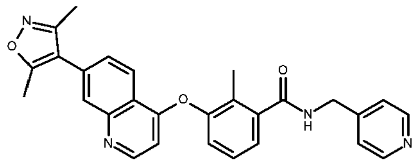

Formula (CXCVIII-50)

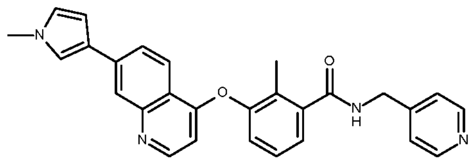

Formula (CXCVIII-51)

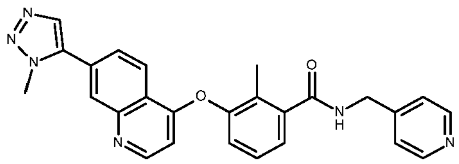

Formula (CXCVIII-52)

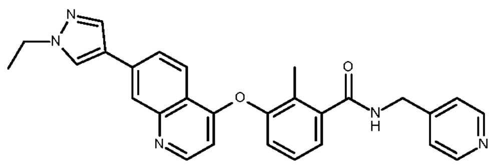

Formula (CXCVIII-53)

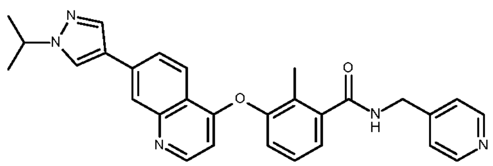

Formula (CXCVIII-54)

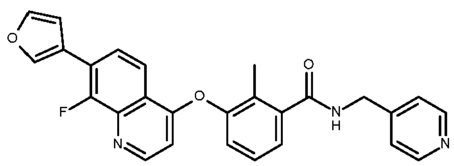

Formula (CXCVIII-55)

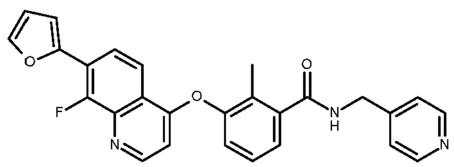

Formula (CXCVIII-56)

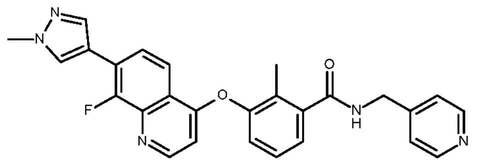

Formula (CXCVIII-57)

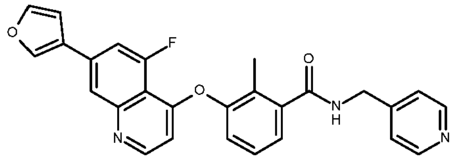

Formula (CXCVIII-58)

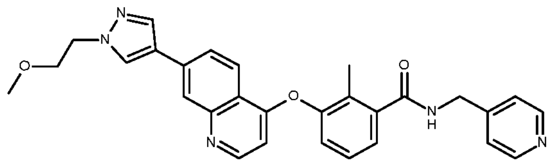

Formula (CXCVIII-59)

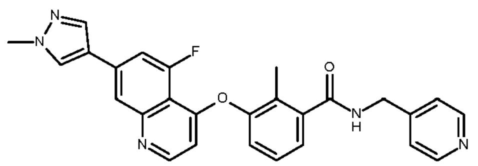

Formula (CXCVIII-60)

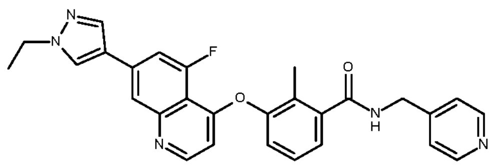

Formula (CXCVIII-61)

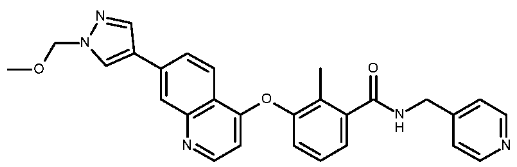

Formula (CXCVIII-62)

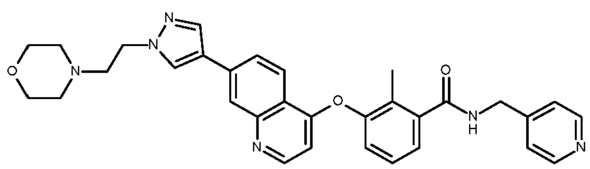

Formula (CXCVIII-63)

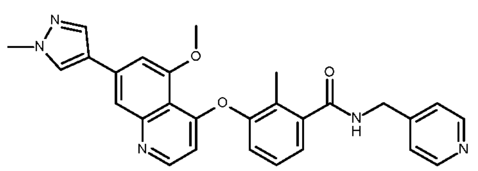

Formula (CXCVIII-64)

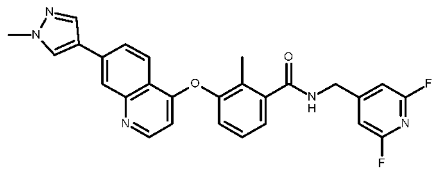

Formula (CXCVIII-65)

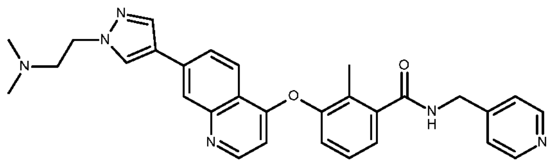

Formula (CXCVIII-66)

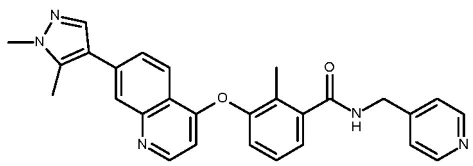

Formula (CXCVIII-67)

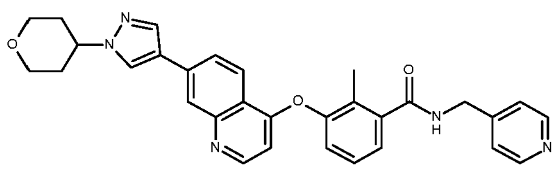

Formula (CXCVIII-68)

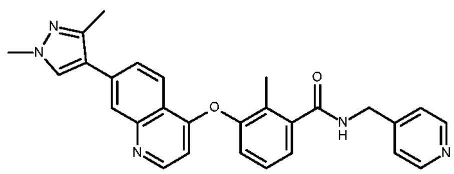

Formula (CXCVIII-69)

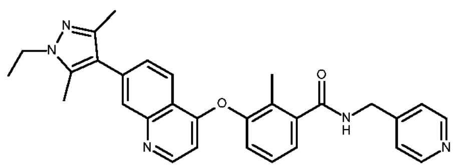

Formula (CXCVIII-70)

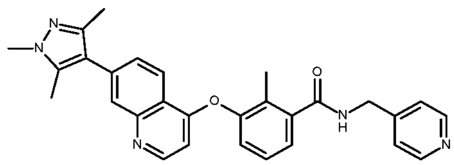

Formula (CXCVIII-71)

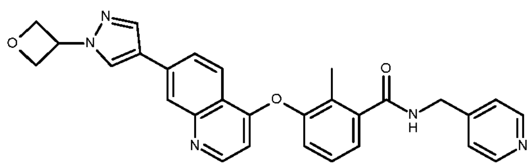

Formula (CXCVIII-72)

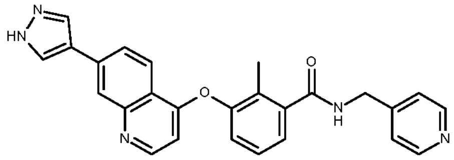

Formula (CXCVIII-73)

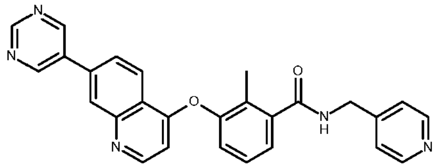

Formula (CXCVIII-74)

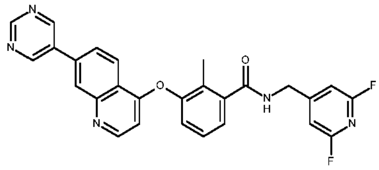

Formula (CXCVIII-75)

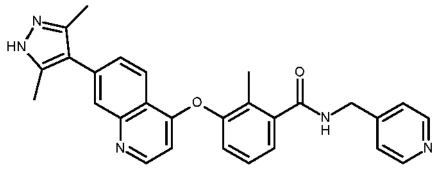

Formula (CXCVIII-76)

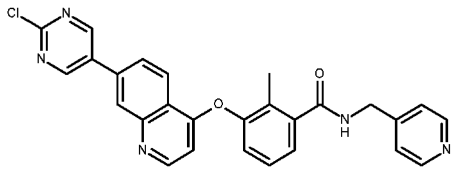

Formula (CXCVIII-77)

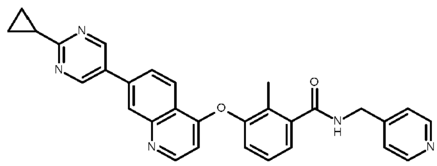

Formula (CXCVIII-78)

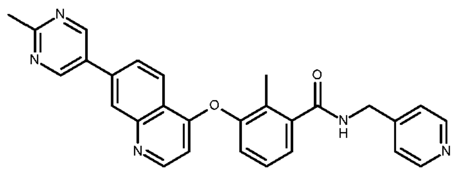

Formula (CXCVIII-79)

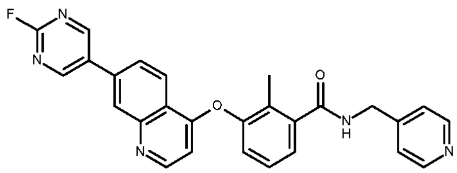

Formula (CXCVIII-80)

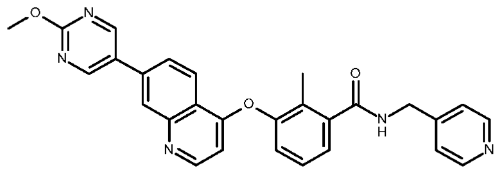

Formula (CXCVIII-81)

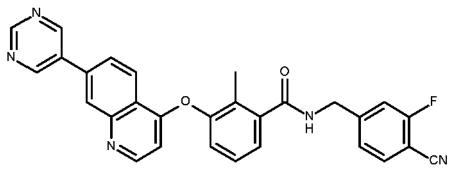

Formula (CXCVIII-82)

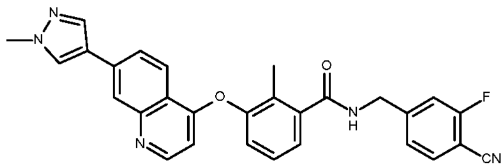

Formula (CXCVIII-83)

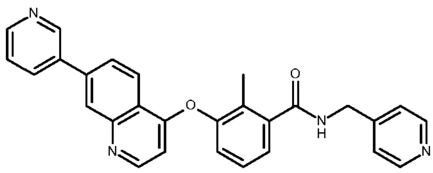

Formula (CXCVIII-84)

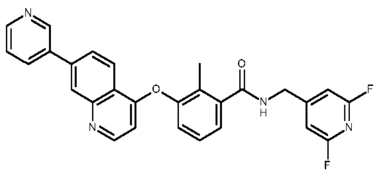

Formula (CXCVIII-85)

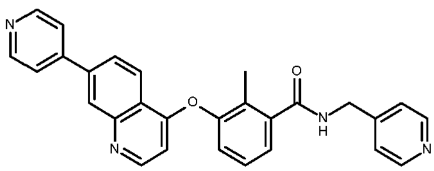

Formula (CXCVIII-86)

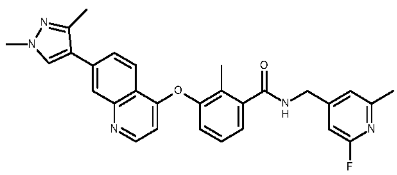

Formula (CXCVIII-87)

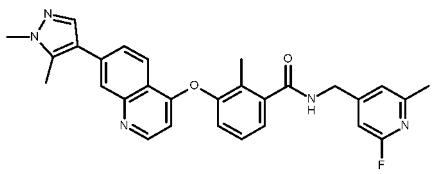

Formula (CXCVIII-88)

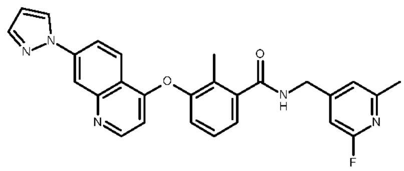

Formula (CXCVIII-89)

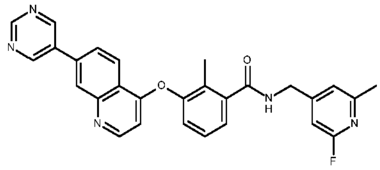

Formula (CXCVIII-90)

In a more preferred embodiment of the present invention, the compound (C), according to the invention, for use as a kinase inhibitor according to general formula (IIIa-a1), or the N-oxide, pharmaceutically acceptable salt, pharmaceutically acceptable solvate or stereoisomer thereof is a compound of formula (CXCVIII-3), (CXCVIII-7), (CXCVIII-11), (CXCVIII-16), (CXCVIII-19)-(CXCVIII-21), (CXCVIII-23)-(CXCVIII-26), (CXCVIII-28)-(CXCVIII-31), (CXCVIII-33)-(CXCVIII-39), (CXCVIII-42)-(CXCVIII-47), (CXCVIII-49), (CXCVIII-51)-(CXCVIII-53), (CXCVIII-56), (CXCVIII-59), (CXCVIII-62), (CXCVIII-66), (CXCVIII-67), (CXCVIII-70), (CXCVIII-71), (CXCVIII-73)- (CXCVIII-75), (CXCVIII-81)-(CXCVIII-86), or (CXCVIII-88)-(CXCVIII-90) herein below:

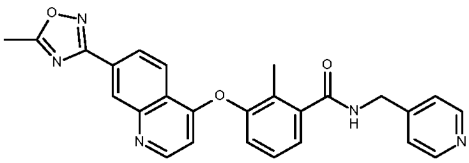

Formula (CXCVIII-3)

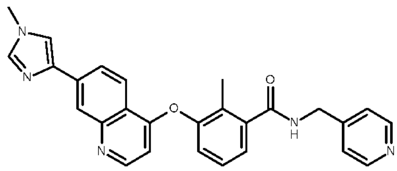

Formula (CXCVIII-7)

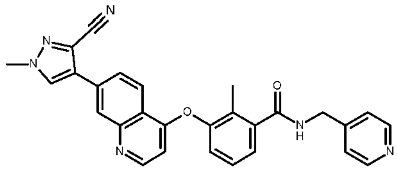

Formula (CXCVIII-11)

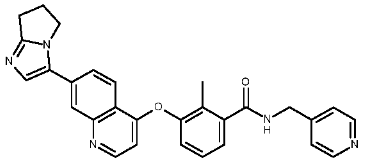

Formula (CXCVIII-16)

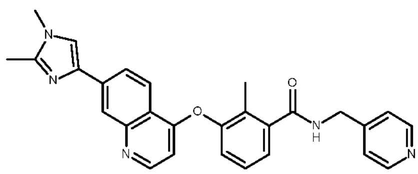

Formula (CXCVIII-19)

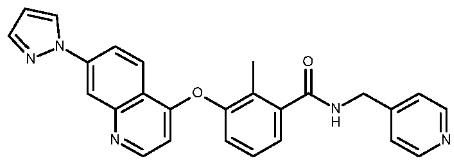

Formula (CXCVIII-20)

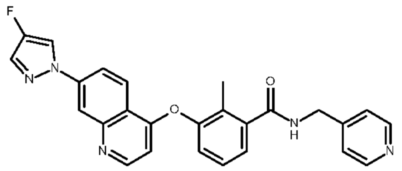

Formula (CXCVIII-21)

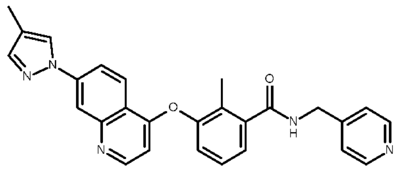

Formula (CXCVIII-23)

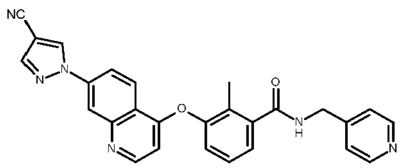

Formula (CXCVIII-24)

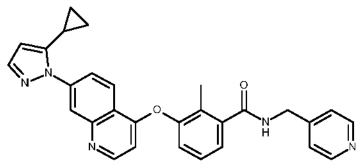

Formula (CXCVIII-25)

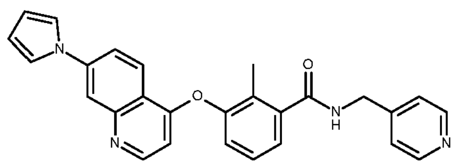

Formula (CXCVIII-26)

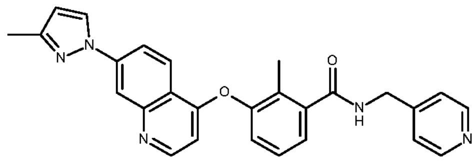

Formula (CXCVIII-28)

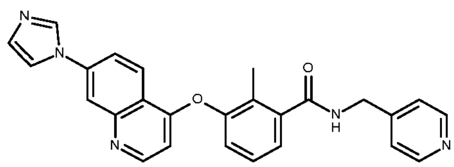

Formula (CXCVIII-29)

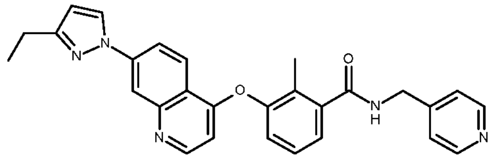

Formula (CXCVIII-30)

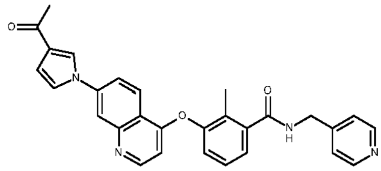

Formula (CXCVIII-31)

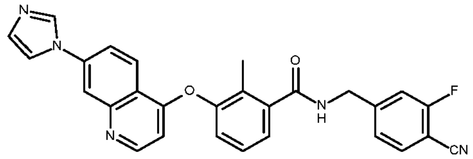

Formula (CXCVIII-33)

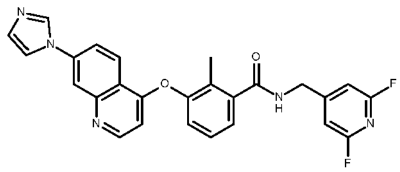

Formula (CXCVIII-34)

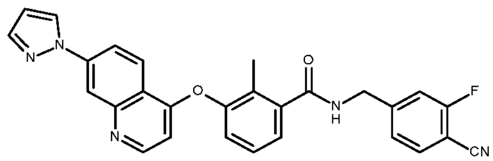

Formula (CXCVIII-35)

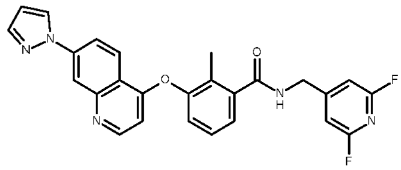

Formula (CXCVIII-36)

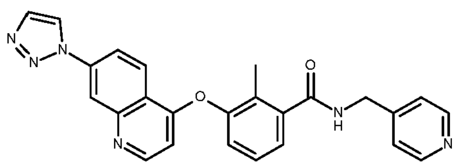

Formula (CXCVIII-37)

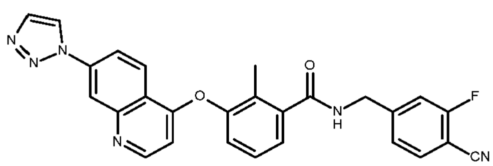

Formula (CXCVIII-38)

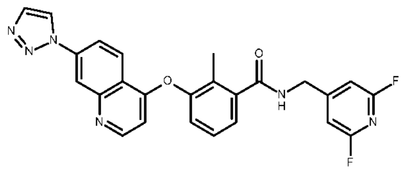

Formula (CXCVIII-39)

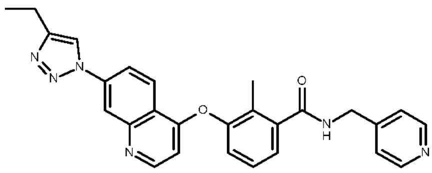

Formula (CXCVIII-42)

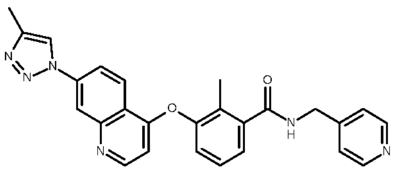

Formula (CXCVIII-43)

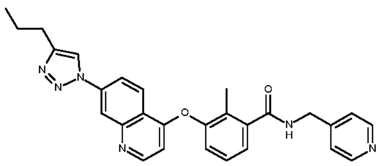

Formula (CXCVIII-44)

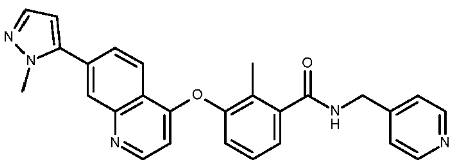

Formula (CXCVIII-45)

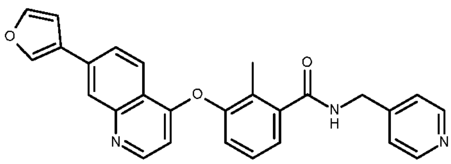

Formula (CXCVIII-46)

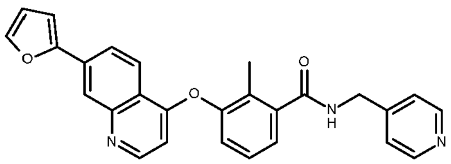

Formula (CXCVIII-47)

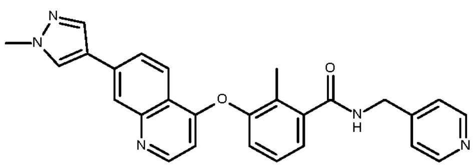

Formula (CXCVIII-49)

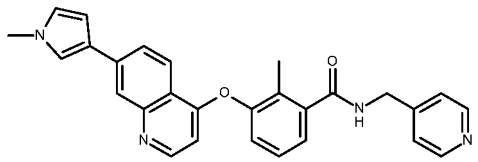

Formula (CXCVIII-51)

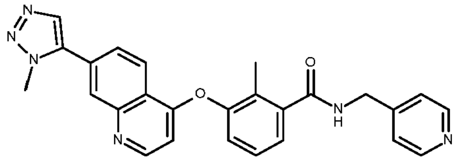

Formula (CXCVIII-52)

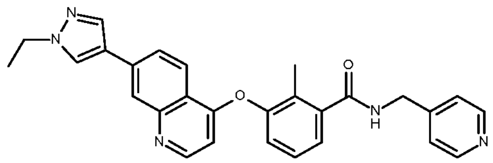

Formula (CXCVIII-53)

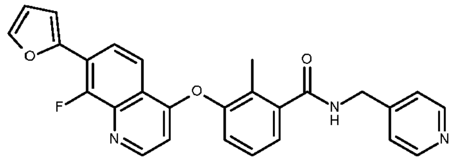

Formula (CXCVIII-56)

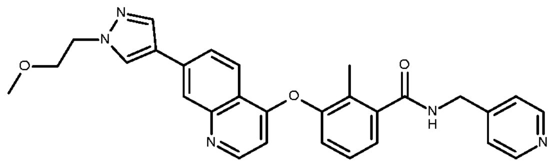

Formula (CXCVIII-59)

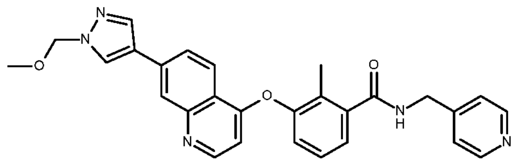

Formula (CXCVIII-62)

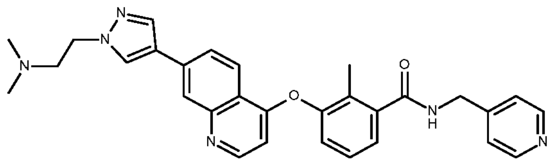

Formula (CXCVIII-66)

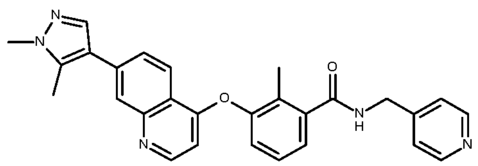

Formula (CXCVIII-67)

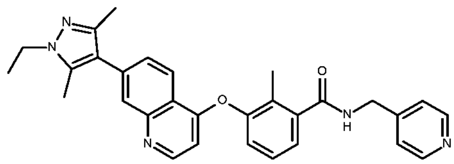

Formula (CXCVIII-70)

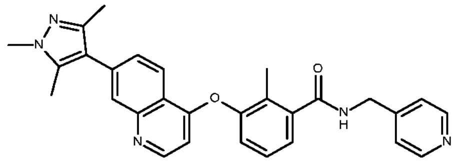

Formula (CXCVIII-71)

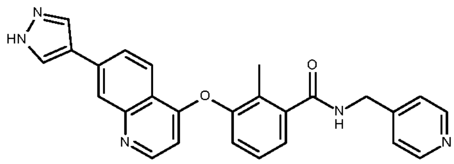

Formula (CXCVIII-73)

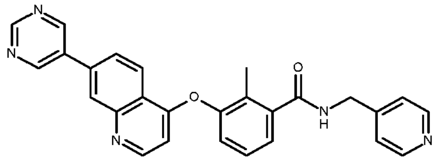

Formula (CXCVIII-74)

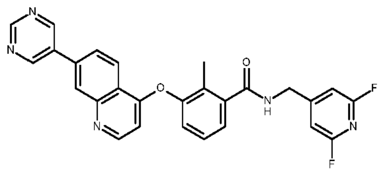

Formula (CXCVIII-75)

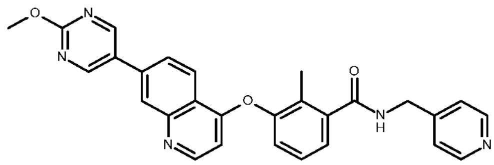

Formula (CXCVIII-81)

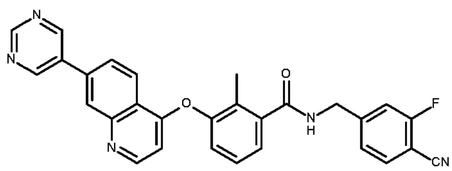

Formula (CXCVIII-82)

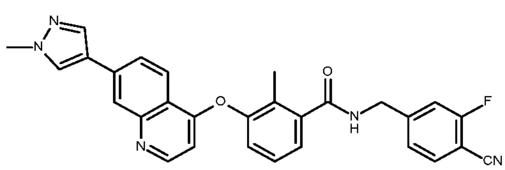

Formula (CXCVIII-83)

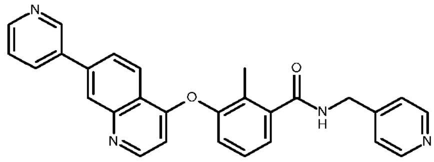

Formula (CXCVIII-84)

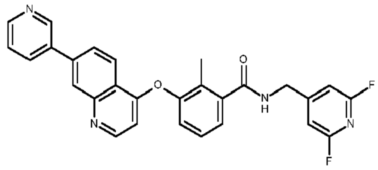

Formula (CXCVIII-85)

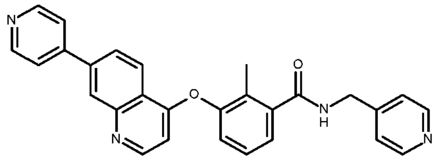

Formula (CXCVIII-86)

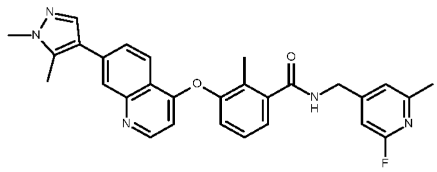

Formula (CXCVIII-88)

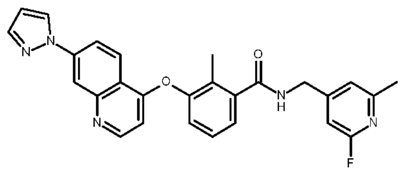

Formula (CXCVIII-89)

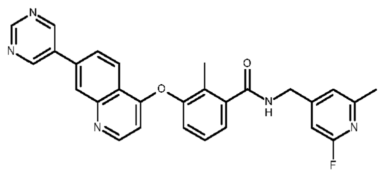

Formula (CXCVIII-90)

In a preferred embodiment of the present invention, the compound (C), according to the invention, for use as a kinase inhibitor according to general formula (IVa-1) is a compound chosen among those of formulae (CXCIX) to (CCI) herein below:

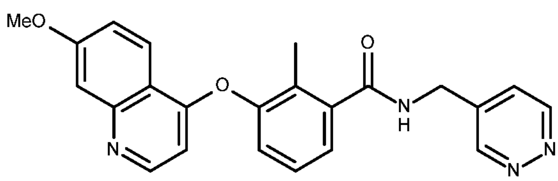

Formula (CXCIX)

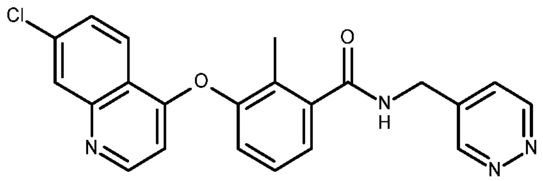

Formula (CC)

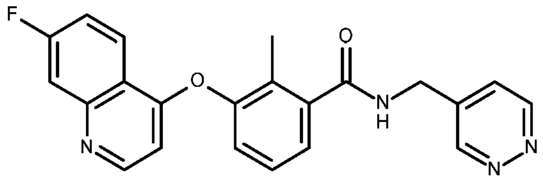

Formula (CCI)

In a preferred embodiment of the present invention, the compound (C), according to the invention, for use as a kinase inhibitor according to general formula (Va-1) is a compound chosen among those of formula (CCII) or (CCIII) herein below:

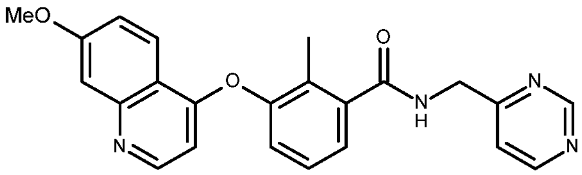

Formula (CCII)

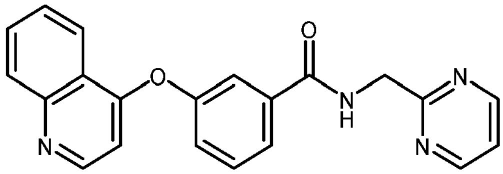

Formula (CCIII)

In a preferred embodiment of the present invention, the compound (C), according to the invention, for use as a kinase inhibitor according to general formula (VIa-1) is a compound according to formula (CCIV) herein below:

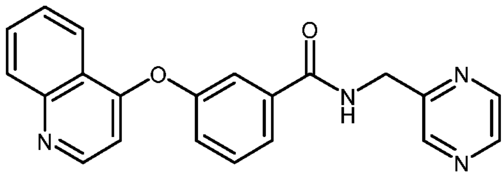

Formula (CCIV)

In a preferred embodiment of the present invention, the compound (C), according to the invention, for use as a kinase inhibitor according to general formula (VIIa-1) is a compound according to formula (CCIV-1) to (CCIV-6) herein below:

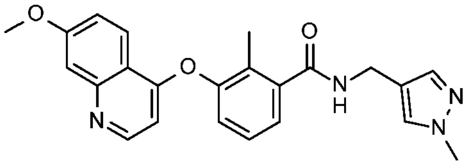

Formula (CCIV-1)

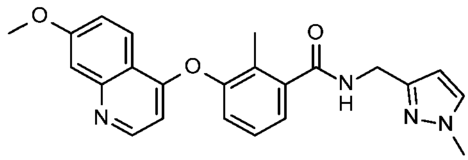

Formula (CCIV-2)

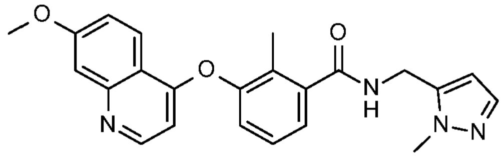

Formula (CCIV-3)

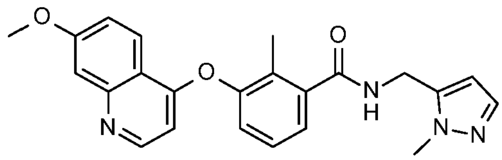

Formula (CCIV-4)

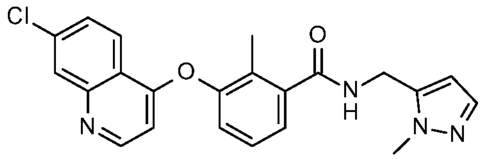

Formula (CCIV-5)

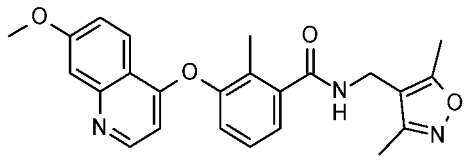

Formula (CCIV-6)

In a preferred embodiment of the present invention, the compound (C), according to the invention, for use as a kinase inhibitor according to general formula (VIIIa-1) is a compound according to formula (CCV) herein below:

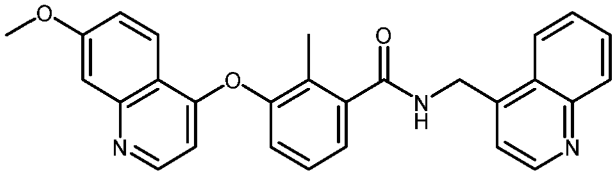

Formula (CCV)

In a preferred embodiment of the present invention, the compound (C), according to the invention, for use as a kinase inhibitor according to general formula (IXa-1) is a compound chosen among those of formulae (CCVI) to (CCXIV) herein below:

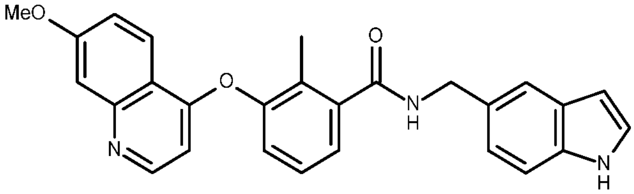

Formula (CCVI)

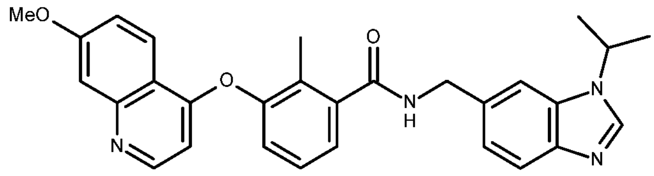

Formula (CCVII)

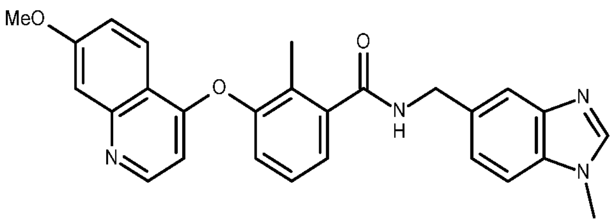

Formula (CCVIII)

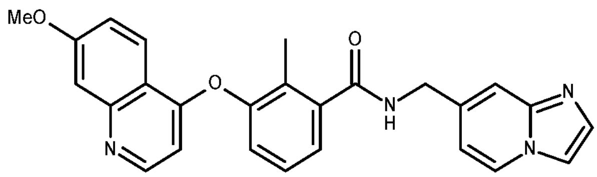

Formula (CCIX)

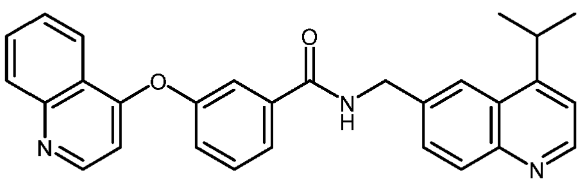

Formula (CCX)

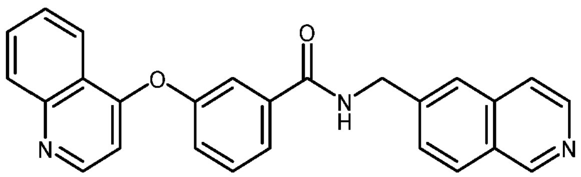

Formula (CCXI)

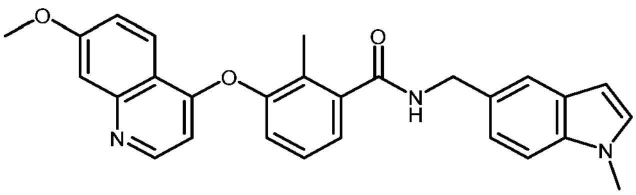

Formula (CCXII)

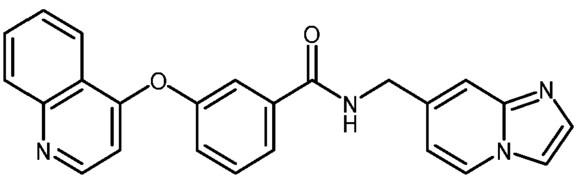

Formula (CCXIII)

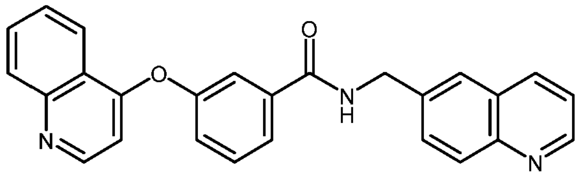

Formula (CCXIV)

In a preferred embodiment of the present invention, the compound (C), according to the invention, for use as a kinase inhibitor according to general formula (XIa-1) is a compound chosen among those of formulae (CCXV) to (CCXIX) herein below:

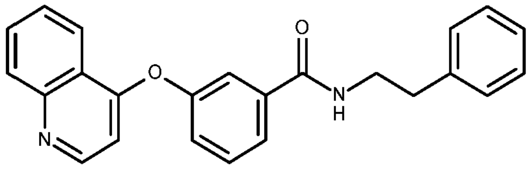

Formula (CCXV)

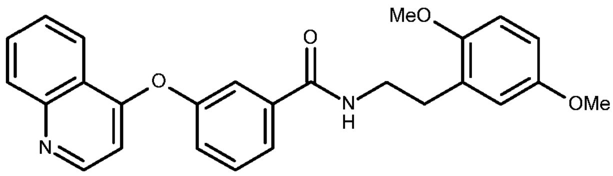

Formula (CCXVI)

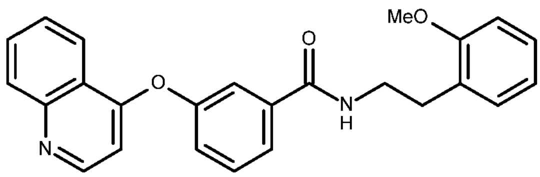

Formula (CCXVII)

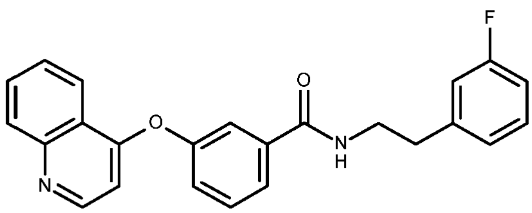

Formula (CCXVIII)

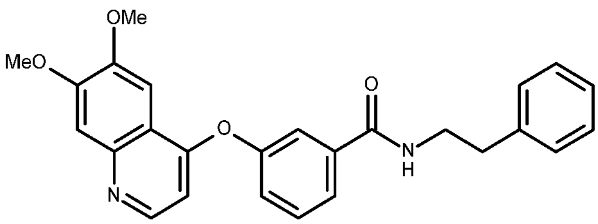

Formula (CCXIX)

In a preferred embodiment of the present invention, the compound (C), according to the invention, for use as a kinase inhibitor according to general formula (XVIIIa-1) is a compound of formula (CCXX) herein below:

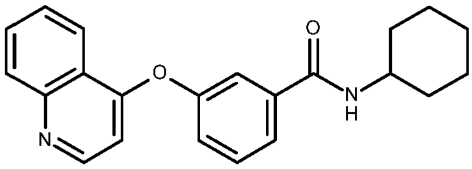

Formula (CCXX)

In a preferred embodiment of the present invention, the compound (C), according to the invention, for use as a kinase inhibitor according to general formula (XXIIIa-1) is a compound chosen among those of formulae (CCXXI) to (CCXXIV) herein below:

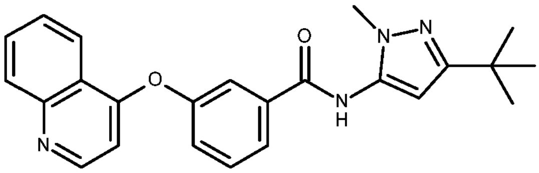

Formula (CCXXI)

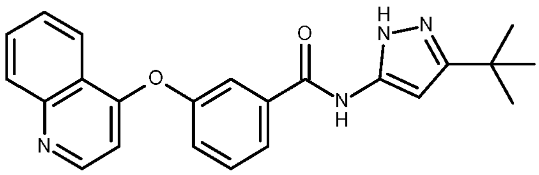

Formula (CCXXII)

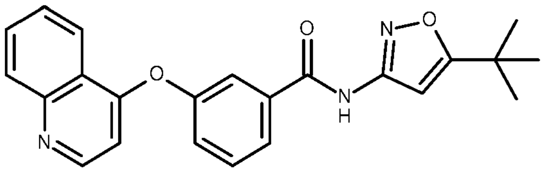

Formula (CCXXIII)

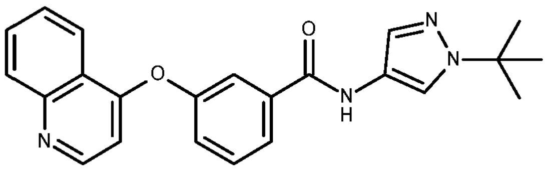

Formula (CCXXIV)

In a preferred embodiment of the present invention, the compound (C), according to the invention, for use as a kinase inhibitor according to general formula (XXIVa-1) is a compound chosen among those of formulae (CCXXV) to (CCXXXII) herein below:

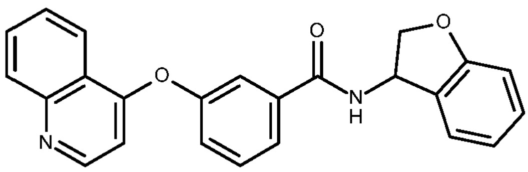

Formula (CCXXV)

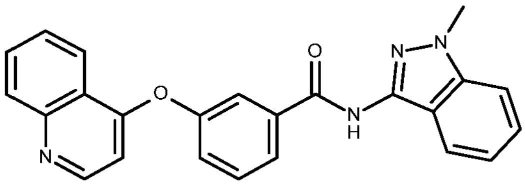

Formula (CCXXVI)

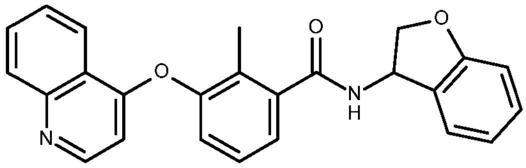

Formula (CCXXVII)

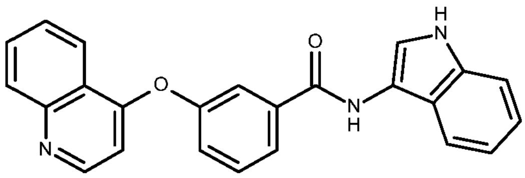

Formula (CCXXVIII)

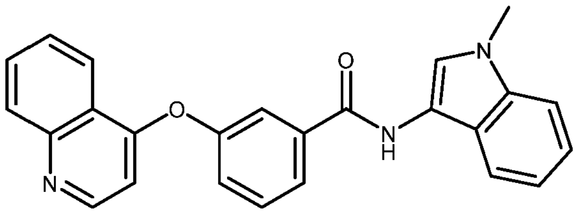

Formula (CCXXIX)

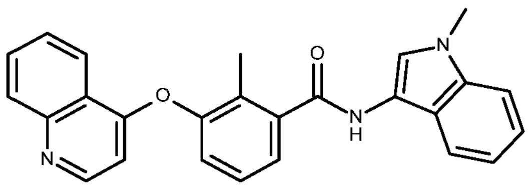

Formula (CCXXX)

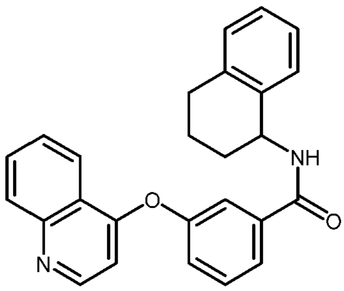

Formula (CCXXXI)

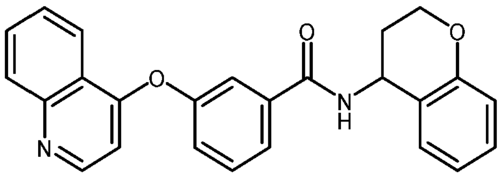

Formula (CCXXXII)

In a preferred embodiment of the present invention, the compound (C), according to the invention, for use as a kinase inhibitor according to general formula (XXVIIa-1) is a compound of formula (CCXXXIII) herein below:

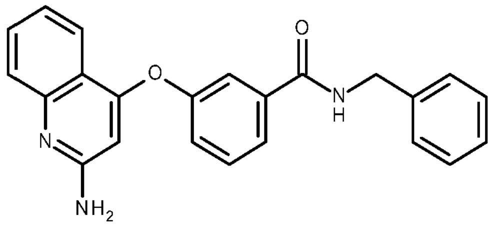

Formula (CCXXXIII)

In a preferred embodiment of the present invention, the compound (C), according to the invention, for use as a kinase inhibitor is a compound chosen among those of formulae (CCXXXIV) to (CCXXXVII) herein below:

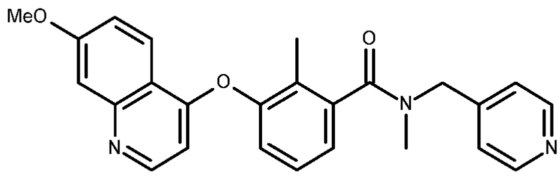

Formula (CCXXXIV)

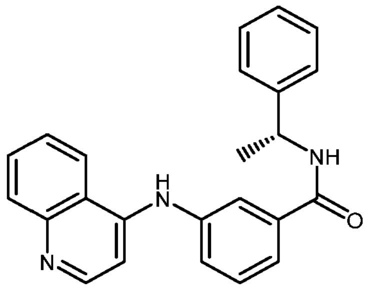

Formula (CCXXXV)

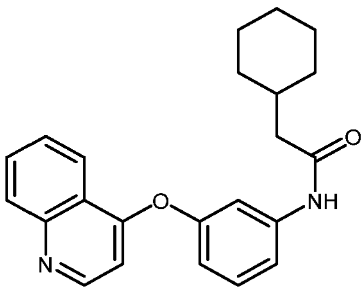

Formula (CCXXXVI)

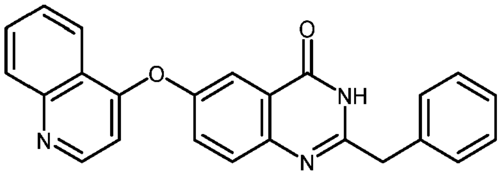

Formula (CCXXXVII)

It is further understood that all definitions and preferences as described for compound (C) above equally apply for this embodiment and all further embodiments, as described below.

As used in the foregoing and hereinafter, the following definitions apply unless otherwise noted.

The term halo - alone or in combination means all halogens, that is, chloro (Cl), bromo (Br), fluoro (F), iodo (I).

The term alkyl - alone or in combination means an alkane-derived radical containing from 1 to 15 carbon atoms, unless otherwise specified, for example $C_{F-G}$ alkyl defines a straight or branched alkyl radical having from F to G carbon atoms, e.g. $C_{1-4}$ alkyl defines a straight or branched alkyl radical having from 1 to 4 carbon atoms such as for example

methyl, ethyl, 1-propyl, 2-propyl, I-butyl, 2-butyl, 2-methyl-1-propyl. An alkyl group may be a straight chain alkyl or branched alkyl. Preferably, straight or branched alkyl groups containing from 1-10, more preferably 1 to 8, even more preferably 1-6 and most preferably 1-4, carbon atoms, such as methyl, ethyl, propyl, isopropyl, butyl, t-butyl and the like. Alkyl also includes a straight chain or branched alkyl group that contains or is interrupted by a cycloalkyl portion. The straight chain or branched alkyl group is attached at any available point to produce a stable compound. Examples of this include, but are not limited to, 4-(isopropyl)-cyclohexylethyl or 2-methyl-cyclopropylpentyl.

The term alkenyl - alone or in combination means a straight or branched hydrocarbon containing 2-15more preferably 2-10, even more preferably 2-8, most preferably 2-4, carbon atoms, unless otherwise specified and at least one, preferably 1-3, more preferably 1-2, most preferably one, carbon to carbon double bond. Examples of alkenyl groups include ethenyl, propenyl, isopropenyl, butenyl, cyclohexenyl, cyclohexenylalkyl and the like. Alkenyl also includes a straight chain or branched alkenyl group that contains or is interrupted by a cycloalkyl portion. Carbon to carbon double bonds may be either contained within a cycloalkyl portion, with the exception of cyclopropyl, or within a straight chain or branched portion.

The term alkynyl - alone or in combination means a straight or branched hydrocarbon containing 2-15 more preferably 2-10, even more preferably 2-8, most preferably 2-4, carbon atoms containing at least one, preferably one, carbon to carbon triple bond. Examples of alkynyl groups include ethynyl, propynyl, butynyl and the like.

The term aryl - alone or in combination means phenyl, naphthyl or anthracenyl optionally carbocyclic fused with a cycloalkyl or heterocyclyl of preferably 5-7, more preferably 5-6, ring members and/or optionally substituted with 1 to 5 groups or substituent. An aryl may be optionally substituted whereby the substituent is attached at one point to the aryl or whereby the the substituent is attached at two points to the aryl to form a bicyclic system e.g. benzodioxole, benzodioxan, benzimidazole.

The term heteroaryl - alone or in combination means a monocyclic aromatic ring structure containing 5 or 6 ring atoms, or a bicyclic aromatic group having 8 to 10 atoms, containing 1-3, heteroatoms independently selected from the group O, S, and N, and optionally substituted with 1 to 5 groups or substituents. Heteroaryl is also intended to include oxidized S or N, such as sulfinyl, sulfonyl and N-oxide of a tertiary ring nitrogen. A carbon or nitrogen atom is the point of attachment of the heteroaryl ring structure such that a stable aromatic ring is retained. More specifically the term heteroaryl includes, but is not limited to, pyridyl, furanyl, thiophenyl, thiazolyl, isothiazolyl, triazolyl, imidazolyl, isoxazolyl, pyrrolyl, pyrazolyl, pyrimidinyl, benzofuranyl, isobenzofuranyl, benzothiazolyl, benzoisothiazolyl, benzotriazolyl, indolyl, isoindolyl, benzoxazolyl, quinolyl, isoquinolyl, benzimidazolyl, benzisoxazolyl, benzothiophenyl, dibenzofuran, and benzodiazepin-2-one-5-yl, and the like.

The term heterocyclyl - alone or in combination is intended to denote a saturated, partially unsaturated or completely unsaturated monocycle, bicycle, or tricycle having 3 to 12 carbon atoms and containing 1 or 2 heteroatoms each independently selected from O, S, P or N, and are optionally benzo fused or fused heteroaryl of 5-6 ring members and/or are optionally substituted as in the case of cycloalkyl. Heterocycyl is also intended to include oxidized S or N, such as sulfinyl, sulfonyl and N-oxide of a tertiary ring nitrogen. The point of attachment is at a carbon or nitrogen atom. In each case the heterocyclyl may be condensed with an aryl to form a bicyclic ring system.

The term cycloalkyl refers to a cyclic or polycyclic alkyl group containing 3 to 7 carbon atoms. Preferably, cycloalkyl groups are monocyclic, bicyclic or tricyclic ring systems of 3-6, ring members per ring, such as cyclopropyl, cyclopentyl, cyclohexyl, adamantyl and the like.

The term aralkyl refers to organic compounds containing an aromatic nucleus to which an alkyl radical is bonded. These alkyl radicals include methyl, ethyl, propyl, butyl, octyl, etc. radicals. The term aralkyl is thus seen to include aralkyl hydrocarbons such as the alkyl benzenes, and the various alkyl naphthalenes. From this definition of the term aralkyl compound it is seen that the term includes compounds such as benzyl, the three isomeric xylyls, the two isomeric trimethyl benzenes, ethyl benzene, p-methyl biphenyl, a-methyl naphthalene, etc.

The present invention further relates to a pharmaceutical composition comprising a carrier, and as active ingredient an effective amount of a compound (C) of formula (IIa)-(IXa) as specified herein, or a compound of any of the subgroups of compounds of formula (IIa-a)-(IXa-a), (IIa-1)-(XXXIIIa-1) and (IIa-a1)-(IXa-a1) as specified herein, and as defined in any one of the embodiments presented herein.

The present invention relates to a compound (C) of formula (IIa)-(IXa) as specified herein, or a compound of any of the subgroups of compounds of formula (IIa-a)-(IXa-a), (IIa-1)-(XXXIIIa-1) and (IIa-a1)-(IXa-a1) as specified herein, and as defined in any one of the embodiments presented herein, for use as a medicament.

The present invention relates to a compound (C) of formula (IIa)-(IXa) as specified herein, or a compound of any of the subgroups of compounds of formula (IIa-a)-(IXa-a), (IIa-1)-(XXXIIIa-1) and (IIa-a1)-(IXa-a1) as specified herein, and as defined in any one of the embodiments presented herein, for use in the treatment of a disease selected from cancer, metabolic disorders (such as diabetes), inflammatory and autoimmune disorders (such as inflammatory bowel diseases, e.g. Crohn's disease and ulcerative colitis, inflammatory pulmonary diseases, rheumatoid arthritis, lupus nephritis, systemic lupus erythematosus and psoriasis and psoriasis arthritis), neurological disorders (such as Alzheimer's disease, Parkinson's disease, multiple sclerosis, Charcot-Marie-Tooth neuropathy, amyotrophic lateral sclerosis and epilepsy),

atherosclerosis and cardiovascular diseases, Sjogren Syndrome, renal allograft rejection, viral induced diseases, circulatory diseases, bone osteolysis and osteoporosis, osteoarthritis, sarcopenia, Langerhans cell histiocytosis, spinal cord injury, endometriosis, asthma and allergic asthma, eye diseases (such as retinopathies, age-related macular degeneration and uveitis) chronic and neuropathic pain, and fibro-proliferative diseases.

It should be noted that the radical positions on any molecular moiety used in the definitions may be anywhere on such moiety as long as it is chemically stable.

Radicals used in the definitions of the variables include all possible isomers unless otherwise indicated. For instance pyridyl includes 2-pyridyl, 3-pyridyl and 4-pyridyl; pentyl includes 1-pentyl, 2-pentyl and 3-pentyl.

When any variable occurs more than one time in any constituent, each definition is independent. Whenever used hereinafter, the term "compounds (C)", or "the present compounds" or similar terms, it is meant to include all the compounds (C) , N-oxides, addition salts, and stereochemically isomeric forms. One embodiment comprises the compounds (C) of formula (IIa)-(IXa) or any subgroup of compounds of formula (IIa-a)-(IXa-a), (IIa-1)-(XXXIIIa-1) and (lla-a1)-(IXa-a1) specified herein, as well as the N-oxides, salts, as the possible stereoisomeric forms thereof. Another embodiment comprises the compounds (C) of formula (IIa)-(IXa), or any subgroup of compounds of formula (IIa-a)-(IXa-a), (IIa-1)-(XXXIIIa-1) and (IIa-a1)-(IXa-a1) specified herein, as well as the salts as the possible stereoisomeric forms thereof.

The compound (C) of formula (IIa)-(IXa) as specified herein, or a compound of any of the subgroups of compounds of formula (IIa-a)-(IXa-a), (IIa-1)-(XXXIlla-1) and (IIa-a1)-(IXa-a1) as specified herein, have several centers of chirality and exist as stereochemically isomeric forms. The term "stereochemically isomeric forms" as used herein defines all the possible compounds made up of the same atoms bonded by the same sequence of bonds but having different three-dimensional structures which are not interchangeable, which the compound (C) of formula (lla)-(IXa) as specified herein, or a compound of any of the subgroups of compounds of formula (IIa-a)-(IXa-a), (IIa-1)-(XXXIIIa-1) and (IIa-a1)-(IXa-a1) as specified herein, may possess.

Unless otherwise mentioned or indicated, the chemical designation of a compound (C) of formula (lla)-(IXa) as specified herein, or a compound of any of the subgroups of compounds of formula (IIa-a)-(IXa-a), (IIa-1)-(XXXIIIa-1) and (IIa-a1)-(IXa-a1) as specified herein, encompasses the mixture of all possible stereochemically isomeric forms, which said compound may possess. Said mixture may contain all diastereomers and/or enantiomers of the basic molecular structure of said compound. All stereochemically isomeric forms of the compounds of the present invention both in pure form or mixed with each other are intended to be embraced within the scope of the present invention.

Pure stereoisomeric forms of the compound (C) of formula (IIa)-(IXa) as specified herein, or a compound of any of the subgroups of compounds of formula (IIa-a)-(IXa-a), (IIa-1)-(XXXIIIa-1) and (IIa-a1)-(IXa-a1) as specified herein, and intermediates as mentioned herein are defined as isomers substantially free of other enantiomeric or diastereomeric forms of the same basic molecular structure of said compounds or intermediates. In particular, the term "stereoisomerically pure" concerns compounds or intermediates having a stereoisomeric excess of at least 80% (i.e. minimum 90% of one isomer and maximum 10% of the other possible isomers) up to a stereoisomeric excess of 100% (i.e. 100% of one isomer and none of the other), more in particular, compounds or intermediates having a stereoisomeric excess of 90% up to 100%, even more in particular having a stereoisomeric excess of 94% up to 100% and most in particular having a stereoisomeric excess of 97% up to 100%. The terms "enantiomerically pure" and "diastereomerically pure" should be understood in a similar way, but then having regard to the enantiomeric excess, and the diastereomeric excess, respectively, of the mixture in question.

Pure stereoisomeric forms of the compounds and intermediates of this invention may be obtained by the application procedures known in the art. For instance, enantiomers may be separated from each other by the selective crystallization of their diastereomeric salts with optically active acids or bases. Examples thereof are tartaric acid, dibenzoyltartaric acid, ditoluoyltartaric acid and camphorsulfonic acid. Alternatively, enantiomers may be separated by chromatographic techniques using chiral stationary phases. Said pure stereochemically isomeric forms may also be derived from the corresponding pure stereochemically isomeric forms of the appropriate starting materials, provided that the reaction occurs stereospecifically. Preferably, if a specific stereoisomer is desired, said compound will be synthesized by stereospecific methods of preparation. These methods will advantageously employ enantiomerically pure starting materials.

The diastereomeric racemates of the compounds (C) of formula (IIa)-(IXa) as specified herein, or a compound of any of the subgroups of compounds of formula (IIa-a)-(IXa-a), (IIa-1)-(XXXIIIa-1) and (IIa-a1)-(IXa-a1) as specified herein, can be obtained separately by conventional methods. Appropriate physical separation methods that may advantageously be employed are, for example, selective crystallization and chromatography, e.g. column chromatography.

For some of the compound (C) of formula (IIa)-(IXa) as specified herein, or a compound of any of the subgroups of compounds of formula (IIa-a)-(IXa-a), (Ia-1)-(XXXIIIa-1) and (IIa-a1)-(IXa-a1) as specified herein, N-oxides, salts, solvates, and the intermediates used in the preparation thereof, the absolute stereochemical configuration was not experimentally determined.

A person skilled in the art is able to determine the absolute configuration of such compounds using art-known methods such as, for example, X-ray diffraction.

The present invention is also intended to include all isotopes of atoms occurring on the present to a compound (C) of formula (IIa)-(IXa) as specified herein, or a compound of any of the subgroups of compounds of formula (IIa-a)-(IXa-a), (IIa-1)-(XXXIIIa-1) and (IIa-a1)-(IXa-a1) as specified herein. Isotopes include those atoms having the same atomic number but different mass numbers. By way of general example and without limitation, isotopes of hydrogen include tritium and deuterium. Isotopes of carbon include C-13 and C-14.

For therapeutic use, salts of the compound (C) of formula (IIa)-(IXa) as specified herein, or a compound of any of the subgroups of compounds of formula (IIa-a)-(IXa-a), (IIa-1)-(XXXIIIa-1) and (IIa-a1)-(IXa-a1) as specified herein, are those wherein the counter-ion is pharmaceutically acceptable, which salts can be referred to as pharmaceutically acceptable acid and base addition salts. However, salts of acids and bases that are non-pharmaceutically acceptable may also find use, for example, in the preparation or purification of a pharmaceutically acceptable compound. All salts, whether pharmaceutically acceptable or not, are included within the ambit of the present invention.

The pharmaceutically acceptable acid and base addition salts as mentioned hereinabove are meant to comprise the therapeutically active nontoxic acid and base addition salt forms that the compounds (C) of formula (IIa)-(IXa) as specified herein, or a compound of any of the subgroups of compounds of formula (IIa-a)-(IXa-a), (IIa-1)-(XXXIIIa-1) and (IIa-a1)-(IXa-a1) as specified herein, are able to form. The pharmaceutically acceptable acid addition salts can conveniently be obtained by treating the base form with such appropriate acid in an anion form. Appropriate anions comprise, for example, trifluoroacetate, acetate, benzenesulfonate , benzoate, bicarbonate, bitartrate, bromide, calcium edetate, camsyiate, carbonate, chloride, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isethionate, lactate, lactobionate, malate, maleate, mandelate, mesylate, methylbromide, methylnitrate, methylsulfate, mucate, napsylate, nitrate, pamoate (embonate), pantothenate, phosphate/diphosphate, polygalacturonate, salicylate, stearate, subacetate, succinate, sulfate, tannate, tartrate, teoclate, triethiodide, and the like. The counterion of choice can be introduced using ion exchange resins. Conversely said salt forms can be converted by treatment with an appropriate base into the free base form.

The compounds (C) of formula (IIa)-(IXa) as specified herein, or a compound of any of the subgroups of compounds of formula (IIa-a)-(IXa-a), (IIa-1)-(XXXIIIa-1) and (IIa-a1)-(IXa-a1) as specified herein, containing an acidic proton may also be converted into their nontoxic metal or amine addition salt forms by treatment with appropriate organic and inorganic bases in a cation form. Appropriate basic salts comprise those formed with organic cations such as benzathine, chloroprocaine, choline, diethanolamine, ethylenediamine, meglumine, procaine, and the like; and those formed with metallic cations such as aluminum, calcium, lithium, magnesium, potassium, sodium, zinc, and the like. Conversely said salt forms can be converted by treatment with an appropriate acid into the free form.

The term addition salt as used hereinabove also comprises the solvates which the compound (C) of formula (IIa)-(IXa) as specified herein, or a compound of any of the subgroups of compounds of formula (IIa-a)-(IXa-a), (IIa-1)-(XXXIIIa-1) and (IIa-a1)-(IXa-a1) as specified herein, as well as the salts thereof, are able to form. Such solvates are for example hydrates, alcoholates and the like.

The N-oxide forms of the present compound (C) of formula (IIa)-(IXa) as specified herein, or a compound of any of the subgroups of compounds of formula (IIa-a)-(IXa-a), (IIa-1)-(XXXIIIa-1) and (IIa-a1)-(IXa-a1) as specified herein, are meant to comprise the compounds of formula (I) wherein one or several nitrogen atoms are oxidized to the so-called N-oxide.

It will be appreciated that the compound (C) of formula (IIa)-(IXa) as specified herein, or a compound of any of the subgroups of compounds of formula (IIa-a)-(IXa-a), (IIa-1)-(XXXIIIa-1) and (IIa-a1)-(IXa-a1) as specified herein, may have metal binding, chelating, complex forming properties and therefore may exist as metal complexes or metal chelates. Such metalated derivatives of the compound (C) of formula (IIa)-(IXa) as specified herein, or a compound of any of the subgroups of compounds of formula (IIa-a)-(IXa-a), (IIb)-(IXb), (IIc)-(IXc), (IIa-1)-(XXXIIIa-1) and (IIa-a1)-(IXa-a1) as specified herein, are intended to be included within the scope of the present invention.

Some of the compound (C) of formula (IIa)-(IXa) as specified herein, or a compound of any of the subgroups of compounds of formula (IIa-a)-(IXa-a), (IIa-1)-(XXXIIIa-1) and (IIa-a1)-(IXa-a1) as specified herein, may also exist in their tautomeric form. Such forms although not explicitly indicated in the above formula are intended to be included within the scope of the present invention.

In a further aspect, the present invention concerns a pharmaceutical composition comprising a therapeutically effective amount of a compound (C) of formula (IIa)-(IXa) as specified herein, or a compound of any of the subgroups of compounds of formula (IIa-a)-(IXa-a), (IIa-1)-(XXXIIIa-1) and (IIa-a1)-(IXa-a1) as specified herein, and a pharmaceutically acceptable carrier. A therapeutically effective amount in this context is an amount sufficient to prophylactically act against, to stabilize or reduce illnesses mediated by protein kinases in ill subjects or subjects being at risk of being ill, in particular a protein kinase selected from the group consisting of CSF1R, FLT3, Kit, PDGFRB (PDGFR beta), PDGFRA (PDGFR alfa), ABL1, ACVR1B (ALK4), AKT1 (PKB alpha), AMPK A1/B1/G1, AURKA (Aurora A), BTK, CDK1/cyclin B, CHEK1 (CHK1), CSNK1G2 (CK1 gamma 2), CSNK2A1 (CK2 alpha 1), DYRK3, EGFR (ErbB1), EPHA2, ERBB2 (HER2), FGFR1, FRAP1 (mTOR), GSK3B (GSK3 beta), IGF1R, IKBKB (IKK beta), INSR, IRAK4, JAK3, KDR (VEGFR2), LCK, MAP2K1 (MEK1),

MAP4K4 (HGK), MAPK1 (ERK2), MAPK14 (p38 alpha), MAPK3 (ERK1), MAPK8 (JNK1), MARK2, MET (cMet), NEK1, PAK4, PHKG2, PIM1, PLK1, PRKACA (PKA), PRKCB1 (PKC beta I), ROCK1, RPS6KA3 (RSK2), RPS6KB1 (p70S6K), SRC, SYK, and TEK (Tie2). Preferably, the protein kinase is selected from the group consisting of CSF1R, FLT3, Kit, PDGFRB (PDGFR beta), PDGFRA (PDGFR alpha).

Examples of illnesses mediated by protein kinases include in particular of illnesses mediated by protein kinases include in particular cancer, metabolic disorders (such as diabetes), inflammatory and autoimmune disorders (such as inflammatory bowel diseases, e.g. Crohn's disease and ulcerative colitis, inflammatory pulmonary diseases, rheumatoid arthritis, lupus nephritis, systemic lupus erythematosus and psoriasis and psoriasis arthritis), neurological disorders (such as Alzheimer's disease, Parkinson's disease, multiple sclerosis, Charcot-Marie-Tooth neuropathy, amyotrophic lateral sclerosis and epilepsy), atherosclerosis and cardiovascular diseases, Sjogren Syndrome, renal allograft rejection, viral induced diseases, circulatory diseases, bone osteolysis and osteoporosis, osteoarthritis, sarcopenia, Langerhans cell histiocytosis, spinal cord injury, endometriosis, asthma and allergic asthma, eye diseases (such as retinopathies, age-related macular degeneration and uveitis) chronic and neuropathic pain, and fibro-proliferative diseases.

In a process of preparing a pharmaceutical composition as specified herein, the process comprises intimately mixing a pharmaceutically acceptable carrier with a therapeutically effective amount of a compound (C) of formula (IIa)-(IXa), as specified herein, or of a compound of any of the subgroups of compounds of formula (IIa-a)-(IXa-a), (IIa-1)-(XXXIIIa-1) and (IIa-a1)-(IXa-a1) as specified herein.

Therefore, the compound (C) of formula (IIa)-(IXa) as specified herein, or a compound of any of the subgroups of compounds of formula (IIa-a)-(IXa-a), (IIa-1)-(XXXIIIa-1) and (IIa-a1)-(IXa-a1) as specified herein, may be formulated into various pharmaceutical forms for administration purposes. As appropriate compositions there may be cited all compositions usually employed for systemically administering drugs. To prepare the pharmaceutical compositions of this invention, an effective amount of the particular compound, optionally in addition salt form or metal complex, as the active ingredient is combined in intimate admixture with a pharmaceutically acceptable carrier, which carrier may take a wide variety of forms depending on the form of preparation desired for administration. These pharmaceutical compositions are desirable in unitary dosage form suitable, particularly, for administration orally, rectally, percutaneously, or by parenteral injection. For example, in preparing the compositions in oral dosage form, any of the usual pharmaceutical media may be employed such as, for example, water, glycols, oils, alcohols and the like in the case of oral liquid preparations such as suspensions, syrups, elixirs, emulsions and solutions; or solid carriers such as starches, sugars, kaolin, lubricants, binders, disintegrating agents and the like in the case of powders, pills, capsules, and tablets.

Because of their ease in administration, tablets and capsules represent the most advantageous oral dosage unit forms, in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. Also included are solid form preparations, which are intended to be converted, shortly before use, to liquid form preparations. In the compositions suitable for percutaneous administration, the carrier optionally comprises a penetration enhancing agent and/or a suitable wetting agent, optionally combined with suitable additives of any nature in minor proportions, which additives do not introduce a significant deleterious effect on the skin.

The compound (C) of formula (IIa)-(IXa) as specified herein, or a compound of any of the subgroups of compounds of formula (IIa-a)-(IXa-a), (IIa-1)-(XXXIIIa-1) and (IIa-a1)-(IXa-a1) as specified herein, of the present invention may also be administered via oral inhalation or insufflation by means of methods and formulations employed in the art for administration via this way. Thus, in general the compound (C) of formula (IIa)-(IXa) as specified herein, or a compound of any of the subgroups of compounds of formula (IIa-a)-(IXa-a), (IIa-1)-(XXXIIIa-1) and (IIa-a1)-(IXa-a1) as specified herein, may be administered to the lungs in the form of a solution, a suspension or a dry powder, a solution being preferred. Any system developed for the delivery of solutions, suspensions or dry powders via oral inhalation or insufflation are suitable for the administration of the present compounds.

Thus, in the present invention a pharmaceutical composition can be adapted for administration by inhalation or insufflation through the mouth comprising a compound (C) of formula (IIa)-(IXa) as specified herein, or a compound of any of the subgroups of compounds of formula (IIa-a)-(IXa-a), (IIa-1)-(XXXIIIa-1) and (IIa-a1)-(IXa-a1) as specified herein, and a pharmaceutically acceptable carrier. Preferably, the compounds of the present invention are administered via inhalation of a solution in nebulized or aerosolized doses.

It is especially advantageous to formulate the aforementioned pharmaceutical compositions in unit dosage form for ease of administration and uniformity of dosage. Unit dosage form as used herein refers to physically discrete units suitable as unitary dosages, each unit containing a predetermined quantity of active ingredient calculated to produce the desired therapeutic effect in association with the required pharmaceutical carrier. Examples of such unit dosage forms are tablets (including scored or coated tablets), capsules, pills, suppositories, powder packets, wafers, injectable solutions or suspensions and the like, and segregated multiples thereof.

The compound (C) of formula (IIa)-(IXa) as specified herein, or a compound of any of the subgroups of compounds of formula (IIa-a)-(IXa-a), (IIa-1)-(XXXIIIa-1) and (IIa-a1)-(IXa-a1) as specified herein, show kinase inhibition properties. Illnesses and diseases treatable using the compounds of the present invention include protein kinase mediated diseases like cancer, metabolic disorders (such as diabetes), inflammatory and autoimmune disorders (such as inflammatory bowel diseases, e.g. Crohn's disease and ulcerative colitis, inflammatory pulmonary diseases, rheumatoid arthritis, lupus nephritis, systemic lupus erythematosus and psoriasis and psoriasis arthritis), neurological disorders (such as Alzheimer's disease, Parkinson's disease, multiple sclerosis, Charcot-Marie-Tooth neuropathy, amyotrophic lateral sclerosis and epilepsy), atherosclerosis and cardiovascular diseases, Sjogren Syndrome, renal allograft rejection, viral induced diseases, circulatory diseases, bone osteolysis and osteoporosis, osteoarthritis, sarcopenia, Langerhans cell histiocytosis, spinal cord injury, endometriosis, asthma and allergic asthma, eye diseases (such as retinopathies, age-related macular degeneration and uveitis) chronic and neuropathic pain, and fibro-proliferative diseases. Many of the compounds of this invention may show a favourable pharmacokinetic profile and have attractive properties in terms of bioavailability, including an acceptable half-life, AUC (area under the curve) and peak values and lacking unfavourable phenomena such as insufficient quick onset and tissue retention.

The combinations of the present invention may be used as medicaments. Said use as a medicine comprises the systemic administration to ill subjects of an amount effective to combat the conditions associated with the illnesses. Consequently, the combinations of the present invention can be used, without forming part of the invention, in the manufacture of a medicament useful for treating, preventing or combating illness or disease associated with protein kinases including cancer, metabolic disorders (such as diabetes), inflammatory and autoimmune disorders (such as inflammatory bowel diseases, e.g. Crohn's disease and ulcerative colitis, inflammatory pulmonary diseases, rheumatoid arthritis, lupus nephritis, systemic lupus erythematosus and psoriasis and psoriasis arthritis), neurological disorders (such as Alzheimer's disease, Parkinson's disease, multiple sclerosis, Charcot-Marie-Tooth neuropathy, amyotrophic lateral sclerosis and epilepsy), atherosclerosis and cardiovascular diseases, Sjogren Syndrome, renal allograft rejection, viral induced diseases, circulatory diseases, bone osteolysis and osteoporosis, osteoarthritis, sarcopenia, Langerhans cell histiocytosis, spinal cord injury, endometriosis, asthma and allergic asthma, eye diseases (such as retinopathies, age-related macular degeneration and uveitis) chronic and neuropathic pain, and fibro-proliferative diseases.

The term "therapeutically effective amount" as used herein means that amount of active compound or component or pharmaceutical agent that elicits the biological or medicinal response in a tissue, system, animal or human that is being sought, in the light of the present invention, by a researcher, veterinarian, medical doctor or other clinician, which includes alleviation of the symptoms of the disease being treated.

In general it is contemplated that an antiviral effective daily amount would be from 0.01 mg to 1500 mg daily, more preferably from 0.1 mg to 50 mg daily. It may be appropriate to administer the required dose as one, two, three, four or more (sub-)doses at appropriate intervals throughout the day. Said (sub-)doses may be formulated as unit dosage forms, for example, containing 1 to 1000 mg, and in particular 5 to 200 mg of active ingredient per unit dosage form.

The exact dosage and frequency of administration depends on the particular to a compound (C) of formula (IIa)-(IXa) or the particular compound of any of the subgroups of compounds of formula (IIa-a)-(IXa-a), (IIa-1)-(XXXIIIa-1) and (IIa-a1)-(IXa-a1) as specified herein, used, the particular condition being treated, the severity of the condition being treated, the age, weight, sex, extent of disorder and general physical condition of the particular patient as well as other medication the individual may be taking, as is well known to those skilled in the art. Furthermore, it is evident that said effective daily amount may be lowered or increased depending on the response of the treated subject and/or depending on the evaluation of the physician prescribing the compounds of the instant invention. The effective daily amount ranges mentioned hereinabove are therefore only guidelines.

## Examples

### Example 1: **General procedure for the synthesis of analogues 3 - 76, 205-210 and 247-265**

The following procedures illustrate the preparation of analogues **3-76, 205-210** and **247-265.** The synthetic scheme is outlined below. The synthesis starts with the coupling of 4-chloroquinoline derivative with the appropriate ethyl or methyl-3-hydroxybenzoate derivative in Method A. In Method B, the carboxylic acid is prepared from the deesterification of the ethyl or methyl-3-hydroxybenzoate derivative. In Method C, D or E, the preparation of the 3-(4-quinolyloxy) benzamide from the corresponding benzoic acid via activation by either DMAP (Method C, compounds 3-67 and 205-210), HOBt (Method D, compounds 68-75) or 2-chloro-1-methyl-pyridinium iodide (Method E, compound 76) followed by reaction of the appropriate amine $R_8NH_2$. Examples of the structures of the final compounds can be found in Table 1.

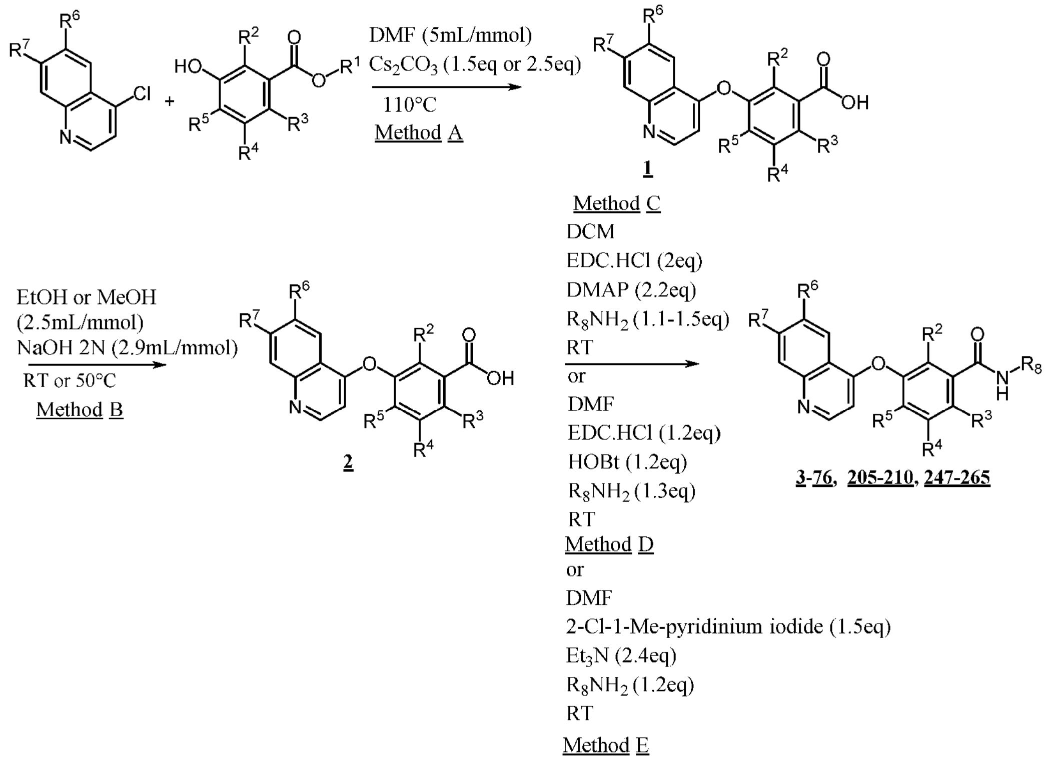


**Method A:** To a solution of ethyl or methyl-3-hydroxybenzoate (1 equiv.) in DMF (5 mL/mmol) under nitrogen was added as solid cesium carbonate (2.5 equiv.) followed by 4-chloroquinoline (1 equiv.). The reaction mixture was stirred at 110°C until completion (from 2h to overnight). A saturated solution of $NH_4Cl$ was added and the aqueous layer was extracted with EtOAc. The organic layer was dried over $Na_2SO_4$, filtered and concentrated under reduced pressure. The crude was purified by flash column chromatography on Biotage (Cyclohexane/EtOAc: 0 to 50%) to give the expected intermediate **1.**

The following compound **1a** is an example illustrating Method A:

Preparation of ethyl 3-(4-quinolyloxy)benzoate **(1a):**

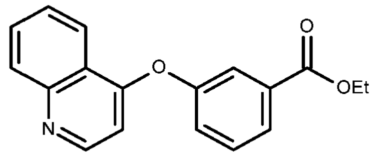

Intermediate **1a** was synthesized from ethyl-3-hydroxybenzoate (6.02 mmol) and 4-chloroquinoline (6.02 mmol) as a colorless oil in 77% yield according to the general method A.

The following table illustrates intermediates **1** prepared from method A:

| Intermediate | Structure | Synthesis procedure |
|---|---|---|
| Compound **1a** | 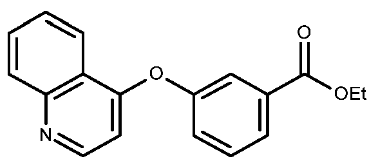 | Method A |

(continued)

| Intermediate | Structure | Synthesis procedure |
|---|---|---|
| Compound **1b** | 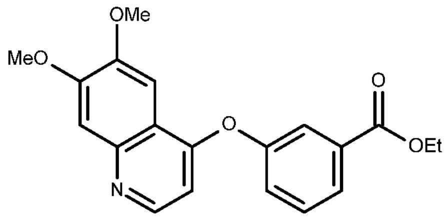 | Method A |
| Compound **1c** | 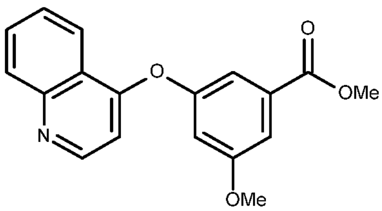 | Method A |
| Compound **1d** | 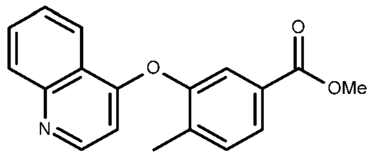 | Method A |
| Compound **1e** | 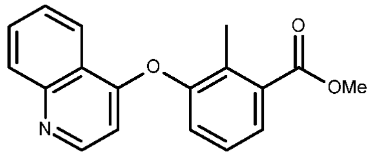 | Method A |
| Compound **1f** | 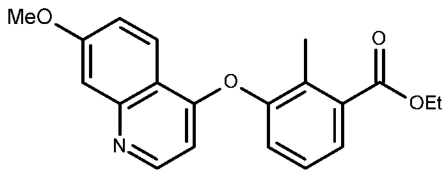 | Method A |
| Compound **1g** | 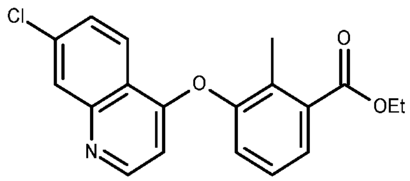 | Method A |
| Compound **1h** | 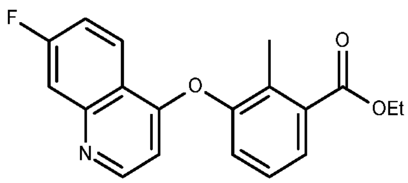 | Method A |

**Method** B: To a solution of appropriate intermediate **1** (1 equiv.) in EtOH or MeOH (2.5 mL/mmol) was added a solution of NaOH 2N (2.9 mL/mmol). The reaction mixture was stirred at 50°C or rt until completion. EtOH or MeOH was removed and the crude was acidified with HCl 1N until pH = 2-3. The precipitate was filtered-off, washed with water and dried over $P_2O_5$ in vacuum desiccators to give the expected intermediate **2.**

The following compound **2a** is an example illustrating Method B:

Preparation of 3-(4-quinolyloxy)benzoic acid (2a):

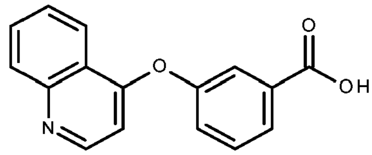

Intermediate **2a** was synthesized from intermediate **1a** (5.28 mmol) as a white powder in 89% yield according to the general method B.

The following table illustrates intermediates **2** prepared from method B:

| Intermediate | Structure | Synthesis procedure |
|---|---|---|
| Compound **2a** | 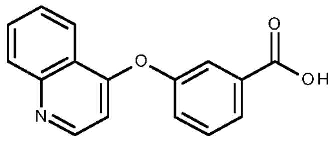 | Method B |
| Compound **2b** | 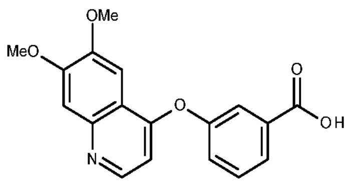 | Method B |
| Compound **2c** | 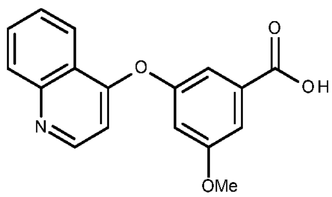 | Method B |
| Compound **2d** | 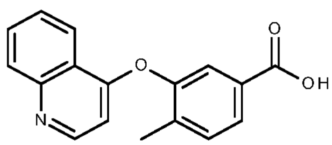 | Method B |
| Compound **2e** | 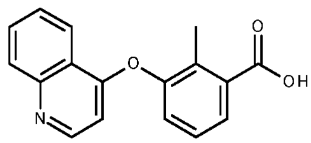 | Method B |
| Compound **2f** | 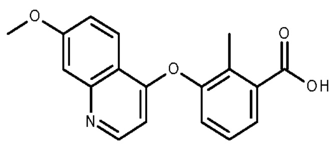 | Method B |
| Compound **2g** | 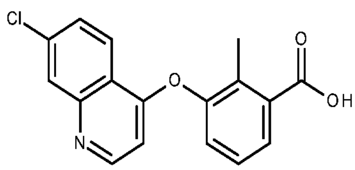 | Method B |

(continued)

| Intermediate | Structure | Synthesis procedure |
|---|---|---|
| Compound **2h** | 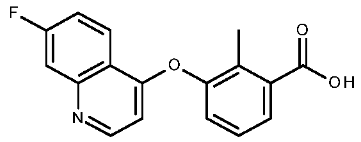 | Method B |

**Method C:** To a suspension of appropriate intermediate **2** (1 equiv.) in DCM (10 mL/mmol) under nitrogen were added DMAP (2.2 equiv.), EDC.HCl (2 equiv.) and appropriate amine (1.1-1.5 equiv.). The reaction mixture was stirred at room temperature until completion (1h-overnight). The reaction mixture was diluted with DCM and washed twice with a saturated solution of $NH_4Cl$. The organic layer was dried over $Na_2SO_4$, filtered and concentrated under reduced pressure. The crude was purified by flash column chromatography on Biotage (DCM/MeOH: 0 to 10%) and reverse phase chromatography ($H_2O$/MeOH: 0 to 100%) to give the expected compound.

The following compound **32** is an example illustrating Method C:

Preparation of 3-[(7-methoxy-4-quinolyl)oxy]-2-methyl-N-(4-pyridylmethyl)benzamide **(32):**

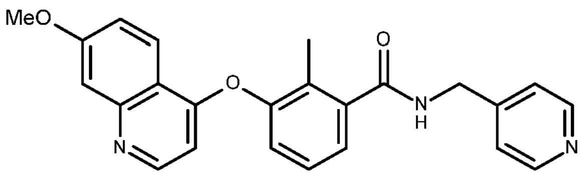

Compound **32** was synthesized from intermediate **2e** (0.30 mmol) and 4-(aminomethyl)pyridine (0.33 mmol) as a white solid in 81% yield (97 mg) according to the general method C. $^1$H NMR (400 MHz, DMSO-$d_6$) δ (ppm): 9.05 (t, *J* = 5.86 Hz, 1H), 8.61 (d, *J* = 5.14 Hz, 1H), 8.54-8.52 (m, 2H), 8.27 (d, *J* = 9.07 Hz, 1H), 7.45-7.43 (m, 3H), 7.36-7.31 (m, 4H), 6.32 (d, *J* = 5.15 Hz, 1H), 4.49 (d, *J* = 6.07 Hz, 2H), 3.95 (s, 3H), 2.15 (s, 3H) ; MS (ESI, $EI^+$): *m/z= 400.20* ($MH^+$).

**Method D:** To a suspension of appropriate intermediate 2 (1 equiv.) in DMF (5 mL/mmol) under nitrogen were added EDC.HCl (1.2 equiv.), HOBt (1.2 equiv.) and the reaction mixture was stirred at room temperature during 5 minutes. Appropriate amine (1.3 equiv.) was added and the reaction mixture was stirred at room temperature overnight (or all the week-end). The reaction mixture was diluted with water and extracted with ethyl acetate. The organic layers were dried over $Na_2SO_4$, filtered and concentrated under reduced pressure. The crude was purified by flash column chromatography on Biotage (DCM/MeOH: 0 to 5%) and reverse phase chromatography ($H_2O$/MeOH: 0 to 100%) to give the expected compound.

The following compound **68** is an example illustrating Method D:

Preparation of 3-methoxy-N-[(2-methoxyphenyl)methyl]-5-(4-quinolyloxy)benzamide **(68):**

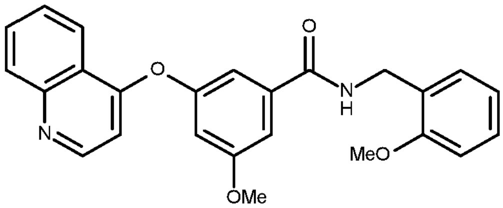

Compound **68** was synthesized from intermediate **2c** (0.237 mmol) and 2-methoxybenzylamine (0.308 mmol) as a white powder in 56% yield (55 mg) according to the general method D. $^1$H NMR (400 MHz, $CDCl_3$) δ (ppm): 8.70 (d, *J* = 5.13 Hz, 1H), 8.31 (dd, *J* = 0.95 Hz and *J* = 8.40 Hz, 1H), 8.11 (d, *J* = 8.44 Hz, 1H), 7.79-7.75 (m, 1H), 7.60-7.56 (m, 1H), 7.34 (dd, *J* = 1.55 Hz and *J* = 7.38 Hz, 1H), 7.30-7.26 (m, 2H), 7.11-7.10 (m, 1H), 6.95-6.91 (m, 1H), 6.89-6.87 (m, 1H), 6.85-6.84 (m, 1H), 6.64 (brs, 1H), 6.61 (d, *J* = 5.18 Hz, 1H), 4.62 (d, *J* = 5.74 Hz, 2H), 3.85 (s, 3H), 3.84 (s, 3H) ; MS (ESI, $EI^+$): *m/z=*

415.4 ($MH^+$).

**Method E:** To a suspension of appropriate intermediate **2** (1 equiv.) in DMF (10 mL/mmol) under nitrogen were added triethylamine (2.4 equiv.), appropriate amine (1.2 equiv.) followed by 2-chloro-1-methylpyridinium iodide (1.5 equiv.). The reaction mixture was stirred at room temperature during 5 minutes. Appropriate amine (1.3 equiv.) was added and the reaction mixture was stirred at room temperature for 2 hours. The reaction mixture was diluted with DCM and the organic layer was washed with brine. The aqueous layer was extracted once with DCM. The combined organic layers were dried over $Na_2SO_4$, filtered and concentrated under reduced pressure. The crude was purified by flash column chromatography on Biotage (DCM/MeOH: 0 to 5%) and reverse phase chromatography ($H_2O$/MeOH: 0 to 100%) to give the expected compound. The following compound **76** is an example illustrating Method E:

Preparation of N-(1-methylindazol-3-yl)-3-(4-quinolyloxy)benzamide (76):

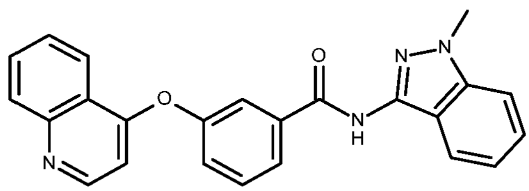

Compound **76** was synthesized from intermediate **2a** (0.186 mmol) and 1-methyl-1,1-indazol-3-amine (0.226 mmol) as a white powder in 65% yield (48 mg) according to the general method E. $^1$H NMR (400 MHz, DMSO-$d_6$) δ (ppm): 10.9 (s, 1H), 8.74 (d, *J* = 5.12 Hz, 1H), 8.34 (dd, *J* = 8.22 Hz and *J* = 0.8 Hz, 1H), 8.07-7.97 (m, 3H), 7.87-7.83 (m, 1H), 7.74-7.67 (m, 3H), 7.60-7.55 (m, 2H), 7.42-7.37 (m, 1H), 7.11-7.07 (m, 2H), 6.75 (d, *J* = 5.23 Hz, 1H), 4.00 (s, 3H) ; MS (ESI, $EI^+$): *m*/*z*= 395.10 ($MH^+$).

**Example 2: General procedure for the preparation of analogues 84-110 and 211**

The following procedures illustrate the preparation of analogues **84-110** and **211,** the synthetic scheme is outline below. The synthesis starts with the amidation of an appropriate 3-hydroxybenzoic acid with an appropriate amine step (hereinafter Method F, G or H) via activation via either HOBt, EDC.HCl and TEA (Method F), PyBOP and DIEA (Method G) or EDC.HCl (Method H). In Method I, the final step, the preparation of the 3-(4-quinolyloxy)benzamide analogues **84-110** and **211** from the corresponding 4-chloroquinoline derivative was achieved. Examples of the structures of the final compounds can be found in table 1.

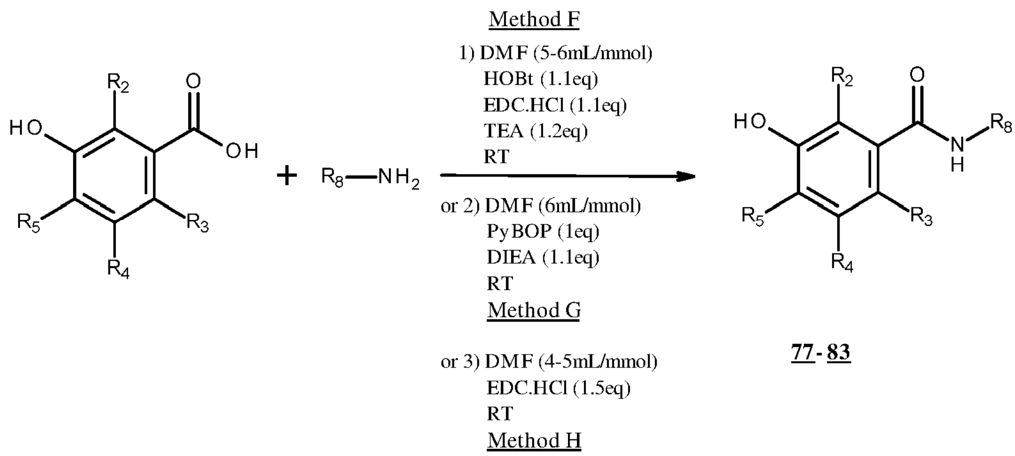


77-83

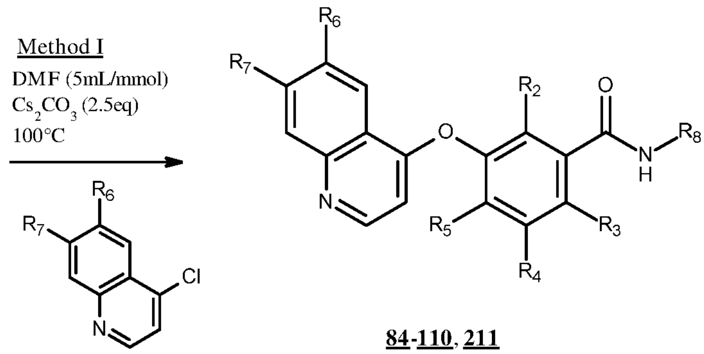


84-110, 211

**Method F:** To a solution of appropriate carboxylic acid (1 equiv.) in DMF (5-6 mL/mmol) under nitrogen were added appropriate amine (1.3 equiv.), triethylamine (1.2 equiv.), HOBt (1.1 equiv.) then EDC.HCl (1.1 equiv.). The reaction mixture was stirred at room temperature overnight (or all the week-end). The mixture was diluted with ethyl acetate and washed with water three times. The organic layer was dried over $Na_2SO_4$ and concentrated under reduced pressure. The crude was purified by flash chromatography on silica gel (DCM/MeOH: 0 to 2.5%) or triturated in $Et_2O$ to give the expected intermediate. The following compound **77a** is an example illustrating Method F:

Preparation of 4-fluoro-3-hydroxy-N-[(2-methoxyphenyl)methyl]benzamide **(77a):**

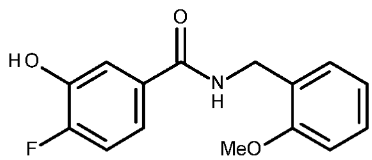

Intermediate **77a** was synthesized from 4-fluoro-3-hydroxy-benzoic acid (0.641 mmol) and 2-methoxybenzylamine (0.833 mmol) as a yellow oil in 94% yield according tothe general method F.

**Method G:** To a stirred solution of appropriate carboxylic acid (1 equiv.) in DMF (6 mL/mmol) under nitrogen were added DIEA (1.1 equiv.), PyBOP (1 equiv.) and appropriate amine (1.5 equiv.). The reaction mixture was stirred at room temperature during 2 hours. The solvent was removed and the crude was co-evaporated with toluene under reduced pressure. The oil was purified by reverse-phase chromatography ($H_2O$/MeOH: 0 to 100%) to give the expected intermediate.

The following compound **77g** is an example illustrating Method G:

Preparation of 4-fluoro-3-hydroxy-N-(4-pyridylmethyl)benzamide **(77a):**

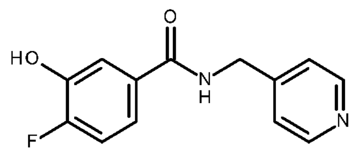

Intermediate **77g** was synthesized from 4-fluoro-3-hydroxy-benzoic acid (3.0 mmol) and 4-(aminomethyl) pyridine (4.50 mmol) as a white powder in 35% yield according to the general method G.

**Method H:** To a stirred solution of appropriate carboxylic acid (1 equiv.) in DMF (4-5 mL/mmol) under nitrogen were added appropriate amine (1.2 equiv.) and EDC.HCl (1.5 equiv.). The reaction mixture was stirred at room temperature overnight. The solvent was removed and water was added. The mixture was extracted with ethyl acetate and the organic layer was dried over $Na_2SO_4$, filtered and concentrated under reduced pressure. The crude was purified by flash chromatography (DCM/MeOH: 0 to 5%) to give the expected intermediate.

The following compound **81e** is an example illustrating Method H:

Preparation of 3-hydroxy-2-methyl-N-(4-pyridylmethyl)benzamide **(81e):**

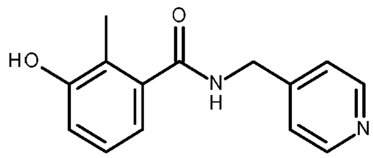

Intermediate **81e** was synthesized from 2-methyl-3-hydroxybenzoic acid (2.00 mmol) and 4-(aminomethyl) pyridine (2.00 mmol) as a white powder in 80% yield according to the general method H. MS (ESI, $EI^+$): $m/z$ = 243.3 ($MH^+$).

The following table illustrates intermediates **77-83** prepared from method F, G or H.

| Intermediate | Structure | Synthesis procedure |
|---|---|---|
| Compound **77a** | 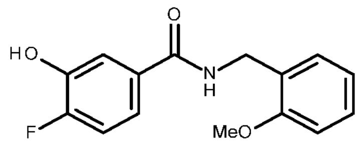 | Method F |
| Compound **77b** | 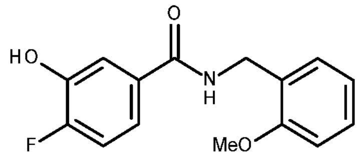 | Method F |
| Compound **77c** | 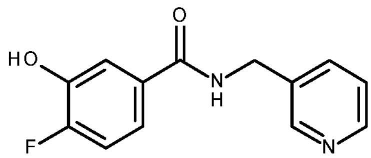 | Method F |
| Compound **77d** | 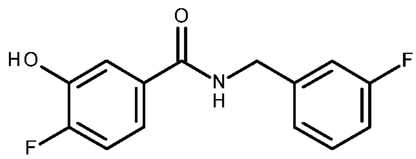 | Method F |
| Compound **77e** | 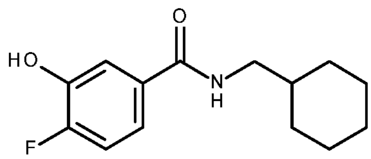 | Method F |
| Compound **77g** | 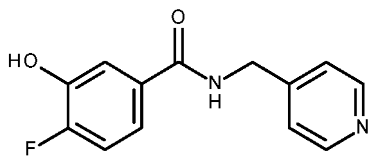 | Method G |
| Compound **78a** | 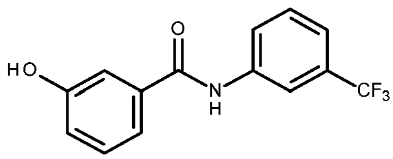 | Method F |

(continued)

| Intermediate | Structure | Synthesis procedure |
|---|---|---|
| Compound **79a** | 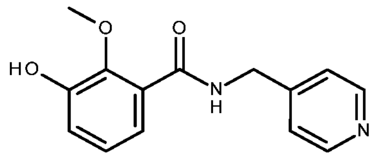 | Method F |
| Compound **80a** | 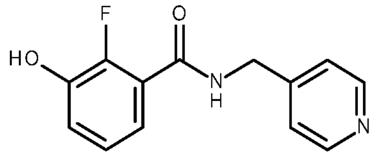 | Method F |
| Compound **81a** | 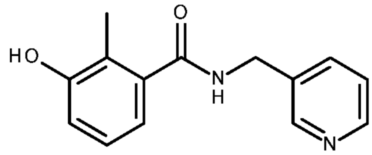 | Method G |
| Compound **81b** | 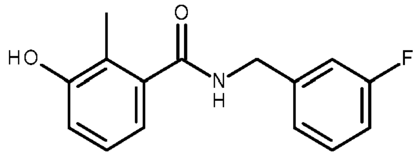 | Method G |
| Compound **81c** | 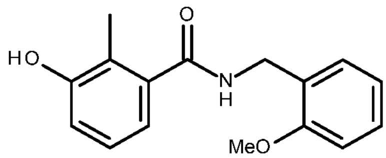 | Method G |
| Compound **81d** | 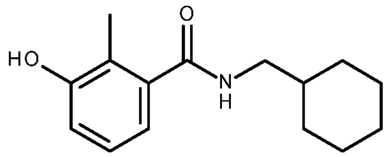 | Method G |
| Compound **81e** | 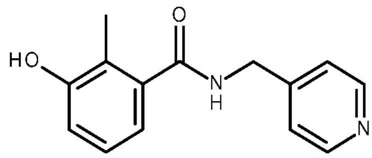 | Method H |
| Compound **81f** | 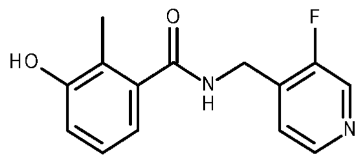 | Method F |
| Compound **82a** | 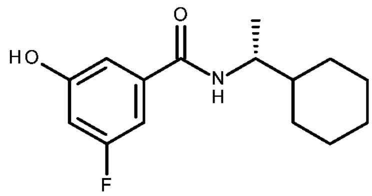 | Method G |

(continued)

| Intermediate | Structure | Synthesis procedure |
|---|---|---|
| Compound **82b** | 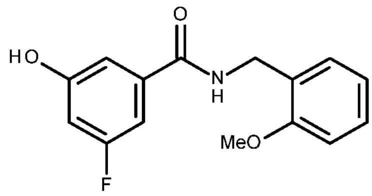 | Method G |
| Compound **83a** | 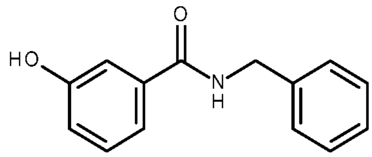 | Method H |
| Compound **83b** | 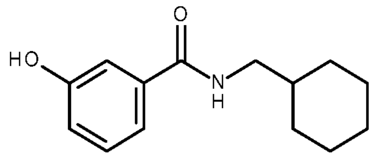 | Method H |
| Compound **83c** | 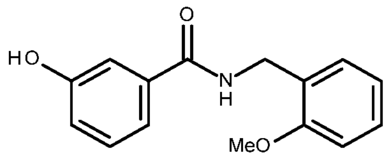 | Method H |
| Compound **83d** | 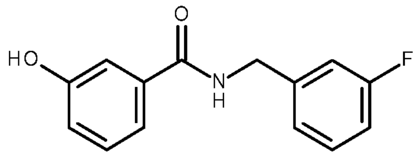 | Method H |
| Compound **83e** | 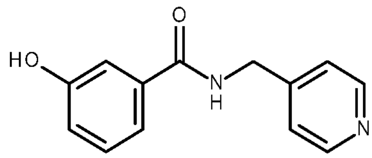 | Method H |

**Method I:** In a oven-dried screw-cap test tube, the appropriate quinoline (1 equiv.) and cesium carbonate (2.5 equiv.) were added to a solution of the appropriate hydroxyl benzamide (1 equiv. or 1.1 equiv.) in DMF (5 mL/mmol).

The reaction mixture was stirred at 90-100°C until completion (from 1h to overnight) and concentrated under reduced pressure. The crude was purified by flash chromatography (DCM/MeOH: 0 to 5%) and reverse phase chromatography ($H_2O$/MeOH: 0 to 100%) to give the expected compound.

The following compound **108** is an example illustrating Method I:

<u>Preparation of 3-[(7-chloro-4-quinolyl)oxy]-2-methyl-N-(4-pyridylmethyl)benzamide **(108):**</u>

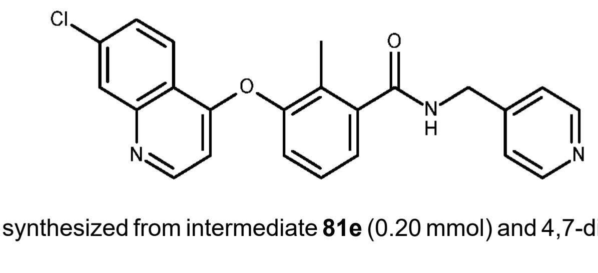

Compound **108** was synthesized from intermediate **81e** (0.20 mmol) and 4,7-dichloroquinoline (0.20 mmol) as a

white solid in 41% yield (33 mg) according to the general method H. $^1H$ NMR (400 MHz, $CDCl_3$) δ (ppm): 9.05 (t, *J* = 6.03 Hz, 1H), 8.73 (d, *J* = 5.15 Hz, 1H), 8.54-8.53 (m, 2H), 8.41 (d, *J* = 9.03 Hz, 1H), 8.11 (d, *J* = 2.08 Hz, 1H), 7.73 (dd, *J* = 8.85 and 1.97 Hz, 1H), 7.47-7.44 (m, 2H), 7.40-7.35 (m, 3H), 6.49 (d, *J* = 5.13 Hz, 1H), 4.50 (d, *J* = 5.90 Hz, 2H), 2.15 (s, 3H) ; MS (ESI, $EI^+$): *m*/*z*= 402.2 ($MH^+$).

**Example 3: General procedure for the preparation of analogues 113-114**

The following procedures illustrate the preparation of analogues **113-114,** the synthetic scheme is outlined below. Examples of the structures of the final compounds can be found in Table 1.

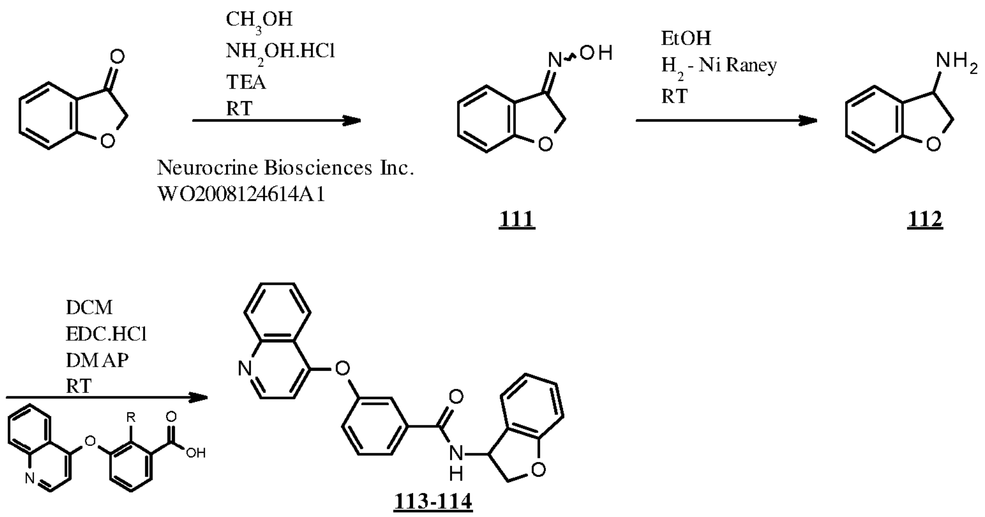


111

112

113-114

The following compound **113** is a example illustrating these procedures.

Preparation of benzofuran-3-one oxime **(111):**

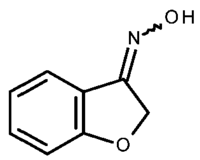

To a solution of 2,3-dihydrobenzofuran-3-one (1.86 mmol) in MeOH (2.5 mL/mmol) were added TEA (3.73 mmol) and hydroxylamine hydrochloride (3.73 mmol). The reaction mixture was stirred at room temperature overnight. The starting material was always present so TEA (1.86 mmol) and hydroxylamine hydrochloride (1.86 mmol) were added and the reaction mixture stirred again during 16 hours. The solvent was removed and the mixture was diluted with ethyl acetate and washed with water three times. The organic layer was dried over $Na_2SO_4$, filtered and concentrated under reduced pressure to give the expected intermediate **111** as a yellow solid in 93% yield. MS (ESI, $EI^+$): *m*/*z*= 150 ($MH^+$).

Preparation of 2,3-dihydrobenzofuran-3-amine **(112):**

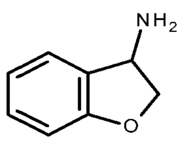

To a solution of intermediate 111 (1.74 mmol) in EtOH (7 mL/mmol) was added Ni Raney 50% in water (500 mg). The reaction mixture was hydrogenated at room temperature overnight. The crude was filtered through a celite pad and

washed with ethanol. The filtrate was concentrated under reduced pressure and the solid was dried under vacuum to give the expected intermediate 112 as an orange dark gum in 90% yield.

Preparation of N-(2,3-dihydrobenzofuran-3-yl)-3-(4-quinolyloxy)benzamide (**113)**:

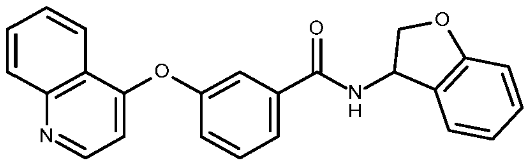

To a solution of intermediate **2a** (0.226 mmol) and intermediate **112** (0.34 mmol) in DCM (2.5 mL) were added as solid DMAP (0.497 mmol) followed by EDC.HCl (0.452 mmol). The reaction mixture was stirred under nitrogen at room temperature overnight. The crude was diluted with DCM and washed with water. The aqueous layer was extracted and the combined organic layers were dried over $Na_2SO_4$, filtered and concentrated under reduced pressure. The crude was purified by flash column chromatography on Biotage (DCM/MeOH: 0 to 10%) to give the expected compound **113** as a white solid in 27% yield (23 mg). $^1H$ NMR (400 MHz, $CDCl_3$) δ (ppm): 8.68 (d, *J* = 5.30 Hz, 1H), 8.35 (d, *J* = 8.39 Hz, 1H), 8.21 (d, *J* = 8.39 Hz, 1H), 7.85-7.79 (m, 1H), 7.72-7.53 (m, 4H), 7.40-7.34 (m, 2H), 7.30-7.25 (m, 1H), 6.97-6.88 (m, 2H), 6.59-6.56 (m, 2H), 5.82-5.76 (m, 1H), 4.82-4.76 (m, 1H), 4.50-4.46 (m, 1H) ; MS (ESI, $EI^+$): *m*/*z*= 383 ($MH^+$).

**Example 4: Procedure for the preparation of N-(1-tert-butylpyrazol-4-yl)-3-(4-quinolyloxy)benzamide 117**

The following procedures illustrate the preparation of N-(1-tert-butylpyrazol-4-yl)-3-(4-quinolyloxy)benzamide **117,** the synthetic scheme is outlined below.

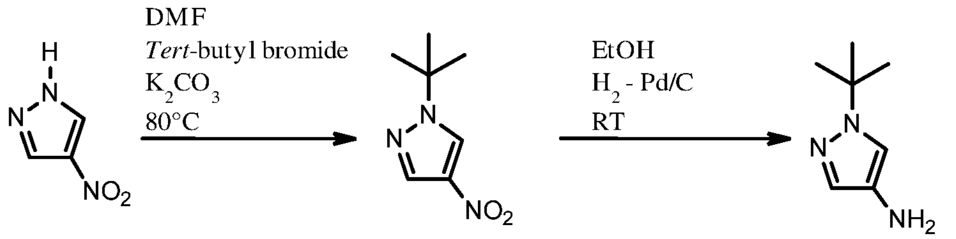


115

116

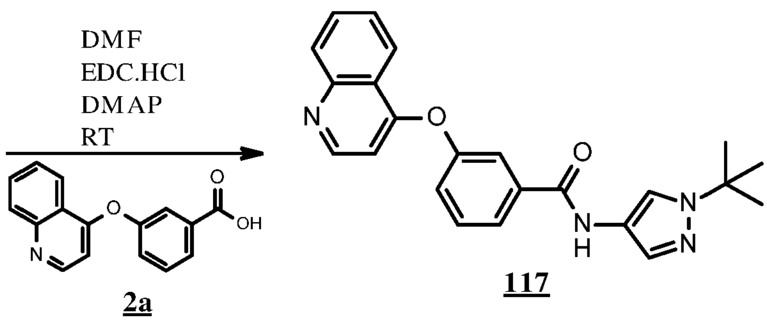


2a

117

Preparation of 1-tert-butyl-4-nitro-pyrazole (115):

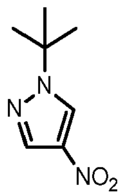

To a stirred solution of 4-nitropyrole (8.84 mmol) and tert-butyl bromide (115 mmol) in DMF (44 mL) was added potassium carbonate (141.5 mmol) under nitrogen. The reaction mixture was warmed to 80°C overnight. DMF was removed and the crude mixture was taken up with DCM and washed with brine twice. The aqueous layer was extracted once and the combined organic layers were dried over $Na_2SO_4$, filtered and concentrated under reduced pressure. The crude was purified by flash column chromatography on Biotage (Cyclohexane/EtOAc: 0 to 30%) to give the expected compound 115 as a white solid in 49% yield (730 mg). MS (ESI, $EI^+$): *m*/*z*= 170 ($MH^+$).

Preparation of 1-tert-butylpyrazol-4-amine (116):

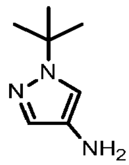

To a solution of intermediate **115** (4.30 mmol) in EtOH (7 mL/mmol) was added Pd/C 10% (80 mg). The reaction mixture was hydrogenated at room temperature overnight. The crude was filtered through a celite pad and washed with ethanol. The filtrate was concentrated under reduced pressure and the solid was dried under vacuum to give the expected intermediate **116** as a dark gum in quantitative yield. MS (ESI, $EI^+$): *m*/*z*= 140 ($MH^+$).

Preparation of N-(1-tert-butylpyrazol-4-yl)-3-(4-quinolyloxy)benzamide (117):

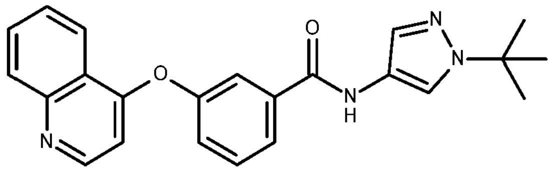

To a solution of intermediate **2a** (0.188 mmol) and intermediate **116** (0.282 mmol) in DMF (2 mL) were added as solid DMAP (0.415 mmol) followed by EDC.HCl (0.376 mmol). The reaction mixture was stirred under nitrogen at room temperature overnight. The crude was diluted with DCM and washed with water. The aqueous layer was extracted and the combined organic layers were dried over $Na_2SO_4$, filtered and concentrated under reduced pressure. The crude was purified by flash column chromatography on Biotage (DCM/MeOH: 0 to 5%) and reverse phase chromatography ($H_2O$/MeOH: 0 to 100%) to give the expected compound **117** as a white solid in 54% yield (39 mg). $^1$H NMR (400 MHz, DMSO-$d_6$) $\delta$ (ppm): 10.51 (s, 1H), 8.80 (d, *J* = 5.09 Hz, 1H), 8.35 (dd, *J* = 8.30 Hz and *J* = 0.91 Hz, 1H), 8.14-8.12 (m, 2H), 8.01-7.98 (m, 1H), 7.94-7.90 (m, 2H), 7.77-7.58 (m, 4H), 6.77 (d, *J* = 5.01 Hz, 1H), 1.57 (s, 9H) ; MS (ESI, $EI^+$): *m*/*z*= 385 ($MH^+$).

**Example 5: General procedure for the preparation of analogues 122-124**

The following procedures illustrate the preparation of analogues **122-124,** the synthetic scheme is outlined below. Examples of the structures of the final compounds can be found in Table 1.

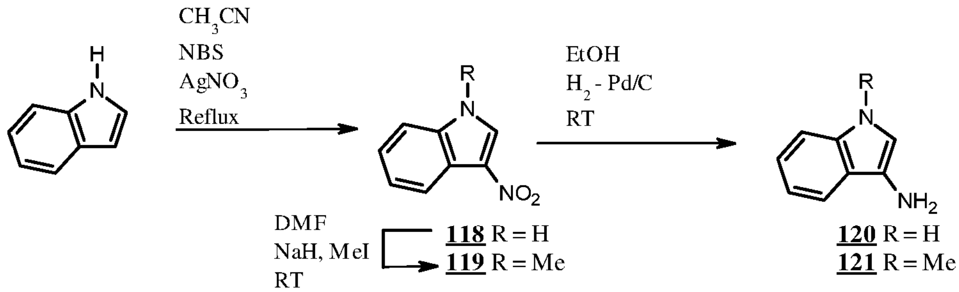


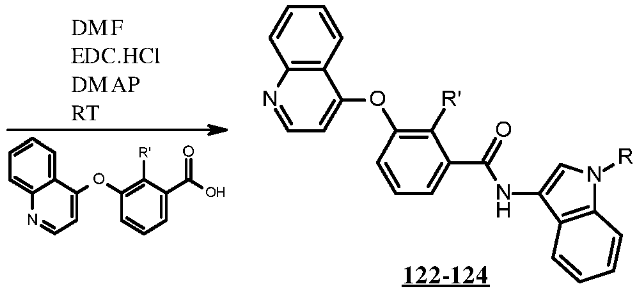


122-124

The following compound **122** is an example illustrating these procedures.

Preparation of 3-nitro-1H-indole **(118):**

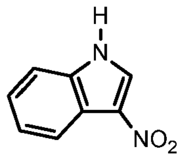

To a stirred solution of NBS (21.34 mmol) in $CH_3CN$ (43 mL) was added $AgNO_3$ (21.34 mmol) as solid under nitrogen. The reaction mixture was warmed to reflux for 5 min and indole (21.34 mmol) was added. The reaction mixture was stirred at 70°C for 3h and then diluted with DCM and washed with a saturated solution of sodium bicarbonate. The aqueous layer was extracted once and the combined organic layers were dried over $Na_2SO_4$, filtered and concentrated under reduced pressure. The crude was purified by flash column chromatography on Biotage (DCM/MeOH: 0 to 3%) to give the expected compound **118** as a brown solid in 24% yield. MS (ESI, $EI^+$): *m/z*= 163 ($MH^+$).

Preparation of 1-methyl-3-nitro-indole **(119):**

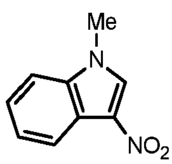

To a solution of intermediate **118** (2.46 mmol) in DMF (12 mL) was added sodium hydride 60% in mineral oil (3.70 mmol). The reaction mixture was stirred at 50°C for 1h. Then, methyl iodide (9.86 mmol) was added at room temperature and the reaction mixture was stirred for 1 h. The reaction mixture was then diluted with EtOAc and washed with saturated solution of ammonium chloride. The aqueous layer was extracted once and the combined organic layers were dried over $Na_2SO_4$, filtered and concentrated under reduced pressure. The crude was purified by flash column chromatography on Biotage (DCM/MeOH: 0 to 2%) to give the expected compound 119 as a beige solid in quantitative yield. MS (ESI, $EI^+$): *m/z*= 177 ($MH^+$).

Preparation of 1H-indol-3-amine (120):

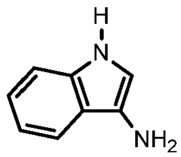

To a solution of intermediate **118** (1.31 mmol) in EtOH (10 mL) was added Pd/C 10% (25 mg). The reaction mixture was hydrogenated at room temperature overnight. The crude was filtered through a celite pad and washed with ethanol. The filtrate was concentrated under reduced pressure and the solid was dried under vacuum to give the expected intermediate **120** as a dark solid in quantitative yield. MS (ESI, $EI^+$): *m*/*z*= 133 ($MH^+$).

Preparation of 1-methylindol-3-amine (121):

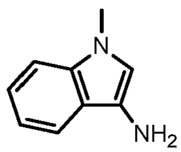

The previous procedure was used with intermediate **120** (2.48 mmol) and Pd/C 10% (50 mg) and yielded to intermediate **121** as a red gum in 75% yield. MS (ESI, $EI^+$): *m*/*z*= 147 ($MH^+$).

Preparation of N-(1H-indol-3-yl)-3-(4-quinolyloxy)benzamide **(122):**

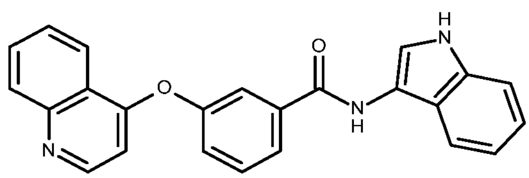

To a solution of intermediate **2a** (0.188 mmol) and intermediate **120** (0.282 mmol) in DMF (2 mL) were added as solid DMAP (0.415 mmol) followed by EDC.HCl (0.376 mmol). The reaction mixture was stirred under nitrogen at room temperature overnight. The crude was diluted with DCM and washed with water. The aqueous layer was extracted and the combined organic layers were dried over $Na_2SO_4$, filtered and concentrated under reduced pressure. The crude was purified by flash column chromatography on Biotage (DCM/MeOH: 0 to 9%) and reverse phase chromatography ($H_2O$/MeOH: 0 to 100%) to give the expected compound **122** as a beige solid in 13% yield (9 mg). $^1H$ NMR (400 MHz, DMSO-$d_6$) δ (ppm): 10.92 (s, 1H), 10.23 (s, 1H), 8.74 (d, *J* = 5.19 Hz, 1H), 8.35 (dd, *J* = 8.16 Hz and *J* = 0.86 Hz, 1H), 8.08-8.05 (m, 1H), 8.01-7.99 (m, 1H), 7.95-7.94 (m, 1H), 7.88-7.77 (m, 3H), 7.72-7.67 (m, 2H), 7.54-7.51 (m, 1H), 7.36-7.35 (m, 1H), 7.11-7.08 (m, 1H), 7.00-6.96 (m, 1H), 6.73 (d, *J* = 5.09 Hz, 1H) ; MS (ESI, $EI^+$): *m*/*z*= 380.10 ($MH^+$).

**Example 6: Procedure for the preparation of 3-[(2-amino-4-quinolyl)oxy]-N-benzyl-benzamide 127**

The following procedures illustrate the preparation of 3-[(2-amino-4-quinolyl)oxy]-N-benzyl-benzamide analogue **127,** the synthetic scheme is outlined below.

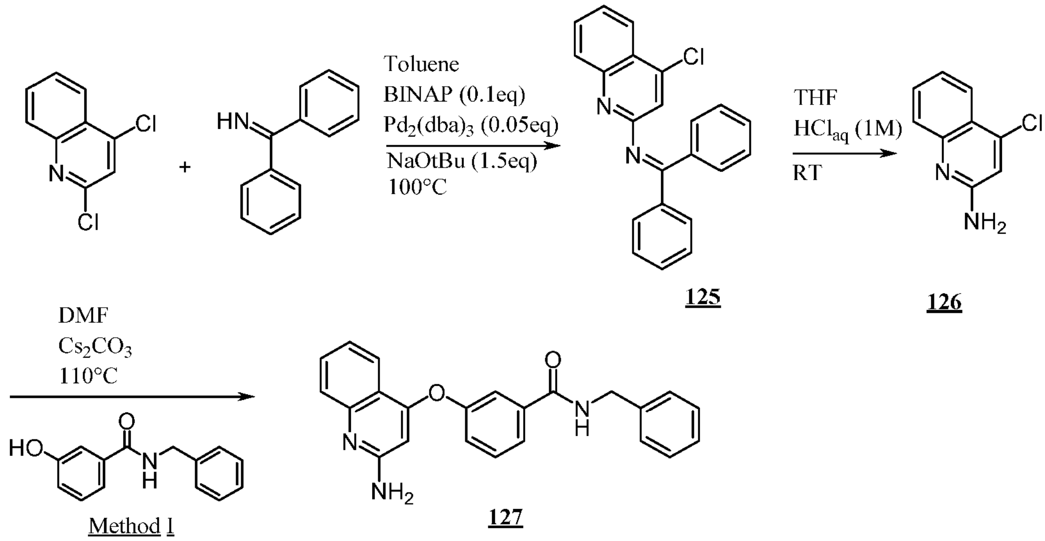


Preparation of N-(4-chloro-2-quinolyl)-1,1-diphenyl-methanimine **(125):**

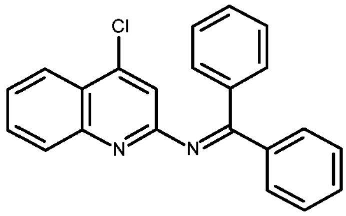

To a solution of 2,4-dichloroquinoline (1.51 mmol) in dry toluene (15mL) were added NaOtBu (2.27 mmol), BINAP (0.15 mmol) and $Pd_2(dba)_3$ (0.076 mmol) and the reaction mixture was stirred at room temperature during 5 minutes. Finally, diphenylmethanamine (1.51 mmol) was added and the reaction mixture was stirred at 100°C during 4 hours. The reaction mixture was concentrated and the crude was partitioned in ethyl acetate and water. The organic layer was washed with water, brine, dried over $Na_2SO_4$ and concentrated to give the expected intermediate **125** as a brown solid and used without purification for the next step.

Preparation of 4-chloroquinolin-2-amine **(126):**

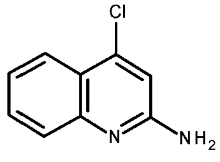

To a solution of intermediate **125** (1.514 mmol) in THF (5 mL) was added an aqueous solution of HCl (1N) (1.5 mL) and the reaction mixture was stirred at room temperature during 1 hour. The reaction mixture was neutralized with aqueous solution of $NaHCO_3$ and extracted with ethyl acetate. The organic layer was washed with water, brine and dried over $Na_2SO_4$ before filtration and concentration. The crude was purified by chromatography on silica gel (DCM/MeOH: 0 to 3%) and by reverse-phase chromatography ($H_2O$/MeOH: 0 to 100%) to give the expected intermediate **126** as a white solid in 57% yield (over 2 steps). MS (ESI, $EI^+$): *m*/*z*= 179.1 ($MH^+$).

Preparation of 3-[(2-amino-4-quinolyl)oxy]-N-benzyl-benzamide **(127):**

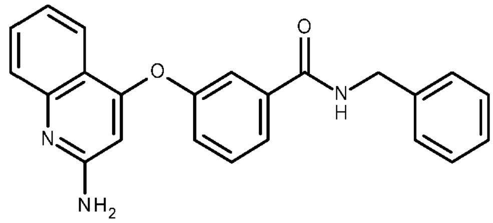

Compound **127** was synthesized from intermediate **83a** (0.308 mmol) and intermediate **126** (0.280 mmol) as a yellow solid in 19% yield (20 mg) according to the general method I. $^{1}H$ NMR (400 MHz, DMSO-$d_6$) δ (ppm): 8.00 (d, *J* = 8.22 Hz, 1H), 7.63-7.60 (m, 1H), 7.57-7.49 (m, 3H), 7.44-7.41 (m, 1H), 7.27-7.26 (m, 4H), 7.24-7.20 (m, 3H), 6.46-6.43 (m, 1H), 5.72 (s, 1H), 4.56 (s, 2H), 4.55 (s, 2H) ; MS (ESI, $EI^+$): *m/z*= 370.1 ($MH^+$).

**Example 7: Procedure for the preparation of 7-amino quinoline analogues 134-153 and 266-272**

The following procedures illustrate the preparation of 7-amino quinoline analogues **134-153** and **266-272,** the synthetic scheme is outlined below.

The synthesis starts with the coupling of 7-bromo-4-chloroquinoline with the intermediates **83a, 83e, 77g, 77d, 81e, or 81b** according to the Method I described in example 2. In Method J, the preparation of the analogues **134-153** and **266-272** via the amination of the bromo position was achieved. Examples of the structures of the final compounds can be found in Table 1.

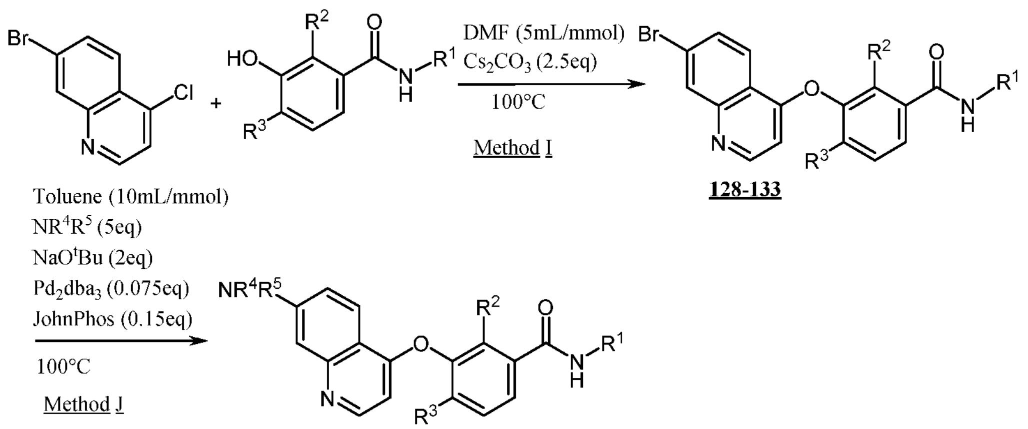


128-133

134-153, 266-272

**Method J:** The appropriate intermediate (1 equiv.), tris(dibenzylideneacetone)dipalladium (0.05-0.075 equiv.), JohnPhos (0.10-0.15 equiv.) and sodium tert-butoxide (2 equiv.) were placed in an oven-dried screw-cap test tube. The tube was then evacuated and backfilled with nitrogen (this sequence was repeated three times). Toluene (10 mL/mmol) and the appropriate amine (5 equiv.) were added. The reaction mixture was stirred and heated at 100°C overnight. The mixture was then concentrated and purified by flash column chromatography on Biotage (DCM/MeOH: 0 to 10%) and reverse phase chromatography ($H_2O$/MeOH: 0 to 100%) to give the expected compound. The following compound **134** is an example illustrating Method J:

<u>Preparation of N-benzyl-3-[[7-(methylamino)-4-quinolyl]oxy]benzamide **(134):**</u>

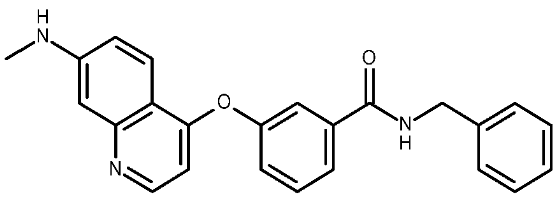

Compound **134** was synthesized from intermediate **128** (0.20 mmol) and methylamine (2M in THF, 1.00 mmol) as

a yellow powder in 50% yield (35 mg) according to the general method J. $^{1}H$ NMR (400 MHz, DMSO-$d_6$) δ (ppm): 9.12 (t, *J* = 5.65 Hz, 1H), 8.46 (d, *J* = 5.21 Hz, 1H), 7.95 (d, *J* = 9.04 Hz, 1H), 7.86-7.84 (m, 1H), 7.73 (t, *J* = 1.88 Hz, 1H), 7.61 (t, *J* = 7.91 Hz, 1H), 7.43-7.40 (m, 1H), 7.35-7.30 (m, 4H), 7.26-7.22 (m, 1H), 7.04 (dd, *J* = 9.08 and 2.23 Hz, 1H), 6.78 (d, *J* = 2.24 Hz, 1H), 6.46 (q, *J* = 4.83 Hz, 1H), 6.30 (d, *J* = 5.26 Hz, 1H), 4.47 (d, *J* = 5.91 Hz, 2H), 2.81 (d, *J* = 5.18 Hz, 3H) ; MS (ESI, $EI^+$): *m/z= 429.2* ($MH^+$).

**Example 8: Procedure for the preparation of 7-amino quinoline analogues 154 and 273**

The following procedure illustrates the preparation of 7-amino quinoline analogues **154** and **273,** the synthetic scheme is outlined below. Examples of the structures of the final compounds can be found in Table 1.

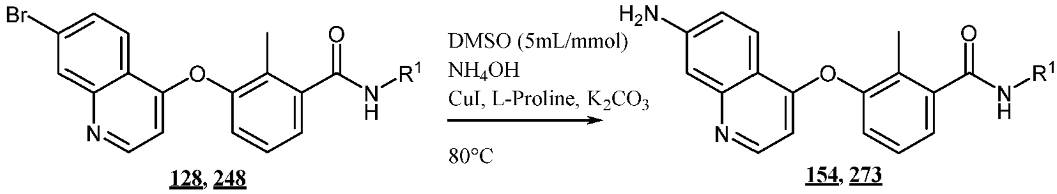


128, 248 154, 273

The following compound **154** is an example illustrating this procedure:
Intermediate **128** (1 equiv.), CuI (0.2 equiv.), L-proline (0.40 equiv.) and potassium carbonate (3 equiv.) were placed in an oven-dried screw-cap test tube. The tube was then evacuated and backfilled with nitrogen (this sequence was repeated three times). DMSO (10 mL/mmol) and ammonium hydroxide (0.6 mL/mmol) were added. The reaction mixture was stirred and heated at 80°C for 40h. The mixture was then partionned between DCM and saturated solution of ammonium chloride. The aqueous layer was extracted twice with DCM and the combined organic layers was dried over $Na_2SO_4$, filtered and concentrated. The crude was then purified by reverse phase chromatography on Biotage ($H_2O$/MeOH: 0 to 100%) to give the expected compound **154** as a yellow powder in 11% yield (8 mg). MS (ESI, EI+): m/z= 370.1 (MH+).

**Example 9: Procedure for the preparation of alkyl and aryl quinoline analogues 155 and 344-387**

The following procedure illustrates the preparation of alkyl and aryl quinoline analogues **155** and **344-387** through a Suzuki-Miyaura cross-coupling reactions according to Method K1 (compounds **155, 344-372)** or Method K2 (compounds **373-387),** the synthetic scheme is outlined below. In case where reactions are conducted with ester derivatives, saponification according to the general method B and peptide coupling according to the general method C, described in example 1, are required to give the final compounds. Examples of the structures of the final compounds can be found in Table 1.

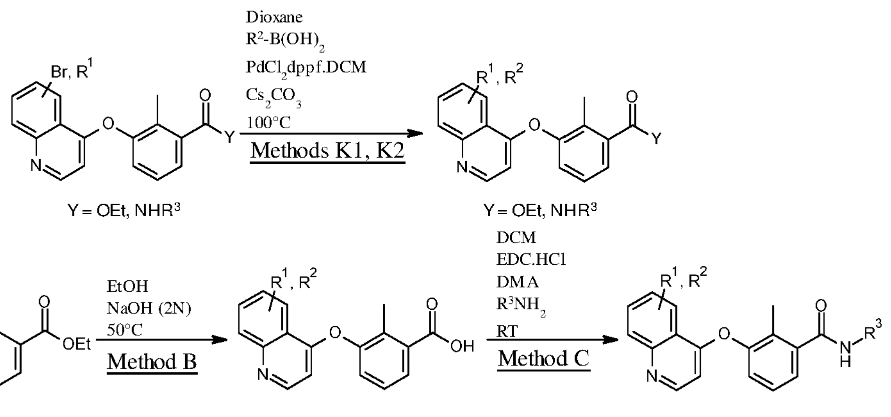


**Method K1:** The appropriate intermediate (1 equiv.), [1,1'-bis(diphenylphosphino)ferrocene]dichloropalladium(II).DCM (0.10 equiv.), alkyl or arylboronic acid or pinacol ester (1.2 equiv.), and cesium carbonate (2.4 equiv.) were placed in an oven-dried screw-cap test tube. The tube was then evacuated and backfilled with nitrogen (this sequence was repeated three times). Dioxane (10 mL/mmol) was added. The reaction mixture was stirred and heated at 100°C until completion (1h-overnight). The mixture was then concentrated and purified by flash column chromatography on Biotage (DCM/MeOH: 0 to 10%) and reverse phase chromatography ($H_2O$/MeOH: 0 to 100%) to give the expected compound.
The following compound **155** is an example illustrating Method K1:

Preparation of N-benzyl-3-[(7-methyl-4-quinolyl)oxy]benzamide **(155):**

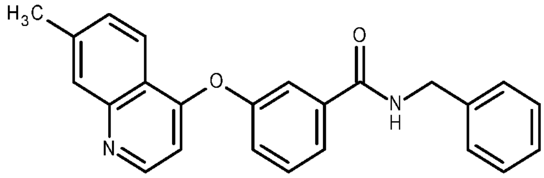

Compound 155 was synthesized from intermediate **128** (0.20 mmol) and methylboronic acid (0.24 mmol) as a white powder in 77% yield (56 mg) according to the general method K1. $^1$H NMR (400 MHz, DMSO-$d_6$) δ (ppm): 9.13 (t, *J* = 5.92 Hz, 1H), 8.67 (d, *J* = 5.15 Hz, 1H), 8.20 (d, *J* = 8.44 Hz, 1H), 7.89-7.84 (m, 2H), 7.78-7.77 (m, 1H), 7.63 (t, *J* = 7.98 Hz, 1H), 7.53-7.46 (m,2H), 7.32-7.30 (m, 4H), 7.26-7.21 (m, 1H), 6.61 (d, *J* = 5.20 Hz, 1H), 4.47 (d, *J* = 5.93 Hz, 2H), 2.55 (s, 3H) ; MS (ESI, $EI^+$): *m*/*z*= 369.2 ($MH^+$).

**Method K2:** The appropriate intermediate (1 equiv.), [1,1'-bis(diphenylphosphino)ferrocene]dichloropalladium(II).DCM (0.10 equiv.) and arylboronic acid or pinacol ester (1.2 equiv.) were placed in an oven-dried screw-cap test tube. The tube was then evacuated and backfilled with nitrogen (this sequence was repeated three times). Dioxane (10 mL/mmol) and cesium carbonate (aq 1M, 3 equiv.) under azote atmosphere were added. The reaction mixture was stirred and heated at 100°C until completion (1h-overnight). The mixture was then concentrated and purified by flash column chromatography on Biotage (DCM/MeOH: 0 to 10%) and reverse phase chromatography ($H_2O$/MeOH: 0 to 100%) to give the expected compound.

The following compound **373** is an example illustrating Method K2:

Preparation of 2-methyl-3-[[7-(1H-pyrazol-4-yl)-4-quinolyl]oxy]-N-(4-pyridylmethyl)benzamide **(373):**

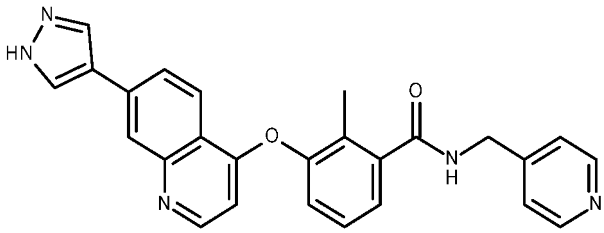

Compound **373** was synthesized from intermediate **132** (0.09 mmol) and 1H-pyrazole-4-boronic acid pinacol ester (0.13 mmol) as a white powder in 28% yield (10 mg) according to the general method K2. $^1$H NMR (400 MHz, DMSO-$d_6$) δ (ppm): 13.0 (bs, 1H), 9.07 (t, *J* = 5.93 Hz, 1H), 8.66 (d, *J* = 5.14 Hz, 1H), 8.54-8.53 (m, 2H), 8.35-8.27 (m, 4H), 7.98 (dd, *J* = 8.68 and 1.64 Hz, 1H), 7.46-7.44 (m, 2H), 7.38-7.35 (m, 3H), 6.37 (d, *J* = 4.97 Hz, 1H), 4.50 (d, *J* = 5.97 Hz, 2H), 2.17 (s, 3H) ; MS (ESI, $EI^+$): *m*/*z*= 436.30 ($MH^+$).

**Example 10: Procedure for the preparation of analogues 160-169**

The following procedures illustrate the preparation of analogues **160-169.** The synthetic scheme is outlined below. Preparation of the appropriate amines is achieved through a reductive hydrogenation of the commercial corresponding nitriles (Method L). A final peptide coupling according the general Method C, described in example 1, yield the analogues **160-169.** Examples of the structures of the final compounds can be found in Table 1.

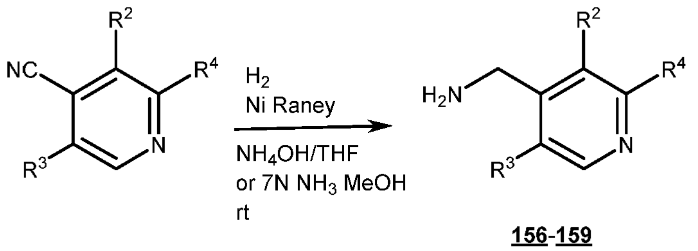


156-159

Method L

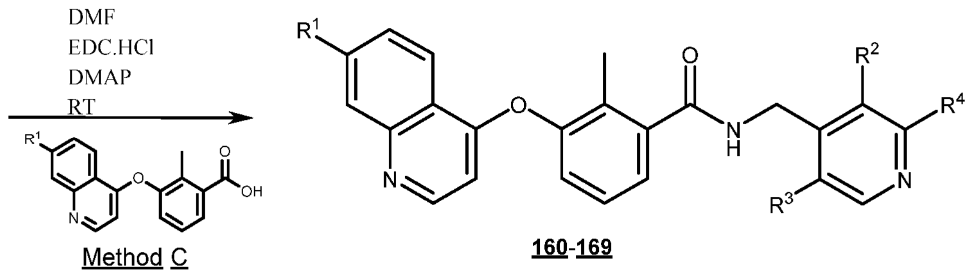


Method C

160-169

The following compound **160** is an example illustrating these procedures.

**Method L:** The appropriate nitrile derivative (1 equiv.) was dissolved in 7N methanolic ammonia (5 mL/mmol) or in a mixture of THF / $NH_4OH$ (1/1, 5 mL/mmol) under azote atmosphere. Ni/Raney (50% in water, 500mg/mmol) was added and the reaction mixture was hydrogenated at room temperature until completion. The crude was filtered through a celite pad and washed with methanol. The filtrate was concentrated under reduced pressure and the solid was dried under vacuum to give the expected amine which was used without further purification.

The following compound **156** is an example illustrating Method L:

Preparation of 3,5-dichloro-4-pyridyl)methanamine **(156):**

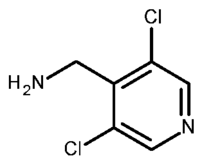

Amine **156** was synthesized from 3,5-dichloroisonicotinonitrile (2.00 mmol) as a dark-green solid in quantitative yield according to the general method L. MS (ESI, $EI^+$): *m/z*= 177.20 ($MH^+$).

The following table illustrates intermediates **156-159** prepared from method L:

| Intermediate | Structure | Synthesis procedure |
|---|---|---|
| Compound **156** | 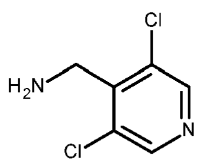 | Method L |
| Compound **157** | 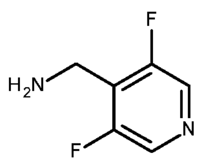 | Method L |

(continued)

| Intermediate | Structure | Synthesis procedure |
|---|---|---|
| Compound **158** | 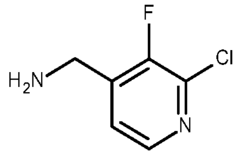 | Method L |
| Compound **159** | 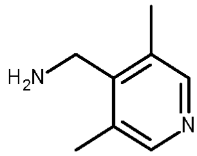 | Method L |

Preparation of 3-[(7-chloro-4-quinolyl)oxy]-N-[(3,5-dichloro-4-pyridyl)methyl]-2-methyl-benzamide (**160**):

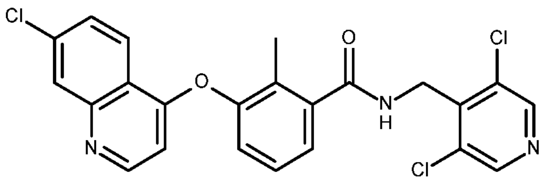

Compound **160** was synthesized from intermediate **2g** (0.10 mmol) and amine **156** (0.25 mmol) as a white powder in 40% yield (19 mg) according to the general method C. $^1$H NMR (400 MHz, DMSO-$d_6$) δ (ppm): 8.92 (t, *J* = 4.74 Hz, 1H), 8.78 (d, *J* = 8.77 Hz, 1H), 8.71 (s, 2H), 8.47 (d, *J* = 8.93 Hz, 1H), 8.17 (d, *J* = 1.99 Hz, 1H), 7.78 (dd, *J* = 2.28 and 9.02 Hz, 1H), 7.50-7.46 (m, 1H), 7.41-7.39 (m, 2H), 6.50 (d, *J* = 5.06 Hz, 1H), 4.74 (d, *J* = 4.80 Hz, 2H), 2.18 (s, 3H) ; MS (ESI, EI$^+$): *m/z*= 474.15 (MH$^+$).

**Example 11: Procedure for the preparation of 2-aryl pyridine quinoline analogues 170 and 388-394**

The following procedure illustrates the preparation of 2-alkyl and aryl pyridine quinoline analogues **170** and **388-394** through a Suzuki-Miyaura cross-coupling reactions according to Method M (compound **170)** or Method K1 (compounds **388-394),** described in example 9. The synthetic scheme is outlined below. Examples of the structures of the final compounds can be found in Table 1.

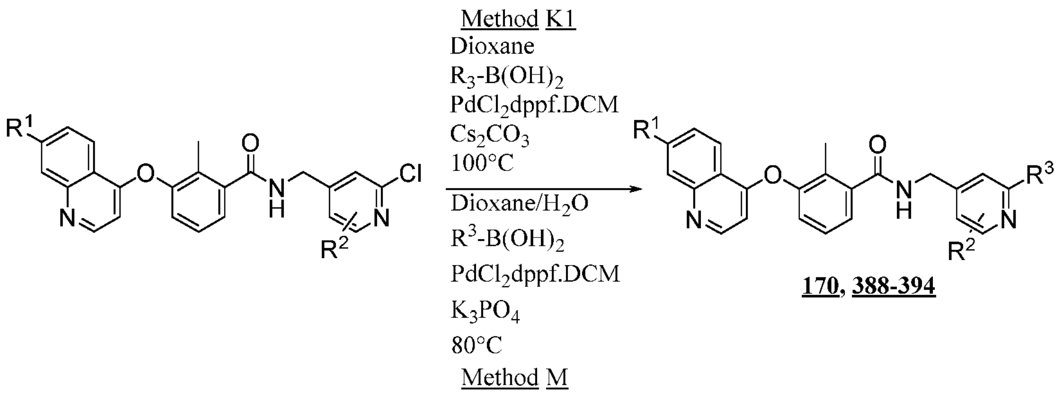


**170, 388-394**

**Method M:** The appropriate intermediate (1 equiv.) was dissolved in a mixture dioxane/water (4/1, 10 mL/mmol) in an oven-dried screw-cap test tube under azote atmosphere. Arylboronic acid (1.2 equiv.) and potassium phosphate (2.0 equiv.) were then added and the tube was evacuated and backfilled with nitrogen (this sequence was repeated three times) before addition of [1,1'-bis(diphenylphosphino)ferrocene]dichloropalladium(II).DCM (0.08 equiv.). The reaction mixture was stirred and heated at 80°C overnight. The mixture was then concentrated and purified by flash column chromatography on Biotage (DCM/MeOH: 0 to 10%) and reverse phase chromatography ($H_2O$/MeOH: 0 to 100%) to give the expected compound.

The following compound **170** is an example illustrating Method M:

Preparation of N-[[2-(3-furyl)-4-pyridyl]methyl]-3-[(7-methoxy-4-quinolyl)oxy]-2-methyl-benzamide **(170):**

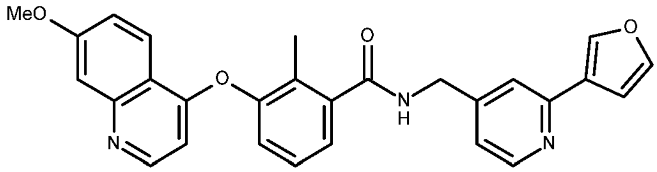

Compound **170** was synthesized from compound **40** (0.115 mmol) and furan-3-boronic acid (0.138 mmol) as an orange powder in 52% yield (28 mg) according to the general method M. $^1$H NMR (400 MHz, DMSO-$d_6$) δ (ppm): 9.06 (t, *J* = 6.11 Hz, 1H), 8.61 (d, *J* = 5.70 Hz, 1H), 8.52 (d, *J* = 5.10 Hz, 1H), 8.31-8.26 (m, 2H), 7.78 (t, *J* = 1.66 Hz, 1H), 7.67 (bs, 1H), 7.47-7.42 (m, 3H), 7.35-7.31 (m, 2H), 7.25-7.23 (m, 1H), 7.02-7.01 (m, 1H), 6.32 (d, *J* = 5.20 Hz, 1H), 4.52 (d, *J* = 5.94 Hz, 2H), 3.95 (s, 3H), 2.15 (s, 3H) ; MS (ESI, EI$^+$): *m*/*z*= 466.25 ($MH^+$).

**Example 12: Procedure for the preparation of 2-cyano pyridine and 2-ketone quinoline analogues 171-179 and 317**

The following procedures illustrate the preparation of 2-cyano pyridine and 2-ketone analogues **171-179** and **317.** The synthetic scheme is outlined below.

The synthesis starts with the preparation of the 2-cyano pyridine quinoline analogues **171-175** and **317** from the appropriate chloro quinoline derivatives described in example 1 according to the Method N. In Method O, the synthesis of the 2-ketone pyridine analogues **176-179** via the addition of alkyl magnesium halide was achieved. Examples of the structures of the final compounds can be found in Table 1.

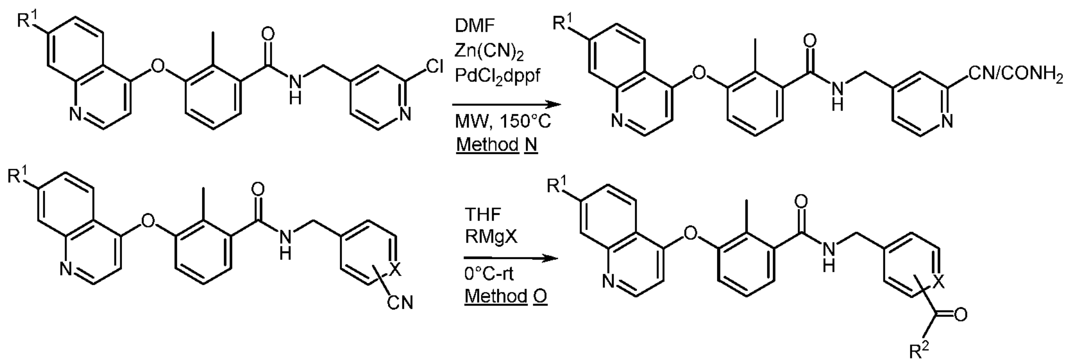


**Method N:** The appropriate compound (1 equiv.) and zinc cyanide (1.2 equiv.) were dissolved in DMF (10 mL/mmol) in an oven-dried screw-cap test tube under azote atmosphere. The tube was evacuated and backfilled with nitrogen (this sequence was repeated three times) before addition [1,1'-bis(diphenylphosphino)ferrocene]dichloropalladium(II) (0.3 equiv.). The reaction mixture was stirred and heated under microwave irradiation at 150°C for 1h. The mixture was diluted with DCM and washed with water. The aqueous layer was extracted three times with DCM and the combined organic layers were washed with cold water and brine, dried over $Na_2SO_4$, filtered and concentrated under reduced pressure. The crude was then purified by flash column chromatography on Biotage (DCM/MeOH: 0 to 10%) and reverse phase chromatography ($H_2O$/MeOH: 0 to 100%) to give the expected nitrile compound and the carboxamide by-product.

The following compounds **171-172** are examples illustrating Method N:

Preparation of 3-[(7-methoxy-4-quinolyl)oxy]-2-methyl-N-[(2-cyano-4-pyridyl)methyl]benzamide **(171)** and 4-[[[3-[(7-methoxy-4-quinolyl)oxy]-2-methyl-benzoyl]amino]methyl]pyridine-2-carboxamide **(172):**

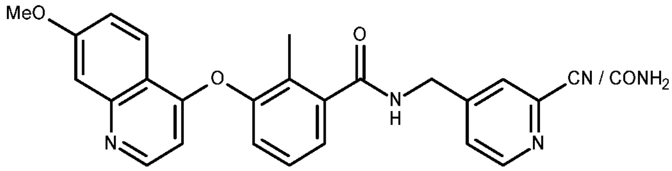

Nitrile compound **171** was synthesized from compound **40** (0.37 mmol) as a beige powder in 55% yield (86 mg) along with the carboxamide by-product **172** (11 mg) according to the general method N.

Compound **171:** $^1$H NMR (400 MHz, DMSO-$d_6$) δ (ppm): 9.12 (t, *J* = 5.82 Hz, 1H), 8.73 (d, *J* = 5.13 Hz, 1H), 8.62 (d, *J* = 5.14 Hz, 1H), 8.27 (d, *J* = 9.14 Hz, 1H), 8.01-8.00 (m, 1H), 7.71-7.70 (m, 1H), 7.51-7.43 (m, 3H), 7.36-7.31 (m, 2H), 6.31 (d, *J* = 5.10 Hz, 1H), 4.57 (d, *J* = 5.83 Hz, 2H), 3.95 (s, 3H), 2.15 (s, 3H) ; MS (ESI, $EI^+$): *m/z*= 425.35 ($MH^+$).

Compound **172:** $^1$H NMR (400 MHz, DMSO-$d_6$) δ (ppm): 9.15 (t, *J* = 5.94 Hz, 1H), 8.62-8.59 (m, 2H), 8.27 (d, *J* = 9.08 Hz, 1H), 8.12 (bs, 1H), 8.05 (bs, 1H), 7.64 (bs, 1H), 7.55 (dd, *J* = 1.79 and 5.10 Hz, 1H), 7.48-7.42 (m, 3H), 7.35-7.31 (m, 2H), 6.32 (d, *J* = 5.21 Hz, 1H), 4.57 (d, *J* = 6.10 Hz, 2H), 3.95 (s, 3H), 2.14 (s, 3H) ; MS (ESI, $EI^+$): *m/z*= 443.35 ($MH^+$).

**Method O:** The appropriate nitrile (1 equiv.) was dissolved in THF (10 mL/mmol) under azote atmosphere. The mixture was cooled to 0°C and the appropriate alkylmagnesium halide (5 equiv.) was added dropwise. The reaction mixture was allowed to warm to room temperature (for alkylmagnesium bromide) or 60°C (for alkyl magnesium chloride) and stirred overnight. The mixture was diluted with ethyl acetate and washed with cold water. The aqueous layer was extracted once with ethyl acetate and the combined organic layers were washed with brine, dried over $Na_2SO_4$, filtered and concentrated under reduced pressure. The crude was then purified by flash column chromatography on Biotage (DCM/MeOH: 0 to 10%) and reverse phase chromatography ($H_2O$/MeOH: 0 to 100%) to give the expected compound.

The following compound **176** is an example illustrating Method O:

Preparation of N-[(2-acetyl-4-pyridyl)methyl]-3-[(7-methoxy-4-quinolyl)oxy]-2-methyl-benzamide **(176):**

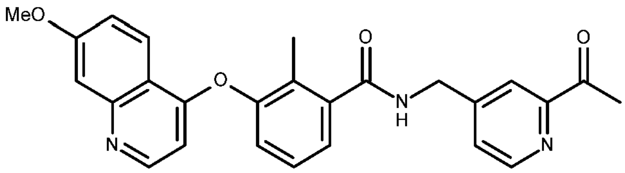

Compound **176** was synthesized from compound **171** (0.15 mmol) and methyl magnesium bromide (3N in ether, 0.76 mmol) as a white powder in 27% yield (18 mg) according to the general method O. $^1$H NMR (400 MHz, DMSO-$d_6$) δ (ppm): 9.15 (t, *J* = 6.19 Hz, 1H), 8.69 (d, *J* = 5.05 Hz, 1H), 8.62 (d, *J* = 5.30 Hz, 1H), 8.27 (d, *J* = 9.16 Hz, 1H), 7.96-7.95 (m, 1H), 7.63 (dd, J = 1.80 and 5.05 Hz, 1H), 7.48-7.41 (m, 3H), 7.36-7.31 (m, 2H), 6.32 (d, *J* = 5.02 Hz, 1H), 4.57 (d, *J* = 6.09 Hz, 2H), 3.95 (s, 3H), 2.64 (s, 3H), 2.15 (s, 3H) ; MS (ESI, $EI^+$): *m/z*= 442.35 ($MH^+$).

**Example 13: Procedure for the preparation of 3-aryl and 3-aminoaryl quinoline analogues**

The following procedures illustrate the preparation of 3-aryl and 3-aminoaryl quinoline analogues. The synthetic scheme is outlined below. Examples of the structures of the final compounds can be found in Table 1.

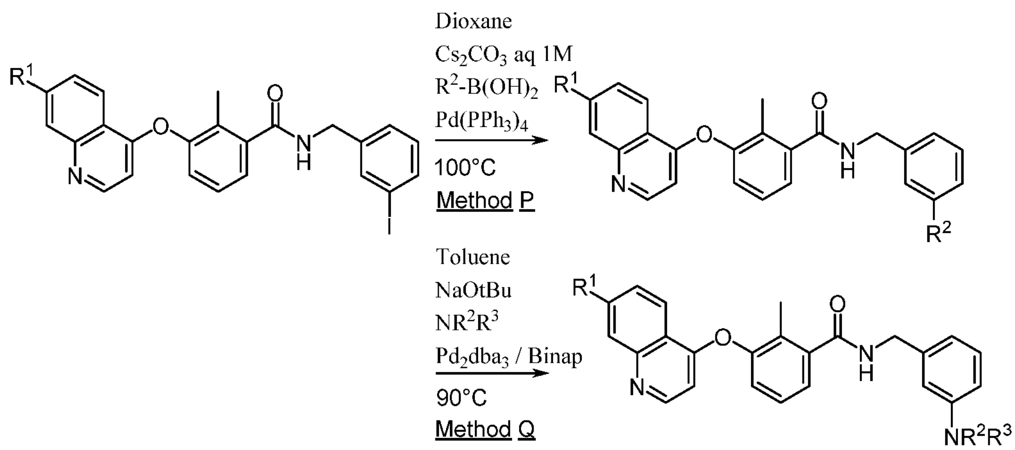


**Method P:** The iodo compound **43** (1 equiv.), arylboronic acid (1.2 equiv.), cesium carbonate (aq 1M, 2 equiv.) were dissolved in dioxane (10 mL/mmol) in an oven-dried screw-cap test tube under azote atmosphere. The tube was evacuated and backfilled with nitrogen (this sequence was repeated three times) before addition palladium tetrakistriphenylphosphine (0.1 equiv.). The reaction mixture was stirred and heated at 100°C overnight. The reaction mixture was then concentrated under reduced pressure and the crude was purified by flash column chromatography on Biotage (DCM/MeOH: 0 to 10%) and reverse phase chromatography ($H_2O$/MeOH: 0 to 100%) to give the expected compound.
The following compound **180** is an example illustrating Method P:

Preparation of N-[[3-(3-furyl)phenyl]methyl]-3-[(7-methoxy-4-quinolyl)oxy]-2-methyl-benzamide **(180):**

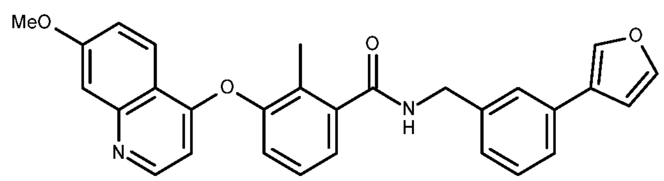

Compound **180** was synthesized from compound **43** (0.08 mmol) and furan-3-boronic acid (0.096 mmol) as a white powder in 68% yield (25 mg) according to the general method P. $^1H$ NMR (400 MHz, DMSO-$d_6$) δ (ppm): 9.03 (t, *J* = 6.16 Hz, 1H), 8.66 (d, *J* = 5.15 Hz, 1H), 8.33 (d, *J* = 9.17 Hz, 1H), 8.21 (bs, 1H), 7.80 (t, *J* = 1.54, 1H), 7.64 (bs, 1H), 7.58-7.31 (m, 8H), 6.98-6.97 (m, 1H), 6.37 (d, *J* = 5.23 Hz, 1H), 4.56 (d, *J* = 5.58 Hz, 2H), 4.00 (s, 3H), 2.20 (s, 3H) ; MS (ESI, $EI^+$): *m/z*= 465.25 ($MH^+$).
**Method Q:** The iodo compound **43** (1 equiv.), tris(dibenzylideneacetone)dipalladium (0.1 equiv.), Binap (0.20 equiv.) and sodium tert-butoxide (5 equiv.) were placed in an oven-dried screw-cap test tube. The tube was then evacuated and backfilled with nitrogen (this sequence was repeated three times). Toluene (10 mL/mmol) and the appropriate amine (10 equiv.) were added. The reaction mixture was stirred and heated at 90°C overnight. The mixture was then concentrated and purified by flash column chromatography on Biotage (DCM/MeOH: 0 to 10%) and reverse phase chromatography ($H_2O$/MeOH: 0 to 100%) to give the expected compound.
The following compound **181** is an example illustrating Method Q:

Preparation of 3-[(7-methoxy-4-quinolyl)oxy]-2-methyl-N-[(3-morpholinophenyl)methyl]benzamide **(181):**

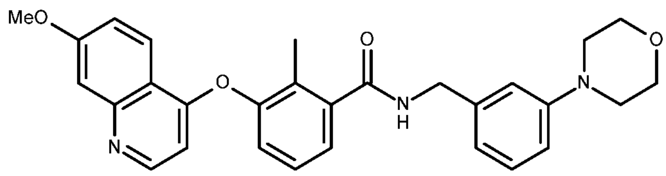

Compound **181** was synthesized from compound **43** (0.10 mmol) and morpholine (1.00 mmol) as a white powder

in 69% yield (33 mg) according to the general method Q. $^{1}H$ NMR (400 MHz, DMSO-$d_6$) δ (ppm): 8.95 (t, $J$ = 5.88 Hz, 1H), 8.66 (d, $J$ = 5.14 Hz, 1H), 8.32 (d, $J$ = 9.31 Hz, 1H), 7.49-7.34 (m, 5H), 7.26 (t, $J$ = 7.85 Hz, 1H), 6.98 (bs, 1H), 6.91-6.85 (m, 2H), 6.37 (d, $J$ = 5.28 Hz, 1H), 4.48 (d, $J$ = 6.08 Hz, 2H), 4.00 (s, 3H), 3.80-3.78 (m, 4H), 3.16-3.14 (m, 4H), 2.19 (s, 3H) ; MS (ESI, $EI^+$): $m/z$= 484.35 ($MH^+$).

**Example 14: Procedure for the preparation of 7-cyano quinoline analogue 182**

The following procedure illustrates the preparation of analogue 182. The synthetic scheme is outlined below.

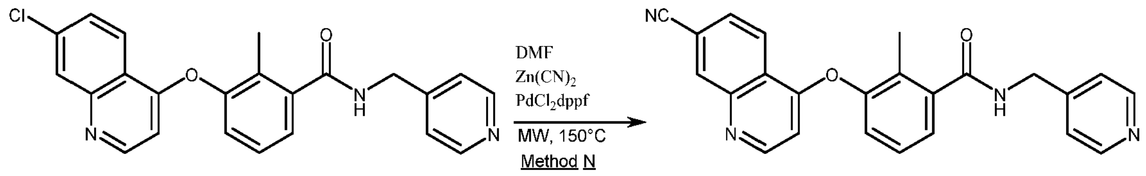


Preparation of 3-[(7-cyano-4-quinolyl)oxy]-2-methyl-N-(4-pyridylmethyl)benzamide (182):

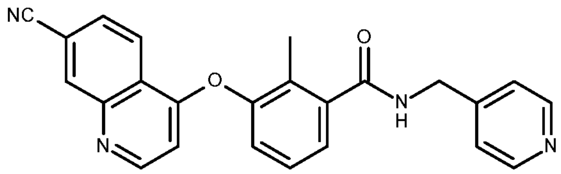

Compound **182** was synthesized from compound **108** (0.18 mmol) as a white powder in 66% yield (46 mg) according to the general method N, described in example 12. $^{1}H$ NMR (400 MHz, DMSO-$d_6$) δ (ppm): 9.07 (t, $J$ = 6.11 Hz, 1H), 8.85 (d, $J$ = 5.17 Hz, 1H), 8.43 (d, $J$ = 1.12 Hz, 1H), 8.57 (bs, 1H), 8.55-8.53 (m, 2H), 8.00 (dd, $J$ = 1.58 and 8.56 Hz, 1H), 7.49-7.34 (m, 5H), 6.61 (d, $J$ = 5.26 Hz, 1H), 4.50 (d, $J$ = 5.88 Hz, 2H), 2.15 (s, 3H) ; MS (ESI, $EI^+$): $m/z$= 395.40 ($MH^+$).

**Example 15: Procedure for the preparation of analogue 186**

The following procedures illustrate the preparation of analogue **186.** The synthetic scheme is outlined below.

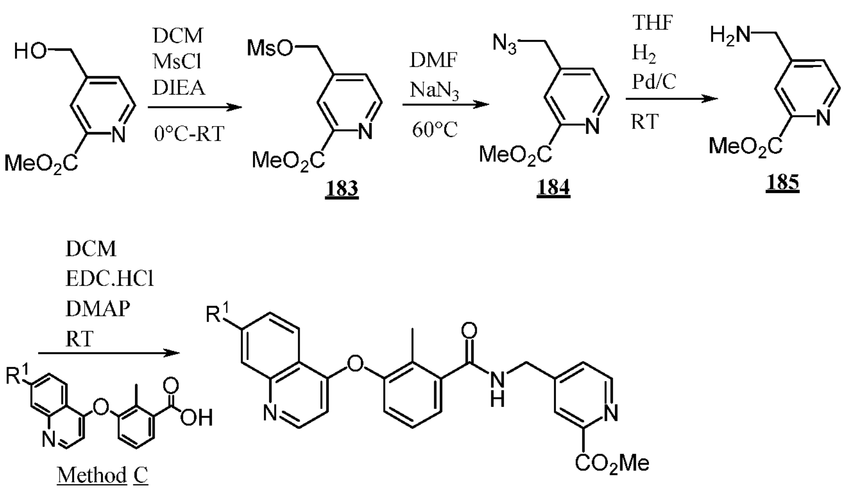


Preparation of ethyl 4-(methylsulfonyloxymethyl)pyridine-2-carboxylate (**183**):

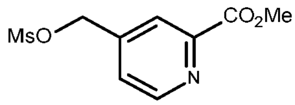

To a stirred solution of methyl 4-(hydroxymethyl)pyridine-2-carboxylate (2.99 mmol) in DCM (15 mL) were added DIEA (8.97 mmol) and methanesulfonyl chloride (3.58 mmol) dropwise under nitrogen at 0°C. The reaction mixture was placed at room temperature and stirred for 45 min. The mixture was then diluted with DCM and washed with water and brine. The organic layer was dried over $Na_2SO_4$, filtered and concentrated under reduced pressure to give the expected compound **183** which was used without purification.

Preparation of methyl 4-(azidomethyl)pyridine-2-carboxylate **(184):**

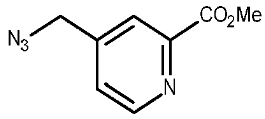

To a solution of intermediate **183** (2.99 mmol) in DMF (9 mL) was added sodium azide (3.59 mmol). The reaction mixture was stirred at 60°C for 3h and then diluted with EtOAc and washed with water. The organic layer was dried over $Na_2SO_4$, filtered and concentrated under reduced pressure. The crude was purified by flash column chromatography on Biotage (DCM/MeOH: 0 to 5%) to give the expected compound **184** as a yellow-brown oil in 87% yield. MS (ESI, $EI^+$): *m/z*= 193.20 ($MH^+$).

Preparation of methyl 4-(aminomethyl)pyridine-2-carboxylate **(185):**

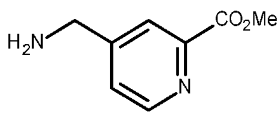

To a solution of intermediate **184** (2.60 mmol) in THF (15 mL) was added Pd/C 10% (100 mg) under nitrogen. The reaction mixture was hydrogenated at room temperature for 4h. The crude was filtered through a celite pad and washed with THF. The filtrate was concentrated under reduced pressure to give the expected intermediate **185** as a yellow oil in quantitative yield. MS (ESI, $EI^+$): *m/z*= 167.25 ($MH^+$).

Preparation of methyl 4-[[[3-[(7-methoxy-4-quinolyl)oxy]-2-methylbenzoyl]amino]methyl]pyridine-2-carboxylate **(186):**

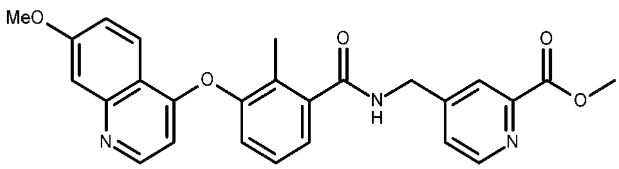

Compound **186** was synthesized from intermediate **2f** (1.61 mmol) and amine **185** (1.78 mmol) as a white powder in 82% yield (604 mg) according to the general method C. $^1H$ NMR (400 MHz, DMSO-$d_6$) δ (ppm): 9.14 (t, *J* = 6.05 Hz, 1H), 8.69-8.60 (m, 2H), 8.27 (d, *J* = 9.35 Hz, 1H), 8.06 (bs, 1H), 7.62-7.60 (m, 1H), 7.47-7.31 (m, 5H), 6.31 (d, *J* = 5.34 Hz, 1H), 4.59-4.57 (m, 2H), 3.95 (s, 3H), 3.87 (s, 3H), 2.15 (s, 3H) ; MS (ESI, $EI^+$): *m/z*= 458.25 ($MH^+$).

**Example 16: Procedure for the preparation of analogue 191**

The following procedures illustrate the preparation of analogue **191.** The synthetic scheme is outlined below.

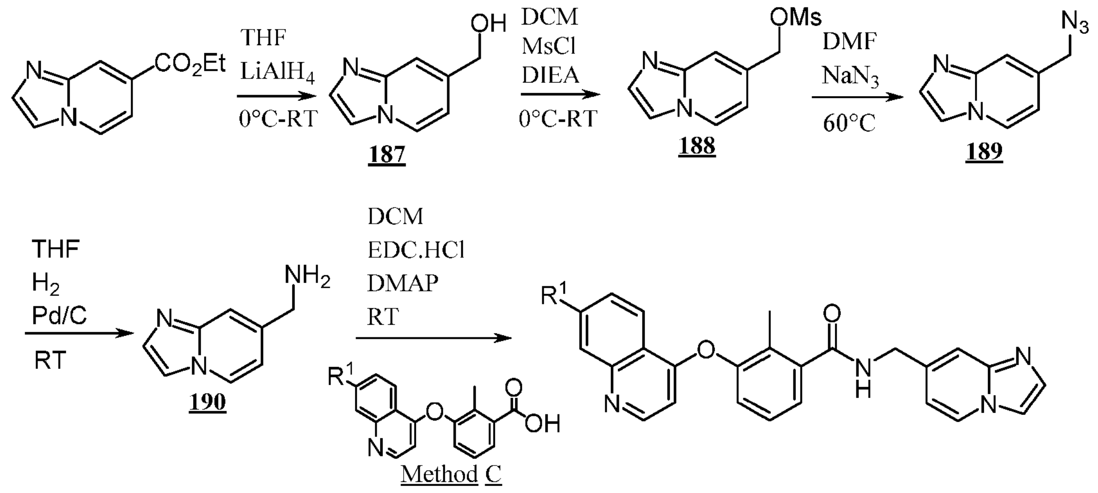


187 188 189 190

Method C

Preparation of imidazol[1,2-a]pyridin-7-ylmethanol **(187):**

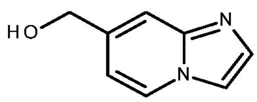

To a stirred solution of ethyl imidazo[1,2-a]pyridine-7-carboxylate (2.00 mmol) in THF (20 mL) was added lithium aluminum hydride (1M in THF, 3 mL) dropwise under nitrogen at 0°C. The reaction mixture was allowed to warm to room temperature and stirred overnight. The reaction was then cooled to 0°C and quenched with an aqueous solution of sodium hydroxide (3%) and stirred for 10 min. The resulting mixture was filtered through a celite pad, washed with ethyl acetate and concentrated under reduced pressure. The crude was purified by flash column chromatography on Biotage (DCM/MeOH: 0 to 20%) to give the expected compound **187** as a white powder in 68% yield. MS (ESI, $EI^+$): *m/z*= 149.25 ($MH^+$).

Preparation of imidazo[1,2-a]pyridin-7-ylmethyl methanesulfonate **(188):**

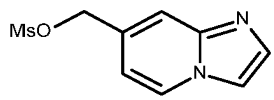

To a stirred solution of intermediate **187** (1.36 mmol) in DCM (8 mL) were added DIEA (4.08 mmol) and methanesulfonyl chloride (1.63 mmol) dropwise under nitrogen at 0°C. The reaction mixture was placed at room temperature and stirred for 1h. The mixture was then diluted with DCM and washed with water and brine. The organic layer was dried over $Na_2SO_4$, filtered and concentrated under reduced pressure to give the expected compound **188** which was used without purification.

Preparation of 7-(azidomethyl)imidazo[1,2-a]pyridine **(189):**

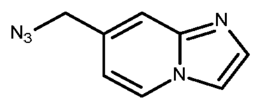

To a solution of intermediate **188** (1.36 mmol) in DMF (4 mL) was added sodium azide (1.63 mmol). The reaction mixture was stirred at 60°C overnight and then diluted with EtOAc and washed with water. The organic layer was dried over $Na_2SO_4$, filtered and concentrated under reduced pressure. The crude was purified by flash column chromatography on Biotage (DCM/MeOH: 0 to 5%) to give the expected compound **189** as a brown oil in 42% yield. MS (ESI, $EI^+$): *m/z*= 174.25

($MH^+$).

Preparation of imidazo[1,2-a]pyridin-7-ylmethanamine **(190):**

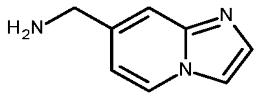

To a solution of intermediate **189** (0.58 mmol) in ethyl acetate (6 mL) was added Pd/C 10% (30 mg) under nitrogen. The reaction mixture was hydrogenated at room temperature for 48h. The crude was filtered through a celite pad and washed with THF. The filtrate was concentrated under reduced pressure to give the expected intermediate **190** as a yellow oil in 87% yield. MS (ESI, $EI^+$): *m/z*= 148.25 ($MH^+$).

Preparation of N-(imidazo[1,2-a]pyridin-7-ylmethyl)-3-[(7-methoxy-4-quinolyl)oxy]-2-methyl-benzamide (**191**):

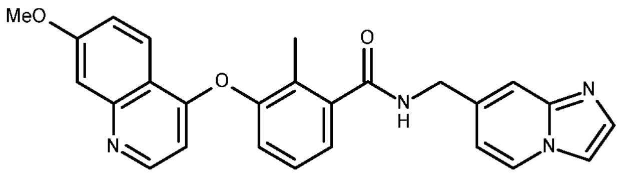

Compound **191** was synthesized from intermediate **2f** (1.61 mmol) and amine **190** (1.78 mmol) as a white powder in 52% yield (23 mg) according to the general method C. $^1$H NMR (400 MHz, DMSO-$d_6$) δ (ppm): 9.03 (t, *J* = 5.82 Hz, 1H), 8.61 (d, *J* = 5.23 Hz, 1H), 8.51 (d, *J* = 7.00 Hz, 1H), 8.27 (d, *J* = 9.26 Hz, 1H), 7.90 (bs, 1H), 7.54-7.52 (m, 1H), 7.47-7.41 (m, 4H), 7.35-7.30 (m, 2H), 6.89 (dd, *J* = 1.49 and 6.81 Hz, 1H), 6.31 (d, *J* = 5.06 Hz, 1H), 4.51 (d, *J* = 5.99 Hz, 2H), 3.96 (s, 3H), 2.14 (s, 3H) ; MS (ESI, $EI^+$): *m/z*= 439.30 ($MH^+$).

**Example 17: Procedure for the preparation of 2-amidopyridine and 4-amidoaryl quinoline analogues 195-204**

The following procedures illustrate the preparation of 2-amidopyridine and 4-amidoaryl quinoline analogues **195-204.** The synthetic scheme is outlined below. The synthesis starts with a saponification of the appropriate methyl ester analogues (method R, compounds **192-194).** The preparation of the amido pyridine or aryl derivatives **195-204** via peptide coupling reaction according the general method C described in example 1, was achieved. Examples of the structures of the final compounds can be found in Table 1 below.

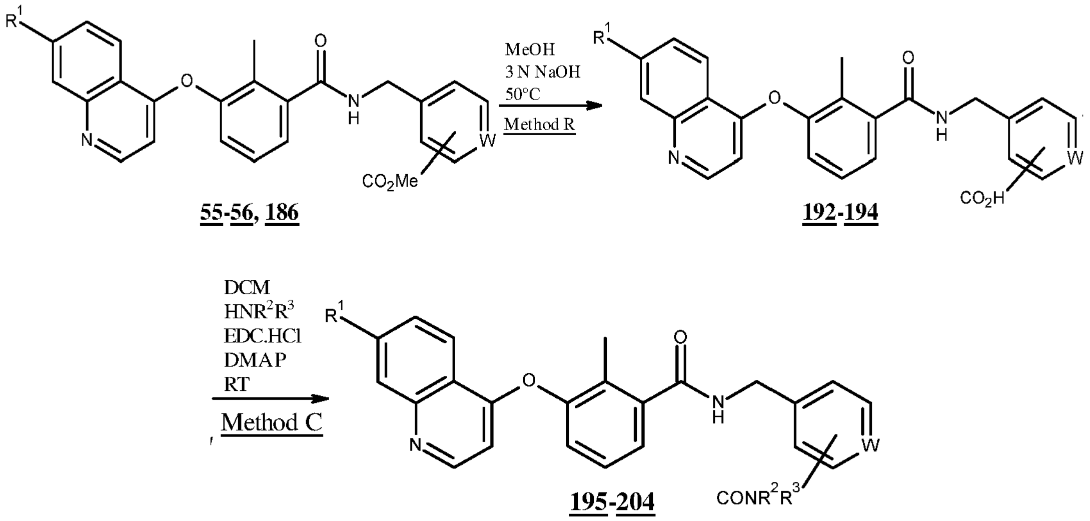


55-56, 186

192-194

195-204

The following compound **195** is an example illustrating these procedures.

**Method R:** To a solution of the appropriate ester compound (1 equiv.) in methanol (5 mL/mmol), was added sodium hydroxide (2N, 5 mL/mmol). The reaction mixture was stirred and heated at 50°C for 6h. MeOH was removed under reduced pressure. The mixture was then acidified by aqueous HCl 1N to pH 2-3 and concentrated. The crude was purified by reverse phase chromatography ($H_2O$/MeOH: 0 to 100%) to give the expected compound.

The following compound **192** is an example illustrating Method R:

Preparation of 3-[[[3-[(7-methoxy-4-quinolyl)oxy]-2-methylbenzoyl]amino]methyl]benzoic acid **(192):**

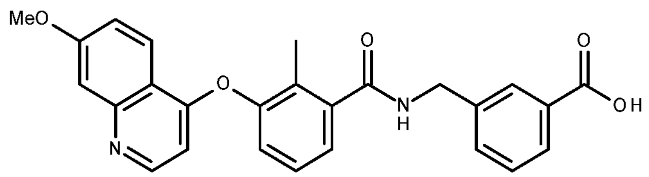

Compound **192** was synthesized from compound **55** (0.10 mmol) as a white powder in 64% yield (28 mg) according to the general method R. $^1$H NMR (400 MHz, DMSO-$d_6$) δ (ppm): 9.04 (t, *J* = 6.53 Hz, 1H), 8.61 (d, *J* = 5.12 Hz, 1H), 8.27 (d, *J* = 9.21 Hz, 1H), 7.96 (bs, 1H), 7.85-7.82 (m, 1H), 7.61-7.58 (m, 1H), 7.50-7.30 (m, 6H), 6.31 (d, *J* = 5.29 Hz, 1H), 4.52 (d, *J* = 6.12 Hz, 2H), 3.95 (s, 3H), 2.14 (s, 3H) ; MS (ESI, EI$^+$): *m/z*= 443.25 (MH$^+$).

Preparation of 3-[(7-methoxy-4-quinolyl)oxy]-2-methyl-N-[[4-(methylcarbamoyl)phenyl]methyl]benzamide **(195):**

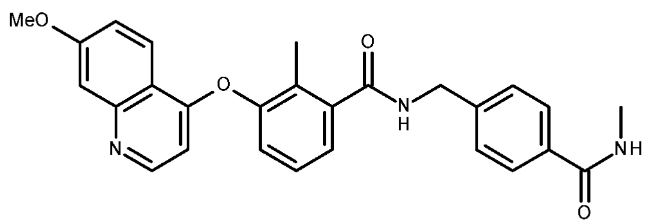

Compound **195** was synthesized from compound **193** (0.10 mmol) and methylamine (2M in THF, 0.11 mmol) as a white powder in 44% yield (20 mg) according to the general method C. $^1$H NMR (400 MHz, DMSO-$d_6$) δ (ppm): 9.00 (t, *J* = 6.52 Hz, 1H), 8.60 (d, *J* = 5.14 Hz, 1H), 8.41-8.38 (m, 1H), 8.26 (d, *J* = 9.16 Hz, 1H), 7.81 (d, *J* = 8.35 Hz, 2H), 7.45-7.38 (m, 5H), 7.33-7.30 (m, 2H), 6.31 (d, *J* = 5.22 Hz, 1H), 4.51 (d, *J* = 6.02 Hz, 2H), 3.95 (s, 3H), 2.78 (d, *J* = 4.58 Hz, 3H), 2.14 (s, 3H) ; MS (ESI, EI$^+$): *m/z*= 456.40 (MH$^+$).

**Example 18: Procedure for the preparation of poly-substituted halo-4-pyridine quinoline analogues 224-231**

The following procedures illustrate the preparation of poly-substituted halo-4-pyridine quinoline analogues **224-231.** The synthetic scheme is outlined below. The synthesis starts with the conversion of the substituted halo pyridine-4-carboxylic acid to the corresponding carboxamide derivatives (method S). The resulting substituted halo pyridine-4-carboxamide analogues were dehydrated in the presence of trifluoroacetic anhydre to give the nitrile compounds (method T). A reductive hydrogenation yielded to the corresponding substituted halo 4-pyridylmethanamine (method U). A final peptide coupling reaction (method V) with the quinoline derivatives was achieved. Examples of the structures of the final compounds can be found in Table 1.

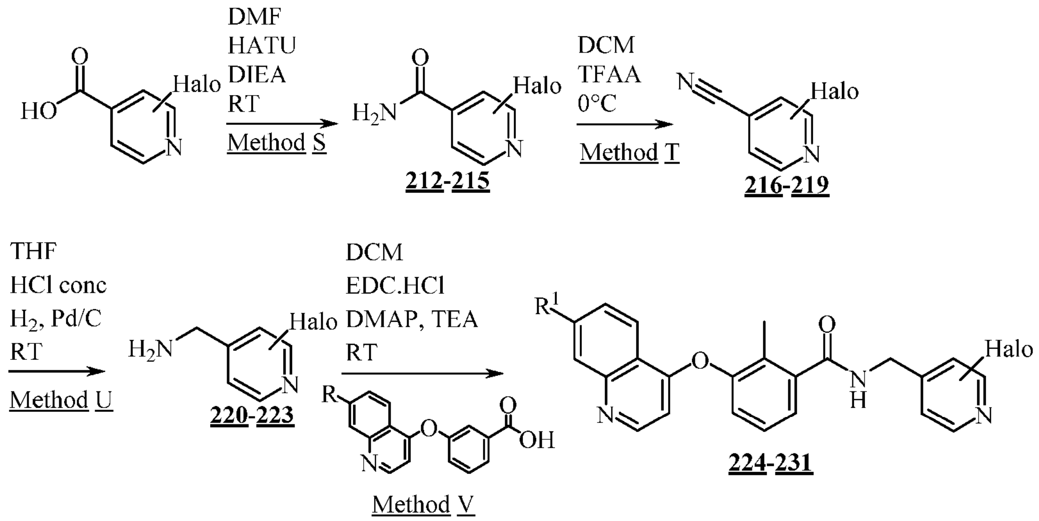


**Method** S: To a solution of the appropriate pyridine-4-carboxylic acid compound (1 equiv.) in DMF (1.5 mL/mmol), were added diisopropylethylamine (3 equiv.), HATU (1.3 equiv.) and ammonium chloride (1.3 equiv.) under azote atmosphere. The reaction mixture was stirred at room temperature for 2h. DMF was removed under reduced pressure and the crude was purified by flash column chromatography on Biotage (cyclohexane/EtOAc: 0 to 100%) to give the expected compound.

The following compound **214** is an example illustrating Method S:

Preparation of 2,6-difluoropyridine-4-carboxamide **(214):**

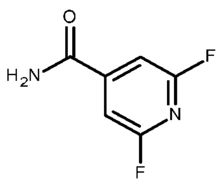

Intermediate **214** was synthesized from 2,6-difluoropyridine-4-carboxylic acid (1.61 mmol) and ammonium chloride (2.09 mmol) as a yellow solid in quantitative yield (274 mg) according to the general method S. MS (ESI, EI+): *m/z*= 159.25 ($MH^+$).

The following table illustrates intermediates **212-215** prepared from method S:

| Intermediate | Structure | Synthesis procedure |
|---|---|---|
| Compound **212** | 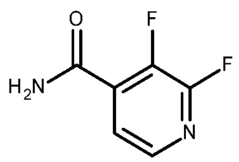 | Method S |
| Compound **213** | 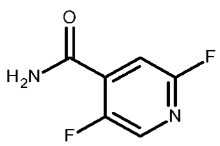 | Method S |

(continued)

| Intermediate | Structure | Synthesis procedure |
|---|---|---|
| Compound **214** | 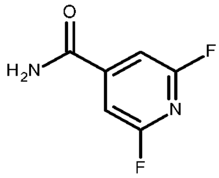 | Method S |
| Compound **215** | 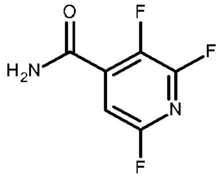 | Method S |

**Method T:** To a solution of the appropriate pyridine-4-carboxamide compound (1 equiv.) in DCM (5 mL/mmol), were added diisopropylethylamine (3 equiv.) and TFAA (2 equiv.) at 0°C under azote atmosphere. The reaction mixture was stirred at 0°C until completion (30 min-2h), then diluted with DCM and washed with brine. The resulting organic layer was dried over $Na_2SO_4$, filtered and carefully concentrated (bath temperature = 30 °C, Final pressure = 550 mbar) to give the nitrile compound with was used without further purification. The following compound **218** is an example illustrating Method T:

Preparation of 2,6-difluoropyridine-4-carbonitrile (218):

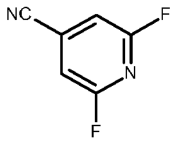

Intermediate **218** was synthesized from intermediate **214** (1.73 mmol) and TFAA (3.46 mmol) as a yellow oil in quantitative yield (243 mg, crude) according to the general method T.

The following table illustrates intermediates **216-219** prepared from method T:

| Intermediate | Structure | Synthesis procedure |
|---|---|---|
| Compound **216** | 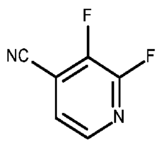 | Method T |
| Compound **217** | 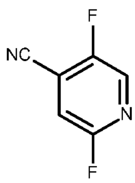 | Method T |
| Compound **218** | 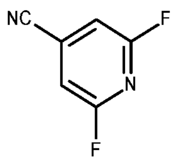 | Method T |

(continued)

| Intermediate | Structure | Synthesis procedure |
|---|---|---|
| Compound **219** | 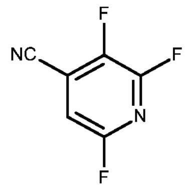 | Method T |

**Method U:** To a solution of the appropriate pyridine-4-carbonitrile compound (1 equiv.) in THF (5 mL/mmol), was added concentrated hydrochloride acid (37%, 0.5 mL/mmol) followed by Pd/C 10% (100% massique) under azote atmosphere. The reaction mixture was hydrogenated at room temperature for 4h-6h30. The crude was filtered through a celite pad and washed with THF. The filtrate was concentrated under reduced pressure to give the expected intermediate as a hydrochloride salt.

The following compound **222** is an example illustrating Method U:

<u>Preparation of (2,6-difluoro-4-pyridyl)methanamine (222):</u>

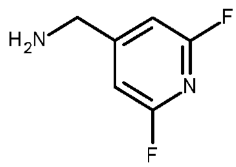

Intermediate **222** was synthesized from intermediate **218** (1.73 mmol) and Pd/C 10% (243 mg) as a yellow gum (493 mg, crude) according to the general method U. MS (ESI, EI$^+$): *m/z*= 145.30 (MH$^+$).

The following table illustrates intermediates **216-219** prepared from method U:

| Intermediate | Structure | Synthesis procedure |
|---|---|---|
| Compound **220** | 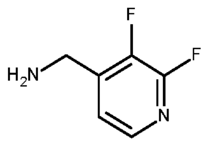 | Method U |
| Compound **221** | 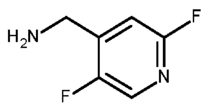 | Method U |
| Compound **222** | 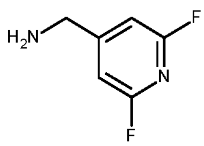 | Method U |
| Compound **223** | 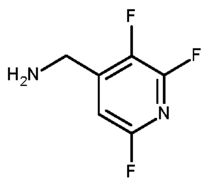 | Method U |

**Method V:** To a suspension of intermediate **2** (1 equiv.) in DCM (10 mL/mmol) under nitrogen were added DMAP (2.2 equiv.), EDC.HCl (2 equiv.), triethylamine (5 equiv.) and appropriate amine (excess). The reaction mixture was stirred

at room temperature overnight. The reaction mixture was diluted with DCM and washed with a saturated solution of $NH_4Cl$. The organic layers were dried over $Na_2SO_4$, filtered and concentrated under reduced pressure. The crude was purified by preparative HPLC-MS (column: Shim-pack GIS C18, 5 μm, 100 x 30 mm) using a linear gradient from 30 % to 100% of $CH_3CN$ + 0.1% HCOOH in $H_2O$ + 0.1% HCOOH to give the expected compound. The following compound **228** is an example illustrating Method V:

Preparation of N-[(2,6-difluoro-4-pyridyl)methyl]-3-[(7-methoxy-4-quinolyl)oxy]-2-methyl-benzamide (**228**):

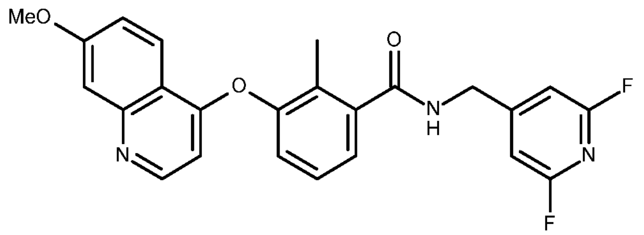

Compound **228** was synthesized from intermediate **2f** (0.15 mmol) and crude intermediate **222** (218 mg) as a grey powder in 56% yield (36 mg) according to the general method V. $^1H$ NMR (400 MHz, DMSO-$d_6$) δ (ppm): 9.12 (t, $J$ = 5.88 Hz, 1H), 8.64 (d, $J$ = 5.33 Hz, 1H), 8.29 (d, $J$ = 9.09 Hz, 1H), 7.51-7.44 (m, 3H), 7.37-7.33 (m, 2H), 7.14 (bs, 2H), 6.34 (d, $J$ = 5.32 Hz, 1H), 4.59 (d, $J$ = 6.01 Hz, 2H), 3.96 (s, 3H), 2.15 (s, 3H) ; $^{19}F$ NMR (400 MHz, DMSO-$d_6$) δ (ppm): -71.22 ; MS (ESI, EI$^+$): $m/z$= 436.25 (MH$^+$).

**Example 19: Procedure for the preparation of polysubstituted quinoline analogues 279-285**

The following procedures illustrate the preparation of analogues **279-285.** The synthetic scheme is outlined below. The synthesis starts with the condensation of Meldrum's acid, triethylorthoformate and substituted aniline (Method W) following a thermolysis in Dowtherm (Method X). A chlorination in the presence of thionyl chloride and catalytic amount of DMF gave the 4-chloroquinoline analogues (Method Y). Finally, the preparation of 3-(4-quinolyloxy)benzamide derivatives **279-285** is achieved according to the general method I, described in example 2. Examples of the final compounds can be found in table 1.

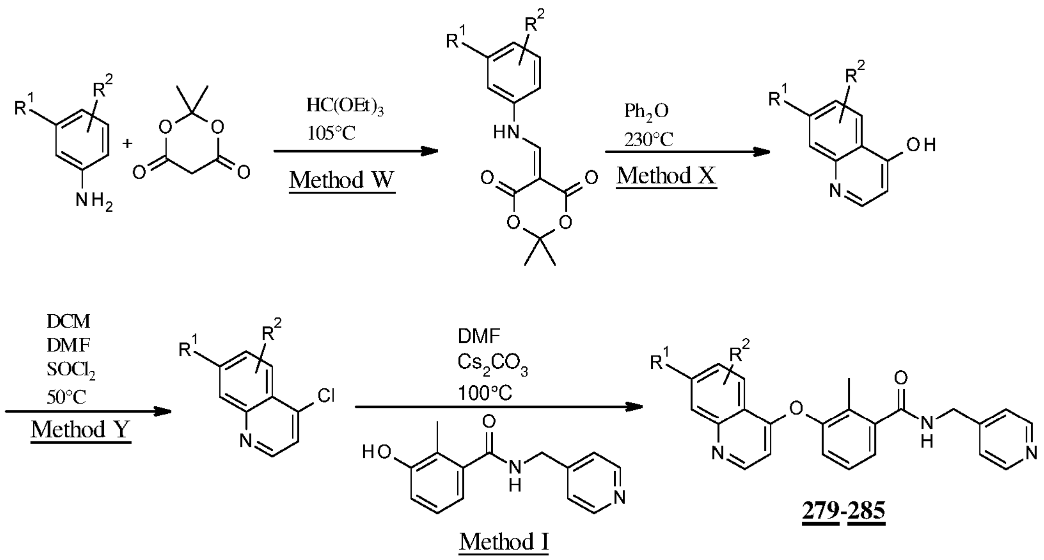


**Method W:** A solution of Meldrum's acid (1 equiv.) in triethylorthoformate (10 equiv.) was heated at 105°C for 1h under nitrogen. The appropriate substituted aniline (1 equiv.) was then added and the reaction mixture was stirred at 105°C for an additional hour. After cooling to room temperature, a solid appeared and pentane was added. The solid was filtered, washed with pentane and dried under vacuum to give the expected intermediate which was used without further purification.

The following compound **276** is an example illustrating Method W:

Preparation of 5-[(3,5-difluoroanilino)methylene]-2,2-dimethyl-1,3-dioxane-4,6-dione (276):

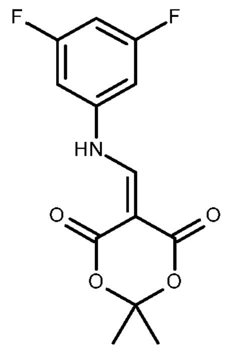

Intermediate **276** was synthesized from 3,5-difluoroaniline (7.74 mmol) as a white solid in 96% yield according to the general method W. MS (ESI, EI+): m/z= 284.3 (MH+).

**Method X:** A solution of the appropriate intermediate (1 equiv.) in diphenyl ether (2.5 mL/mmol) was heated at 230°C for 1h. After cooling to room temperature, a solid appeared and pentane was added. The solid was filtered, washed with pentane and dried under vacuum to give the expected intermediate which was used without further purification.

The following compound **277** is an example illustrating Method X:

Preparation of 5,7-difluoroquinolin-4-ol **(277):**

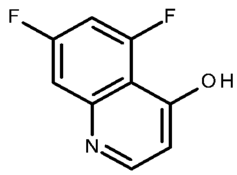

Intermediate **277** was synthesized from compound **276** (7.45 mmol) as a beige powder in 65% yield according to the general method X. MS (ESI, EI+): m/z= 182.2 (MH+).

**Method Y:** To a stirred solution of the appropriate intermediate (1 equiv.) in dichloromethane, were added thionyl chloride (10 equiv.) and two drops of DMF under nitrogen. The reaction mixture was heated at 80°C for 1h. The reaction mixture was concentrated under reduced pressure then quenched slowly with ammonia solution 7N in methanol until pH = 10. The mixture was concentrated and co-evaporated with toluene twice. The crude was purified by flash column chromatography on Biotage (DCM/MeOH: 0 to 5%) to give the expected intermediate.

The following compound **278** is an example illustrating Method Y:

Preparation of 4-chloro-5,7-difluoro-quinoline **(278):**

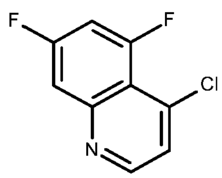

Intermediate **278** was synthesized from compound **277** (1.10 mmol) as a white powder in 67% yield according to the general method Y. MS (ESI, EI+): m/z= 280.3 (MH+).

Preparation of 3-[(5,7-difluoro-4-quinolyl)oxyl-2-methyl-N-(4-pyridylmethyl)benzamide **(279):**

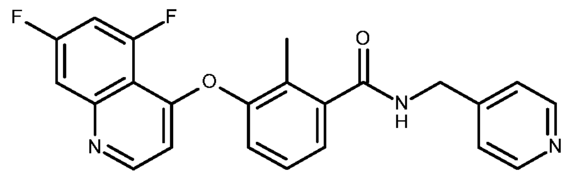

Compound **279** was synthesized from intermediate **278** (0.15 mmol) and intermediate **81e** (0.15 mmol) as a white powder in 20% yield (12 mg) according to the general method I. $^1$H NMR (400 MHz, DMSO-$d_6$) δ (ppm): 9.05 (t, *J* = 5.80 Hz, 1H), 8.73 (d, *J* = 5.33 Hz, 1H), 8.54-8.53 (m, 2H), 7.70-7.67 (m, 1H), 7.64-7.58 (m, 1H), 7.46-7.44 (m, 2H), 7.36-7.34 (m, 3H), 6.51 (d, *J* = 5.30 Hz, 1H), 4.50 (d, J= 6.02 Hz, 2H), 2.16 (s, 3H) ; MS (ESI, EI+): m/z= 406.20 (MH+).

**Example 20: Procedure for the preparation of 7-substituted quinoline analogues 288-291**

The following procedures illustrate the preparation of analogues **288-291.** The synthetic scheme is outlined below. The synthesis starts with a peptide coupling reaction between 4-chloroquinoline-7-carboxylic acid and the appropriate amine (Method Z). For the synthesis of 4-chloroquinoline-7-carboxamide, method S, described in example 18, was used. The preparation of 7-substituted 3-(4-quinolyloxy)benzamide derivatives **288-291** is achieved according to the general method I, described in example 2. Examples of the final compounds can be found in table 1.

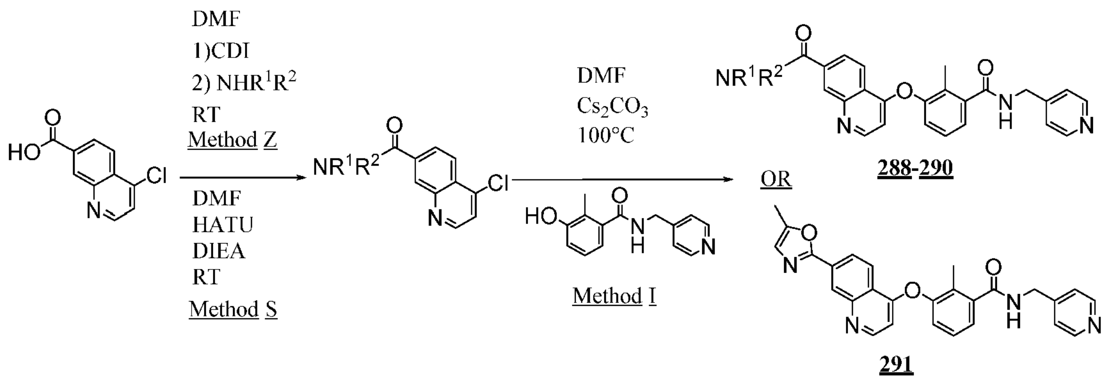


**Method Z:** To a stirred solution of 4-chloroquinoline-7-carboxylic acid (1 equiv.) in DMF (10 mL/mmol) was added carbonyldiimidazole (1.2 equiv.) under nitrogen. The reaction mixture was stirred at room temperature for 30 min. Then, the appropriate amine (1.2 equiv.) was added and stirred overnight. The reaction mixture was concentrated and co-evaporated with toluene twice under reduced pressure to give the expected intermediate which was used without further purification.

The following compounds **286-287** are examples illustrating Method Z.

Preparation of 4-chloro-N-prop-2-ynyl-quinoline-7-carboxamide **(286)**

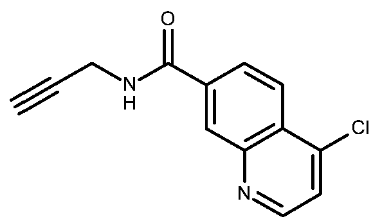

Intermediate **286** was synthesized from 4-chloroquinoline-7-carboxylic acid (0.15 mmol) and propargyl amine (0.17 mmol) as a colorless gum in a quantitative yield according to the general method Z.

Preparation of 4-chloroquinoline-7-carboxamide **(287)**

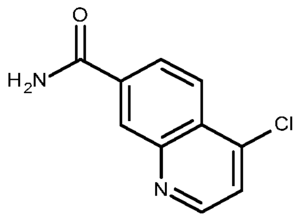

Intermediate **287** was synthesized from 4-chloroquinoline-7-carboxylic acid (0.50 mmol), and ammonium chloride (0.65 mmol) as a beige solid in a quantitative yield according to the general method S.

Preparation of 4-[2-methyl-3-(4-pyridylmethylcarbamoyl)phenoxy]quinoline-7-carboxamide **(288)**

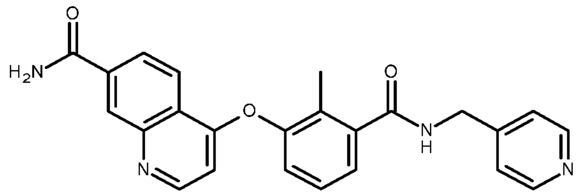

Compound **288** was synthesized from intermediate **287** (0.50 mmol) and intermediate **81e** (0.50 mmol) as a white powder in 25% yield (50 mg) according to the general method I. $^1$H NMR (400 MHz, DMSO-$d_6$) δ (ppm): 9.06 (t, *J* = 6.11 Hz, 1H), 8.77 (d, *J* = 5.10 Hz, 1H), 8.60-8.59 (m, 1H), 8.55-8.53 (m, 2H), 8.44 (d, *J* = 8.74 Hz, 1H), 8.33 (bs, 1H), 8.13 (dd, *J* = 8.74 and 1.64 Hz, 1H), 7.63 (bs, 1H), 7.47-7.46 (m, 2H), 7.40-7.36 (m, 3H), 6.52 (d, *J* = 5.22 Hz, 1H), 4.50 (d, *J* = 6.10 Hz, 2H), 2.16 (s, 3H) ; MS (ESI, EI+): m/z= 413.15 (MH+).

Preparation of 2-methyl-3-[[7-(5-methyloxazol-2-yl)-4-quinolyl]oxy]-N-(4-pyridylmethyl)benzamide **(291)**

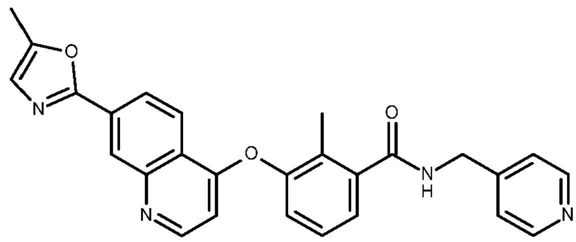

Compound **291** was synthesized from intermediate **286** (0.15 mmol) and intermediate **81e** (0.15 mmol) as a white powder in 14% yield (9 mg) according to the general method I. $^1$H NMR (400 MHz, DMSO-$d_6$) δ (ppm): 9.07 (t, *J* = 6.05 Hz, 1H), 8.77 (d, *J* = 5.15 Hz, 1H), 8.55-8.51 (m, 4H), 8.23 (dd, *J* = 8.75 and 1.44 Hz, 1H), 7.47-7.44 (m, 2H), 7.41-7.36 (m, 3H), 7.14 (d, *J* = 1.25 Hz, 1H), 6.50 (d, *J* = 5.17 Hz, 1H), 4.50 (d, *J* = 5.96 Hz, 2H), 2.48 (s, 3H), 2.18 (s, 3H) ; MS (ESI, EI+): m/z= 451.15 (MH+).

**Example 21: Procedure for the preparation of 7-heteroaryl quinoline analogues 293-294**

The following procedures illustrate the preparation of analogues **293-294,** the synthetic scheme is outlined below.

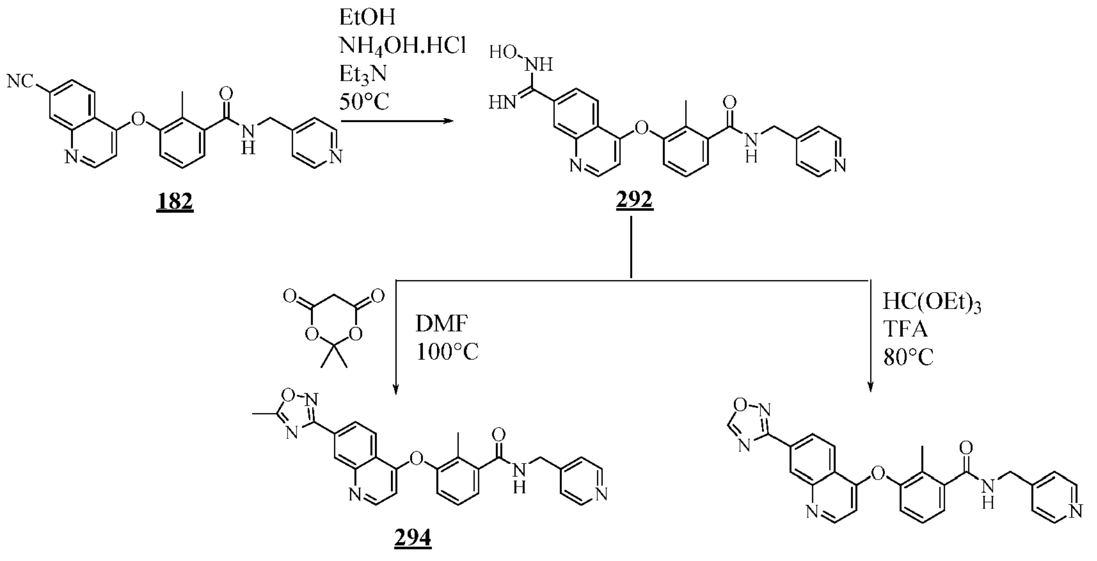


182 292 294 293

Preparation of 3-[[7-(N-hydroxycarbamimidoyl)-4-quinolyl]oxy]-2-methyl-N-(4-pyridylmethyl)benzamide **(292)**

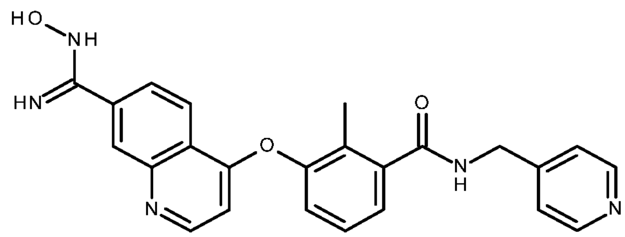

To a stirred solution of compound **182** (0.38 mmol) in ethanol (4 mL), were added hydroxylamine hydrochloride (1.14 mmol) and triethylamine (1.14 mmol) under nitrogen atmosphere. The reaction mixture was heated at 50°C for 1h30, then concentrated under reduced pressure and dried under vaccum to give the expected intermediate **292** as a white powder in a quantitative yield and used without further purification. MS (ESI, EI+): m/z= 428.15 (MH+).

Preparation of 2-methyl-3-[[7-(1,2,4-oxadiazol-3-yl)-4-quinolyl]oxy]-N-(4-pyridylmethyl)benzamide **(293)**

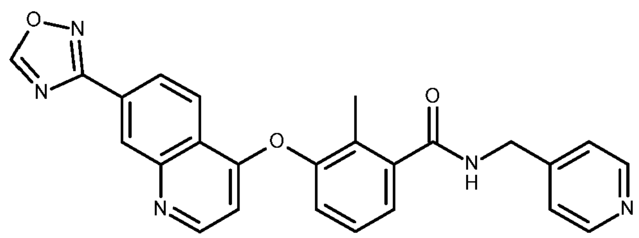

To a stirred solution of compound **292** (0.12 mmol) in triethylorthoformate (1 mL), was added trifluoroacetic acid (2 drops) under nitrogen atmosphere. The reaction mixture was heated at 60°C for 2h and at 80°C for 2h. The reaction mixture was then diluted with DCM and the organic layer was washed with a saturated solution of ammonium chloride. The aqueous layer was extracted once with DCM. The combined organic layers were dried over $Na_2SO_4$, filtered and concentrated under reduced pressure. The crude was purified by flash column chromatography on Biotage (DCM/MeOH: 0 to 10%) and reverse phase chromatography ($H_2O$/MeOH: 0 to 100%) to give the expected compound **293** as a white powder in 18% yield (9 mg). $^1$H NMR (400 MHz, DMSO-*d*$_6$) δ (ppm): 9.85 (bs, 1H), 9.08 (t, *J* = 6.02 Hz, 1H), 8.81 (d, *J* = 5.15 Hz, 1H), 8.69 (bs, 1H), 8.59 (d, *J* = 8.83 Hz, 1H), 8.54 (d, *J* = 4.34 Hz, 2H), 8.30 (d, *J* = 8.56 Hz, 1H), 7.48-7.45 (m, 2H),

7.42-7.36 (m, 3H), 6.56 (d, *J* = 5.08 Hz, 1H), 4.50 (d, *J* = 5.59 Hz, 2H), 2.18 (s, 3H) ; MS (ESI, EI+): m/z= 438.05 (MH+).

Preparation of 2-methyl-3-[[7-(5-methyl-1,2,4-oxadiazol-3-yl)-4-quinolyl]oxy]-N-(4-pyridylmethyl)benzamide **(294)**

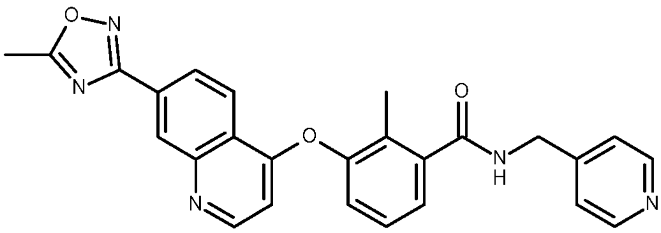

To a stirred solution of compound **292** (0.16 mmol) in DMF (2 mL), was added meldrum's acid (0.24 mmol) under nitrogen atmosphere. The reaction mixture was heated at 100°C for 1h. The reaction mixture was concentrated under reduced pressure and the crude was purified by flash column chromatography on Biotage (DCM/MeOH: 0 to 10%) and by preparative HPLC-MS (column: Shim-pack GIS C18, 5 µm, 100 x 30 mm) using a linear gradient from 5 % to 60% of $CH_3CN$ + 0.1% HCOOH in $H_2O$ + 0.1% HCOOH to give the expected compound **294** as a white powder in 18% yield (13 mg). $^1H$ NMR (400 MHz, DMSO-$d_6$) δ (ppm): 9.07 (t, *J* = 5.96 Hz, 1H), 8.80 (d, *J* = 5.18 Hz, 1H), 8.64 (d, *J* = 1.28 Hz, 1H), 8.58-8.53 (m, 3H), 8.26 (dd, *J* = 8.64 and 1.56 Hz, 1H), 7.48-7.45 (m, 2H), 7.42-7.35 (m, 3H), 6.56 (d, *J* = 5.15 Hz, 1H), 4.50 (d, *J* = 6.00 Hz, 2H), 2.75 (s, 3H), 2.18 (s, 3H) ; MS (ESI, EI+): m/z= 452.10 (MH+).

**Example 22: Procedure for the preparation of 7-heteroaryl quinoline analogue 297**

The following procedures illustrate the preparation of analogue **297,** the synthetic scheme is outlined below.

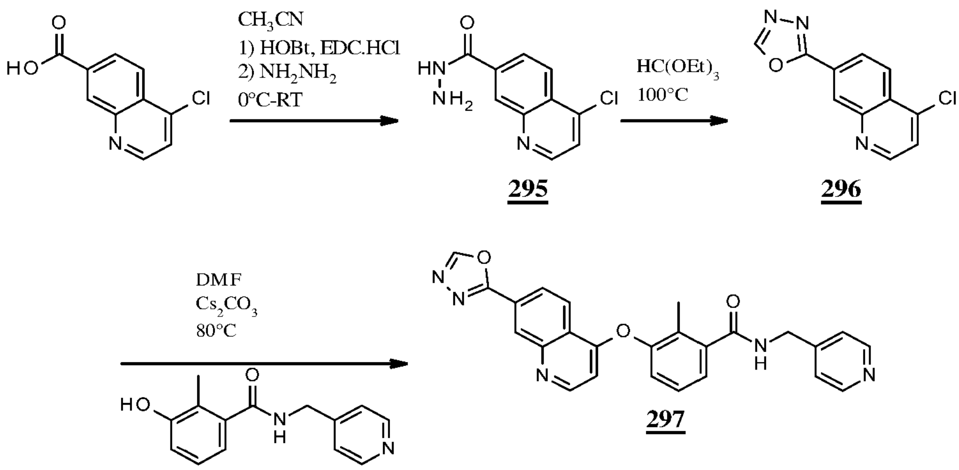


295

296

297

Preparation of 4-chloroquinoline-7-carbohydrazide **(295)**

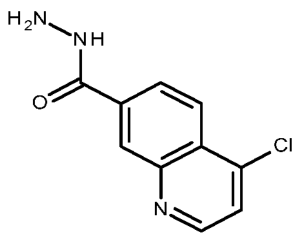

To a stirred solution of 4-chloroquinoline-7-carboxylic acid (0.19 mmol) in acetonitrile (2 mL), were added EDC.HCl (0.23 mmol) and HOBt (0.23 mmol) under nitrogen atmosphere. The reaction mixture was stirred at room

temperature for 1h30. After cooling at 0°C, hydrazine (1M in THF, 0.39 mmol) was added dropwise. The reaction mixture was allowed to warm to room temperature and stirred overnight. Acetonitrile was removed under reduced pressure and the crude was taken up in water to give a solid which was filtered, washed with water and dried over $P_2O_5$ in vacuum desiccators to give the expected intermediate **295** as a white powder in 86% yield and used without further purification.

Preparation of 2-(4-chloro-7-quinolyl)-1,3,4-oxadiazole **(296)**

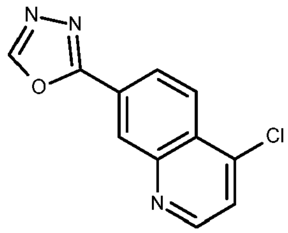

A stirred solution of intermediate **295** (0.16 mmol) in triethylorthoformate (1.5 mL) was heated at 100°C for 1h30. The reaction mixture was concentrated under reduced pressure to give the expected intermediate **296** as a white powder in a quantitative yield and used without further purification. MS (ESI, EI+): m/z= 232.05 (MH+).

Preparation of 2-methyl-3-[[7-(1,3,4-oxadiazol-2-yl)-4-quinolyl]oxy]-N-(4-pyridylmethyl)benzamide **(297)**

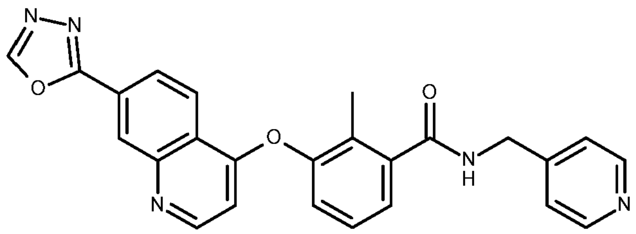

To a stirred solution of compound **296** (0.09 mmol) in DMF (2 mL), were added intermediate **81e** (0.09 mmol) and cesium carbonate (0.13 mmol) under nitrogen atmosphere. The reaction mixture was heated at 80°C for 1h30. The reaction mixture was concentrated under reduced pressure and the crude was purified by flash column chromatography on Biotage (DCM/MeOH: 0 to 10%) and reverse phase chromatography ($H_2O$/MeOH: 0 to 100%) to give the expected compound **297** as a white powder in 8% yield (3 mg). $^1$H NMR (400 MHz, DMSO-$d_6$) δ (ppm): 9.48 (s, 1H), 9.08 (t, *J* = 5.83 Hz, 1H), 8.82 (d, *J* = 5.12 Hz, 1H), 8.64-8.61 (m, 2H), 8.54-8.53 (m, 2H), 8.31 (dd, *J* = 8.78 and 1.63 Hz, 1H), 7.49-7.46 (m, 2H), 7.43-7.36 (m, 3H), 6.58 (d, *J* = 5.12 Hz, 1H), 4.50 (d, *J* = 6.02 Hz, 2H), 2.18 (s, 3H) ; MS (ESI, EI+): m/z= 438.10 (MH+).

**Example 23: Procedure for the preparation of 7-heteroaryl quinoline analogue 301**

The following procedures illustrate the preparation of analogue **301,** the synthetic scheme is outlined below.

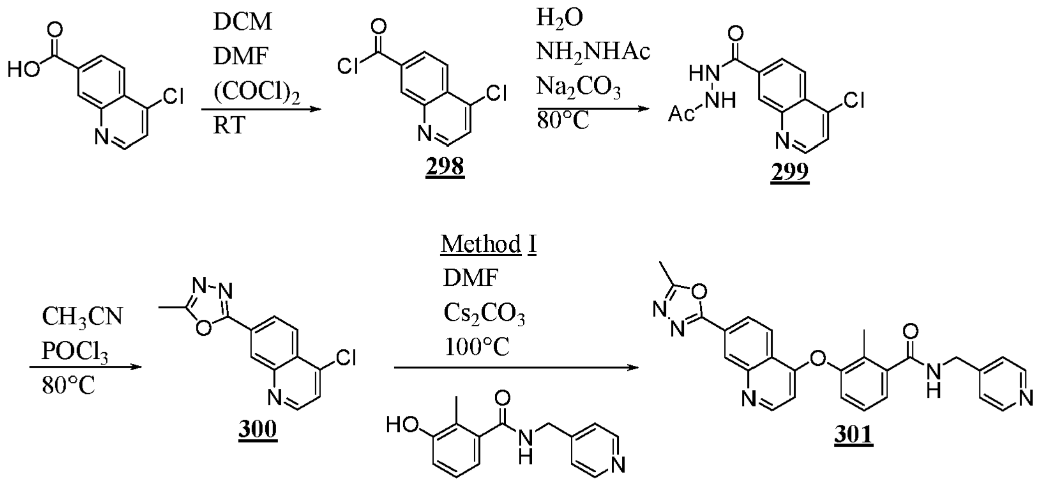


Preparation of 4-chloroquinoline-7-carbonyl chloride **(298)**

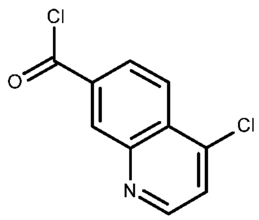

To a stirred solution of 4-chloroquinoline-7-carboxylic acid (0.24 mmol) in DCM (2.5 mL), were added oxalyl chloride (0.48 mmol) and DMF (2 drops) under nitrogen atmosphere. The reaction mixture was stirred at room temperature for 1h. The reaction mixture was concentrated under reduced pressure to give the expected intermediate **298** as a white powder in a quantitative yield and used without further purification.

Preparation of N'-acetyl-4-chloro-quinoline-7-carbohydrazide **(299)**

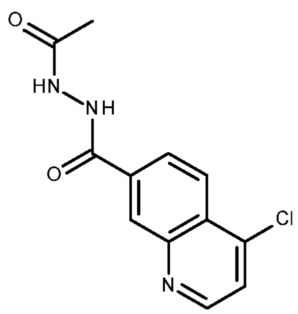

To a stirred solution of acetyl hydrazine (0.24 mmol) and sodium carbonate (0.24) in water (2 mL), was added intermediate **298** (0.24 mmol) at 0°C under nitrogen atmosphere. The reaction mixture was then stirred at room temperature for 1h. The reaction mixture was concentrated under reduced pressure and purified by flash column chromatography on Biotage (DCM/MeOH: 0 to 15%) to give the expected intermediate **299** as a white powder in 89% yield. MS (ESI, EI+): m/z= 264.05 (MH+).

Preparation of 2-(4-chloro-7-quinolyl)-5-methyl-1,3,4-oxadiazole **(300)**

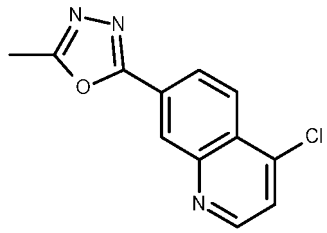

To a stirred solution of intermediate **299** (0.15 mmol) in acetonitrile (4 mL), was added $POCl_3$ (0.30 mmol) under nitrogen atmosphere. The reaction mixture was heated at 80°C for 1h. The reaction mixture was concentrated under reduced pressure and purified by flash column chromatography on Biotage (DCM/MeOH: 0 to 10%) to give the expected intermediate **300** as a white powder in 60% yield. MS (ESI, EI+): m/z= 246.05 (MH+).

Preparation of 2-methyl-3-[[7-(5-methyl-1,3,4-oxadiazol-2-yl)-4-quinolyl]oxy]-N-(4-pyridylmethyl)benzamide **(301)**

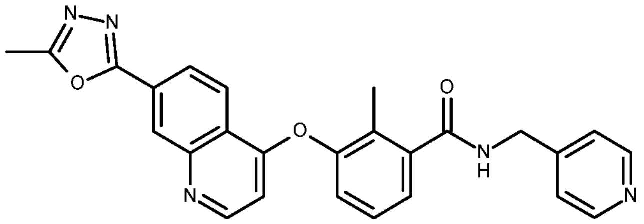

Compound **301** was synthesized from intermediate **300** (0.09 mmol) and intermediate **81e** (0.09 mmol) as a white powder in 33% yield (13 mg) according to the general method I. $^1$H NMR (400 MHz, DMSO-$d_6$) δ (ppm): 9.08 (t, $J$ = 6.00 Hz, 1H), 8.82 (d, $J$ = 5.19 Hz, 1H), 8.61-8.53 (m, 4H), 8.26 (dd, $J$ = 8.70 and 1.69 Hz, 1H), 7.49-7.45 (m, 2H), 7.42-7.36 (m, 3H), 6.57 (d, $J$ = 5.11 Hz, 1H), 4.50 (d, $J$ = 6.05 Hz, 2H), 2.67 (s, 3H), 2.18 (s, 3H) ; MS (ESI, EI+): m/z= 452.15 (MH+).

**Example 24: Procedure for the preparation of 7-heteroaryl quinoline analogues 303-316**

The following procedures illustrate the preparation of analogues **303-316,** the synthetic scheme is outlined below. The synthesis starts with the preparation of 7-boronic acid quinoline pinacol ester derivative **302,** then Suzuki-Miyaura cross coupling reactions afford the analogues **303-316** according to the general procedure K2, described in example 9. Examples of the final compounds can be found in table 1.

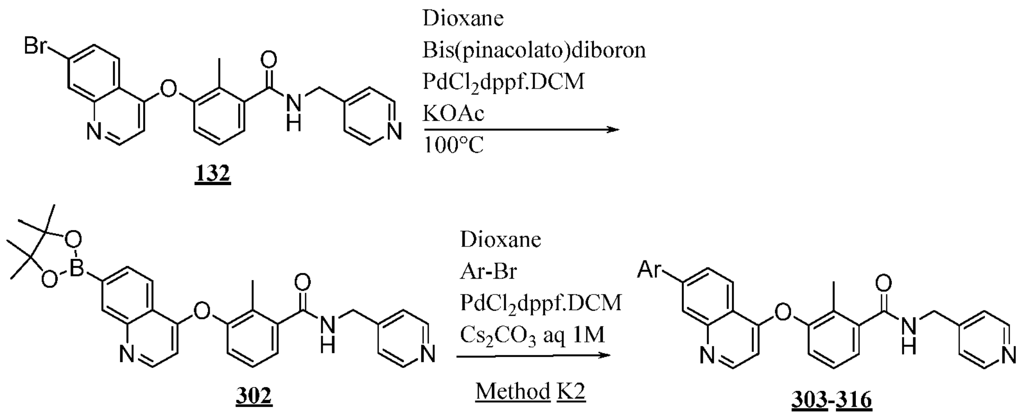


The following compound **303** is an example illustrating theses procedures.

Preparation of 2-methyl-N-(4-pyridylmethyl)-3-[[7-(4,4,5,5-tetramethyl-1,3,2-dioxaborolan-2-yl)-4-quinolyl]oxy]benzamide **(302)**

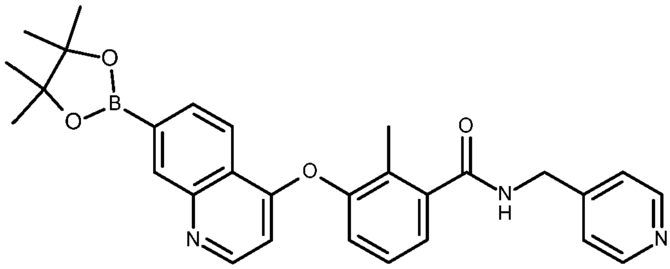

To a stirred solution of intermediate **132** (0.10 mmol) in dioxane (1 mL), were added bis(pinacolato)diboron (0.15 mmol), potassium acetate (0.30 mmol) and 1,1'-bis(diphenylphosphino)ferrocene]dichloropalladium(II).DCM (0.10 equiv.) under azote atmosphere. The reaction mixture was heated at 100°C for 1h. The reaction mixture was then diluted with DCM and the organic layer was washed with brine. The aqueous layer was extracted with DCM three times and the combined organic layers were dried over $Na_2SO_4$, filtered and concentrated under reduced pressure to give the expected intermediate **302** as a brown gum in a quantitative yield and used without further purification.

Preparation of 2-methyl-3-[[7-(3-methylimidazol-4-yl)-4-quinolyl]oxy]-N-(4-pyridylmethyl)benzamide **(303)**

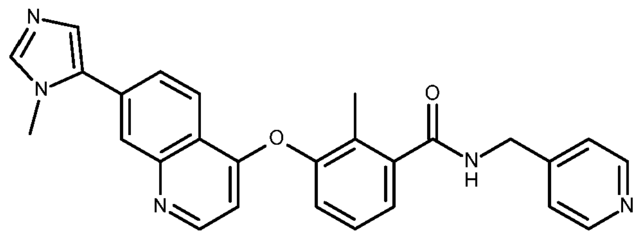

Compound **303** was synthesized from intermediate **302** (0.10 mmol) and 5-bromo-1-methyl-1H-imidazole (0.10 mmol) as a green powder in 42% yield (19 mg) according to the general method K2. $^1$H NMR (400 MHz, DMSO-$d_6$) δ (ppm): 9.07 (t, *J* = 6.31 Hz, 1H), 8.73 (d, *J* = 4.98 Hz, 1H), 8.54 (d, *J* = 5.76 Hz, 2H), 8.43 (d, *J* = 8.67 Hz, 1H), 8.15 (bs, 1H), 7.87-7.83 (m, 2H), 7.47-7.33 (m, 6H), 6.47 (d, *J* = 5.20 Hz, 1H), 4.50 (d, *J* = 6.02 Hz, 2H), 3.85 (s, 3H), 2.18 (s, 3H) ; MS (ESI, EI+): m/z= 450.15 (MH+).

**Example 25: Procedure for the preparation of 7-heteroaryl quinoline analogues 318-334**

The following procedures illustrate the preparation of analogues **318-334,** the synthetic scheme is outlined below. A copper cross-coupling reaction with the appropriate 7-bromo quinoline derivatives and (substituted) pyrrole or pyrazole is achieved to afford the expected 7- pyrrole or pyrazole analogues. In case where reactions are conducted with ester derivatives, saponification according to the general method B and peptide coupling according to the general method C, described in example 1, are required to give the final compounds. Examples of the final compounds can be found in table 1.

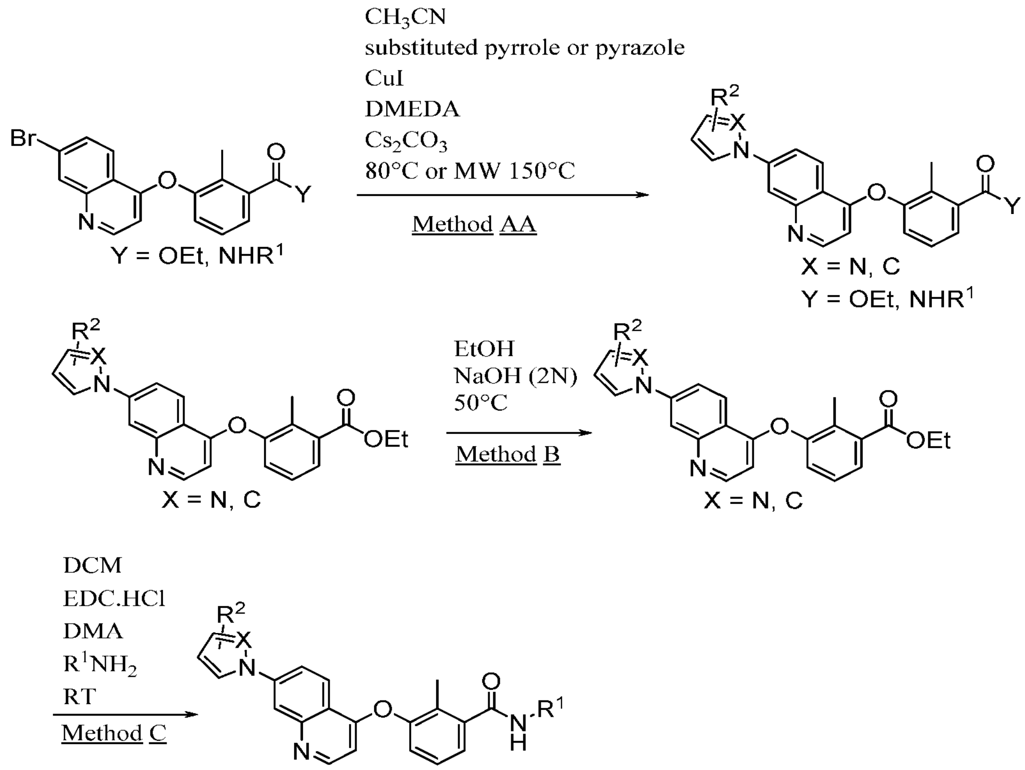


**Method AA:** The appropriate 7-bromo quinoline derivative (1 equiv.), copper iodide (2.2 equiv.) and cesium carbonate (4 equiv.) were placed in an oven-dried screw-cap test tube. The tube was then evacuated and backfilled with nitrogen (this sequence was repeated three times). Acetonitrile (10 mL/mmol), DMEDA (3.5 equiv.) and the appropriate (substituted) pyrrole or pyrazole (2.1 equiv.) were added. The reaction mixture was heated at 80°C overnight or heated under microwave irradiation at 150°C for 1h30. The reaction mixture was then concentrated under reduced pressure and purified by flash column chromatography on Biotage (DCM/MeOH: 0 to 10%) and reverse phase chromatography ($H_2O$/MeOH: 0 to 100%) in case of amide derivative to give the expected compound.

The following compound **318** is an example illustrating Method AA:

Preparation of 2-methyl-3-[(7-pyrazol-1-yl-4-quinolyl)oxy]-N-(4-pyridylmethyl)benzamide **(318)**

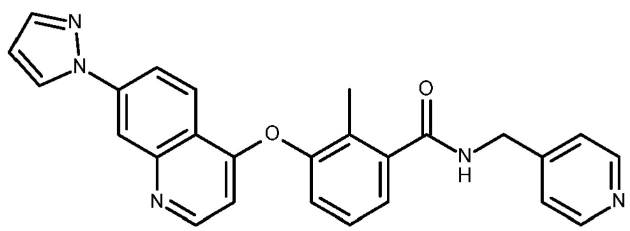

Compound **318** was synthesized from intermediate **132** (0.22 mmol) and 1H-pyrazole (0.47 mmol), as a white powder in 47% yield (45 mg) according to the general method AA. $^1$H NMR (400 MHz, DMSO-$d_6$) δ (ppm): 9.07 (t, $J$ = 6.09 Hz, 1H), 8.82 (d, $J$ = 2.57 Hz, 1H), 8.73 (d, $J$ = 5.16 Hz, 1H), 8.55-8.47 (m, 4H), 8.30 (dd, $J$ = 9.10 and 2.29 Hz, 1H), 7.88 (d, $J$ = 1.63 Hz, 1H), 7.47-7.35 (m, 5H), 6.66-6.65 (m, 1H), 6.44 (d, $J$ = 5.15 Hz, 1H), 4.50 (d, $J$ = 5.98 Hz, 2H), 2.18 (s, 3H) ; MS (ESI, El+): m/z= 436.10 (MH+).

**Example 26: Procedure for the preparation of 7-triazole quinoline analogues 336-338**

The following procedures illustrate the preparation of analogues **336-338,** the synthetic scheme is outlined below. The synthesis starts with the preparation of 7-azido quinoline derivatives (Method AB). A copper-catalyzed azide-alkyne cycloaddition leads to the expected triazole derivatives (Methode AC). In case where reactions are conducted with

ester derivatives, saponification according to the general method B and peptide coupling according to the general method C, described in example 1, are required to give the final compounds. Examples of the final compounds can be found in table 1.

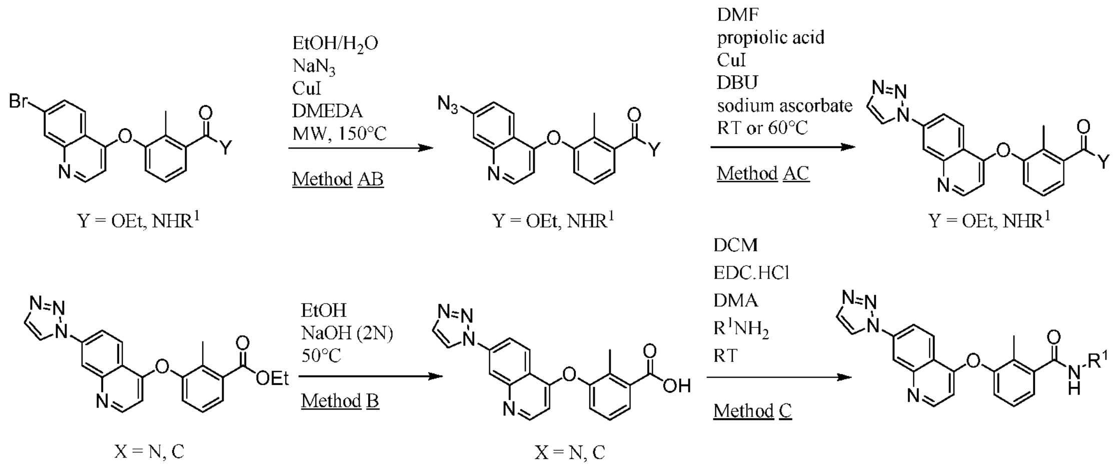


**Method AB:** The appropriate 7-bromo quinoline derivative (1 equiv.), sodium azide (2.5 equiv.) and copper iodide (0.3 equiv.) were placed in an oven-dried screw-cap test tube. The tube was then evacuated and backfilled with nitrogen (this sequence was repeated three times). EtOH/$H_2O$ (7/3, 4 mL/mmol) and DMEDA (0.6 equiv.) were added. The reaction mixture was heated under microwave irradiation at 100°C for 15min. The reaction mixture was then concentrated under reduced pressure and purified by flash column chromatography on Biotage (DCM/MeOH: 0 to 10%) to give the expected compound.

The following compound **335** is an example illustrating Method AB:

Preparation of 3-[(7-azido-4-quinolyl)oxy]-N-[(4-pyridyl)methyl]-2-methyl-benzamide **(335)**

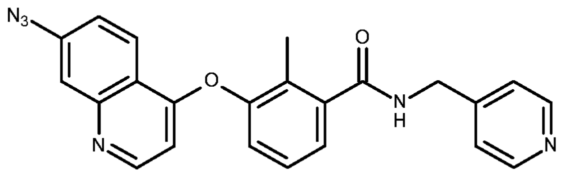

Compound **335** was synthesized from intermediate **132** (0.07 mmol) as a yellow oil in 77% yield according to the general method AB. MS (ESI, EI+): m/z= 447.15 (MH+).

**Method AC:** To a stirred solution of the appropriate 7-azido quinoline derivative (1 equiv) in DMF (10 mL/mmol), were added copper iodide (0.2 equiv.), sodium ascorbate (0.4 equiv.), propiolic acid (2 equiv.) and DBU (0.5 - 1 equiv) under azote atmosphere. The reaction mixture was stirred at room temperature overnight or heated at 60°C for 1h. The reaction mixture was then concentrated under reduced pressure, co-evaporated with toluene twice and purified by flash column chromatography on Biotage (DCM/MeOH: 0 to 10%) and reverse phase chromatography ($H_2O$/MeOH: 0 to 100%) in case of amide derivative to give the expected compound.

The following compound **336** is an example illustrating Method AC:

Preparation of 2-methyl-N-(4-pyridylmethyl)-3-[[7-(triazol-1-yl)-4-quinolylloxylbenzamide **(336)**

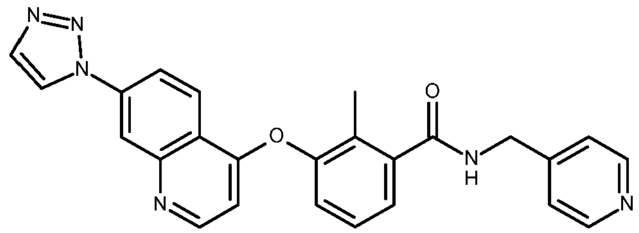

Compound **336** was synthesized from intermediate **335** (0.05 mmol) and propiolic acid (0.10 mmol) as a white powder in 63% yield (13 mg) according to the general method AC. $^1$H NMR (400 MHz, DMSO-$d_6$) δ (ppm): 9.16 (d, *J* = 1.19 Hz, 1H), 9.07 (t, *J* = 5.89 Hz, 1H), 8.80 (d, *J* = 5.12 Hz, 1H), 8.63 (bs, 1H), 8.61-8.60 (m, 1H), 8.54-8.53 (m, 2H), 8.35 (dd, *J* = 8.99 and 2.26 Hz, 1H), 8.09 (d, *J* = 1.15 Hz, 1H), 7.49-7.36 (m, 5H), 6.52 (d, *J* = 5.30 Hz, 1H), 4.50 (d, *J* = 5.94 Hz, 2H), 2.18 (s, 3H) ; MS (ESI, EI+): m/z= 437.10 (MH+).

**Example 27: Procedure for the preparation of 7-triazole quinoline analogues 339-340**

The following procedures illustrate the preparation of analogues **339-340,** the synthetic scheme is outlined below. Examples of the final compounds can be found in table 1.

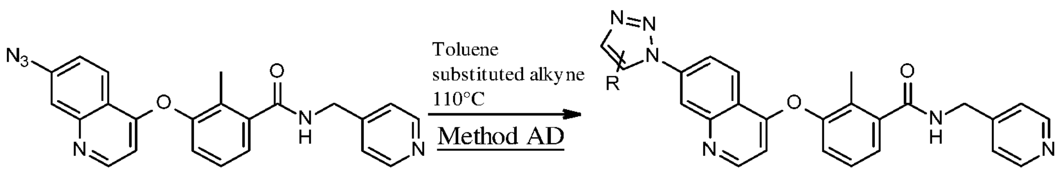


335 339-340

**Method AD:** To a stirred solution of 7-azido quinoline derivative (1 equiv.) in toluene (20 moL/mmol), was added the appropriate alkyne (20 equiv.) in an oven-dried screw-cap test tube under azote atmosphere. The reaction mixture was heated at 100°C for 24h, then concentrated under reduced pressure and purified by flash column chromatography on Biotage (DCM/MeOH: 0 to 10%) and reverse phase chromatography ($H_2O$/MeOH: 0 to 100%) to give the expected compound. In case of compound **340,** trimethylsilyl-protected alkyne was used, thus an additional step synthesis for deprotection of trimethylsilyl group with TBAF (1.1 equiv.) in THF was required.

The following compound **339** is an example illustrating Method AD:

Preparation of 3-[[7-(4,5-dimethyltriazol-1-yl)-4-quinolyl]oxy]-2-methyl-N-(4-pyridylmethyl)benzamide **(339)**

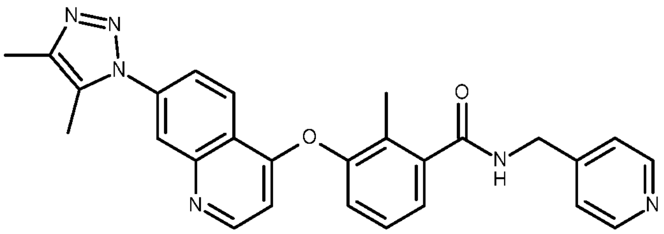

Compound **339** was synthesized from intermediate **335** (0.08 mmol) and 2-butyne (1.66 mmol) as a white powder in 18% yield (7 mg) according to the general method AD. $^1$H NMR (400 MHz, DMSO-$d_6$) δ (ppm): 9.08 (t, *J* = 6.17 Hz, 1H), 8.82 (d, *J* = 5.21 Hz, 1H), 8.61 (d, *J* = 8.83 Hz, 1H), 8.55-8.53 (m, 2H), 8.24 (d, *J* = 2.09 Hz, 1H), 7.93 (dd, *J* = 8.88 and 2.03 Hz, 1H), 7.49-7.36 (m, 5H), 6.56 (d, *J* = 5.28 Hz, 1H), 4.50 (d, *J* = 5.95 Hz, 2H), 2.40 (s, 3H), 2.32 (s, 3H), 2.19 (s, 3H) ; MS (ESI, EI+): m/z= 465.15 (MH+).

**Example 28: Procedure for the preparation of 7-triazole quinoline analogues 341-343**

The following procedures illustrate the preparation of analogues **341-343,** the synthetic scheme is outlined below. Examples of the final compounds can be found in table 1.

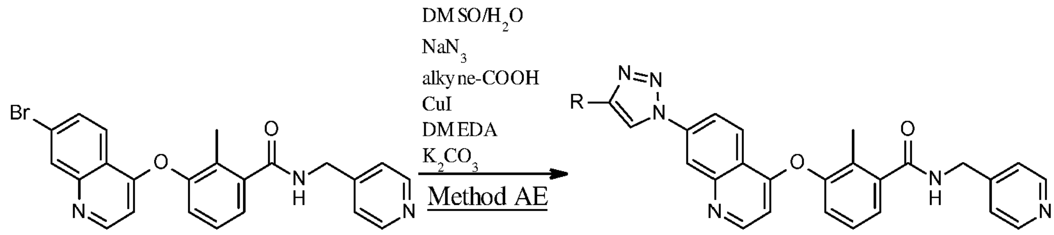


132 341-343

**Method AE:** 7-bromo quinoline derivative (1 equiv.), sodium azide (2.5 equiv.), the appropriate carboxylic alkyne acid (1.5 equiv.), cooper iodide (0.3 equiv.) and potassium carbonate (1.5 equiv.) were placed in an oven-dried screw-cap test tube. The tube was then evacuated and backfilled with nitrogen (this sequence was repeated three times). DMSO/$H_2O$ (7/3, 4 mL/mmol) and DMEDA (0.6 equiv.) were added. The reaction mixture was heated under microwave irradiation at 100°C for 45min. Water was then added and the resulting precipitate was filtered and dried over $P_2O_5$ in vacuum desiccators. The solid was purified by flash column chromatography on Biotage (DCM/MeOH: 0 to 10%) and reverse phase chromatography ($H_2O$/MeOH: 0 to 100%) to give the expected compound.

The following compound **341** is an example illustrating Method AE:

Preparation of 3-[[7-(4-ethyltriazol-1-yl)-4-quinolyl]oxy]-2-methyl-N-(4-pyridylmethyl)benzamide **(341)**

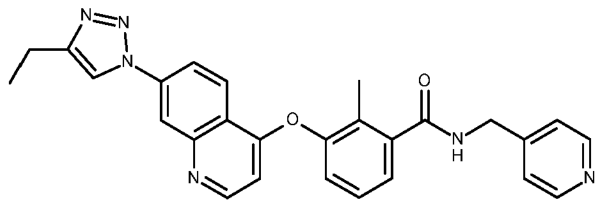

Compound **341** was synthesized from intermediate **132** (0.10 mmol) and 2-pentynoic acid (0.15 mmol) as a white powder in 61% yield (28 mg) according to the general method AE. $^1$H NMR (400 MHz, DMSO-$d_6$) δ (ppm): 9.07 (t, *J* = 6.13 Hz, 1H), 8.91 (bs, 1H), 8.80 (d, *J* = 5.14 Hz, 1H), 8.60 (d, *J* = 9.10 Hz, 1H), 8.55-8.53 (m, 3H), 8.32 (dd, *J* = 9.08 and 2.13 Hz, 1H), 7.48 (d, *J* = 4.47 Hz, 2H), 7.41 (q, *J* = 4.90 Hz, 1H), 7.37-7.36 (m, 2H), 6.51 (d, *J* = 5.14 Hz, 1H), 4.51 (d, *J* = 6.04 Hz, 2H), 2.80 (q, *J* = 7.66 Hz, 2H), 2.18 (s, 3H), 1.33 (t, *J* = 7.70 Hz, 3H) ; MS (ESI, EI+): m/z= 465.15 (MH+).

**Table 1: Examples of compounds according to the invention**

| | |
|---|---|
| **3** | 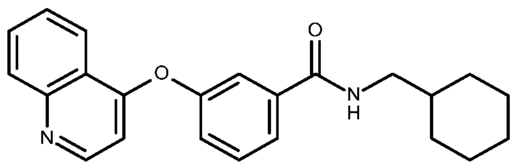 |
| **4** | 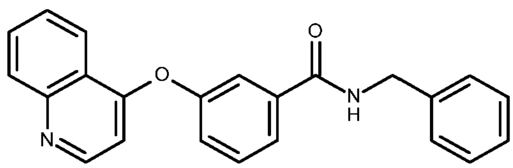 |
| **5** | 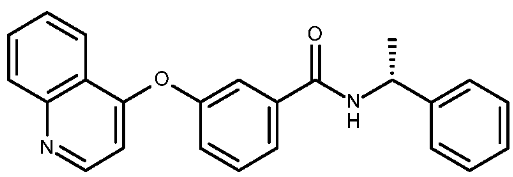 |

(continued)

| | |
|---|---|
| **6** | 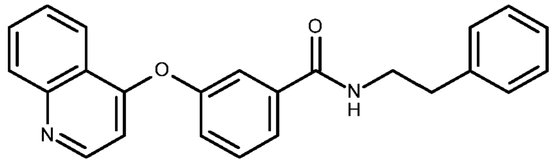 |
| **7** | 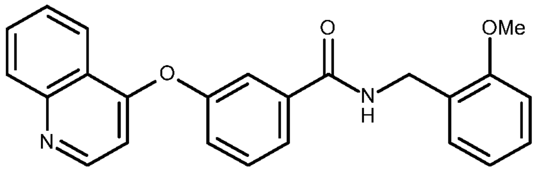 |
| **8** | 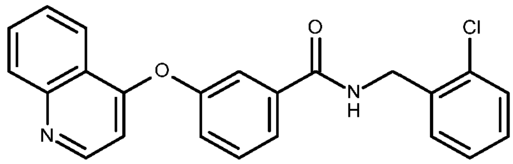 |
| **9** | 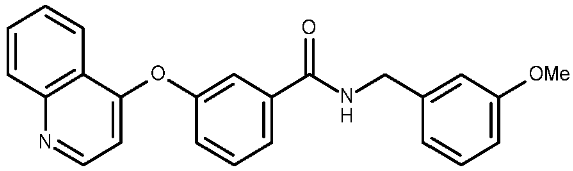 |
| **10** | 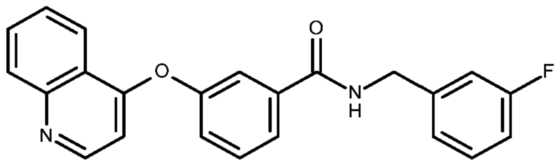 |
| **11** | 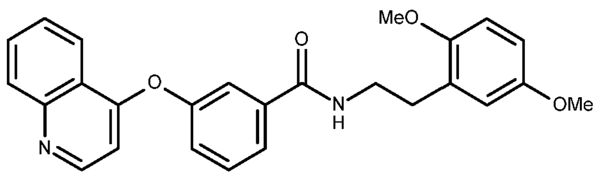 |
| **12** | 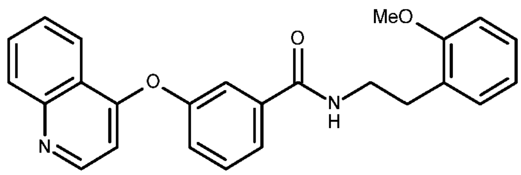 |
| **13** | 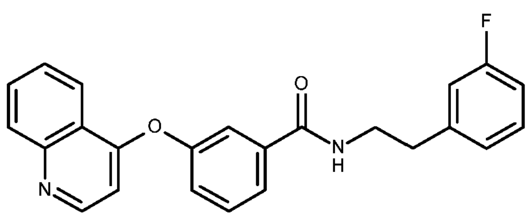 |
| **14** | 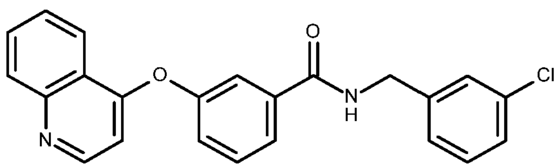 |

(continued)

| | |
|---|---|
| **15** | 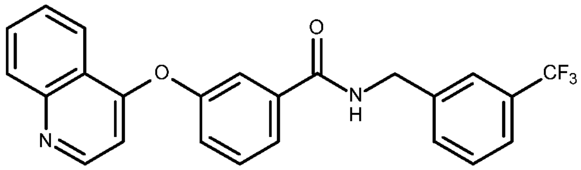 |
| **16** | 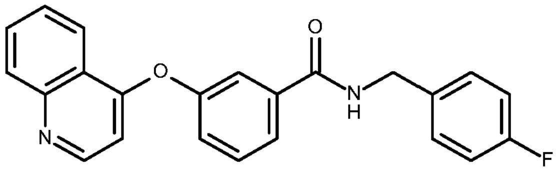 |
| **17** | 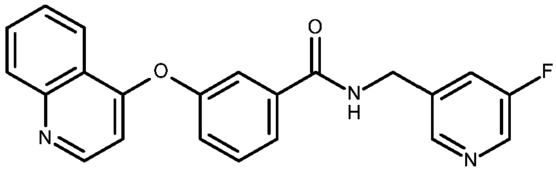 |
| **18** | 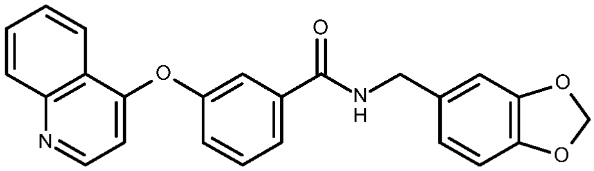 |
| **19** | 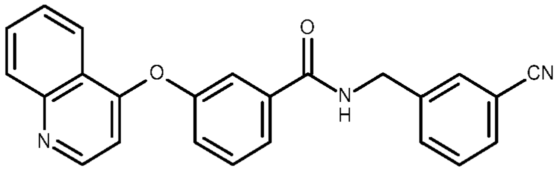 |
| **20** | 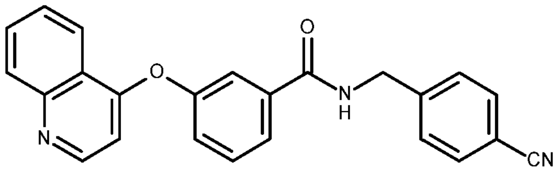 |
| **21** | 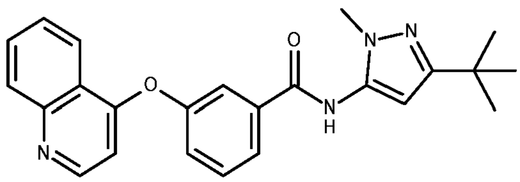 |
| **22** | 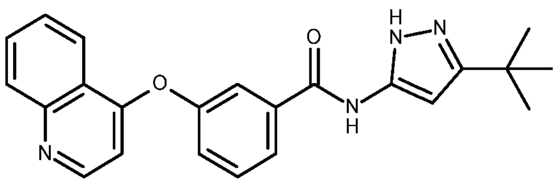 |
| **23** | 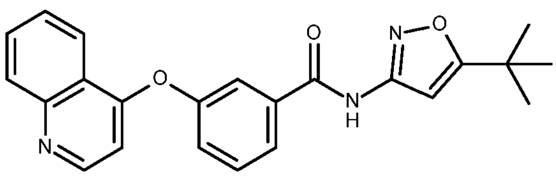 |

(continued)

| | |
|---|---|
| **24** | 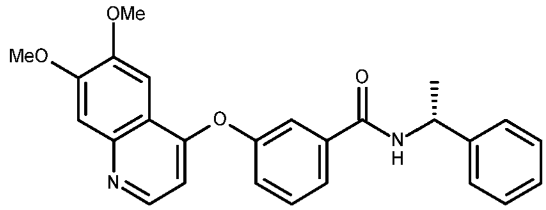 |
| **25** | 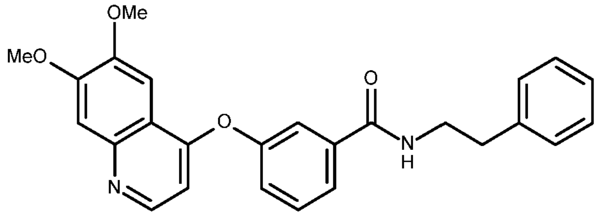 |
| **26** | 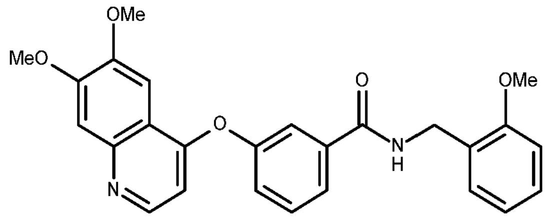 |
| **27** | 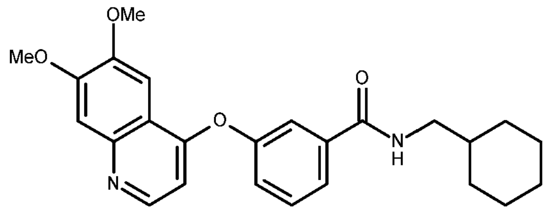 |
| **28** | 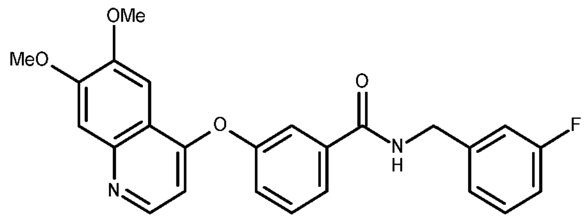 |
| **29** | 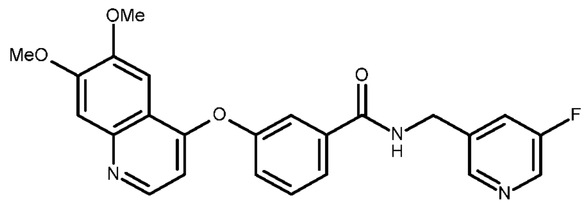 |
| **30** | 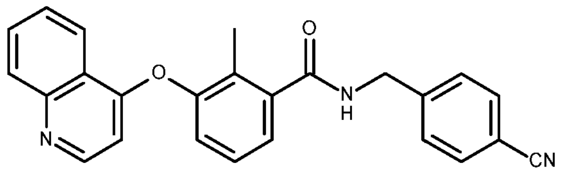 |
| **31** | 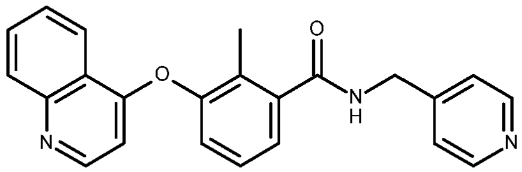 |

(continued)

| | |
|---|---|
| **32** | 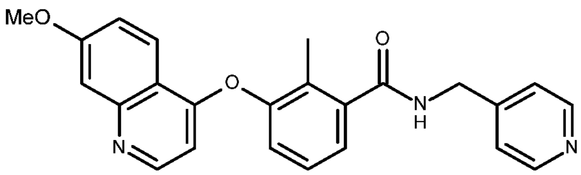 |
| **33** | 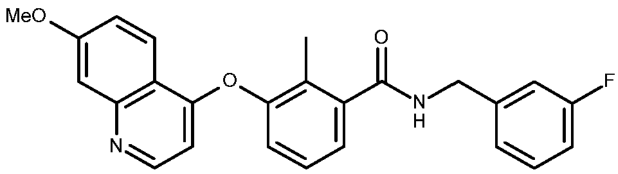 |
| **34** | 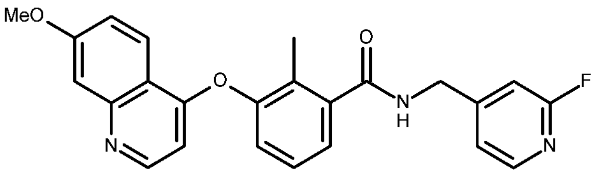 |
| **35** | 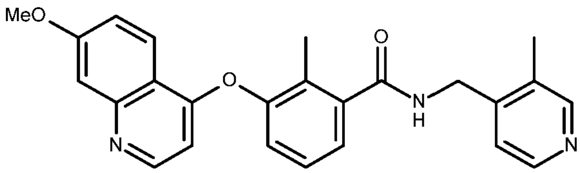 |
| **36** | 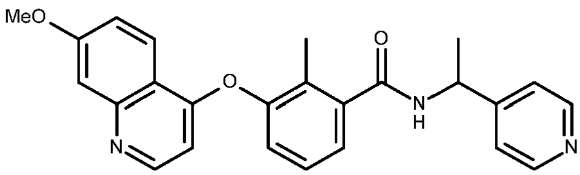 |
| **37** | 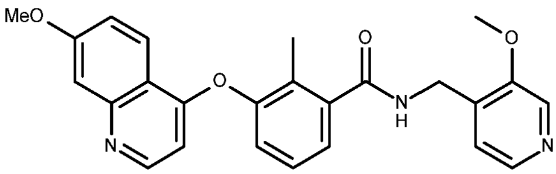 |
| **38** | 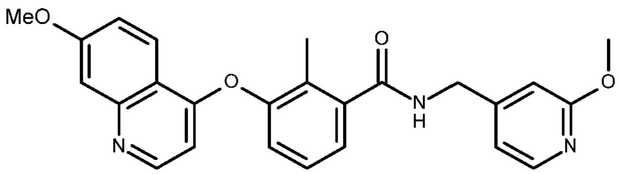 |
| **39** | 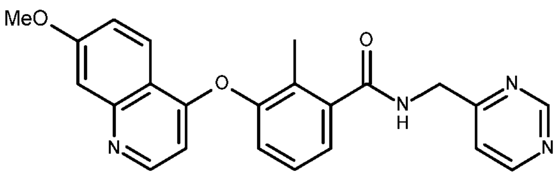 |
| **40** | 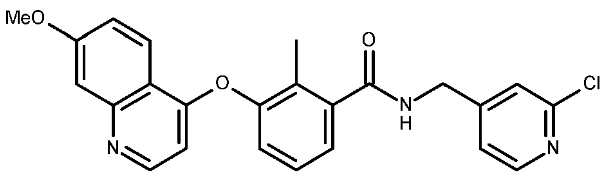 |

(continued)

| | |
|---|---|
| 41 | 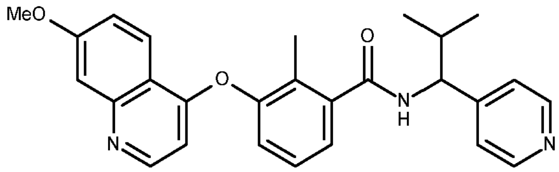 |
| 42 | 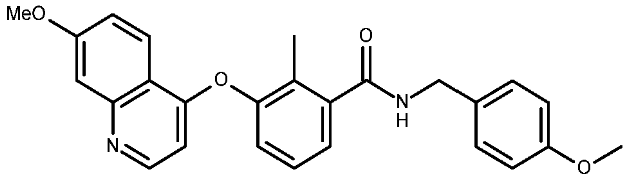 |
| 43 | 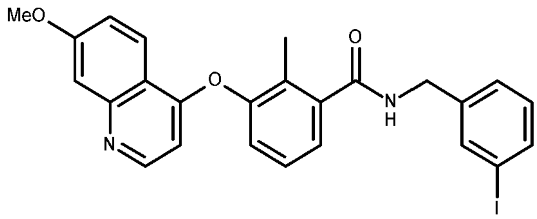 |
| 44 | 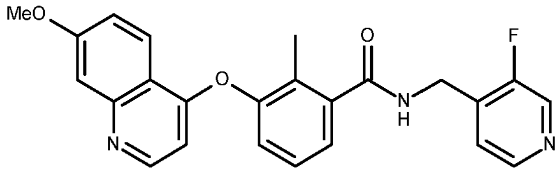 |
| 45 | 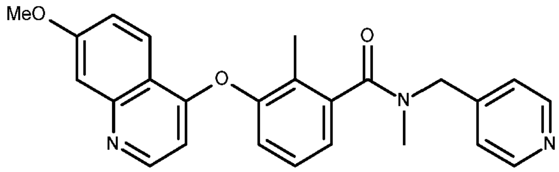 |
| 46 | 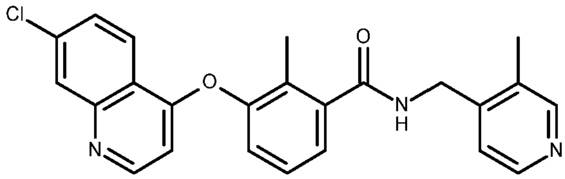 |
| 47 | 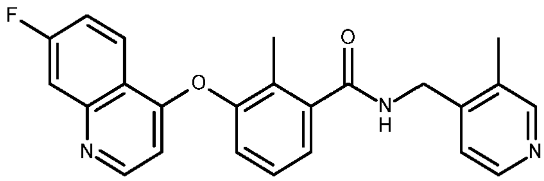 |
| 48 | 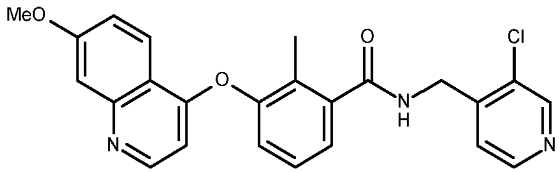 |

(continued)

| | |
|---|---|
| **49** | 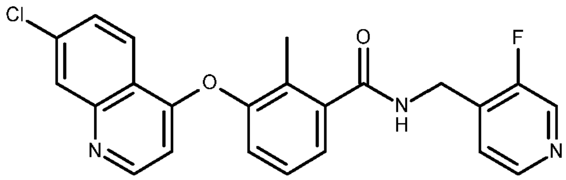 |
| **50** | 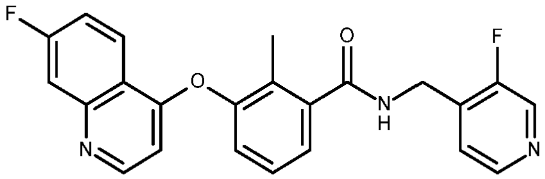 |
| **51** | 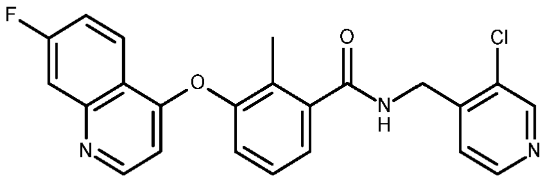 |
| **52** | 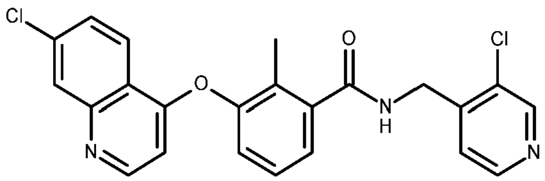 |
| **53** | 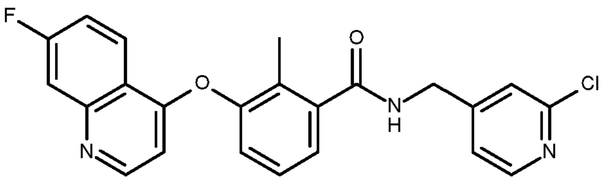 |
| **54** | 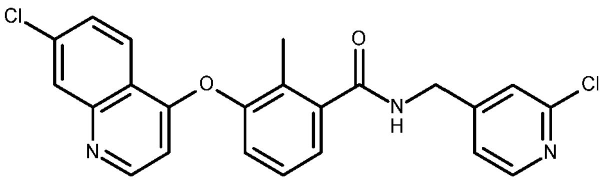 |
| **55** | 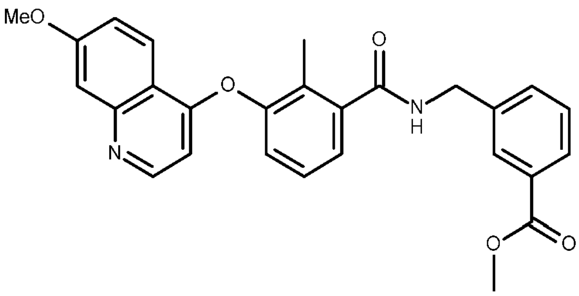 |
| **56** | 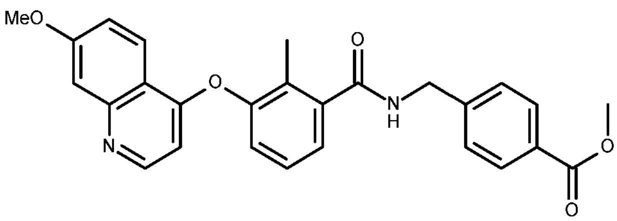 |

(continued)

| | |
|---|---|
| 57 | 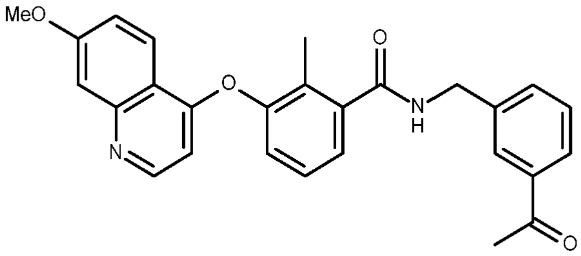 |
| 58 | 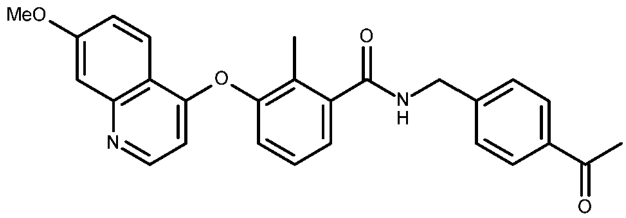 |
| 59 | 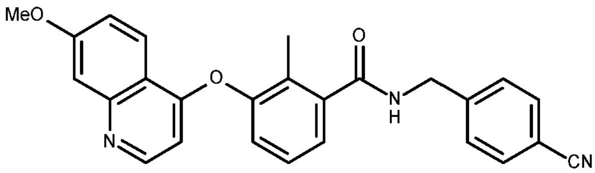 |
| 60 | 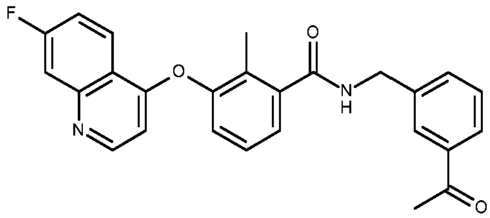 |
| 61 | 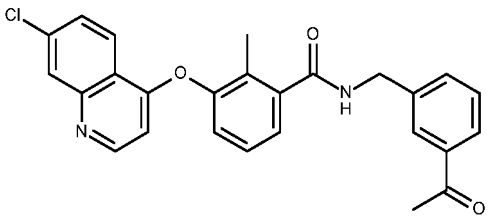 |
| 62 | 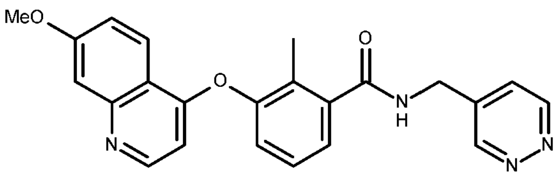 |
| 63 | 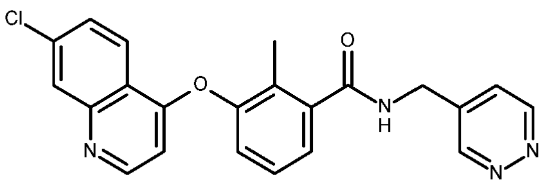 |
| 64 | 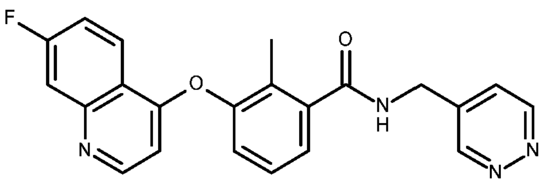 |

(continued)

| | |
|---|---|
| **65** | 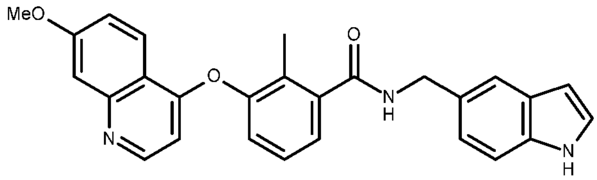 |
| **66** | 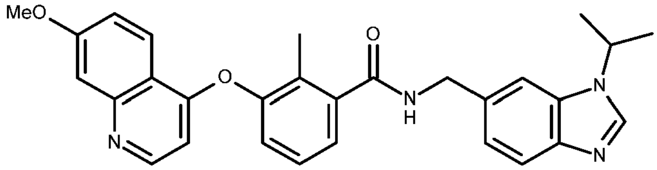 |
| **67** | 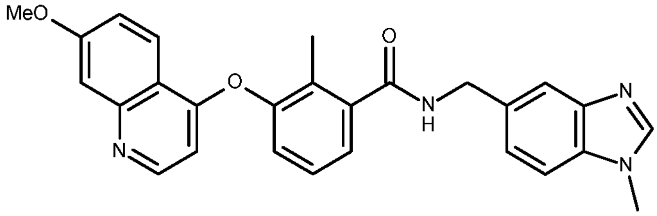 |
| **68** | 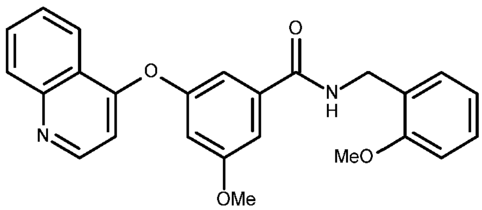 |
| **69** | 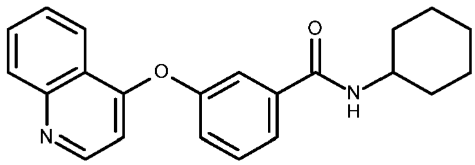 |
| **70** | 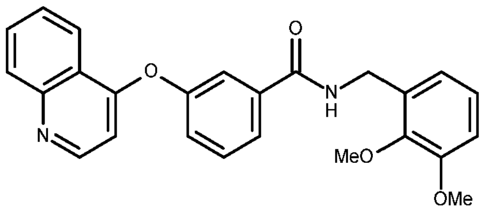 |
| **71** | 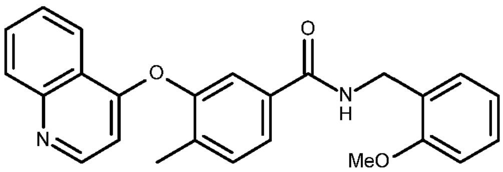 |
| **72** | 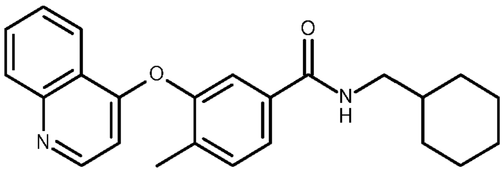 |

(continued)

| | |
|---|---|
| **73** | 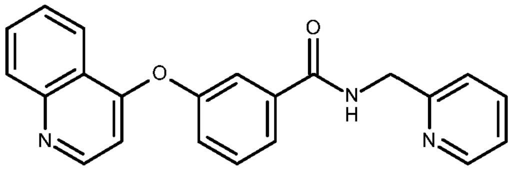 |
| **74** | 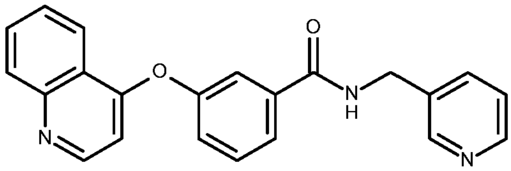 |
| **75** | 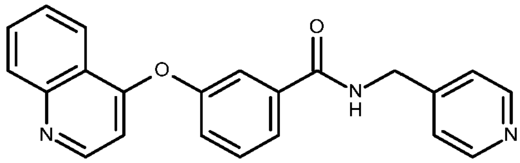 |
| **76** | 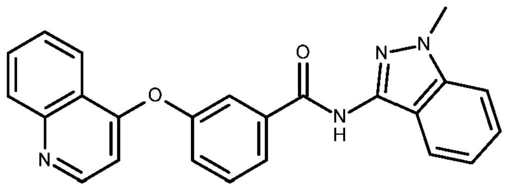 |
| **84** | 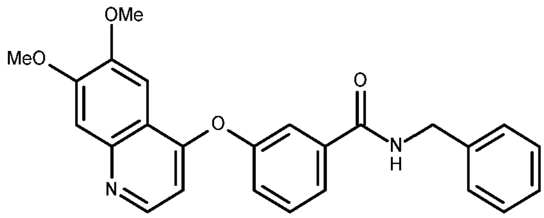 |
| **85** | 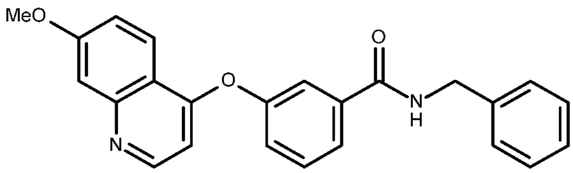 |
| **86** | 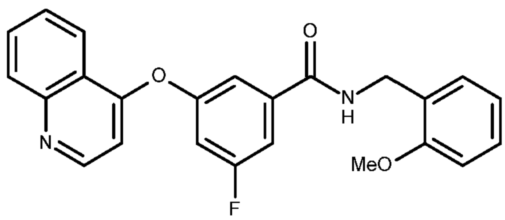 |
| **87** | 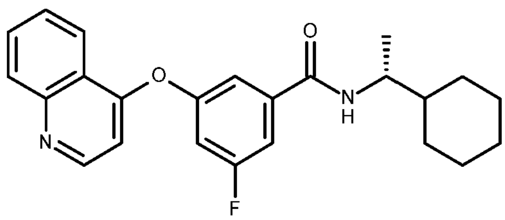 |

(continued)

| | |
|---|---|
| 88 | 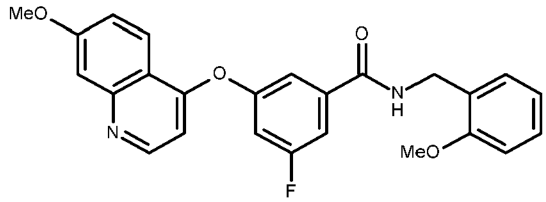 |
| 89 | 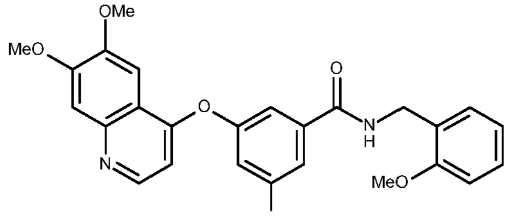 |
| 90 | 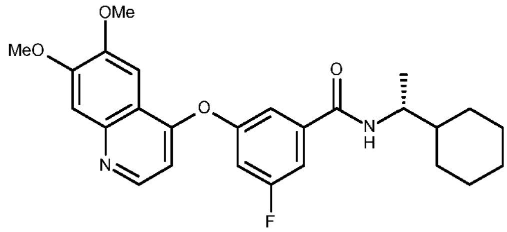 |
| 91 | 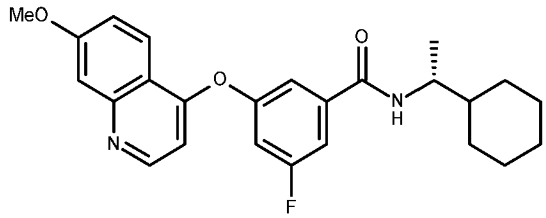 |
| 92 | 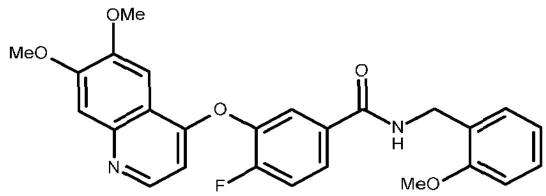 |
| 93 | 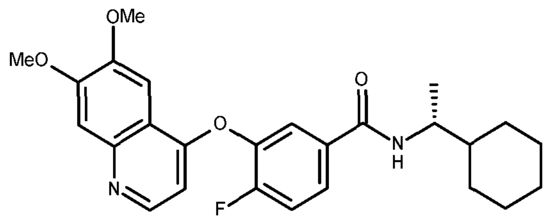 |
| 94 | 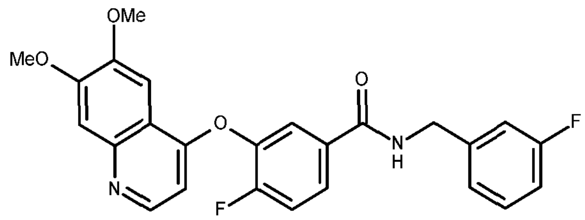 |

(continued)

| | |
|---|---|
| **95** | 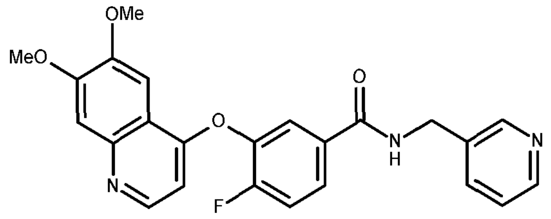 |
| **96** | 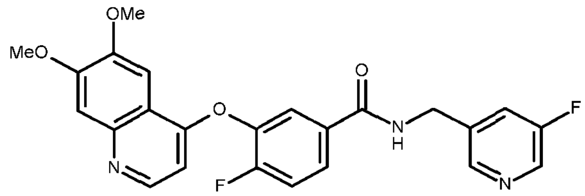 |
| **97** | 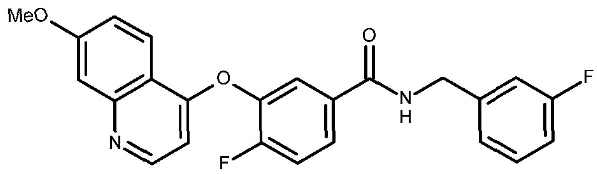 |
| **98** | 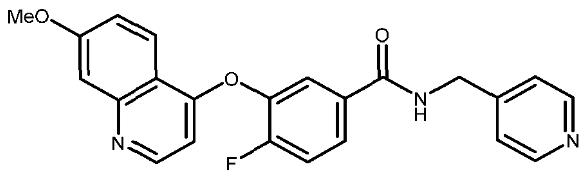 |
| **99** | 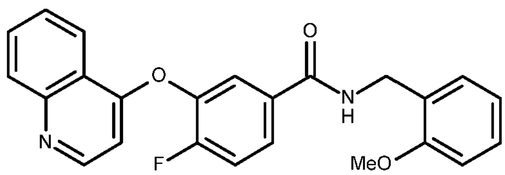 |
| **100** | 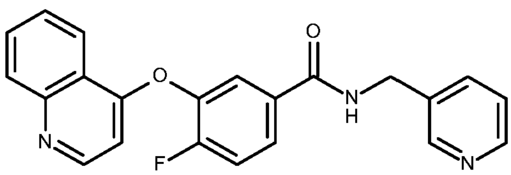 |
| **101** | 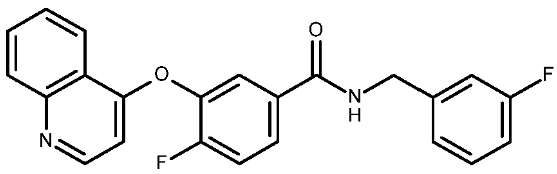 |
| **102** | 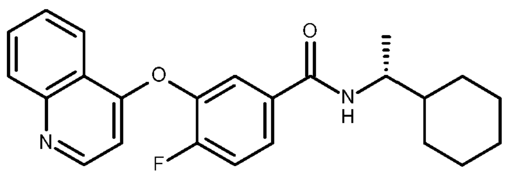 |

(continued)

| | |
|---|---|
| **103** | 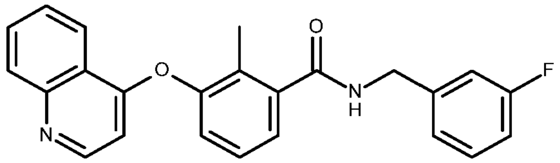 |
| **104** | 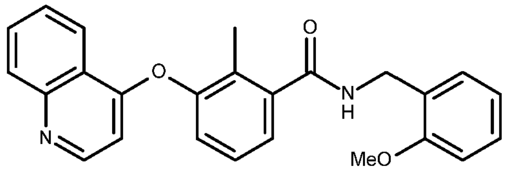 |
| **105** | 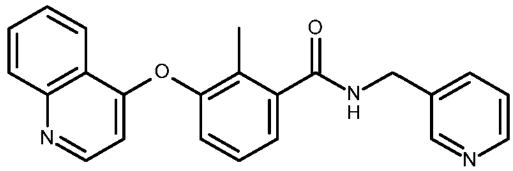 |
| **106** | 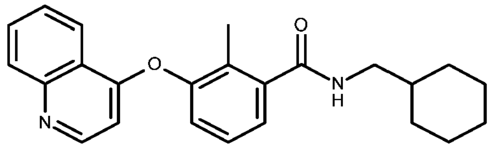 |
| **107** | 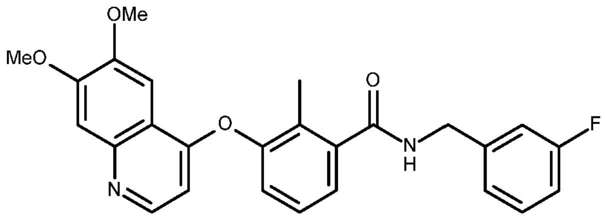 |
| **108** | 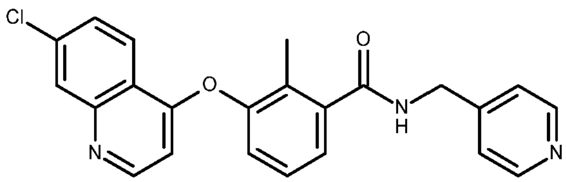 |
| **109** | 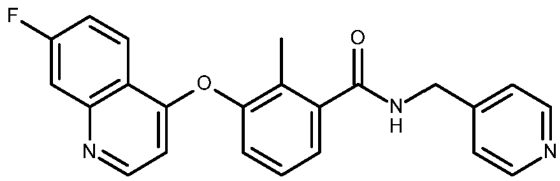 |
| **110** | 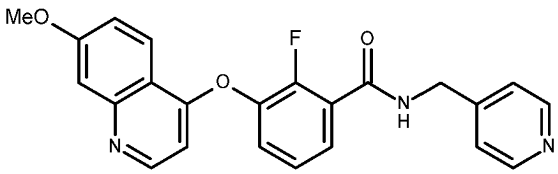 |
| **113** | 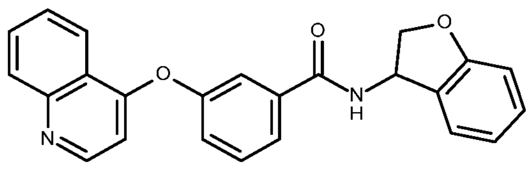 |

(continued)

| | |
|---|---|
| 114 | 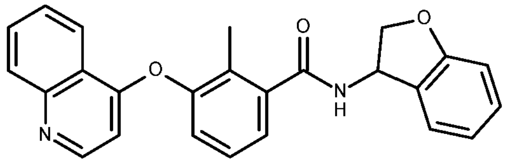 |
| 117 | 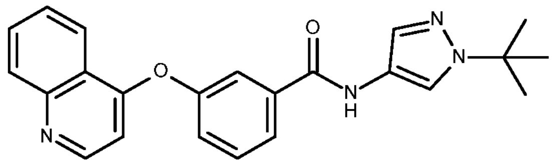 |
| 122 | 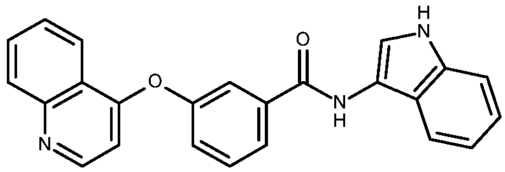 |
| 123 | 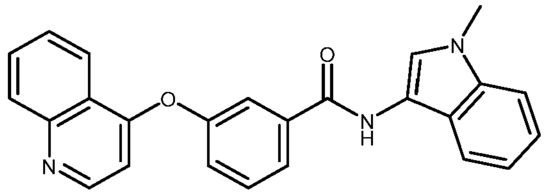 |
| 124 | 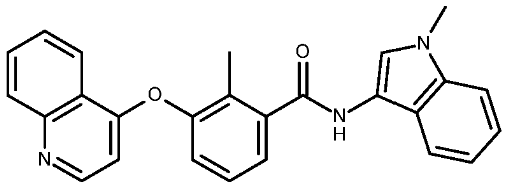 |
| 127 | 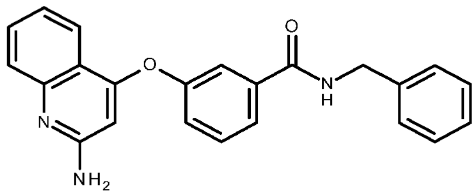 |
| 128 | 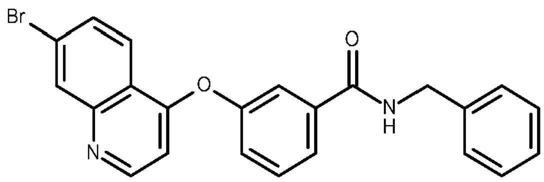 |
| 129 | 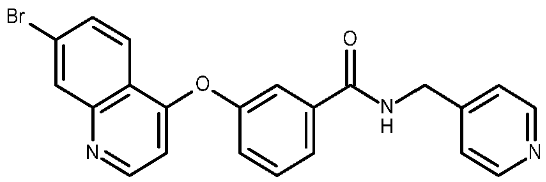 |

(continued)

| | |
|---|---|
| **130** | 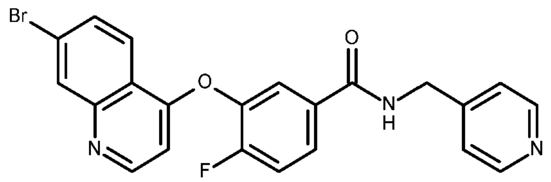 |
| **131** | 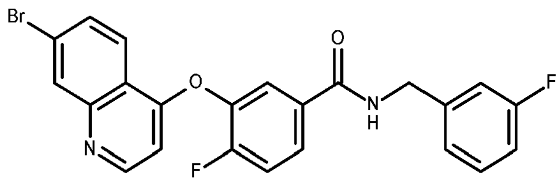 |
| **132** | 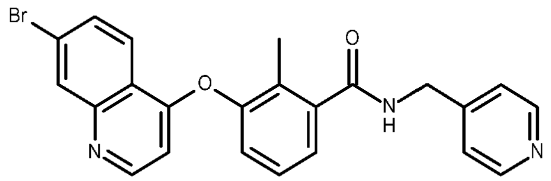 |
| **133** | 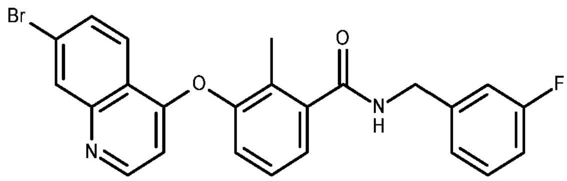 |
| **134** | 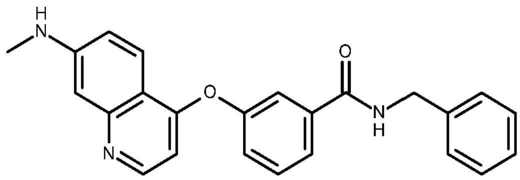 |
| **135** | 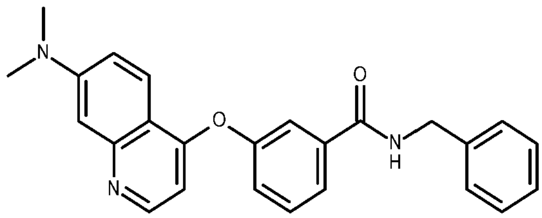 |
| **136** | 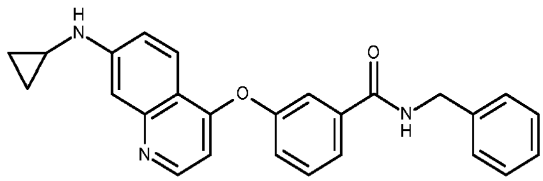 |
| **137** | 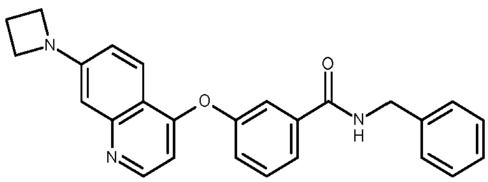 |

(continued)

| | |
|---|---|
| **138** | 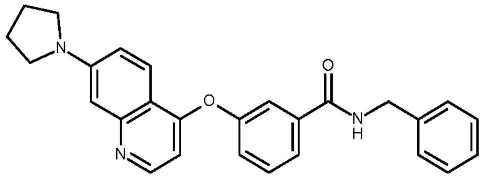 |
| **139** | 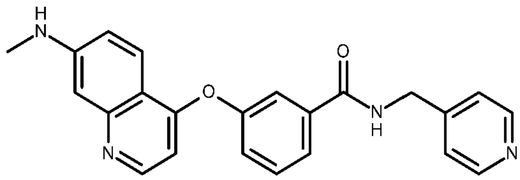 |
| **140** | 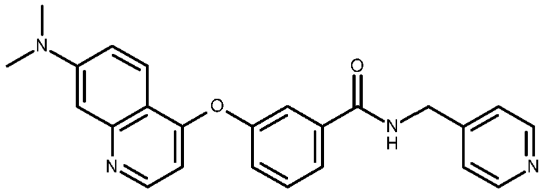 |
| **141** | 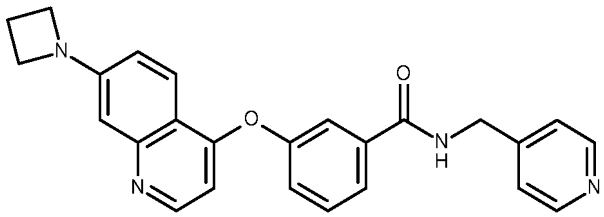 |
| **142** | 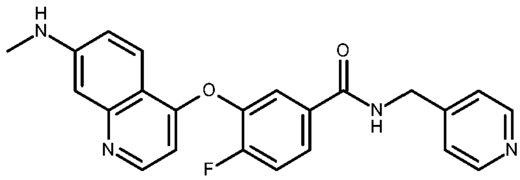 |
| **143** | 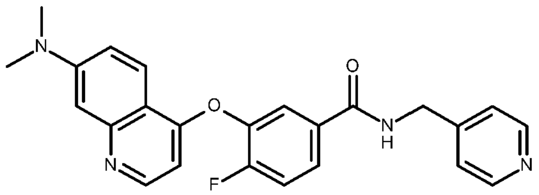 |
| **144** | 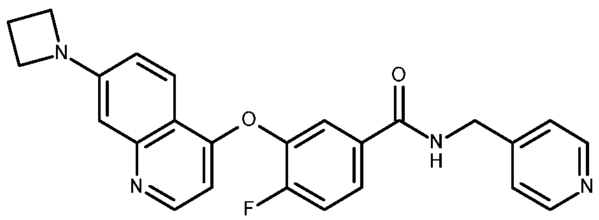 |
| **145** | 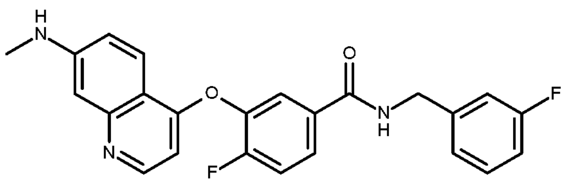 |

(continued)

| | |
|---|---|
| **146** | 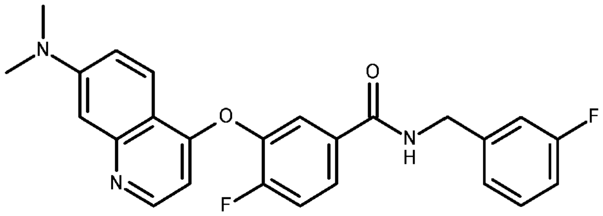 |
| **147** | 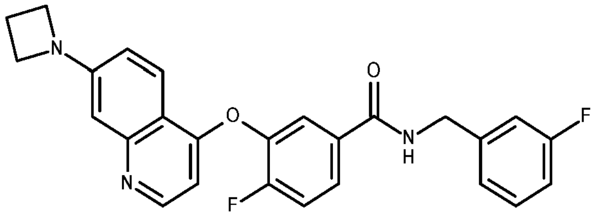 |
| **148** | 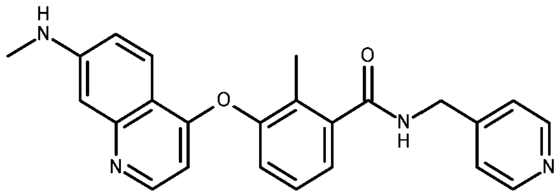 |
| **149** | 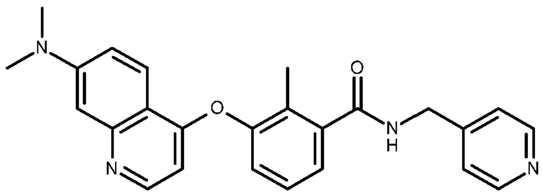 |
| **150** | 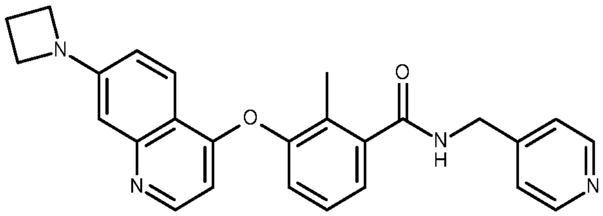 |
| **151** | 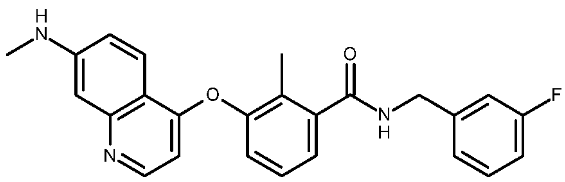 |
| **152** | 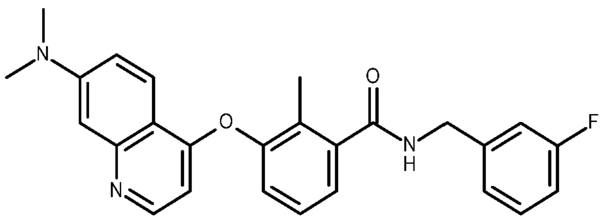 |

(continued)

| | |
|---|---|
| **153** | 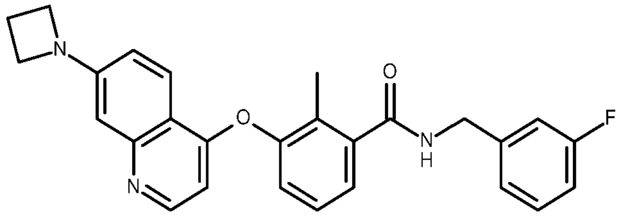 |
| **154** | 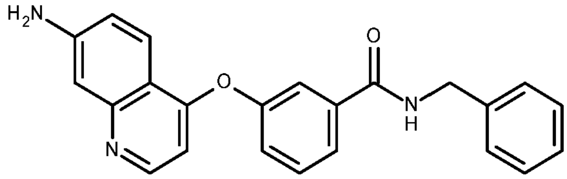 |
| **155** | 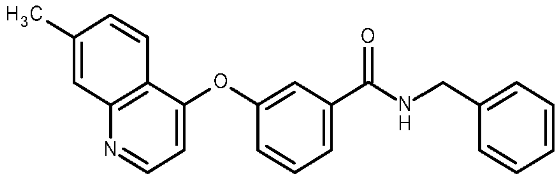 |
| **160** | 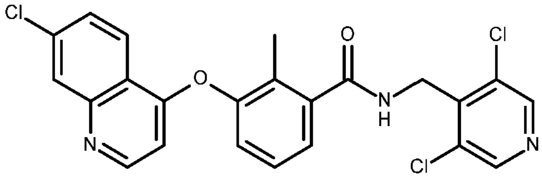 |
| **161** | 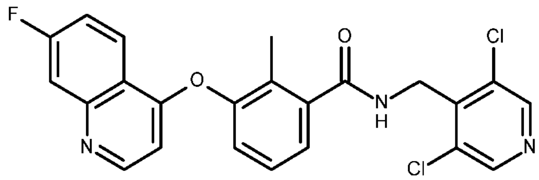 |
| **162** | 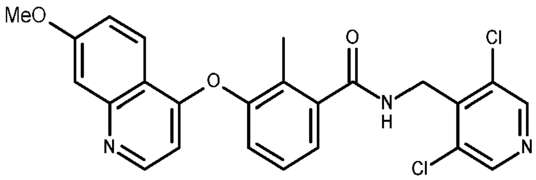 |
| **163** | 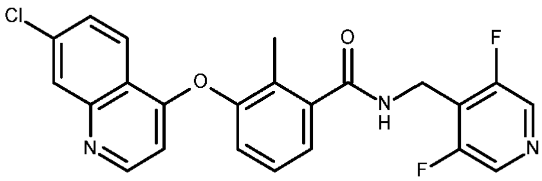 |
| **164** | 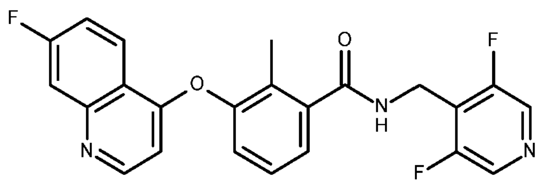 |

(continued)

| | |
|---|---|
| **165** | 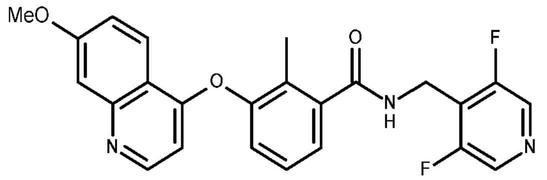 |
| **166** | 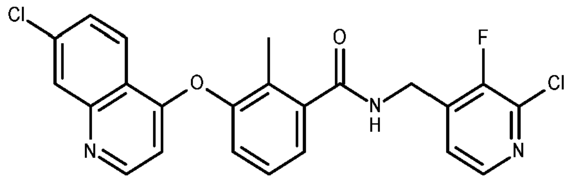 |
| **167** | 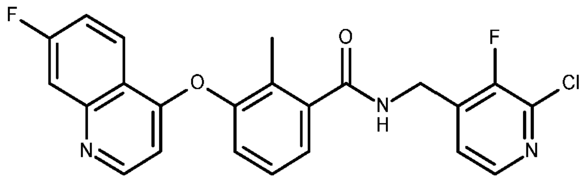 |
| **168** | 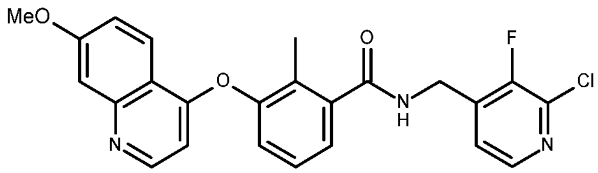 |
| **169** | 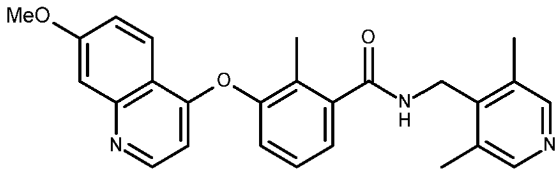 |
| **170** | 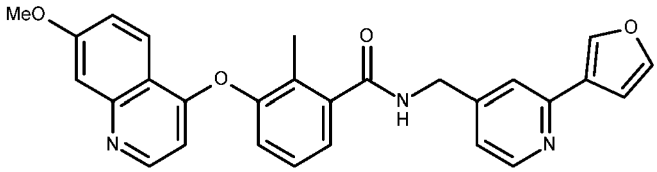 |
| **171** | 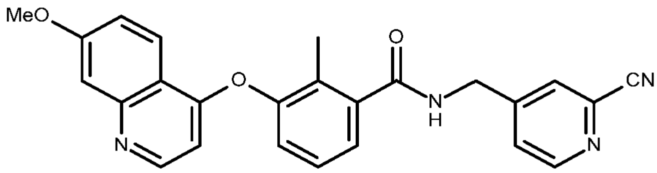 |
| **172** | 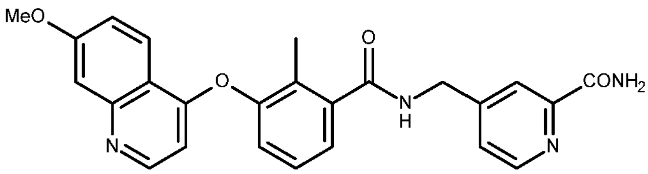 |
| **173** | 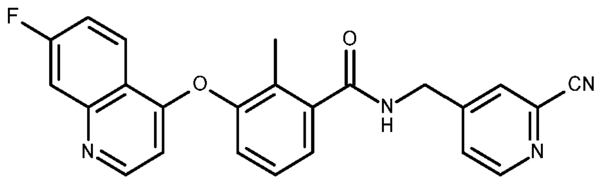 |

(continued)

| | |
|---|---|
| **174** | 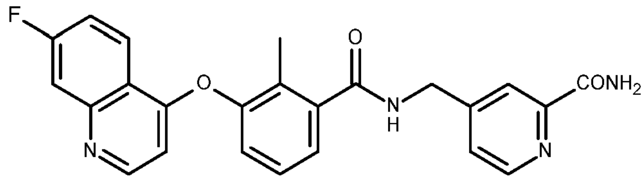 |
| **175** | 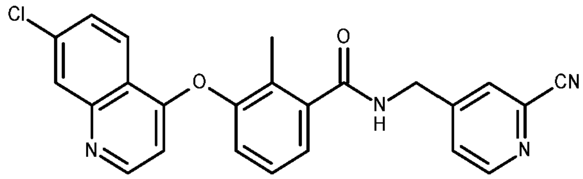 |
| **176** | 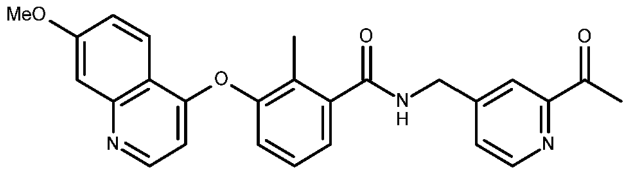 |
| **177** | 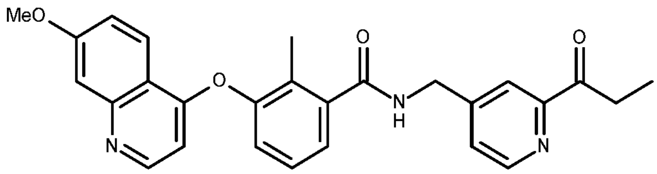 |
| **178** | 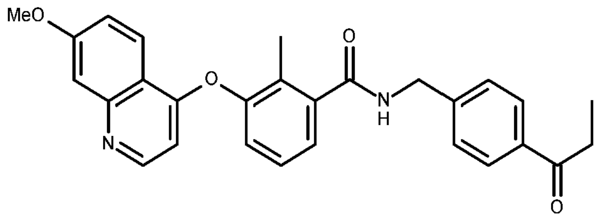 |
| **179** | 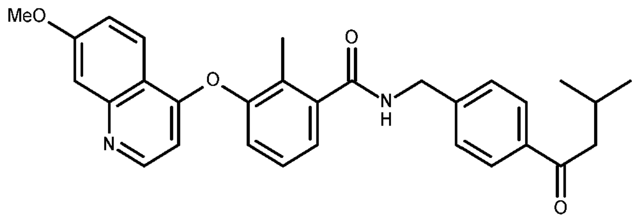 |
| **180** | 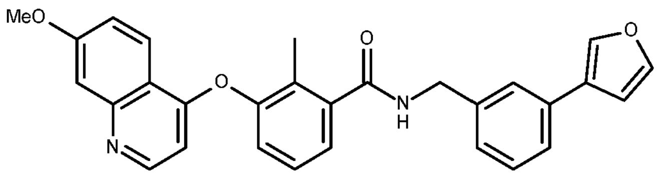 |
| **181** | 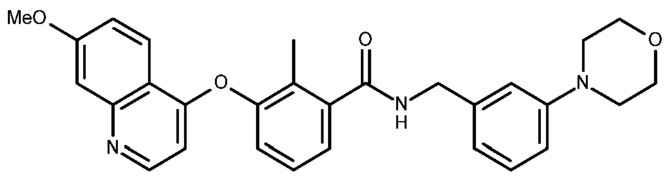 |

(continued)

| | |
|---|---|
| **182** | 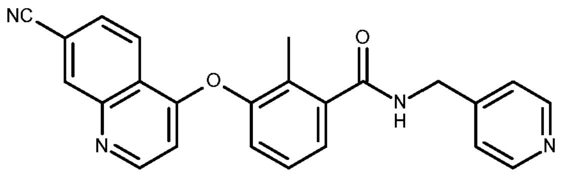 |
| **186** | 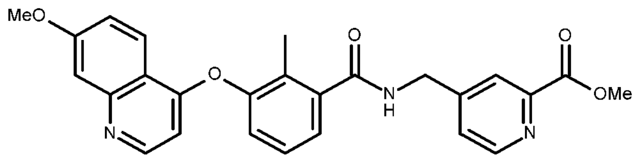 |
| **191** | 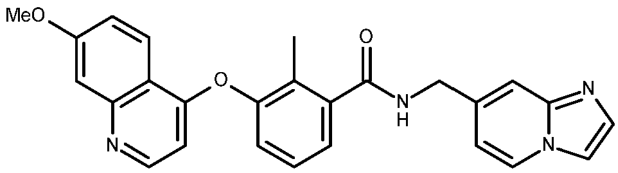 |
| **192** | 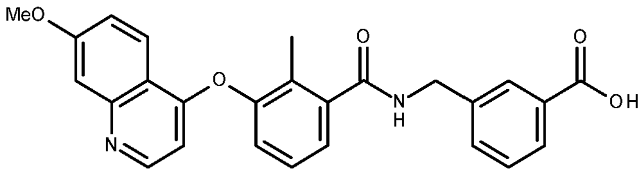 |
| **193** | 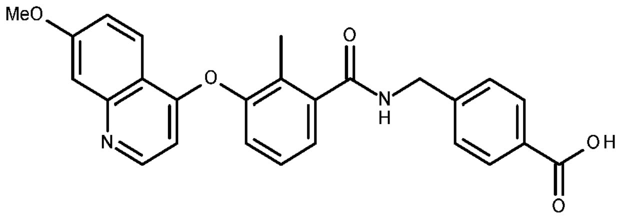 |
| **194** | 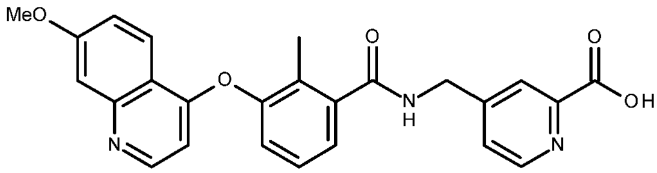 |
| **195** | 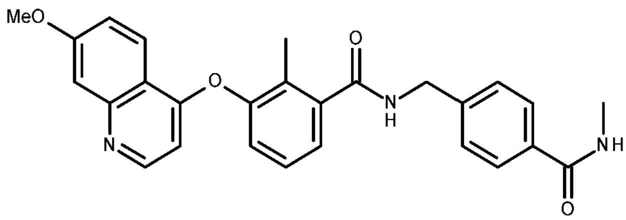 |
| **196** | 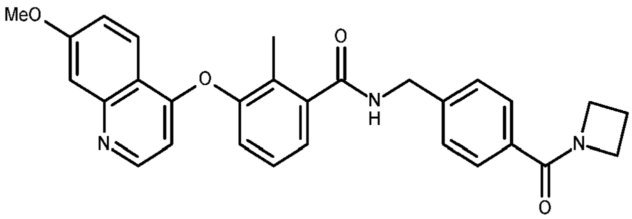 |

(continued)

| | |
|---|---|
| **197** | 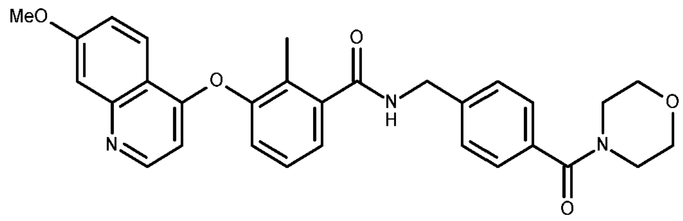 |
| **198** | 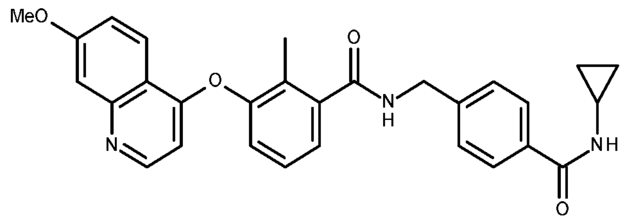 |
| **199** | 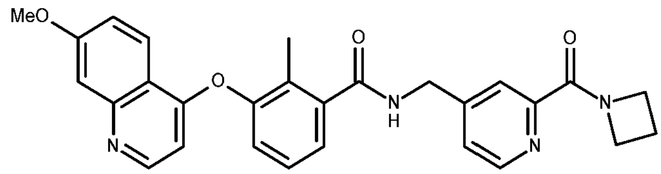 |
| **200** | 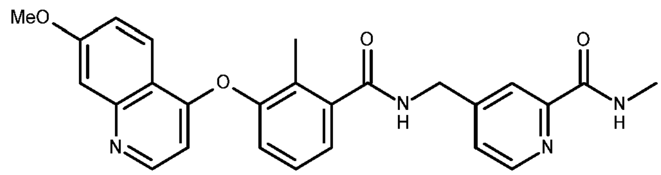 |
| **201** | 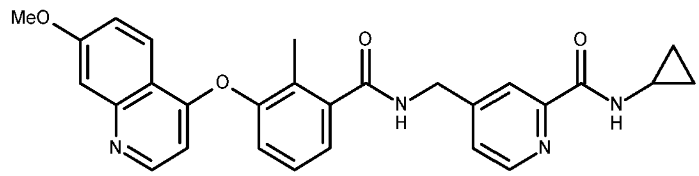 |
| **202** | 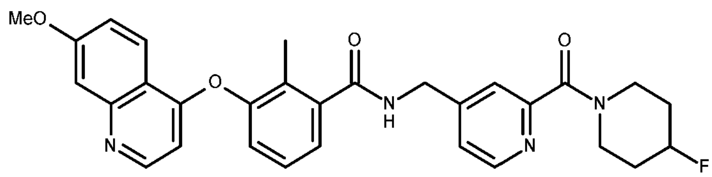 |
| **203** | 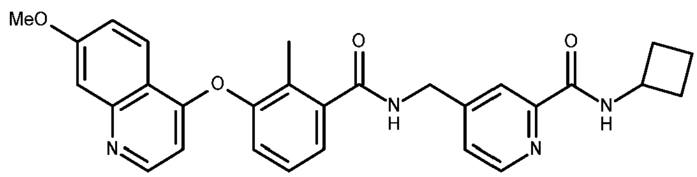 |
| **204** | 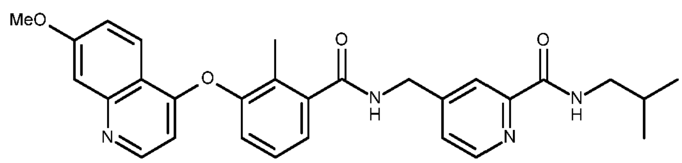 |

(continued)

| | |
|---|---|
| 205 | 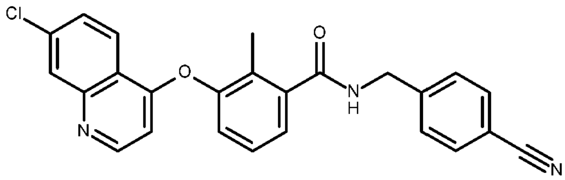 |
| 206 | 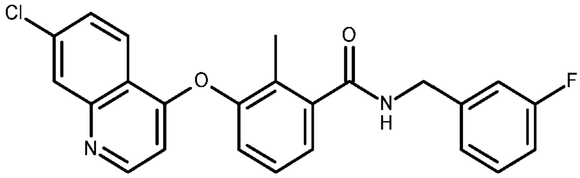 |
| 207 | 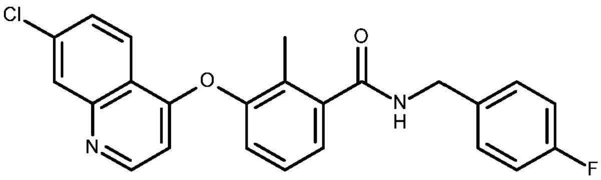 |
| 208 | 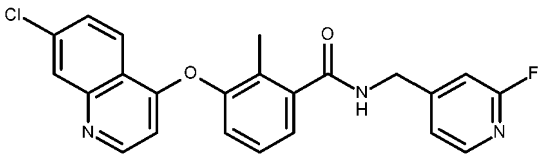 |
| 209 | 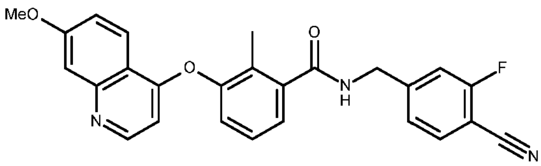 |
| 210 | 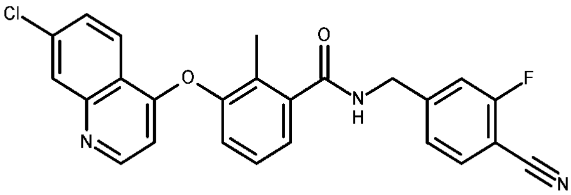 |
| 211 | 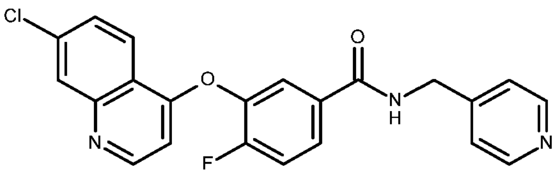 |
| 224 | 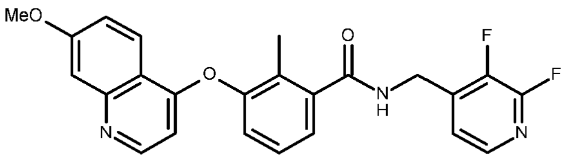 |
| 225 | 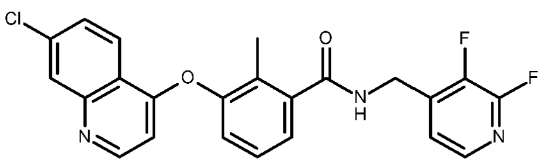 |

(continued)

| | |
|---|---|
| 226 | 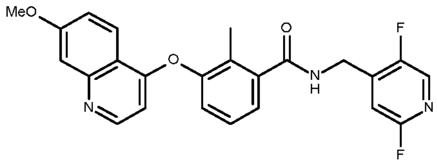 |
| 227 | 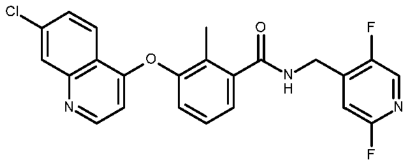 |
| 228 | 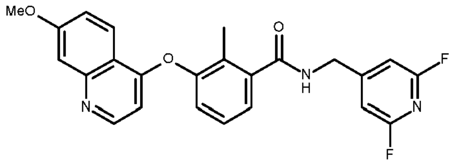 |
| 229 | 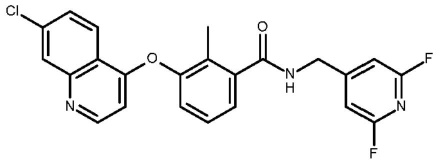 |
| 230 | 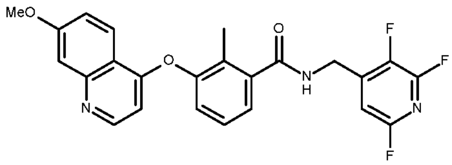 |
| 231 | 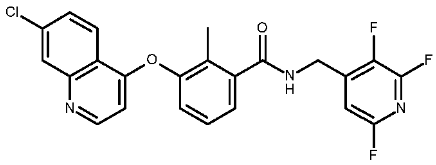 |
| 232 | 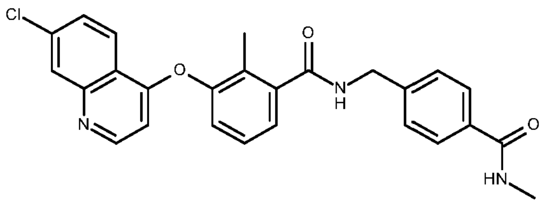 |
| 233 | 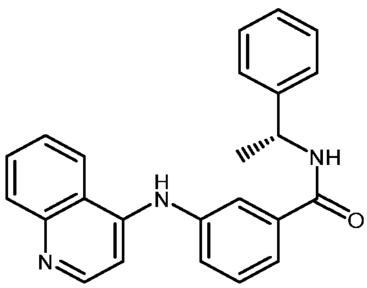 |

(continued)

| | |
|---|---|
| 235 | 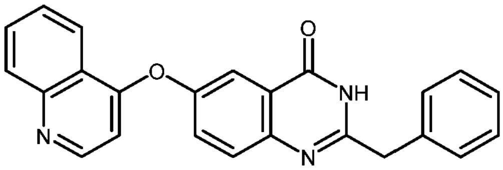 |
| 236 | 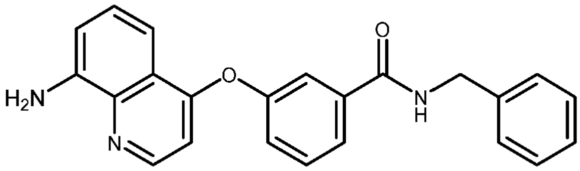 |
| 237 | 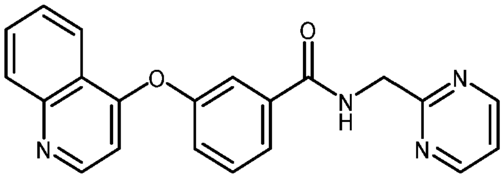 |
| 238 | 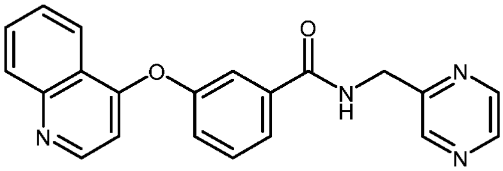 |
| 239 | 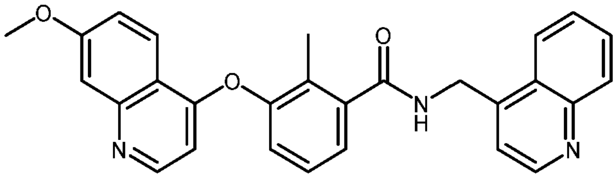 |
| 240 | 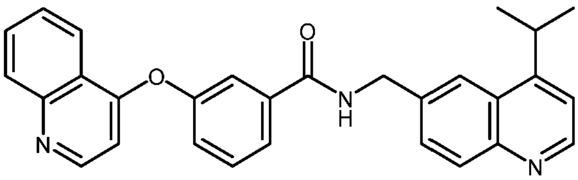 |
| 241 | 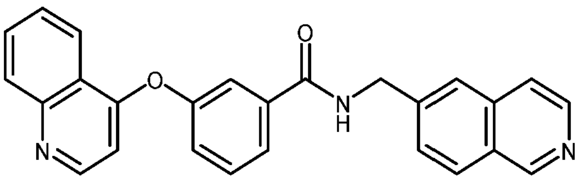 |
| 242 | 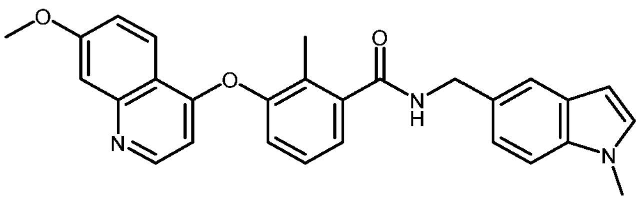 |

(continued)

| | |
|---|---|
| 243 | 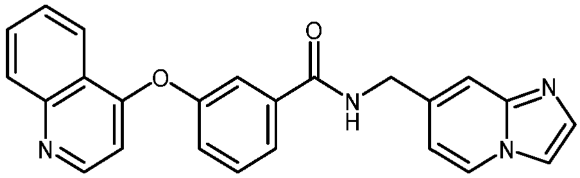 |
| 244 | 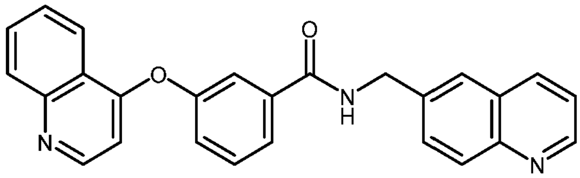 |
| 245 | 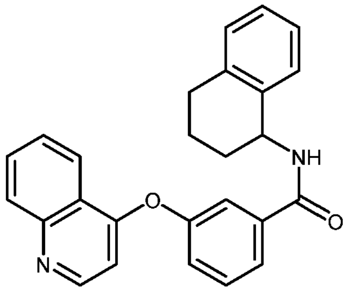 |
| 246 | 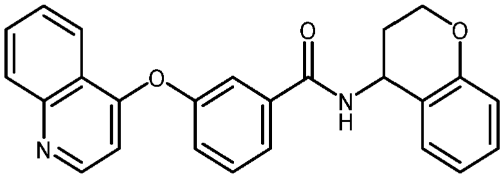 |
| 247 | 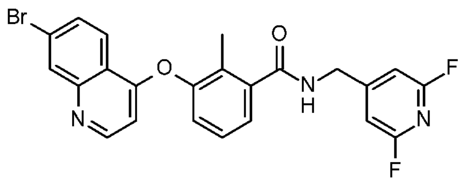 |
| 248 | 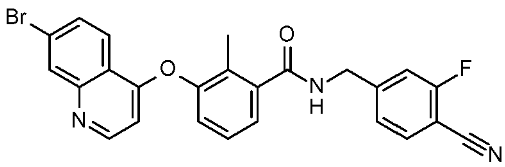 |
| 249 | 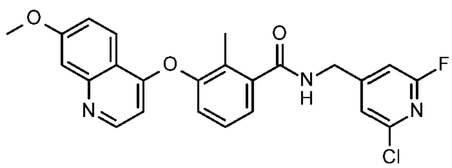 |
| 250 | 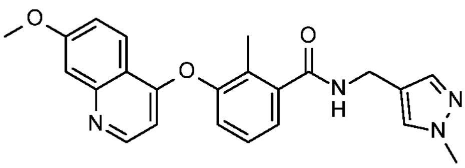 |

(continued)

| | |
|---|---|
| 251 | 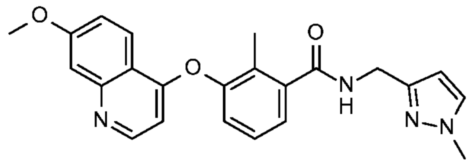 |
| 252 | 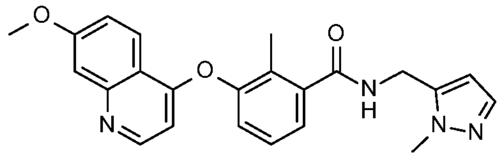 |
| 253 | 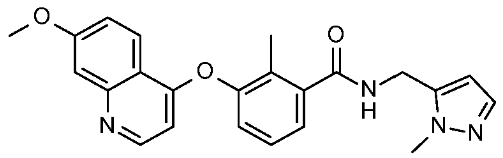 |
| 254 | 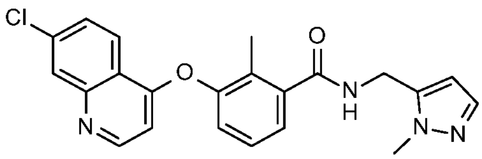 |
| 255 | 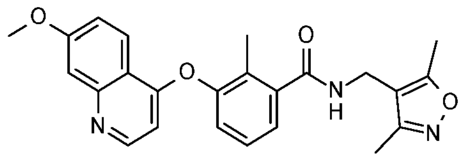 |
| 256 | 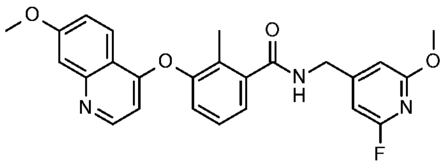 |
| 257 | 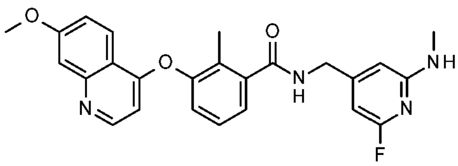 |
| 258 | 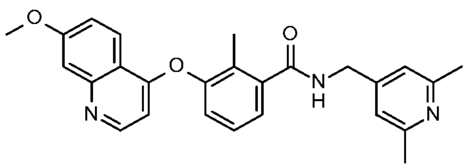 |
| 259 | 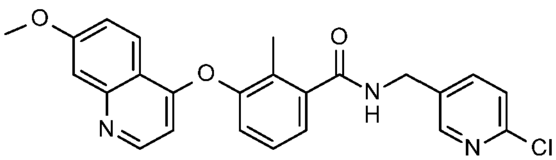 |

(continued)

| | |
|---|---|
| 260 | 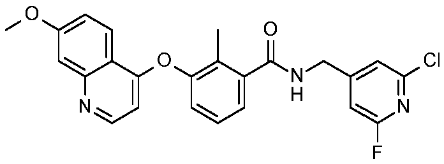 |
| 261 | 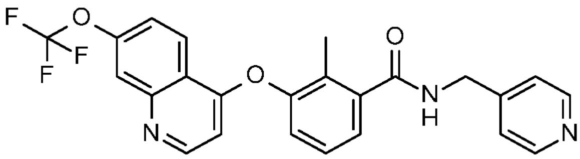 |
| 262 | 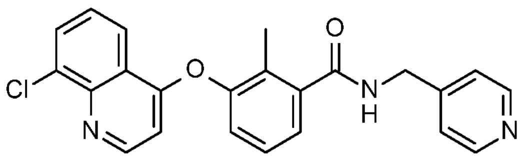 |
| 263 | 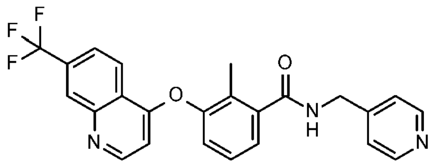 |
| 264 | 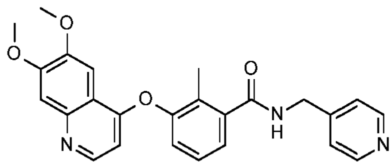 |
| 265 | 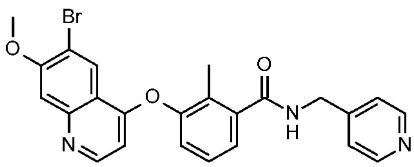 |
| 266 | 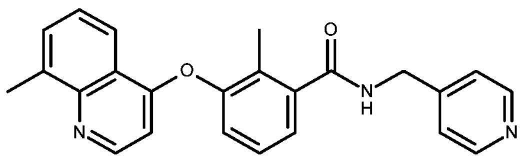 |
| 267 | 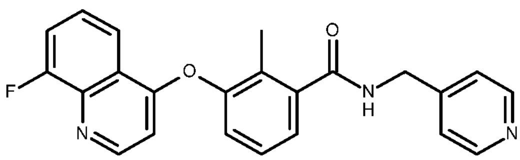 |
| 268 | 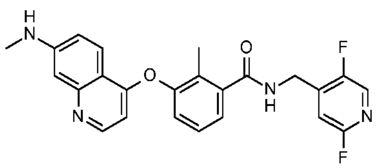 |

(continued)

| | |
|---|---|
| 269 | 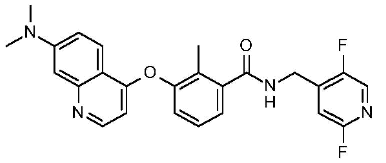 |
| 270 | 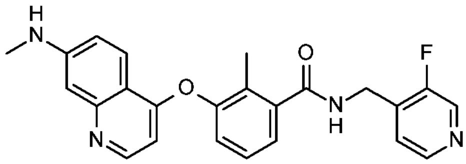 |
| 271 | 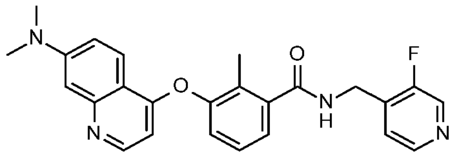 |
| 272 | 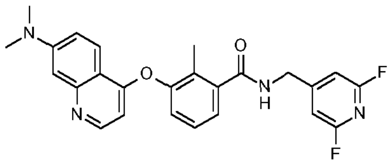 |
| 273 | 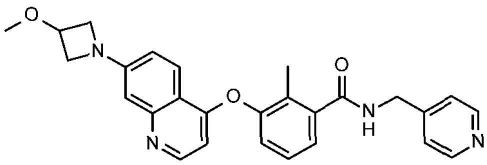 |
| 274 | 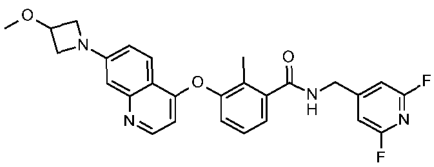 |
| 275 | 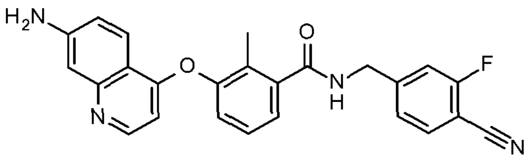 |
| 279 | 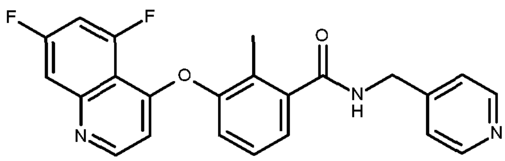 |
| 280 | 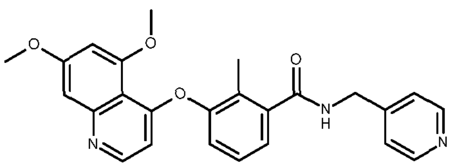 |

(continued)

| | |
|---|---|
| 281 | 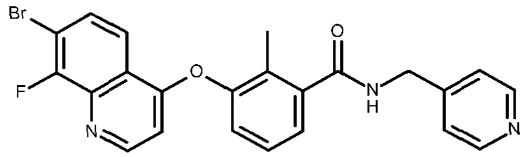 |
| 282 | 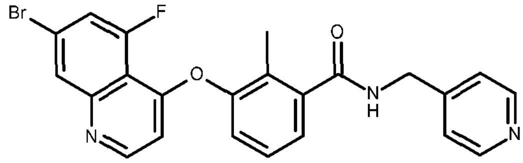 |
| 283 | 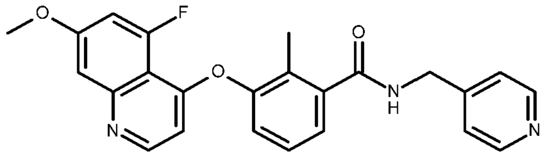 |
| 284 | 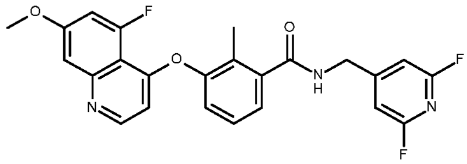 |
| 285 | 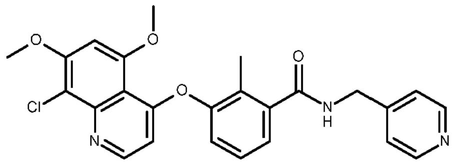 |
| 288 | 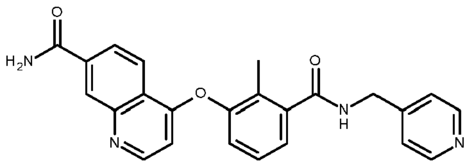 |
| 289 | 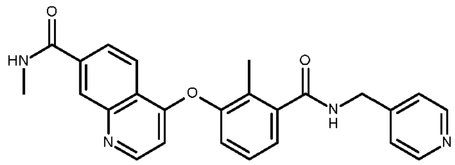 |
| 290 | 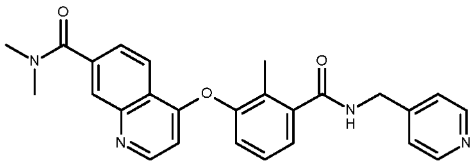 |
| 291 | 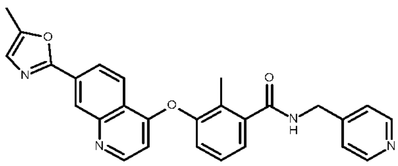 |

(continued)

| | |
|---|---|
| 293 | 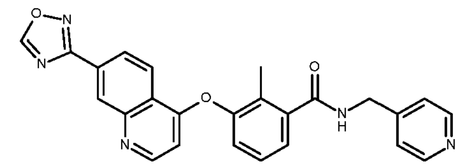 |
| 294 | 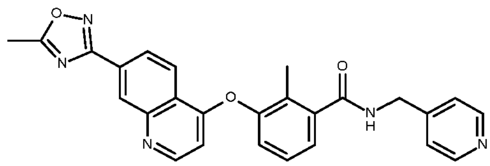 |
| 297 | 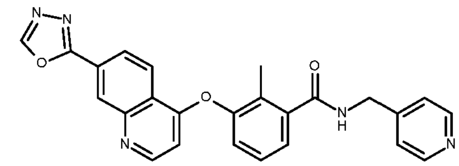 |
| 301 | 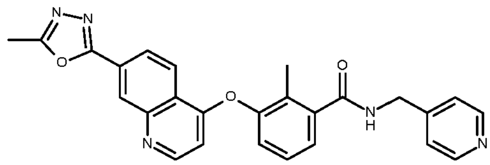 |
| 303 | 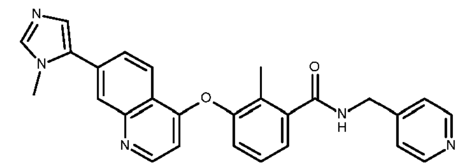 |
| 304 | 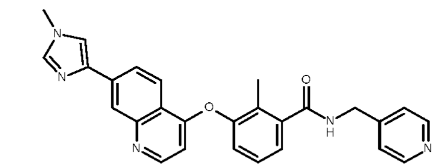 |
| 305 | 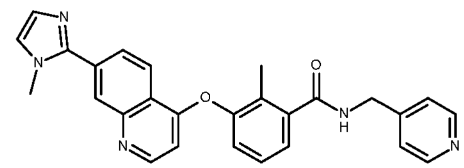 |
| 306 | 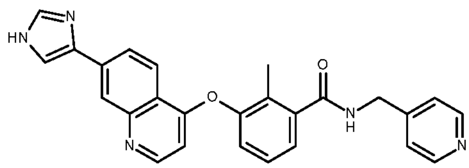 |
| 307 | 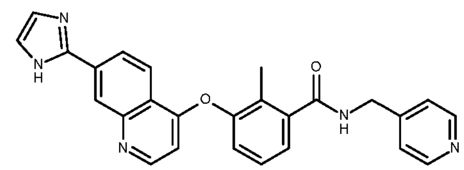 |

(continued)

| | |
|---|---|
| 308 | 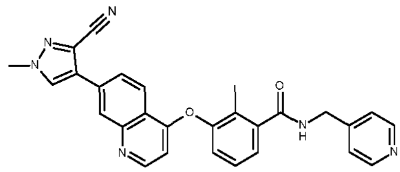 |
| 309 | 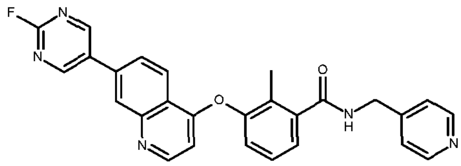 |
| 310 | 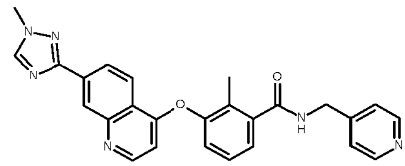 |
| 311 | 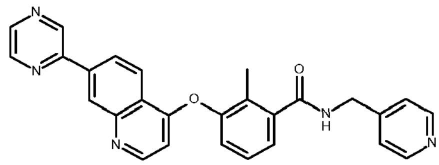 |
| 312 | 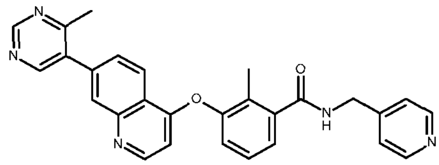 |
| 313 | 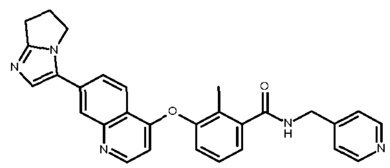 |
| 314 | 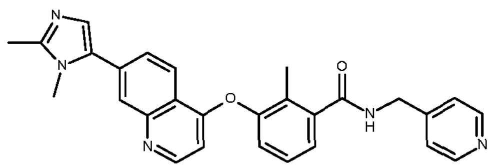 |
| 315 | 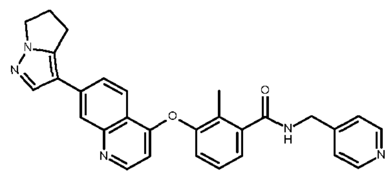 |
| 316 | 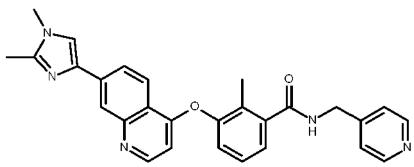 |

(continued)

| | |
|---|---|
| 317 | 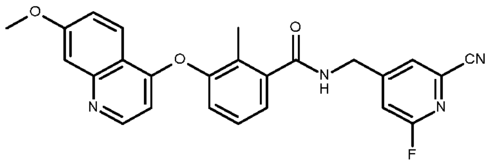 |
| 318 | 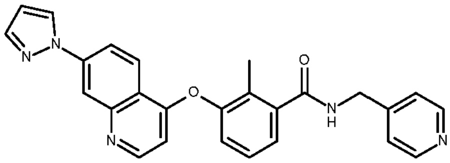 |
| 319 | 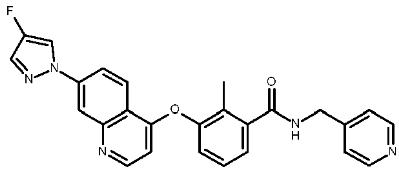 |
| 320 | 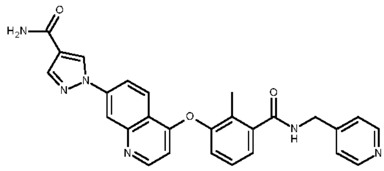 |
| 321 | 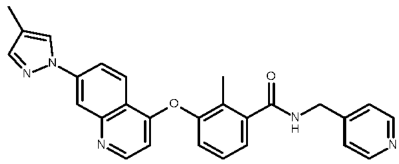 |
| 322 | 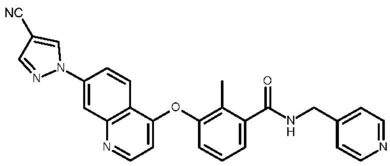 |
| 323 | 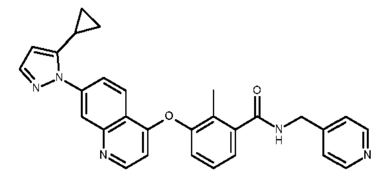 |
| 324 | 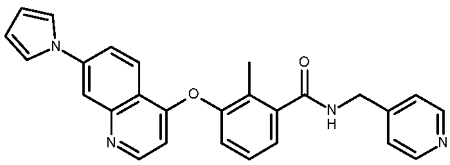 |
| 325 | 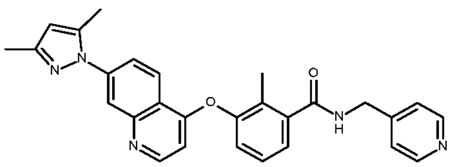 |

(continued)

| | |
|---|---|
| 326 | 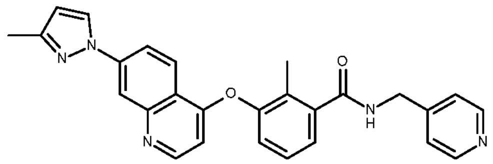 |
| 327 | 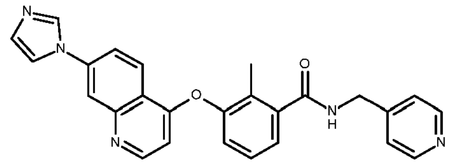 |
| 328 | 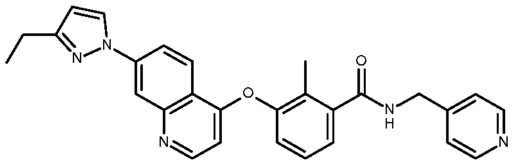 |
| 329 | 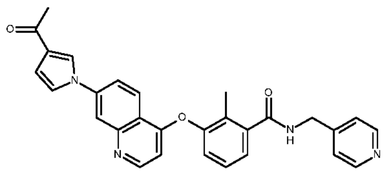 |
| 330 | 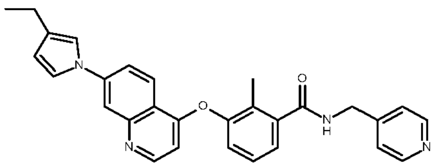 |
| 331 | 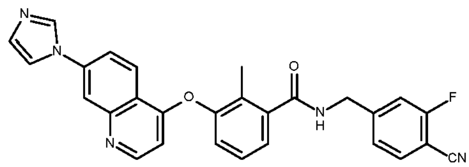 |
| 332 | 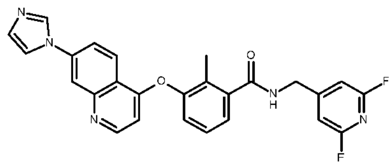 |
| 333 | 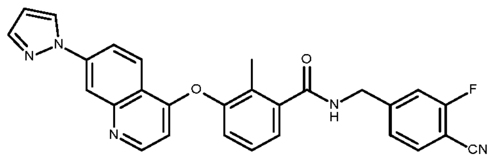 |
| 334 | 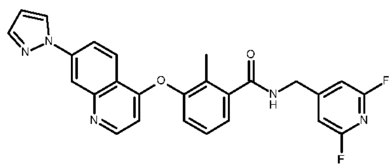 |

(continued)

| | |
|---|---|
| 336 | 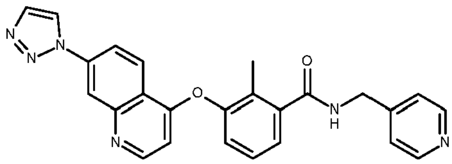 |
| 337 | 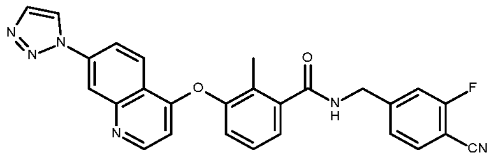 |
| 338 | 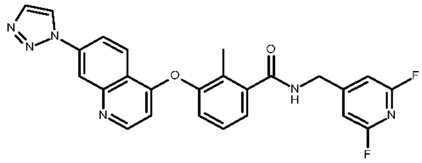 |
| 339 | 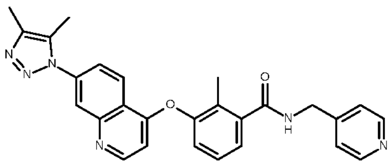 |
| 340 | 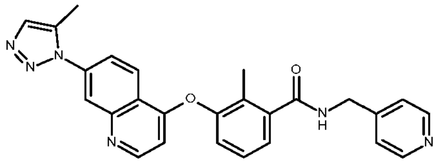 |
| 341 | 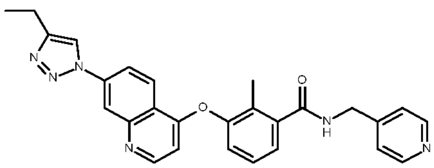 |
| 342 | 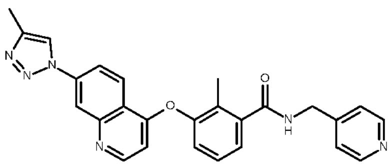 |
| 343 | 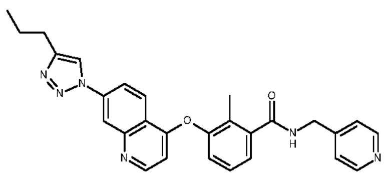 |
| 344 | 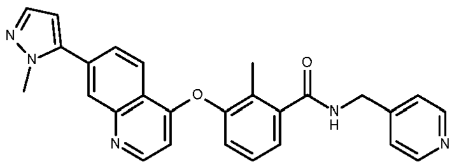 |

(continued)

| | |
|---|---|
| 345 | 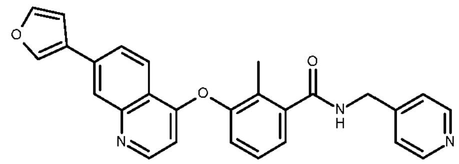 |
| 346 | 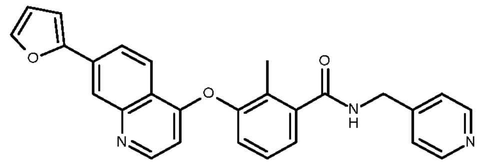 |
| 347 | 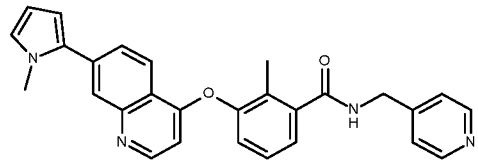 |
| 348 | 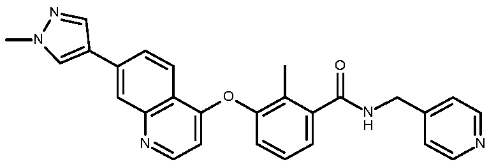 |
| 349 | 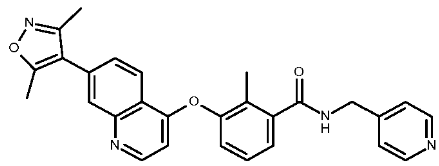 |
| 350 | 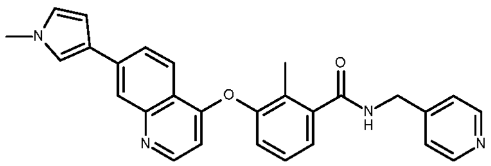 |
| 351 | 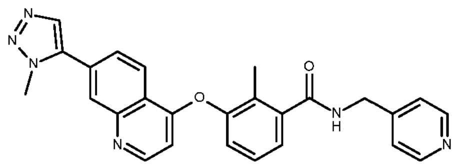 |
| 352 | 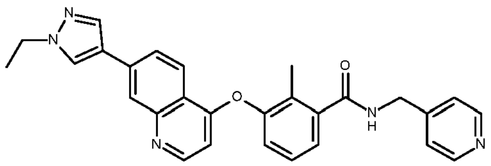 |
| 353 | 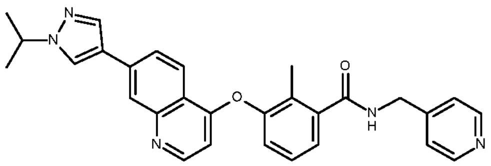 |

(continued)

| | |
|---|---|
| 354 | 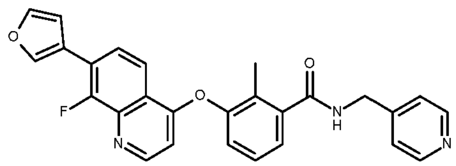 |
| 355 | 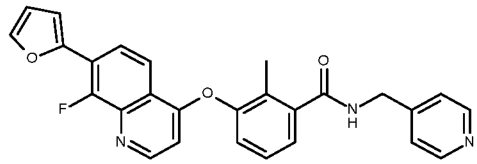 |
| 356 | 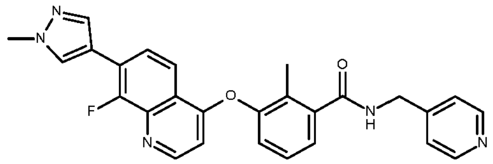 |
| 357 | 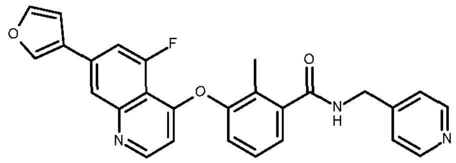 |
| 358 | 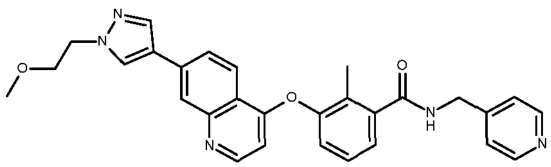 |
| 359 | 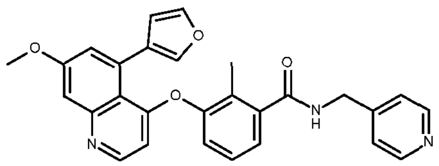 |
| 360 | 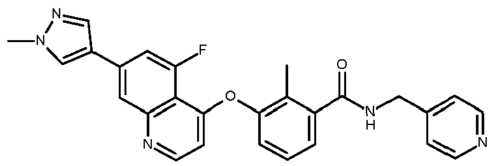 |
| 361 | 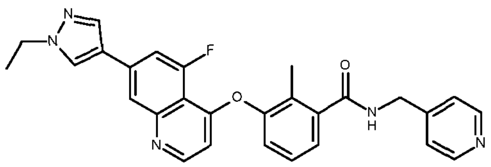 |
| 362 | 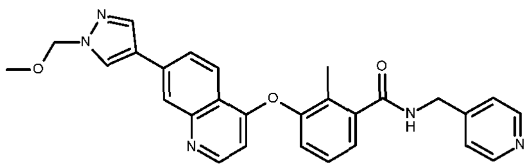 |

(continued)

| | |
|---|---|
| 363 | 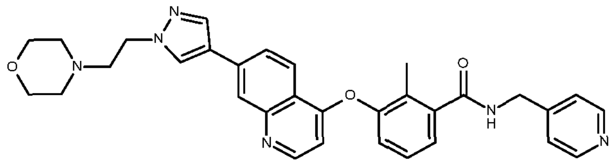 |
| 364 | 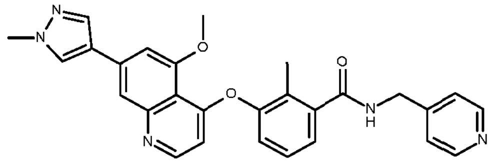 |
| 365 | 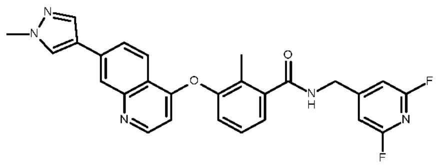 |
| 366 | 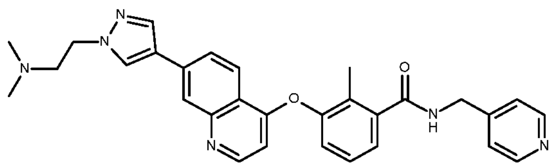 |
| 367 | 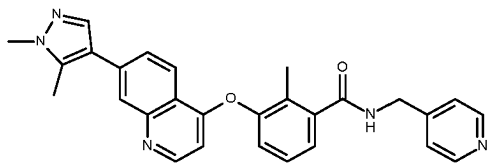 |
| 368 | 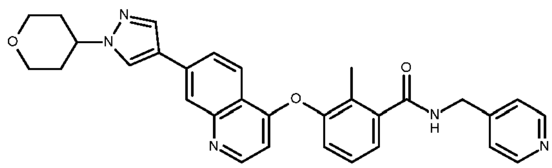 |
| 369 | 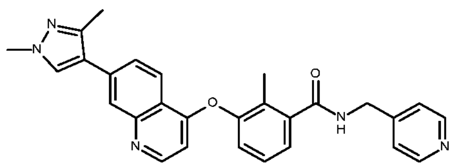 |
| 370 | 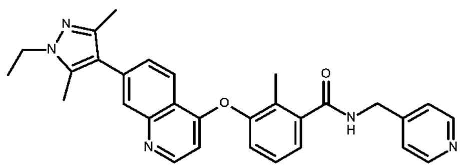 |
| 371 | 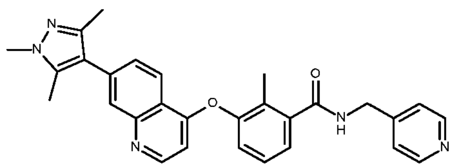 |

(continued)

| | |
|---|---|
| 372 | 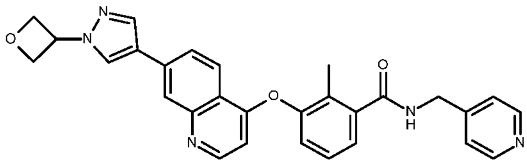 |
| 373 | 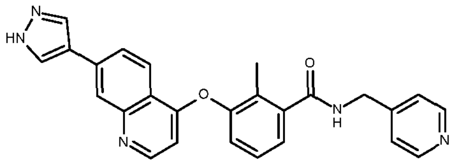 |
| 374 | 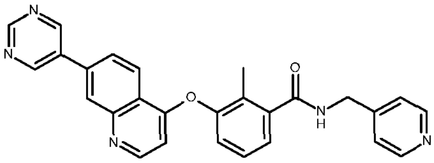 |
| 375 | 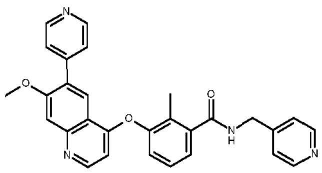 |
| 376 | 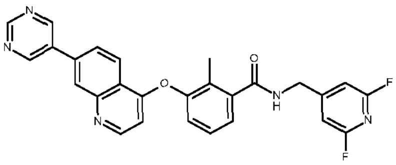 |
| 377 | 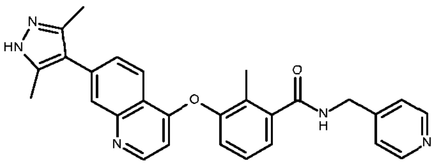 |
| 378 | 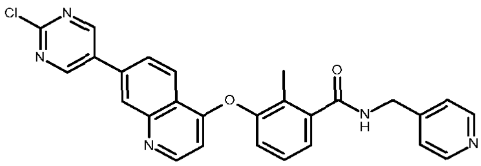 |
| 379 | 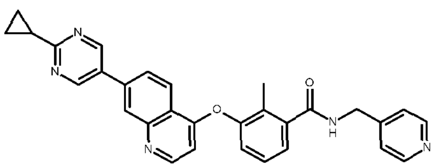 |
| 380 | 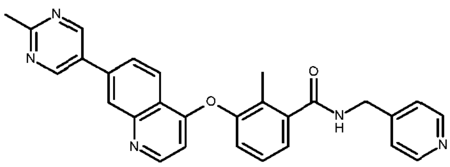 |

(continued)

| | |
|---|---|
| 381 | 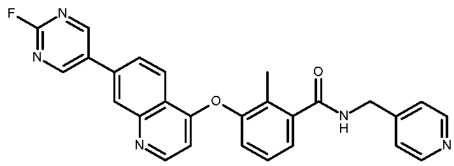 |
| 382 | 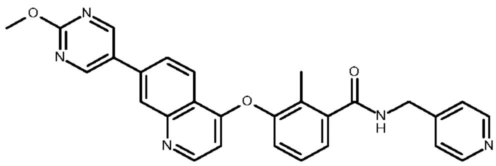 |
| 383 | 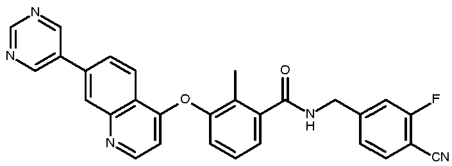 |
| 384 | 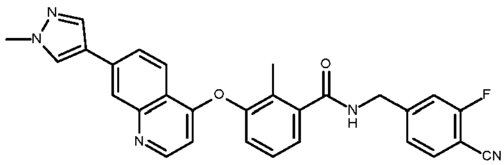 |
| 385 | 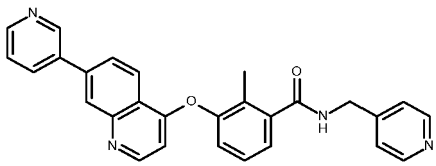 |
| 386 | 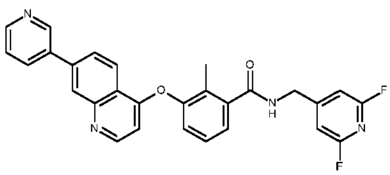 |
| 387 | 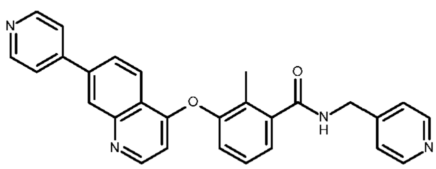 |
| 388 | 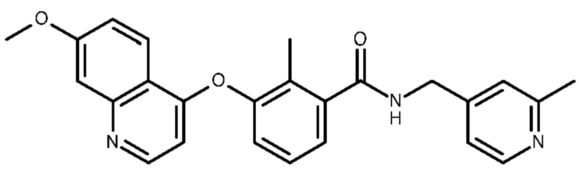 |
| 389 | 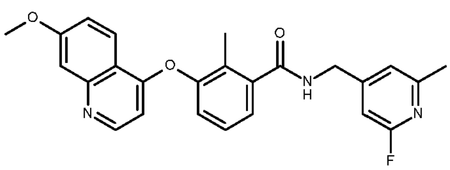 |

(continued)

| | |
|---|---|
| 390 | 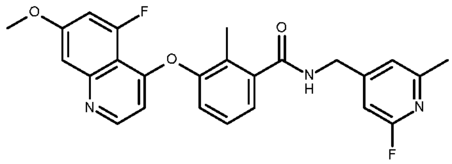 |
| 391 | 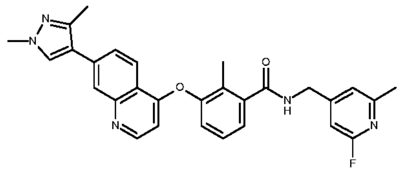 |
| 392 | 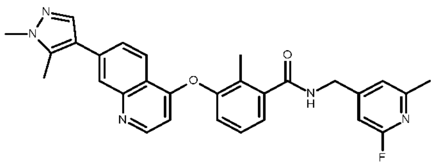 |
| 393 | 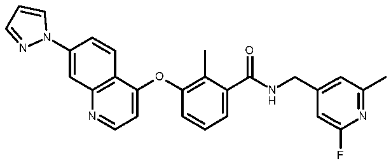 |
| 394 | 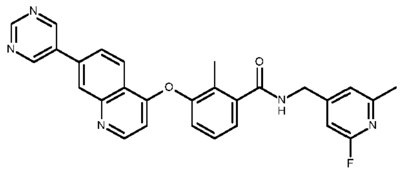 |

**Example 29: Cell-based assays: Biological assay measuring cell proliferation in leukemia cell lines**

Compounds were evaluated in different leukemia cell lines (Molm-13, MV4-11, HL-60 and M-NFS-60). For each, cell proliferation were measured. The protocols of these assays are described below.

MV4.11: Exponential growing MV4.11 cells (DSMZ, ACC-102) were seeded at 2.10^4 per 200 μl of complete medium. 20 μL of test compound dilution were added to each well and the plates were incubated for 72 h at 37 °C, 5% $CO_2$. Untreated cells and positive control (0,5% triton X-100, for the last 15 min) served as reference for maximum and minimum viability. At the end of incubation 100 μl of supernatant were removed and replaced by 10 μl of WST-1 solution (Cell Proliferation Reagent WST-1, Roche Applied Science). After 3 h incubation at 37 °C, 5% $CO_2$, optical densities were measured at 450 nm and 620 nm for the background on microplate reader (Envision 2105, Perkinelmer).

MOLM-13: Exponential growing MOLM-13 cells (DSMZ, ACC-554) were seeded at 2.10^4 per 200 μl of complete medium. 20 μL of test compound dilution were added to each well and the plates were incubated for 72 h at 37 °C, 5% $CO_2$. Untreated cells and positive control (0,5% triton X-100, for the last 15 min) served as reference for maximum and minimum viability. At the end of incubation 100 μl of supernatant were removed and replaced by 10 μl of WST-1 solution (Cell Proliferation Reagent WST-1, Roche Applied Science). After 3 h incubation at 37 °C, 5% $CO_2$, optical densities were measured at 450 nm and 620 nm for the background on microplate reader (Envision 2105, Perkinelmer).

M-NFS-60: Exponential growing M-NFS-60 cells (ATCC, CRL-1838) were seeded at 10^4 per 200 μl of complete medium with beta-mercaptoethanol and M-CSF (62 ng/mL) or IL34 (500 ng/mL). Twenty μL of test compound dilution were added to each well and the plates were incubated for 72 h at 37 °C, 5% $CO_2$. Untreated cells and positive control (0.5% triton X-100, for the last 15 min) served as reference for maximum and minimum viability. At the end of incubation 100 μl of supernatant were removed and replaced by 10 μl of WST-1 solution (Cell Proliferation Reagent WST-1, Roche Applied Science). After 3 h incubation at 37 °C, 5% $CO_2$, optical densities were measured at 450 nm and 620 nm for the background on microplate reader (Envision 2105, Perkinelmer).

HL-60: Exponential growing HL-60 cells (DSMZ, ACC-3) were seeded at 2.10^4 per 200 μl of complete RPMI medium. 20 μL of test compound dilution were added to each well and the plates were incubated for 72 h at 37 °C, 5% $CO_2$.

Untreated cells and positive control (0.5% triton X-100, for the last 15 min) served as reference for maximum and minimum viability. At the end of incubation 100 µl of supernatant were removed and replaced by 10 µl of WST-1 solution (Cell Proliferation Reagent WST-1, Roche Applied Science). After 3 h incubation at 37 °C, 5% $CO_2$, optical densities were measured at 450 nm and 620 nm for the background on microplate reader (Envision 2105, Perkinelmer).

IC50 were measured and experiments were performed at least in duplicate. Some biological results of these assays are presented in the following table.

*NB IC50 are reported as follows*

| > 1000 nM | 200-1000 nM | < 200 nM |
|---|---|---|

**Table 2: Results biological cell-based assay measuring cell proliferation in leukemia cell lines**

| Compounds | MV4.11 | MOLM-13 | M-NFS-60 | HL-60 |
|---|---|---|---|---|
| 3 | | | | |
| 4 | | | | |
| 5 | | | | |
| 6 | | | | |
| 7 | | | | |
| 8 | | | | |
| 10 | | | | |
| 13 | | | | |
| 14 | | | | |
| 15 | | | | |
| 16 | | | | |
| 17 | | | | |
| 18 | | | | |

| | | | | |
|---|---|---|---|---|
| 19 | | | | |
| 20 | | | | |
| 21 | | | | |
| 22 | | | | |
| 23 | | | | |
| 25 | | | | |
| 27 | | | | |
| 28 | | | | |
| 29 | | | | |
| 30 | | | | |
| 31 | | | | |
| 32 | | | | |
| 33 | | | | |
| 34 | | | | |
| 35 | | | | |
| 36 | | | | |
| 37 | | | | |
| 38 | | | | |
| 39 | | | | |
| 40 | | | | |
| 41 | | | | |
| 42 | | | | |
| 44 | | | | |
| 45 | | | | |
| 46 | | | | |
| 47 | | | | |
| 48 | | | | |
| 49 | | | | |
| 50 | | | | |
| 51 | | | | |

| | | | | |
|---|---|---|---|---|
| 52 | | | | |
| 54 | | | | |
| 55 | | | | |
| 56 | | | | |
| 57 | | | | |
| 58 | | | | |
| 59 | | | | |
| 60 | | | | |
| 61 | | | | |
| 62 | | | | |
| 63 | | | | |
| 64 | | | | |
| 65 | | | | |
| 66 | | | | |
| 67 | | | | |
| 68 | | | | |
| 71 | | | | |
| 72 | | | | |
| 74 | | | | |
| 75 | | | | |
| 76 | | | | |
| 84 | | | | |
| 85 | | | | |
| 86 | | | | |
| 87 | | | | |
| 88 | | | | |
| 89 | | | | |
| 90 | | | | |
| 91 | | | | |

| | | | | |
|---|---|---|---|---|
| 92 | | | | |
| 93 | | | | |
| 94 | | | | |
| 95 | | | | |
| 96 | | | | |
| 97 | | | | |
| 98 | | | | |
| 99 | | | | |
| 100 | | | | |
| 101 | | | | |
| 102 | | | | |
| 103 | | | | |
| 104 | | | | |
| 105 | | | | |
| 106 | | | | |
| 107 | | | | |
| 108 | | | | |
| 109 | | | | |
| 110 | | | | |
| 113 | | | | |
| 114 | | | | |
| 117 | | | | |
| 122 | | | | |
| 123 | | | | |
| 124 | | | | |
| 127 | | | | |
| 132 | | | | |
| 134 | | | | |
| 135 | | | | |

| | | | | |
|---|---|---|---|---|
| 136 | | | | |
| 137 | | | | |
| 138 | | | | |
| 139 | | | | |
| 140 | | | | |
| 141 | | | | |
| 142 | | | | |
| 143 | | | | |
| 144 | | | | |
| 145 | | | | |
| 146 | | | | |
| 147 | | | | |
| 148 | | | | |
| 149 | | | | |
| 150 | | | | |
| 151 | | | | |
| 152 | | | | |
| 153 | | | | |
| 154 | | | | |
| 155 | | | | |
| 160 | | | | |
| 161 | | | | |
| 162 | | | | |
| 163 | | | | |
| 164 | | | | |
| 165 | | | | |
| 166 | | | | |
| 167 | | | | |
| 168 | | | | |

| | | | | |
|---|---|---|---|---|
| 169 | | | | |
| 170 | | | | |
| 171 | | | | |
| 172 | | | | |
| 173 | | | | |
| 174 | | | | |
| 175 | | | | |
| 176 | | | | |
| 177 | | | | |
| 178 | | | | |
| 179 | | | | |
| 180 | | | | |
| 181 | | | | |
| 182 | | | | |
| 186 | | | | |
| 191 | | | | |
| 192 | | | | |
| 193 | | | | |
| 194 | | | | |
| 195 | | | | |
| 196 | | | | |
| 197 | | | | |
| 198 | | | | |
| 199 | | | | |
| 200 | | | | |
| 201 | | | | |
| 202 | | | | |
| 203 | | | | |
| 204 | | | | |

| | | | | |
|---|---|---|---|---|
| 205 | | | | |
| 206 | | | | |
| 207 | | | | |
| 208 | | | | |
| 209 | | | | |
| 210 | | | | |
| 211 | | | | |
| 224 | | | | |
| 225 | | | | |
| 226 | | | | |
| 227 | | | | |
| 228 | | | | |
| 229 | | | | |
| 232 | | | | |
| 250 | | | | |
| 251 | | | | |
| 252 | | | | |
| 253 | | | | |
| 254 | | | | |
| 257 | | | | |
| 258 | | | | |
| 259 | | | | |
| 261 | | | | |
| 262 | | | | |
| 263 | | | | |
| 264 | | | | |
| 266 | | | | |
| 267 | | | | |
| 268 | | | | |

| | | | | |
|---|---|---|---|---|
| 269 | | | | |
| 270 | | | | |
| 271 | | | | |
| 272 | | | | |
| 273 | | | | |
| 274 | | | | |
| 275 | | | | |
| 279 | | | | |
| 280 | | | | |
| 283 | | | | |
| 284 | | | | |
| 285 | | | | |
| 288 | | | | |
| 289 | | | | |
| 290 | | | | |
| 291 | | | | |
| 293 | | | | |
| 294 | | | | |
| 297 | | | | |
| 301 | | | | |
| 303 | | | | |
| 304 | | | | |
| 305 | | | | |
| 306 | | | | |
| 307 | | | | |
| 308 | | | | |
| 309 | | | | |
| 310 | | | | |
| 311 | | | | |

| | | | | |
|---|---|---|---|---|
| 312 | | | | |
| 313 | | | | |
| 314 | | | | |
| 315 | | | | |
| 316 | | | | |
| 317 | | | | |
| 318 | | | | |
| 319 | | | | |
| 320 | | | | |
| 321 | | | | |
| 322 | | | | |
| 323 | | | | |
| 324 | | | | |
| 325 | | | | |
| 326 | | | | |
| 327 | | | | |
| 328 | | | | |
| 329 | | | | |
| 330 | | | | |
| 331 | | | | |
| 332 | | | | |
| 333 | | | | |
| 334 | | | | |
| 336 | | | | |
| 337 | | | | |
| 338 | | | | |
| 339 | | | | |
| 340 | | | | |
| 341 | | | | |

| | | | | |
|---|---|---|---|---|
| 342 | | | | |
| 343 | | | | |
| 344 | | | | |
| 345 | | | | |
| 346 | | | | |
| 347 | | | | |
| 348 | | | | |
| 349 | | | | |
| 350 | | | | |
| 351 | | | | |
| 352 | | | | |
| 353 | | | | |
| 354 | | | | |
| 355 | | | | |
| 356 | | | | |
| 357 | | | | |
| 358 | | | | |
| 359 | | | | |
| 360 | | | | |
| 361 | | | | |
| 352 | | | | |
| 363 | | | | |
| 364 | | | | |
| 365 | | | | |
| 366 | | | | |
| 367 | | | | |
| 368 | | | | |
| 369 | | | | |
| 370 | | | | |

| | | | | |
|---|---|---|---|---|
| 371 | | | | |
| 372 | | | | |
| 373 | | | | |
| 374 | | | | |
| 375 | | | | |
| 376 | | | | |
| 377 | | | | |
| 378 | | | | |
| 379 | | | | |
| 378 | | | | |
| 379 | | | | |
| 380 | | | | |
| 381 | | | | |
| 382 | | | | |
| 383 | | | | |
| 384 | | | | |
| 385 | | | | |
| 386 | | | | |
| 387 | | | | |
| 388 | | | | |
| 389 | | | | |
| 390 | | | | |
| 391 | | | | |
| 392 | | | | |
| 393 | | | | |
| 394 | | | | |

**Example 30: Cell-based assays: Biological assay measuring cell proliferation in non-cancer cell lines**

CSF1R receptor has been expressed in HEK cell lines following the protocols below.

HEK-CSF1 R-STAT5-Luc: Exponential growing HEK293T cells (ATCC® CRL-3216™), ectopically expressing human CSF1R receptor (Origene) and five copies of a STAT5 response element (STAT5 RE, promega) that drives transcription of the luciferase reporter were seeded at 5.10^3 per 20 µl of complete DMEM medium. The next day, 2.25 µL of test compound dilution were added to each well and stimulated with 600 ng/ml of M-CSF. The plates were incubated for 24 h at 37 °C, 5% CO2. Unstimulated and stimulated cells served as reference for maximum and minimum induction. At the end of incubation 25 µl of Steady-Glo® Luciferase Assay System (Promega) were added after 5 min of lysis, luminescence was measured on microplate reader (Envision 2105, Perkinelmer).

HEK-CSF1 R-SRE-Luc: Exponential growing HEK293T cells (ATCC® CRL-3216™), ectopically expressing

human CSF1R receptor (Origène) and Serum Response Element (SRE, promega) that drives transcription of the luciferase reporter were seeded at 5.10^3 per 20 μl of complete DMEM medium. The next day, 2.25 μL of test compound dilution were added to each well and stimulated with 600 ng/ml of M-CSF. The plates were incubated for 24 h at 37 °C, 5% CO2. Unstimulated and stimulated cells served as reference for maximum and minimum induction. At the end of incubation 25 μl of Steady-Glo® Luciferase Assay System (Promega) were added after 5 min of lysis, luminescence was measured on microplate reader (Envision 2105, Perkinelmer).

HEK-CSF1R-WST-1: Exponential growing HEK293T cells (ATCC® CRL-3216™), were seeded at 5.10^3 per 200 μl of complete DMEM medium. The next day, twenty μL of test compound dilution were added to each well and the plates were incubated for 72 h at 37 °C, 5% CO2. Untreated cells and positive control (0,5% triton X-100, for the last 15 min) served as reference for maximum and minimum viability. At the end of incubation 100 μl of supernatant were removed and replaced by 10 μl of WST-1 solution (Cell Proliferation Reagent WST-1, Roche Applied Science). After 3 h incubation at 37 °C, 5% CO2, optical densities were measured at 450 nm and 620 nm for the background, on microplate reader (Envision 2105, Perkinelmer).

IC50 were measured and experiments were performed at least in duplicate. Some biological results of these assays are presented in the following table.

*NB IC50 are reported* as *follows*

| | | |
|---|---|---|
| *> 500 nM* | *100-500 nM* | *< 100 nM* |

**Table 3: Results biological cell-based assay measuring cell proliferation in non-cancer cell lines**

| Compounds | HEK-CSF1R-STAT5 Luciferase | HEK-CSF1R-STAT5 WST-1 | HEK-CSF1R-SRE Luciferase | HEK-CSF1R-SRE WST-1 |
|---|---|---|---|---|
| 17 | | | | |
| 23 | | | | |
| 29 | | | | |
| 30 | | | | |
| 31 | | | | |
| 32 | | | | |
| 33 | | | | |
| 34 | | | | |
| 35 | | | | |
| 40 | | | | |
| 44 | | | | |
| 48 | | | | |
| 49 | | | | |
| 50 | | | | |
| 54 | | | | |
| 56 | | | | |

| | | | | |
|---|---|---|---|---|
| 58 | | | | |
| 59 | | | | |
| 62 | | | | |
| 63 | | | | |
| 64 | | | | |
| 65 | | | | |
| 66 | | | | |
| 67 | | | | |
| 84 | | | | |
| 88 | | | | |
| 89 | | | | |
| 90 | | | | |
| 92 | | | | |
| 93 | | | | |
| 94 | | | | |
| 95 | | | | |
| 96 | | | | |
| 97 | | | | |
| 98 | | | | |
| 99 | | | | |
| 101 | | | | |
| 103 | | | | |
| 105 | | | | |
| 107 | | | | |
| 108 | | | | |
| 109 | | | | |
| 113 | | | | |
| 134 | | | | |
| 135 | | | | |

| | | | | |
|---|---|---|---|---|
| 136 | | | | |
| 137 | | | | |
| 138 | | | | |
| 139 | | | | |
| 140 | | | | |
| 143 | | | | |
| 145 | | | | |
| 146 | | | | |
| 147 | | | | |
| 148 | | | | |
| 149 | | | | |
| 150 | | | | |
| 151 | | | | |
| 152 | | | | |
| 153 | | | | |
| 154 | | | | |
| 163 | | | | |
| 165 | | | | |
| 168 | | | | |
| 171 | | | | |
| 172 | | | | |
| 173 | | | | |
| 174 | | | | |
| 175 | | | | |
| 176 | | | | |
| 177 | | | | |
| 178 | | | | |
| 179 | | | | |
| 182 | | | | |

| | | | | |
|---|---|---|---|---|
| 186 | | | | |
| 191 | | | | |
| 194 | | | | |
| 195 | | | | |
| 196 | | | | |
| 197 | | | | |
| 198 | | | | |
| 199 | | | | |
| 200 | | | | |
| 201 | | | | |
| 202 | | | | |
| 203 | | | | |
| 204 | | | | |
| 205 | | | | |
| 206 | | | | |
| 207 | | | | |
| 208 | | | | |
| 209 | | | | |
| 210 | | | | |
| 211 | | | | |
| 224 | | | | |
| 225 | | | | |
| 226 | | | | |
| 227 | | | | |
| 228 | | | | |
| 229 | | | | |
| 232 | | | | |
| 250 | | | | |
| 251 | | | | |

| | | | | |
|---|---|---|---|---|
| 252 | | | | |
| 253 | | | | |
| 254 | | | | |
| 255 | | | | |
| 256 | | | | |
| 257 | | | | |
| 258 | | | | |
| 259 | | | | |
| 261 | | | | |
| 262 | | | | |
| 263 | | | | |
| 264 | | | | |
| 266 | | | | |
| 267 | | | | |
| 268 | | | | |
| 269 | | | | |
| 270 | | | | |
| 271 | | | | |
| 272 | | | | |
| 273 | | | | |
| 274 | | | | |
| 275 | | | | |
| 279 | | | | |
| 280 | | | | |
| 283 | | | | |
| 284 | | | | |
| 285 | | | | |
| 288 | | | | |
| 289 | | | | |

| | | | | |
|---|---|---|---|---|
| 290 | | | | |
| 291 | | | | |
| 293 | | | | |
| 294 | | | | |
| 297 | | | | |
| 301 | | | | |
| 303 | | | | |
| 304 | | | | |
| 305 | | | | |
| 306 | | | | |
| 307 | | | | |
| 308 | | | | |
| 309 | | | | |
| 310 | | | | |
| 311 | | | | |
| 312 | | | | |
| 313 | | | | |
| 314 | | | | |
| 315 | | | | |
| 316 | | | | |
| 317 | | | | |
| 318 | | | | |
| 319 | | | | |
| 320 | | | | |
| 321 | | | | |
| 322 | | | | |
| 323 | | | | |
| 324 | | | | |
| 325 | | | | |

| | | | | |
|---|---|---|---|---|
| 326 | | | | |
| 327 | | | | |
| 328 | | | | |
| 329 | | | | |
| 330 | | | | |
| 331 | | | | |
| 332 | | | | |
| 333 | | | | |
| 334 | | | | |
| 336 | | | | |
| 337 | | | | |
| 338 | | | | |
| 339 | | | | |
| 340 | | | | |
| 341 | | | | |
| 342 | | | | |
| 343 | | | | |
| 344 | | | | |
| 345 | | | | |
| 346 | | | | |
| 347 | | | | |
| 348 | | | | |
| 349 | | | | |
| 350 | | | | |
| 351 | | | | |
| 352 | | | | |
| 353 | | | | |
| 354 | | | | |
| 355 | | | | |

| | | | | |
|---|---|---|---|---|
| 356 | | | | |
| 357 | | | | |
| 358 | | | | |
| 359 | | | | |
| 360 | | | | |
| 361 | | | | |
| 362 | | | | |
| 363 | | | | |
| 364 | | | | |
| 365 | | | | |
| 366 | | | | |
| 367 | | | | |
| 368 | | | | |
| 369 | | | | |
| 370 | | | | |
| 371 | | | | |
| 372 | | | | |
| 373 | | | | |
| 374 | | | | |
| 375 | | | | |
| 376 | | | | |
| 377 | | | | |
| 378 | | | | |
| 379 | | | | |
| 380 | | | | |
| 381 | | | | |
| 382 | | | | |
| 383 | | | | |
| 384 | | | | |

| | | | | |
|---|---|---|---|---|
| **385** | | | | |
| **386** | | | | |
| **387** | | | | |
| **388** | | | | |
| **389** | | | | |
| **390** | | | | |
| **391** | | | | |
| **392** | | | | |
| **393** | | | | |
| **394** | | | | |

**Example 31: Target-based assays: Biological assay measuring the protein kinase inhibition**

A biochemical assay was performed in order to measure the protein kinase inhibition. The kinase binding assays were performed against different kinases using the SelectScreen Biochemical Kinase Profiling Service® platform (ThermoFisher Scientific). For all kinases, the Z'-Lyte ®biochemical kinase assay technology was used. Inhibitions were measured at 50 nM in duplicate for each compound and are reported in percentage. Some data are presented in the following table.

**Table 4: Results biological target-based assay**

| Compounds | CSF1R | FLT3 | Kit | PDGFRA | PDGFRB |
|---|---|---|---|---|---|
| **3** | 71 | 6 | 7 | 18 | 27 |
| **7** | 85 | 14 | 20 | 16 | 22 |
| **10** | 71 | 18 | 11 | 17 | 24 |
| **16** | 81 | 7 | 15 | 23 | 24 |
| **17** | 83 | -4 | 14 | 15 | 12 |
| **24** | 97 | 44 | 29 | 24 | 34 |
| **25** | 95 | 48 | 32 | 27 | 39 |
| **26** | 102 | 47 | 32 | 35 | 50 |
| **27** | 93 | 39 | 26 | 23 | 44 |
| **29** | 93 | 2 | 17 | 21 | 28 |
| **30** | 72 | 10 | 8 | 8 | 11 |
| **32** | 96 | 8 | 2 | 2 | 6 |
| **33** | 78 | 16 | 4 | 10 | 14 |
| **74** | 83 | 25 | 20 | 7 | 12 |
| **75** | 79 | 16 | 20 | -1 | 10 |
| **84** | 91 | 37 | 34 | 16 | 54 |
| **85** | 85 | 26 | 31 | 22 | 28 |
| **88** | 85 | 29 | 30 | 31 | 53 |
| **89** | 86 | 36 | 21 | 49 | 48 |
| **90** | 77 | 26 | 9 | 28 | 28 |

(continued)

| Compounds | CSF1R | FLT3 | Kit | PDGFRA | PDGFRB |
|---|---|---|---|---|---|
| **92** | 94 | 50 | 13 | 24 | 60 |
| **93** | 82 | 3 | 2 | 23 | 24 |
| **94** | 93 | 27 | 32 | 30 | 45 |
| **98** | 79 | 6 | 5 | 6 | 23 |
| **99** | 75 | 26 | 0 | 17 | 20 |
| **101** | 81 | 37 | 27 | 25 | 40 |
| **103** | 73 | 8 | 7 | -5 | 13 |
| **107** | 85 | 19 | 11 | 22 | 25 |
| **108** | 88 | 6 | 10 | 11 | 13 |
| **140** | 83 | 8 | 3 | 11 | 19 |
| **142** | 61 | 9 | 13 | 11 | 9 |
| **143** | 77 | 3 | 8 | 14 | 32 |
| **148** | 75 | 7 | 0 | 4 | 12 |
| **150** | 81 | 12 | 3 | 8 | 15 |
| **151** | 79 | 0 | 4 | 9 | 21 |
| **152** | 77 | 13 | 3 | 10 | 25 |

**Example 32: Efficacy of compound 32 in a streptozotocin-induced diabetic neuropathic pain model**

The experiment has been conducted in 8 weeks old male C57Bl6J mice (from Charles River). For this study, 6 mice per group have been used.

Diabetes has been induced by a single IP injection of 200μg/mL streptozotocin solution (100μL/10g). Glycemia has been tested before, D+3 and D+7 post-injection. All injected mice presented blood glucose concentration <350 mg/dL at D+7, and then have been used for analgesic testing of the compounds.

Compound **32** has been orally administrated at 6 mg/mL in Capryol 90 solution (50μL/10g) and pregabalin has been subcutaneously injected at 0.5 mg/mL in phosphate buffer (100μL/10g) at D+14 and D+16. A time course measurement at +1h, +2h, +4h and +6h has been performed after compound administration. Mechanical threshold response of mice has been measured with calibrated Von Frey filaments using the up/down method, blind for manipulator. Measurements have been performed before streptozotocin injection and 14 days after. Statistical analysis was performed using SigmaPlot 12.5 software. Percentage of maximum possible effect is calculated as follow

$$\%MPE = [(Test-Prest)/(cut\text{-}off-Prest)]*100$$

Where cut-off, Test and Pretest values correspond respectively to baseline measure, timepoint post-administration measure and measure before administration.

**Table 5: Analgesic effect of compound 32 after administration expressed as % of baseline**

| Treatment | Baseline | Pre-drug | +1h | +2h | +4h | +6h |
|---|---|---|---|---|---|---|
| **Vehicle (Capryol 90)** | 100.0 ±6.0 | 20.8 ±3.6 | 18.0 ±2.1 | 18.9 ±1.9 | 13.3 ±2.1 | 13.9 ±1.2 |
| **Pregabalin** | 100.0 ±3.7 | 17.5 ±3.1 | 81.2 ±7.9 | 86.0 ±9.1 | 45.0 ±5.3 | 23.5 ±2.1 |
| **Compound 32** | 100.0 ±5.9 | 26.6 ±3.5 | 59.3 ±7.2 | 58.2 ±12.0 | 37.9 ±5.3 | 22.2 ± 2.3 |

**Table 6: Analgesic effect of compound 32 after administration expressed as % MPE**

| Treatment | +1h | +2h | +4h | +6h |
|---|---|---|---|---|
| **Vehicle (Capryol 90)** | -6.2±6.1 | -4.9±5.6 | -12.4±6.4 | -11.9±6.9 |

(continued)

| Treatment | +1h | +2h | +4h | +6h |
|---|---|---|---|---|
| **Pregabalin** | 80.7±10.8 | 84.6±10.6 | 33.9±5.8 | 5.9±4.3 |
| **Compound 32** | 46.9±8.9 | 48.2±18.1 | 15.4±7.4 | 4.4±3.6 |

**Example 33: Efficacy of compound 108 in a streptozotocin-induced diabetic neuropathic pain model**

The experiment has been conducted in 8 weeks old male C57Bl6J mice (from Charles River). For this study, 10 mice per group have been used.

Diabetes has been induced by a single IP injection of 200μg/mL streptozotocin solution (100μL/10g). Glycemia has been tested before, D+3 and D+7 post-injection. All injected mice presented blood glucose concentration <350 mg/dL at D+7, and then have been used for analgesic testing of the compounds.

Compound **108** has been orally administrated at 2, 5 and 10 mg/mL in 0.9% NaCl solution (50μL/10g) and pregabalin has been subcutaneously injected at 0.5 mg/mL in phosphate buffer (100μL/10g) at D+14 and D+17. A time course measurement at +1h, +2h, +4h and +6h has been performed after compound administration.

Mechanical threshold response of mice has been measured with calibrated Von Frey filaments using the up/down method, blind for manipulator. Measurements have been performed before streptozotocin injection and 14 days after. Statistical analysis was performed using SigmaPlot 12.5 software. Percentage of maximum possible effect is calculated as follow

$$\%MPE = [(Test-Prest)/(cut\text{-}off-Prest)]*100$$

Where cut-off, Test and Pretest values correspond respectively to baseline measure, timepoint post-administration measure and measure before administration.

**Table 7: Analgesic effect of compound 108 after administration expressed as % of baseline**

| Treatment | Baseline | Pre-drug | +1h | +2h | +4h | +6h |
|---|---|---|---|---|---|---|
| **Vehicle (0.9% NaCl)** | 100.0 ±8.6 | 23.9 ±3.5 | 19.8 ±2.9 | 22.7 ±3.1 | 17.0 ±2.8 | 24.7 ±3.6 |
| **Pregabalin** | 100.0 ±8.2 | 21.2 ±3.4 | 80.6 ±12.4 | 85.7 ±10.7 | 45.6 ±13.0 | 30.3 ±4.8 |
| **Compound 108, 2 mg/mL** | 100.0 ±5.7 | 27.8 ±3.8 | 74.6 ±9.1 | 66.2 ±9.0 | 48.9 ±6.9 | 27.6 ± 3.5 |
| **Compound 108, 5 mg/mL** | 100.0 ±4.2 | 22.3 ±2.9 | 65.2 ±7.0 | 80.1 ±11.4 | 48.3 ±7.0 | 29.6 ± 3.5 |
| **Compound 108, 10 mg/mL** | 100.0 ±6.6 | 20.4 ±2.7 | 103.5 ±14.9 | 112.9 ±19.1 | 58.9 ±11.6 | 41.0 ± 8.9 |

**Table 8: Analgesic effect of compound 108 after administration expressed as % MPE**

| Treatment | +1h | +2h | +4h | +6h |
|---|---|---|---|---|
| **Vehicle (0.9% NaCl)** | -5.9 ±3.2 | -2.5 ±4.9 | -10.0 ±4.8 | -0.9 ±9.4 |
| **Pregabalin** | 76.6 ±15.4 | 79.5 ±13.7 | 31.0 ±17.0 | 10.9 ±7.5 |
| **Compound 108, 2 mg/mL** | 63.5 ±12.5 | 58.7 ±12.4 | 29.7 ±9.3 | 1.6 ±8.6 |
| **Compound 108, 5 mg/mL** | 53.5 ±9.2 | 68.9 ±14.1 | 33.2 ±9.6 | 16.9 ±6.5 |
| **Compound 108, 10 mg/mL** | 105.6 ±19.1 | 117.4 ±20.7 | 42.3 ±10.8 | 20.36 ±8.8 |

**Comparative example 34: Procedure for the preparation of quinazoline-based analogues 395-397 (i.e comparative compounds 395-397, i.e. CC395, CC396, and CC397)**

The following procedures illustrate the preparation of quinazoline-based comparative compounds according to Method I, described in example 2. The synthetic scheme is outlined below.

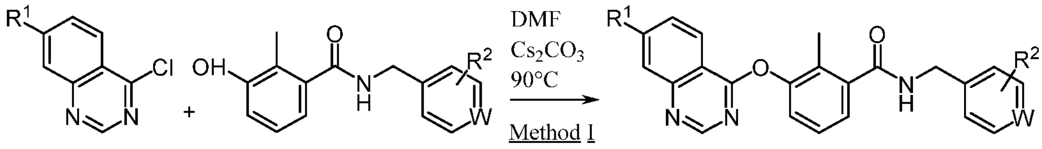


Preparation of 3-(7-methoxyquinazolin-4-yl)oxy-2-methyl-N-(4-pyridylmethyl)benzamide **(CC395):**

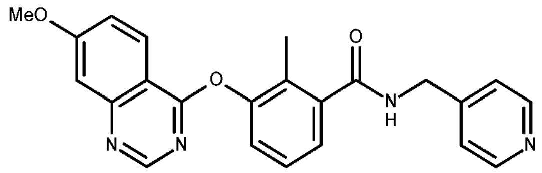

Compound **CC395** was synthesized from intermediate **81e** (0.20 mmol) and 4-chloro-7-methoxyquinazoline (0.20 mmol) as a white powder in 65% yield (52 mg) according to the general method I. $^1$H NMR (400 MHz, DMSO-$d_6$) δ (ppm): 9.08 (t, *J* = 5.93 Hz, 1H), 8.63 (bs, 1H), 8.54-8.52 (m, 2H), 8.34-8.32 (m, 1H), 7.42-7.34 (m, 7H), 4.49 (d, *J* = 5.94 Hz, 2H), 3.99 (s, 3H), 2.11 (s, 3H) ; MS (ESI, EI$^+$): *m/z*= 401.35 (MH$^+$).

Preparation of N-[(3-fluoro-4-pyridyl)methyl]-3-(7-methoxyquinazolin-4-yl)oxy-2-methyl-benzamide **(CC396):**

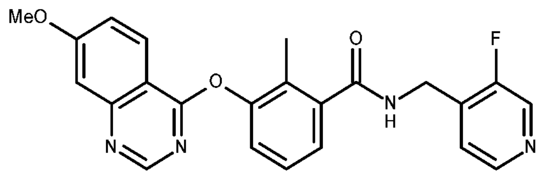

Compound **CC396** was synthesized from intermediate **81f** (0.20 mmol) and 4-chloro-7-methoxyquinazoline (0.20 mmol) as a white powder in 44% yield (37 mg) according to the general method I. $^1$H NMR (400 MHz, DMSO-$d_6$) δ (ppm): 9.10 (t, *J* = 5.73 Hz, 1H), 8.63 (bs, 1H), 8.54 (d, *J* = 1.68 Hz, 1H), 8.43-8.42 (m, 1H), 8.34-8.32 (m, 1H), 7.47-7.36 (m, 6H), 4.55 (d, *J* = 5.92 Hz, 2H), 3.99 (s, 3H), 2.10 (s, 3H) ; MS (ESI, EI$^+$): *m/z*= 419.35 (MH$^+$).

Preparation of 3-(7-chloroquinazolin-4-yl)oxy-2-methyl-N-(4-pyridylmethyl)benzamide (**CC397**):

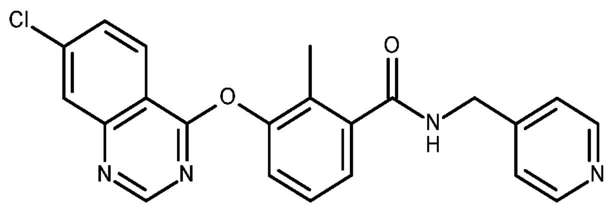

Compound **CC397** was synthesized from intermediate **81e** (0.30 mmol) and 4,7-dichloroquinazoline (0.30 mmol) as a white powder in 57% yield (69 mg) according to the general method I. $^1$H NMR (400 MHz, DMSO-$d_6$) δ (ppm): 9.10 (t, *J* = 6.13 Hz, 1H), 8.75 (bs, 1H), 8.55-8.53 (m, 2H), 8.47 (d, *J* = 8.72 Hz, 1H), 8.13 (d, *J* = 1.97 Hz, 1H), 7.86 (dd, *J* = 1.99 and 8.66 Hz, 1H), 7.44-7.41 (m, 3H), 7.37-7.35 (m, 2H), 4.49 (d, *J* = 6.03 Hz, 2H), 2.13 (s, 3H) ; MS (ESI, EI$^+$): *m/z*= 405.25 (MH$^+$).

**Comparative example 35: Cell-based assays comparison between compounds 32, 44, 108 and quinazoline-based comparative compounds CC395-CC397**

Cell-based assays of compounds **CC395-CC397,** depicted above, against M-NFS-60 and HEK-CSF1R have been performed following the procedures described in example 29 and 30 and compared with their quinoline analogues **32, 44** and **108.** IC50 are presented in the table 9 below:

**Table 9: Results biological cell-based assays measuring cell proliferation in cancer and non-cancer cell lines**

| | Structure | M-NFS-60 | HEK-CSF1R-STAT5 |
|---|---|---|---|
| | | IC 50 (nM) | |
| **CC 395** | 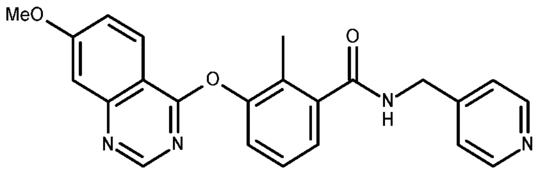 | 636 | 926 |
| **32** | 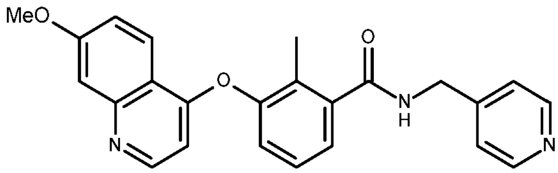 | 34 | 26 |
| **CC 396** | 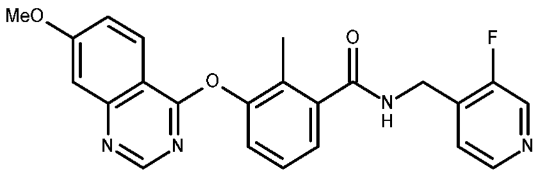 | 415 | 600 |
| **44** | 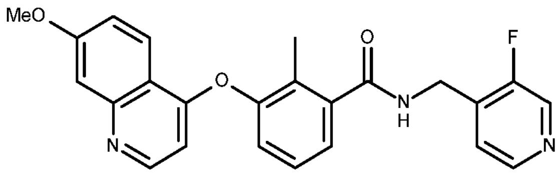 | 19 | 7 |
| **CC 397** | 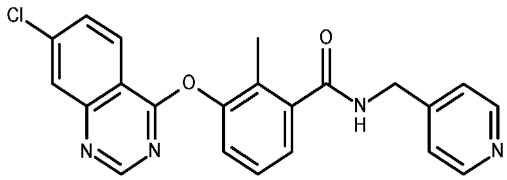 | 1260 | 4147 |
| **108** | 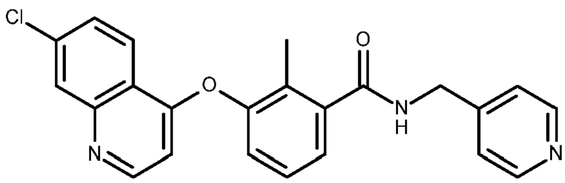 | 58 | 29 |

It has been found that the quinoline analogues **32, 44** and **108,** show respectively a 19-fold, 22-fold and 22-fold decrease in IC50 against M-NFS-60, and respectively a 36-fold, 86-fold and 145-fold decrease in IC50 against HEK-CSF1R-STAT5 compared to the comparative example quinazoline-based comparative compounds **CC395, CC396** and **CC397,** therefore showing a superior inhibiting effect of compounds of the present invention towards cancer M-NFS-60 and non-cancer HEK-CSF1R-STAT5 cell lines.

**Comparative example 36: Analogues 398 - 401 (i.e. compound 398 and comparative compounds 399-401, i.e. CC399, CC400 and CC401)**

Analogues **398-401** have been prepared to illustrate the non-equivalence of the bridging group CONH. The biological results are reported in the table below.

**Table 10: Results biological cell-based assays measuring cell proliferation in cancer and non-cancer cell lines**

| | Structure | M-NFS-60 | HEK-CSF1R-STAT5 |
|---|---|---|---|
| | | IC 50 (nM) | |
| **398** | 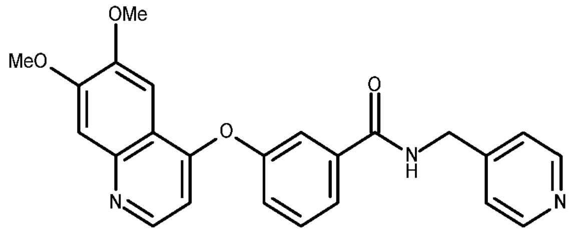 | 198 | 58 |
| **CC399** | 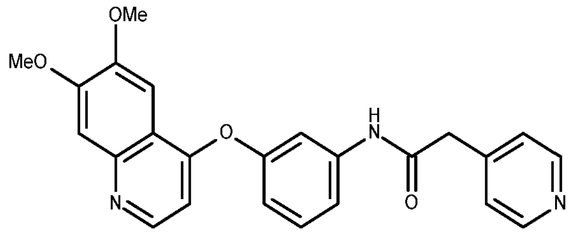 | 5213 | 3539 |
| **CC400** | 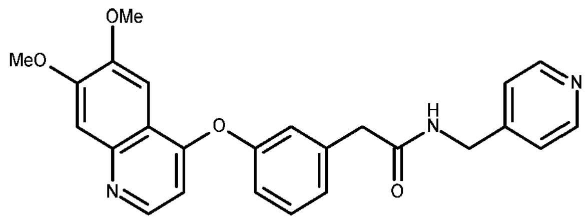 | 4215 | 2266 |
| | | | |
| **CC401** | 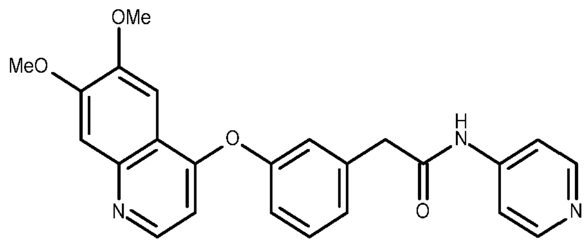 | 7512 | 3846 |

It has been found that the carboxamide **398** shows respectively a 26-fold, 21-fold and 38-fold decrease in IC50 against M-NFS-60, and respectively a 61-fold, 39-fold and 67-fold decrease in IC50 against HEK-CSF1R-STAT5 compared to the comparative compounds **CC399, CC400** and **CC401,** therefore showing a superior inhibiting effect of compounds of the present invention towards cancer M-NFS-60 and non-cancer HEK-CSF1R-STAT5 cell lines.

## Claims

1. A compound or the N-oxide, pharmaceutically acceptable salt, pharmaceutically acceptable solvate, or stereoisomer thereof, wherein said compound is of formulae (IIa) to (IXa) [compounds (C) of class (I) herein after]:

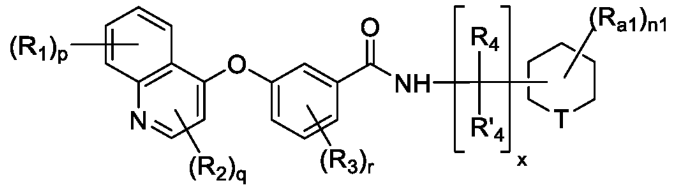

Formula (IIa)

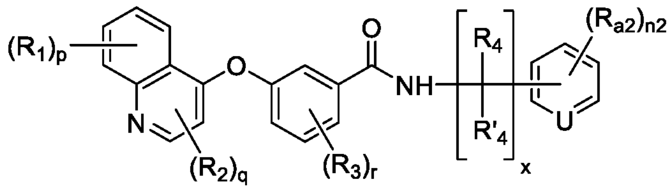

Formula (IIIa)

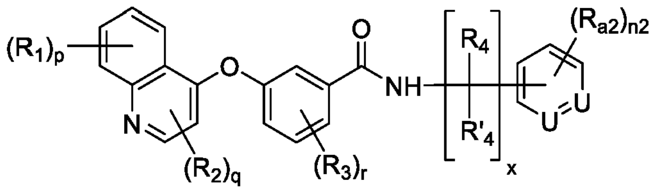

Formula (IVa)

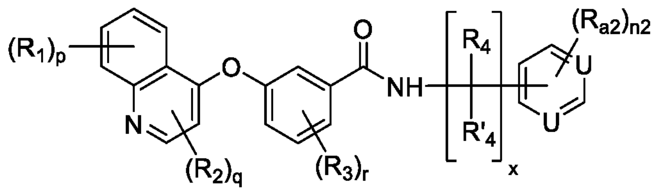

Formula (Va)

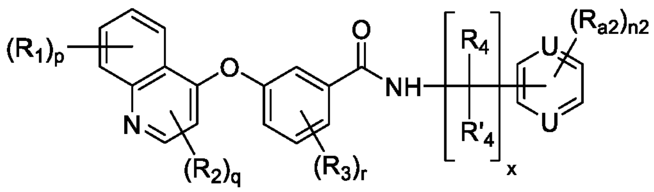

Formula (VIa)

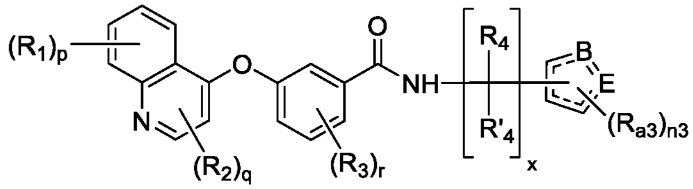

Formula (VIIa)

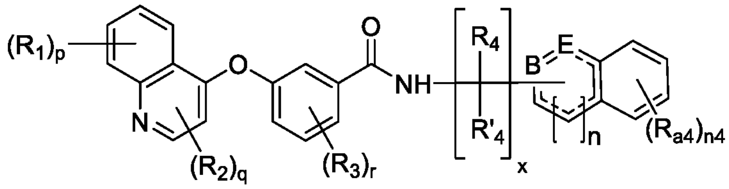

Formula (VIIIa)

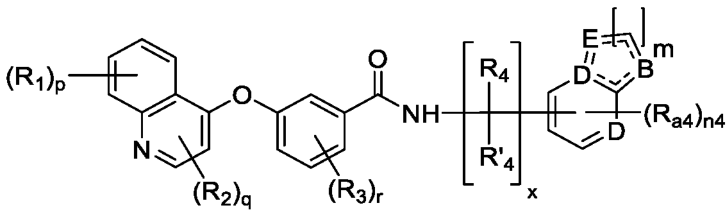

Formula (IXa)

wherein the dash bond represents an optional double bond and wherein:

- each of $R_1$ and $R_2$, independently from each other and at each occurrence, is selected from the group consisting of hydrogen, halo, $C_{1-6}$ alkyl, $C_{2-5}$ alkenyl, $C_{2-5}$ alkynyl, cycloalkyl, heterocyclyl, aryl, and heteroaryl, $CF_3$, CN, $NO_2$, $OR_{21}$, $SR_{21}$, $N(R_{21})_2$, $COR_{21}$, $C(O)OR_{21}$, $CON(R_{21})_2$, $OC(O)R_{21}$, $OCON(R_{21})_2$, $NC(O)R_{21}$, $NCON(R_{21})_2$, $OC(R_{21})_2O$ and $OC(R_{21})_2C(R_{22})_2O$, wherein said alkyl, alkenyl, alkynyl, cycloalkyl, heterocyclyl, aryl, and heteroaryl are optionally substituted with one or more substituents selected from halo, $C_{1-6}$ alkyl, $C_{2-5}$ alkenyl, $C_{2-5}$ alkynyl, cycloalkyl, heterocyclyl, $CF_3$, $COR_{21}$, $CON(R_{21})_2$, $C(O)OR_{21}$, $N(R_{21})_2$, CN, or $OR_{21}$, and each optional alkyl, alkenyl, alkynyl, cycloalkyl, heterocyclyl, aryl or heteroaryl substituent is further optionally substituted with heterocyclyl, $N(R_{11})_2$, or $OR_{11}$; and wherein each of $R_{21}$ and $R_{22}$, independently from each other and at each occurrence, is selected from the group consisting of hydrogen, $C_{1-6}$ alkyl, $C_{2-5}$ alkenyl, $C_{2-5}$ alkynyl, cycloalkyl, heterocyclyl, aryl, heteroaryl, and aralkyl, and wherein said alkyl, alkenyl, alkynyl, cycloalkyl, heterocyclyl, aryl, heteroaryl, and aralkyl substituents are optionally substituted with halo, $C_{1-6}$ alkyl, cycloalkyl, heterocyclyl, aryl, $OR_{31}$ or $N(R_{32})_2$, wherein each of $R_{31}$ and $R_{32}$, independently from each other and at each occurrence, is selected from the group consisting of hydrogen and $C_{1-4}$ alkyl; each of p is an integer in the range from 0 to 4; each of q is an integer in the range from 0 to 2;
- each of $R_3$, independently from each other and at each occurrence, is selected from the group consisting of hydrogen, halo, $C_{1-15}$ alkyl, $C_{2-15}$ alkenyl, $C_{2-15}$ alkynyl, cycloalkyl, heterocyclyl, $CF_3$, CN, $OR_{21}$, $SR_{21}$, $N(R_{21})_2$, $NC(O)R_{21}$, $NCON(R_{21})_2$, $COR_{21}$, $C(O)OR_{21}$, $CON(R_{21})_2$, $OC(O)R_{21}$, $OCON(R_{21})_2$, $OC(R_{21})_2O$, and $OC(R_{21})_2C(R_{22})_2O$, wherein said alkyl, alkenyl, alkynyl, cycloalkyl, and heterocyclyl, are optionally substituted with one or more substituents selected from halo, $C_{1-15}$ alkyl, $CF_3$, $N(R_{21})_2$, CN, or $OR_{21}$; and wherein each of $R_{21}$ and $R_{22}$, independently from each other and at each occurrence, is selected from the group consisting of hydrogen, $C_{1-15}$ alkyl, $C_{2-15}$ alkenyl, $C_{2-15}$ alkynyl, cycloalkyl, heterocyclyl, aryl, heteroaryl, and aralkyl, and wherein said alkyl, alkenyl, alkynyl, cycloalkyl, heterocyclyl, aryl, heteroaryl, and aralkyl substituents are optionally substituted with halo, alkyl, cycloalkyl, heterocyclyl, aryl, $OR_{31}$ or $N(R_{32})_2$, wherein each of $R_{31}$ and $R_{32}$, independently from each other and at each occurrence, is selected from the group consisting of hydrogen and $C_{1-4}$ alkyl; each of r is an integer in the range from 0 to 3;
- each of $R_4$ and $R'_4$, independently from each other and at each occurrence, are selected from the group consisting of hydrogen, $CF_3$, $C_{1-6}$ alkyl, $C_{2-5}$ alkenyl, $C_{2-5}$ alkynyl, cycloalkyl and heterocyclyl, wherein said alkyl, alkenyl, alkynyl, cycloalkyl and heterocyclyl are optionally substituted with a halogen atom, an aryl group, an aralkyl group, $OR_{13}$, $SR_{13}$, $N(R_{13})_2$, $CF_3$ or CN, wherein each of $R_{13}$, independent from each other, is selected from hydrogen, $C_{1-12}$ alkyl, $C_{2-12}$ alkenyl, $C_{2-12}$ alkynyl, cycloalkyl, heterocyclyl, aryl, heteroaryl or aralkyl which are optionally substituted by a halogen atom, an aryl group, an aralkyl group, $OR_{31}$ or $N(R_{32})_2$, wherein each of $R_{31}$ and $R_{32}$, independently from each other and at each occurrence, is selected from the group consisting of hydrogen and $C_{1-4}$ alkyl, and x is an integer in the range from 0 to 7;
- T is selected from $CH_2$, N-R, O or S, wherein R is selected from hydrogen, $C_{1-10}$ alkyl or cycloalkyl which are optionally substituted by a halogen atom, an aryl group or an aralkyl group;
- each of U is selected, independently and at each occurrence, from C-halo, C-R or N; and wherein R is selected from hydrogen, $OR_{11}$, $N(R_{11})_2$, a $C_{1-10}$ alkyl or a cycloalkyl which are optionally substituted by a halogen atom, an aryl group or an aralkyl group, wherein each of $R_{11}$, independently from each other and at each occurrence, is selected from the group consisting of hydrogen and $C_{1-4}$ alkyl;
- each of D is selected, independently and at each occurrence, from C, C-R or N, wherein R is selected from hydrogen, $C_{1-5}$ alkyl or cycloalkyl;
- m is an integer equal to 1 or 2;
- n is an integer equal to 0 or 1;
- each of $R_{a1}$, independently from each other and at each occurrence, is selected from the group consisting of hydrogen, $C_{1-5}$ alkyl, $C_{2-15}$ alkenyl and $C_{2-15}$ alkynyl, wherein said $C_{1-5}$ alkyl, $C_{2-15}$ alkenyl and $C_{2-15}$ alkynyl are optionally substituted with a halogen atom, an aryl group or an aralkyl group; each of n1 is an integer in the range from 0 to 5;

- each of $R_{a2}$, independently from each other and at each occurrence is selected from the group consisting of $C_{1-15}$ alkyl, $C_{2-15}$ alkenyl, $C_{2-15}$ alkynyl, cycloalkyl, heterocyclyl, aryl, heteroaryl, aralkyl, halo, $NO_2$, $CF_3$, CN, $OR_{11}$, $SR_{11}$, $N(R_{11})_2$, $OC(R_{11})_2O$, $OC(R_{11})_2C(R_{11})_2O$, $COOR_{11}$, $CO(R_{11})_2$, $CON(R_{11})_2$, and $SO_2N(R_{11})_2$, and each optional alkyl, alkenyl, alkynyl cycloalkyl, aryl, heterocyclyl, heteroaryl substituent is further optionally substituted with halo, $C_{1-6}$ alkyl, cycloalkyl, aryl, $N(R_{11})_2$, $CF_3$, CN, $COOR_{11}$, $CO(R_{11})_2$, $CON(R_{11})_2$, $SO_2N(R_{11})_2$ or $OR_{11}$ and wherein each of $R_{11}$ , independently from each other and at each occurrence, is selected from the group consisting of hydrogen, $C_{1-6}$ alkyl, cycloalkyl, heterocyclyl, aryl, heteroaryl, and aralkyl, wherein said alkyl, cycloalkyl, heterocyclyl, aryl, heteroaryl, and aralkyl substituents are optionally substituted with halo, $C_{1-6}$ alkyl, cycloalkyl, heterocyclyl, $OR_{31}$ or $N(R_{32})_2$, wherein each of $R_{31}$ and $R_{32}$, independently from each other and at each occurrence, is selected from the group consisting of hydrogen and $C_{1-4}$ alkyl; each of n2 is an integer in the range from 0 to 4;
- each of B, independently from each other and at each occurrence is selected from C-R, O, N, S and $NR_{7'}$, wherein R is selected from hydrogen or an $C_{1-10}$ alkyl which is optionally substituted by a halogen atom, an aryl group or an aralkyl group, wherein $R_{7'}$ is selected from the group consisting of hydrogen, $C_{1-6}$ alkyl, $C_{1-6}$ alkenyl, cycloalkyl, heterocyclyl, aryl, aralkyl and $CF_3$;
- each of E, independently from each other and at each occurrence is selected from C-R, O, N, S and $NR_{7'}$, wherein R is selected from hydrogen or an $C_{1-10}$ alkyl which is optionally substituted by a halogen atom, an aryl group or an aralkyl group, wherein $R_{7'}$ is selected from the group consisting of hydrogen, $C_{1-6}$ alkyl, $C_{1-6}$ alkenyl, cycloalkyl, heterocyclyl, aryl, aralkyl and $CF_3$;
- each of $R_{a4}$, independently from each other and at each occurrence is selected from the group consisting of $C_{1-15}$ alkyl, $C_{2-15}$ alkenyl, $C_{2-15}$ alkynyl, cycloalkyl, heterocyclyl, aryl, heteroaryl, aralkyl, halo, $NO_2$, $CF_3$, CN, $OR_{11}$, $SR_{11}$, $N(R_{11})_2$, $OC(R_{11})_2O$, $OC(R_{11})_2C(R_{11})_2O$, $COOR_{11}$, $CO(R_{11})_2$, and $CON(R_{11})_2$, and each optional alkyl, alkenyl, alkynyl, cycloalkyl, aryl, heterocyclyl, heteroaryl, aralkyl substituent is further optionally substituted with halo, alkyl, cycloalkyl, aryl, $N(R_{11})_2$, CN, or $OR_{11}$ and wherein each of $R_{11}$ , independently from each other and at each occurrence, is selected from the group consisting of hydrogen, $C_{1-6}$ alkyl, cycloalkyl, heterocyclyl, aryl, heteroaryl, and aralkyl, wherein said alkyl, alkenyl, alkynyl, cycloalkyl, heterocyclyl, aryl, heteroaryl, and aralkyl substituents are optionally substituted with halo, alkyl, cycloalkyl, heterocyclyl, $OR_{31}$ or $N(R_{32})_2$, wherein each of $R_{31}$ and $R_{32}$, independently from each other and at each occurrence, is selected from the group consisting of hydrogen and $C_{1-4}$ alkyl; each of n4 is an integer in the range from 0 to 4;
- each of $R_{a3}$, independently from each other and at each occurrence is selected from the group consisting of $C_{1-15}$ alkyl, $C_{2-15}$ alkenyl, $C_{2-15}$ alkynyl, cycloalkyl, heterocyclyl, aryl, heteroaryl, aralkyl, halo, $CF_3$, $OR_{11}$, $SR_{11}$, $N(R_{11})_2$, $COOR_{11}$, $CO(R_{11})_2$, $CON(R_{11})_2$, and each optional alkyl, alkenyl, alkynyl, cycloalkyl, aryl, heterocyclyl, heteroaryl, aralkyl substituent is further optionally substituted with halo, alkyl, cycloalkyl, aryl, $N(R_{11})_2$, CN, or $OR_{11}$ and wherein each of $R_{11}$ , independently from each other and at each occurrence, is selected from the group consisting of hydrogen, $C_{1-6}$ alkyl, cycloalkyl, heterocyclyl, aryl, heteroaryl, and aralkyl, wherein said alkyl, cycloalkyl, heterocyclyl, aryl, heteroaryl, and aralkyl substituents are optionally substituted with halo, alkyl, cycloalkyl, heterocyclyl, $OR_{31}$ or $N(R_{32})_2$, wherein each of $R_{31}$ and $R_{32}$, independently from each other and at each occurrence, is selected from the group consisting of hydrogen and $C_{1-4}$ alkyl; each of n3 is an integer in the range from 0 to 2;

wherein said cycloalkyl is a monocyclic, bicyclic or tricyclic ring system of 3-7 ring members per ring; said heterocyclyl is a saturated, partially saturated or completely saturated monocycle, bicycle or tricycle containing 3 to 12 carbon atoms and 1 or 2 heteroatoms independently selected from O or N; said aryl is phenyl, naphthyl or anthracenyl optionally carbocyclic fused with a cycloalkyl or heterocyclyl of 5-7 ring members; said heteroaryl is a monocyclic ring structure containing 5 or 6 ring atoms, or a bicyclic aromatic group having 8 to 10 atoms, containing 1-3 heteroatoms independently selected from O or N.

**2.** The compound (C) according to general formulae (IIa) to (IXa) and according to claim 1, or the N-oxide, pharmaceutically acceptable salt, pharmaceutically acceptable solvate, or stereoisomer thereof, wherein:

- each of $R_1$ and $R_2$, independently from each other and at each occurrence, is selected from the group consisting of hydrogen, halo, $C_{1-6}$ alkyl, $C_{2-5}$ alkenyl, $C_{2-5}$ alkynyl, cycloalkyl, heterocyclyl, aryl, and heteroaryl, $CF_3$, CN, $NO_2$, $OR_{21}$, $SR_{21}$, $N(R_{21})_2$, $COR_{21}$, $C(O)OR_{21}$, $CON(R_{21})_2$, $OC(O)R_{21}$, $OCON(R_{21})_2$, $NC(O)R_{21}$, $NCON(R_{21})_2$, $OC(R_{21})_2O$ and $OC(R_{21})_2C(R_{22})_2O$, wherein said alkyl, alkenyl, alkynyl, cycloalkyl, heterocyclyl, aryl, and heteroaryl are optionally substituted with one or more substituents selected from halo, $C_{1-6}$ alkyl, cycloalkyl, heterocyclyl, $CF_3$, $COR_{21}$, $N(R_{21})_2$, CN, $CONR_{21}$, $C(O)OR_{21}$ or $OR_{21}$, and each optional alkyl substituent is further optionally substituted with heterocyclyl, $N(R_{11})_2$, or $OR_{11}$ ; and wherein each of $R_{21}$ and $R_{22}$, independently from each other and at each occurrence, is selected from the group consisting of hydrogen, $C_{1-6}$ alkyl, $C_{2-5}$

alkenyl, $C_{2-5}$ alkynyl, cycloalkyl, heterocyclyl, phenyl, heteroaryl, and aralkyl, and wherein said alkyl, alkenyl, alkynyl, cycloalkyl, heterocyclyl, phenyl, heteroaryl, and aralkyl substituents are optionally substituted with halo, $C_{1-6}$ alkyl, cycloalkyl, heterocyclyl, phenyl, $OR_{31}$ or $N(R_{32})_2$, wherein each of $R_{31}$ and $R_{32}$, independently from each other and at each occurrence, is selected from the group consisting of hydrogen and $C_{1-4}$ alkyl; each of p is an integer in the range from 0 to 4; each of q is an integer in the range from 0 to 2;

- each of $R_3$, independently from each other and at each occurrence, is selected from the group consisting of hydrogen, halo, $C_{1-15}$ alkyl, $C_{2-15}$ alkenyl, $C_{2-15}$ alkynyl, cycloalkyl, heterocyclyl, $CF_3$, CN, $OR_{21}$, $SR_{21}$, $N(R_{21})_2$, $NC(O)R_{21}$, $NCON(R_{21})_2$, $COR_{21}$, $C(O)OR_{21}$, $CON(R_{21})_2$, $OC(O)R_{21}$, $OCON(R_{21})_2$, $OC(R_{21})_2O$, and $OC(R_{21})_2C(R_{22})_2O$, wherein said alkyl, alkenyl, alkynyl, cycloalkyl, and heterocyclyl, are optionally substituted with one or more substituents selected from halo, $C_{1-15}$ alkyl, $CF_3$, $N(R_{21})_2$, CN, or $OR_{21}$; and wherein each of $R_{21}$ and $R_{22}$, independently from each other and at each occurrence, is selected from the group consisting of hydrogen, $C_{1-15}$ alkyl, $C_{2-15}$ alkenyl, $C_{2-15}$ alkynyl, cycloalkyl, heterocyclyl, aryl, heteroaryl, and aralkyl, and wherein said alkyl, alkenyl, alkynyl, cycloalkyl, heterocyclyl, aryl, heteroaryl, and aralkyl substituents are optionally substituted with halo, alkyl, cycloalkyl, heterocyclyl, aryl, $OR_{31}$ or $N(R_{32})_2$, wherein each of $R_{31}$ and $R_{32}$, independently from each other and at each occurrence, is selected from the group consisting of hydrogen and $C_{1-4}$ alkyl; each of r is an integer in the range from 0 to 3;
- each of $R_4$ and $R'_4$, independently from each other and at each occurrence, are selected from the group consisting of hydrogen, $C_{1-6}$ alkyl, and $C_{2-6}$ alkenyl, wherein said $C_{1-6}$ alkyl, and $C_{2-6}$ alkenyl are optionally substituted with a halogen atom, and x is an integer in the range from 0 to 7.

3. The compound according to claim 1, or the N-oxide, pharmaceutically acceptable salt, pharmaceutically acceptable solvate, or stereoisomer thereof, wherein said compound is of formulae (IIa-a) to (IXa-a) [compounds (C) of class (I) herein after]:

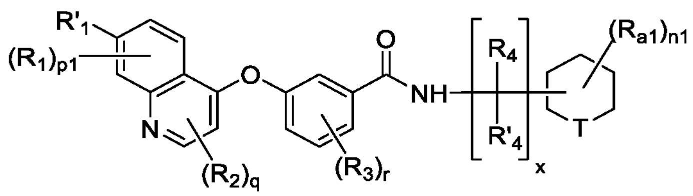

Formula (IIa-a)

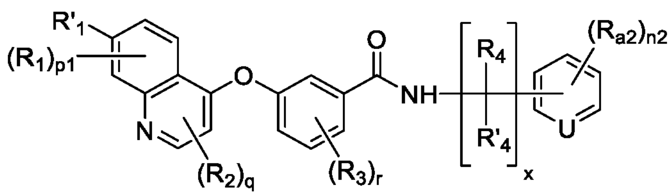

Formula (IIIa-a)

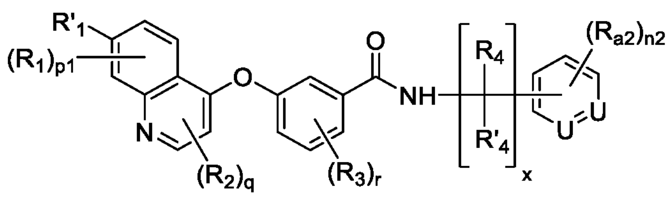

Formula (IVa-a)

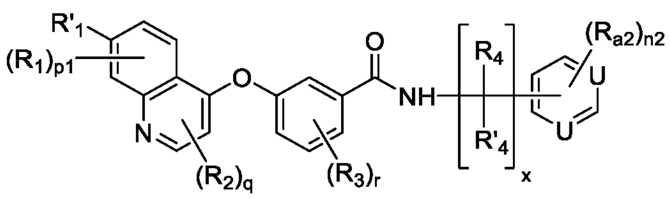

Formula (Va-a)

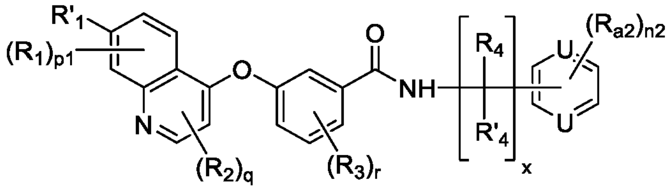

Formula (VIa-a)

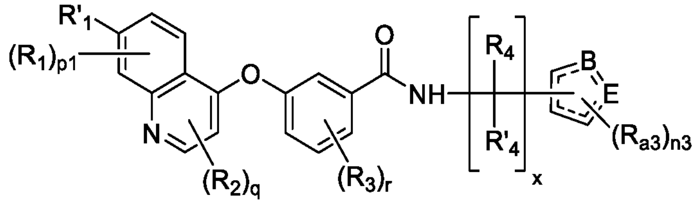

Formula (VIIa-a)

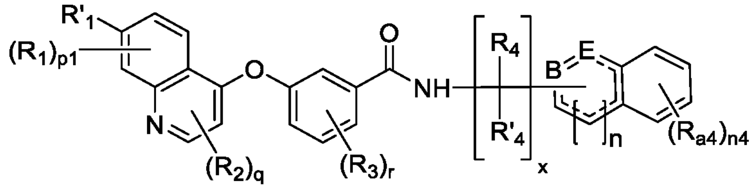

Formula (VIIIa-a)

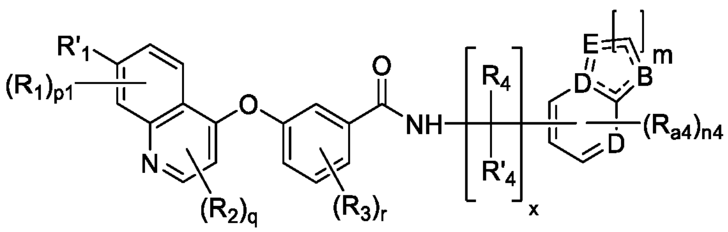

Formula (IXa-a)

wherein $R_1$, $R_2$, $R_3$, $R_4$, $R_4$', $R_{a1}$, $R_{a2}$, $R_{a3}$, $R_{a4}$, T, U, B, E, D, q, r, x, n1, n2, n3, n4, m, n are as defined as in claim 1 and wherein:

- R'$_1$ is selected from the group consisting of (R'$_1$-a) to (R'$_1$-d):

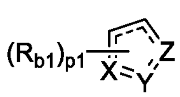

(R'$_1$-a)

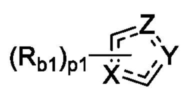

(R'$_1$-b)

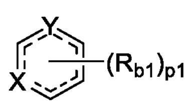

(R'$_1$-c)

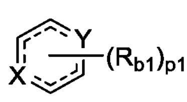

(R'$_1$-d)

wherein:

- each of X, Y and Z independently from each other and at each occurrence is selected from C-R, O, N, S and $NR_{7'}$, wherein R is selected from hydrogen or a $C_{1-6}$ alkyl which is optionally substituted by a halogen atom, an aryl group or an aralkyl group, wherein $R_{7'}$ is selected from the group consisting of hydrogen, $C_{1-6}$ alkyl, $C_{1-6}$ alkenyl, cycloalkyl, heterocyclyl, aryl, aralkyl and $CF_3$, and each alkyl, alkenyl, cycloalkyl, heterocyclyl, aryl or aralkyl substituent is further optionally substituted with heterocyclyl, $N(R_{11})_2$, or $OR_{11}$;
- each of $R_{b1}$, independently and at each occurrence, are selected from the group consisting of hydrogen,

halo, $C_{1-6}$ alkyl, cycloalkyl, heterocyclyl, CN, $CF_3$, $COR_{11}$, $CON(R_{11})_2$, $NR_{11}$ and $OR_{11}$, wherein said alkyl, cycloalkyl and heterocyclyl are optionally substituted with one or more substituents selected from heterocyclyl, $OR_{11}$, $NR_{11}$ and wherein each of $R_{11}$ , independently from each other and at each occurrence, is selected from the group consisting of hydrogen, halo and $C_{1-6}$ alkyl; each of p1 is an integer in the range from 0 to 3;

• the dash bond represents an optional double bond;

wherein said cycloalkyl is a monocyclic, bicyclic or tricyclic ring system of 3-7 ring members per ring; said heterocyclyl is a saturated, partially saturated or completely saturated monocycle, bicycle or tricycle containing 3 to 12 carbon atoms and 1 or 2 heteroatoms independently selected from O or N; said aryl is phenyl, naphthyl or anthracenyl optionally carbocyclic fused with a cycloalkyl or heterocyclyl of 5-7 ring members; said heteroaryl is a monocyclic ring structure containing 5 or 6 ring atoms, or a bicyclic aromatic group having 8 to 10 atoms, containing 1-3 heteroatoms independently selected from O or N.

**4.** The compound (C) according to general formulae (IIa-a) to (IXa-a) and according to claim 3, or the N-oxide, pharmaceutically acceptable salt, pharmaceutically acceptable solvate, or stereoisomer thereof, wherein $R_1$, $R_2$, $R_3$, $R_4$, $R_4$', p, q, r, x, $R_{a1}$, $R_{a2}$, $R_{a3}$, $R_{a4}$, T, U, B, E, D n1, n2, n3, n4, m, n are as defined as in claim 2.

**5.** The compound according to claim 1, or the N-oxide, pharmaceutically acceptable salt, pharmaceutically acceptable solvate, or stereoisomer thereof, wherein said compound is of formulae (IIa-1) to (IXa-1) [compounds (C) of class (I) herein after]:

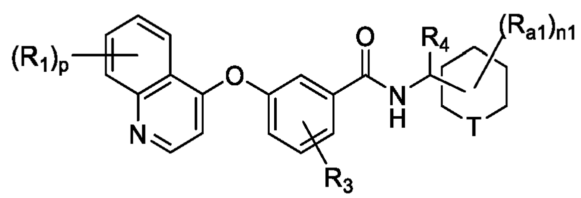

Formula (IIa-1)

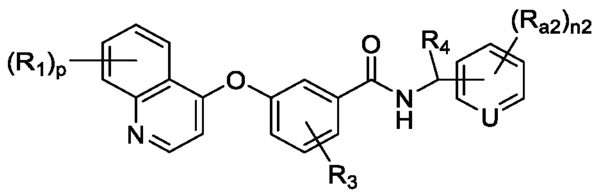

Formula (IIIa-1)

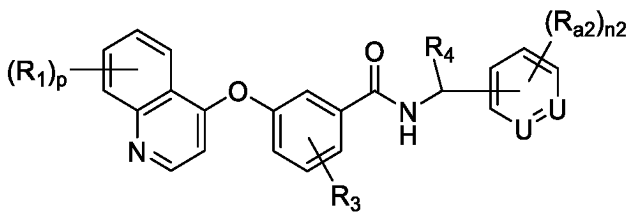

Formula (IVa-1)

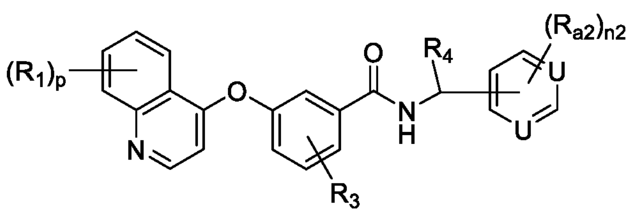

Formula (Va-1)

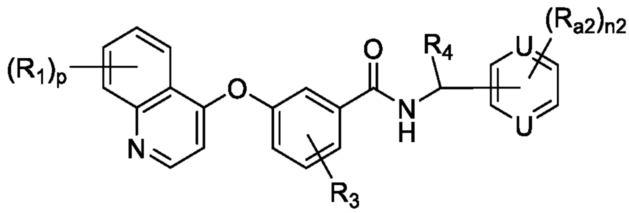

Formula (VIa-1)

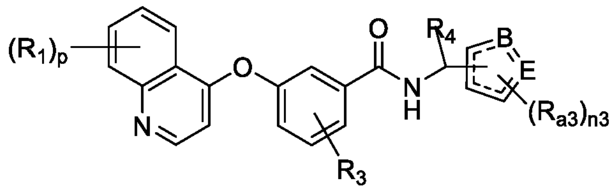

Formula (VIIa-1)

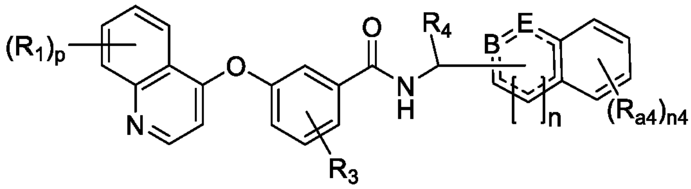

Formula (VIIIa-1)

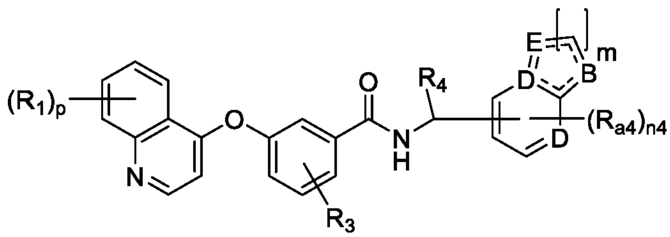

Formula (IXa-1)

wherein $R_1$, $R_{a1}$, $R_{a2}$, $R_{a3}$, $R_{a4}$, T, U, B, E, D, p, n1, n2, n3, n4, m, n are as defined as in claim 1 and the dash bond represents an optional double bond and wherein:

- each of $R_4$, independently from each other and at each occurrence is selected from hydrogen, $CF_3$, $C_{1-4}$ alkyl, or cycloalkyl;
- each of $R_3$, independently from each other and at each occurrence is selected from the group consisting of hydrogen, halo, $CF_3$, $C_{1-4}$ alkyl, cycloalkyl, $OR_{21}$, and $N(R_{21})_2$, and wherein each of $R_{21}$, independently from each other and at each occurrence, is hydrogen, $C_{1-4}$ alkyl such as methyl, ethyl, propyl, isopropyl, butyl, *tert-butyl,* isobutyl or $C_{3-6}$ cycloalkyl such as cyclopropyl, cyclobutyl, cyclopentyl, cyclohexyl.

wherein said cycloalkyl is a monocyclic, bicyclic or tricyclic ring system of 3-7 ring members per ring.

**6.** The compound (C) according to general formulae (IIa-1) to (IXa-1) and according to claim 5, or the N-oxide, pharmaceutically acceptable salt, pharmaceutically acceptable solvate, or stereoisomer thereof, wherein $R_1$, p, $R_{a1}$, $R_{a2}$, $R_{a3}$, $R_{a4}$, T, U, B, E, D, n1, n2, n3, n4, m, n are as defined as in claim 2.

**7.** The compound according to claim 1, or the N-oxide, pharmaceutically acceptable salt, pharmaceutically acceptable solvate, or stereoisomer thereof, wherein said compound is of formulae (IIa-a1) to (IXa-a1) [compounds (C) of class (I) herein after]:

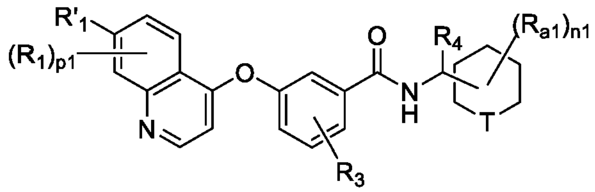

Formula (IIa-a1)

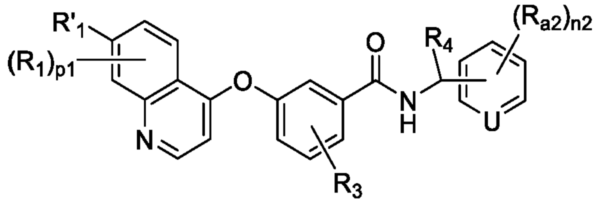

Formula (IIIa-a1)

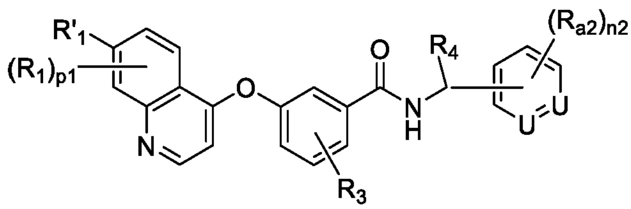

Formula (IVa-a1)

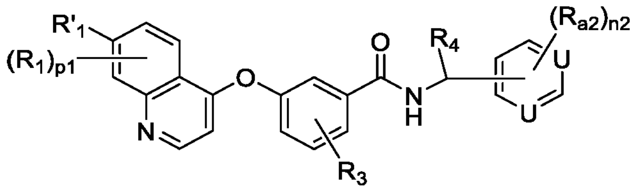

Formula (Va-a1)

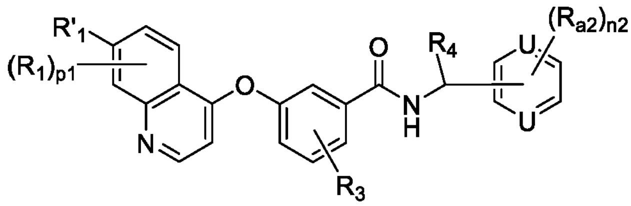

Formula (VIa-a1)

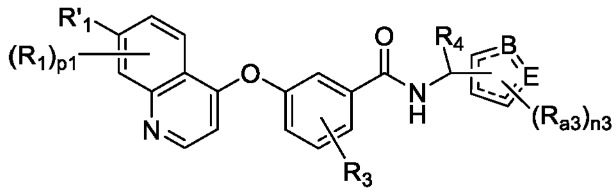

Formula (VIIa-a1)

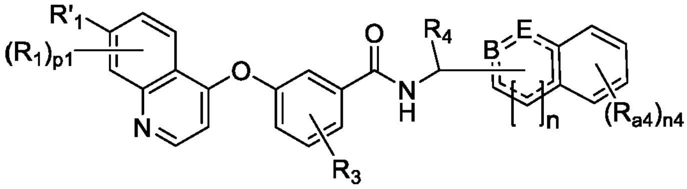

Formula (VIIIa-a1)

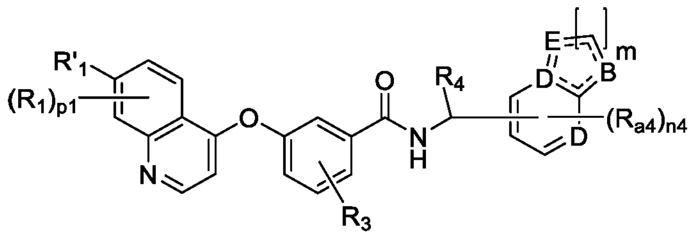

Formula (IXa-a1)

wherein $R_1$, $R'_1$, $R_{a1}$, $R_{a2}$, $R_{a3}$, $R_{a4}$, T, U, B, E, D, p1, n1, n2, n3, n4, m, n are as defined as in claim 3 and the dash bond represents an optional double bond and wherein:

- each of $R_4$, independently from each other and at each occurrence is selected from hydrogen, $CF_3$, $C_{1-4}$ alkyl, or cycloalkyl;
- each of $R_3$, independently from each other and at each occurrence is selected from the group consisting of hydrogen, halo, $CF_3$, $C_{1-4}$ alkyl, cycloalkyl, $OR_{21}$, and $N(R_{21})_2$, and wherein each of $R_{21}$, independently from each other and at each occurrence, is hydrogen, $C_{1-4}$ alkyl such as methyl, ethyl, propyl, isopropyl, butyl, *tert-butyl,* isobutyl or $C_{3-6}$ cycloalkyl such as cyclopropyl, cyclobutyl, cyclopentyl, cyclohexyl;

wherein said cycloalkyl is a monocyclic, bicyclic or tricyclic ring system of 3-7 ring members per ring.

**8.** The compound (C) according to general formulae (IIa-a1) to (IXa-a1) and according to claim 7, or the N-oxide, pharmaceutically acceptable salt, pharmaceutically acceptable solvate, or stereoisomer thereof, wherein $R_1$, $R_{a1}$, $R_{a2}$, $R_{a3}$, $R_{a4}$, T, U, B, E, D, n1, n2, n3, n4, m, n are as defined as in claim 2.

**9.** The compound according to claim 1, or the N-oxide, pharmaceutically acceptable salt, pharmaceutically acceptable solvate, or stereoisomer thereof, wherein said compound is of formulae (Xa-1) to (XVIIa-1) [compounds (C) of class (I) herein after]:

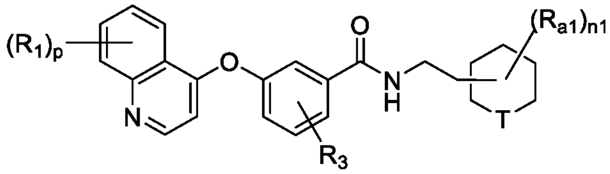

Formula (Xa-1)

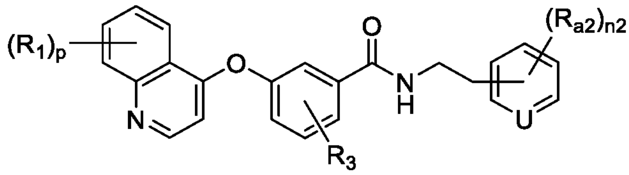

Formula (XIa-1)

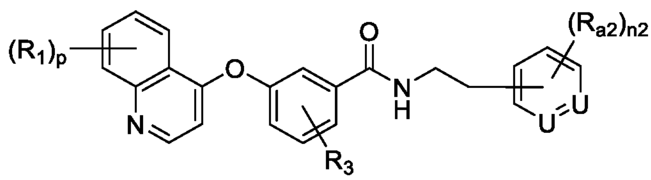

Formula (XIIa-1)

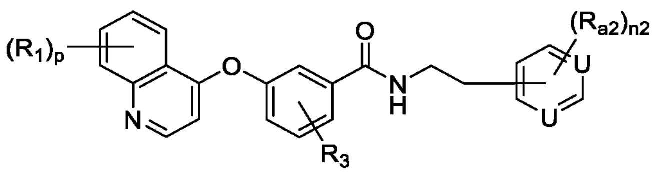

Formula (XIIIa-1)

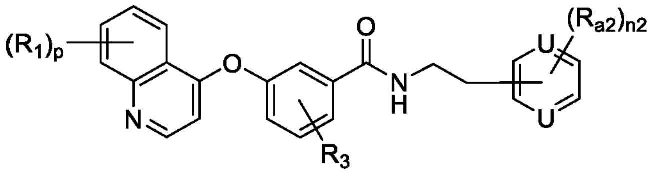

Formula (XIVa-1)

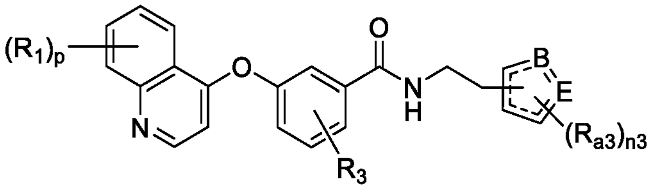

Formula (XVa-1)

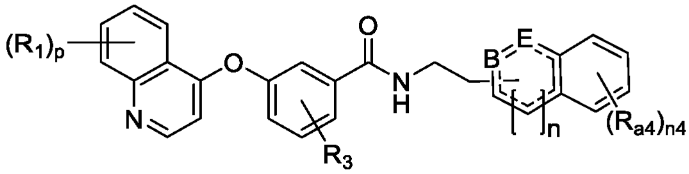

Formula (XVIa-1)

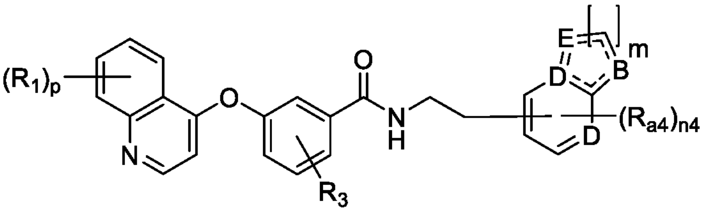

Formula (XVIIa-1)

wherein $R_1$, $R_{a1}$, $R_{a2}$, $R_{a3}$, $R_{a4}$, T, U, B, E, D, p, n1, n2, n3, n4, m, n are as defined as in claim 1 and the dash bond represents an optional double bond and wherein:

- each of $R_3$, independently from each other and at each occurrence is selected from the group consisting of hydrogen, $CF_3$, halo, $C_{1-4}$ alkyl, cycloalkyl, $OR_{21}$, and $N(R_{21})_2$, and wherein each of $R_{21}$, independently from each other and at each occurrence, is hydrogen, $C_{1-4}$ alkyl such as methyl, ethyl, propyl, isopropyl, butyl, *tert*-butyl, isobutyl or $C_{3-6}$ cycloalkyl such as cyclopropyl, cyclobutyl, cyclopentyl, cyclohexyl;

wherein said cycloalkyl is a monocyclic, bicyclic or tricyclic ring system of 3-7 ring members per ring.

**10.** The compound (C) according to general formulae (Xa-1) to (XVIIa-1) and according to claim 9, or the N-oxide, pharmaceutically acceptable salt, pharmaceutically acceptable solvate, or stereoisomer thereof, wherein $R_1$, p, $R_{a1}$, $R_{a2}$, $R_{a3}$, $R_{a4}$, T, U, B, E, D, n1, n2, n3, n4, m, n are as defined as in claim 2.

**11.** The compound according to claim 1, or the N-oxide, pharmaceutically acceptable salt, pharmaceutically acceptable solvate, or stereoisomer thereof, wherein said compound is of formulae (XVIIIa-1) to (XXVa-1) [compounds (C) of class (I) herein after]:

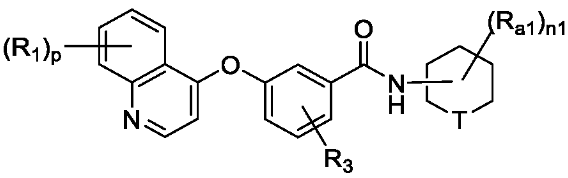

Formula (XVIIIa-1)

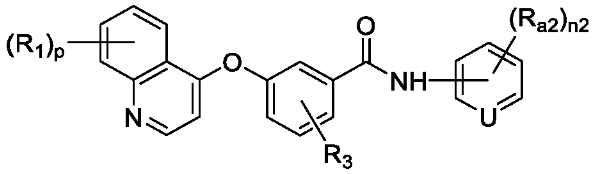

Formula (XIXa-1)

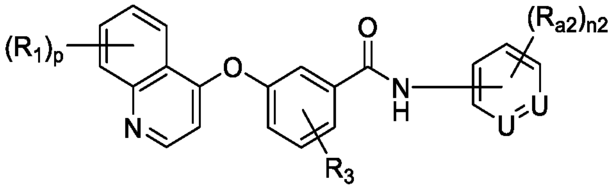

Formula (XXa-1)

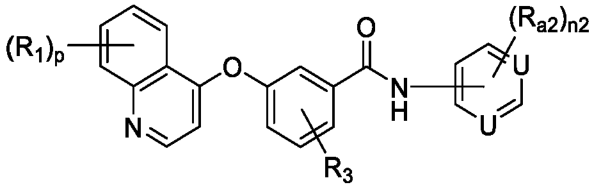

Formula (XXIa-1)

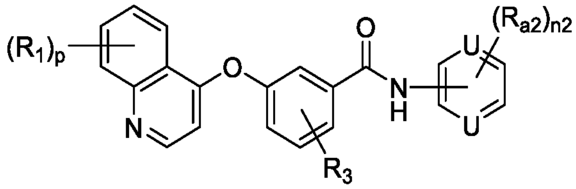

Formula (XXIIa-1)

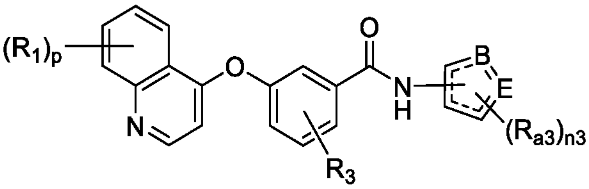

Formula (XXIIIa-1)

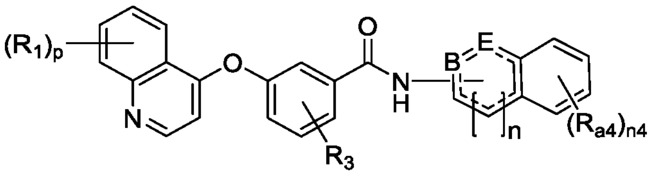

Formula (XXIVa-1)

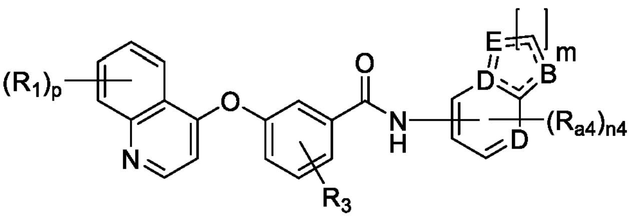

Formula (XXVa-1)

wherein $R_1$, $R_{a1}$, $R_{a2}$, $R_{a3}$, $R_{a4}$, T, U, B, E, D, p, n1, n2, n3, n4, m, n are as defined as in claim 1 and the dash bond represents an optional double bond and wherein

- each of $R_3$, independently from each other and at each occurrence is selected from the group consisting of

hydrogen, $CF_3$, halo, $C_{1-4}$ alkyl, cycloalkyl, $OR_{21}$, and $N(R_{21})_2$, and wherein each of $R_{21}$, independently from each other and at each occurrence, is hydrogen, $C_{1-4}$ alkyl such as methyl, ethyl, propyl, isopropyl, butyl, *tert*-butyl, isobutyl or $C_{3-6}$ cycloalkyl such as cyclopropyl, cyclobutyl, cyclopentyl, cyclohexyl;

wherein said cycloalkyl is a monocyclic, bicyclic or tricyclic ring system of 3-7 ring members per ring.

**12.** The compound (C) according to general formulae (XVIIIa-1) to (XXVa-1) and according to claim 11, or the N-oxide, pharmaceutically acceptable salt, pharmaceutically acceptable solvate, or stereoisomer thereof, wherein $R_1$, p $R_{a1}$, $R_{a2}$, $R_{a3}$, $R_{a4}$, T, U, B, E, D, n1, n2, n3, n4, m, n are as defined as in claim 2.

**13.** The compound (C) according to claim 5 or the N-oxide, pharmaceutically acceptable salt, pharmaceutically acceptable solvate, or stereoisomer thereof, wherein compound of formula (IIIa-1) is a compound according to formula (LXI), (LXIII), (LXXII), (LXXIV)-(LXXXV), (LXXXVI), (CXIII), (CLXXXVI), (CLXXXVIII), (CXC)-(CXCVI), (CXCVII-6)-(CXCVII-7), (CXCVII-16)-(CXCVII-17), (CXCVII-19)-(CXCVII-20), (CXCVII-27), (CXCVII-33)-(CXCVII-35), or (CXCVII-37)-(CXCVII-38) herein below:

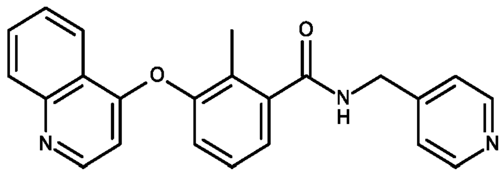

Formula (LXI)

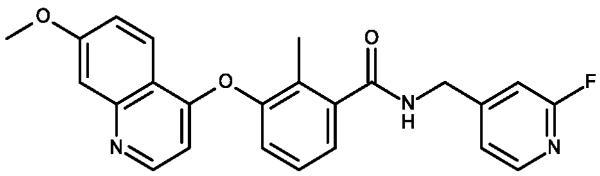

Formula (LXIII)

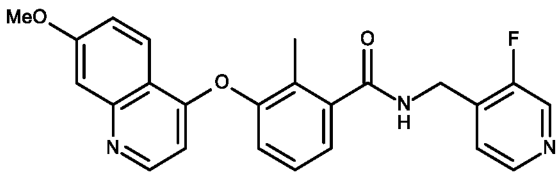

Formula (LXXII)

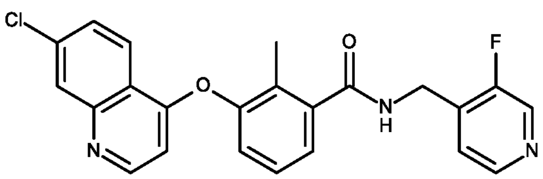

Formula (LXXIV)

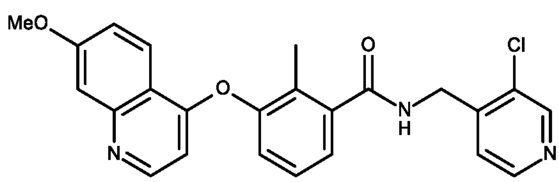

Formula (LXXV)

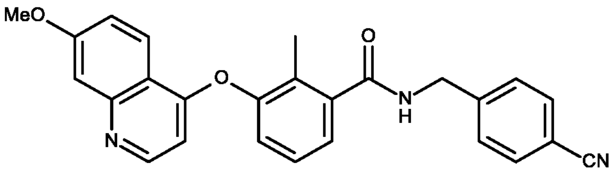

Formula (LXXXVI)

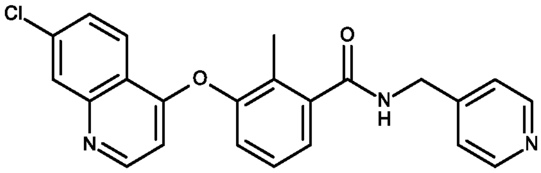

Formula (CXIII)

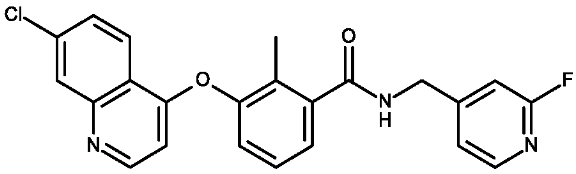

Formula (CLXXXVI)

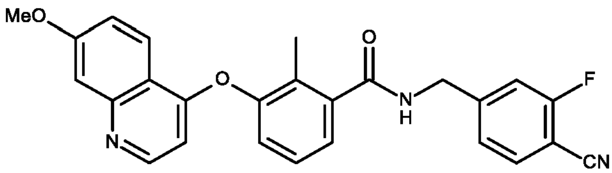

Formula (CLXXXVII)

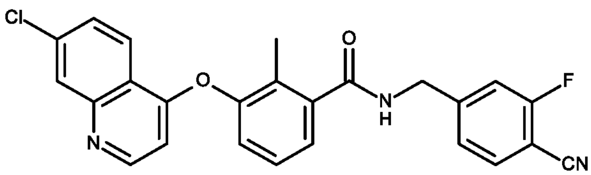

Formula (CLXXXVIII)

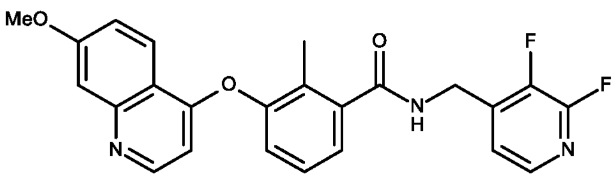

Formula (CXC)

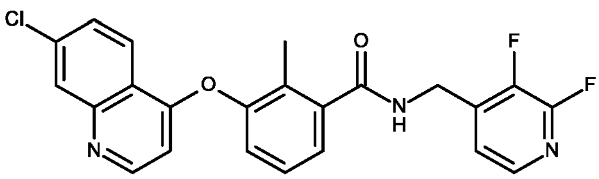

Formula (CXCI)

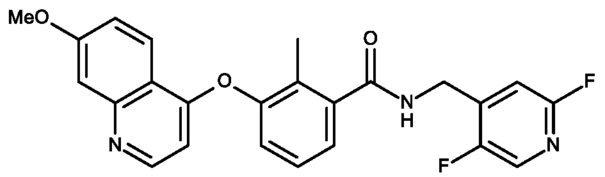

Formula (CXCII)

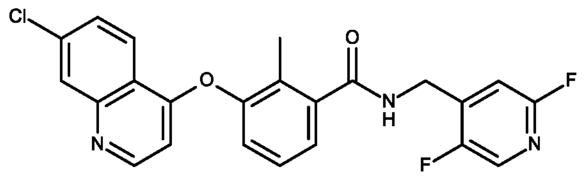

Formula (CXCIII)

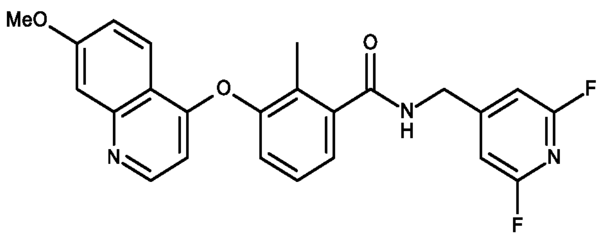

Formula (CXCIV)

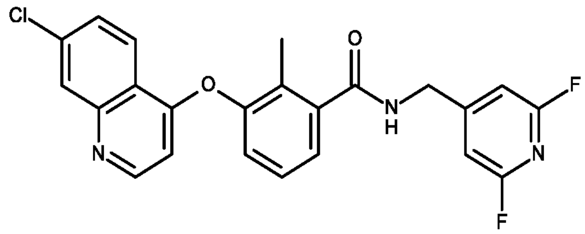

Formula (CXCV)

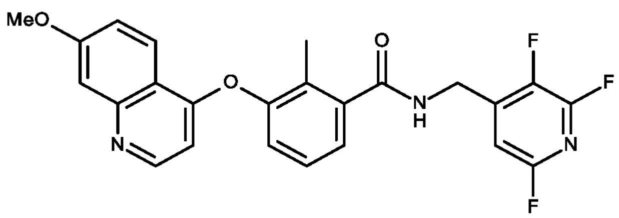

Formula (CXCVI)

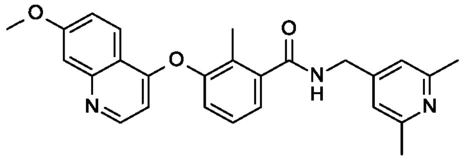

Formula (CXCVIII-6)

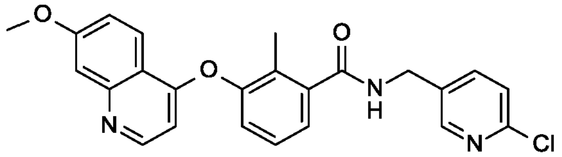

Formula (CXCVIII-7)

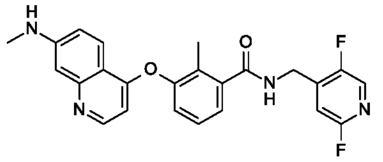

Formula (CXCVIII-16)

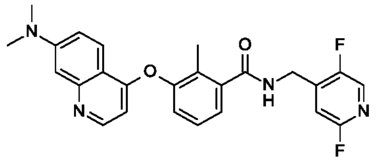

Formula (CXCVIII-17)

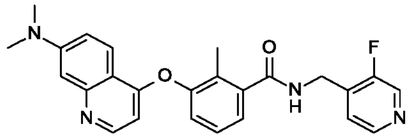

Formula (CXCVIII-19)

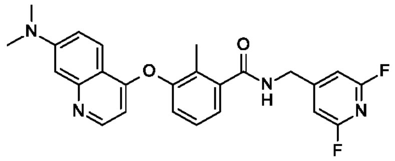

Formula (CXCVIII-20)

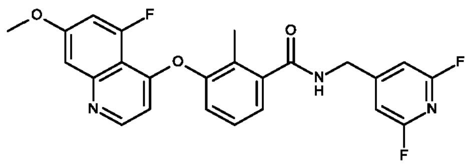

Formula (CXCVIII-27)

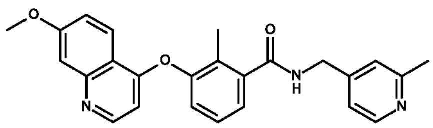

Formula (CXCVIII-33)

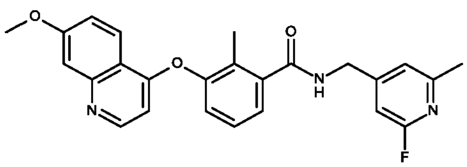

Formula (CXCVIII-34)

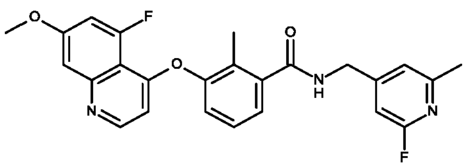

Formula (CXCVIII-35)

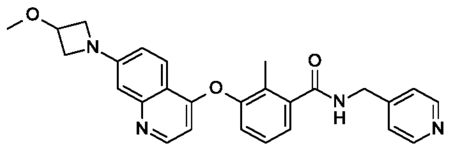

Formula (CXCVIII-37)

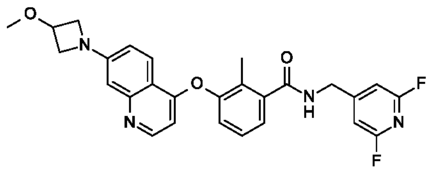

Formula (CXCVIII-38)

**14.** The compound (C) according to claim 7 or the N-oxide, pharmaceutically acceptable salt, pharmaceutically acceptable solvate, or stereoisomer thereof, wherein compound of formula (IIIa-a1) is a compound according to formula (CXCVIII-3), (CXCVIII-7), (CXCVIII-11), (CXCVIII-16), (CXCVIII-19)-(CXCVIII-21), (CXCVIII-23)-(CXCVIII-26), (CXCVIII-28)-(CXCVIII-31), (CXCVIII-33)-(CXCVIII-39), (CXCVIII-42)-(CXCVIII-47), (CXCVIII-49), (CXCVIII-51)-(CXCVIII-53), (CXCVIII-56), (CXCVIII-59), (CXCVIII-62), (CXCVIII-66), (CXCVIII-67), (CXCVIII-70), (CXCVIII-71), (CXCVIII-73)- (CXCVIII-75), (CXCVIII-81)-(CXCVIII-86), or (CXCVIII-88)-(CXCVIII-90) herein below:

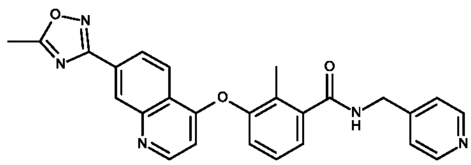

Formula (CXCVIII-3)

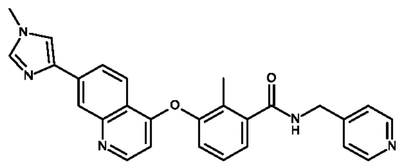

Formula (CXCVIII-7)

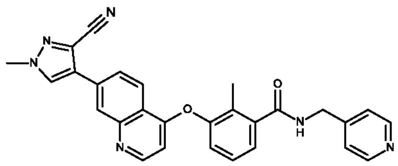

Formula (CXCVIII-11)

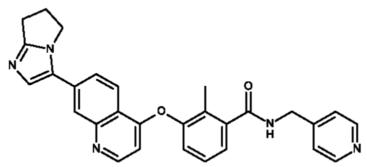

Formula (CXCVIII-16)

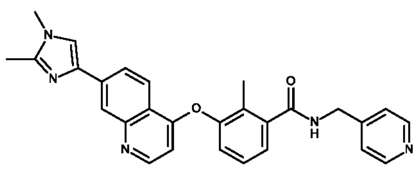

Formula (CXCVIII-19)

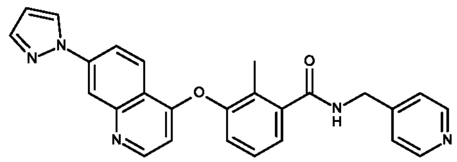

Formula (CXCVIII-20)

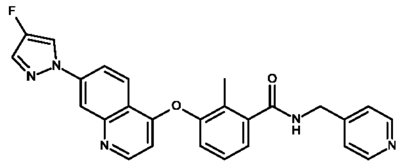

Formula (CXCVIII-21)

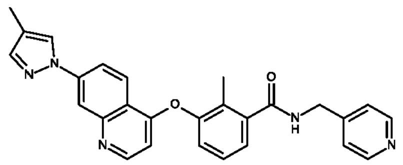

Formula (CXCVIII-23)

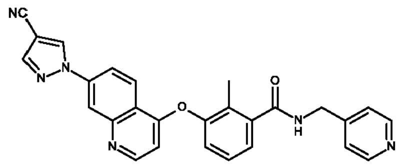

Formula (CXCVIII-24)

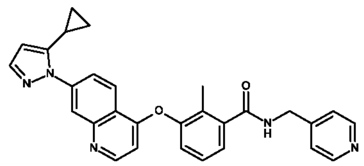

Formula (CXCVIII-25)

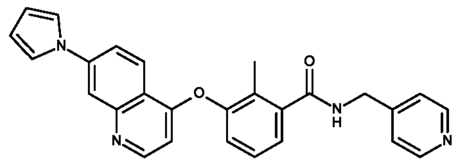

Formula (CXCVIII-26)

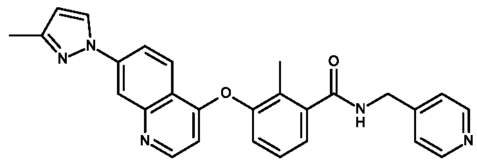

Formula (CXCVIII-28)

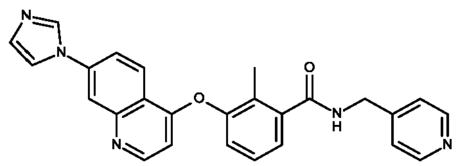

Formula (CXCVIII-29)

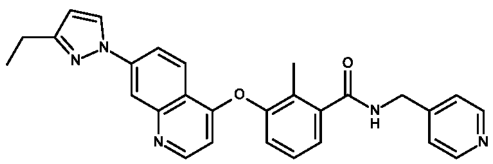

Formula (CXCVIII-30)

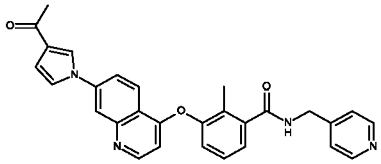

Formula (CXCVIII-31)

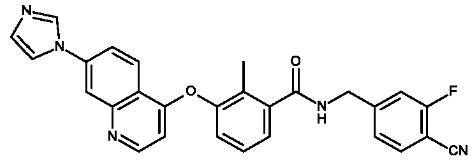

Formula (CXCVIII-33)

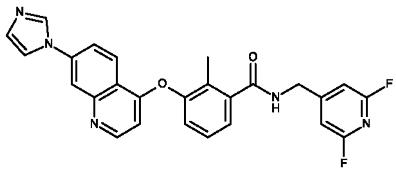

Formula (CXCVIII-34)

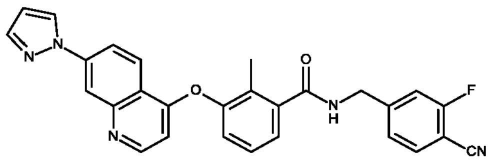

Formula (CXCVIII-35)

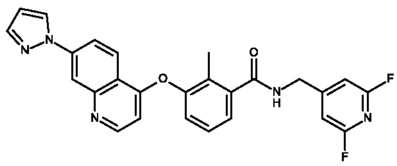

Formula (CXCVIII-36)

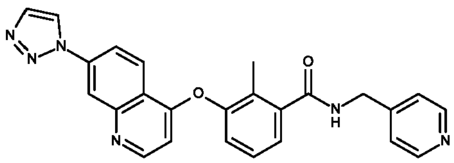

Formula (CXCVIII-37)

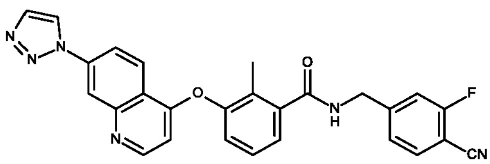

Formula (CXCVIII-38)

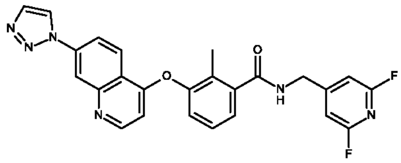

Formula (CXCVIII-39)

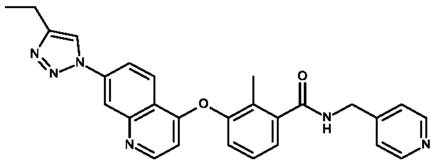

Formula (CXCVIII-42)

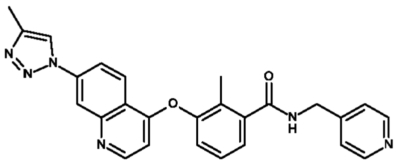

Formula (CXCVIII-43)

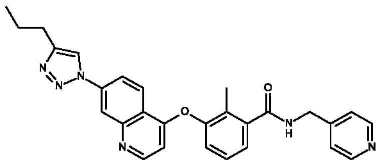

Formula (CXCVIII-44)

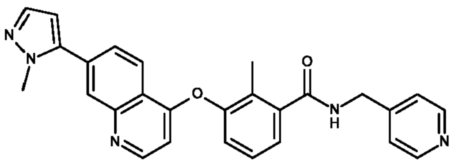

Formula (CXCVIII-45)

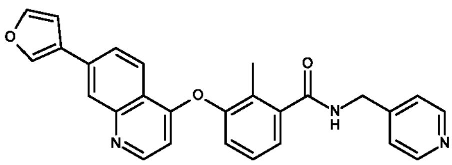

Formula (CXCVIII-46)

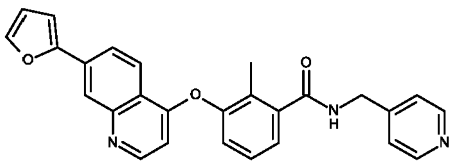

Formula (CXCVIII-47)

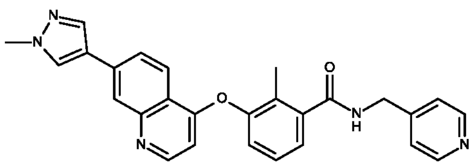

Formula (CXCVIII-49)

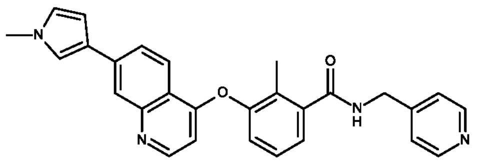

Formula (CXCVIII-51)

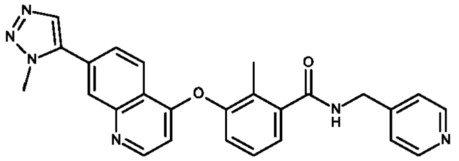

Formula (CXCVIII-52)

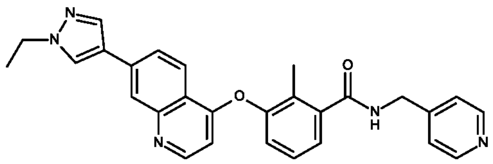

Formula (CXCVIII-53)

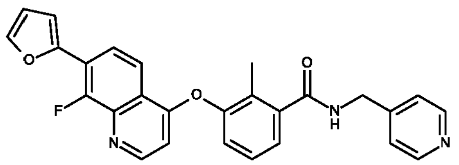

Formula (CXCVIII-56)

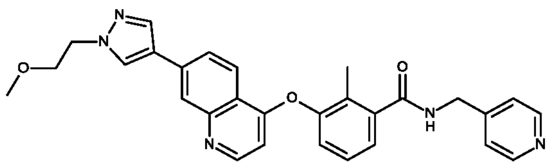

Formula (CXCVIII-59)

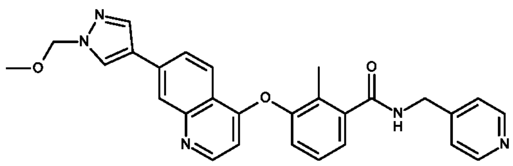

Formula (CXCVIII-62)

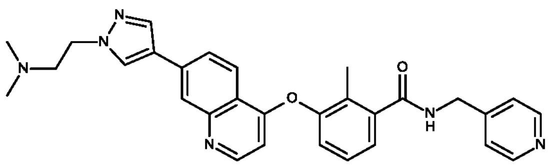

Formula (CXCVIII-66)

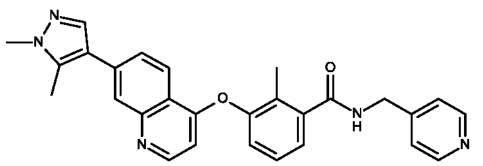

Formula (CXCVIII-67)

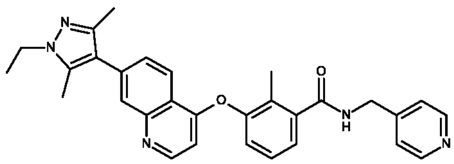

Formula (CXCVIII-70)

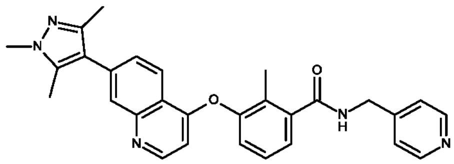

Formula (CXCVIII-71)

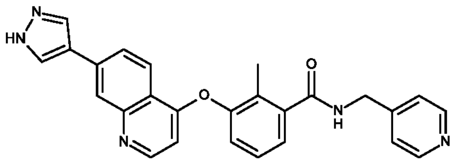

Formula (CXCVIII-73)

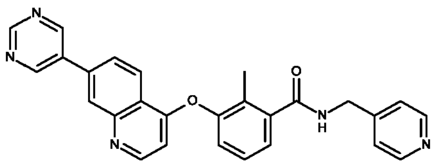

Formula (CXCVIII-74)

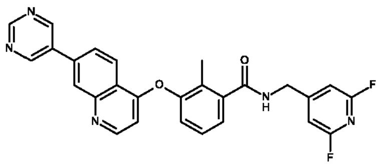

Formula (CXCVIII-75)

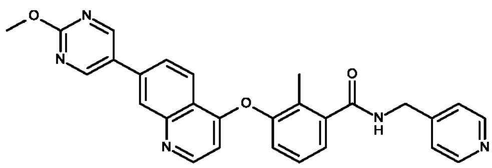

Formula (CXCVIII-81)

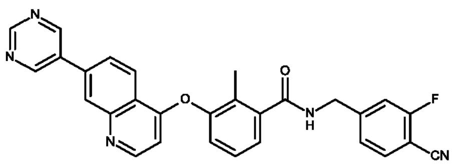

Formula (CXCVIII-82)

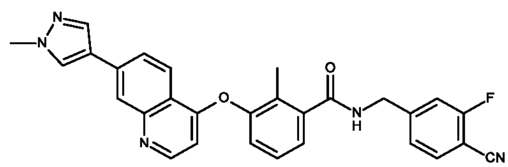

Formula (CXCVIII-83)

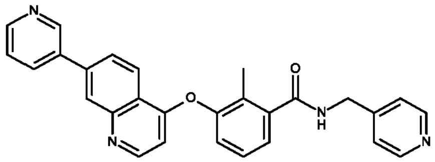

Formula (CXCVIII-84)

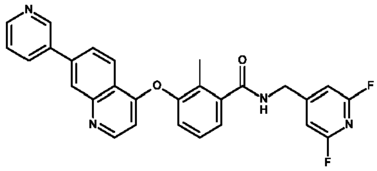

Formula (CXCVIII-85)

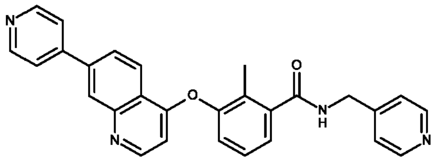

Formula (CXCVIII-86)

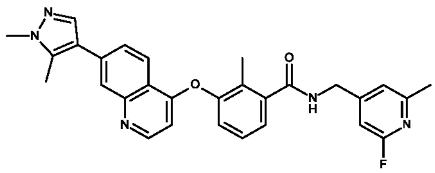

Formula (CXCVIII-88)

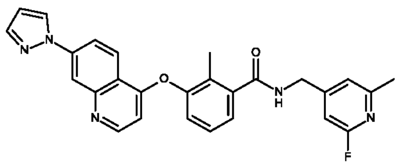

Formula (CXCVIII-89)

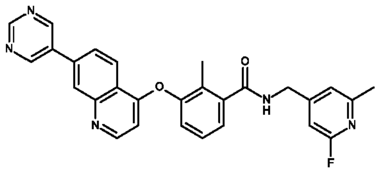

Formula (CXCVIII-90)

**15.** A pharmaceutical composition comprising a carrier, and as active ingredient the compound (C), as defined according to any one of claims 1 to 14.

**16.** A compound (C), as defined according to any one of claims 1 to 14, for use as a medicament.

**17.** A compound as defined according to any one of claims 1 to 14, for use in the treatment of a disease selected from cancer, metabolic disorders, inflammatory and autoimmune disorders, neurological disorders, atherosclerosis and cardiovascular diseases, Sjogren Syndrome, renal allograft rejection, viral induced diseases, circulatory diseases, bone osteolysis and osteoporosis, osteoarthritis, sarcopenia, Langerhans cell histiocytosis, spinal cord injury, endometriosis, asthma and allergic asthma, eye diseases chronic and neuropathic pain, and fibro-proliferative diseases.

**Patentansprüche**

1. Eine Verbindung oder das N-Oxid, pharmazeutisch akzeptables Salz, pharmazeutisch akzeptables Solvat, oder Stereoisomer davon, wobei die Verbindung die Formeln (IIa) bis (IXa) hat [nachstehend Verbindungen (C) der Klasse (I)]:

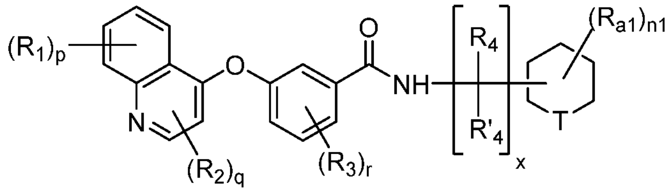

Formel (IIa)

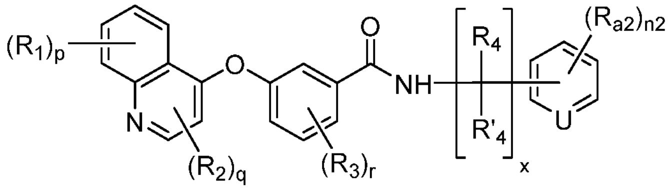

Formel (IIIa)

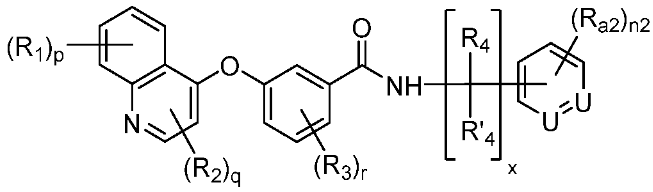

Formel (IVa)

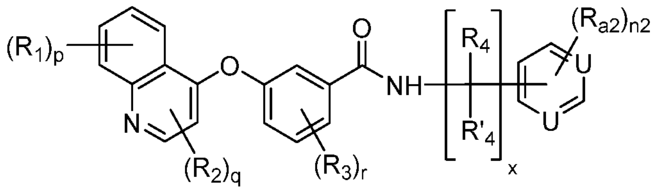

Formel (Va)

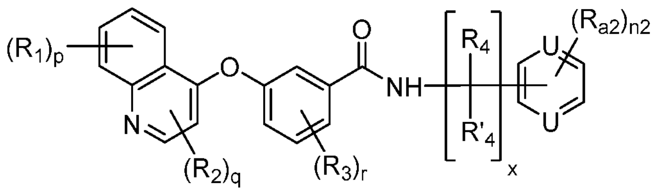

Formel (VIa)

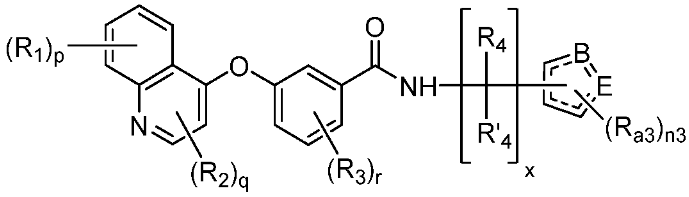

Formel (VIIa)

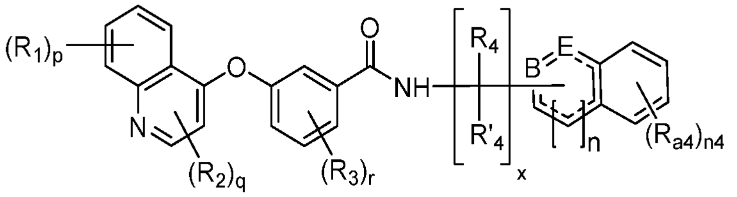

Formel (VIIIa)

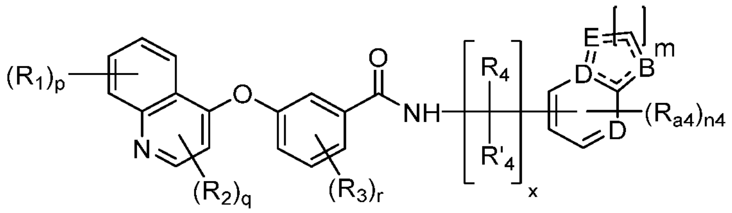

Formel (IXa)

wobei die Strich-Bindung eine optionale Doppelbindung repräsentiert und wobei:

- jedes von $R_1$ und $R_2$, unabhängig voneinander und bei jedem Vorkommen, ausgewählt ist aus der Gruppe bestehend aus Wasserstoff, Halo, $C_{1-6}$-Alkyl, $C_{2-5}$-Alkenyl, $C_{2-5}$-Alkynyl, Cycloalkyl, Heterocyclyl, Aryl, und Heteroaryl, $CF_3$, CN, $NO_2$, $OR_{21}$, $SR_{21}$, $N(R_{21})_2$, $COR_{21}$, $C(O)OR_{21}$, $CON(R_{21})_2$, $OC(O)R_{21}$, $OCON(R_{21})_2$, $NC(O)R_{21}$, $NCON(R_{21})_2$, $OC(R_{21})_2O$ und $OC(R_{21})_2C(R_{22})_2O$, wobei die Alkyl, Alkenyl, Alkynyl, Cycloalkyl, Heterocyclyl, Aryl, und Heteroaryl optional ersetzt sind durch einen oder mehrere Substituenten ausgewählt aus Halo, $C_{1-6}$-Alkyl, $C_{2-5}$-Alkenyl, $C_{2-5}$-Alkynyl, Cycloalkyl, Heterocyclyl, $CF_3$, $COR_{21}$, $CON(R_{21})_2$, $C(O)OR_{21}$, $N(R_{21})_2$, CN, oder $OR_{21}$, und jeder optionale Alkyl-, Alkenyl-, Alkynyl-, Cycloalkyl-, Heterocyclyl-, Aryl- oder Heteroarylsubstituent ferner optional ersetzt ist durch Heterocyclyl, $N(R_{11})_2$, oder $OR_{11}$; und wobei jedes von $R_{21}$ und $R_{22}$, unabhängig voneinander und bei jedem Vorkommen, ausgewählt ist aus der Gruppe bestehend aus Wasserstoff, $C_{1-6}$-Alkyl, $C_{2-5}$-Alkenyl, $C_{2-5}$-Alkynyl, Cycloalkyl, Heterocyclyl, Aryl, Heteroaryl, und Aralkyl, und wobei die Alkyl-, Alkenyl-, Alkynyl-, Cycloalkyl-, Heterocyclyl-, Aryl-, Heteroaryl-, und Aralkylsubstituenten optional ersetzt sind durch Halo, $C_{1-6}$-Alkyl, Cycloalkyl, Heterocyclyl, Aryl, $OR_{31}$ oder $N(R_{32})_2$, wobei jedes von $R_{31}$ und $R_{32}$, unabhängig voneinander und bei jedem Vorkommen, ausgewählt ist aus der Gruppe bestehend aus Wasserstoff und $C_{1-4}$-Alkyl; jedes p eine Ganzzahl im Bereich von 0 bis 4 ist; jedes q eine Ganzzahl im Bereich von 0 bis 2 ist;
- jedes $R_3$, unabhängig voneinander und bei jedem Vorkommen, ausgewählt ist aus der Gruppe bestehend aus Wasserstoff, Halo, $C_{1-15}$-Alkyl, $C_{2-15}$-Alkenyl, $C_{2-15}$-Alkynyl, Cycloalkyl, Heterocyclyl, $CF_3$, CN, $OR_{21}$, $SR_{21}$, $N(R_{21})_2$, $NC(O)R_{21}$, $NCON(R_{21})_2$, $COR_{21}$, $C(O)OR_{21}$, $CON(R_{21})_2$, $OC(O)R_{21}$, $OCON(R_{21})_2$, $OC(R_{21})_2O$, und $OC(R_{21})_2C(R_{22})_2O$, wobei die Alkyl, Alkenyl, Alkynyl, Cycloalkyl, und Heterocyclyl, optional ersetzt sind durch einen oder mehrere Substituenten ausgewählt aus Halo, $C_{1-15}$-Alkyl, $CF_3$, $N(R_{21})_2$, CN, oder $OR_{21}$; und wobei jedes von $R_{21}$ und $R_{22}$, unabhängig voneinander und bei jedem Vorkommen, ausgewählt ist aus der Gruppe bestehend aus Wasserstoff, $C_{1-15}$-Alkyl, $C_{2-15}$-Alkenyl, $C_{2-15}$-Alkynyl, Cycloalkyl, Heterocyclyl, Aryl, Heteroaryl, und Aralkyl, und wobei die Alkyl-, Alkenyl-, Alkynyl-, Cycloalkyl-, Heterocyclyl-, Aryl-, Heteroaryl-, und Aralkylsubstituenten optional ersetzt sind durch Halo, Alkyl, Cycloalkyl, Heterocyclyl, Aryl, $OR_{31}$ oder $N(R_{32})_2$, wobei jedes von $R_{31}$ und $R_{32}$, unabhängig voneinander und bei jedem Vorkommen, ausgewählt ist aus der Gruppe bestehend aus Wasserstoff und $C_{1-4}$-Alkyl; jedes r eine Ganzzahl im Bereich von 0 bis 3 ist;
- jedes von $R_4$ und $R'_4$, unabhängig voneinander und bei jedem Vorkommen, ausgewählt ist aus der Gruppe bestehend aus Wasserstoff, $CF_3$, $C_{1-6}$-Alkyl, $C_{2-5}$-Alkenyl, $C_{2-5}$-Alkynyl, Cycloalkyl und Heterocyclyl, wobei die Alkyl, Alkenyl, Alkynyl, Cycloalkyl und Heterocyclyl optional ersetzt sind durch ein Halogenatom, eine Arylgruppe, eine Aralkylgruppe, $OR_{13}$, $SR_{13}$, $N(R_{13})_2$, $CF_3$ oder CN, wobei jedes $R_{13}$, unabhängig voneinander, ausgewählt ist aus Wasserstoff, $C_{1-12}$-Alkyl, $C_{2-12}$-Alkenyl, $C_{2-12}$-Alkynyl, Cycloalkyl, Heterocyclyl, Aryl, Heteroaryl oder Aralkyl, die optional ersetzt sind durch ein Halogenatom, eine Arylgruppe, eine Aralkylgruppe, $OR_{31}$ oder $N(R_{32})_2$, wobei jedes von $R_{31}$ und $R_{32}$, unabhängig voneinander und bei jedem Vorkommen, ausgewählt ist aus der Gruppe bestehend aus Wasserstoff und $C_{1-4}$-Alkyl, und x eine Ganzzahl im Bereich von 0 bis 7 ist;
- T ausgewählt ist aus $CH_2$, N-R, O oder S, wobei R ausgewählt ist aus Wasserstoff, $C_{1-10}$-Alkyl oder Cycloalkyl, die optional ersetzt sind durch ein Halogenatom, eine Arylgruppe oder eine Aralkylgruppe;
- jedes U ausgewählt ist, unabhängig und bei jedem Vorkommen, aus C-Halo, C-R oder N; und wobei R ausgewählt ist aus Wasserstoff, $OR_{11}$, $N(R_{11})_2$, einem $C_{1-10}$-Alkyl oder einem Cycloalkyl, die optional ersetzt sind durch ein Halogenatom, eine Arylgruppe oder eine Aralkylgruppe, wobei jedes $R_{11}$, unabhängig voneinander und

bei jedem Vorkommen, ausgewählt ist aus der Gruppe bestehend aus Wasserstoff und $C_{1-4}$-Alkyl;
- jedes D ausgewählt ist, unabhängig und bei jedem Vorkommen, aus C, C-R oder N, wobei R ausgewählt ist aus Wasserstoff, $C_{1-5}$-Alkyl oder Cycloalkyl;
- m eine Ganzzahl gleich 1 oder 2 ist;
- n eine Ganzzahl gleich 0 oder 1 ist;
- jedes $R_{a1}$, unabhängig voneinander und bei jedem Vorkommen, ausgewählt ist aus der Gruppe bestehend aus Wasserstoff, $C_{1-5}$-Alkyl, $C_{2-15}$-Alkenyl und $C_{2-15}$-Alkynyl, wobei die $C_{1-5}$-Alkyl, $C_{2-15}$-Alkenyl und $C_{2-15}$-Alkynyl optional ersetzt sind durch ein Halogenatom, eine Arylgruppe oder eine Aralkylgruppe; jedes n1 eine Ganzzahl im Bereich von 0 bis 5 ist;
- jedes $R_{a2}$, unabhängig voneinander und bei jedem Vorkommen, ausgewählt ist aus der Gruppe bestehend aus $C_{1-15}$-Alkyl, $C_{2-15}$-Alkenyl, $C_{2-15}$-Alkynyl, Cycloalkyl, Heterocyclyl, Aryl, Heteroaryl, Aralkyl, Halo, $NO_2$, $CF_3$, CN, $OR_{11}$, $SR_{11}$, $N(R_{11})_2$, $OC(R_{11})_2O$, $OC(R_{11})_2C(R_{11})_2O$, $COOR_{11}$, $CO(R_{11})_2$, $CON(R_{11})_2$, und $SO_2N(R_{11})_2$, und jeder optionale Alkyl-, Alkenyl-, Alkynyl-, Cycloalkyl-, Aryl-, Heterocyclyl-, Heteroarylsubstituent ferner optional ersetzt ist durch Halo, $C_{1-6}$-Alkyl, Cycloalkyl, Aryl, $N(R_{11})_2$, $CF_3$, CN, $COOR_{11}$, $CO(R_{11})_2$, $CON(R_{11})_2$, $SO_2N(R_{11})_2$ oder $OR_{11}$ und wobei jedes $R_{11}$ , unabhängig voneinander und bei jedem Vorkommen, ausgewählt ist aus der Gruppe bestehend aus Wasserstoff, $C_{1-6}$-Alkyl, Cycloalkyl, Heterocyclyl, Aryl, Heteroaryl, und Aralkyl, wobei die Alkyl-, Cycloalkyl-, Heterocyclyl-, Aryl-, Heteroaryl-, und Aralkylsubstituenten optional ersetzt sind durch Halo, $C_{1-6}$-Alkyl, Cycloalkyl, Heterocyclyl, $OR_{31}$ oder $N(R_{32})_2$, wobei jedes von $R_{31}$ und $R_{32}$, unabhängig voneinander und bei jedem Vorkommen, ausgewählt ist aus der Gruppe bestehend aus Wasserstoff und $C_{1-4}$-Alkyl; jedes n2 eine Ganzzahl im Bereich von 0 bis 4 ist;
- jedes B, unabhängig voneinander und bei jedem Vorkommen, ausgewählt ist aus C-R, O, N, S und $NR_{7'}$, wobei R ausgewählt ist aus Wasserstoff oder einem $C_{1-10}$-Alkyl, welches optional ersetzt ist durch ein Halogenatom, eine Arylgruppe oder eine Aralkylgruppe, wobei $R_{7'}$ ausgewählt ist aus der Gruppe bestehend aus Wasserstoff, $C_{1-6}$-Alkyl, $C_{1-6}$-Alkenyl, Cycloalkyl, Heterocyclyl, Aryl, Aralkyl und $CF_3$;
- jedes E, unabhängig voneinander und bei jedem Vorkommen, ausgewählt ist aus C-R, O, N, S und $NR_{7'}$, wobei R ausgewählt ist aus Wasserstoff oder einem $C_{1-10}$-Alkyl, welches optional ersetzt ist durch ein Halogenatom, eine Arylgruppe oder eine Aralkylgruppe, wobei $R_{7'}$ ausgewählt ist aus der Gruppe bestehend aus Wasserstoff, $C_{1-6}$-Alkyl, $C_{1-6}$-Alkenyl, Cycloalkyl, Heterocyclyl, Aryl, Aralkyl und $CF_3$;
- jedes $R_{a4}$, unabhängig voneinander und bei jedem Vorkommen, ausgewählt ist aus der Gruppe bestehend aus $C_{1-15}$-Alkyl, $C_{2-15}$-Alkenyl, $C_{2-15}$-Alkynyl, Cycloalkyl, Heterocyclyl, Aryl, Heteroaryl, Aralkyl, Halo, $NO_2$, $CF_3$, CN, $OR_{11}$, $SR_{11}$, $N(R_{11})_2$, $OC(R_{11})_2O$, $OC(R_{11})_2C(R_{11})_2O$, $COOR_{11}$, $CO(R_{11})_2$, und $CON(R_{11})_2$, und jeder optionale Alkyl-, Alkenyl-, Alkynyl-, Cycloalkyl-, Aryl-, Heterocyclyl-, Heteroaryl-, Aralkylsubstituent ferner optional ersetzt ist durch Halo, Alkyl, Cycloalkyl, Aryl, $N(R_{11})_2$, CN, oder $OR_{11}$ und wobei jedes $R_{11}$ , unabhängig voneinander und bei jedem Vorkommen, ausgewählt ist aus der Gruppe bestehend aus Wasserstoff, $C_{1-6}$-Alkyl, Cycloalkyl, Heterocyclyl, Aryl, Heteroaryl, und Aralkyl, wobei die Alkyl-, Alkenyl-, Alkynyl-, Cycloalkyl-, Heterocyclyl-, Aryl-, Heteroaryl-, und Aralkylsubstituenten optional ersetzt sind durch Halo, Alkyl, Cycloalkyl, Heterocyclyl, $OR_{31}$ oder $N(R_{32})_2$, wobei jedes von $R_{31}$ und $R_{32}$, unabhängig voneinander und bei jedem Vorkommen, ausgewählt ist aus der Gruppe bestehend aus Wasserstoff und $C_{1-4}$-Alkyl; jedes n4 eine Ganzzahl im Bereich von 0 bis 4 ist;
- jedes $R_{a3}$, unabhängig voneinander und bei jedem Vorkommen, ausgewählt ist aus der Gruppe bestehend aus $C_{1-15}$-Alkyl, $C_{2-15}$-Alkenyl, $C_{2-15}$-Alkynyl, Cycloalkyl, Heterocyclyl, Aryl, Heteroaryl, Aralkyl, Halo, $CF_3$, $OR_{11}$, $SR_{11}$, $N(R_{11})_2$, $COOR_{11}$, $CO(R_{11})_2$, $CON(R_{11})_2$, und jeder optionale Alkyl-, Alkenyl-, Alkynyl-, Cycloalkyl-, Aryl-, Heterocyclyl-, Heteroaryl-, Aralkylsubstituent ferner optional ersetzt ist durch Halo, Alkyl, Cycloalkyl, Aryl, $N(R_{11})_2$, CN, oder $OR_{11}$ und wobei jedes $R_{11}$ , unabhängig voneinander und bei jedem Vorkommen, ausgewählt ist aus der Gruppe bestehend aus Wasserstoff, $C_{1-6}$-Alkyl, Cycloalkyl, Heterocyclyl, Aryl, Heteroaryl, und Aralkyl, wobei die Alkyl-, Cycloalkyl-, Heterocyclyl-, Aryl-, Heteroaryl-, und Aralkylsubstituenten optional ersetzt sind durch Halo, Alkyl, Cycloalkyl, Heterocyclyl, $OR_{31}$ oder $N(R_{32})_2$, wobei jedes von $R_{31}$ und $R_{32}$, unabhängig voneinander und bei jedem Vorkommen, ausgewählt ist aus der Gruppe bestehend aus Wasserstoff und $C_{1-4}$-Alkyl; jedes n3 eine Ganzzahl im Bereich von 0 bis 2 ist;

wobei das Cycloalkyl ein monozyklisches, bizyklisches oder trizyklisches Ringsystem aus 3-7 Ringgliedern pro Ring ist; das Heterocyclyl ein gesättigter, teilweise gesättigter oder völlig gesättigter Monocyclus, Bicyclus oder Tricyclus ist, der 3 bis 12 Kohlenstoffatomen und 1 oder 2 Heteroatomen unabhängig ausgewählt aus O oder N enthält; das Aryl Phenyl, Naphthyl oder Anthracenyl optional karbozyklisch fusioniert mit einem Cycloalkyl oder Heterocyclyl mit 5-7 Ringgliedern ist; das Heteroaryl eine monozyklische Ringstruktur mit 5 oder 6 Ringatomen, oder eine bizyklische aromatische Gruppe mit 8 bis 10 Atomen ist, die 1-3 Heteroatome unabhängig ausgewählt aus O oder N enthält.

**2.** Die Verbindung (C) nach den allgemeinen Formeln (IIa) bis (IXa) und nach Anspruch 1, oder das N-Oxid, pharmazeutisch akzeptables Salz, pharmazeutisch akzeptables Solvat, oder Stereoisomer davon, wobei:

- jedes von $R_1$ und $R_2$, unabhängig voneinander und bei jedem Vorkommen, ausgewählt ist aus der Gruppe bestehend aus Wasserstoff, Halo, $C_{1-6}$-Alkyl, $C_{2-5}$-Alkenyl, $C_{2-5}$-Alkynyl, Cycloalkyl, Heterocyclyl, Aryl, und Heteroaryl, $CF_3$, CN, $NO_2$, $OR_{21}$, $SR_{21}$, $N(R_{21})_2$, $COR_{21}$, $C(O)OR_{21}$, $CON(R_{21})_2$, $OC(O)R_{21}$, $OCON(R_{21})_2$, $NC(O)R_{21}$, $NCON(R_{21})_2$, $OC(R_{21})_2O$ und $OC(R_{21})_2C(R_{22})_2O$, wobei die Alkyl, Alkenyl, Alkynyl, Cycloalkyl, Heterocyclyl, Aryl, und Heteroaryl optional ersetzt sind durch einen oder mehrere Substituenten ausgewählt aus Halo, $C_{1-6}$-Alkyl, Cycloalkyl, Heterocyclyl, $CF_3$, $COR_{21}$, $N(R_{21})_2$, CN, $CONR_{21}$, $C(O)OR_{21}$ oder $OR_{21}$, und jeder optionale Alkylsubstituent ferner optional ersetzt ist durch Heterocyclyl, $N(R_{11})_2$, oder $OR_{11}$ ; und wobei jedes von $R_{21}$ und $R_{22}$, unabhängig voneinander und bei jedem Vorkommen, ausgewählt ist aus der Gruppe bestehend aus Wasserstoff, $C_{1-6}$-Alkyl, $C_{2-5}$-Alkenyl, $C_{2-5}$-Alkynyl, Cycloalkyl, Heterocyclyl, Phenyl, Heteroaryl, und Aralkyl, und wobei die Alkyl-, Alkenyl-, Alkynyl-, Cycloalkyl-, Heterocyclyl-, Phenyl-, Heteroaryl-, und Aralkylsubstituenten optional ersetzt sind durch Halo, $C_{1-6}$-Alkyl, Cycloalkyl, Heterocyclyl, Phenyl, $OR_{31}$ oder $N(R_{32})_2$, wobei jedes von $R_{31}$ und $R_{32}$, unabhängig voneinander und bei jedem Vorkommen, ausgewählt ist aus der Gruppe bestehend aus Wasserstoff und $C_{1-4}$-Alkyl; jedes p eine Ganzzahl im Bereich von 0 bis 4 ist; jedes q eine Ganzzahl im Bereich von 0 bis 2 ist;
- jedes $R_3$, unabhängig voneinander und bei jedem Vorkommen, ausgewählt ist aus der Gruppe bestehend aus Wasserstoff, Halo, $C_{1-15}$-Alkyl, $C_{2-15}$-Alkenyl, $C_{2-15}$-Alkynyl, Cycloalkyl, Heterocyclyl, $CF_3$, CN, $OR_{21}$, $SR_{21}$, $N(R_{21})_2$, $NC(O)R_{21}$, $NCON(R_{21})_2$, $COR_{21}$, $C(O)OR_{21}$, $CON(R_{21})_2$, $OC(O)R_{21}$, $OCON(R_{21})_2$, $OC(R_{21})_2O$, und $OC(R_{21})_2C(R_{22})_2O$, wobei die Alkyl, Alkenyl, Alkynyl, Cycloalkyl, und Heterocyclyl, optional ersetzt sind durch einen oder mehrere Substituenten ausgewählt aus Halo, $C_{1-15}$-Alkyl, $CF_3$, $N(R_{21})_2$, CN, oder $OR_{21}$; und wobei jedes von $R_{21}$ und $R_{22}$, unabhängig voneinander und bei jedem Vorkommen, ausgewählt ist aus der Gruppe bestehend aus Wasserstoff, $C_{1-15}$-Alkyl, $C_{2-15}$-Alkenyl, $C_{2-15}$-Alkynyl, Cycloalkyl, Heterocyclyl, Aryl, Heteroaryl, und Aralkyl, und wobei die Alkyl-, Alkenyl-, Alkynyl-, Cycloalkyl-, Heterocyclyl-, Aryl-, Heteroaryl-, und Aralkylsubstituenten optional ersetzt sind durch Halo, Alkyl, Cycloalkyl, Heterocyclyl, Aryl, $OR_{31}$ oder $N(R_{32})_2$, wobei jedes von $R_{31}$ und $R_{32}$, unabhängig voneinander und bei jedem Vorkommen, ausgewählt ist aus der Gruppe bestehend aus Wasserstoff und $C_{1-4}$-Alkyl; jedes r eine Ganzzahl im Bereich von 0 bis 3 ist;
- jedes von $R_4$ und $R'_4$, unabhängig voneinander und bei jedem Vorkommen, ausgewählt ist aus der Gruppe bestehend aus Wasserstoff, $C_{1-6}$-Alkyl, und $C_{2-6}$-Alkenyl, wobei das $C_{1-6}$-Alkyl, und $C_{2-6}$-Akenyl optional ersetzt sind durch ein Halogenatom, und x eine Ganzzahl im Bereich von 0 bis 7 ist.

**3.** Eine Verbindung nach Anspruch 1, oder das N-Oxid, pharmazeutisch akzeptables Salz, pharmazeutisch akzeptables Solvat, oder Stereoisomer davon, wobei die Verbindung die Formeln (IIa-a) bis (IXa-a) hat [nachstehend Verbindungen (C) der Klasse (I)]:

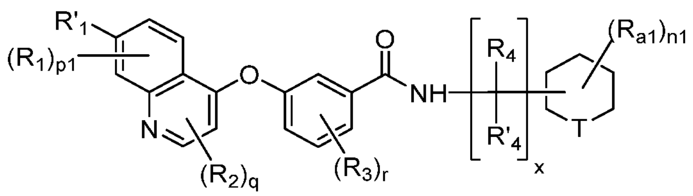

Formel (IIa-a)

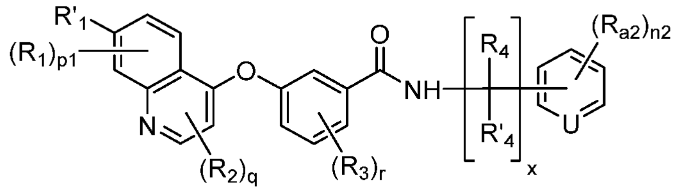

Formel (IIIa-a)

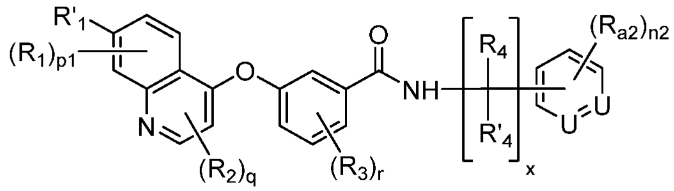

Formel (IVa-a)

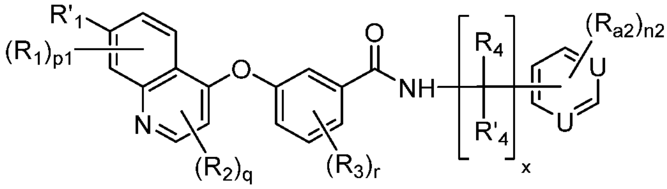

Formel (Va-a)

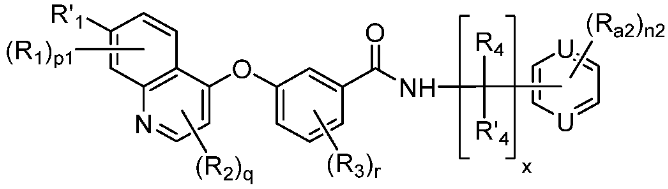

Formel (VIa-a)

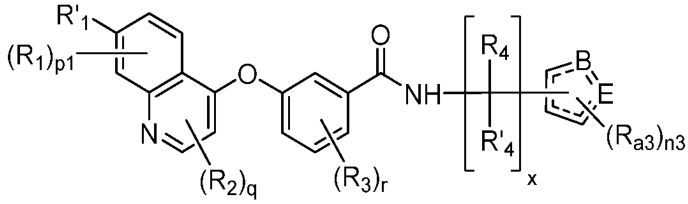

Formel (VIIa-a)

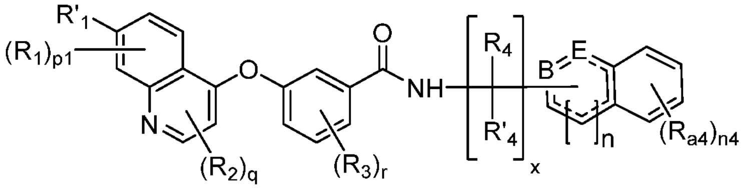

Formel (VIIIa-a)

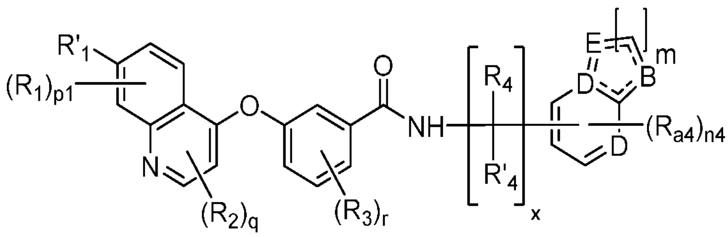

Formel (IXa-a)

wobei $R_1$, $R_2$, $R_3$, $R_4$, $R_4$', $R_{a1}$, $R_{a2}$, $R_{a3}$, $R_{a4}$, T, U, B, E, D, q, r, x, n1, n2, n3, n4, m, n wie in Anspruch 1 definiert sind und wobei:

- $R'_1$ ausgewählt ist aus der Gruppe bestehend aus ($R'_1$-a) bis ($R'_1$-d):

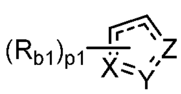

($R'_1$-a)

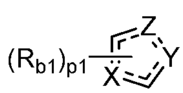

($R'_1$-b)

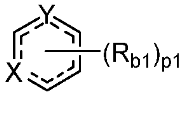

($R'_1$-c)

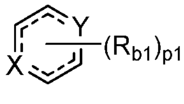

($R'_1$-d)

wobei:

- jedes von X, Y und Z unabhängig voneinander und bei jedem Vorkommen ausgewählt ist aus C-R, O, N, S und $NR_{7'}$, wobei R ausgewählt ist aus Wasserstoff oder einem $C_{1-6}$-Alkyl, welches optional ersetzt ist durch ein Halogenatom, eine Arylgruppe oder eine Aralkylgruppe, wobei $R_{7'}$ ausgewählt ist aus der Gruppe bestehend aus Wasserstoff, $C_{1-6}$-Alkyl, $C_{1-6}$-Alkenyl, Cycloalkyl, Heterocyclyl, Aryl, Aralkyl und $CF_3$, und jeder Alkyl-, Alkenyl-, Cycloalkyl-, Heterocyclyl-, Aryl- oder Aralkylsubstituent ferner optional ersetzt ist durch Heterocyclyl, $N(R_{11})_2$, oder $OR_{11}$;
- jedes $R_{b1}$, unabhängig voneinander und bei jedem Vorkommen, ausgewählt ist aus der Gruppe bestehend aus Wasserstoff, Halo, $C_{1-6}$-Alkyl, Cycloalkyl, Heterocyclyl, CN, $CF_3$, $COR_{11}$, $CON(R_{11})_2$, $NR_{11}$ und $OR_{11}$, wobei die Akyl, Cycloalkyl und Heterocyclyl optional ersetzt sind durch einen oder mehrere Substituenten ausgewählt aus Heterocyclyl, $OR_{11}$, $NR_{11}$ und wobei jedes $R_{11}$ , unabhängig voneinander und bei jedem Vorkommen, ausgewählt ist aus der Gruppe bestehend aus Wasserstoff, Halo und $C_{1-6}$-Alkyl; jedes p1 eine Ganzzahl im Bereich von 0 bis 3 ist;
- die Strich-Bindung eine optionale Doppelbindung repräsentiert;

wobei das Cycloalkyl ein monozyklisches, bizyklisches oder trizyklisches Ringsystem aus 3-7 Ringgliedern pro Ring ist; das Heterocyclyl ein gesättigter, teilweise gesättigter oder völlig gesättigter Monocyclus, Bicyclus oder Tricyclus ist, der 3 bis 12 Kohlenstoffatome und 1 oder 2 Heteroatome unabhängig ausgewählt aus O oder N enthält; das Aryl Phenyl, Naphthyl oder Anthracenyl optional karbozyklisch fusioniert mit einem Cycloalkyl oder Heterocyclyl mit 5-7 Ringgliedern ist; das Heteroaryl eine monozyklische Ringstruktur mit 5 oder 6 Ringatomen, oder eine bizyklische aromatische Gruppe mit 8 bis 10 Atomen ist, die 1-3 Heteroatome unabhängig ausgewählt aus O oder N enthält.

**4.** Die Verbindung (C) nach den allgemeinen Formeln (IIa-a) bis (IXa-a) und nach Anspruch 3, oder das N-Oxid, pharmazeutisch akzeptables Salz, pharmazeutisch akzeptables Solvat, oder Stereoisomer davon, wobei $R_1$, $R_2$, $R_3$, $R_4$, $R_4$', p, q, r, x, $R_{a1}$, $R_{a2}$, $R_{a3}$, $R_{a4}$, T, U, B, E, D n1, n2, n3, n4, m, n wie in Anspruch 2 definiert sind.

**5.** Die Verbindung nach Anspruch 1, oder das N-Oxid, pharmazeutisch akzeptables Salz, pharmazeutisch akzeptables Solvat, oder Stereoisomer davon, wobei die Verbindung die Formeln (IIa-1) bis (IXa-1) hat [nachstehend Verbindungen (C) der Klasse (I)]:

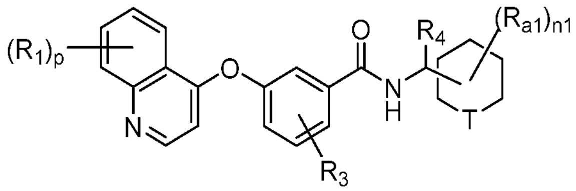

Formel (IIa-1)

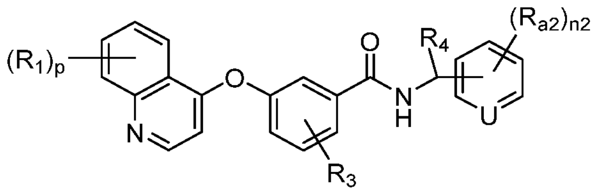

Formel (IIIa-1)

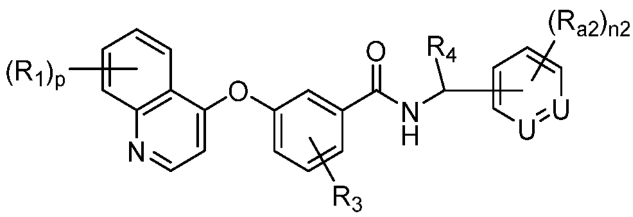

Formel (IVa-1)

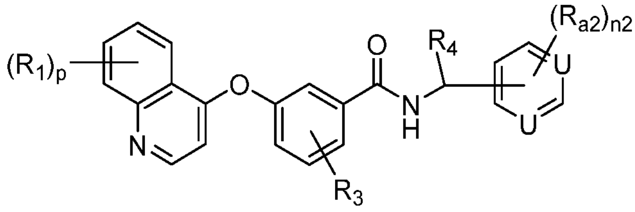

Formel (Va-1)

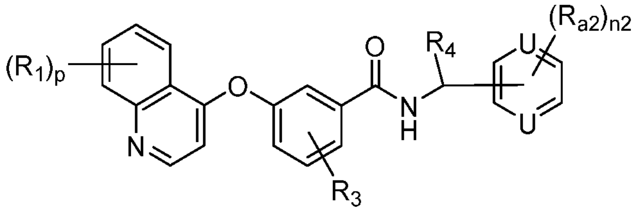

Formel (VIa-1)

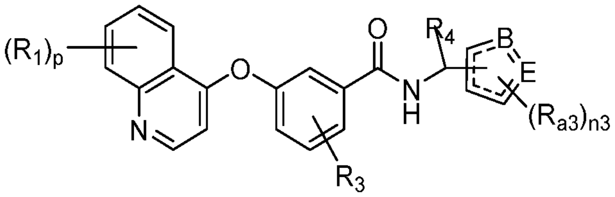

Formel (VIIa-1)

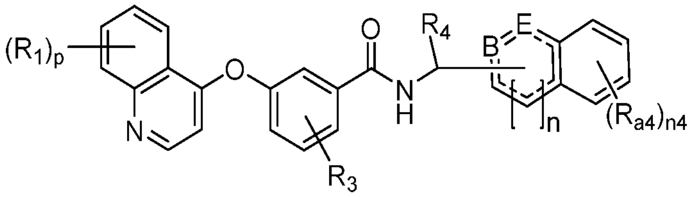

Formel (VIIIa-1)

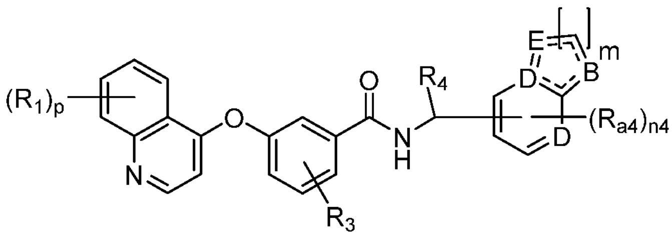

Formel (IXa-1)

wobei $R_1$, $R_{a1}$, $R_{a2}$, $R_{a3}$, $R_{a4}$, T, U, B, E, D, p, n1, n2, n3, n4, m, n wie in Anspruch 1 definiert sind und die Strich-Bindung eine optionale Doppelbindung repräsentiert und wobei:

- jedes $R_4$, unabhängig voneinander und bei jedem Vorkommen, ausgewählt ist aus Wasserstoff, $CF_3$, $C_{1-4}$-Alkyl, oder Cycloalkyl;
- jedes $R_3$, unabhängig voneinander und bei jedem Vorkommen, ausgewählt ist aus der Gruppe bestehend aus Wasserstoff, Halo, $CF_3$, $C_{1-4}$-Alkyl, Cycloalkyl, $OR_{21}$, und $N(R_{21})_2$, und wobei jedes $R_{21}$, unabhängig voneinander und bei jedem Vorkommen, Wasserstoff, $C_{1-4}$-Alkyl wie Methyl, Ethyl, Propyl, Isopropyl, Butyl, tert-Butyl, Isobutyl oder $C_{3-6}$-Cycloalkyl wie Cyclopropyl, Cyclobutyl, Cyclopentyl, Cyclohexyl ist.

wobei das Cycloalkyl ein monozyklisches, bizyklisches oder trizyklisches Ringsystem mit 3-7 Ringgliedern pro Ring ist.

**6.** Die Verbindung (C) nach den allgemeinen Formeln (IIa-1) bis (IXa-1) und nach Anspruch 5, oder das N-Oxid, pharmazeutisch akzeptables Salz, pharmazeutisch akzeptables Solvat, oder Stereoisomer davon, wobei $R_1$, p, $R_{a1}$, $R_{a2}$, $R_{a3}$, $R_{a4}$, T, U, B, E, D, n1, n2, n3, n4, m, n wie in Anspruch 2 definiert sind.

7. Die Verbindung nach Anspruch 1, oder das N-Oxid, pharmazeutisch akzeptables Salz, pharmazeutisch akzeptables Solvat, oder Stereoisomer davon, wobei die Verbindung die Formeln (IIa-a1) bis (IXa-a1) hat [nachstehend Verbindungen (C) der Klasse (I)]:

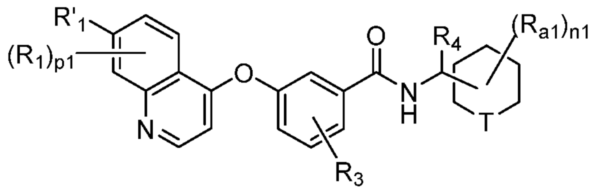

Formel (IIa-a1)

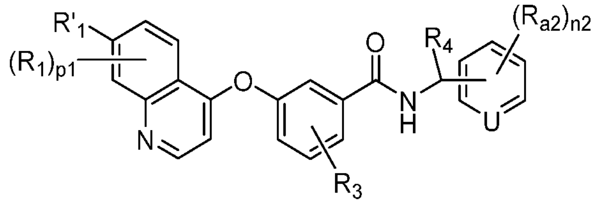

Formel (IIIa-a1)

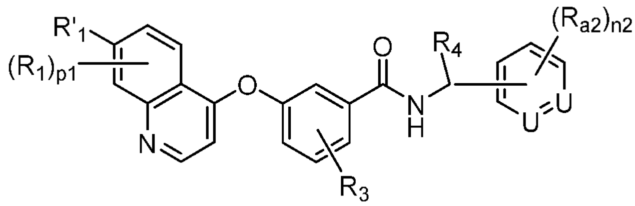

Formel (IVa-a1)

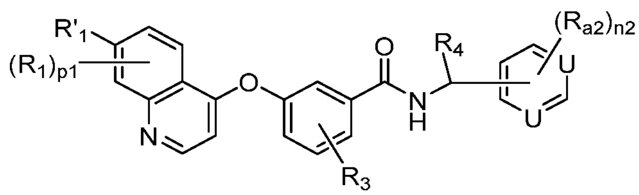

Formel (Va-a1)

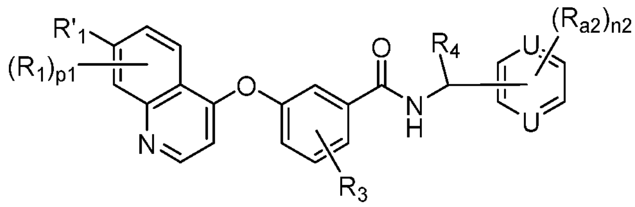

Formel (VIa-a1)

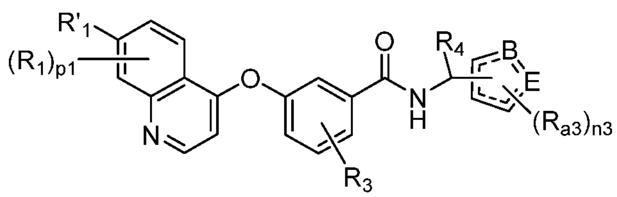

Formel (VIIa-a1)

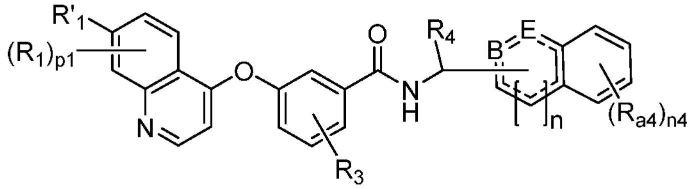

Formel (VIIIa-a1)

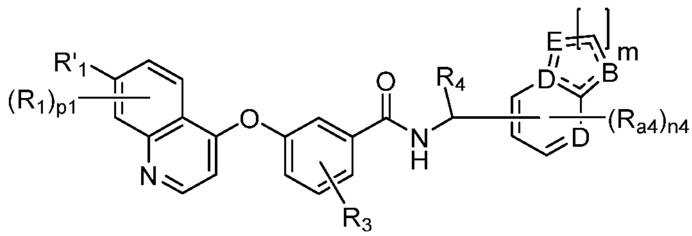

Formel (IXa-a1)

wobei $R_1$, $R'_1$, $R_{a1}$, $R_{a2}$, $R_{a3}$, $R_{a4}$, T, U, B, E, D, p1, n1, n2, n3, n4, m, n wie in Anspruch 3 definiert sind und die Strich-Bindung eine optionale Doppelbindung repräsentiert und wobei:

- jedes $R_4$, unabhängig voneinander und bei jedem Vorkommen, ausgewählt ist aus Wasserstoff, $CF_3$, $C_{1-4}$-Alkyl, oder Cycloalkyl;
- jedes $R_3$, unabhängig voneinander und bei jedem Vorkommen, ausgewählt ist aus der Gruppe bestehend aus Wasserstoff, Halo, $CF_3$, $C_{1-4}$-Alkyl, Cycloalkyl, $OR_{21}$, und $N(R_{21})_2$, und wobei jedes $R_{21}$, unabhängig voneinander und bei jedem Vorkommen, Wasserstoff, $C_{1-4}$-Alkyl wie Methyl, Ethyl, Propyl, Isopropyl, Butyl, tert-Butyl, Isobutyl oder $C_{3-6}$-Cycloalkyl wie Cyclopropyl, Cyclobutyl, Cyclopentyl, Cyclohexyl ist;

wobei das Cycloalkyl ein monozyklisches, bizyklisches oder trizyklisches Ringsystem mit 3-7 Ringgliedern pro Ring ist.

**8.** Die Verbindung (C) nach den allgemeinen Formeln (IIa-a1) bis (IXa-a1) und nach Anspruch 7, oder das N-Oxid, pharmazeutisch akzeptables Salz, pharmazeutisch akzeptables Solvat, oder Stereoisomer davon, wobei $R_1$, $R_{a1}$, $R_{a2}$, $R_{a3}$, $R_{a4}$, T, U, B, E, D, n1, n2, n3, n4, m, n wie in Anspruch 2 definiert sind.

**9.** Die Verbindung nach Anspruch 1, oder das N-Oxid, pharmazeutisch akzeptables Salz, pharmazeutisch akzeptables Solvat, oder Stereoisomer davon, wobei die Verbindung die Formeln (Xa-1) bis (XVIIa-1) hat [nachstehend Verbindungen (C) der Klasse (I)]:

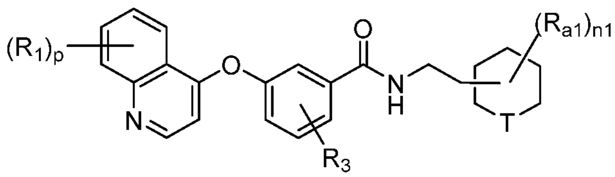

Formel (Xa-1)

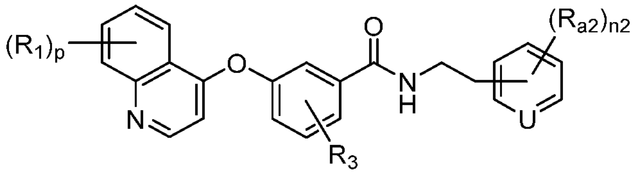

Formel (XIa-1)

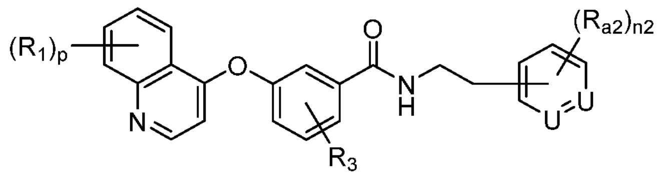

Formel (XIIa-1)

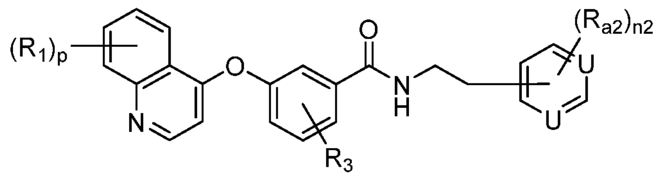

Formel (XIIIa-1)

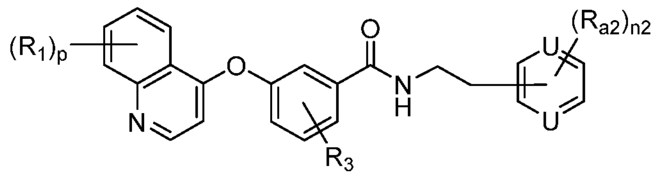

Formel (XIVa-1)

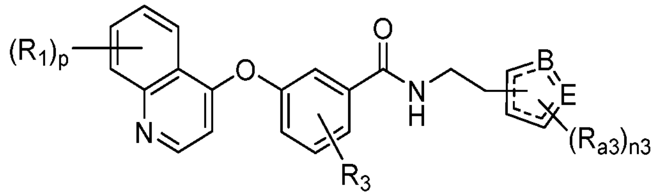

Formel (XVa-1)

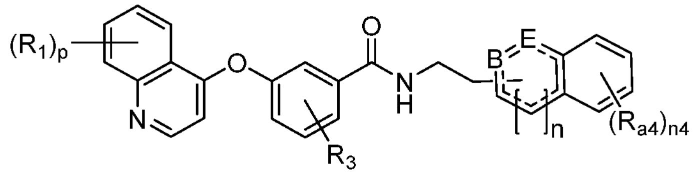

Formel (XVIa-1)

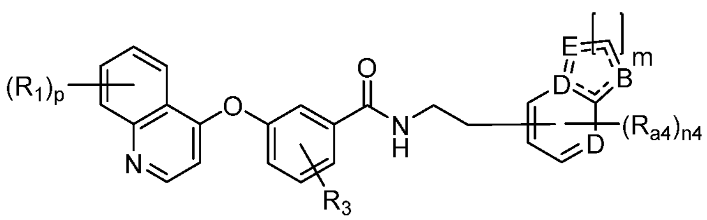

Formel (XVIIa-1)

wobei $R_1$, $R_{a1}$, $R_{a2}$, $R_{a3}$, $R_{a4}$, T, U, B, E, D, p, n1, n2, n3, n4, m, n wie in Anspruch 1 definiert sind und die Strich-Bindung eine optionale Doppelbindung repräsentiert und wobei:

- jedes $R_3$, unabhängig voneinander und bei jedem Vorkommen, ausgewählt ist aus der Gruppe bestehend aus Wasserstoff, $CF_3$, Halo, $C_{1-4}$-Alkyl, Cycloalkyl, $OR_{21}$, und $N(R_{21})_2$, und wobei jedes $R_{21}$, unabhängig voneinander und bei jedem Vorkommen, Wasserstoff, $C_{1-4}$-Alkyl wie Methyl, Ethyl, Propyl, Isopropyl, Butyl, tert-Butyl, Isobutyl oder $C_{3-6}$-Cycloalkyl wie Cyclopropyl, Cyclobutyl, Cyclopentyl, Cyclohexyl ist;

wobei das Cycloalkyl ein monozyklisches, bizyklisches oder trizyklisches Ringsystem mit 3-7 Ringgliedern pro Ring ist.

**10.** Die Verbindung (C) nach den allgemeinen Formeln (Xa-1) bis (XVIIa-1) und nach Anspruch 9, oder das N-Oxid,

pharmazeutisch akzeptables Salz, pharmazeutisch akzeptables Solvat, oder Stereoisomer davon, wobei $R_1$, p, $R_{a1}$, $R_{a2}$, $R_{a3}$, $R_{a4}$, T, U, B, E, D, n1, n2, n3, n4, m, n wie in Anspruch 2 definiert sind.

**11.** Die Verbindung nach Anspruch 1, oder das N-Oxid, pharmazeutisch akzeptables Salz, pharmazeutisch akzeptables Solvat, oder Stereoisomer davon, wobei die Verbindung die Formeln (XVIIIa-1) bis (XXVa-1) hat [nachstehend Verbindungen (C) der Klasse (I)]:

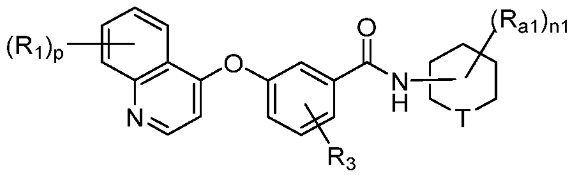

Formel (XVIIIa-1)

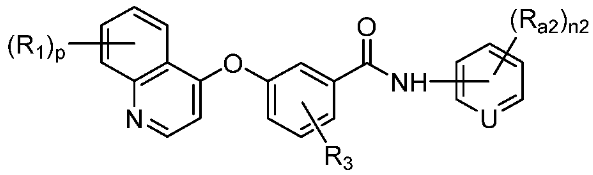

Formel (XIXa-1)

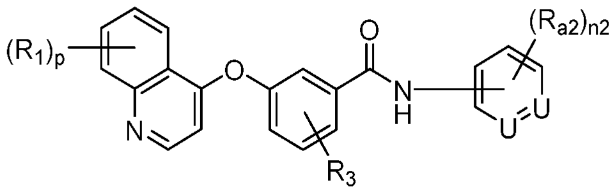

Formel (XXa-1)

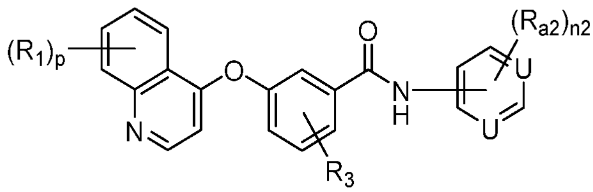

Formel (XXIa-1)

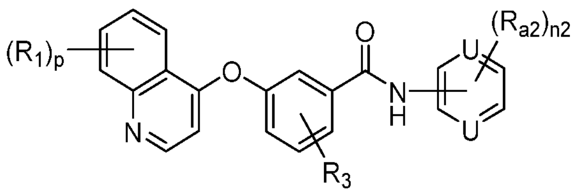

Formel (XXIIa-1)

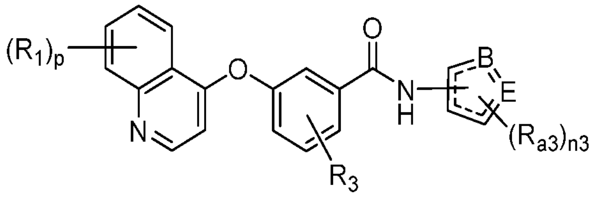

Formel (XXIIIa-1)

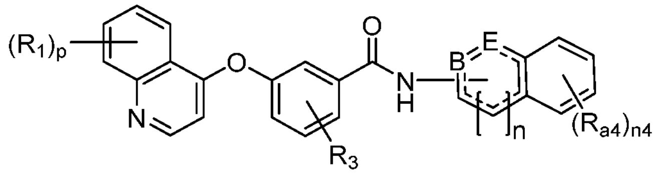

Formel (XXIVa-1)

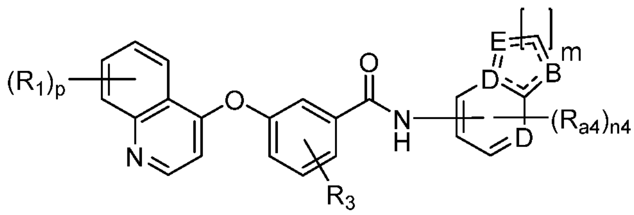

Formel (XXVa-1)

wobei $R_1$, $R_{a1}$, $R_{a2}$, $R_{a3}$, $R_{a4}$, T, U, B, E, D, p, n1, n2, n3, n4, m, n wie definiert in Anspruch 1 sind und die Strich-Bindung eine optionale Doppelbindung repräsentiert und wobei

- jedes $R_3$, unabhängig voneinander und bei jedem Vorkommen, ausgewählt ist aus der Gruppe bestehend aus Wasserstoff, $CF_3$, Halo, $C_{1-4}$-Alkyl, Cycloalkyl, $OR_{21}$, und $N(R_{21})_2$, und wobei jedes $R_{21}$, unabhängig voneinander und bei jedem Vorkommen, Wasserstoff, $C_{1-4}$-Alkyl wie Methyl, Ethyl, Propyl, Isopropyl, Butyl, tert-Butyl, Isobutyl oder $C_{3-6}$-Cycloalkyl wie Cyclopropyl, Cyclobutyl, Cyclopentyl, Cyclohexyl ist;

wobei das Cycloalkyl ein monozyklisches, bizyklisches oder trizyklisches Ringsystem mit 3-7 Ringgliedern pro Ring ist.

**12.** Die Verbindung (C) nach den allgemeinen Formeln (XVIIIa-1) bis (XXVa-1) und nach Anspruch 11, oder das N-Oxid, pharmazeutisch akzeptables Salz, pharmazeutisch akzeptables Solvat, oder Stereoisomer davon, wobei $R_1$, p, $R_{a1}$, $R_{a2}$, $R_{a3}$, $R_{a4}$, T, U, B, E, D, n1, n2, n3, n4, m, n wie in Anspruch 2 definiert sind.

**13.** Die Verbindung (C) nach Anspruch 5 oder das N-Oxid, pharmazeutisch akzeptables Salz, pharmazeutisch akzeptables Solvat, oder Stereoisomer davon, wobei die Verbindung der Formel (IIIa-1) eine Verbindung nach Formel (LXI), (LXIII), (LXXII), (LXXIV)-(LXXXV), (LXXXVI), (CXIII), (CLXXXVI), (CLXXXVIII), (CXC)-(CXCVI), (CXCVII-6)-(CXCVII-7), (CXCVII-16)-(CXCVII-17), (CXCVII-19)-(CXCVII-20), (CXCVII-27), (CXCVII-33)-(CXCVII-35), oder (CXCVII-37)-(CXCVII-38) wie hier unten ist:

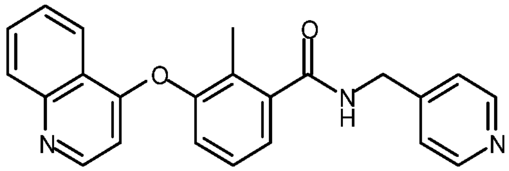

Formel (LXI)

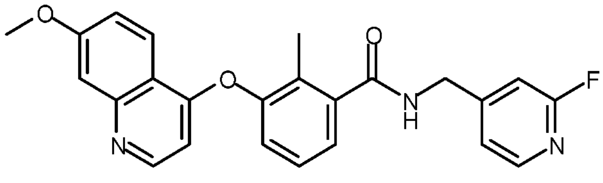

Formel (LXIII)

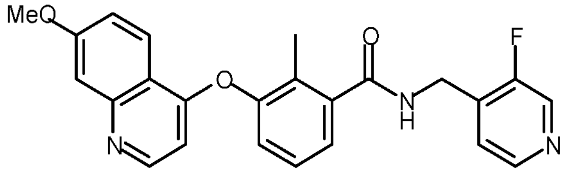

Formel (LXXII)

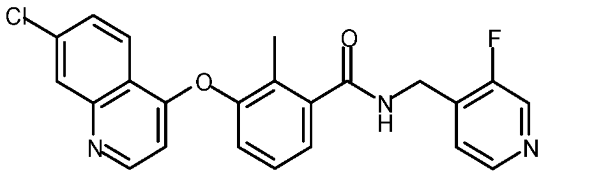

Formel (LXXIV)

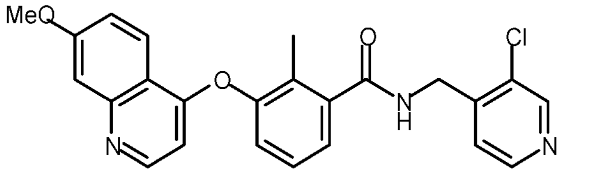

Formel (LXXV)

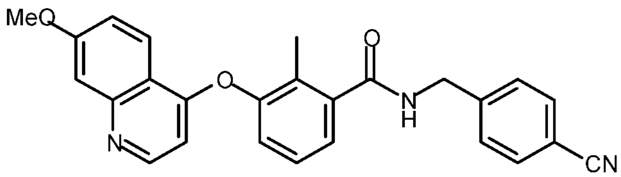

Formel (LXXXVI)

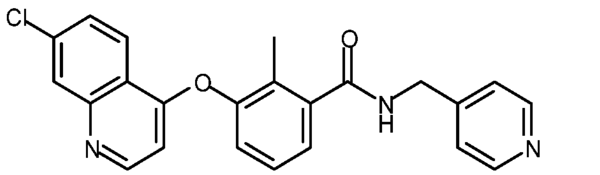

Formel (CXIII)

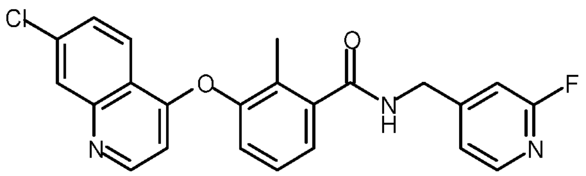

Formel (CLXXXVI)

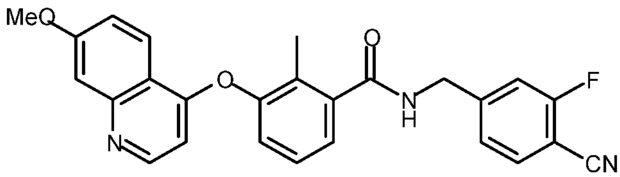

Formel (CLXXXVII)

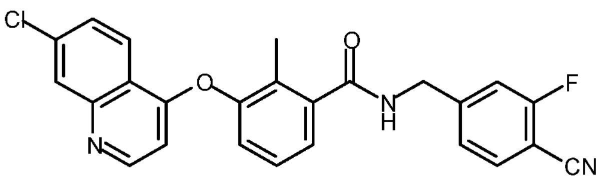

Formel (CLXXXVIII)

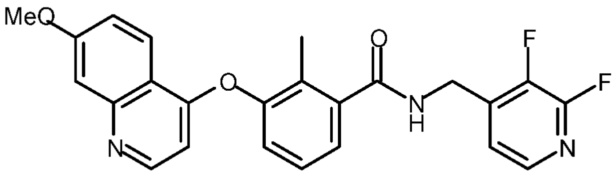

Formel (CXC)

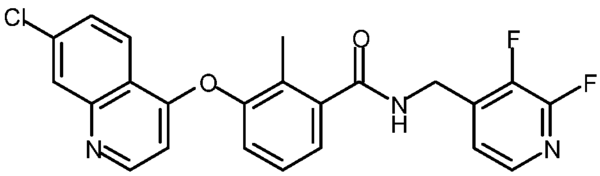

Formel (CXCI)

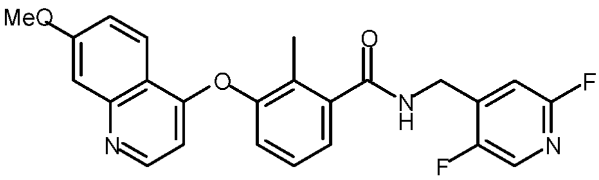

Formel (CXCII)

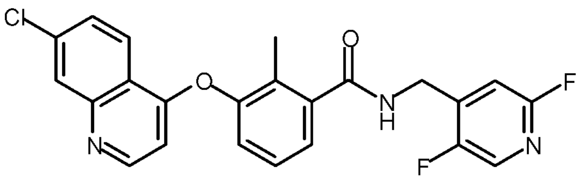

Formel (CXCIII)

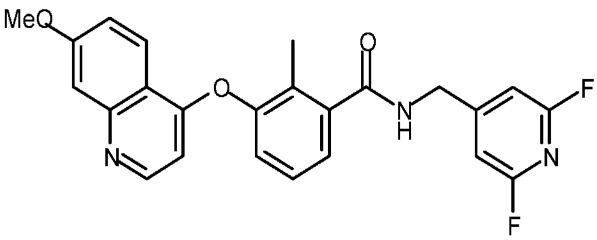

Formel (CXCIV)

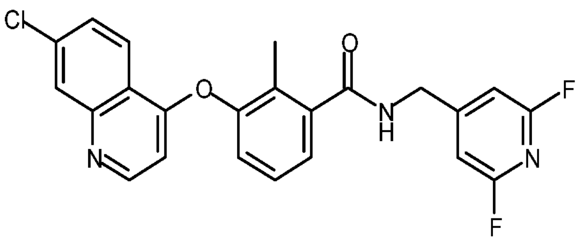

Formel (CXCV)

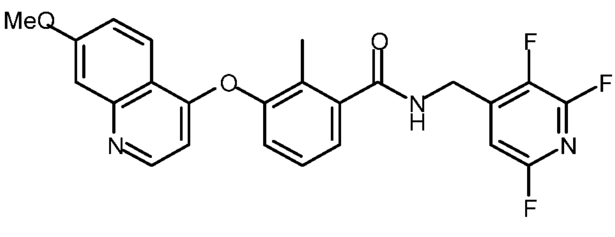

Formel (CXCVI)

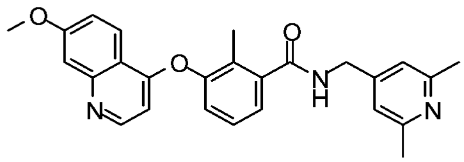

Formel (CXCVIII-6)

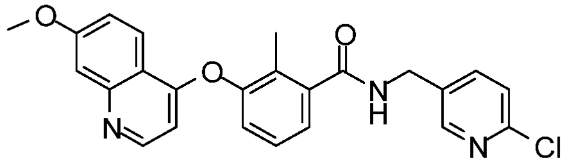

Formel (CXCVIII-7)

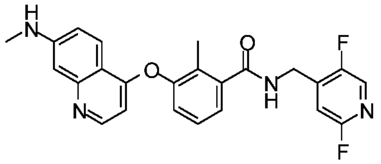

Formel (CXCVIII-16)

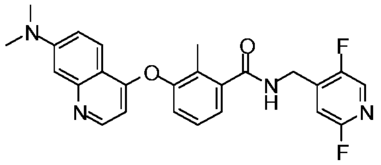

Formel (CXCVIII-17)

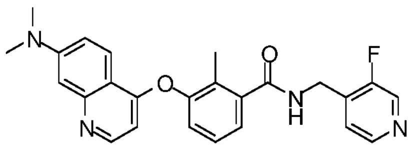

Formel (CXCVIII-19)

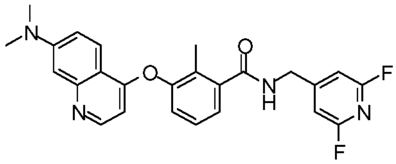

Formel (CXCVIII-20)

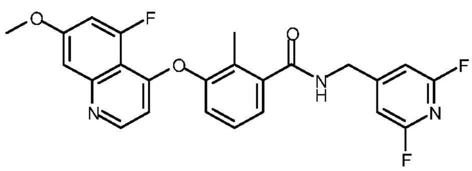

Formel (CXCVIII-27)

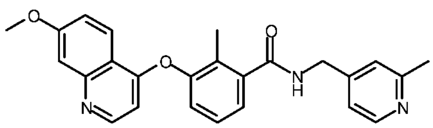

Formel (CXCVIII-33)

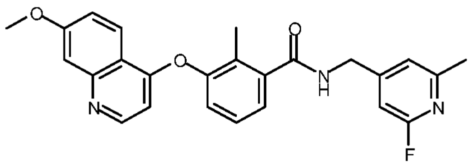

Formel (CXCVIII-34)

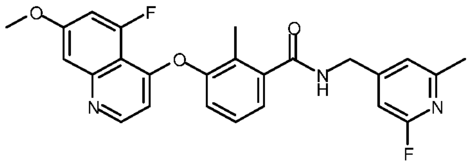

Formel (CXCVIII-35)

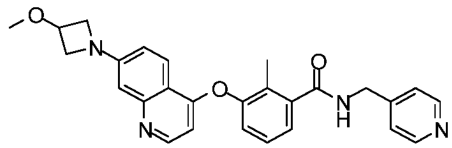

Formel (CXCVIII-37)

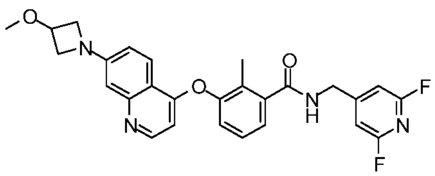

Formel (CXCVIII-38)

**14.** Die Verbindung (C) nach Anspruch 7 oder das N-Oxid, pharmazeutisch akzeptables Salz, pharmazeutisch akzeptables Solvat, oder Stereoisomer davon, wobei die Verbindung der Formel (IIIa-a1) eine Verbindung nach Formel (CXCVIII-3), (CXCVIII-7), (CXCVIII-11), (CXCVIII-16), (CXCVIII-19)-(CXCVIII-21), (CXCVIII-23)-(CXCVIII-26), (CXCVIII-28)-(CXCVIII-31), (CXCVIII-33)-(CXCVIII-39), (CXCVIII-42)-(CXCVIII-47), (CXCVIII-49), (CXCVIII-51)-(CXCVIII-53), (CXCVIII-56), (CXCVIII-59), (CXCVIII-62), (CXCVIII-66), (CXCVIII-67), (CXCVIII-70), (CXCVIII-71), (CXCVIII-73)-(CXCVIII-75), (CXCVIII-81)-(CXCVIII-86), oder (CXCVIII-88)-(CXCVIII-90) wie hier unten ist:

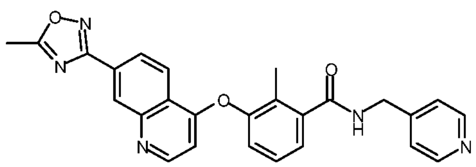

Formel (CXCVIII-3)

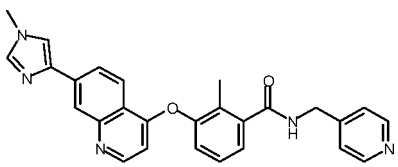

Formel (CXCVIII-7)

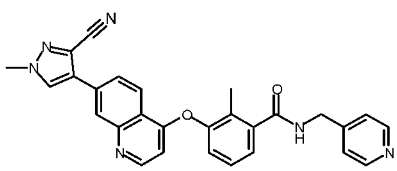

Formel (CXCVIII-11)

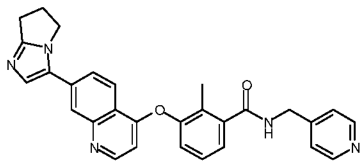

Formel (CXCVIII-16)

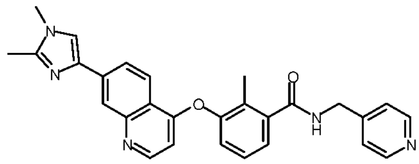

Formel (CXCVIII-19)

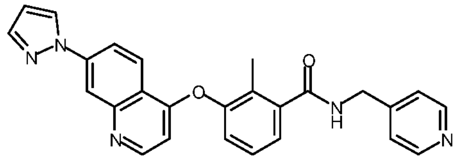

Formel (CXCVIII-20)

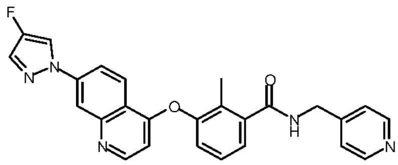

Formel (CXCVIII-21)

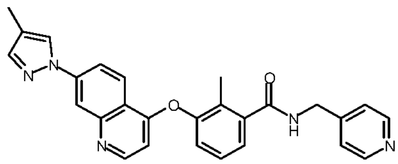

Formel (CXCVIII-23)

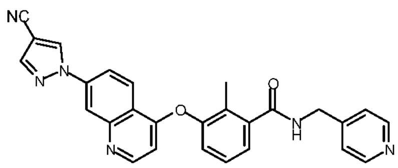

Formel (CXCVIII-24)

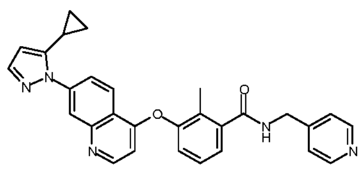

Formel (CXCVIII-25)

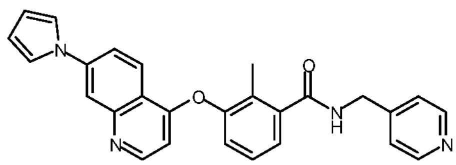

Formel (CXCVIII-26)

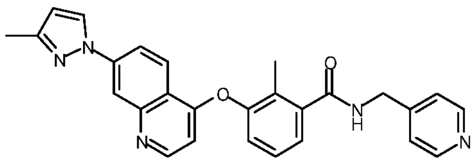

Formel (CXCVIII-28)

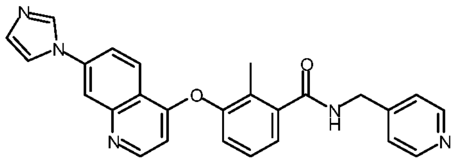

Formel (CXCVIII-29)

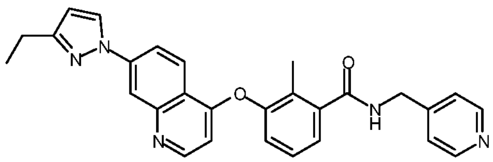

Formel (CXCVIII-30)

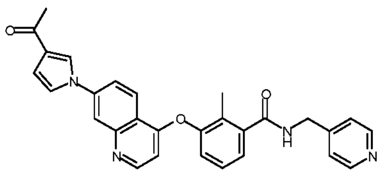

Formel (CXCVIII-31)

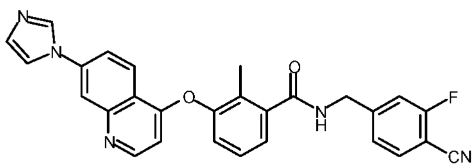

Formel (CXCVIII-33)

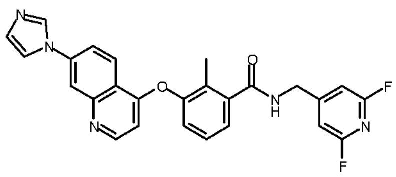

Formel (CXCVIII-34)

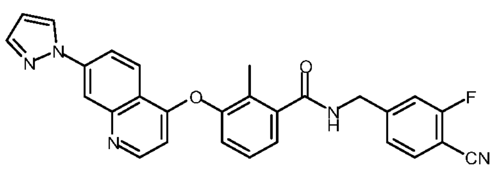

Formel (CXCVIII-35)

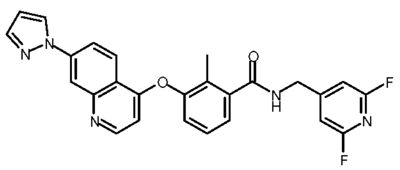

Formel (CXCVIII-36)

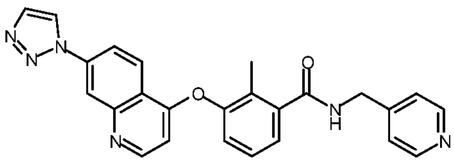

Formel (CXCVIII-37)

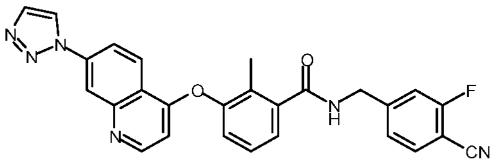

Formel (CXCVIII-38)

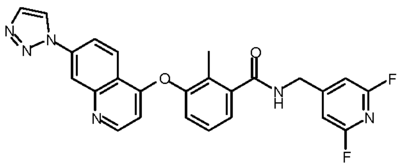

Formel (CXCVIII-39)

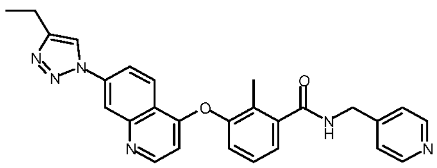

Formel (CXCVIII-42)

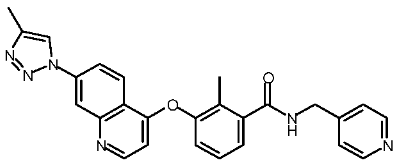

Formel (CXCVIII-43)

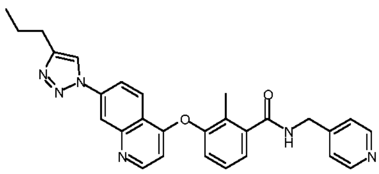

Formel (CXCVIII-44)

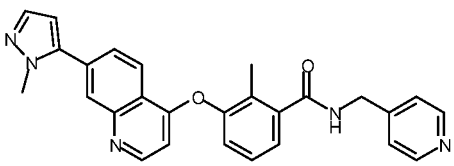

Formel (CXCVIII-45)

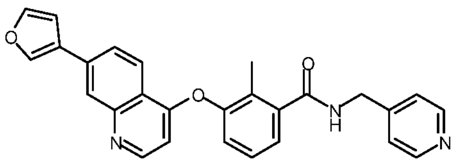

Formel (CXCVIII-46)

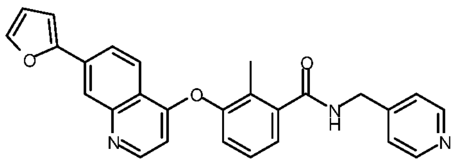

Formel (CXCVIII-47)

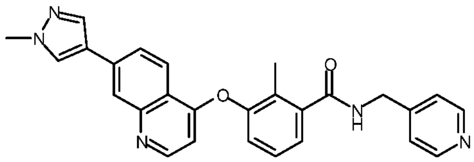

Formel (CXCVIII-49)

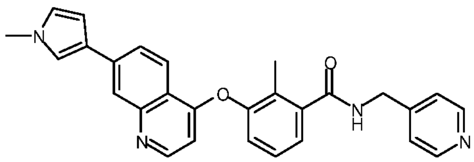

Formel (CXCVIII-51)

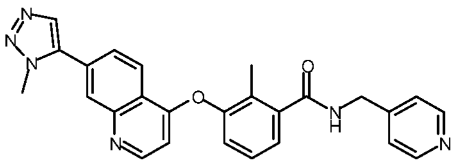

Formel (CXCVIII-52)

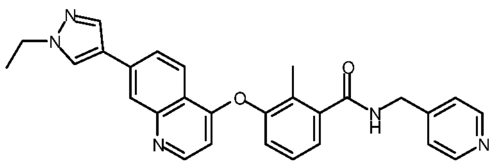

Formel (CXCVIII-53)

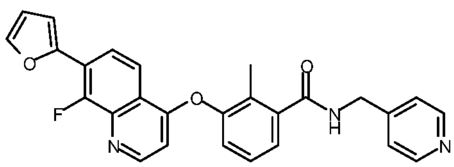

Formel (CXCVIII-56)

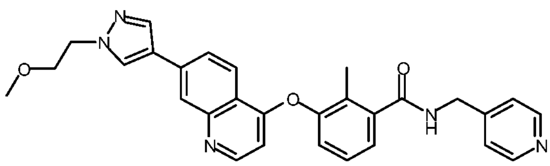

Formel (CXCVIII-59)

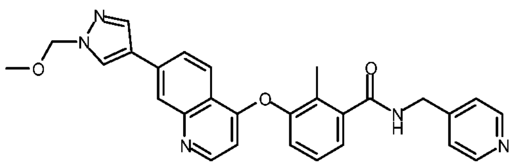

Formel (CXCVIII-62)

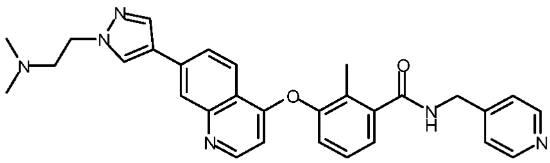

Formel (CXCVIII-66)

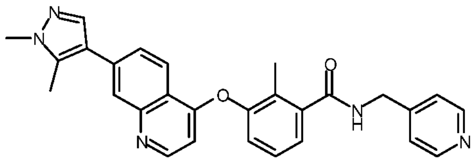

Formel (CXCVIII-67)

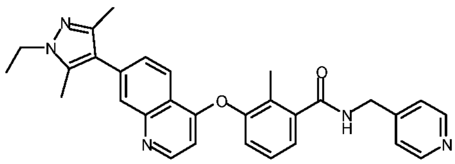

Formel (CXCVIII-70)

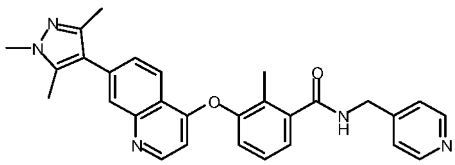

Formel (CXCVIII-71)

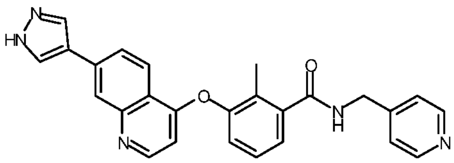

Formel (CXCVIII-73)

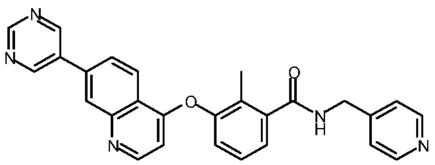

Formel (CXCVIII-74)

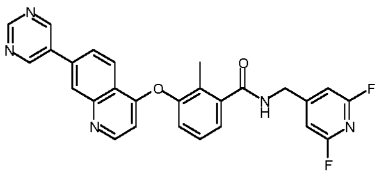

Formel (CXCVIII-75)

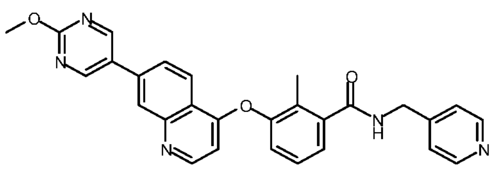

Formel (CXCVIII-81)

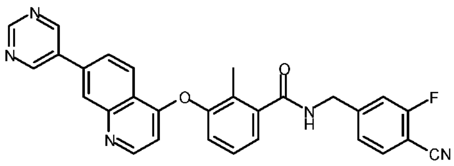

Formel (CXCVIII-82)

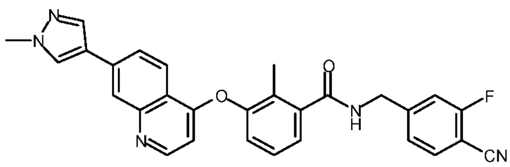

Formel (CXCVIII-83)

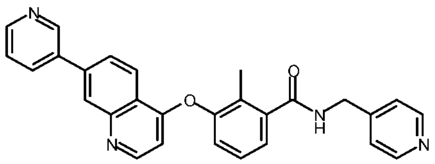

Formel (CXCVIII-84)

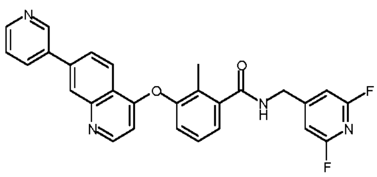

Formel (CXCVIII-85)

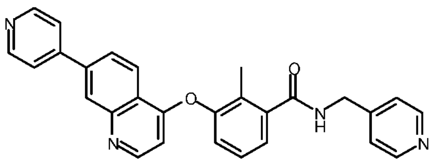

Formel (CXCVIII-86)

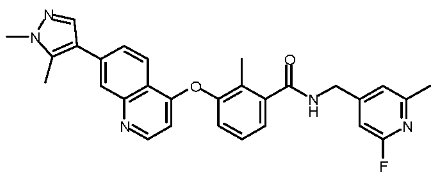

Formel (CXCVIII-88)

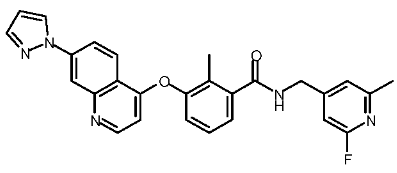

Formel (CXCVIII-89)

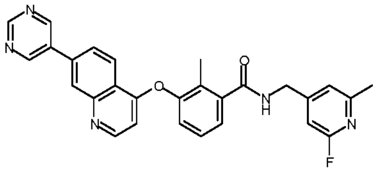

Formel (CXCVIII-90)

**15.** Eine pharmazeutische Zusammensetzung, welche einen Träger, und als Wirkstoff die Verbindung (C), wie nach

irgendeinem der Ansprüche 1 bis 14 definiert, umfasst.

**16.** Eine Verbindung (C), wie nach irgendeinem der Ansprüche 1 bis 14 definiert zur Verwendung als Arzneimittel.

**17.** Eine Verbindung wie nach irgendeinem der Ansprüche 1 bis 14 definiert, zur Verwendung in der Behandlung einer Erkrankung ausgewählt aus Krebs, Stoffwechselerkrankungen, entzündlichen und Autoimmunerkrankungen, neurologischen Erkrankungen, Arteriosklerose und Herz-Kreislauf-Erkrankungen, Sjögren-Syndrom, Abstoßungsreaktion nach Nierentransplantation, viral induzierten Erkrankungen, Kreislauferkrankungen, Osteolyse und Osteoporose, Osteoarthritis, Sarkopenie, Langerhans-Zell-Histiozytose, Rückenmarksverletzung, Endometriose, Asthma und allergischem Asthma, Augenerkrankungen, chronischen und neuropathischen Schmerzen, und fibroproliferativen Erkrankungen.

## Revendications

**1.** Composé ou le N-oxyde, le sel pharmaceutiquement acceptable, le solvate pharmaceutiquement acceptable ou le stéréo-isomère de celui-ci, dans lequel ledit composé est de la formule (IIa) à (IXa) [composés (C) de la classe (I) ci-après] :

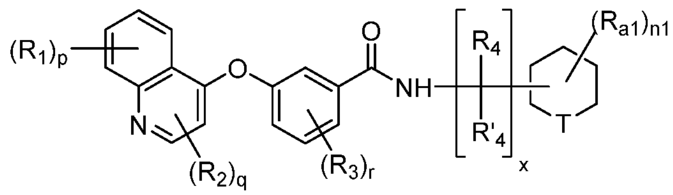

Formule (IIa)

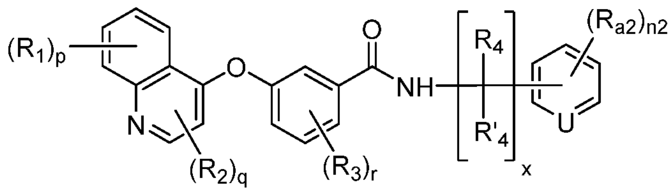

Formule (IIIa)

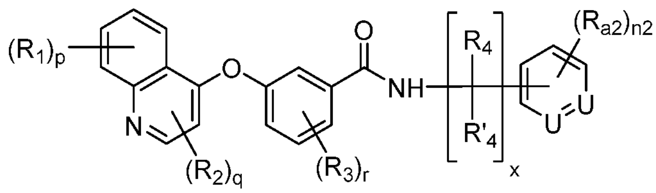

Formule (IVa)

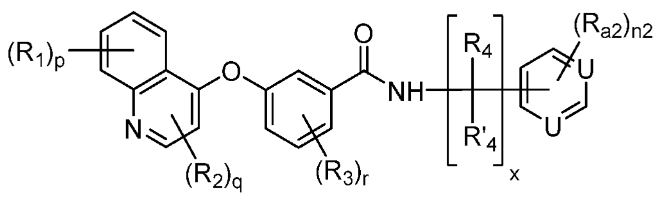

Formule (Va)

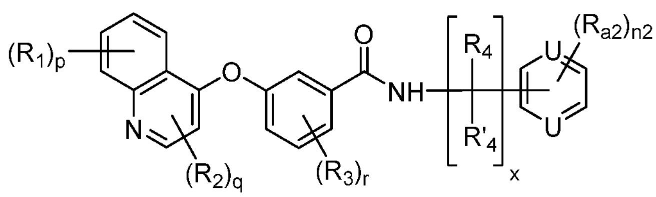

Formule (VIa)

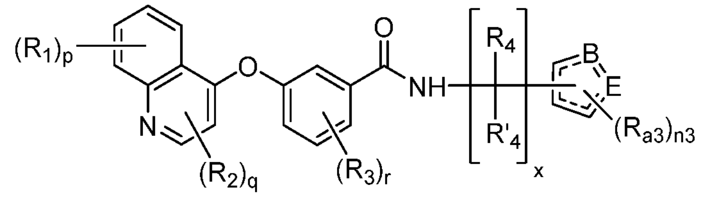

Formule (VIIa)

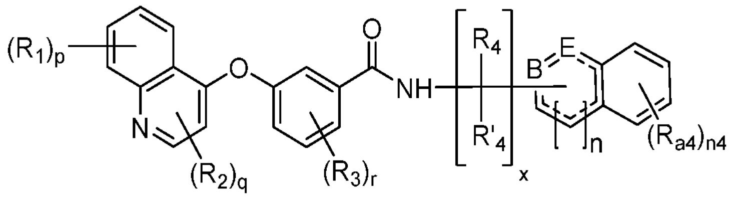

Formule (VIIIa)

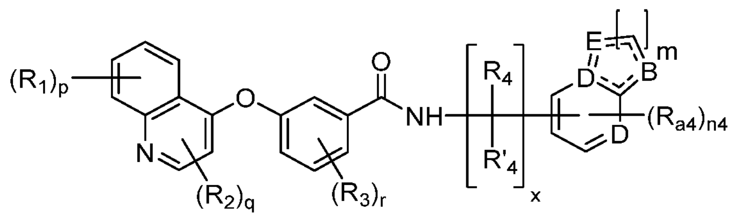

Formule (IXa)

dans lequel la liaison en pointillés représente une double liaison facultative et dans lequel :

- chacun des $R_1$ et $R_2$, indépendamment l'un de l'autre et à chaque occurrence, est choisi dans le groupe constitué de l'hydrogène, le halo, l'alkyle en $C_{1-6}$, l'alcényle en $C_{2-5}$, l'alcynyle en $C_{2-5}$, le cycloalkyle, l'hétérocyclyle, l'aryle et l'hétéroaryle, le $CF_3$, le CN, le $NO_2$, l'$OR_{21}$, le $SR_{21}$, le $N(R_{21})_2$, le $COR_{21}$, le $C(O)OR_{21}$, le $CON(R_{21})_2$, l'$OC(O)R_{21}$, l'$OCON(R_{21})_2$, le $NC(O)R_{21}$, le $NCON(R_{21})_2$, l'$OC(R_{21})_2O$ et l'$OC(R_{21})_2C(R_{22})_2O$, dans lequel lesdits alkyle, alcényle, alcynyle, cycloalkyle, hétérocyclyle, aryle et hétéroaryle sont éventuellement substitués par un ou plusieurs substituants choisis parmi le halo, l'alkyle en $C_{1-6}$, l'alcényle en $C_{2-5}$, l'alcynyle en $C_{2-5}$, le cycloalkyle, l'hétérocyclyle, le $CF_3$, le $COR_{21}$, le $CON(R_{21})_2$, le $C(O)OR_{21}$, le $N(R_{21})_2$, le CN, ou l'$OR_{21}$, et chaque substituant facultatif d'alkyle, d'alcényle, d'alcynyle, de cycloalkyle, d'hétérocyclyle, d'aryle ou d'hétéroaryle est en outre éventuellement substitué par un hétérocyclyle, le $N(R_{11})_2$, ou l'$OR_{11}$; et dans lequel chacun des $R_{21}$ et $R_{22}$, indépendamment l'un de l'autre et à chaque occurrence, est choisi dans le groupe constitué de l'hydrogène, l'alkyle en $C_{1-6}$, l'alcényle en $C_{2-5}$, l'alcynyle en $C_{2-5}$, le cycloalkyle, l'hétérocyclyle, l'aryle, l'hétéroaryle et l'aralkyle, et dans lesdits substituants alkyle, alcényle, alcynyle, cycloalkyle, hétérocyclyle, aryle, hétéroaryle et aralkyle sont éventuellement substitués par le halo, l'alkyle en $C_{1-6}$, le cycloalkyle, l'hétérocyclyle, l'aryle, $OR_{31}$ ou $N(R_{32})_2$, où chacun de $R_{31}$ et $R_{32}$, indépendamment l'un de l'autre et à chaque occurrence, est choisi dans le groupe constitué de l'hydrogène et l'alkyle en $C_{1-4}$; chacun des p est un nombre entier compris entre 0 et 4 ; chacun des q est un nombre entier compris entre 0 et 2 ;
- chacun des $R_3$, indépendamment l'un de l'autre et à chaque occurrence, est choisi dans le groupe constitué de l'hydrogène, le halo, l'alkyle en $C_{1-15}$, l'alcényle en $C_{2-15}$, l'alcynyle en $C_{2-15}$, le cycloalkyle, l'hétérocyclyle, le $CF_3$, le CN, l'$OR_{21}$, le $SR_{21}$, le $N(R_{21})_2$, le $NC(O)R_{21}$, le $NCON(R_{21})_2$, le $COR_{21}$, le $C(O)OR_{21}$, le $CON(R_{21})_2$, l'$OC(O)R_{21}$, l'$OCON(R_{21})_2$, l'$OC(R_{21})_2O$, et l'$OC(R_{21})_2C(R_{22})_2O$, dans lequel lesdits alkyle, alcényle, alcynyle, cycloalkyle, et hétérocyclyle, sont éventuellement substitués par un ou plusieurs substituants choisis parmi le halo, l'alkyle en $C_{1-15}$, le $CF_3$, le $N(R_{21})_2$, le CN, ou l'$OR_{21}$ ; et dans lequel $R_{21}$ et $R_{22}$, indépendamment l'un de l'autre et à chaque occurrence, sont choisis dans le groupe constitué de l'hydrogène, l'alkyle en $C_{1-15}$, l'alcényle en $C_{2-15}$, l'alcynyle en $C_{2-15}$, le cycloalkyle, l'hétérocyclyle, l'aryle, l'hétéroaryle et l'aralkyle, et dans lequel lesdits substituants d'alkyle, d'alcényle, d'alcynyle, de cycloalkyle, d'hétérocyclyle, d'aryle, d'hétéroaryle et d'aralkyle sont éventuellement substitués par le halo, l'alkyle, le cycloalkyle, l'hétérocyclyle, l'aryle, l'$OR_{31}$ ou le $N(R_{32})_2$, où chacun des $R_{31}$ et $R_{32}$, indépendamment l'un de l'autre et à chaque occurrence, est choisi dans le groupe constitué de l'hydrogène et de l'alkyle en $C_{1-4}$ ; chacun des r est un nombre entier compris entre 0 et 3 ;
- chacun des $R_4$ et $R'_4$, indépendamment les uns des autres et à chaque occurrence, sont choisis dans le groupe constitué de l'hydrogène, $CF_3$, l'alkyle en $C_{1-6}$, l'alcényle en $C_{2-5}$, l'alcynyle en $C_{2-5}$, le cycloalkyle et l'hétérocyclyle, dans lequel lesdits alkyle, alcényle, cycloalkyle et hétérocyclyle sont éventuellement substitués

par un atome d'halogène, un groupe aryle, un groupe aralkyle, l'$OR_{13}$, le $SR_{13}$, le $N(R_{13})_2$, le $CF_3$ ou le CN, dans lequel chacun des $R_{13}$, indépendamment les uns des autres, est choisi parmi l'hydrogène, l'alkyle en $C_{1-12}$, l'alcényle en $C_{2-12}$, l'alcynyle en $C_{2-12}$, le cycloalkyle, le hétérocyclyle, l'aryle, le hétéroaryle ou l'aralkyle qui sont éventuellement substitués par un atome d'halogène, un groupe aryle, un groupe aralkyle, l'$OR_{31}$ ou le $N(R_{32})_2$, dans lequel chacun des $R_{31}$ et $R_{32}$, indépendamment l'un de l'autre et à chaque occurrence, est choisi dans le groupe constitué de l'hydrogène et un alkyle en $C_{1-4}$, et x est un nombre entier compris entre 0 et 7 ;

- T est choisi parmi le $CH_2$, le N-R, le O ou le S, où R est choisi parmi l'hydrogène, l'alkyle en $C_{1-10}$ ou le cycloalkyle, qui sont éventuellement substitués par un atome d'halogène, un groupe aryle ou un groupe aralkyle ;
- chacun de U est choisi, indépendamment et à chaque occurrence, parmi C-halo, C-R ou N ; et dans lequel R est choisi parmi l'hydrogène, $OR_{11}$, $N(R_{11})_2$, un alkyle en $C_{1-10}$ ou un cycloalkyle qui sont éventuellement substitués par un atome d'halogène, un groupe aryle ou un groupe aralkyle, où chacun de $R_{11}$, indépendamment l'un de l'autre et à chaque occurrence, est choisi dans le groupe constitué de l'hydrogène et de l'alkyle en $C_{1-4}$ ;
- chacun des D est choisi, indépendamment et à chaque occurrence, parmi C, C-R ou N, où R est choisi parmi l'hydrogène, l'alkyle en $C_{1-5}$ ou le cycloalkyle ;
- m est un nombre entier égal à 1 ou 2 ;
- n est un nombre entier égal à 0 ou 1 ;
- chacun de $R_{a1}$, indépendamment l'un de l'autre et à chaque occurrence, est choisi dans le groupe constitué de l'hydrogène, l'alkyle en $C_{1-5}$, l'alcényle en $C_{2-15}$ et l'alcynyle en $C_{2-15}$, dans lequel l'alkyle en $C_{1-5}$, l'alcényle en $C_{2-15}$ et l'alcynyle en $C_{2-15}$ sont éventuellement substitués par un atome d'halogène, un groupe aryle ou un groupe aralkyle ; chacun de n1 est un nombre entier dans la gamme de 0 à 5 ;
- chacun des $R_{a2}$, indépendamment l'un de l'autre et à chaque occurrence, est choisi dans le groupe constitué de l'alkyle en $C_{1-15}$, l'alcényle en $C_{2-15}$, l'alcynyle en $C_{2-15}$, le cycloalkyle, le hétérocyclyle, l'aryle, le hétéroaryle, l'aralkyle, le halo, le $NO_2$, le $CF_3$, le CN, l'$OR_{11}$, le $SR_{11}$, le $N(R_{11})_2$, l'$OC(R_{11})_2O$, l'$OC(R_{11})_2C(R_{11})_2O$, le $COOR_{11}$, le $CO(R_{11})_2$, le $CON(R_{11})_2$, et le $SO_2N(R_{11})_2$, et chaque substituant facultatif l'alkyle, l'alcényle, l'alcynyl cycloalkyle, l'aryle, le hétérocyclyle, l'hétéroaryle est en outre éventuellement substitué par le halo, l'alkyle en $C_{1-6}$, le cycloalkyle, l'aryle, le $N(R_{11})_2$, le $CF_3$, le CN, le $COOR_{11}$, le $CO(R_{11})_2$, le $CON(R_{11})_2$, le $SO_2N(R_{11})_2$ ou l'$OR_{11}$ et dans lequel chacun des $R_{11}$, indépendamment l'un de l'autre et à chaque occurrence, est choisi dans le groupe constitué de l'hydrogène, l'alkyle en $C_{1-6}$, le cycloalkyle, l'hétérocyclyle, l'aryle, l'hétéroaryle et l'aralkyle, dans lequel lesdits substituants alkyle, cycloalkyle, hétérocyclyle, aryle, hétéroaryle et aralkyle sont éventuellement substitués par le halo, l'alkyle en $C_{1-6}$, le cycloalkyle, le hétérocyclyle, le $OR_{31}$ ou $N(R_{32})_2$, où chacun des $R_{31}$ et $R_{32}$, indépendamment l'un de l'autre et à chaque occurrence, sont choisis dans le groupe constitué de l'hydrogène et de l'alkyle en $C_{1-4}$ chacun de n2 est un nombre entier compris entre 0 et 4 ;
- chacun des B, indépendamment l'un de l'autre et à chaque occurrence, est choisi parmi C-R, O, N, S et $NR_{7'}$, dans lequel R est choisi parmi l'hydrogène ou un alkyle en $C_{1-10}$ éventuellement substitué par un atome d'halogène, un groupe aryle ou un groupe aralkyle, dans lequel $R_{7'}$ est choisi parmi le groupe constitué de l'hydrogène, l'alkyle en $C_{1-6}$, l'alcényle en $C_{1-6}$, le cycloalkyle, l'hétérocyclyle, l'aryle, l'aralkyle et le $CF_3$ ;
- chacun des E, indépendamment l'un de l'autre et à chaque occurrence, est choisi parmi C-R, O, N, S et $NR_{7'}$, où R est choisi parmi l'hydrogène ou un alkyle en $C_{1-10}$ éventuellement substitué par un atome d'halogène, un groupe aryle ou un groupe aralkyle, où $R_{7'}$ est choisi parmi le groupe constitué de l'hydrogène, l'alkyle en $C_{1-6}$, l'alcényle en $C_{1-6}$, le cycloalkyle, l'hétérocyclyle, l'aryle, l'aralkyle et le $CF_3$ ;
- chacun des $R_{a4}$, indépendamment l'un de l'autre et à chaque occurrence, est choisi dans le groupe constitué de l'alkyle en $C_{1-15}$, l'alcényle en $C_{2-15}$ l'alcynyle en $C_{2-15}$, le cycloalkyle, le hétérocyclyle, l'aryle, le hétéroaryle, l'aralkyle, le halo, le $NO_2$, le $CF_3$, le CN, l'$OR_{11}$, le $SR_{11}$, le $N(R_{11})_2$, l'$OC(R_{11})_2O$, l'$OC(R_{11})_2C(R_{11})_2O$, le $COOR_{11}$, le $CO(R_{11})_2$ et le $CON(R_{11})_2$, et chaque substituant facultatif d'alkyle, d'alcényle, d'alcynyle, de cycloalkyle, d'aryle, d'hétérocyclyle, d'hétéroaryle, d'aralkyle est en outre éventuellement substitué par le halo, l'alkyle, le cycloalkyle, l'aryle, le $N(R_{11})_2$, le CN, ou l'$OR_{11}$ et dans lequel chacun des $R_{11}$, indépendamment l'un de l'autre et à chaque occurrence, est choisi dans le groupe constitué de l'hydrogène, l'alkyle en $C_{1-6}$, le cycloalkyle, l'hétérocyclyle, l'aryle, l'hétéroaryle et l'aralkyle, dans lequel lesdits substituants d'alkyle, alcényle, alcynyle, cycloalkyle, hétérocyclyle, aryle, hétéroaryle et aralkyle sont éventuellement substitués par le halo, l'alkyle, le cycloalkyle, le hétérocyclyle, $OR_{31}$ ou $N(R_{32})_2$, où chacun de $R_{31}$ et $R_{32}$, indépendamment l'un de l'autre et à chaque occurrence, est choisi dans le groupe constitué de l'hydrogène et de l'alkyle en $C_{1-4}$ ; chacun des n4 est un nombre entier compris entre 0 et 4 ;
- chacun des $R_{a3}$, indépendamment l'un de l'autre et à chaque occurrence, est choisi dans le groupe constitué de l'alkyle en $C_{1-15}$, l'alcényle en $C_{2-15}$ l'alcynyle en $C_{2-15}$, le cycloalkyle, le hétérocyclyle, l'aryle, le hétéroaryle, l'aralkyle, le halo, le $CF_3$, l'$OR_{11}$, le $SR_{11}$, le $N(R_{11})_2$, le $COOR_{11}$, le $CO(R_{11})_2$, le $CON(R_{11})_2$, et chaque substituant facultatif d'alkyle, d'alcényle, d'alcynyle, de cycloalkyle, d'aryle, d'hétérocyclyle, d'hétéroaryle, d'aralkyle est en outre éventuellement substitué par le halo, l'alkyle, le cycloalkyle, l'aryle, le $N(R_{11})_2$, le CN, ou l'$OR_{11}$ et dans lequel chacun des $R_{11}$, indépendamment l'un de l'autre et à chaque occurrence, est choisi dans le groupe

constitué de l'hydrogène, l'alkyle en $C_{1-6}$, le cycloalkyle, l'hétérocyclyle, l'aryle, l'hétéroaryle et l'aralkyle, dans lequel lesdits substituants d'alkyle, de cycloalkyle, d'hétérocyclyle, d'aryle, d'hétéroaryle et d'aralkyle sont éventuellement substitués par un halo, un alkyle, un cycloalkyle, un hétérocyclyle, l'$OR_{31}$ ou le $N(R_{32})_2$, dans lequel chacun de $R_{31}$ et $R_{32}$, indépendamment l'un de l'autre et à chaque occurrence, est choisi dans le groupe constitué de l'hydrogène et l'alkyle en $C_{1-4}$ ; chacun de n3 est un nombre entier dans la plage de 0 à 2 ;

dans lequel ledit cycloalkyle est un système cyclique monocyclique, bicyclique ou tricyclique de 3 à 7 membres par cycle ; ledit hétérocyclyle est un monocycle, un bicyclique ou un tricycle saturé, partiellement saturé ou complètement saturé contenant de 3 à 12 atomes de carbone et 1 ou 2 hétéroatomes choisis indépendamment parmi O ou N ; ledit aryle est un phényle, un naphtyle ou un anthracényle éventuellement carbocyclique fusionné avec un cycloalkyle ou un hétérocyclyle de 5 à 7 éléments de cycle ; ledit hétéroaryle est une structure cyclique monocyclique contenant 5 ou 6 atomes cycliques, ou un groupe aromatique bicyclique ayant 8 à 10 atomes, contenant 1 à 3 hétéroatomes indépendamment choisis parmi O ou N.

**2.** Composé (C) selon les formules générales (IIa) à (IXa) et selon la revendication 1, ou le N-oxyde, le sel pharmaceutiquement acceptable, le solvate pharmaceutiquement acceptable, ou le stéréo-isomère de celui-ci, dans lequel :

- chacun de $R_1$ et $R_2$, indépendamment l'un de l'autre et à chaque occurrence, est choisi dans le groupe constitué de l'hydrogène, le halo, l'alkyle en $C_{1-6}$, l'alcényle en $C_{2-5}$, l'alcynyle en $C_{2-5}$, le cycloalkyle, le hétérocyclyle, l'aryle et le hétéroaryle, le $CF_3$, le CN, le $NO_2$, l'$OR_{21}$, le $SR_{21}$, le $N(R_{21})_2$, le $COR_{21}$, le $C(O)OR_{21}$, le $CON(R_{21})_2$, l'$OC(O)R_{21}$, l'$OCON(R_{21})_2$, le $NC(O)R_{21}$, le $NCON(R_{21})_2$, l'$OC(R(_{21})_2O$ et l'$OC(R(_{21})_2C(R_{22})_2O$, dans lequel lesdits alkyle, alcényle, alcynyle, cycloalkyle, hétérocyclyle, aryle et hétéroaryle sont éventuellement substitués par un ou plusieurs substituants choisis parmi le halo, l'alkyle en $C_{1-6}$, le cycloalkyle, le hétérocyclyle, le $CF_3$, le $COR_{21}$, le $N(R_{21})_2$, le CN, le $CONR_{21}$, le $C(O)OR_{21}$ ou l'$OR_{21}$, et chaque substituant alkyle facultatif est en outre éventuellement substitué par le hétérocyclyle, le $N(R_{11})_2$, ou l'$OR_{11}$ ; et dans lequel chacun des $R_{21}$ et $R_{22}$, indépendamment l'un de l'autre et à chaque occurrence, est choisi dans le groupe constitué de l'hydrogène, l'alkyle en $C_{1-6}$, l'alcényle en $C_{2-5}$, l'alcynyle en $C_{2-5}$, le cycloalkyle, l'hétérocyclyle, le phényle, l'hétéroaryle et l'aralkyle, et dans lequel lesdits substituants d'alkyle, d'alcényle, d'alcynyle, de cycloalkyle, d'hétérocyclyle, de phényle, d'hétéroaryle et d'aralkyle sont éventuellement substitués par le halo, l'alkyle en $C_{1-6}$, le cycloalkyle, le hétérocyclyle, le phényle, l'$OR_{31}$ ou le $N(R_{32})_2$, où chacun des $R_{31}$ et $R_{32}$, indépendamment l'un de l'autre et à chaque occurrence, sont choisis dans le groupe constitué de l'hydrogène et de l'alkyle en $C_{1-4}$ ; chacun des p est un nombre entier compris entre 0 et 4 ; chacun des q est un nombre entier compris entre 0 et 2 ;
- chacun des $R_3$, indépendamment l'un de l'autre et à chaque occurrence, est choisi dans le groupe constitué de l'hydrogène, le halo, l'alkyle en $C_{1-15}$, l'alcényle en $C_{2-15}$, l'alcynyle en $C_{2-15}$, le cycloalkyle, l'hétérocyclyle, le $CF_3$, le CN, l'$OR_{21}$, le $SR_{21}$, le $N(R_{21})_2$, le $NC(O)R_{21}$, le $NCON(R_{21})_2$, le $COR_{21}$, le $C(O)OR_{21}$, le $CON(R_{21})_2$, l'$OC(O)R_{21}$, l'$OCON(R_{21})_2$, l'$OC(R_{21})_2O$, et l'$OC(R_{21})_2C(R_{22})_2O$, dans lequel lesdits alkyle, alcényle, alcynyle, cycloalkyle, et hétérocyclyle, sont éventuellement substitués par un ou plusieurs substituants choisis parmi le halo, l'alkyle en $C_{1-15}$, le $CF_3$, le $N(R_{21})_2$, le CN, ou l'$OR_{21}$, et dans lequel chacun des $R_{21}$ et $R_{22}$, indépendamment l'un de l'autre et à chaque occurrence, sont choisis dans le groupe constitué de l'hydrogène, l'alkyle en $C_{1-15}$, l'alcényle en $C_{2-15}$, l'alcynyle en $C_{2-15}$, le cycloalkyle, l'hétérocyclyle, l'aryle, l'hétéroaryle et l'aralkyle, et dans lequel lesdits substituants d'alkyle, d'alcényle, d'alcynyle, de cycloalkyle, d'hétérocyclyle, d'aryle, d'hétéroaryle et d'aralkyle sont éventuellement substitués par le halo, l'alkyle, le cycloalkyle, l'hétérocyclyle, l'aryle, l'$OR_{31}$ ou le $N(R_{32})_2$, où chacun de $R_{31}$ et $R_{32}$, indépendamment l'un de l'autre et à chaque occurrence, est choisi dans le groupe constitué de l'hydrogène et de l'alkyle en $C_{1-4}$; chacun des r est un nombre entier compris entre 0 et 3 ;
- chacun des $R_4$ et $R'_4$, indépendamment l'un de l'autre et à chaque occurrence, sont choisis dans le groupe constitué de l'hydrogène, l'alkyle en $C_{1-6}$ et l'alcényle en $C_{2-6}$, dans lequel l'alkyle en $C_{1-6}$ et l'alcényle en $C_{2-6}$ sont éventuellement substitués par un atome d'halogène, et x est un nombre entier compris entre 0 et 7.

**3.** Composé selon la revendication 1, ou le N-oxyde, le sel pharmaceutiquement acceptable, le solvate pharmaceutiquement acceptable ou le stéréo-isomère de celui-ci, dans lequel ledit composé est de la formule (IIa-a) à (IXa-a) [composés (C) de la classe (I) ci-après] :

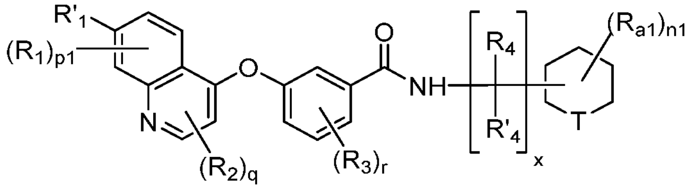

Formule (IIa-a)

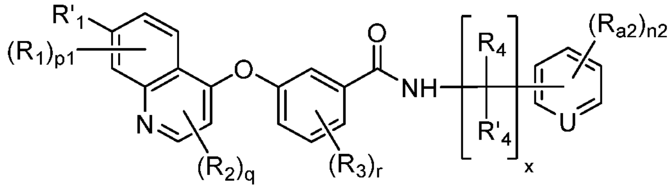

Formule (IIIa-a)

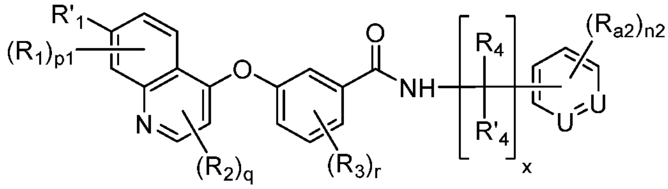

Formule (IVa-a)

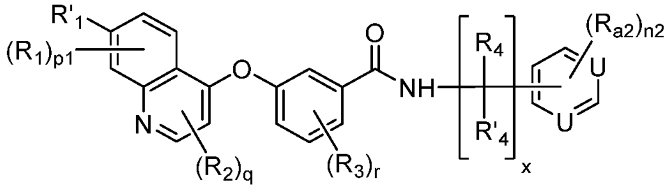

Formule (Va-a)

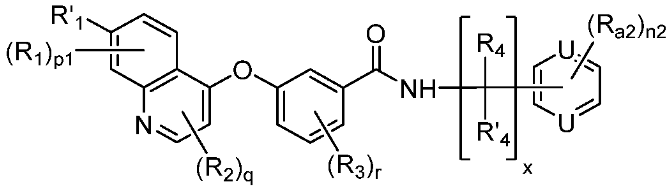

Formule (VIa-a)

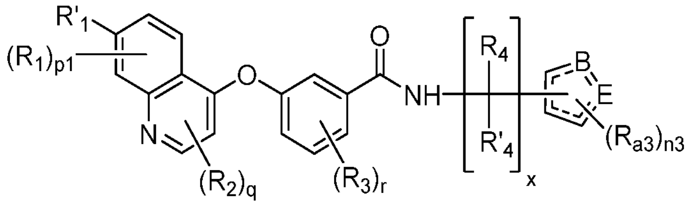

Formule (VIIa-a)

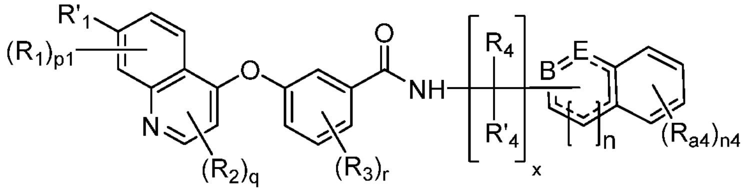

Formule (VIIIa-a)

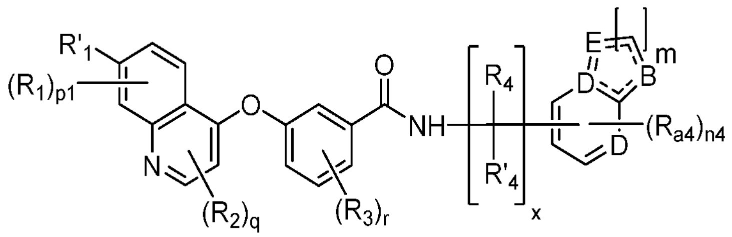

Formule (IXa-a)

dans lequel $R_1$, $R_2$, $R_3$, $R_4$, $R_4$', $R_{a1}$, $R_{a2}$, $R_{a3}$, $R_{a4}$, T, U, B, E, D, q, r, x, n1, n2, n3, n4, m, n sont tels que définis dans la revendication 1 et dans laquelle :

- R'$_1$ est choisi dans le groupe constitué de (R'$_1$-a) à (R'$_1$-d) :

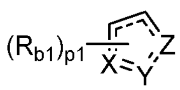

(R'$_1$-a)

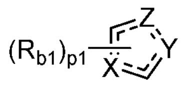

(R'$_1$-b)

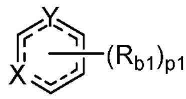

(R'$_1$-c)

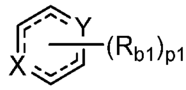

(R'$_1$-d)

dans lequel :

- chacun de X, Y et Z indépendamment l'un de l'autre et à chaque occurrence est choisi parmi C-R, O, N, S et $NR_{7'}$, dans lequel R est choisi parmi l'hydrogène ou un alkyle en $C_{1-6}$ éventuellement substitué par un atome d'halogène, un groupe aryle ou un groupe aralkyle, dans lequel $R_{7'}$ est choisi dans le groupe constitué de l'hydrogène, l'alkyle en $C_{1-6}$, l'alcényle en $C_{1-6}$, le cycloalkyle, l'hétérocyclyle, l'aryle, l'aralkyle et le $CF_3$, et chaque substituant alkyle, alcényle, cycloalkyle, hétérocyclyle, aryle ou aralkyle est en outre éventuellement substitué par l'hétérocyclyle, $N(R_{11})_2$, ou $OR_{11}$ ;
- chacun des $R_{b1}$, indépendamment et à chaque occurrence, est choisi dans le groupe constitué de l'hydrogène, le halo, l'alkyle en $C_{1-6}$, le cycloalkyle, l'hétérocyclyle, le CN, le $CF_3$, le $COR_{11}$, le $CON(R_{11})_2$, le $NR_{11}$ et l'$OR_{11}$, dans lequel lesdits alkyle, cycloalkyle et hétérocyclyle sont éventuellement substitués un ou plusieurs substituants choisis parmi l'hétérocyclyle, l'$OR_{11}$, le $NR_{11}$ et dans lequel chacun des $R_{11}$, indépendamment l'un de l'autre et à chaque occurrence, est choisi dans le groupe constitué de l'hydrogène, le halo et l'alkyle en $C_{1-6}$ ; chacun des p1 est un nombre entier compris entre 0 et 3 ;
- la liaison en pointillés représente une double liaison facultative ;

dans lequel ledit cycloalkyle est un système cyclique monocyclique, bicyclique ou tricyclique de 3 à 7 membres par cycle ; ledit hétérocyclyle est un monocycle, un bicycle ou un tricycle saturé, partiellement saturé ou complètement saturé contenant de 3 à 12 atomes de carbone et 1 ou 2 hétéroatomes choisis indépendamment parmi O ou N ; ledit aryle est un phényle, un naphtyle ou un anthracényle éventuellement carbocyclique fusionné avec un cycloalkyle ou un hétérocyclyle de 5 à 7 membres cycliques ; ledit hétéroaryle est une structure cyclique monocyclique contenant 5 ou 6 atomes cycliques, ou un groupe aromatique bicyclique ayant 8 à 10 atomes, contenant 1 à 3 hétéroatomes indépendamment choisis parmi O ou N.

**4.** Composé (C) selon les formules générales (IIa-a) à (IXa-a) et selon la revendication 3, ou le N-oxyde, le sel pharmaceutiquement acceptable, le solvate pharmaceutiquement acceptable, ou le stéréo-isomère de celui-ci, dans lequel $R_1$, $R_2$, $R_3$, $R_4$, $R_4$', p, q, r, x, $R_{a1}$, $R_{a2}$, $R_{a3}$, $R_{a4}$, T, U, B, E, D n1, n2, n3, n4, m, n sont tels que définis dans la revendication 2.

**5.** Composé selon la revendication 1, ou le N-oxyde, le sel pharmaceutiquement acceptable, le solvate pharmaceutiquement acceptable, ou le stéréo-isomère de celui-ci, dans lequel ledit composé est de la formule (IIa-1) à (IXa-1)

[composés (C) de la classe (I) ci-après] :

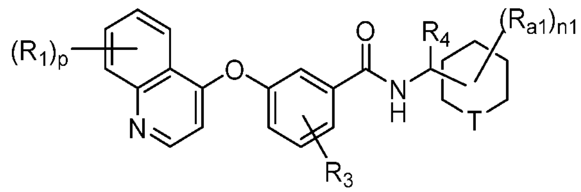

Formule (IIa-1)

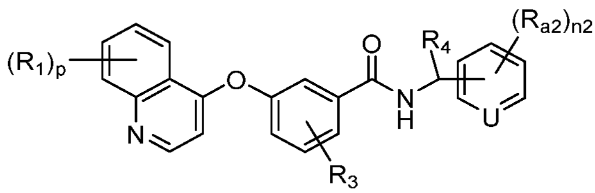

Formule (IIIa-1)

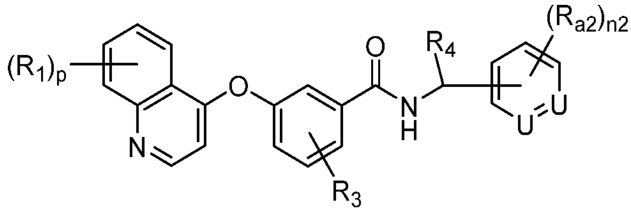

Formule (IVa-1)

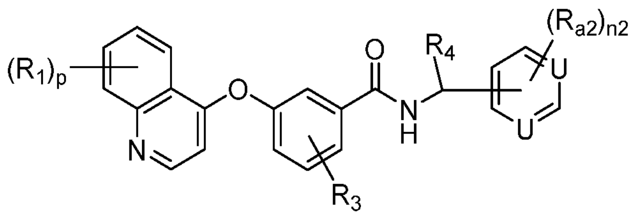

Formule (Va-1)

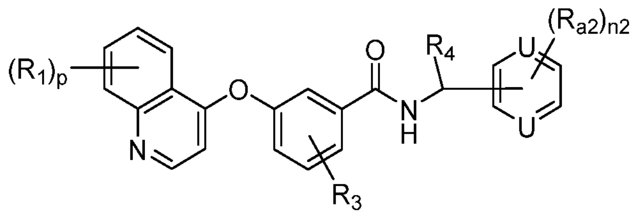

Formule (VIa-1)

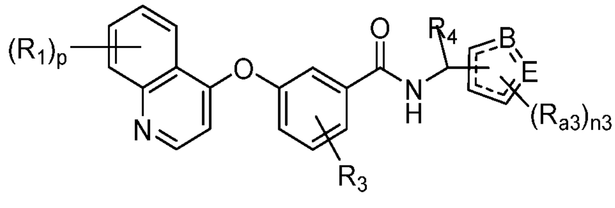

Formule (VIIa-1)

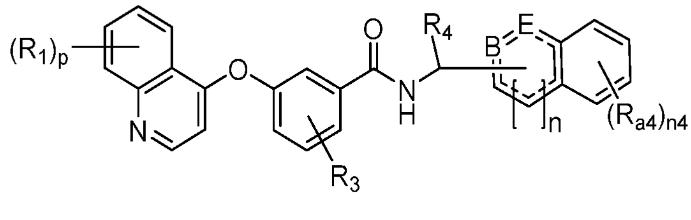

Formule (VIIIa-1)

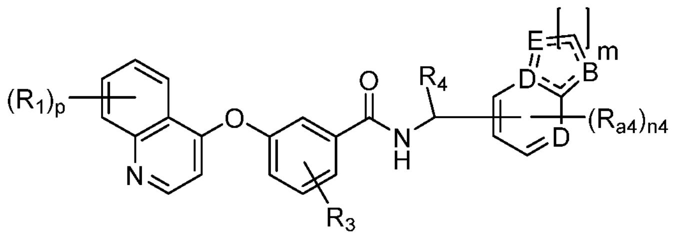

Formule (IXa-1)

dans lequel $R_1$, $R_{a1}$, $R_{a2}$, $R_{a3}$, $R_{a4}$, T, U, B, E, D, p, n1, n2, n3, n4, m, n sont tels que définis dans la revendication 1 et la liaison en pointillés représente une double liaison facultative et dans lequel :

- chacun des $R_4$, indépendamment l'un de l'autre et à chaque occurrence, est choisi parmi l'hydrogène, le $CF_3$, l'alkyle en $C_{1-4}$ ou le cycloalkyle ;
- chacun des $R_3$, indépendamment l'un de l'autre et à chaque occurrence, est choisi dans le groupe constitué de l'hydrogène, l'halo, le $CF_3$, l'alkyle en $C_{1-4}$, le cycloalkyle, l'$OR_{21}$ et le $N(R_{21})_2$, et dans lequel chacun des $R_{21}$, indépendamment l'un de l'autre et à chaque occurrence, est un hydrogène, un alkyle en $C_{1-4}$ tel que le méthyle, l'éthyle, le propyle, l'isopropyle, le butyle, le *tert-butyle,* l'isobutyle ou le cycloalkyle en $C_{3-6}$ tel que le cyclopropyle, le cyclobutyle, le cyclopentyle, le cyclohexyle .

dans lequel ledit cycloalkyle est un système cyclique monocyclique, bicyclique ou tricyclique de 3 à 7 membres cycliques par cycle.

**6.** Composé (C) selon les formules générales (IIa-1) à (IXa-1) et selon la revendication 5, ou le N-oxyde, le sel pharmaceutiquement acceptable, le solvate pharmaceutiquement acceptable, ou le stéréo-isomère de celui-ci, dans lequel $R_1$, p, $R_{a1}$, $R_{a2}$, $R_{a3}$, $R_{a4}$, T, U, B, E, D, n1, n2, n3, n4, m, n sont tels que définis dans la revendication 2.

**7.** Composé selon la revendication 1, ou le N-oxyde, le sel pharmaceutiquement acceptable, le solvate pharmaceutiquement acceptable, ou le stéréo-isomère de celui-ci, dans lequel ledit composé est de la formule (IIa-a1) à (IXa-a1) [composés (C) de la classe (I) ci-après] :

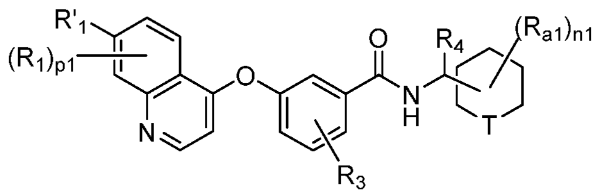

Formule (IIa-a1)

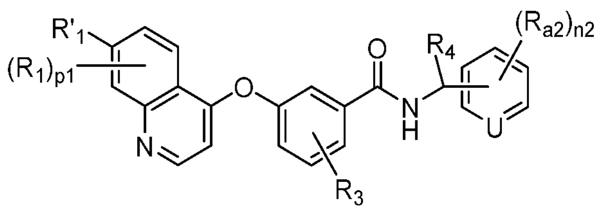

Formule (IIIa-a1)

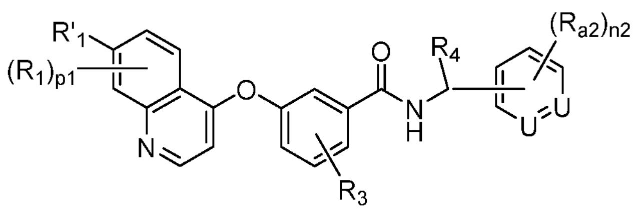

Formule (IVa-a1)

Formule (IVa-a1)

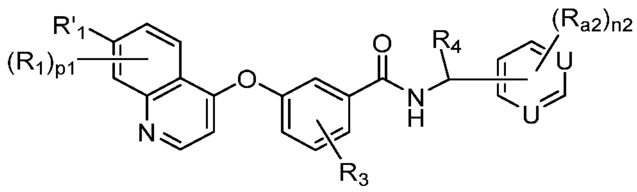

Formule (Va-a1)

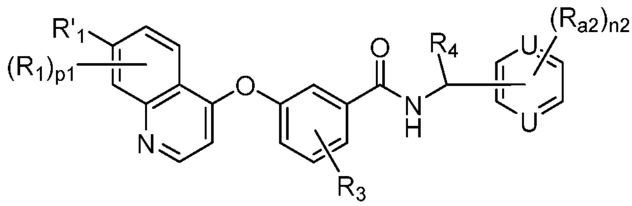

Formule (VIa-a1)

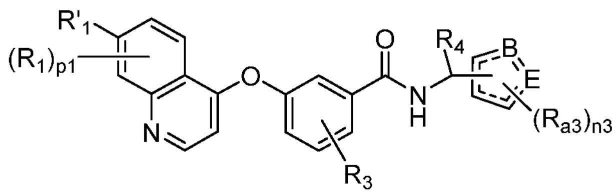

Formule (VIIa-a1)

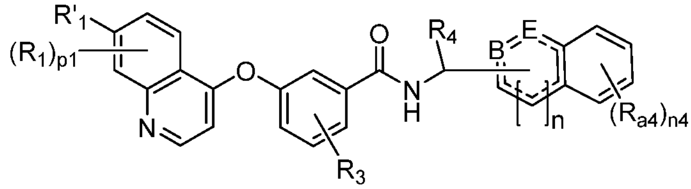

Formule (VIIIa-a1)

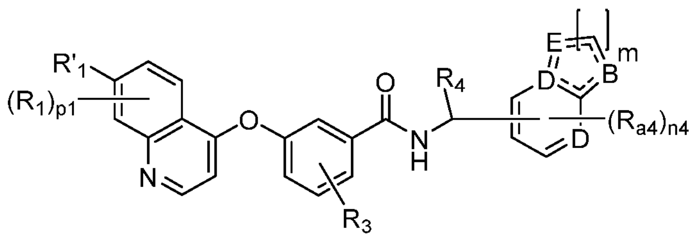

Formule (IXa-a1)

dans lequel $R_1$, $R'_1$, $R_{a1}$, $R_{a2}$, $R_{a3}$, $R_{a4}$, T, U, B, E, D, p1, n1, n2, n3, n4, m, n sont tels que définis dans la revendication 3 et la liaison en pointillés représente une double liaison optionnelle et dans lequel :

- chacun des $R_4$, indépendamment l'un de l'autre et à chaque occurrence, est choisi parmi l'hydrogène, le $CF_3$, l'alkyle en $C_{1-4}$ ou le cycloalkyle ;
- chacun des $R_3$, indépendamment l'un de l'autre et à chaque occurrence, est choisi dans le groupe constitué de l'hydrogène, le halo, le $CF_3$, l'alkyle en $C_{1-4}$, le cycloalkyle, l'$OR_{21}$ et le $N(R_{21})_2$, et dans lequel chacun des $R_{21}$, indépendamment l'un de l'autre et à chaque occurrence, est un hydrogène, un alkyle en $C_{1-4}$ tel que le méthyle, l'éthyle, le propyle, l'isopropyle, le butyle, le *tert-butyle,* l'isobutyle ou un cycloalkyle en $C_{3-6}$ tel que le cyclopropyle, le cyclobutyle, le cyclopentyle, le cyclohexyle ;

dans lequel ledit cycloalkyle est un système cyclique monocyclique, bicyclique ou tricyclique de 3 à 7 membres cycliques par cycle.

**8.** Composé (C) selon les formules générales (IIa-a1) à (IXa-a1) et selon la revendication 7, ou le N-oxyde, sel pharmaceutiquement acceptable, le solvate pharmaceutiquement acceptable, ou le stéréo-isomère de celui-ci,

dans lequel $R_1$, $R_{a1}$, $R_{a2}$, $R_{a3}$, $R_{a4}$, T, U, B, E, D, n1, n2, n3, n4, m, n sont tels que définis dans la revendication 2.

**9.** Composé selon la revendication 1, ou le N-oxyde, le sel pharmaceutiquement acceptable, le solvate pharmaceutiquement acceptable, ou le stéréo-isomère de celui-ci, dans lequel ledit composé est des formules (Xa-1) à (XVIIa-1) [composés (C) de la classe (I) ci-après] :

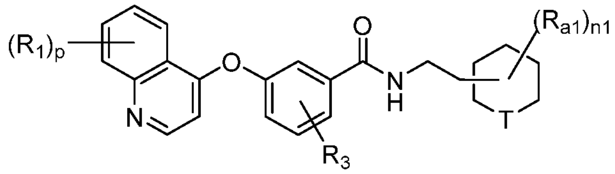

Formule (Xa-1)

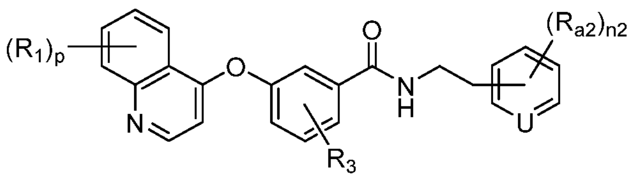

Formule (XIa-1)

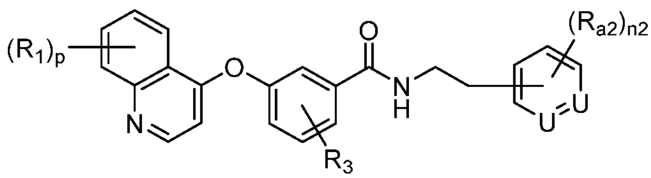

Formule (XIIa-1)

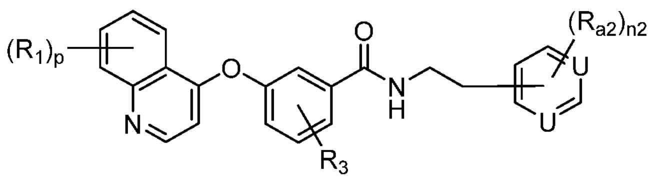

Formule (XIIIa-1)

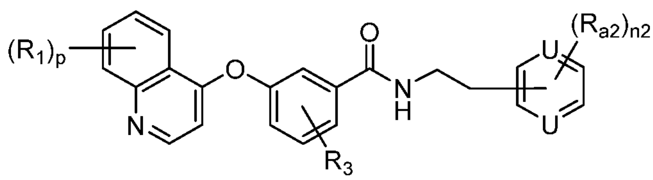

Formule (XIVa-1)

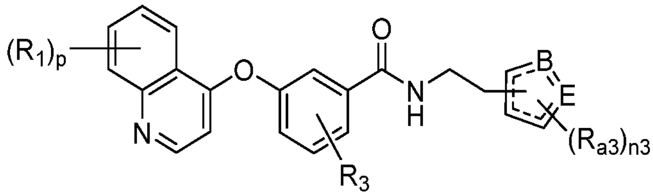

Formule (XVa-1)

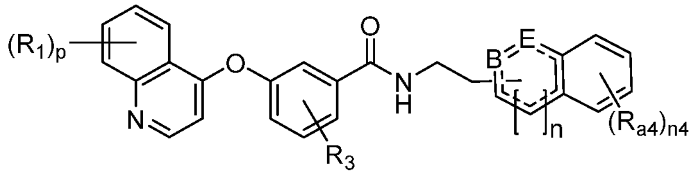

Formule (XVIa-1)

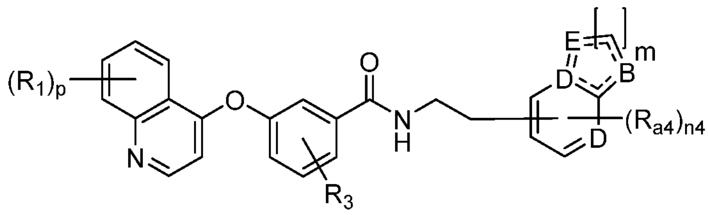

Formule (XVIIa-1)

dans lequel $R_1$, $R_{a1}$, $R_{a2}$, $R_{a3}$, $R_{a4}$, T, U, B, E, D, p, n1, n2, n3, n4, m, n sont tels que définis dans la revendication 1 et la liaison en pointillés représente une double liaison optionnelle et dans lequel :

- chacun des $R_3$, indépendamment l'un de l'autre et à chaque occurrence, est choisi dans le groupe constitué de l'hydrogène, $CF_3$, le halo, l'alkyle en $C_{1-4}$, le cycloalkyle, l'$OR_{21}$, et le $N(R_{21})_2$, et dans lequel chacun des $R_{21}$, indépendamment l'un de l'autre et à chaque occurrence, est un hydrogène, un alkyle en $C_{1-4}$ tel que le méthyle, l'éthyle, le propyle, l'isopropyle, le butyle, le *tert-butyle,* l'isobutyle ou un cycloalkyle en $C_{3-6}$ tel que le cyclopropyle, le cyclobutyle, le cyclopentyle, le cyclohexyle ;

dans lequel ledit cycloalkyle est un système cyclique monocyclique, bicyclique ou tricyclique de 3 à 7 membres par cycle.

**10.** Composé (C) selon les formules générales (Xa-1) à (XVIIa-1) et selon la revendication 9, ou le N-oxyde, le sel pharmaceutiquement acceptable, le solvate pharmaceutiquement acceptable, ou le stéréo-isomère de celui-ci, dans lequel $R_1$, p, $R_{a1}$, $R_{a2}$, $R_{a3}$, $R_{a4}$, T, U, B, E, D, n1, n2, n3, n4, m, n sont tels que définis dans la revendication 2.

**11.** Composé selon la revendication 1, ou le N-oxyde, le sel pharmaceutiquement acceptable, le solvate pharmaceutiquement acceptable, ou le stéréo-isomère de celui-ci, dans lequel ledit composé est de la formule (XVIIIa-1) à (XXVa-1) [composés (C) de la classe (I) ci-après] :

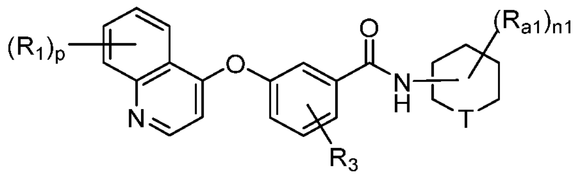

Formule (XVIIIa-1)

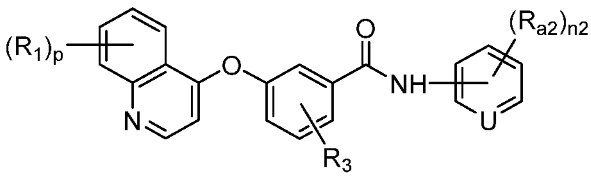

Formule (XIXa-1)

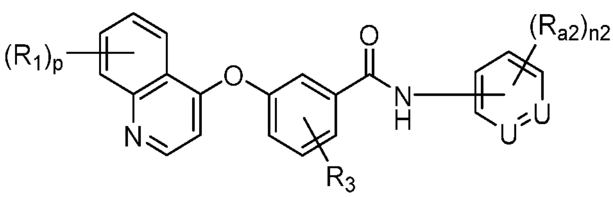

Formule (XXa-1)

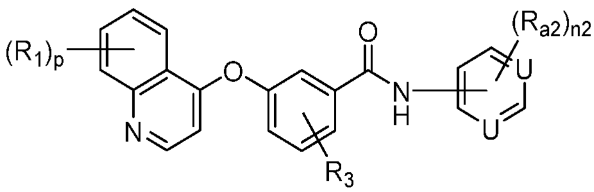

Formule (XXIa-1)

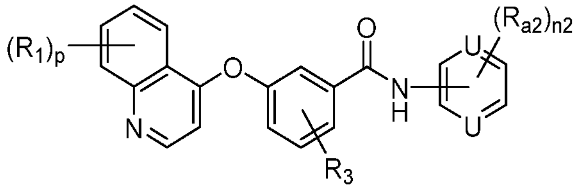

Formule (XXIIa-1)

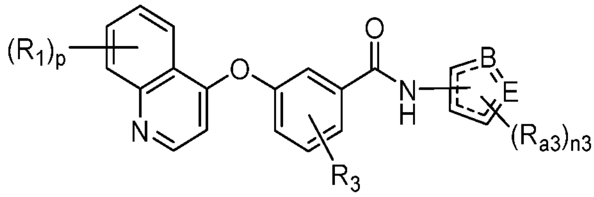

Formule (XXIIIa-1)

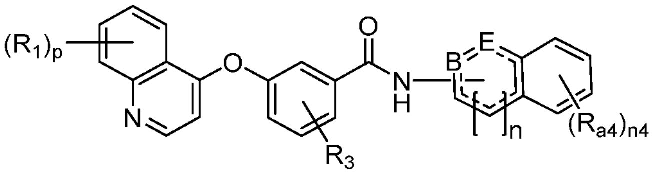

Formule (XXIVa-1)

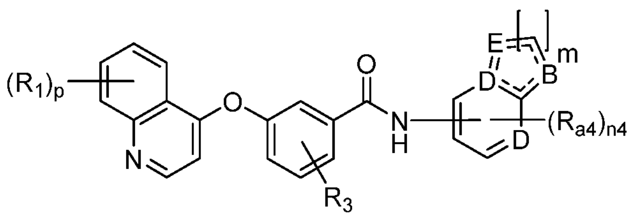

Formule (XXVa-1)

dans lequel $R_1$, $R_{a1}$, $R_{a2}$, $R_{a3}$, $R_{a4}$, T, U, B, E, D, p, n1, n2, n3, n4, m, n sont définis comme dans la revendication 1 et la liaison en pointillés représente une double liaison facultative et dans lequel

- chacun des $R_3$, indépendamment l'un de l'autre et à chaque occurrence, est choisi dans le groupe constitué de l'hydrogène, le $CF_3$, l'halo, l'alkyle en $C_{1-4}$, le cycloalkyle, l'$OR_{21}$, et le $N(R_{21})_2$, et dans lequel chacun des $R_{21}$, indépendamment l'un de l'autre et à chaque occurrence, est un hydrogène, un alkyle en $C_{1-4}$ tel que le méthyle, l'éthyle, le propyle, l'isopropyle, le butyle, le *tert-butyle,* l'isobutyle ou un cycloalkyle en $C_{3-6}$ tel que le cyclopropyle, le cyclobutyle, le cyclopentyle, le cyclohexyle ;

dans lequel ledit cycloalkyle est un système cyclique monocyclique, bicyclique ou tricyclique de 3 à 7 membres par cycle.

**12.** Composé (C) selon les formules générales (XVIIIa-1) à (XXVa-1) et selon la revendication 11, ou le N-oxyde, le sel pharmaceutiquement acceptable, le solvate pharmaceutiquement acceptable, ou le stéréo-isomère de celui-ci, dans lequel R1, p $R_{a1}$, $R_{a2}$, $R_{a3}$, $R_{a4}$, T, U, B, E, D, n1, n2, n3, n4, m, n sont tels que définis dans la revendication 2.

**13.** Composé (C) selon la revendication 5 ou le N-oxyde, le sel pharmaceutiquement acceptable, le solvate pharmaceutiquement acceptable ou le stéréo-isomère de celui-ci, dans lequel le composé de formule (IIIa-1) est un composé selon la formule (LXI), (LXIII), (LXXII), (LXXIV)-(LXXXV), (LXXXVI), (CXIII), (CLXXXVI), (CLXXXVIII), (CXC)-(CXCVI), (CXCVII-6)-(CXCVII-7), (CXCVII-16)-(CXCVII-17), (CXCVII-19)-(CXCVII-20), (CXCVII-27), (CXCVII-33)-(CXCVII-35), ou (CXCVII-37)-(CXCVII-38) ci-dessous :

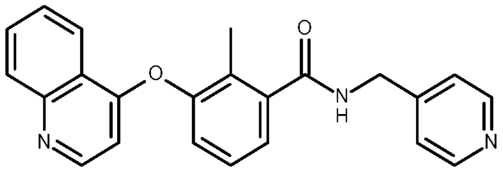

Formule (LXI)

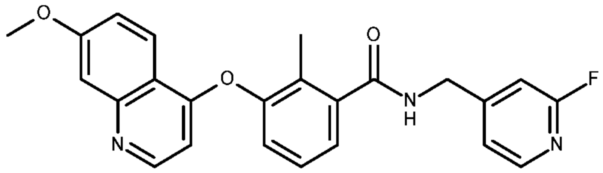

Formule (LXIII)

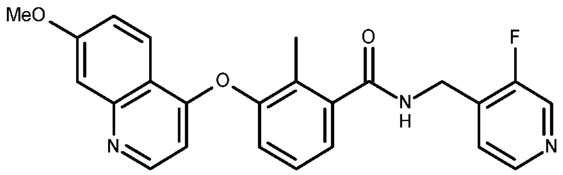

Formule (LXXII)

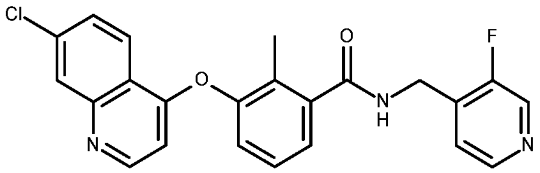

Formule (LXXIV)

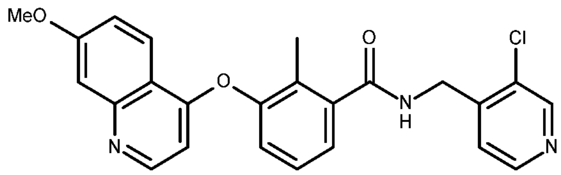

Formule (LXXV)

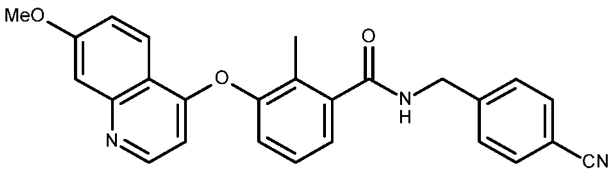

Formule (LXXXVI)

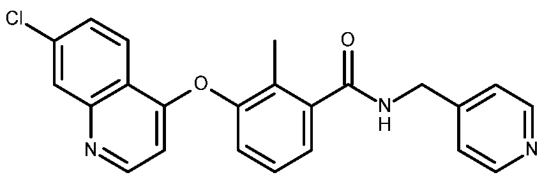

Formule (CXIII)

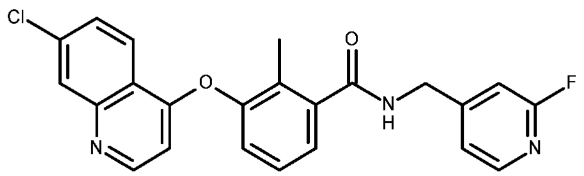

Formule (CLXXXVI)

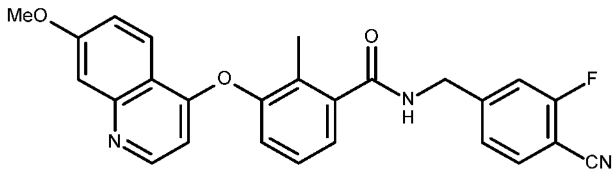

Formule (CLXXXVII)

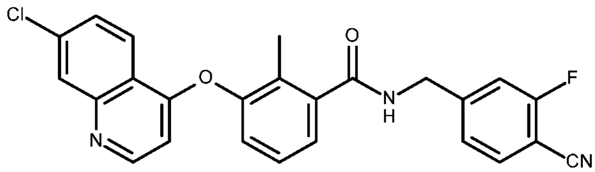

Formule (CLXXXVIII)

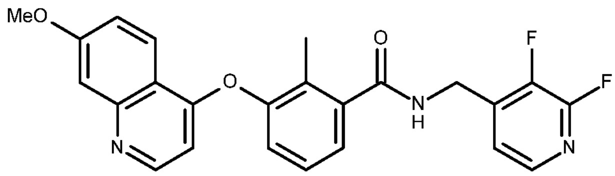

Formule (CXC)

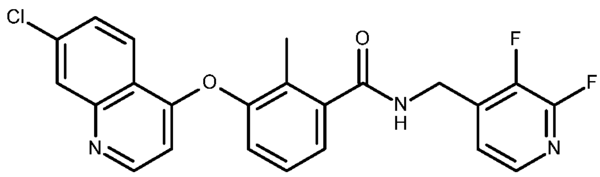

Formule (CXCI)

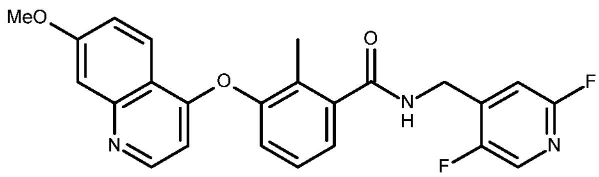

Formule (CXCII)

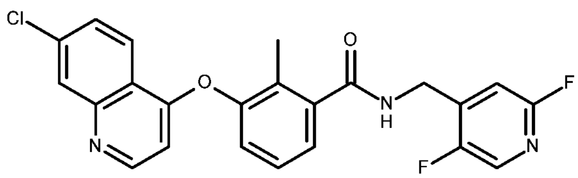

Formule (CXCIII)

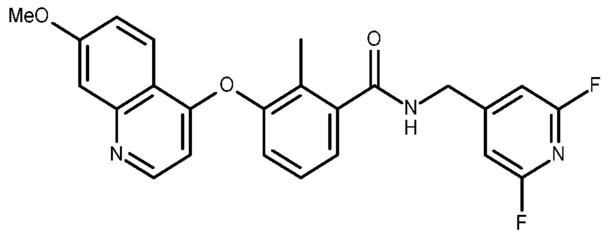

Formule (CXCIV)

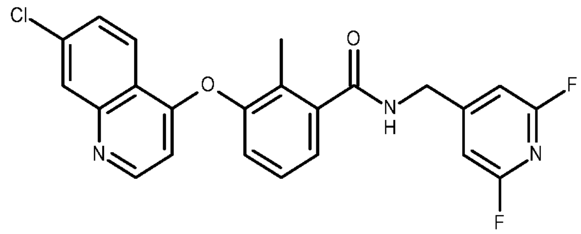

Formule (CXCV)

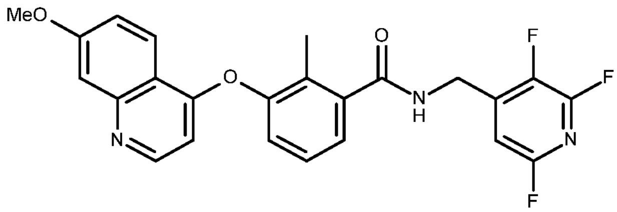

Formule (CXCVI)

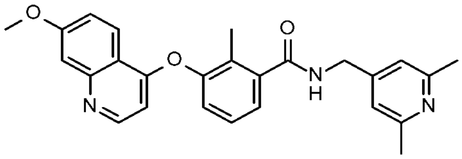

Formule (CXCVIII-6)

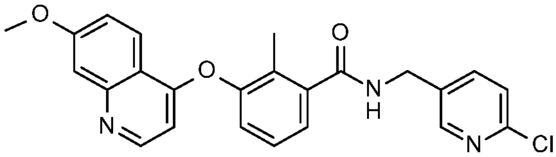

Formule (CXCVIII-7)

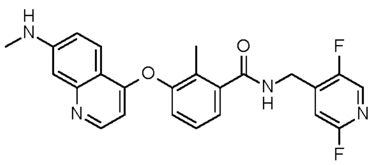

Formule (CXCVIII-16)

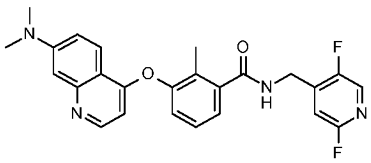

Formule (CXCVIII-17)

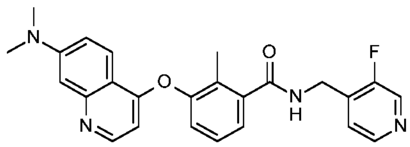

Formule (CXCVIII-19)

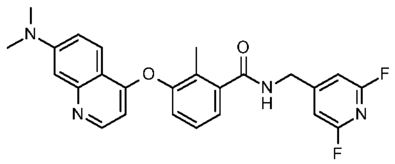

Formule (CXCVIII-20)

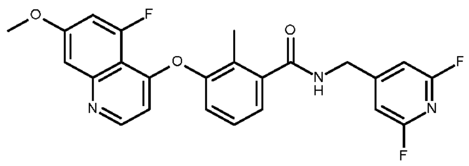

Formule (CXCVIII-27)

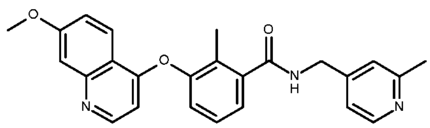

Formule (CXCVIII-33)

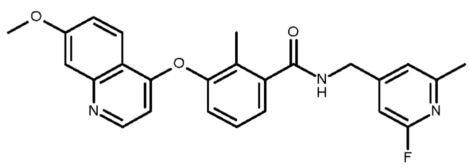

Formule (CXCVIII-34)

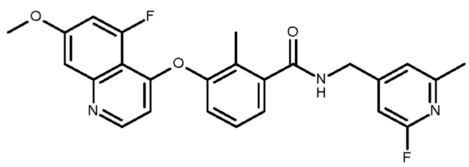

Formule (CXCVIII-35)

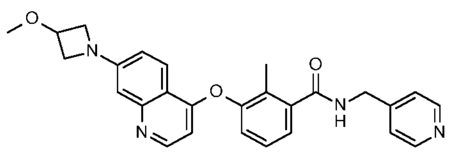

Formule (CXCVIII-37)

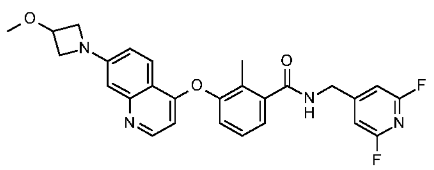

Formule (CXCVIII-38)

**14.** Composé (C) selon la revendication 7 ou le N-oxyde, le sel pharmaceutiquement acceptable, le solvate pharmaceutiquement acceptable ou le stéréo-isomère de celui-ci, dans lequel le composé de formule (IIIa-a1) est un composé selon la formule (CXCVIII-3), (CXCVIII-7), (CXCVIII-11), (CXCVIII-16), (CXCVIII-19)-(CXCVIII-21), (CXCVIII-23)-(CXCVIII-26), (CXCVIII-28)-(CXCVIII-31), (CXCVIII-33)-(CXCVIII-39), (CXCVIII-42)-(CXCVIII-47),

(CXCVIII-49), (CXCVIII-51)-(CXCVIII-53), (CXCVIII-56), (CXCVIII-59), (CXCVIII-62), (CXCVIII-66), (CXCVIII-67), (CXCVIII-70), (CXCVIII-71), (CXCVIII-73)-(CXCVIII-75), (CXCVIII-81)-(CXCVIII-86), ou (CXCVIII-88)-(CXCVIII-90) ci-dessous :

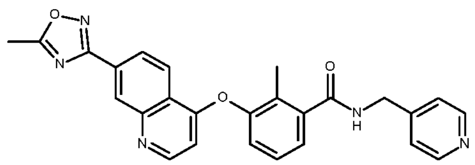

Formule (CXCVIII-3)

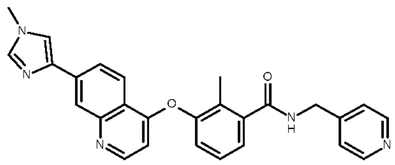

Formule (CXCVIII-7)

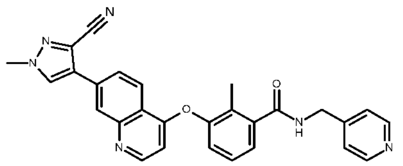

Formule (CXCVIII-11)

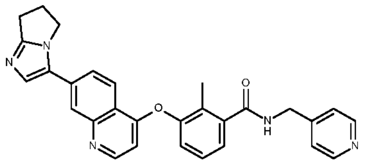

Formule (CXCVIII-16)

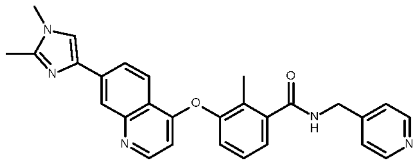

Formule (CXCVIII-19)

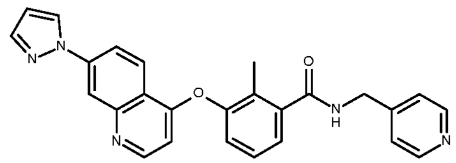

Formule (CXCVIII-20)

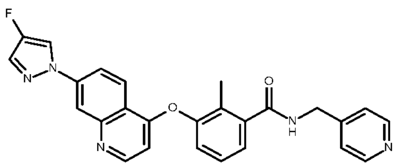

Formule (CXCVIII-21)

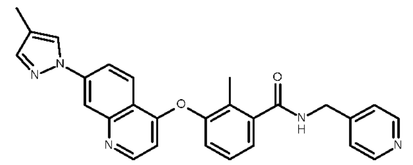

Formule (CXCVIII-23)

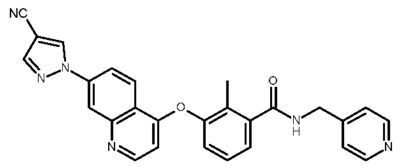

Formule (CXCVIII-24)

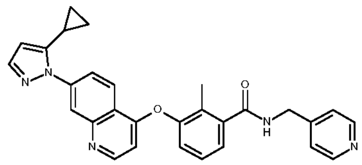

Formule (CXCVIII-25)

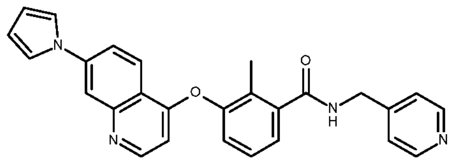

Formule (CXCVIII-26)

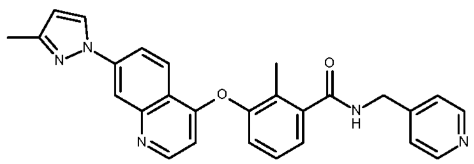

Formule (CXCVIII-28)

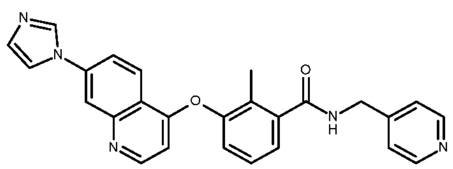

Formule (CXCVIII-29)

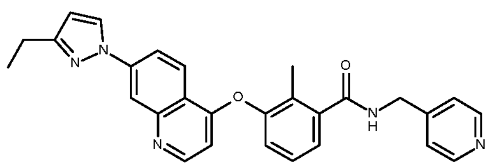

Formule (CXCVIII-30)

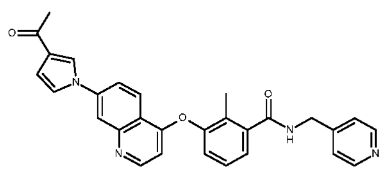

Formule (CXCVIII-31)

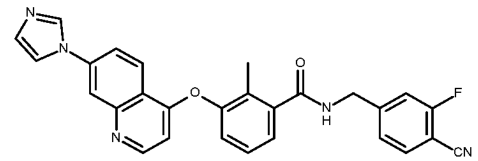

Formule (CXCVIII-33)

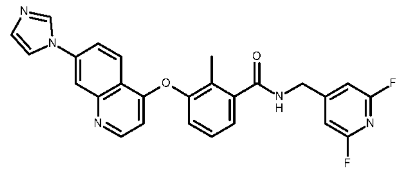

Formule (CXCVIII-34)

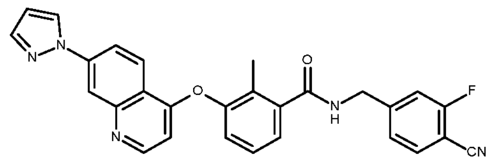

Formule (CXCVIII-35)

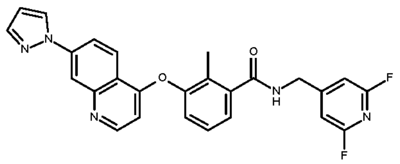

Formule (CXCVIII-36)

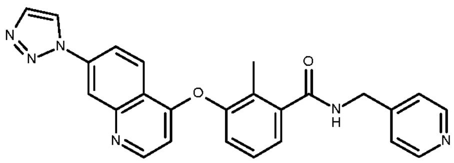

Formule (CXCVIII-37)

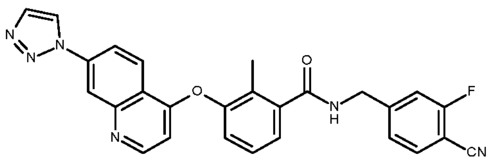

Formule (CXCVIII-38)

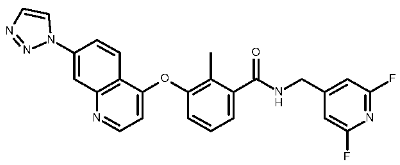

Formule (CXCVIII-39)

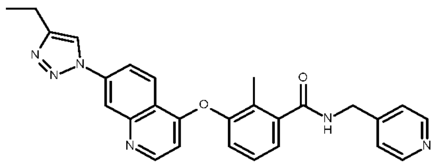

Formule (CXCVIII-42)

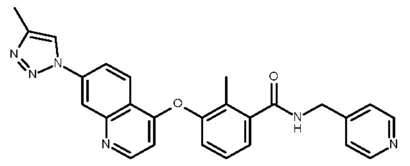

Formule (CXCVIII-43)

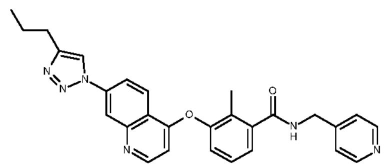

Formule (CXCVIII-44)

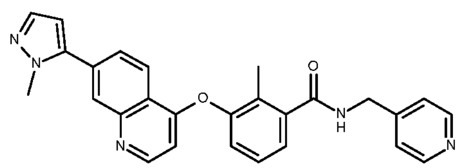

Formule (CXCVIII-45)

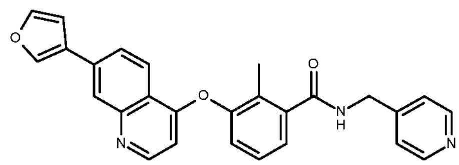

Formule (CXCVIII-46)

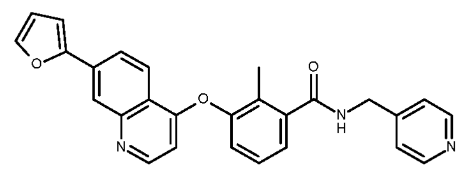

Formule (CXCVIII-47)

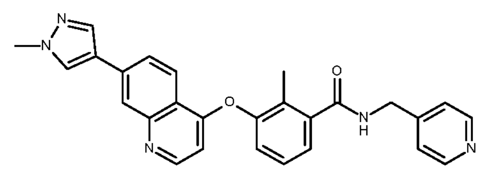

Formule (CXCVIII-49)

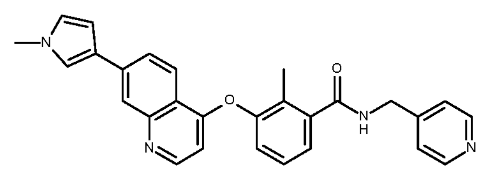

Formule (CXCVIII-51)

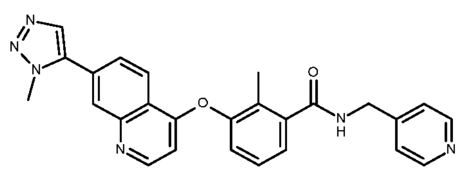

Formule (CXCVIII-52)

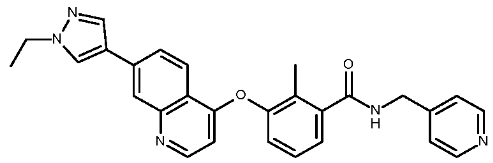

Formule (CXCVIII-53)

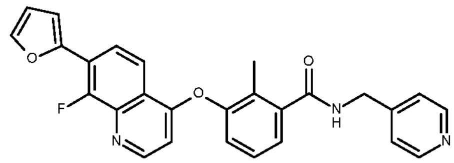

Formule (CXCVIII-56)

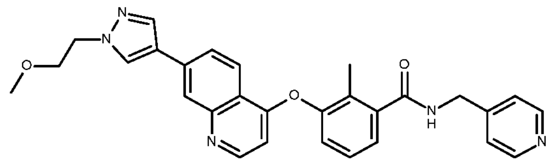

Formule (CXCVIII-59)

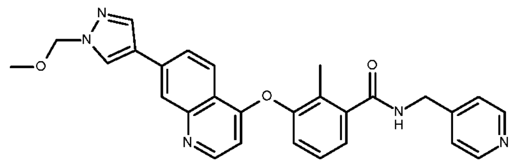

Formule (CXCVIII-62)

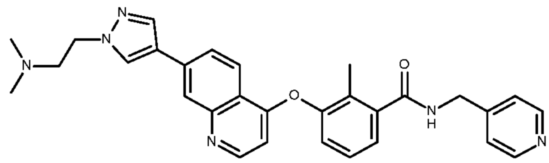

Formule (CXCVIII-66)

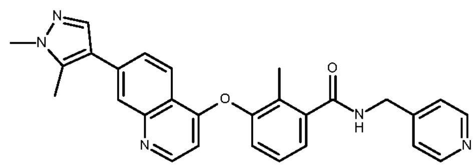

Formule (CXCVIII-67)

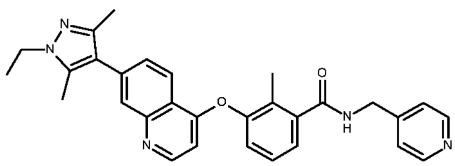

Formule (CXCVIII-70)

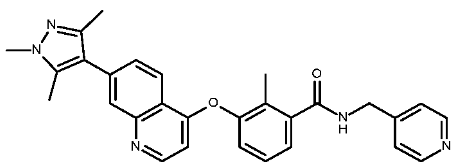

Formule (CXCVIII-71)

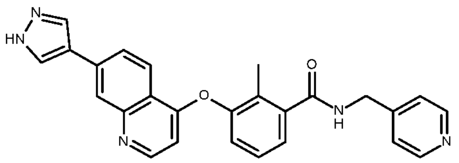

Formule (CXCVIII-73)

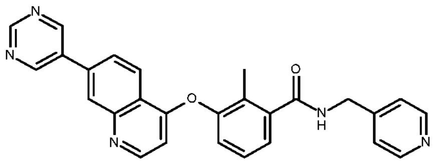

Formule (CXCVIII-74)

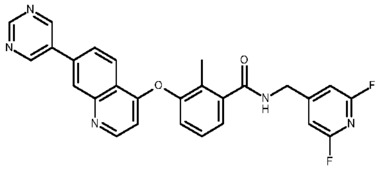

Formule (CXCVIII-75)

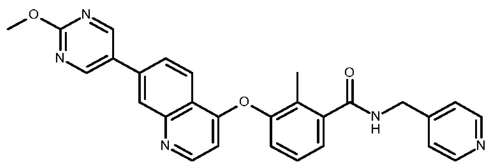

Formule (CXCVIII-81)

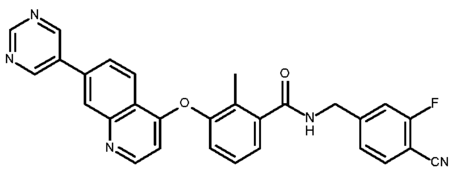

Formule (CXCVIII-82)

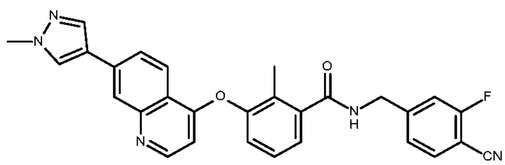

Formule (CXCVIII-83)

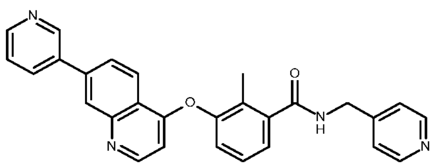

Formule (CXCVIII-84)

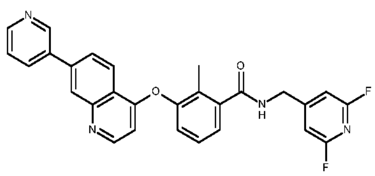

Formule (CXCVIII-85)

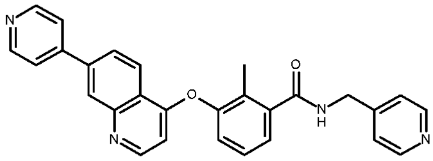

Formule (CXCVIII-86)

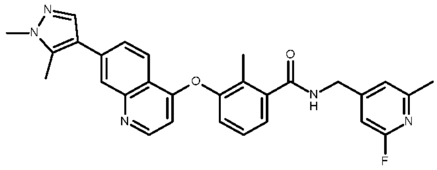

Formule (CXCVIII-88)

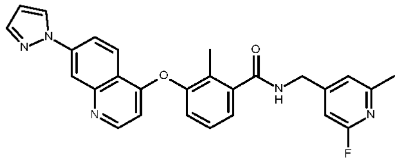

Formule (CXCVIII-89)

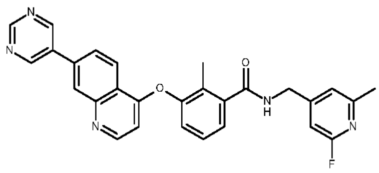

Formule (CXCVIII-90)

**15.** Composition pharmaceutique comprenant un support, et comme ingrédient actif le composé (C), tel que défini selon l'une quelconque des revendications 1 à 14.

**16.** Composé (C), tel que défini selon l'une quelconque des revendications 1 à 14, pour utilisation comme médicament.

**17.** Composé tel que défini selon l'une quelconque des revendications 1 à 14, pour utilisation dans le traitement d'une maladie choisie parmi le cancer, les troubles métaboliques, les troubles inflammatoires et auto-immuns, les troubles neurologiques, l'athérosclérose et les maladies cardiovasculaires, le syndrome de Sjögren, le rejet d'allogreffe rénale, les maladies d'origine virale, les maladies circulatoires, l'ostéolyse osseuse et l'ostéoporose, l'arthrose, la sarcopénie, l'histiocytose à cellules de Langerhans, les lésions de la moelle épinière, l'endométriose, l'asthme et l'asthme allergique, les maladies oculaires, les douleurs chroniques et neuropathiques, et les maladies fibro-prolifératives.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004022572 A
- WO 2006116713 A
- WO 2010039248 A
- EP 3293177 A
- EP 0269574 A
- WO 2003104482 A
- WO 1996040705 A
- WO 2001040245 A
- WO 2005117882 A
- WO 2011090738 A2
- US 20090325945 A
- US 20150182526 A
- WO 2017049462 A1


### Non-patent literature cited in the description


- **KUBO KAZUO et al.** *Biooganic & medicinal chemistry*, vol. 1 (23), 5117-5133
- **J. ZHANG et al.** *Mini-Reviews in Medicinal Chemistry*, 2011, vol. 11, 920-946